(19) Europäisches Patentamt
European Patent Office
Office européen des brevets

(11) EP 0 596 406 B1

(12) EUROPEAN PATENT SPECIFICATION

(45) Date of publication and mention of the grant of the patent:
**16.12.1998 Bulletin 1998/51**

(51) Int Cl.[6]: **C07D 471/04**, A61K 31/435

(21) Application number: **93117474.2**

(22) Date of filing: **28.10.1993**

# (54) Imidazo (1,2-a) Pyridines as bradykinin antagonists

Imidazo (1,2-a)pyridine als Bradykinin-Antagonisten

Imidazo (1,2-a) pyridines antagonistes de la bradykinine

(84) Designated Contracting States:
**AT BE CH DE DK ES FR GB GR IE IT LI LU NL PT SE**

(30) Priority: **02.11.1992 GB 9222947**
**03.03.1993 GB 9304249**

(43) Date of publication of application:
**11.05.1994 Bulletin 1994/19**

(73) Proprietor: **FUJISAWA PHARMACEUTICAL CO., LTD.**
**Osaka-shi Osaka 541 (JP)**

(72) Inventors:
- **Oku, Teruo**
  **Tsukuba-shi, Ibaraki 305 (JP)**
- **Kayakiri, Hiroshi**
  **Tsukuba-shi, Ibaraki 305 (JP)**
- **Satoh, Shigeki**
  **Tsukuba-shi, Ibaraki 305 (JP)**
- **Abe, Yoshito**
  **Inashiki-gun, Ibaraki 300-12 (JP)**
- **Sawada, Yuki**
  **Tsukuba-shi, Ibaraki 305 (JP)**
- **Tanaka, Hirokazu**
  **Takarazuka-shi, Hyogo 665 (JP)**

(74) Representative: **Türk, Gille, Hrabal**
**Brucknerstrasse 20**
**40593 Düsseldorf (DE)**

(56) References cited:
**EP-A- 0 150 255** **FR-A- 2 638 161**

- **CHEMICAL ABSTRACTS, vol. 109, no. 7, 15 August 1988, Columbus, Ohio, US; abstract no. 48452v,**
- **J. MED. CHEM., vol.28, no.7, 1985 pages 876 - 892 J.J. KAMINSKI ET AL.**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 596 406 B1

## Description

This invention relates to new heterocyclic compounds and pharmaceutically acceptable salts thereof.

More particularly, it relates to new heterocyclic compounds and pharmaceutically acceptable salts thereof which have activities as bradykinin antagonists, to processes for preparation thereof, to a pharmaceutical composition comprising the same, and to methods of using the same therapeutically in the prevention and/or the treatment of bradykinin or its analogues mediated diseases such as allergy, inflammation, autoimmune disease, shock, or pain, in human being or animals.

One object of this invention is to provide new and useful heterocyclic compounds and pharmaceutically acceptable salts thereof which possess activities as bradykinin antagonists.

Another object of this invention is to provide processes for the preparation of said compounds and salts thereof.

A further object of this invention is to provide a pharmaceutical composition comprising, as an active ingredient, said heterocyclic compounds and pharmaceutically acceptable salts thereof.

Still further object of this invention is to provide a therapeutical method for the prevention and/or the treatment of bradykinin or its analogues mediated diseases such as allergy, inflammation, autoimmune disease, shock, or pain using said heterocyclic compounds and pharmaceutically acceptable salts thereof.

Some heterocyclic compounds have been known as described, for example, in J. Med. Chem., <u>28</u>, 876-892 (1985). However, it is not known that said compounds have activities as bradykinin antagonists.

The object heterocyclic compounds of this invention are new and can be represented by the following general formula [I] :

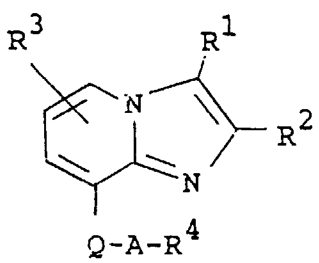

[I]

wherein $R^1$, $R^2$, $R^4$, Q, and A are each as defined in claim 1.

The object compound [I] or its salt can be prepared by processes as illustrated in the following reaction schemes.

<u>Process 1</u>

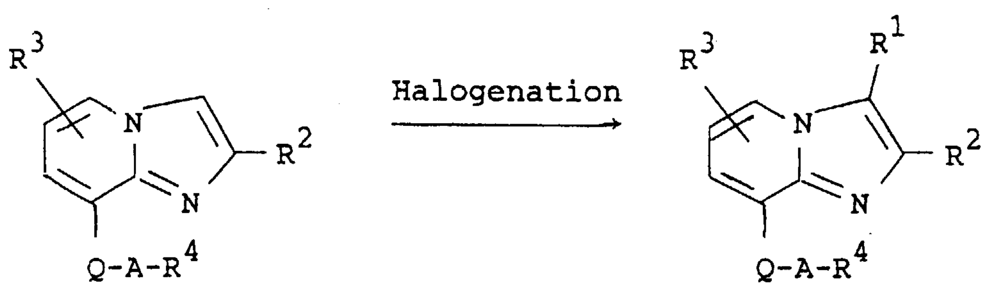


[II]
or its salt

[I]
or its salt

Process 2

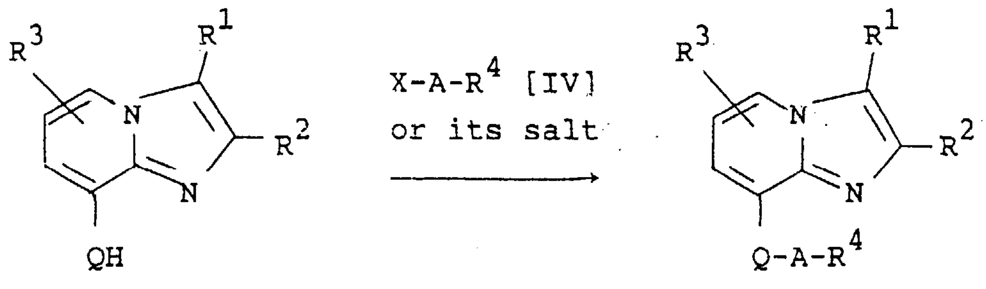


[III]
or its salt

[I]
or its salt

Process 3

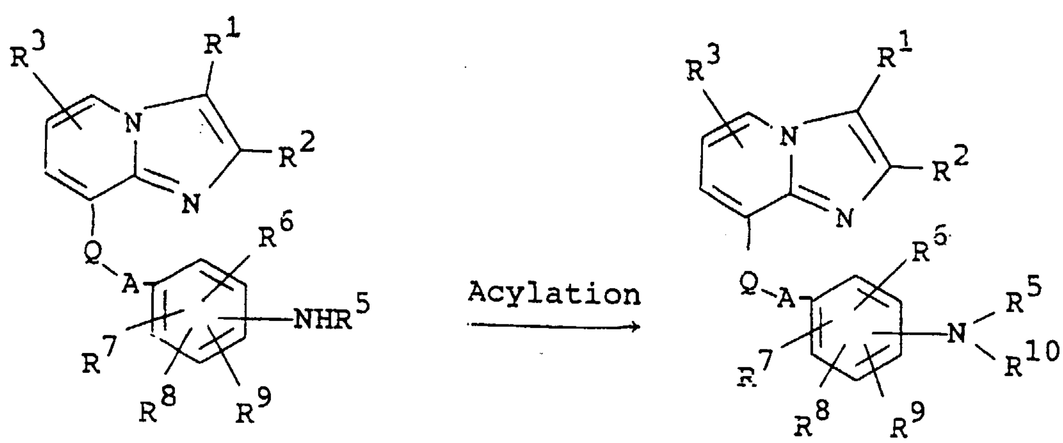


[Ia]
or its salt

[Ib]
or its salt

Process 4

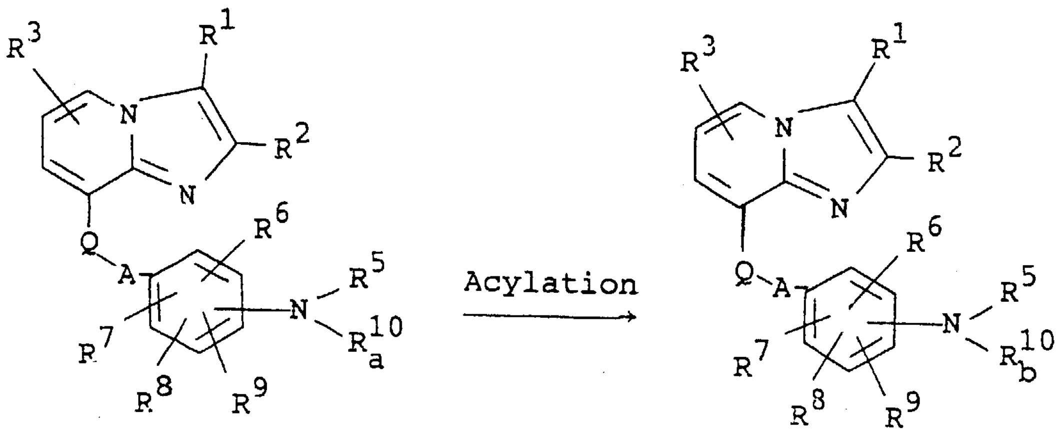


[Ic]
or its salt

[Id]
or its salt

Process 5

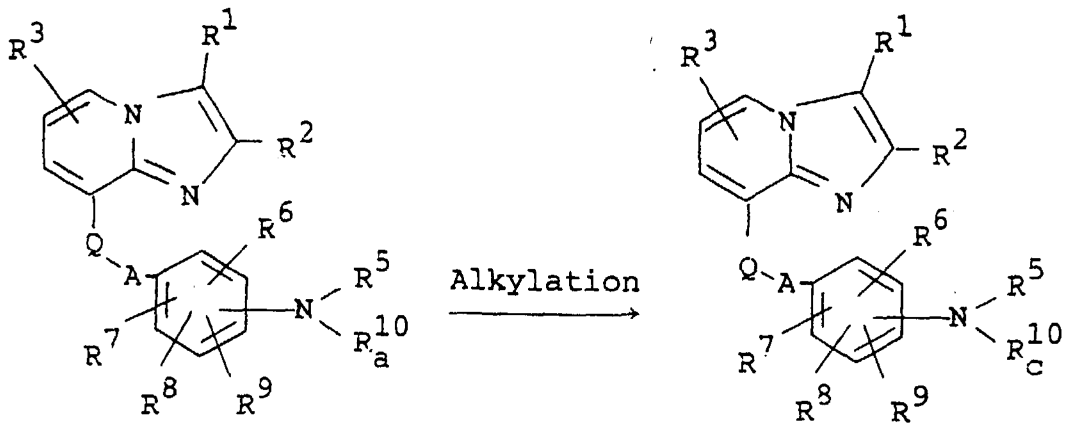


[Ic]
or its salt

[Ie]
or its salt

Process 6

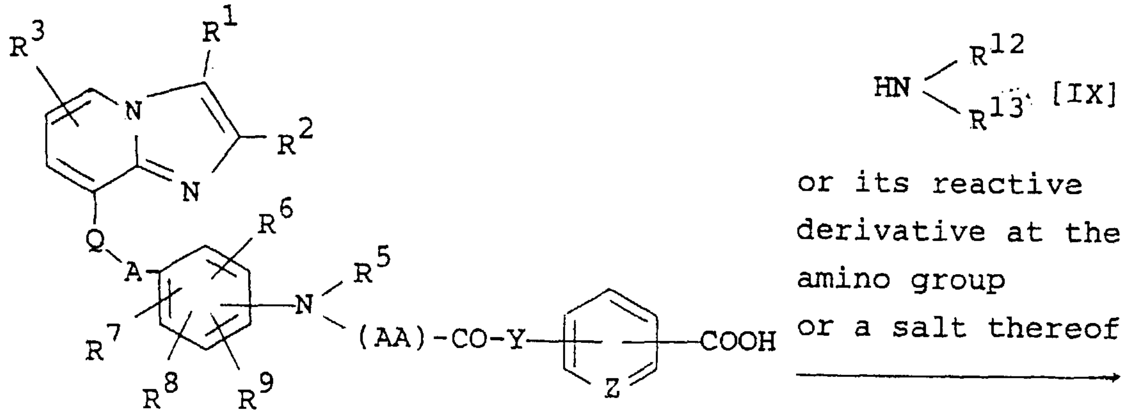


[If]

or its reactive derivative
at the carboxy group
or a salt thereof

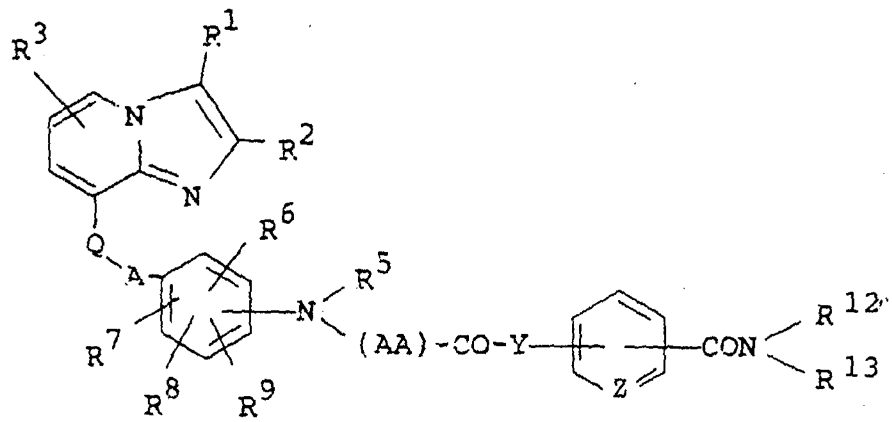

[Ig]

or its salt
wherein $R^5$, $R^6$, $R^7$, $R^8$ $R^9$ $R^{10}$, $R^{10}_a$ $R^{10}_b$ $R^{10}_c$ $R^{12}$ $R^{13}$, (AA), X Y, Z, $R^1$, $R^2$, $R^3$, $R^4$, Q and A are each as defined in the claims.

In the above and subsequent description of the present specification, suitable examples of the various definitions to be included within the scope of the invention are explained in detail in the following.

"Halogen" may be fluorine, chlorine, bromine and iodine.

"Aryl" is phenyl, naphthyl, phenyl substituted with $C_1$-$C_6$ alkyl [e.g. tolyl, xylyl, mesityl, cumenyl, di(tert-butyl)phenyl] in which preferable one is phenyl and tolyl.

Suitable "$C_1$-$C_6$ alkyl" and $C_1$-$C_6$ alkyl moiety in the terms "heterocyclic($C_1$-$C_6$)alkyl", and "$C_1$-$C_6$ alkylamino" may be straight or branched one such as methyl, ethyl, propyl, isopropyl, butyl, isobutyl, tert-butyl, pentyl, or hexyl in which preferable one is $C_1$-$C_4$ lower alkyl such as methyl, ethyl, propyl, isobutyl or tert-butyl.

Suitable "$C_2$-$C_6$ alkylene" may be a straight or branched one such as methylene, ethylene, trimethylene, methylmethylene, tetramethylene, ethylethylene, propylene, pentamethylene, hexamethylene, in which the most preferable

one is methylene.

Suitable "halo($C_1$-$C_6$)alkyl" may be fluoromethyl, difluoromethyl, trifluoromethyl, chloromethyl, dichloromethyl, bromomethyl, fluoroethyl, difluoroethyl, chloroethyl, dichloroethyl.

Suitable "($C_2$-$C_6$)alkenylene" may be a straight or branched $C_2$-$C_6$ alkenylene such as vinylene, methylvinylene, propenylene, 1,3-butadienylene, in which the most preferable one is vinylene.

Suitable "acyl" is alkanoyl selected from formyl, acetyl, propionyl, butyryl, isobutyryl, valeryl, isovaleryl, pivaloyl, hexanoyl, heptanoyl, and 3,3-dimethylbutyryl, halo-($C_1$-$C_6$)alkanoyl [e.g. chloroacetyl, trifluoroacetyl, bromoacetyl, bromobutyryl, heptafluorobutyryl], hydroxy-($C_1$-$C_6$)alkanoyl [e.g. glycoloyl, lactoyl, 3-hydroxypropionyl, glyceroyl], ($C_1$-$C_6$)alkylsulfonyloxy($C_1$-$C_6$)alkanoyl [e.g. mesyloxyacetyl, ethylsulfonyloxyacetyl, mesyloxypropionyl], ($C_1$-$C_6$)alkoxy($C_1$-$C_6$)alkanoyl [e.g. methoxyacetyl, ethoxyacetyl, methoxypropionyl, ethoxypropionyl, propoxypropionyl, methoxybutyryl], ($C_1$-$C_6$)alkylthio-($C_1$-$C_6$)alkanoyl [e.g. methylthioacetyl, ethylthioacetyl, methylthiopropionyl, ethylthiopropionyl, propylthiopropionyl, methylthiobutyryl], ($C_1$-$C_6$)alkanoyloxy($C_1$-$C_6$)alkanoyl [e.g. acetyloxyacetyl, acetyloxypropionyl, propionyloxyacetyl], aryloxy($C_1$-$C_6$)alkanoyl [e.g. phenyloxyacetyl, phenyloxypropionyl, tolyloxyacetyl, naphthyloxyacetyl], aroyl($C_1$-$C_6$)alkanoyl [e.g. phenyloxalyl, benzoylacetyl, benzoylpropionyl], carboxy($C_1$-$C_6$)alkanoyl [e.g. oxalo, carboxyacetyl, 3-carboxypropionyl, 3-carboxybutyryl, 4-carboxybutyryl, 4-carboxyvaleryl $C_1$-$C_6$ alkoxycarbonyl($C_1$-$C_6$)alkanoyl [e.g. methoxycarbonylacetyl, ethoxycarbonylacetyl, methoxycarbonylpropionyl, ethoxycarbonylpropionyl], carbamoyl($C_1$-$C_6$)alkanoyl [e.g. carbamoylacetyl, carbamoylpropionyl], $C_1$-$C_6$ alkylcarbamoyl($C_1$-$C_6$)alkanoyl [e.g. methylcarbamoylacetyl, methylcarbamoylpropionyl, ethylcarbamoylpropionyl, dimethylcarbamoylpropionyl, (N-methyl-N-ethylcarbamoyl)-propionyl], ar($C_1$-$C_6$)alkanoyl [e.g. phenylacetyl, tolylacetyl, naphthylacetyl, 2-phenylpropionyl, 3-phenylpropionyl, 4-phenylbutyryl, tritylcarbonyl], optionally substituted heterocyclic($C_1$-$C_6$)alkanoyl [e.g. morpholinoacetyl, thiomorpholinoacetyl, morpholinopropionyl, thiomorpholinopropionyl, piperidinopropionyl, piperazinylpropionyl, pyridylacetyl, pyrrolidinylpropionyl, imidazolidinylpropionyl, piperidinoacetyl, pyrrolidinylacetyl, hexamethyleneiminoacetyl, hexamethyleneiminopropionyl, imidazolylacetyl, furylacetyl, thienylacetyl, methylpiperazinylacetyl, pyridylpiperazinylacetyl], heterocyclicthio($C_1$-$C_6$)alkanoyl [e.g. pyridylthioacetyl, pyrimidinylthioacetyl, imidazolylthiopropionyl], $C_3$-$C_6$ alkenoyl [e.g. acryloyl, crotonoyl, isocrotonoyl, 3-butenoyl, 3-pentenoyl, 4-pentenoyl, methacryloyl], $C_3$-$C_6$ alkynoyl [e.g. propioloyl, 2-butynoyl, 3-butynoyl], cyclo($C_3$-$C_6$)alkylcarbonyl [e.g. cyclopropylcarbonyl, cyclobutylcarbonyl, cyclopentylcarbonyl, cyclohexylcarbonyl], cyclo($C_3$-$C_6$)alkenylcarbonyl [e.g. cyclopentenylcarbonyl, cyclohexenylcarbonyl], carboxy, $C_1$-$C_6$ alkoxycarbonyl [e.g. methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl], aryloxycarbonyl [e.g. phenoxycarbonyl] aroyl [e.g. benzoyl, toluoyl, xyloyl, naphthoyl], $C_1$-$C_6$ alkoxyaroyl [e.g. methoxybenzoyl], haloaroyl [e.g. chlorobenzoyl, fluorobenzoyl $C_1$-$C_6$ alkoxycarbonylaroyl [e.g. methoxycarbonylbenzoyl ar(($C_3$-$C_6$))alkenoyl [e.g. cinnamoyl, allocinnamoyl, $\alpha$-methylcinnamoyl, 4-methylcinnamoyl], $C_1$-$C_6$ alkoxy-ar($C_3$-$C_6$)alkenoyl [e.g. methoxycinnamoyl, ethoxycinnamoyl, dimethoxycinnamoyl], $C_1$-$C_6$ alkylenedioxy-ar($C_3$-$C_6$)alkenoyl [e.g. methylenedioxycinnamoyl, ethylenedioxycinnamoyl], nitro-ar($C_3$-$C_6$)alkenoyl [e.g. nitrocinnamoyl], halo-ar($C_3$-$C_6$)alkenoyl [e.g. chlorocinnamoyl, fluorocinnamoyl], hydroxy-ar($C_3$-$C_6$)alkenoyl [e.g. hydroxycinnamoyl], hydroxy($C_1$-$C_6$)alkoxy-ar($C_3$-$C_6$)alkenoyl [e.g. hydroxymethoxycinnamoyl, hydroxyethoxycinnamoyl], amino($C_1$-$C_6$)alkoxy-ar($C_3$-$C_6$)alkenoyl [e.g. aminoethoxycinnamoyl], $C_1$-$C_6$ alkylamino($C_1$-$C_6$)-alkoxy-ar($C_3$-$C_6$)alkenoyl [e.g. methylaminomethoxycinnamoyl, dimethylaminoethoxycinnamoyl], heterocyclic($C_1$-$C_6$)alkoxy-ar($C_3$-$C_6$)alkenoyl [e.g. pyridylmethoxycinnamoyl], optionally substituted heterocyclic-ar($C_3$-$C_6$)alkenoyl [e.g. morpholinocinnamoyl, methylpiperazinylcinnamoyl, pyrrolidinylcinnamoyl, oxopyrrolidinylcinnamoyl, oxopiperidinocinnamoyl, dioxopyrrolidinylcinnamoyl, oxooxazolidinylcinnamoyl, pyrrolylcinnamoyl], amino-ar($C_3$-$C_6$)alkenoyl [e.g. aminocinnamoyl], $C_1$-$C_6$ alkylamino-ar($C_3$-$C_6$)alkenoyl [e.g. methylaminocinnamoyl, dimethylaminocinnamoyl], $C_1$-$C_6$ alkanoylamino-ar($C_3$-$C_6$)alkenoyl [e.g. acetylaminocinnamoyl, propionylaminocinnamoyl], hydroxy($C_1$-$C_6$)alkanoylamino-ar($C_3$-$C_6$)alkenoyl [e.g. hydroxyacetylaminocinnamoyl, hydroxypropionylaminocinnamoyl], $C_1$-$C_6$ alkoxy($C_1$-$C_6$)alkanoylamino-ar($C_3$-$C_6$)alkenoyl [e.g. methoxyacetylaminocinnamoyl, methoxypropionylaminocinnamoyl], halo($C_1$-$C_6$)alkanoylamino-ar($C_3$-$C_6$)alkenoyl [e.g. chloroacetylaminocinnamoyl, bromobutyrylaminocinnamoyl], amino($C_1$-$C_6$)alkanoylamino-ar($C_3$-$C_6$)alkenoyl [e.g. aminoacetylaminocinnamoyl, aminopropionylaminocinnamoyl], $C_1$-$C_6$ alkylamino($C_1$-$C_6$)alkanoylamino-ar($C_3$-$C_6$)-alkenoyl [e.g. methylaminoacetylaminocinnamoyl, dimethylaminoacetylaminocinnamoyl], $C_1$-$C_6$-alkanoylamino($C_1$-$C_6$)alkanoylamino-ar($C_3$-$C_6$)alkenoyl [e.g. acetylaminoacetylaminocinnamoyl, acetylaminopropionylaminocinnamoyl], carboxy($C_1$-$C_6$)alkanoylamino-ar($C_3$-$C_6$)alkenoyl [e.g. carboxyacetylaminocinnamoyl, carboxypropionylaminocinnamoyl], $C_1$-$C_6$-alkoxycarbonyl($C_1$-$C_6$)alkanoylamino-ar($C_3$-$C_6$)alkenoyl [e.g. ethoxycarbonylacetylaminocinnamoyl, ethoxycarbonylpropionylaminocinnamoyl], $C_1$-$C_6$-alkoxycarbonyl($C_3$-$C_6$)alkenoylamino-ar($C_3$-$C_6$)alkenoyl [e.g. ethoxycarbonylacryloylaminocinnamoyl], halo($C_1$-$C_6$)alkoxycarbonylamino-ar($C_3$-$C_6$)alkenoyl [e.g. chloroethoxycarbonylaminocinnamoyl], heterocyclic($C_1$-$C_6$)alkanoylamino-ar($C_3$-$C_6$)alkenoyl [e.g. pyridylacetylaminocinnamoyl], aroylamino-ar($C_3$-$C_6$)alkenoyl [e.g. benzoylaminocinnamoyl], heterocycliccarbonylamino-ar($C_3$-$C_6$)alkenoyl [e.g. nicotinoylaminocinnamoyl, isonicotinoylaminocinnamoyl, morpholinocarbonylaminocinnamoyl], $C_1$-$C_6$ alkylsulfonylamino-ar($C_3$-$C_6$)alkenoyl [e.g. mesylaminocinnamoyl, ethylsulfonylaminocinnamoyl], N-($C_1$-$C_6$ alkanoyl)-N-($C_1$-$C_6$ alkyl)amino-ar($C_3$-$C_6$)alkenoyl [e.g.

N-acetyl-N-methylaminocinnamoyl, N-acetyl-N-ethylaminocinnamoyl, N-propionyl-N-methylaminocinnamoyl], N-[$C_1$-$C_6$ alkoxy($C_1$-$C_6$)alkanoyl]-N-($C_1$-$C_6$ alkyl)amino-ar($C_3$-$C_6$)alkenoyl [e.g. N-methoxyacetyl-N-methylaminocinnamoyl, N-methoxypropionyl-N-methylaminocinnamoyl], N-($C_1$-$C_6$ alkanoyl)-N-[heterocyclic($C_1$-$C_6$)alkyl]amino-ar($C_3$-$C_6$)alkenoyl [e.g. N-acetyl-N-pyridylmethylaminocinnamoyl], ureido-ar($C_3$-$C_6$)alkenoyl [e.g. ureidocinnamoyl], $C_1$-$C_6$ alkylureido-ar($C_3$-$C_6$)alkenoyl [e.g. methylureidocinnamoyl, ethylureidocinnamoyl, dimethylureidocinnamoyl], heterocyclicureido-ar($C_3$-$C_6$)alkenoyl [e.g. pyridylureidocinnamoyl, pyrimidinylureidocinnamoyl, thienylureidocinnamoyl], $C_1$-$C_6$-alkanoyl-ar($C_3$-$C_6$)alkenoyl [e.g. formylcinnamoyl, acetylcinnamoyl, propionylcinnamoyl], carboxy-ar($C_3$-$C_6$)alkenoyl [e.g. carboxycinnamoyl], $C_1$-$C_6$ alkoxycarbonyl-ar($C_3$-$C_6$)alkenoyl [e.g. methoxycarbonylcinnamoyl, ethoxycarbonylcinnamoyl], carbamoyl-ar($C_3$-$C_6$)alkenoyl [e.g. carbamoylcinnamoyl], $C_1$-$C_6$ alkylcarbamoyl-ar($C_3$-$C_6$)alkenoyl [e.g. methylcarbamoylcinnamoyl, ethylcarbamoylcinnamoyl, dimethylcarbamoylcinnamoyl, propylcarbamoylcinnamoyl, isopropylcarbamoylcinnamoyl, diethylcarbamoylcinnamoyl, N-methyl-N-ethylcarbamoylcinnamoyl], hydroxy($C_1$-$C_6$)alkylcarbamoyl-ar($C_3$-$C_6$)alkenoyl [e.g. hydroxyethylcarbamoylcinnamoyl, bis(hydroxyethyl)carbamoylcinnamoyl], N-[hydroxy($C_1$-$C_6$)alkyl]-N-($C_1$-$C_6$ alkyl)carbamoyl-ar($C_3$-$C_6$)alkenoyl [e.g. N-hydroxyethyl-N-methylcarbamoylcinnamoyl], $C_1$-$C_6$ alkoxy($C_1$-$C_6$)alkylcarbamoyl-ar($C_3$-$C_6$)alkenoyl [e.g. methoxymethylcarbamoylcinnamoyl, methoxyethylcarbamoylcinnamoyl, bis(methoxyethyl)carbamoylcinnamoyl, ethoxyethylcarbamoylcinnamoyl, methoxypropylcarbamoylcinnamoyl, bis(ethoxyethyl)carbamoylcinnamoyl], N-[$C_1$-$C_6$ alkoxy($C_1$-$C_6$)alkyl]-N-($C_1$-$C_6$ alkyl)carbamoyl-ar($C_3$-$C_6$)alkenoyl [e.g. N-methoxyethyl-N-methylcarbamoylcinnamoyl], heterocycliccarbamoyl-ar($C_3$-$C_6$)alkenoyl [e.g. morpholinylcarbamoylcinnamoyl, thienylcarbamoylcinnamoyl, pyridylcarbamoylcinnamoyl, pyrimidinylcarbamoylcinnamoyl], heterocycliccarbonyl-ar($C_3$-$C_6$)alkenoyl [e.g. morpholinocarbonylcinnamoyl, pyrrolidinylcarbonylcinnamoyl, piperidinocarbonylcinnamoyl] ar($C_3$-$C_6$)alkynoyl [e.g. phenylpropioloyl], heterocyclic($C_3$-$C_6$)alkenoyl [e.g. morpholinylacryloyl, pyridylacryloyl, thienylacryloyl], amino-heterocyclic($C_3$-$C_6$)alkenoyl [e.g. aminopyridylacryloyl], $C_1$-$C_6$-alkylamino-heterocyclic($C_3$-$C_6$)alkenoyl [e.g. methylaminopyridylacryloyl, dimethylaminopyridylacryloyl], $C_1$-$C_6$ alkanoylamino-heterocyclic($C_3$-$C_6$)alkenoyl [e.g. acetylaminopyridylacryloyl, propionylaminopyridylacryloyl $C_1$-$C_6$ alkylureido-heterocyclic($C_3$-$C_6$)alkenoyl [e.g. methylureidopyridylacryloyl], carboxy-heterocyclic($C_3$-$C_6$)alkenoyl [e.g. carboxypyridylacryloyl], $C_1$-$C_6$-alkoxycarbonyl-heterocyclic($C_3$-$C_6$)alkenoyl [e.g. ethoxycarbonylpyridylacryloyl], $C_1$-$C_6$-alkylcarbamoyl-heterocyclic($C_3$-$C_6$)alkenoyl [e.g. methylcarbamoylpyridylacryloyl, ethylcarbamoylpyridylacryloyl, dimethylcarbamoylpyridylacryloyl, diethylcarbamoylpyridylacryloyl, isopropylcarbamoylpyridylacryloyl], $C_1$-$C_6$-alkoxy($C_1$-$C_6$)alkylcarbamoyl-heterocyclic($C_3$-$C_6$)alkenoyl [e.g. methoxymethylcarbamoylpyridylacryloyl, methoxyethylcarbamoylpyridylacryloyl, methoxypropylcarbamoylpyridylacryloyl, ethoxyethylcarbamoylpyridylacryloyl, bis(methoxyethyl)carbamoylpyridylacryloyl], hydroxy($C_1$-$C_6$)alkylcarbamoyl-heterocyclic($C_3$-$C_6$)alkenoyl [e.g. hydroxymethylcarbamoylpyridylacryloyl, hydroxyethylcarbamoylpyridylacryloyl, bis(hydroxyethyl)carbamoylpyridylacryloyl, etc.], etc., etc., heterocycliccarbonyl [e.g. furoyl, thenoyl, nicotinoyl, isonicotinoyl, morpholinocarbonyl, piperidinocarbonyl 1,2,3,6-tetrahydropyridylcarbonyl, pyrrolidinylcarbonyl, indolylcarbonyl, and heterocyclic carbonyl substituted with a substituent selected from 4-methyl-1-piperazinylcarbonyl, 4-ethyl-1-piperazinylcarbonyl, dimethylaminopiperidinocarbonyl, 4-methylcarbamoyl-1-piperazinylcarbonyl; aryloxycarbonyl which may be substituted with nitro [e.g. phenyloxycarbonyl, nitrophenyloxycarbonyl], ar($C_1$-$C_6$)alkoxycarbonyl which may be substituted with nitro [e.g. benzyloxycarbonyl, nitrobenzyloxycarbonyl], carbamoyl, thiocarbamoyl, $C_1$-$C_6$ alkylcarbamoyl [e.g. methylcarbamoyl, ethylcarbamoyl, propylcarbamoyl, isopropylcarbamoyl, butylcarbamoyl, isobutylcarbamoyl, tert-butylcarbamoyl, pentylcarbamoyl, dimethylcarbamoyl, diethylcarbamoyl, N-ethyl-N-methylcarbamoyl], carboxy($C_1$-$C_6$)alkylcarbamoyl [e.g. carboxymethylcarbamoyl, carboxyethylcarbamoyl] $C_1$-$C_{6-}$ alkoxycarbonyl($C_1$-$C_6$)alkylcarbamoyl [e.g. methoxycarbonylmethylcarbamoyl, ethoxycarbonylmethylcarbamoyl, ethoxycarbonylethylcarbamoyl], $C_2$-$C_{6-}$ alkenylcarbamoyl [e.g. vinylcarbamoyl, allylcarbamoyl], cyclo($C_3$-$C_6$)alkylcarbamoyl [e.g. cyclopropylcarbamoyl, cyclobutylcarbamoyl, cyclopentylcarbamoyl, cyclohexylcarbamoyl], halo($C_1$-$C_6$)alkanoylcarbamoyl [e.g. trichloroacetylcarbamoyl], arylcarbamoyl [e.g. phenylcarbamoyl, tolylcarbamoyl, xylylcarbamoyl, naphthylcarbamoyl, ethylphenylcarbamoyl], arylthiocarbamoyl [e.g. phenylthiocarbamoyl], $C_1$-$C_6$ alkoxy-arylcarbamoyl [e.g. methoxyphenylcarbamoyl], halo-arylcarbamoyl [e.g. fluorophenylcarbamoyl, chlorophenylcarbamoyl], halo($C_1$-$C_6$)alkylarylcarbamoyl [e.g. trifluoromethylphenylcarbamoyl], nitro-arylcarbamoyl [e.g. nitrophenylcarbamoyl], cyano-arylcarbamoyl [e.g. cyanophenylcarbamoyl], hydroxy($C_1$-$C_6$)alkyl-arylcarbamoyl [e.g. hydroxymethylphenylcarbamoyl, hydroxyethylphenylcarbamoyl], amino-arylcarbamoyl [e.g. aminophenylcarbamoyl], $C_1$-$C_6$ alkylamino-arylcarbamoyl [e.g. methylaminophenylcarbamoyl, ethylaminophenylcarbamoyl, dimethylaminophenylcarbamoyl], $C_1$-$C_6$ alkanoylamino-arylcarbamoyl [e.g. acetylaminophenylcarbamoyl, propionylaminophenylcarbamoyl], N-($C_1$-$C_6$ alkanoyl)-N-($C_1$-$C_6$ alkyl)amino-arylcarbamoyl [e.g. N-acetyl-N-methylaminophenylcarbamoyl, N-propionyl-N-methylaminophenylcarbamoyl], $C_1$-$C_6$ alkoxy($C_1$-$C_6$)alkanoylamino-arylcarbamoyl [e.g. methoxyacetylaminophenylcarbamoyl, methoxypropionylaminophenylcarbamoyl], $C_1$-$C_6$-alkoxycarbonyl($C_1$-$C_6$)alkanoylamino-arylcarbamoyl [e.g. ethoxycarbonylacetylaminophenylcarbamoyl, methoxycarbonylpropionylaminophenylcarbamoyl], carboxyamino-arylcarbamoyl [e.g. carboxyaminophenylcarbamoyl], $C_1$-$C_6$ alkoxycarbonylamino-arylcarbamoyl [e.g. ethoxycarbonylaminophenylcarbamoyl], ureido-arylcarbamoyl [e.g. ureidophenylcarbamoyl], $C_1$-$C_6$ alkylureido-arylcarbamoyl [e.g. methylureidophenylcarbamoyl, ethylureidophenylcarbamoyl], hydroxyimi-

no($C_1$-$C_6$)alkyl-arylcarbamoyl [e.g. hydroxyiminoethylphenylcarbamoyl], $C_1$-$C_6$ alkoxyimino($C_1$-$C_6$)alkyl-arylcarbamoyl [e.g. methoxyiminoethylphenylcarbamoyl], $C_1$-$C_6$ alkylhydrazono($C_1$-$C_6$)alkyl-arylcarbamoyl [e.g. methylhydrazonoethylphenylcarbamoyl, dimethylhydrazonoethylphenylcarbamoyl], optionally substituted heterocyclic-arylcarbamoyl [e.g. oxopyrrolidinylphenylcarbamoyl, oxopiperidinophenylcarbamoyl, dioxopyrrolidinylphenylcarbamoyl, oxooxazolidinylphenylcarbamoyl, pyrrolylphenylcarbamoyl], carboxy-arylcarbamoyl [e.g. carboxyphenylcarbamoyl], $C_1$-$C_6$ alkoxycarbonyl-arylcarbamoyl [e.g. ethoxycarbonylphenylcarbamoyl], heterocycliccarbonyl-arylcarbamoyl [e.g. morpholinocarbonylphenylcarbamoyl, pyrrolidinylcarbonylphenylcarbamoyl, piperidinocarbonylphenylcarbamoyl, 1,2,3,6-tetrahydropyridylcarbonylphenylcarbamoyl, piperazinylcarbonylphenylcarbamoyl, thiomorpholinocarbonylphenylcarbamoyl], heterocycliccarbonyl-arylcarbamoyl substituted with $C_1$-$C_6$ alkyl [e.g. methylpiperazinylcarbonylphenylcarbamoyl, ethylpiperazinylcarbonylphenylcarbamoyl], heterocycliccarbonyl-arylcarbamoyl substituted with aryl [e.g. phenylpiperazinylcarbonylphenylcarbamoyl], heterocycliccarbonyl-arylcarbamoyl substituted with a heterocyclic group [e.g. pyridylpiperazinylcarbonylphenylcarbamoyl], heterocycliccarbonyl-arylcarbamoyl substituted with $C_1$-$C_6$ alkanoyl [e.g. acetylpiperazinylcarbonylphenylcarbamoyl], heterocycliccarbonyl-arylcarbamoyl substituted with $C_1$-$C_6$ alkoxycarbonyl [e.g. ethoxycarbonylpiperazinylcarbonylphenylcarbamoyl], heterocycliccarbonyl-arylcarbamoyl substituted with $C_1$-$C_6$ alkylamino [e.g. methylaminopiperazinylcarbonylphenylcarbamoyl, dimethylaminopiperidinocarbonylphenylcarbamoyl], heterocycliccarbonyl-arylcarbamoyl substituted with $C_1$-$C_6$ alkylcarbamoyl [e.g. methylcarbamoylpiperazinylcarbonylphenylcarbamoyl], carbamoyl-arylcarbamoyl [e.g. carbamoylphenylcarbamoyl], $C_1$-$C_6$ alkylcarbamoyl-arylcarbamoyl [e.g. methylcarbamoylphenylcarbamoyl, ethylcarbamoylphenylcarbamoyl, dimethylcarbamoylphenylcarbamoyl, diethylcarbamoylphenylcarbamoyl, N-ethyl-N-methylcarbamoylphenylcarbamoyl, N-isopropyl-N-methylcarbamoylphenylcarbamoyl], hydroxy($C_1$-$C_6$)alkylcarbamoyl-arylcarbamoyl [e.g. hydroxymethylcarbamoylphenylcarbamoyl, hydroxyethylcarbamoylphenylcarbamoyl, bis(hydroxyethyl)carbamoylphenylcarbamoyl], N-[hydroxy($C_1$-$C_6$)alkyl]-N-($C_1$-$C_6$-alkyl)carbamoyl-arylcarbamoyl [e.g. N-(hydroxyethyl)-N-methylcarbamoylphenylcarbamoyl], $C_1$-$C_6$ alkoxy($C_1$-$C_6$)alkylcarbamoyl-arylcarbamoyl [e.g. methoxymethylcarbamoylphenylcarbamoyl, methoxyethylcarbamoylphenylcarbamoy1, bis(methoxyethyl)-carbamoylphenylcarbamoyl, bis(ethoxyethyl)carbamoylphenylcarbamoyl], N-[$C_1$-$C_6$ alkoxy($C_1$-$C_6$)alkyl]-N-($C_1$-$C_6$ alkyl)carbamoyl-arylcarbamoyl [e.g. N-(methoxyethyl)-N-methylcarbamoylphenylcarbamoyl, N-(methoxypropyl)-N-methylcarbamoylphenylcarbamoyl], $C_1$-$C_6$ alkylamino($C_1$-$C_6$)alkylcarbamoyl-arylcarbamoyl [e.g. methylaminoethylcarbamoylphenylcarbamoyl, dimethylaminoethylcarbamoylphenylcarbamoyl], N-[$C_1$-$C_6$ alkylamino($C_1$-$C_6$)alkyl]-N-($C_1$-$C_6$ alkyl)carbamoyl-arylcarbamoyl [e.g. N-(dimethylaminoethyl)-N-methylcarbamoylphenylcarbamoyl, N-(dimethylaminopropyl)-N-methylcarbamoylphenylcarbamoyl], heterocycliccarbamoyl-arylcarbamoyl [e.g. morpholinylcarbamoylphenylcarbamoyl, thienylcarbamoylphenylcarbamoyl, pyridylcarbamoylphenylcarbamoyl, pyrimidinylcarbamoylphenylcarbamoyl], N-(heterocyclic)-N-($C_1$-$C_6$ alkyl)carbamoyl-arylcarbamoyl [e.g. N-pyridyl-N-methylcarbamoylphenylcarbamoyl], heterocyclic($C_1$-$C_6$)alkylcarbamoyl-arylcarbamoyl [e.g. pyridylmethylcarbamoylphenylcarbamoyl, pyridylethylcarbamoylphenylcarbamoyl, thienylmethylcarbamoylphenylcarbamoyl], N-[heterocyclic($C_1$-$C_6$)alkyl]-N-($C_1$-$C_6$ alkyl)carbamoyl-arylcarbamoyl [e.g. N-pyridylmethyl-N-methylcarbamoylphenylcarbamoyl], N-[heterocyclic($C_1$-$C_6$)alkyl]-N-[$C_1$-$C_6$ alkoxy($C_1$-$C_6$)alkyl]-carbamoyl-arylcarbamoyl [e.g. N-pyridylmethyl-N-methoxyethylcarbamoylphenylcarbamoyl] arylcarbamoyl-arylcarbamoyl [e.g. phenylcarbamoylphenylcarbamoyl], $C_1$-$C_6$-alkylamino-arylcarbamoyl-arylcarbamoyl [e.g. dimethylaminophenylcarbamoylphenylcarbamoyl], $C_1$-$C_6$ alkanoyl-arylcarbamoyl [e.g. acetylphenylcarbamoyl, propionylphenylcarbamoyl], ar($C_1$-$C_6$)alkylcarbamoyl [e.g. benzylcarbamoyl, phenethylcarbamoyl], heterocycliccarbamoyl [e.g. furylcarbamoyl, thienylcarbamoyl, pyridylcarbamoyl, quinolylcarbamoyl, isoquinolylcarbamoyl, pyrimidinylcarbamoyl, pyrazolylcarbamoyl], heterocyclic($C_1$-$C_6$)alkylcarbamoyl [e.g. pyridylmethylcarbamoyl, pyridylethylcarbamoyl, thienylmethylcarbamoyl], arylaminocarbamoyl [e.g. phenylaminocarbamoyl], aroylcarbamoyl [e.g. benzoylcarbamoyl, $C_1$-$C_6$-alkylsulfonyl [e.g. mesyl, ethylsulfonyl, propylsulfonyl, isopropylsulfonyl, tert-butylsulfonyl, pentylsulfonyl], arylsulfonyl [e.g. tosyl, phenylsulfonyl], ar($C_1$-$C_6$)alkylsulfonyl [e.g. benzylsulfonyl, phenethylsulfonyl], ar($C_2$-$C_6$)alkenylsulfonyl [e.g. styrylsulfonyl, cinnamylsulfonyl], phthaloyl, amino acid residue wherein the amino acid is selected from glycine, sarcosine, alanine, β-alanine, valine, norvaline, leucine, isoleucine, norleucine, serine, threonine, cysteine, methionine, phenylalanine, phenylglycine, tryptophan, tyrosine, proline, hydroxyproline, glutamic acid, aspartic acid, glutamine, asparagine, lysine, arginine, histidine, and ornithine, which may be substituted with substituent(s) selected from $C_1$-$C_6$ alkyl [e.g. ethylglycyl, isopropylglycyl, dimethylglycyl, diethylglycyl, ethylsarcosyl, isopropylsarcosyl, methylalanyl, methyl-β-alanyl, dimethyl-β-alanyl, etc.], aryl [e.g. N-phenylglycyl, N-tolylglycyl, N-phenylalanyl, N-phenylsarcosyl], ar($C_1$-$C_6$)alkyl [e.g. benzylglycyl, tritylglycyl, phenethylglycyl, benzylsarcosyl, benzylalanyl], heterocyclic group [e.g. morpholinoglycyl, piperidinoglycyl, pyridylglycyl], heterocyclic($C_1$-$C_6$)alkyl [e.g. pyridylmethylglycyl, imidazolylmethylglycyl, furylmethylglycyl, thienylmethylglycyl], cycloalkyl [e.g. cyclopropylglycyl, cyclobutylglycyl, cyclopentylglycyl, cyclohexylglycyl, cycloheptylglycyl, cyclooctylglycyl, adamantylglycyl, cyclohexylsarcosyl, cycloheptylsarcosyl, cyclohexylalanyl], amidino, [e.g. amidinoglycyl], substituted amidino selected from methylamidino and N-ethyl-N'-cyanoamidino; alkanoyl selected from formyl, acetyl, propionyl, butyryl, isobutyryl, valeryl, isovaleryl, pivaloyl, hexanoyl and heptanoyl [e.g. formylglycyl, acetylglycyl, acetylsarcosyl, acetylalanyl, acetyl-β-alanyl, propionylglycyl, butyrylglycyl, isobutyrylglycyl, valerylglycyl, isovalerylglycyl, pivaloylglycyl, hexanoylglycyl, heptanoylglycyl],

halo($C_1$-$C_6$)alkanoyl [e.g. trifluoroacetylglycyl, trifluoroacetylsarcosyl, trifluoroacetylalanyl, bromoacetylglycyl, heptafluorobutyrylglycyl], hydroxy($C_1$-$C_6$)alkanoyl [e.g. glycoloylglycyl, glycoloylsarcosyl, lactoylglycyl, lactoylalanyl], $C_1$-$C_6$ alkylsulfonyloxy($C_1$-$C_6$)alkanoyl [e.g. mesyloxyacetylglycyl, ethylsulfonyloxyacetylglycyl, mesyloxyacetylsarcosyl], $C_1$-$C_6$ alkoxy($C_1$-$C_6$)alkanoyl [e.g. methoxyacetylglycyl, ethoxyacetylglycyl, methoxyacetylsarcosyl, methoxypropionylalanyl], aryloxy($C_1$-$C_6$)alkanoyl [e.g. phenyloxyacetylglycyl, phenyloxypropionylglycyl, phenyloxyacetylsarcosyl], $C_1$-$C_6$ alkylthio($C_1$-$C_6$)alkanoyl [e.g. methylthioacetylglycyl, methylthiopropionylglycyl], $C_1$-$C_6$ alkylcarbamoyl-($C_1$-$C_6$)alkanoyl [e.g. methylcarbamoylpropionylglycyl, methylcarbamoylpropionylalanyl], $C_1$-$C_6$ alkanoyloxy($C_1$-$C_6$)alkanoyl [e.g. acetyloxyacetylglycyl, acetyloxyacetylsarcosyl, propionyloxyacetylglycyl, acetyloxypropionylalanyl], carboxy($C_1$-$C_6$)alkanoyl [e.g. carboxyacetylglycyl, carboxypropionylglycyl, carboxypropionylsarcosyl, carboxyacetylalanyl], $C_1$-$C_6$-alkoxycarbonyl($C_1$-$C_6$)alkanoyl [e.g. methoxycarbonylacetylglycyl, ethoxycarbonylpropionylglycyl, methoxycarbonylacetylsarcosyl], ar($C_1$-$C_6$)alkanoyl [e.g. phenylacetylglycyl, phenylacetylsarcosyl, phenylpropionylalanyl, phenylpropionylglycyl, naphthylacetylglycyl, phenylbutyrylglycyl], optionally substituted heterocyclic($C_1$-$C_6$)alkanoyl [e.g. morpholinoacetylglycyl, thiomorpholinoacetylglycyl, its oxide or dioxide, pyridylacetylglycyl, morpholinopropionylalanyl, imidazolylacetylglycyl, piperidinoacetylglycyl, pyrrolidinylacetylglycyl, hexamethyleneiminoacetylglycyl, methylpiperazinylacetylglycyl, pyridylpiperazinylacetylglycyl], $C_3$-$C_6$ alkenoyl [e.g. acryloylglycyl, crotonoylglycyl, 3-pentenoylglycyl, 3-butenoylglycyl, 4-pentenoylglycyl, 3-butenoylsarcosyl], ar($C_3$-$C_6$)alkenoyl [e.g. cinnamoylglycyl, allocinnamoylglycyl, α-methylcinnamoylglycyl, 4-methylcinnamoylglycyl, cinnamoylsarcosyl], $C_1$-$C_6$ alkoxy-ar($C_3$-$C_6$)alkenoyl [e.g. methoxycinnamoylglycyl, ethoxycinnamoylglycyl, dimethoxycinnamoylglycyl], $C_1$-$C_6$ alkylenedioxy-ar($C_3$-$C_6$)alkenoyl [e.g. methylenedioxycinnamoylglycyl, ethylenedioxycinnamoylglycyl], nitro-ar($C_3$-$C_6$)alkenoyl [e.g. nitrocinnamoylglycyl], halo-ar($C_3$-$C_6$)alkenoyl [e.g. chlorocinnamoylglycyl, fluorocinnamoylglycyl], hydroxy-ar($C_3$-$C_6$)alkenoyl [e.g. hydroxycinnamoylglycyl], hydroxy($C_1$-$C_6$)alkoxy-ar($C_3$-$C_6$)alkenoyl [e.g. hydroxymethoxycinnamoylglycyl, hydroxyethoxycinnamoylglycyl], amino($C_1$-$C_6$)alkoxy-ar($C_3$-$C_6$)alkenoyl, $C_1$-$C_6$-alkylamino($C_1$-$C_6$)alkoxy-ar($C_3$-$C_6$)alkenoyl [e.g. methylaminomethoxycinnamoylglycyl, dimethylaminoethoxycinnamoylglycyl], heterocyclic($C_1$-$C_6$)alkoxy-ar($C_3$-$C_6$)alkenoyl [e.g. pyridylmethoxycinnamoylglycyl], optionally substituted heterocyclic-ar($C_3$-$C_6$)alkenoyl [e.g. morpholinocinnamoylglycyl, methylpiperazinylcinnamoylglycyl, pyrrolidinylcinnamoylglycyl, oxopyrrolidinylcinnamoylglycyl, oxopiperidinocinnamoylglycyl, dioxopyrrolidinylcinnamoylglycyl, oxooxazolidinylcinnamoylglycyl, pyrrolylcinnamoylglycyl], amino-ar($C_3$-$C_6$)alkenoyl [e.g. aminocinnamoylglycyl], $C_1$-$C_6$-alkylamino-ar($C_3$-$C_6$)alkenoyl [e.g. methylaminocinnamoylglycyl, dimethylaminocinnamoylglycyl], $C_1$-$C_6$-alkanoylamino-ar($C_3$-$C_6$)alkenoyl [e.g. acetylaminocinnamoylglycyl, propionylaminocinnamoylglycyl], hydroxy($C_1$-$C_6$)alkanoylamino-ar($C_3$-$C_6$)alkenoyl [e.g. hydroxyacetylaminocinnamoylglycyl, hydroxypropionylaminocinnamoylglycyl], $C_1$-$C_6$-alkoxy($C_1$-$C_6$)alkanoylamino-ar($C_3$-$C_6$)alkenoyl [e.g. methoxyacetylaminocinnamoylglycyl, methoxypropionylaminocinnamoylglycyl], halo($C_1$-$C_6$)alkanoylamino-ar($C_3$-$C_6$)alkenoyl [e.g. chloroacetylaminocinnamoylglycyl, bromobutyrylaminocinnamoylglycyl], amino($C_1$-$C_6$)alkanoylamino-ar($C_3$-$C_6$)alkenoyl [e.g. aminoacetylaminocinnamoylglycyl, aminopropionylaminocinnamoylglycyl], $C_1$-$C_{6-}$ alkylamino($C_1$-$C_6$)alkanoylamino-ar($C_3$-$C_6$)alkenoyl [e.g. methylaminoacetylaminocinnamoylglycyl, dimethylaminoacetylaminocinnamoylglycyl], $C_1$-$C_{6-}$ alkanoylamino($C_1$-$C_6$)alkanoylamino-ar($C_3$-$C_6$)alkenoyl [e.g. acetylaminoacetylaminocinnamoylglycyl, acetylaminopropionylaminocinnamoylglycyl] carboxy($C_1$-$C_6$)alkanoylamino-ar($C_3$-$C_6$)alkenoyl [e.g. carboxyacetylaminocinnamoylglycyl, carboxypropionylaminocinnamoylglycyl], $C_1$-$C_{6-}$ alkoxycarbonyl($C_1$-$C_6$)alkanoylamino-ar($C_3$-$C_6$)alkenoyl [e.g. ethoxycarbonylacetylaminocinnamoylglycyl, ethoxycarbonylpropionylaminocinnamoylglycyl], $C_1$-$C_{6-}$ alkoxycarbonyl($C_3$-$C_6$)alkenoylamino-ar($C_3$-$C_6$)alkenoyl [e.g. ethoxycarbonylacryloylaminocinnamoylglycyl], halo($C_1$-$C_6$)alkoxycarbonylamino-ar($C_3$-$C_6$)alkenoyl [e.g. chloroethoxycarbonylaminocinnamoylglycyl], heterocyclic($C_1$-$C_6$)alkanoylamino-ar($C_3$-$C_6$)alkenoyl [e.g. pyridylacetylaminocinnamoylglycyl], aroylamino-ar($C_3$-$C_6$)alkenoyl [e.g. benzoylaminocinnamoylglycyl], heterocycliccarbonylamino-ar($C_3$-$C_6$)alkenoyl [e.g. nicotinoylaminocinnamoylglycyl, isonicotinoylaminocinnamoylglycyl, morpholinocarbonylaminocinnamoylglycyl], $C_1$-$C_{6-}$ alkylsulfonylamino-ar($C_3$-$C_6$)alkenoyl [e.g. mesylaminocinnamoylglycyl, ethylsulfonylaminocinnamoylglycyl] N-($C_1$-$C_6$-alkanoyl)-N-($C_1$-$C_6$ alkyl)amino-ar($C_3$-$C_6$)alkenoyl [e.g. N-acetyl-N-methylaminocinnamoylglycyl, N-acetyl-N-ethylaminocinnamoylglycyl, N-propionyl-N-methylaminocinnamoylglycyl], N-[$C_1$-$C_{6-}$ alkoxy($C_1$-$C_6$)alkanoyl]-N-($C_1$-$C_6$ alkyl)amino-ar($C_3$-$C_6$)alkenoyl [e.g. N-methoxyacetyl-N-methylaminocinnamoylglycyl, N-methoxypropionyl-N-methylaminocinnamoylglycyl], N-($C_1$-$C_6$ alkanoyl)-N-[heterocyclic($C_1$-$C_6$)alkyl]amino-ar($C_3$-$C_6$)alkenoyl [e.g. N-acetyl-N-pyridylmethylaminocinnamoylglycyl], ureido-ar($C_3$-$C_6$)alkenoyl [e.g. ureidocinnamoylglycyl], $C_1$-$C_{6-}$ alkylureido-ar($C_3$-$C_6$)alkenoyl [e.g. methylureidocinnamoylglycyl, ethylureidocinnamoylglycyl, dimethylureidocinnamoylglycyl], heterocyclicureido-ar($C_3$-$C_6$)alkenoyl [e.g. pyridylureidocinnamoylglycyl, pyrimidinylureidocinnamoylglycyl, thienylureidocinnamoylglycyl], $C_1$-$C_6$ alkanoyl-ar($C_3$-$C_6$)alkenoyl [e.g. formylcinnamoylglycyl, acetylcinnamoylglycyl, propionylcinnamoylglycyl], carboxy-ar($C_3$-$C_6$)-alkenoyl [e.g. carboxycinnamoylglycyl], $C_1$-$C_{6-}$ alkoxycarbonyl-ar($C_3$-$C_6$)alkenoyl [e.g. methoxycarbonylcinnamoylglycyl, ethoxycarbonylcinnamoylglycyl], carbamoyl-ar($C_3$-$C_6$)-alkenoyl [e.g. carbamoylcinnamoylglycyl], $C_1$-$C_{6-}$ alkylcarbamoyl-ar($C_3$-$C_6$)alkenoyl [e.g. methylcarbamoylcinnamoylglycyl, ethylcarbamoylcinnamoylglycyl, dimethylcarbamoylcinnamoylglycyl, propylcarbamoylcinnamoylglycyl, isopropylcarbamoylcinnamoylglycyl, diethylcarbamoylcinnamoylglycyl, N-methyl-N-ethylcarbamoylcin-

namoylglycyl], hydroxy($C_1$-$C_6$)alkylcarbamoyl-ar($C_3$-$C_6$)alkenoyl [e.g. hydroxyethylcarbamoylcinnamoylglycyl, bis(hydroxyethyl)carbamoylcinnamoylglycyl], N-[hydroxy($C_1$-$C_6$)alkyl]-N-($C_1$-$C_6$-alkyl)carbamoyl-ar($C_3$-$C_6$)alkenoyl [e.g. N-hydroxyethyl-N-methylcarbamoylcinnamoylglycyl], $C_1$-$C_{6-}$ alkoxy($C_1$-$C_6$)alkylcarbamoyl-ar($C_3$-$C_6$)alkenoyl [e.g. methoxymethylcarbamoylcinnamoylglycyl, methoxyethylcarbamoylcinnamoylglycyl, bis(methoxyethyl)carbamoylcinnamoylglycyl, ethoxyethylcarbamoylcinnamoylglycyl, methoxypropylcarbamoylcinnamoylglycyl, bis(ethoxyethyl)carbamoylcinnamoylglycyl], N-[$C_1$-$C_{6-}$ alkoxy($C_1$-$C_6$)alkyl]-N-($C_1$-$C_6$ alkyl)carbamoyl-ar($C_3$-$C_6$)alkenoyl [e.g. N-methoxyethyl-N-methylcarbamoylcinnamoylglycyl], heterocycliccarbamoyl-ar($C_3$-$C_6$)alkenoyl [e.g. morpholinylcarbamoylcinnamoylglycyl, thienylcarbamoylcinnamoylglycyl, pyridylcarbamoylcinnamoylglycyl, pyrimidinylcarbamoylcinnamoylglycyl], heterocycliccarbonyl-ar($C_3$-$C_6$)alkenoyl [e.g. morpholinocarbonylcinnamoylglycyl, pyrrolidinylcarbonylcinnamoylglycyl, piperidinocarbonylcinnamoylglycyl], ar($C_3$-$C_6$)alkynoyl [e.g. phenylpropioloylglycyl], heterocyclic($C_3$-$C_6$)alkenoyl [e.g. morpholinylacryloylglycyl, pyridylacryloylglycyl, thienylacryloylglycyl], amino-heterocyclic($C_3$-$C_6$)alkenoyl [e.g. aminopyridylacryloylglycyl], $C_1$-$C_{6-}$ alkylamino-heterocyclic($C_3$-$C_6$)alkenoyl [e.g. methylaminopyridylacryloylglycyl, dimethylaminopyridylacryloylglycyl], $C_1$-$C_{6-}$ alkanoylamino-heterocyclic($C_3$-$C_6$)alkenoyl [e.g. acetylaminopyridylacryloylglycyl, propionylaminopyridylacryloylglycyl], $C_1$-$C_{6-}$ alkylureido-heterocyclic($C_3$-$C_6$)alkenoyl [e.g. methylureidopyridylacryloylglycyl], carboxy-heterocyclic($C_3$-$C_6$)alkenoyl [e.g. carboxypyridylacryloylglycyl], $C_1$-$C_{6-}$ alkoxycarbonyl-heterocyclic($C_3$-$C_6$)alkenoyl [e.g. ethoxycarbonylpyridylacryloylglycyl], $C_1$-$C_{6-}$ alkylcarbamoyl-heterocyclic($C_3$-$C_6$)alkenoyl [e.g. methylcarbamoylpyridylacryloylglycyl, ethylcarbamoylpyridylacryloylglycyl, dimethylcarbamoylpyridylacryloylglycyl, diethylcarbamoylpyridylacryloylglycyl, isopropylcarbamoylpyridylacryloylglycyl], $C_1$-$C_{6-}$ alkoxy($C_1$-$C_6$)alkylcarbamoyl-heterocyclic($C_1$-$C_6$)alkenoyl [e.g. methoxymethylcarbamoylpyridylacryloylglycyl, methoxyethylcarbamoylpyridylacryloylglycyl, methoxypropylcarbamoylpyridylacryloylglycyl, ethoxyethylcarbamoylpyridylacryloylglycyl, bis(methoxyethyl)carbamoylpyridylacryloylglycyl], hydroxy($C_1$-$C_6$)alkylcarbamoyl-heterocyclic($C_3$-$C_6$)alkenoyl [e.g. hydroxymethylcarbamoylpyridylacryloylglycyl, hydroxyethylcarbamoylpyridylacryloylglycyl, bis(hydroxyethyl)carbamoylpyridylacryloylglycyl], heterocyclicthio($C_1$-$C_6$)alkanoyl [e.g. pyridylthioacetylglycyl, pyrimidinylthioacetylglycyl, imidazolylthiopropionylglycyl], optionally substituted heterocycliccarbonyl [e.g. morpholinocarbonylglycyl, indolylcarbonylglycyl, 4-methyl-1-piperazinylcarbonylglycyl], cyclo($C_1$-$C_6$)alkylcarbonyl [e.g. cyclopropylcarbonylglycyl, cyclopentylcarbonylglycyl, cyclohexylcarbonylglycyl, cyclohexylcarbonylsarcosyl], $C_1$-$C_6$ alkoxycarbonyl [e.g. methoxycarbonylglycyl, tert-butoxycarbonylglycyl, tert-butoxycarbonylsarcosyl, tert-butoxycarbonylalanyl], aryloxycarbonyl [e.g. phenoxycarbonylglycyl], aroyl($C_1$-$C_6$)alkanoyl [e.g. phenyloxalylglycyl, benzoylpropionylglycyl], aroyl [e.g. benzoylglycyl, naphthoylglycyl, benzoylsarcosyl, benzoylalanyl], nitro-aryloxycarbonyl [e.g. nitrophenyloxycarbonylglycyl], carbamoyl [e.g. carbamoylglycyl, carbamoylalanyl, carbamoylsarcosyl, carbamoyl-β-alanyl], $C_1$-$C_6$-alkylcarbamoyl [e.g. methylcarbamoylglycyl, ethylcarbamoylglycyl, propylcarbamoylglycyl, isopropylcarbamoylglycyl, methylcarbamoylsarcosyl, ethylcarbamoylalanyl, isopropylcarbamoyl-β-alanyl, pentylcarbamoylglycyl], $C_1$-$C_6$ alkoxycarbonyl($C_1$-$C_6$)alkylcarbamoyl [e.g. methoxycarbonylmethylcarbamoylglycyl, ethoxycarbonylmethylcarbamoylglycyl], $C_2$-$C_6$ alkenylcarbamoyl [e.g. vinylcarbamoylglycyl, allylcarbamoylglycyl, allylcarbamoylsarcosyl], cyclo($C_3$-$C_6$)alkylcarbamoyl [e.g. cyclopropylcarbamoylglycyl, cyclohexylcarbamoylglycyl, cyclohexylcarbamoylsarcosyl], arylcarbamoyl [e.g. phenylcarbamoylglycyl, naphthylcarbamoylglycyl, tolylcarbamoylglycyl, ethylphenylcarbamoylglycyl, phenylcarbamoylalanyl, phenylcarbamoylsarcosyl], $C_1$-$C_6$-alkoxy-arylcarbamoyl [e.g. methoxyphenylcarbamoylglycyl, ethoxyphenylcarbamoylglycyl, methoxyphenylcarbamoylalanyl], halo($C_1$-$C_6$)alkyl-arylcarbamoyl [e.g. trifluoromethylphenylcarbamoylglycyl, trifluoromethylphenylcarbamoylalanyl, trifluoromethylphenylcarbamoylsarcosyl], halo-arylcarbamoyl [e.g. chlorophenylcarbamoylglycyl, fluorophenylcarbamoylglycyl, fluorophenylcarbamoylalanyl], hydroxy($C_1$-$C_6$)alkyl-arylcarbamoyl [e.g. hydroxymethylphenylcarbamoylglycyl, hydroxyethylphenylcarbamoylglycyl, hydroxyethylphenylcarbamoylalanyl], nitro-arylcarbamoyl [e.g. nitrophenylcarbamoylglycyl], cyano-arylcarbamoyl [e.g. cyanophenylcarbamoylglycyl], amino-arylcarbamoyl [e.g. aminophenylcarbamoylglycyl], $C_1$-$C_6$ alkylamino-arylcarbamoyl [e.g. methylaminophenylcarbamoylglycyl, ethylaminophenylcarbamoylglycyl, dimethylaminophenylcarbamoylglycyl], $C_1$-$C_6$ alkanoylamino-arylcarbamoyl [e.g. acetylaminophenylcarbamoylglycyl, propionylaminophenylcarbamoylglycyl], N-($C_1$-$C_6$ alkanoyl)-N-($C_1$-$C_6$ alkyl)amino-arylcarbamoyl [e.g. N-acetyl-N-methylaminophenylcarbamoylglycyl, N-propionyl-N-methylaminophenylcarbamoylglycyl], $C_1$-$C_6$-alkoxy($C_1$-$C_6$)alkanoylamino-arylcarbamoyl [e.g. methoxyacetylaminophenylcarbamoylglycyl, methoxypropionylaminophenylcarbamoylglycyl], $C_1$-$C_6$ alkoxycarbonyl($C_1$-$C_6$)alkanoylamino-arylcarbamoyl [e.g. ethoxycarbonylacetylaminophenylcarbamoylglycyl, methoxycarbonylpropionylaminophenylcarbamoylglycyl], carboxyamino-arylcarbamoyl [e.g. carboxyaminophenylcarbamoylglycyl], $C_1$-$C_6$ alkoxycarbonylamino-arylcarbamoyl [e.g. ethoxycarbonylaminophenylcarbamoylglycyl], ureido-arylcarbamoyl [e.g. ureidophenylcarbamoylglycyl], $C_1$-$C_6$ alkylureido-arylcarbamoyl [e.g. methylureidophenylcarbamoylglycyl, ethylureidophenylcarbamoylglycyl], hydroxyimino($C_1$-$C_6$)alkyl-arylcarbamoyl [e.g. hydroxyiminoethylphenylcarbamoylglycyl], $C_1$-$C_6$ alkoxyimino($C_1$-$C_6$)alkyl-arylcarbamoyl [e.g. methoxyiminoethylphenylcarbamoylglycyl], amino acid residue substituted with $C_1$-$C_6$ alkylhydrazono($C_1$-$C_6$)alkyl-arylcarbamoyl [e.g. methylhydrazonoethylphenylcarbamoylglycyl, dimethylhydrazonoethylphenylcarbamoylglycyl], optionally substituted heterocyclic-arylcarbamoyl [e.g. oxopyrrolidinylphenylcarbamoylglycyl, oxopiperidinophenylcarbamoylglycyl, dioxopyrrolidinylphenylcarbamoylglycyl, oxooxazolidinylphenylcarbamoylglycyl, pyr-

rolylphenylcarbamoylglycyl], $C_1$-$C_6$ alkanoyl-arylcarbamoyl [e.g. acetylphenylcarbamoylglycyl, propionylphenylcarbamoylglycyl], heterocycliccarbonyl-arylcarbamoyl [e.g. morpholinocarbonylphenylcarbamoylglycyl, piperidinocarbonylphenylcarbamoylglycyl, piperazinylcarbonylphenylcarbamoylglycyl, thiomorpholinocarbonylphenylcarbamoylalanyl, pyrrolidinylcarbonylphenylcarbamoylglycyl, 1,2,3,6-tetrahydropyridylcarbonylphenylcarbamoylglycyl], carboxy-arylcarbamoyl [e.g. carboxyphenylcarbamoylglycyl], $C_1$-$C_6$ alkoxycarbonyl-arylcarbamoyl [e.g. methoxycarbonylphenylcarbamoylglycyl, ethoxycarbonylphenylcarbamoylglycyl], $C_1$-$C_6$ alkylcarbamoyl-arylcarbamoyl [e.g. methylcarbamoylphenylcarbamoylglycyl, dimethylcarbamoylphenylcarbamoylglycyl, diethylcarbamoylphenylcarbamoylglycyl, N-ethyl-N-methylcarbamoylphenylcarbamoylglycyl, N-isopropyl-N-methylcarbamoylphenylcarbamoylglycyl] heterocycliccarbonyl-arylcarbamoyl having $C_1$-$C_6$ alkyl [e.g. methylpiperazinylcarbonylphenylcarbamoylglycyl, ethylpiperazinylcarbonylphenylcarbamoylglycyl], heterocycliccarbonyl-arylcarbamoyl having aryl [e.g. phenylpiperazinylcarbonylphenylcarbamoylglycyl], heterocycliccarbonyl-arylcarbamoyl having a heterocyclic group [e.g. pyridylpiperazinylcarbonylphenylcarbamoylglycyl], heterocycliccarbonyl-arylcarbamoyl having $C_1$-$C_6$ alkanoyl [e.g. acetylpiperazinylcarbonylphenylcarbamoylglycyl], heterocycliccarbonyl-arylcarbamoyl having $C_1$-$C_6$ alkoxycarbonyl [e.g. ethoxycarbonylpiperazinylcarbonylphenylcarbamoylglycyl], heterocycliccarbonyl-arylcarbamoyl having $C_1$-$C_6$ alkylamino [e.g. methylaminopiperazinylcarbonylphenylcarbamoylglycyl, dimethylaminopiperidinocarbonylphenylcarbamoylglycyl], heterocycliccarbonyl-arylcarbamoyl having $C_1$-$C_6$ alkylcarbamoyl [e.g. methylcarbamoylpiperazinylcarbonylphenylcarbamoylglycyl], hydroxy($C_1$-$C_6$)alkylcarbamoyl-arylcarbamoyl [e.g. hydroxymethylcarbamoylphenylcarbamoylglycyl, hydroxyethylcarbamoylphenylcarbamoylglycyl, bis(hydroxyethyl)carbamoylphenylcarbamoylglycyl], N-[hydroxy($C_1$-$C_6$)alkyl]-N-($C_1$-$C_6$ alkyl)carbamoyl-arylcarbamoyl [e.g. N-(hydroxyethyl)-N-methylcarbamoylphenylcarbamoylglycyl], amino acid residue substituted with $C_1$-$C_6$ alkoxy($C_1$-$C_6$)alkylcarbamoyl-arylcarbamoyl [e.g. methoxymethylcarbamoylphenylcarbamoylglycyl, methoxyethylcarbamoylphenylcarbamoylglycyl, bis(methoxyethyl)carbamoylphenylcarbamoylglycyl, bis(ethoxyethyl)carbamoylphenylcarbamoylglycyl], N-[$C_1$-$C_6$ alkoxy($C_1$-$C_6$)alkyl]-N-($C_1$-$C_6$ alkyl)carbamoyl-arylcarbamoyl [e.g. N-(methoxyethyl)-N-methylcarbamoylphenylcarbamoylglycyl, N-(methoxypropyl)-N-methylcarbamoylphenylcarbamoylglycyl], $C_1$-$C_6$-alkylamino($C_1$-$C_6$)alkylcarbamoyl-arylcarbamoyl [e.g. methylaminoethylcarbamoylphenylcarbamoylglycyl, dimethylaminoethylcarbamoylphenylcarbamoylglycyl], N-[$C_1$-$C_6$ alkylamino($C_1$-$C_6$)alkyl]-N-($C_1$-$C_6$ alkyl)carbamoyl-arylcarbamoyl [e.g. N-(dimethylaminoethyl)-N-methylcarbamoylphenylcarbamoylglycyl, N-(dimethylaminopropyl)-N-methylcarbamoylphenylcarbamoylglycyl], heterocycliccarbamoyl-arylcarbamoyl [e.g. morpholinylcarbamoylphenylcarbamoylglycyl, thienylcarbamoylphenylcarbamoylglycyl, pyridylcarbamoylphenylcarbamoylglycyl, pyrimidinylcarbamoylphenylcarbamoylglycyl], N-(heterocyclic)-N-($C_1$-$C_6$ alkyl)carbamoyl-arylcarbamoyl [e.g. N-pyridyl-N-methylcarbamoylphenylcarbamoylglycyl], heterocyclic($C_1$-$C_6$)alkylcarbamoyl-arylcarbamoyl [e.g. pyridylmethylcarbamoylphenylcarbamoylglycyl, pyridylethylcarbamoylphenylcarbamoylglycyl, thienylmethylcarbamoylphenylcarbamoylglycyl], N-[heterocyclic($C_1$-$C_6$)alkyl]-N-($C_1$-$C_6$ alkyl)carbamoyl-arylcarbamoyl [e.g. N-pyridylmethyl-N-methylcarbamoylphenylcarbamoylglycyl], N-[heterocyclic($C_1$-$C_6$)alkyl]-N-[$C_1$-$C_6$ alkoxy($C_1$-$C_6$)alkyl]carbamoyl-arylcarbamoyl [e.g. N-pyridylmethyl-N-methoxyethylcarbamoylphenylcarbamoylglycyl], arylcarbamoyl-arylcarbamoyl [e.g. phenylcarbamoylphenylcarbamoylglycyl], $C_1$-$C_6$ alkylaminoarylcarbamoyl-arylcarbamoyl [e.g. dimethylaminophenylcarbamoylphenylcarbamoylglycyl], arylthiocarbamoyl [e.g. phenylthiocarbamoylglycyl, naphthylthiocarbamoylglycyl, phenylthiocarbamoylalanyl, phenylthiocarbamoylsarcosyl], ar($C_1$-$C_6$)alkylcarbamoyl [e.g. benzylcarbamoylglycyl, benzylcarbamoylsarcosyl, benzylcarbamoylalanyl], aroylcarbamoyl [e.g. benzoylcarbamoylglycyl], heterocycliccarbamoyl [e.g. pyridylcarbamoylglycyl, pyridylcarbamoylalanyl, pyridylcarbamoylsarcosyl, thienylcarbamoylglycyl, pyrazolylcarbamoylglycyl, pyrimidinylcarbamoylglycyl, quinolylcarbamoylglycyl, isoquinolylcarbamoylglycyl], heterocyclic($C_1$-$C_6$)alkylcarbamoyl [e.g. pyridylmethylcarbamoylglycyl, pyridylethylcarbamoylglycyl, thienylmethylcarbamoylglycyl], arylaminocarbamoyl [e.g. phenylaminocarbamoylglycyl], ar-($C_2$-$C_6$)alkenylsulfonyl [e.g. styrylsulfonylglycyl, cinnamylsulfonylglycyl], ($C_1$-$C_6$)alkylsulfonyl [e.g. mesylglycyl, ethylsulfonylglycyl, mesylsarcosyl, mesylalanyl], phthaloyl (e.g. phthaloylglycyl, phthaloylalanyl, phthaloyl-β-alanyl], unsubstituted amino acid residue selected from glycine, sarcosine, alanine, β-alanine, valine, norvaline, leucine, isoleucine, norleucine, serine, threonine, cysteine, methionine, phenylalanine, phenylglycine, tryptophan, tyrosine, proline, hydroxyproline, glutamic acid, aspartic acid, glutamine, asparagine, lysine, arginine, histidine, and ornithine [e.g. glycylglycyl, alanylglycyl, sarcosylglycyl, prolylglycyl, glycylsarcosyl, prolylsarcosyl], amino acid residue substituted with ($C_1$-$C_6$)alkyl wherein the amino acid is as defined for the unsubstituted amino acid residue defined before (e.g. dimethylglycylglycyl, diethylglycylglycyl, dimethylglycylsarcosyl, ethylsarcosylglycyl, isopropylsarcosylglycyl, ethylglycylglycyl, propylglycylglycyl, isopropylglycylglycyl, ethylglycylalanyl, dimethylglycylalanyl, dimethylalanyglycyl, dimethyl-β-alanylglycyl) amino acid residue substituted with a heterocyclic group wherein the amino acid is as defined for the unsubstituted amino acid residue defined before (e.g. morpholinoglycylglycyl, piperidinoglycylglycyl, pyridylglycylglycyl, piperidinosarcosylglycyl), amino acid residue substituted with heterocyclic($C_1$-$C_6$)alkyl wherein the amino acid is as defined for the unsubstituted amino acid defined before (e.g. pyridylmethylglycylglycyl, imidazolylmethylglycylglycyl, furylmethylglycylglycyl, thienylmethylsarcosylglycyl), amino acid residue substituted with cycloalkyl selected from cyclopropyl, cyclobutyl, cyclopentyl, cyclohexyl, cycloheptyl, cyclooctyl and adamantyl (e.g. cyclopropylglycylglycyl, cyclobutylglycylglycyl, cyclopentylglycylglycyl, cyclohexylglycylglycyl, cycloheptylglycylglycyl, cyclooctylglycylglycyl, adamantylglycylglycyl, cyclohexylsarcosylglycyl, cycloheptylsarcosylglycyl, cyclohexylglycylsarcosyl, cyclohexylglycylalanyl), amino acid residue substituted with aryl wherein the amino acid is as defined for the unsubstituted amino acid residue defined before (e.g. phenylglycylglycyl, phenylsarcosylglycyl), amino acid residue substituted with ($C_1$-$C_6$)alkanoyl wherein the amino acid is as defined for the unsubstituted amino acid residue defined before (e.g. acetylglycylglycyl, acetylprolylglycyl, propionylglycylglycyl, acetylalanylglycyl), amino acid residue substituted with ($C_1$-$C_6$)alkoxycarbonyl wherein the amino acid is as defined for the unsubstituted amino acid residue defined before (e.g. tert-butoxycarbonylglycylglycyl, tert-butoxycarbonylprolylglycyl), amino acid residue substituted with phthaloyl wherein the amino acid is as defined for the unsubstituted amino acid residue defined before (e.g. phthaloylglycylglycyl), amino acid residue substituted with ar($C_1$-$C_6$)alkyl wherein the amino acid is as defined for the unsubstituted amino acid residue defined before (e.g. benzylglycylglycyl),
in the above definition of "acyl", heterocyclic group or moiety, substituents thereof and substituents of aryl are each as defined below.

Groups of the formulas of the compounds [If] and [Ig] :

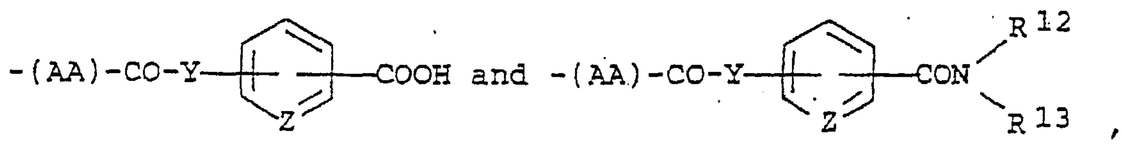

wherein $R^{12}$, $R^{13}$, (AA), Y and Z are each as defined above, are also included within the above defined "acyl".

Suitable "acyl having amino" as defined above may be unsubstituted amino acid residue, amino acid residue having unsubstituted amino acid residue, and preferred examples thereof can be referred to those exemplified above wherein the amino acid is as defined for the unsubstituted amino acid residue defined before.

Suitable "protected or unprotected hydroxy($C_1$-$C_6$)alkyl" may be hydroxymethyl, hydroxyethyl, hydroxypropyl, benzyloxymethyl, tert-butyldiphenylsilyloxyethyl.

Suitable "($C_1$-$C_6$)alkoxy($C_1$-$C_6$)alkyl" may be methoxymethyl, methoxyethyl, methoxypropyl, ethoxymethyl, ethoxyethyl.

Suitable "($C_1$-$C_6$)alkylamino($C_1$-$C_6$)alkyl" may be methylaminomethyl, methylaminoethyl, methylaminopropyl, dimethylaminomethyl, dimethylaminoethyl, dimethylaminopropyl, diethylaminoethyl.

Suitable "acyl having ($C_1$-$C_6$) alkylamino" as defined above may be amino acid residue substituted with ($C_1$-$C_6$)alkyl, amino acid residue having amino acid residue substituted with ($C_1$-$C_6$) alkyl wherein the amino acid is as defined above for the unsubstituted amino acid residue defined before and preferred examples thereof can be referred to those exemplified above.

Suitable "acyl having ar($C_1$-$C_6$)alkylamino" as defined above may be amino acid residue substituted with ar-($C_1$-$C_6$)alkyl, amino acid residue having amino acid residue substituted with ar($C_1$-$C_6$)alkyl, wherein the amino acid is as defined above for the unsubstituted amino acid residue defined before and preferred examples thereof can be referred to those exemplified above.

Suitable "heterocyclic group" and heterocyclic moiety in the term "heterocyclic($C_1$-$C_6$)alkyl" is selected from :

- unsaturated 3 to 8-membered, preferably 5 or 6-membered heteromonocyclic group containing 1 to 4 nitrogen atom(s), for example, pyrrolyl, pyrrolinyl, imidazolyl, pyrazolyl, pyridyl, and its N-oxide, pyrimidinyl, pyrazinyl, pyridazinyl, triazolyl, tetrazolyl, dihydrotriazinyl;
- saturated 3 to 8-membered, preferably 4 or 6-membered heteromonocyclic group containing 1 to 4 nitrogen atom(s), for example, azetidinyl, pyrrolidinyl, imidazolidinyl, piperidyl, pyrazolidinyl, piperazinyl;
- unsaturated condensed 7 to 12-membered heterocyclic group containing 1 to 5 nitrogen atom(s), for example, indolyl, isoindolyl, indolizinyl, benzimidazolyl, quinolyl, isoquinolyl, tetrahydroquinolyl, indazolyl, benzotriazolyl, imidazopyridyl;
- unsaturated 3 to 8-membered, preferably 5 or 6-membered heteromonocyclic group containing an oxygen atom, for example, furyl;
- unsaturated condensed 7 to 12-membered heterocyclic group containing 1 to 2 oxygen atom(s), for example, benzofuryl, piperonyl;
- unsaturated 3 to 8-membered, preferably 5 or 6-membered heteromonocyclic group containing a sulfur atom, for example, thienyl;
- unsaturated condensed 7 to 12-membered heterocyclic group containing 1 to 2 sulfur atom(s), for example, benzothienyl;
- unsaturated 3 to 8-membered, preferably 5 or 6-membered heteromonocyclic group containing 1 to 2 oxygen atom(s) and 1 to 3 nitrogen atom(s), for example, oxazolyl, isoxazolyl, oxadiazolyl;

- saturated 3 to 8-membered, preferably 5 or 6-membered heteromonocyclic group containing 1 to 2 oxygen atom(s) and 1 to 3 nitrogen atom(s), for example, morpholinyl;
- unsaturated condensed 7 to 12-membered heterocyclic group containing 1 to 2 oxygen atom(s) and 1 to 3 nitrogen atom(s), for example, benzoxazolyl, benzoxadiazolyl;
- unsaturated 3 to 8-membered, preferably 5 or 6-membered heteromonocyclic group containing 1 to 2 sulfur atom(s) and 1 to 3 nitrogen atom(s), for example, thiazolyl, isothiazolyl, thiazolinyl, thiadiazolyl;
- saturated 3 to 8-membered, preferably 5 or 6-membered heteromonocyclic group containing 1 to 2 sulfur atom(s) and 1 to 3 nitrogen atom(s), for example, thiazolidinyl;
- unsaturated condensed 7 to 12-membered heterocyclic group containing 1 to 2 sulfur atom(s) and 1 to 3 nitrogen atom(s), for example, benzothiazolyl, benzothiadiazolyl, benzothiazinyl, benzothiazolinyl.

Substituents of "aryl" as defined above and "heterocyclic group" as defined above substituent(s)" are selected from the above-mentioned halogen; the above-mentioned halo($C_1$-$C_6$)alkyl; the above-mentioned $C_1$-$C_6$ alkyl; the above-mentioned aryl optionally substituted with substituent(s) selected from halogen and cyano [e.g. chlorophenyl, cyanophenyl]; ar($C_1$-$C_6$)alkyl substituted with hydroxy [e.g. hydroxybenzyl]; $C_1$-$C_6$ alkoxy [e.g. methoxy, ethoxy, propoxy, isopropoxy, butoxy, isobutoxy, tert-butoxy, pentyloxy, hexyloxy]; oxo; nitro; amino; optionally substituted with the above-mentioned $C_1$-$C_6$ alkyl, the above-mentioned acyl, ar($C_1$-$C_6$)alkyl [e.g. benzyl, phenethyl, trityl], heterocyclic($C_1$-$C_6$)alkyl wherein the heterocyclic moiety is as defined above [e.g. pyridylmethyl], carboxy($C_1$-$C_6$)alkyl [e.g. carboxymethyl, carboxyethyl], $C_1$-$C_6$ alkylaminomethylene [e.g. dimethylaminomethylene, diethylaminomethylene], N-methylpyrrolidinylidene; the above-mentioned heterocyclic group optionally substituted with oxo [e.g. pyrrolidonyl].

Suitable "heterocyclic group" as defined above formed by $R^{12}$, $R^{13}$ and the attached nitrogen atom may be morpholino, thiomorpholino, pyrrolidin-1-yl, piperidino, 1,2,3,6-tetrahydropyridin-1-yl, piperazin-1-yl. And said heterocyclic group as defined above may be substituted with substituent(s) selected from the above-mentioned $C_1$-$C_6$ alkyl, the above-mentioned heterocyclic group, the above-mentioned acyl, $C_1$-$C_6$ alkylamino, the above-mentioned aryl.

Preferred examples of "heterocyclic($C_1$-$C_6$)alkyl" as defined above may be morpholinomethyl, morpholinoethyl, pyridylmethyl, pyridylethyl, thienylmethyl, piperidinomethyl, imidazolylmethyl.

Particularly, the preferred embodiments of $R^1$, $R^2$, $R^3$, $R^4$, Q and A are as follows :

$R^1$ is halogen such as fluorine, chlorine, bromine and iodine;

$R^2$ is $C_1$-$C_6$ alkyl such as methyl, ethyl, propyl, isopropyl, butyl, isobutyl, tert-butyl, pentyl and hexyl;

$R^4$ is phenyl substituted with substituent(s) such as

- halogen such as fluorine, chlorine, bromine and iodine,
- $C_1$-$C_6$ alkyl such as methyl, ethyl, propyl and isopropyl,
- halo($C_1$-$C_6$)alkyl such as trifluoromethyl,
- phenyl,
- cyanophenyl,
- hydroxvbenzyl,
- $C_1$-$C_6$ alkoxy such as methoxy, ethoxy, propoxy and isopropoxy,
- nitro,
- a group of the formula :

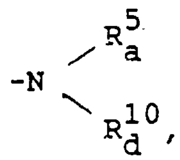

in which

$R^5_a$ is hydrogen; $C_1$-$C_6$ alkyl such as methyl, ethyl, propyl and butyl; carboxy($C_1$-$C_6$)alkyl such as carboxymethyl and carboxyethyl; and the above defined acyl such as $C_1$-$C_6$ alkanoyl [e.g. formyl, acetyl, propionyl], carboxy and $C_1$-$C_6$ alkoxycarbonyl (e.g. methoxycarbonyl, ethoxycarbonyl, tert-butoxycarbonyl),

$R^{10}_d$ is hydrogen; $C_1$-$C_6$ alkyl such as methyl, ethyl, propyl, isopropyl and butyl; phenyl($C_1$-$C_6$)alkyl such as benzyl; pyridyl($C_1$-$C_6$)alkyl $C_1$-$C_6$ [e.g. pyridylmethyl, pyridylethyl]; and the above defined acyl such as $C_1$-$C_6$ alkanoyl [e.g. formyl, acetyl, propionyl, butyryl, isobutyryl], halo($C_1$-$C_6$)alkanoyl [e.g. trifluoroacetyl], carboxy, $C_1$-$C_6$ alkoxycarbonyl (e.g. methoxycarbonyl, ethoxycarbonyl,

tert-butoxycarbonyl), hydroxy($C_1$-$C_6$)alkanoyl [e.g. glycoloyl, lactoyl, 3-hydroxypropionyl], $C_1$-$C_6$ alkanoyloxy($C_1$-$C_6$)alkanoyl [e.g. acetyloxyacetyl, acetyloxypropionyl], $C_1$-$C_6$ alkoxy($C_1$-$C_6$)alkanoyl [e.g. methoxyacetyl, methoxypropionyl], benzoyl, toluoyl, bensoyl substituted with $C_1$-$C_6$ alkoxy [e.g. methoxybenzoyl], benzoyl substituted with $C_1$-$C_6$ alkoxycarbonylbenzoyl (e.g. methoxycarbonylbenzoyl, tert-butoxycarbonylbenzoyl), benzoyl substituted with halogen [e.g. chlorobenzoyl, fluorobenzoyl], phenoxycarbonyl optionally substituted with nitro, $C_1$-$C_6$ alkylsulfonyl [e.g. mesyl, ethylsulfonyl], carbamoyl, $C_1$-$C_6$ alkylcarbamoyl [e.g. methylcarbamoyl, ethylcarbamoyl, isopropylcarbamoyl], halo($C_1$-$C_6$)alkanoylcarbamoyl [e.g. trichloroacetylcarbamoyl], phenylcarbamoyl, unsubstituted amino acid residue as defined above [e.g. glycyl, sarcosyl, alanyl, β-alanyl] and substituted amino acid residue as defined above [e.g. amino acid residue substituted with $C_1$-$C_6$ alkyl (e.g. ethylglycyl, isopropylglycyl, dimethylglycyl, diethylglycyl, ethylsarcosyl, isopropylsarcosyl, methylalanyl, methyl-β-alanyl), amino acid residue as defined above substituted with optionally substituted amidino (e.g. amidinoglycyl, N-ethyl-N'-cyanoamidinoglycyl), amino acid residue as defined above substituted with alkanoyl selected from formyl, acetyl, propionyl, butyryl, isobutyryl, valeryl, novaleryl, pivaloyl, hexanoyl, heptanoyl, and 3,3-dimethylbutyryl (e.g. formylglycyl, acetylglycyl, acetylsarcosyl, acetylalanyl, acetyl-β-alanyl, propionylglycyl, butyrylglycyl, isobutyrylglycyl, valerylglycyl, isovalerylglycyl, pivaloylglycyl, hexanoylglycyl, heptanoylglycyl), amino acid residue as defined above substituted with halo($C_1$-$C_6$)alkanoyl (e.g. trifluoroacetylglycyl, trifluoroacetylsarcosyl, trifluoroacetylalanyl, bromoacetylglycyl, heptafluorobutyrylglycyl), amino acid residue as defined above substituted with hydroxy($C_1$-$C_6$)alkanoyl (e.g. glycoloylglycyl, glycoloylsarcosyl, lactoylglycyl, lactoylalanyl), amino acid residue as defined above substituted with $C_1$-$C_6$ alkylsulfonyloxy($C_1$-$C_6$)alkanoyl (e.g. mesyloxyacetylglycyl, ethylsulfonyloxyacetylglycyl, mesyloxyacetylsarcosyl), amino acid residue as defined above substituted with $C_1$-$C_6$ alkoxy($C_1$-$C_6$)alkanoyl (e.g. methoxyacetylglycyl, ethoxyacetylglycyl, methoxyacetylsarcosyl, methoxypropionylalanyl), amino acid residue as defined above substituted with aryloxy($C_1$-$C_6$)alkanoyl (e.g. phenyloxyacetylglycyl, phenyloxypropionylglycyl, phenyloxyacetylsarcosyl), amino acid residue as defined above substituted with $C_1$-$C_6$-alkylthio($C_1$-$C_6$)alkanoyl (e.g. methylthioacetylglycyl, methylthiopropionylglycyl), amino acid residue as defined above substituted with $C_1$-$C_6$ alkylcarbamoyl($C_1$-$C_6$)alkanoyl (e.g. methylcarbamoylpropionylglycyl, methylcarbamoylpropionylalanyl), amino acid residue as defined above substituted with $C_1$-$C_6$ alkanoyloxy($C_1$-$C_6$)alkanoyl (e.g. acetyloxyacetylglycyl, acetyloxyacetylsarcosyl, propionyloxyacetylglycyl, acetyloxypropionylalanyl), amino acid residue as defined above substituted with carboxy($C_1$-$C_6$)alkanoyl (e.g. carboxyacetylglycyl, carboxypropionylglycyl, carboxypropionylsarcosyl, carboxyacetylalanyl), amino acid residue as defined above substituted with $C_1$-$C_6$ alkoxycarbonyl($C_1$-$C_6$)alkanoyl (e.g. methoxycarbonylacetylglycyl, ethoxycarbonylpropionylglycyl, methoxycarbonylacetylsarcosyl), amino acid residue as defined above substituted with ar($C_1$-$C_6$)alkanoyl (e.g. phenylacetylglycyl, phenylpropionylglycyl, phenylbutyrylglycyl, phenylacetylsarcosyl, phenylpropionylalanyl, naphthylacetylglycyl), amino acid residue as defined above substituted with optionally substituted heterocyclic($C_1$-$C_6$)alkanoyl (e.g. morpholinoacetylglycyl, pyridylacetylglycyl, morpholinopropionylalanyl, imidazolylacetylglycyl, piperidinoacetylglycyl, pyrrolidinylacetylglycyl, hexamethyleneiminoacetylglycyl, methylpiperazinylacetylglycyl, pyridylpiperazinylacetylglycyl, thiomorpholinoacetylglycyl, its oxide or dioxide), amino acid residue as defined above substituted with $C_3$-$C_6$ alkenoyl (e.g. acryloylglycyl, crotonoylglycyl, 3-pentenoylglycyl, 3-butenoylglycyl, 4-pentenoylglycyl, 3-butenoylsarcosyl), amino acid residue as defined above substituted with ar($C_3$-$C_6$)alkenoyl (e.g. cinnamoylglycyl, α-methylcinnamoylglycyl, 4-methylcinnamoylglycyl), amino acid residue as defined above substituted with $C_1$-$C_6$-alkoxy-ar($C_3$-$C_6$)alkenoyl (e.g. methoxycinnamoylglycyl, ethoxycinnamoylglycyl, dimethoxycinnamoylglycyl), amino acid residue as defined above substituted with $C_1$-$C_6$ alkylenedioxy-ar($C_3$-$C_6$)alkenoyl (e.g. methylenedioxycinnamoylglycyl, ethylenedioxycinnamoylglycyl), amino acid residue as defined above substituted with nitro-ar($C_3$-$C_6$)alkenoyl (e.g. nitrocinnamoylglycyl), amino acid residue as defined above substituted with halo-ar($C_3$-$C_6$)alkenoyl (e.g. chlorocinnamoylglycyl, fluorocinnamoylglycyl), amino acid residue substituted with hydroxy-ar($C_3$-$C_6$)alkenoyl (e.g. hydroxycinnamoylglycyl), amino acid residue as defined above substituted with hydroxy($C_1$-$C_6$)alkoxy-ar($C_3$-$C_6$)alkenoyl (e.g. hydroxymethoxycinnamoylglycyl, hydroxyethoxycinnamoylglycyl), amino acid residue as defined above substituted with amino($C_1$-$C_6$)alkoxy-ar($C_3$-$C_6$)alkenoyl (e.g. aminoethoxycinnamoylglycyl), amino acid residue as defined above substituted with $C_1$-$C_6$ alkylamino($C_1$-$C_6$)alkoxy-ar($C_3$-$C_6$)alkenoyl (e.g. methylaminomethoxycinnamoylglycyl, dimethylaminoethoxycinnamoylglycyl), amino acid residue as

defined above substituted with heterocyclic($C_1$-$C_6$)alkoxy-ar($C_3$-$C_6$)alkenoyl (e.g. pyridylmethoxycinnamoylglycyl, etc.), amino acid residue as defined above substituted with optionally substituted heterocyclic-ar($C_3$-$C_6$)alkenoyl (e.g. morpholinocinnamoylglycyl, methylpiperazinylcinnamoylglycyl, pyrrolidinylcinnamoylglycyl, oxopyrrolidinylcinnamoylglycyl, oxopiperidinocinnamoylglycyl, dioxopyrrolidinylcinnamoylglycyl, oxooxazolidinylcinnamoylglycyl, pyrrolylcinnamoylglycyl), amino acid residue as defined above substituted with amino-ar($C_3$-$C_6$)alkenoyl (e.g. aminocinnamoylglycyl), amino acid residue as defined above substituted with $C_1$-$C_6$-alkylamino-ar($C_3$-$C_6$)alkenoyl (e.g. methylaminocinnamoylglycyl, dimethylaminocinnamoylglycyl), amino acid residue as defined above substituted with $C_1$-$C_6$-alkanoylamino-ar($C_3$-$C_6$)alkenoyl (e.g. acetylaminocinnamoylglycyl, propionylaminocinnamoylglycyl), amino acid residue as defined above substituted with hydroxy($C_1$-$C_6$)alkanoylamino-ar($C_3$-$C_6$)alkenoyl (e.g. hydroxyacetylaminocinnamoylglycyl, hydroxypropionylaminocinnamoylglycyl), amino acid residue as defined above substituted with $C_1$-$C_6$-alkoxy($C_1$-$C_6$)alkanoylamino-ar($C_3$-$C_6$)alkenoyl (e.g. methoxyacetylaminocinnamoylglycyl, methoxypropionylaminocinnamoylglycyl), amino acid residue as defined above substituted with halo($C_1$-$C_6$)alkanoylamino-ar($C_3$-$C_6$)alkenoyl (e.g. chloroacetylaminocinnamoylglycyl, bromobutyrylaminocinnamoylglycyl), amino acid residue as defined above substituted with amino($C_1$-$C_6$)alkanoylamino-ar($C_3$-$C_6$)alkenoyl (e.g. aminoacetylaminocinnamoylglycyl, aminopropionylaminocinnamoylglycyl), amino acid residue as defined above substituted with $C_1$-$C_6$ alkylamino($C_1$-$C_6$)alkanoylamino-ar($C_3$-$C_6$)alkenoyl (e.g. methylaminoacetylaminocinnamoylglycyl, dimethylaminoacetylaminocinnamoylglycyl), amino acid residue as defined above substituted with $C_1$-$C_6$ alkanoylamino($C_1$-$C_6$)alkanoylamino-ar($C_3$-$C_6$)alkenoyl (e.g. acetylaminoacetylaminocinnamoylglycyl, acetylaminopropionylaminocinnamoylglycyl), amino acid residue as defined above substituted with carboxy($C_1$-$C_6$)alkanoylamino-ar($C_3$-$C_6$)alkenoyl (e.g. carboxyacetylaminocinnamoylglycyl, carboxypropionylaminocinnamoylglycyl), amino acid residue as defined above substituted with $C_1$-$C_6$ alkoxycarbonyl($C_1$-$C_6$)alkanoylamino-ar($C_3$-$C_6$)alkenoyl (e.g. ethoxycarbonylacetylaminocinnamoylglycyl, ethoxycarbonylpropionylaminocinnamoylglycyl), amino acid residue as defined above substituted with $C_1$-$C_6$ alkoxycarbonyl($C_3$-$C_6$)alkenoylamino-ar($C_3$-$C_6$)alkenoyl (e.g. ethoxycarbonylacryloylaminocinnamoylglycyl), amino acid residue as defined above substituted with halo($C_1$-$C_6$)alkoxycarbonylamino-ar($C_3$-$C_6$)alkenoyl (e.g. chloroethoxycarbonylaminocinnamoylglycyl), amino acid residue as defined above substituted with heterocyclic($C_1$-$C_6$)alkanoylamino-ar($C_3$-$C_6$)alkenoyl (e.g. pyridylacetylaminocinnamoylglycyl), amino acid residue as defined above substituted with aroylamino-ar($C_3$-$C_6$)alkenoyl (e.g. benzoylaminocinnamoylglycyl), amino acid residue as defined above substituted with heterocycliccarbonylamino-ar($C_3$-$C_6$)alkenoyl (e.g. nicotinoylaminocinnamoylglycyl, isonicotinoylaminocinnamoylglycyl, morpholinocarbonylaminocinnamoylglycyl), amino acid residue as defined above substituted with $C_1$-$C_6$ alkylsulfonylamino-ar($C_3$-$C_6$)alkenoyl (e.g. mesylaminocinnamoylglycyl, ethylsulfonylaminocinnamoylglycyl), amino acid residue as defined above substituted with N-($C_1$-$C_6$ alkanoyl)-N-($C_1$-$C_6$ alkyl)amino-ar($C_3$-$C_6$)alkenoyl (e.g. N-acetyl-N-methylaminocinnamoylglyclyl, N-acetyl-N-ethylaminocinnamoylglycyl, N-propionyl-N-methylaminocinnamoylglycyl, etc.), amino acid residue as defined above substituted with N-[$C_1$-$C_6$ alkoxy($C_1$-$C_6$)alkanoyl]-N-($C_1$-$C_6$ alkyl)amino-ar($C_3$-$C_6$)alkenoyl (e.g. N-methoxyacetyl-N-methylaminocinnamoylglycyl, N-methoxypropionyl-N-methylaminocinnamoylglycyl), amino acid residue as defined above substituted with N-($C_1$-$C_6$ alkanoyl)-N-[heterocyclic($C_1$-$C_6$)alkyl]-amino-ar($C_3$-$C_6$)alkenoyl (e.g. N-acetyl-N-pyridylmethylaminocinnamoylglycyl), amino acid residue as defined above substituted with ureido-ar($C_3$-$C_6$)alkenoyl (e.g. ureidocinnamoylglycyl), amino acid residue as defined above substituted with $C_1$-$C_6$ alkylureido-ar($C_3$-$C_6$)alkenoyl (e.g. methylureidocinnamoylglycyl, ethylureidocinnamoylglycyl, dimethylureidocinnamoylglycyl), amino acid residue as defined above substituted with heterocyclicureido-ar($C_3$-$C_6$)alkenoyl (e.g. pyridylureidocinnamoylglycyl, pyrimidinylureidocinnamoylglycyl, thienylureidocinnamoylglycyl), amino acid residue as defined above substituted with $C_1$-$C_6$ alkanoyl-ar($C_3$-$C_6$)alkenoyl (e.g. formylcinnamoylglycyl, acetylcinnamoylglycyl, propionylcinnamoylglycyl), amino acid residue as defined above substituted with carboxy-ar($C_3$-$C_6$)alkenoyl (e.g. carboxycinnamoylglycyl), amino acid residue substituted with $C_1$-$C_6$ alkoxycarbonyl-ar($C_3$-$C_6$)alkenoyl (e.g. methoxycarbonylcinnamoylglycyl, ethoxycarbonylcinnamoylglycyl), amino acid residue as defined above substituted with carbamoyl-ar($C_3$-$C_6$)alkenoyl (e.g. carbamoylcinnamoylglycyl), amino acid residue as defined above substituted with $C_1$-$C_6$ alkylcarbamoyl-ar($C_3$-$C_6$)alkenoyl (e.g. methylcarbamoylcinnamoylglycyl, ethylcarbamoylcinnamoylglycyl, dimethylcarbamoylcinnamoylglycyl, propylcarbamoylcinnamoylglycyl, isopropylcarbamoylcinnamoylglycyl, diethylcarbamoylcinnamoylglycyl, N-methyl-N-ethylcarbamoylcinnamoylglycyl), amino acid residue as defined above substituted with hydroxy($C_1$-$C_6$)alkylcarbamoyl-ar($C_3$-$C_6$)alkenoyl (e.g. hydroxyethylcarbamoylcinnamoylglycyl, bis(hydroxyethyl)carbamoylcinnamoylglycyl), amino acid residue as defined above substituted with N-[hydroxy($C_1$-$C_6$)alkyl]-N-($C_1$-$C_6$)carbamoyl-ar($C_3$-$C_6$)alkenoyl (e.g. N-hydroxyethyl-N-methylcarbamoylcinnamoylglycyl), amino acid residue as defined above substituted with $C_1$-$C_6$ alkoxy($C_1$-$C_6$)alkylcarbamoyl-ar($C_3$-$C_6$)alkenoyl (e.g. methoxymethylcarbamoylcinnamoylglycyl, methoxyethylcarbamoylcinnamoylglycyl, bis(methoxyethyl)carbamoylcinnamoylglycyl, ethoxyethylcarbamoylcinnamoylglycyl, methoxypropylcarbamoylcinnamoylglycyl, bis(ethoxyethyl)carbamoylcinnamoylglycyl), amino acid residue as defined above substituted with N-[$C_1$-$C_6$ alkoxy($C_1$-$C_6$)alkyl]-N-($C_1$-$C_6$ alkyl)carbamoyl-ar($C_3$-$C_6$)alkenoyl (e.g. N-methoxyethyl-N-methylcarbamoylcinnamoylglycyl), amino acid residue as defined above substituted with heterocycliccarbamoyl-ar($C_3$-$C_6$)alkenoyl (e.g. morpholinylcarbamoylcinnamoylglycyl, thienylcarbamoylcinnamoylglycyl, pyridylcarbamoylcinnamoylglycyl, pyrimidinylcarbamoylcinnamoylglycyl), amino acid residue as defined above substituted with heterocycliccarbonyl-ar($C_3$-$C_6$)alkenoyl (e.g. morpholinocarbonylcinnamoylglycyl, pyrrolidinylcarbonylcinnamoylglycyl, piperidinocarbonylcinnamoylglycyl), amino acid residue as defined above substituted with ar($C_3$-$C_6$)alkynoyl (e.g. phenylpropioloylglycyl), amino acid residue as defined above substituted with heterocyclic($C_3$-$C_6$)alkenoyl (e.g. morpholinylacryloylglycyl, pyridylacryloylglycyl, thienylacryloylglycyl), amino acid residue as defined above substituted with amino-heterocyclic($C_3$-$C_6$)alkenoyl (e.g. aminopyridylacryloylglycyl) amino acid residue as defined above substituted with $C_1$-$C_6$ alkylamino-heterocyclic($C_3$-$C_6$)alkenoyl (e.g. methylaminopyridylacryloylglycyl, dimethylaminopyridylacryloylglycyl), amino acid residue as defined above substituted with $C_1$-$C_6$ alkanoylamino-heterocyclic($C_3$-$C_6$)alkenoyl (e.g. acetylaminopyridylacryloylglycyl, propionylaminopyridylacryloylglycyl), amino acid residue as defined above substituted with $C_1$-$C_6$ alkylureido-heterocyclic(lower)alkenoyl (e.g. methylureidopyridylacryloylglycyl), amino acid residue as defined above substituted with carboxy-heterocyclic($C_3$-$C_6$)alkenoyl (e.g. carboxypyridylacryloylglycyl), amino acid residue as defined above substituted with $C_1$-$C_6$ alkoxycarbonyl-heterocyclic($C_3$-$C_6$)alkenoyl (e.g. ethoxycarbonylpyridylacryloylglycyl), amino acid residue as defined above substituted with $C_1$-$C_6$ alkylcarbamoyl-heterocyclic($C_3$-$C_6$)alkenoyl (e.g. methylcarbamoylpyridylacryloylglycyl, ethylcarbamoylpyridylacryloylglycyl, dimethylcarbamoylpyridylacryloylglycyl, diethylcarbamoylpyridylacryloylglycyl, isopropylcarbamoylpyridylacryloylglycyl), amino acid residue as defined above substituted with $C_1$-$C_6$ alkoxy($C_1$-$C_6$)alkylcarbamoylheterocyclic($C_3$-$C_6$)alkenoyl (e.g. methoxymethylcarbamoylpyridylacryloylglycyl, methoxyethylcarbamoylpyridylacryloylglycyl, methoxypropylcarbamoylpyridylacryloylglycyl, ethoxyethylcarbamoylpyridylacryloylglycyl, bis(methoxyethyl)carbamoylpyridylacryloylglycyl), amino acid residue as defined above substituted with hydroxy($C_1$-$C_6$)alkylcarbamoylheterocyclic($C_3$-$C_6$)alkenoyl (e.g. hydroxymethylcarbamoylpyridylacryloylglycyl, hydroxyethylcarbamoylpyridylacryloylglycyl, bis(hydroxyethyl)carbamoylpyridylacryloylglycyl), amino acid residue as defined above substituted with heterocyclicthio($C_1$-$C_6$)alkanoyl (e.g. pyridylthioacetylglycyl, pyrimidinylthioacetylglycyl, imidazolylthiopropionylglycyl), amino acid residue as defined above substituted with optionally substituted heterocycliccarbonyl (e.g. morpholinocarbonylglycyl, indolylcarbonylglycyl, 4-methyl-1-piperazinylcarbonylglycyl), amino acid residue as defined above substituted with cyclo($C_3$-$C_6$)alkylcarbonyl (e.g. cyclopropylcarbonylglycyl, cyclopentylcarbonylglycyl, cyclohexylcarbonylglycyl, cyclohexylcarbonylsarcosyl), amino acid residue as defined above substituted with $C_1$-$C_6$ alkoxycarbonyl (e.g. methoxycarbonylglycyl, tert-butoxycarbonylglycyl, tert-butoxycarbonylsarcosyl, tert-butoxycarbonylalanyl), amino acid residue as defined above substituted with aryloxycarbonyl (e.g. phenoxycarbonylglycyl), amino acid residue as defined above substituted with aroyl($C_3$-$C_6$)alkanoyl (e.g. phenyloxalylglycyl, benzoylpropionylglycyl), amino acid residue as defined above substituted with aroyl (e.g. benzoylglycyl, benzoylsarcosyl, naphthoylglycyl, benzoylalanyl), amino acid residue as defined above substituted with nitro-aryloxycarbonyl (e.g. nitrophenyloxycarbonylglycyl), amino acid residue as defined above substituted with carbamoyl (e.g. carbamoylglycyl, carbamoylalanyl, carbamoylsarcosyl, carbamoyl-β-alanyl), amino acid residue as defined above substituted with $C_1$-$C_6$ alkylcarbamoyl (e.g. methylcarbamoylglycyl, ethylcarbamoylglycyl, propylcarbamoylglycyl, isopropylcarbamoylglycyl, pentylcarbamoylglycyl, methylcarbamoylsarcosyl, ethylcarbamoylalanyl, isopropylcarbamoyl-β-alanyl), amino acid residue as defined above substituted with $C_1$-$C_6$-alkoxycarbonyl($C_1$-$C_6$)alkylcarbamoyl (e.g. methoxycarbonylmethylcarbamoylglycyl, ethoxycarbonylmethylcarbamoylglycyl), amino acid residue as de-

fined above substituted with $C_2$-$C_6$ alkenylcarbamoyl (e.g. vinylcarbamoylglycyl, allylcarbamoylglycyl, allylcarbamoylsarcosyl), amino acid residue as defined above substituted with cyclo($C_3$-$C_6$)alkylcarbamoyl (e.g. cyclopropylcarbamoylglycyl, cyclohexylcarbamoylglycyl, cyclohexylcarbamoylsarcosyl), amino acid residue as defined above substituted with arylcarbamoyl (e.g. phenylcarbamoylglycyl, naphthylcarbamoylglycyl, tolylcarbamoylglycyl, ethylphenylcarbamoylglycyl, phenylcarbamoylalanyl, phenylcarbamoylsarcosyl), amino acid residue as defined above substituted with $C_1$-$C_6$ alkoxy-arylcarbamoyl (e.g. methoxyphenylcarbamoylglycyl, ethoxyphenylcarbamoylglycyl, methoxyphenylcarbamoylalanyl), amino acid residue as defined above substituted with halo($C_1$-$C_6$)alkyl-arylcarbamoyl (e.g. trifluoromethylphenylcarbamoylglycyl, trifluoromethylphenylcarbamoylalanyl, trifluoromethylphenylcarbamoylsarcosyl), amino acid residue as defined above substituted with halo-arylcarbamoyl (e.g. chlorophenylcarbamoylglycyl, fluorophenylcarbamoylglycyl, fluorophenylcarbamoylalanyl), amino acid residue as defined above substituted with $C_1$-$C_6$-alkanoyl-arylcarbamoyl (e.g. acetylphenylcarbamoylglycyl, propionylphenylcarbamoylalanyl), amino acid residue as defined above substituted with hydroxy($C_1$-$C_6$)alkyl-arylcarbamoyl (e.g. hydroxymethylphenylcarbamoylglycyl, hydroxyethylphenylcarbamoylglycyl, hydroxyethylphenylcarbamoylalanyl), amino acid residue as defined above substituted with heterocycliccarbonyl-arylcarbamoyl (e.g. morpholinocarbonylphenylcarbamoylglycyl, piperidinocarbonylphenylcarbamoylglycyl, thiomorpholinocarbonylphenylcarbamoylalanyl, piperazinylcarbonylphenylcarbamoylglycyl, pyrrolidinylcarbonylphenylcarbamoylglycyl, 1,2,3,6-tetrahydropyridylcarbonylphenylcarbamoylglycyl), amino acid residue as defined above substituted with carboxy-arylcarbamoyl (e.g. carboxyphenylcarbamoylglycyl), amino acid residue as defined above substituted with $C_1$-$C_6$-alkoxycarbonyl-arylcarbamoyl (e.g. methoxycarbonylphenylcarbamoylglycyl, ethoxycarbonylphenylcarbamoylglycyl), amino acid residue as defined above substituted with $C_1$-$C_6$ alkylcarbamoyl-arylcarbamoyl (e.g. methylcarbamoylphenylcarbamoylglycyl, dimethylcarbamoylphenylcarbamoylglycyl, diethylcarbamoylphenylcarbamoylglycyl, N-ethyl-N-methylcarbamoylphenylcarbamoylglycyl, N-isopropyl-N-methylcarbamoylphenylcarbamoylglycyl), amino acid residue as defined above substituted with nitro-arylcarbamoyl (e.g. nitrophenylcarbamoylglycyl), amino acid residue as defined above substituted with cyano-arylcarbamoyl (e.g. cyanophenylcarbamoylglycyl), amino acid residue as defined above substituted with amino-arylcarbamoyl (e.g. aminophenylcarbamoylglycyl), amino acid residue as defined above substituted with $C_1$-$C_6$ alkylamino-arylcarbamoyl (e.g. methylaminophenylcarbamoylglycyl, ethylaminophenylcarbamoylglycyl, dimethylaminophenylcarbamoylglycyl), amino acid residue as defined above substituted with $C_1$-$C_6$ alkanoylamino-arylcarbamoyl (e.g. acetylaminophenylcarbamoylglycyl, propionylaminophenylcarbamoylglycyl), amino acid residue as defined above substituted with N-($C_1$-$C_6$ alkanoyl)-N-($C_1$-$C_6$ alkyl)amino-arylcarbamoyl (e.g. N-acetyl-N-methylaminophenylcarbamoylglycyl, N-propionyl-N-methylaminophenylcarbamoylglycyl), amino acid residue as defined above substituted with $C_1$-$C_6$ alkoxy($C_1$-$C_6$)alkanoylamino-arylcarbamoyl (e.g. methoxyacetylaminophenylcarbamoylglycyl, methoxypropionylaminophenylcarbamoylglycyl), amino acid residue as defined above substituted with $C_1$-$C_6$ alkoxycarbonyl($C_1$-$C_6$)alkanoylamino-arylcarbamoyl (e.g. ethoxycarbonylacetylaminophenylcarbamoylglycyl, methoxycarbonylpropionylaminophenylcarbamoylglycyl], amino acid residue as defined above substituted with carboxyamino-arylcarbamoyl (e.g. carboxyaminophenylcarbamoylglycyl), amino acid residue as defined above substituted with $C_1$-$C_6$-alkoxycarbonylamino-arylcarbamoyl (e.g. ethoxycarbonylaminophenylcarbamoylglycyl), amino acid residue as defined above substituted with ureido-arylcarbamoyl (e.g. ureidophenylcarbamoylglycyl), amino acid residue as defined above substituted with $C_1$-$C_6$ alkylureido-arylcarbamoyl (e.g. methylureidophenylcarbamoylglycyl, ethylureidophenylcarbamoylglycyl), amino acid residue as defined above substituted with hydroxyimino($C_1$-$C_6$)alkyl-arylcarbamoyl (e.g. hydroxyiminoethylphenylcarbamoylglycyl), amino acid residue as defined above substituted with $C_1$-$C_6$-alkoxyimino($C_1$-$C_6$)alkyl-arylcarbamoyl (e.g. methoxyiminoethylphenylcarbamoylglycyl), amino acid residue as defined above substituted with $C_1$-$C_6$ alkylhydrazono($C_1$-$C_6$)alkyl-arylcarbamoyl (e.g. methylhydrazonoethylphenylcarbamoylglycyl, dimethylhydrazonoethylphenylcarbamoylglycyl), amino acid residue as defined above substituted with optionally substituted heterocyclic-arylcarbamoyl (e.g. oxopyrrolidinylphenylcarbamoylglycyl, oxopiperidinophenylcarbamoylglycyl, dioxopyrrolidinylphenylcarbamoylglycyl, oxooxazolidinylphenylcarbamoylglycyl, pyrrolylphenylcarbamoylglycyl), amino acid residue as defined above substituted with heterocycliccarbonyl-arylcarbamoyl having $C_1$-$C_6$ alkyl (e.g. methylpiperazinylcarbonylphenylcarbamoylglycyl, ethylpiperazinylcarbonylphenylcarbamoylglycyl), amino acid residue as defined above substituted with heterocycliccarbonyl-arylcarbamoyl having aryl (e.g. phenylpiperazinylcarbonylphenylcarbamoylglycyl), amino acid residue as defined above substituted with heterocycliccarbonyl-arylcarbamoyl having a heterocyclic group (e.g. pyridylpiperazinylcarbonylphenylcarbamoylglycyl), amino acid residue as defined above substituted with heterocycliccarbonyl-arylcarbamoyl having $C_1$-$C_6$ alkanoyl (e.g. acetylpiperazinylcarbonylphenylcarbamoylglycyl), amino acid residue as defined above substituted with heterocycliccarbonyl-arylcarbamoyl having $C_1$-$C_6$ alkoxycarbonyl (e.g. ethoxycarbonylpiperazinylcarbonylphenylcarbamoylglycyl), amino acid residue as defined above substituted with heterocycliccarbonyl-arylcarbamoyl having $C_1$-$C_6$ alkylamino (e.g. methylaminopiperazinylcarbonylphenylcarbamoylglycyl, dimethylaminopiperidinocarbonylphenylcarbamoylglycyl), amino acid residue as defined above substituted with heterocycliccarbonyl-arylcarbamoyl having $C_1$-$C_6$ alkylcarbamoyl (e.g. methylcarbamoylpiperazinylcarbonylphenylcarbamoylglycyl), amino acid residue as defined above substituted with hydroxy($C_1$-$C_6$)alkylcarbamoyl-arylcarbamoyl (e.g. hydroxymethylcarbamoylphenylcarbamoylglycyl, hydroxyethylcarbamoylphenylcarbamoylglycyl, bis(hydroxyethyl)carbamoylphenylcarbamoylglycyl), amino acid residue as defined above substituted with N-[hydroxy($C_1$-$C_6$)alkyl]-N-($C_1$-$C_6$ alkyl)carbamoyl-arylcarbamoyl (e.g. N-(hydroxyethyl)-N-methylcarbamoylphenylcarbamoylglycyl), amino acid residue as defined above substituted with $C_1$-$C_6$ alkoxy($C_1$-$C_6$)alkylcarbamoyl-arylcarbamoyl (e.g. methoxymethylcarbamoylphenylcarbamoylglycyl, methoxyethylcarbamoylphenylcarbamoylglycyl, bis(methoxyethyl)carbamoylphenylcarbamoylglycyl, bis(ethoxyethyl)carbamoylphenylcarbamoylglycyl), amino acid residue as defined above substituted with N-[$C_1$-$C_6$ alkoxy($C_1$-$C_6$)alkyl]-N-($C_1$-$C_6$ alkyl)carbamoyl-arylcarbamoyl (e.g. N-(methoxyethyl)-N-methylcarbamoylphenylcarbamoylglycyl, N-(methoxypropyl)-N-methylcarbamoylphenylcarbamoylglycyl), amino acid residue as defined above substituted with $C_1$-$C_6$ alkylamino($C_1$-$C_6$)alkylcarbamoyl-arylcarbamoyl (e.g. methylaminoethylcarbamoylphenylcarbamoylglycyl, dimethylaminoethylcarbamoylphenylcarbamoylglycyl), amino acid residue as defined above substituted with N-[$C_1$-$C_6$ alkylamino($C_1$-$C_6$)alkyl]-N-($C_1$-$C_6$ alkyl)carbamoyl-arylcarbamoyl (e.g. N-(dimethylaminoethyl)-N-methylcarbamoylphenylcarbamoylglycyl, N-(dimethylaminopropyl)-N-methylcarbamoylphenylcarbamoylglycyl), amino acid residue as defined above substituted with heterocycliccarbamoyl-arylcarbamoyl (e.g. morpholinylcarbamoylphenylcarbamoylglycyl, thienylcarbamoylphenylcarbamoylglycyl, pyridylcarbamoylphenylcarbamoylglycyl, pyrimidinylcarbamoylphenylcarbamoylglycyl), amino acid residue as defined above substituted with N-(heterocyclic)-N-($C_1$-$C_6$ alkyl)carbamoyl-arylcarbamoyl (e.g. N-pyridyl-N-methylcarbamoylphenylcarbamoylglycyl), amino acid residue as defined above substituted with heterocyclic($C_1$-$C_6$)alkylcarbamoyl-arylcarbamoyl (e.g. pyridylmethylcarbamoylphenylcarbamoylglycyl, pyridylethylcarbamoylphenylcarbamoylglycyl, thienylmethylcarbamoylphenylcarbamoylglycyl), amino acid residue as defined above substituted with N-[heterocyclic($C_1$-$C_6$)alkyl]-N-($C_1$-$C_6$ alkyl)carbamoyl-arylcarbamoyl (e.g. N-pyridylmethyl-N-methylcarbamoylphenylcarbamoylglycyl), amino acid residue as defined above substituted with N-[heterocyclic($C_1$-$C_6$)alkyl]-N-[$C_1$-$C_6$ alkoxy($C_1$-$C_6$)alkyl]carbamoyl-arylcarbamoyl (e.g. N-pyridylmethyl-N-methoxyethylcarbamoylphenylcarbamoylglycyl), amino acid residue as defined above substituted with arylcarbamoyl-arylcarbamoyl (e.g. phenylcarbamoylphenylcarbamoylglycyl), amino acid residue as defined above substituted with $C_1$-$C_6$ alkylaminoarylcarbamoyl-arylcarbamoyl (e.g. dimethylaminophenylcarbamoylphenylcarbamoylglycyl), amino acid residue as defined above substituted with arylthiocarbamoyl (e.g. phenylthiocarbamoylglycyl, naphthylthiocarbamoylglycyl, phenylthiocarbamoylalanyl, phenylthiocarbamoylsarcosyl), amino acid residue as defined above substituted with ar($C_1$-$C_6$)alkylcarbamoyl (e.g. benzylcarbamoylglycyl, benzylcarbamoylsarcosyl, benzylcarbamoylalanyl), amino acid residue as defined above substituted with aroylcarbamoyl (e.g. benzoylcarbamoylglycyl), amino acid residue as defined above substituted with heterocycliccarbamoyl (e.g. pyridylcarbamoylglycyl, pyridylcarbamoylalanyl, pyridylcarbamoylsarcosyl, thienylcarbamoylglycyl, pyrazolylcarbamoylglycyl, pyrimidinylcarbamoylglycyl, quinolylcarbamoylglycyl, isoquinolylcarbamoylglycyl), amino acid residue as defined above substituted with heterocyclic($C_1$-$C_6$)alkylcarbamoyl (e.g. pyridylmethylcarbamoylglycyl, pyridylethylcarbamoylglycyl, thienylmethylcarbamoylglycyl), amino acid residue as defined above substituted with arylaminocarbamoyl (e.g. phenylaminocarbamoylglycyl), amino acid residue as defined above substituted with ar($C_2$-$C_6$)alkenylsulfonyl (e.g. styrylsulfonylglycyl, cinnamylsulfonylglycyl), amino acid residue as defined above substituted with $C_1$-$C_6$ alkylsulfonyl (e.g. mesylglycyl, ethylsulfonylglycyl, mesylsarcosyl, mesylalanyl), amino acid residue as defined above substituted with phthaloyl

(e.g. phthaloylglycyl, phthaloylalanyl, phthaloyl-β-alanyl), amino acid residue as defined above having unsubstituted amino acid residue (e.g. glycylglycyl, alanylglycyl, sarcosylglycyl, prolylglycyl, glycylsarcosyl, prolylsarcosyl), amino acid residue as defined above having substituted amino acid residue as defined above such as amino acid residue having amino acid residue as defined above substituted with $C_1$-$C_6$ alkyl (e.g. dimethylglycylglycyl, diethylglycylglycyl, dimethylglycylsarcosyl, ethylsarcosylglycyl, isopropylsarcosylglycyl, ethylglycylglycyl, propylglycylglycyl, isopropylglycylglycyl, ethylglycylalanyl, dimethylglycylalanyl, dimethylalanylglycyl, dimethyl-β-alanylglycyl, etc.), amino acid residue as defined above having amino acid residue as defined above substituted with a heterocyclic group (e.g. morpholinoglycylglycyl, piperidinoglycylglycyl, pyridylglycylglycyl, piperidinosarcosylglycyl), amino acid residue as defined above having amino acid residue as defined above substituted with heterocyclic($C_1$-$C_6$)alkyl (e.g. pyridylmethylglycylglycyl, imidazolylmethylglycylglycyl, furylmethylglycylglycyl, thienylmethylsarcosylglycyl), amino acid residue as defined above having amino acid residue as defined above substituted with cycloalkyl selected from cyclopropyl, cyclobutyl, cyclopentyl, cyclohexyl, cycloheptyl, cyclooctyl and adamantyl (e.g. cyclopropylglycylglycyl, cyclobutylglycylglycyl, cyclopentylglycylglycyl, cyclohexylglycylglycyl, cycloheptylglycylglycyl, cyclooctylglycylglycyl, adamantylglycylglycyl, cyclohexylsarcosylglycyl, cycloheptylsarcosylglycyl, cyclohexylglycylsarcosyl, cyclohexylglycylalanyl), amino acid residue as defined above having amino acid residue as defined above substituted with aryl (e.g. phenylglycylglycyl, phenylsarcosylglycyl), amino acid residue as defined above having amino acid residue as defined above substituted with $C_1$-$C_6$ alkanoyl (e.g. acetylglycylglycyl, acetylprolylglycyl, propionylglycylglycyl, acetylalanylglycyl), amino acid residue as defined above having amino acid residue as defined above substituted with $C_1$-$C_6$ alkoxycarbonyl (e.g. tert-butoxycarbonylglycylglycyl, tert-butoxycarbonylprolylglycyl), amino acid residue as defined above having amino acid residue as defined above substituted with ar($C_1$-$C_6$)alkyl (e.g. benzylglycylglycyl) and amino acid residue as defined above having amino acid residue as defined above substituted with phthaloyl (e.g. phthaloylglycylglycyl); wherein in the above definition of "acyl" aryl and heterocyclic group and moiety respectively are each as defined above

- carboxy; $C_1$-$C_6$ alkoxycarbonyl (e.g. methoxycarbonyl, ethoxycarbonyl, tert-butoxycarbonyl) and; benzoyl 4-methyl-1-piperazinylcarbonyl,
- $C_1$-$C_6$ alkylaminomethyleneamino such as dimethylaminomethyleneamino and diethylaminomethyleneamino,
- pyrrolidinyl, optionally
- substituted with oxo such as pyrrolidonyl,
  and/or
- N-methylpyrrolidinylideneamino; or heterocyclic group such as thienyl, benzofuryl, benzothiazolinyl, benzothiazinyl and piperonyl, each of which may be substituted with substituent(s) selected from halogen [e.g. fluorine, chlorine, bromine and iodine], $C_1$-$C_6$ alkyl [e.g. methyl, ethyl, propyl, isopropyl], halo-aryl [e.g. chlorophenyl] and oxo;

A is $C_1$-$C_6$ alkylene such as methylene, ethylene, methylmethylene and propylene.

Suitable "a leaving group" may be a conventional acid residue such as halogen [e.g. fluoro, chloro, bromo and iodo], arenesulfonyloxy [e.g. benzenesulfonyloxy, tosyloxy], alkanesulfonyloxy [e.g. mesyloxy, ethanesulfonyloxy.

Suitable pharmaceutically acceptable salts of the object compound [I] are conventional non-toxic salts and include a metal salt such as an alkali metal salt [e.g. sodium salt, potassium salt] and an alkaline earth metal salt [e.g. calcium salt, magnesium salt], an ammonium salt, an organic base salt [e.g. trimethylamine salt, triethylamine salt, pyridine salt, picoline salt, dicyclohexylamine salt, N,N'-dibenzylethylenediamine salt], an organic acid addition salt [e.g. formate, acetate, trifluoroacetate, maleate, tartrate, oxalate, methanesulfonate, benzenesulfonate, toluenesulfonate], an inorganic acid addition salt [e.g. hydrochloride, hydrobromide, sulfate, phosphate], a salt with an amino acid [e.g. arginine salt, aspartic acid salt, glutamic acid salt], an intramolecular salt.

With respect to the salts of the compounds [Ia] to [Ig] in the Processes 3 to 6, it is to be noted that these compounds are included within the scope of the compound [I], and accordingly the suitable examples of the salts of these compounds are to be referred to those as exemplified for the object compound [I].

The processes for preparing the object compound [I] are explained in detail in the following.

Process 1

The compound [I] or its salt can be prepared by halogenating a compound [II] or its salt.

Suitable salts of the compound [II] may be the same as those exemplified for the compound [I].

The halogenation is carried out in the presence of a halogenating agent.

Suitable halogenating agents of this reaction may include conventional ones such as N-halosuccinimide [e.g. N-chlorosuccinimide, N-bromosuccinimide], and the like. These halogenating agents may be selected according to the kind of the starting compound [II] to be used.

This reaction is usually carried out in a conventional solvent such as chloroform, methylene chloride, carbon tetrachloride, dimethylformamide, methanol, ethanol, dioxane.

The reaction temperature is not critical, and the reaction is usually carried out at ambient temperature or under warming or heating.

Process 2

The object compound [I] or its salt can be prepared by reacting a compound [III] or its salt with a compound [IV] or its salt.

Suitable salts of the compounds [III] and [IV] may be the same as those exemplified for the compound [I].

The reaction is preferably carried out in the presence of a base such as alkali metal [e.g. lithium, sodium, potassium], the hydroxide or carbonate or bicarbonate thereof [e.g. sodium hydroxide, potassium carbonate, potassium bicarbonate], alkali metal alkoxide [e.g. sodium methoxide, sodium ethoxide, potassium tert-butoxide], or the like.

This reaction is usually carried out in a conventional solvent such as tetrahydrofuran, dioxane, N,N-dimethylformamide, acetone.

The reaction temperature is not critical, and the reaction is usually carried out under cooling to heating.

Process 3

The object compound [Ib] or its salt can be prepared by acylating a compound [Ia] or its salt.

The acylation is carried out in the presence of an acylating agent.

Suitable acylating agents are the corresponding carboxylic acid or sulfonic acid compounds, which are represented by the formula : R-OH wherein R is acyl as defined in claim 1, and reactive derivatives thereof, and the corresponding isocyanate or isothiocyanate compounds.

As suitable said reactive derivatives, there may be mentioned acid halides, acid anhydrides, active amides and active esters. Suitable examples are acid halides such as acid chloride and acid bromide, mixed acid anhydrides with various acids [e.g. substituted phosphoric acid such as dialkyl phosphoric acid, sulfuric acid, aliphatic carboxylic acid, aromatic carboxylic acid], symmetric acid anhydrides, active amides with various imidazoles, and active esters such as p-nitrophenyl ester and N-hydroxysuccinimide ester. The kind of such reactive derivatives can be selected depending on the kind of acyl group to be introduced.

The reaction is usually carried out in a conventional solvent, such as methylene chloride, chloroform, pyridine, dioxane, tetrahydrofuran, N,N-dimethylformamide. In case that the acylating agent is liquid, it can also be used as a solvent. In case that the carboxylic acid or sulfonic acid compounds are used as acylating agent in the free acid form or salt form, it is preferable to carry out the reaction in the presence of a conventional condensing agent such as N,N'-dicyclohexylcarbodiimide.

The reaction temperature is not critical and the reaction can be carried out under cooling, at ambient temperature, or under heating.

This reaction is preferably carried out in the presence of a conventional inorganic base or in the presence of a conventional organic base.

Process 4

The object compound [Id] or its salt can be prepared by acylating a compound [Ic] or its salt.

This reaction can be carried out in substantially the same manner as Process 3, and therefore the reaction mode and reaction condition of this reaction are to be referred to those explained in Process 3.

Process 5

The object compound [Ie] or its salt can be prepared by alkylating a compound [Ic] or its salt.

This alkylation is carried out in the presence of an alkylating agent.

Suitable alkylating agents to be used in this reaction may be halide compounds such as lower alkyl halide [e.g. methyl iodide, ethyl iodide, propyl iodide, butyl iodide] or ar($C_1$-$C_6$)alkyl halide [e.g. benzylbromide], aldehyde compounds, ketone compounds.

When halide compounds are used as alkylating agents, the reaction is usually carried out in the presence of a base such as an alkali metal [e.g. sodium, potassium], an alkaline earth metal [e.g. magnesium, calcium], the hydride or hydroxide thereof, alkali metal alkoxide [e.g. sodium methoxide, sodium ethoxide, potassium tert-butoxide.

When aldehyde compounds or ketone compounds are used as alkylating agents, the reaction is usually carried out under acidic condition in the presence of reducing agent such as formic acid, sodium cyanoborohydride, or the like.

The reaction of this process is usually carried out in a conventional solvent such as methanol, ethanol, tetrahydrofuran, dioxane, N,N-dimethylformamide, dimethyl sulfoxide. And in case that the above-mentioned alkylating agent is in liquid, it can be also used as a solvent.

The reaction temperature is not critical, and the reaction is usually carried out under cooling, at ambient temperature or under warming or heating.

Process 6

The object compound [Ig] or its salt can be prepared by reacting a compound [If] or its reactive derivative at the carboxy group or a salt thereof with a compound [IX] or its reactive derivative at the amino group or a salt thereof.

Suitable reactive derivative at the carboxy group of the compound [If] may include an acid halide, an acid anhydride, an activated amide, and an activated ester. Suitable examples of the reactive derivatives may be an acid chloride; an acid azide; a mixed acid anhydride with an acid such as dialkylphosphoric acid, sulfuric acid aliphatic carboxylic acid or aromatic carboxylic acid; a symmetrical acid anhydride; an activated amide with imidazole; or an activated ester [e.g. p-nitrophenyl ester]. These reactive derivatives can optionally be selected from them according to the kind of the compound [If] to be used.

Suitable reactive derivative at the amino group of the compound [IX] may be a silyl derivative formed by the reaction of the compound [IX] with a silyl compound such as bis(trimethylsilyl)acetamide or mono(trimethylsilyl)acetamide.

Suitable salts of the compound [IX] and its reactive derivative can be referred to the organic or inorganic acid addition salts as exemplified for the compound [I].

This reaction can be carried out in substantially the same manner as Process 3, and therefore the reaction mode and reaction condition of this reaction are to be referred to those explained in Process 3.

The starting compounds [II] or [III] or salts thereof can be prepared by the following reaction schemes.

Process A

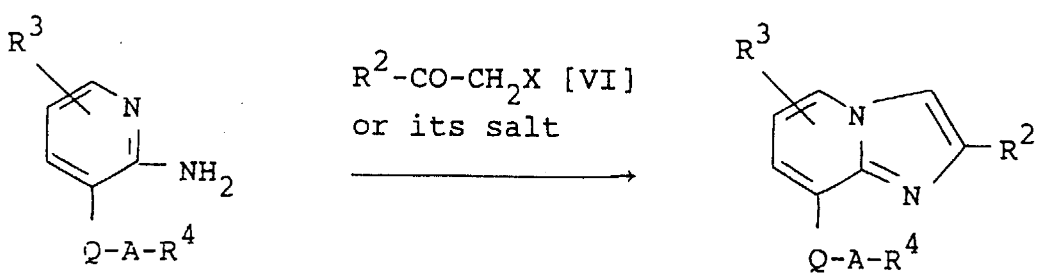


[V] or its salt

[II] or its salt

Process B

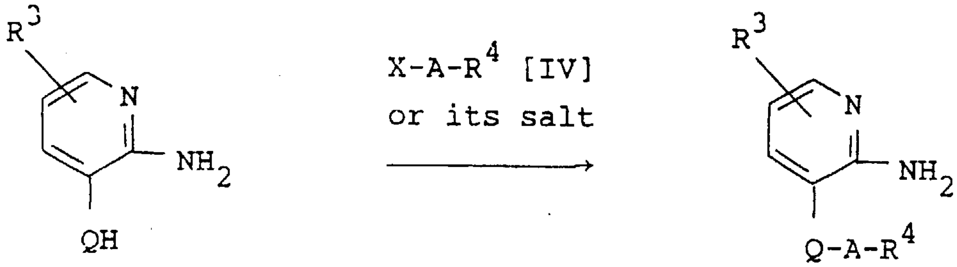


[VII] or its salt

[V] or its salt

Process C

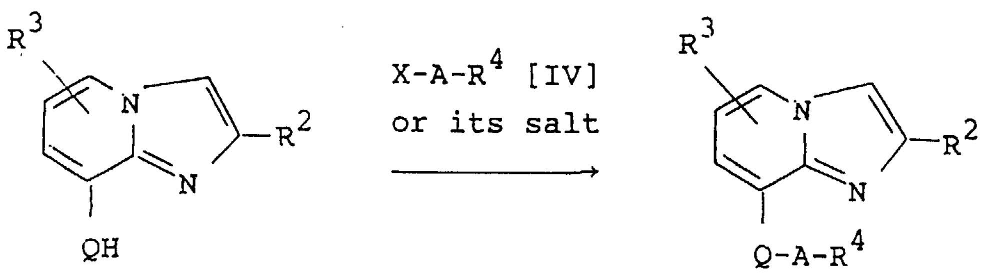


[VIII] or its salt

[II] or its salt

Process D

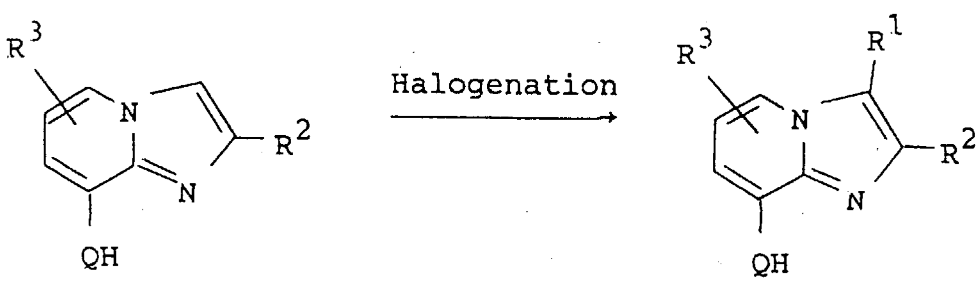


[VIII] or its salt

[III] or its salt

wherein $R^1$, $R^2$, $R^3$, $R^4$, Q, A and X are each as defined above.

The above-mentioned processes for preparing the starting compounds are explained in detail in the following.

Process A

The compound [II] or its salt can be prepared by reacting a compound [V] or its salt with a compound [VI] or its salt.

Suitable salts of the compounds [V] and [VI] may be the same as those exemplified for the compound [I].

This reaction is usually carried out in a conventional solvent such as methanol, ethanol, acetonitrile, N,N-dimethylformamide, or dimethyl sulfoxide.

The reaction temperature is not critical, and the reaction is usually carried out at ambient temperature or under warming or heating.

Process B

The compound [V] or its salt can be prepared by reacting a compound [VII] or its salt with a compound [IV] or its salt.

Suitable salts of the compound [VII] are the same as those exemplified for the compound [I].

This reaction can be carried out in substantially the same manner as Process 2, and therefore the reaction mode and reaction condition of this reaction are to be referred to those explained in Process 2.

Process C

The compound [II] or its salt can be prepared by reacting a compound [VIII] or its salt with a compound [IV] or its salt.

Suitable salts of the compound [VIII] are the same as those exemplified for the compound [I].

This reaction can be carried out in substantially the same manner as Process 2, and therefore the reaction mode and reaction condition of this reaction are to be referred to those explained in Process 2.

Process D

The compound [III] or its salt can be prepared by halogenating a compound [VIII] or its salt.

This reaction can be carried out in substantially the same manner as Process 1, and therefore the reaction mode and reaction condition of this reaction are to be referred to those explained in Process 1.

The object compound [I] and the starting compounds can also be prepared by the methods of Examples and Preparations mentioned below or similar manners thereto or conventional manners.

The compounds obtained by the above processes can be isolated and purified by a conventional method such as pulverization, recrystallization, chromatography, or reprecipitation.

It is to be noted that the compound [I] and the other compounds may include one or more stereoisomers and geometrical isomers due to asymmetric carbon atoms and double bonds, and all of such isomers and mixture thereof are included within the scope of this invention.

The object compound [I] and pharmaceutically acceptable salts thereof possess strong activities as bradykinin antagonists, and are useful for the treatment and/or the prevention of bradykinin or its analogues mediated diseases such as allergy, inflammation, autoimmune disease, shock, or pain, and more particularly for the prevention and/or the treatment of asthma, cough, bronchitis, rhinitis, rhinorrhea, obstructive pulmonary disease [e.g. pulmonary emphysema], expectoration, pneumonitis, systemic inflammatory response syndrome (SIRS), septic shock, endotoxin shock, anaphylactic shock, adult respiratory distress syndrome, disseminated intravascular coagulopathy, arthritis, rheumatism, osteoarthritis, lumbago, inflammation-induced bone resorption, conjunctivitis, vernal conjunctivitis, uveitis, iritis, iridocyclitis, headache, migraine, toothache, backache, superficial pain, cancerous pain, postoperative pain, tenalgia, trauma [e.g. wound, burn], rash, erythema, eczema or dermatitis [e.g. contact dermatitis, atopic dermatitis], urticaria, herpes, itching, psoriasis, lichen, inflammatory bowel disease [e.g. ulcerative colitis, Crohn's disease], diarrhea, hepatitis, pancreatitis, gastritis, esophagitis, food allergy, ulcer, irritable bowel syndrome, nephritis, angina, periodontitis, edema, hereditary angioneurotic edema, cerebral edema, low blood pressure, thrombosis, myocardial infarction, cerebral vasospasm, congestion, coagulation, gout, central nervous system injury, premature labor, arteriosclerosis, postgastrectomy dumping syndrome, carcinoid syndrome, altered sperm mobility, diabetic neuropathy, neuralgia, graft rejection in transplantation, in human being or animals.

And further, it is known that bradykinin relates to the release of mediators such as prostaglandins, leukotrienes, tachykinins, histamine, thromboxanes, so the compound [I] is expected to be useful for the prevention and/or the treatment of such mediators mediated diseases.

In order to illustrate the usefulness of the object compound [I], the pharmacological test data of some representative compounds of the compound [I] are shown in the following.

$^{3}$H-Bradykinin receptor binding

(i) Test Method :

(a) Crude ileum membrane preparation

Male Hartly strain guinea pigs were sacrificed by decapitation. The ileum was removed and homogenized in buffer (50 mM trimethylaminoethanesulfonic acid (TES), 1 mM 1,10-phenanthroline pH 6.8). The homogenate was centrifuged (1000 xg, 20 minutes) to remove tissue clumps and the supernatant was centrifuges (100,000 xg, 60 minutes) to yield a pellet. The pellet was resuspended in buffer (50 mM TES, 1 mM 1,10-phenanthroline, 140 mg/ℓ bacitracin, 1 mM dithiothreiol, 0.1 % bovine serum albumin pH 6.8) and homogenized with a glass-teflon homogenizer to yield suspension which was referred to as crude membrane suspension. The obtained membrane suspension was stored at -80°C until use.

(b) $^3$H-Bradykinin binding to the membrane

The frozen crude membrane suspension was thawed. In binding assays, $^3$H-Bradykinin (0.06 nM) and drug (1 x $10^{-5}$M) were incubated with 50 μl of the membrane suspension at room temperature for 60 minutes in a final volume of 250 μl. Separation of receptor-bound from free $^3$H-Bradykinin is achieved by immediate filtration under vacuum and washed three times with 5 ml of ice-cold buffer (50 mM Tris-HCl pH 7.5). Non-specific binding was defined as binding in the presence of 0.1 μM Bradykinin. The radioactivity retained on rinsed filters was determined by a liquid-scintillation counter.

(ii) Test Results

| Test Compound (Example No.) | Inhibition % of $^3$H-Bradykinin binding (concentration : 1 x $10^{-5}$M) |
|---|---|
| 35 | 99 |
| 40-(21) | 97 |
| 47 | 100 |
| 49 | 100 |
| 60 | 100 |
| 61-(1) | 95 |
| 61-(5) | 99 |
| 61-(7) | 100 |
| 61-(11) | 98 |
| 61-(13) | 98 |
| 61-(16) | 100 |
| 61-(18) | 97 |
| 61-(23) | 100 |
| 61-(28) | 99 |
| 61-(41) | 100 |
| 61-(51) | 100 |
| 61-(54) | 100 |

The effects of the compound [I] on bradykinin-induced bronchoconstriction and carrageenin-induced paw edema were measured according to similar manners described in British Journal of Pharmacology, 102, 774-777 (1991).

For therapeutic purpose, the compound [I] and a pharmaceutically acceptable salt thereof of the present invention can be used in a form of pharmaceutical preparation containing one of said compounds, as an active ingredient, in admixture with a pharmaceutically acceptable carrier such as an organic or inorganic solid, semi-solid or liquid excipient sutable for oral, parenteral such as intravenous, intramuscular, subcutaneous or intraarticular, external such as topical, enteral, intrarectal, transvaginal, inhalant, ophthalmic, nasal or hypoglossal administration. The pharmaceutical preparations may be capsules, tablets, dragees, granules, suppositories, solution, lotion, suspension, emulsion, ointment, gel, cream, or the like. If desired, there may be included in these preparations, auxiliary substances, stabilizing agents, wetting or emulsifying agents, buffers and other commonly used additives.

While the dosage of the compound [I] will vary depending upon the age and condition of the patient, an average single dose of about 0.1 mg, 1 mg, 10 mg, 50 mg, 100 mg, 250 mg, 500 mg and 1000 mg of the compound [I] may be effective for preventing and/or treating the above-mentioned diseases. In general, amounts between 0.1 mg/body and about 1,000 mg/body may be administered per day.

The following Preparations and Examples are given for the purpose of illustrating this invention.

Preparation 1

Concentrated sulfuric acid (2.8 ml) was added dropwise to 70% nitric acid (4.0 ml) in an ice-water bath. This mixture was added to a solution of 2,4,6-trichlorobenzoic acid (5.13 g) in concentrated sulfuric acid (23 ml) dropwise for 20 minutes in an ice-water bath. The mixture was stirred for 16 hours at ambient temperature and poured into ice-water (300 ml) slowly. This mixture was stirred for 1 hour at ambient temperature. The precipitate was collected by vacuum filtration and washed with water to give 2,4,6-trichloro-3-nitrobenzoic acid (5.26 g) as colorless fine crystals.

| | |
|---|---|
| mp : | 162-164°C |
| NMR ($CDCl_3$, δ) : | 5.72 (1H, br s), 7.60 (1H, s) |

Preparation 2

To a solution of 2,6-dichloro-3-nitrotoluene (50 g) in acetic acid (400 ml) and ethanol (200 ml) was added iron (67.8 g) and the mixture was refluxed for 1.5 hours under nitrogen atmosphere. The insoluble material was filtered off and the filtrate was concentrated. To the residue was added a saturated aqueous solution of sodium bicarbonate and the mixture was extracted with ethyl acetate three times. The combined organic layers were washed with a saturated aqueous solution of sodium bicarbonate three times and brine, dried over magnesium sulfate, and concentrated in vacuo to give 2,4-dichloro-3-methylaniline (41 g).

| | |
|---|---|
| mp : | 54-56°C |
| NMR ($CDCl_3$, δ) : | 2.45 (3H, s), 4.06 (2H, br s), 6.58 (1H, d, J=9Hz), 7.07 (1H, d, J=9Hz) |

Preparation 3

(1) To a mixture of 2,4-dichloro-6-mercapto-3-methylaniline (861 mg), sodium hydrogen carbonate (1.16 g), tetrahydrofuran (9 ml) and water (9 ml) was added chloroacetyl chloride (0.36 ml) dropwise under an ice-water bath cooling. The mixture was stirred under an ice-water bath cooling for 15 minutes and then heated under reflux for 1 hour. The reaction mixture was cooled and poured into a mixture of dichloromethane and brine. The aqueous layer and insoluble precipitate was extracted twice with a mixture of dichloromethane and methanol (4:1, V/V). The organic layer and extracts were combined, dried over magnesium sulfate and evaporated in vacuo to give an yellow powder of 2H-5,7-dichloro-6-methyl-1,4-benzothiazin-3(4H)-one (801 mg). This powder was used for the next step without further purification.

(2) To a solution of 2H-5,7-dichloro-6-methyl-1,4-benzothiazin-3(4H)-one (846 mg) in N,N-dimethylformamide (17 ml) was added sodium hydride (40% in oil, 150 mg) under an ice-water bath cooling. The mixture was stirred at ambient temperature for 30 minutes and then, methyl iodide (0.4 ml) was added thereto. After stirring for 30 minutes, the mixture was partitioned between ethyl acetate and brine. The aqueous layer was extracted with ethyl acetate. The organic layers were combined, dried over magnesium sulfate and evaporated in vacuo. The residue was purified by silica gel column chromatography (dichloromethane-n-hexane) followed by crystallization from n-hexane to give 2H-5,7-dichloro-4,6-dimethyl-1,4-benzothiazin-3(4H)-one (590 mg) as pale yellow crystals.

| | |
|---|---|
| mp : | 98-99°C |
| NMR ($CDCl_3$, δ) : | 2.50 (3H, s), 3.35 (2H, s), 3.40 (3H, s), 7.39 (1H, s) |

Preparation 4

To a solution of 2,4-dichloro-6-mercapto-3-methylaniline (2.08 g) in dichloromethane (40 ml) was added 1,1'-carbonyldiimidazole (1.78 g) at ambient temperature. The mixture was stirred for one hour at the same temperature. The

separated precipitate was collected by filtration, washed with dichloromethane, and dried to give 4,6-dichloro-5-methyl-2-benzothiazolinone (1.40 g).

mp : >250°C
NMR (DMSO-$d_6$, δ) : 2.42 (3H, s), 7.26 (1H, s)

Preparation 5

The following compounds were obtained according to a similar manner to that of Preparation 3-(2).

(1) 4,6-Dichloro-3,5-dimethyl-2-benzothiazolinone

mp : 120-122°C
NMR ($CDCl_3$, δ) : 2.52 (3H, s), 3.87 (3H, s), 7.33 (1H, s)

(2) 4,6-Dichloro-3-ethyl-5-methyl-2-benzothiazolinone

mp : 101-103°C
NMR ($CDCl_3$, δ) : 1.39 (3H, t, J=7.5Hz), 2.53 (3H, s), 4.49 (2H, q, J=7.5Hz), 7.36 (1H, s)

Preparation 6

The following compounds were obtained according to similar manners to those of Example 14 or 15 mentioned below.

(1) 3-Acetylamino-2,6-dichlorotoluene

mp : 118-119°C
NMR ($CDCl_3$, δ) : 2.24 (3H, s), 2.49 (3H, s), 7.29 (1H, d, J=9Hz), 7.63 (1H, br s), 8.20 (1H, d, J=9Hz)

(2) 3-(4-Chlorobutyryl)amino-2,6-dichlorotoluene

mp : 103-105°C
NMR ($CDCl_3$, δ) : 2.23 (2H, m), 2.49 (3H, s), 2.66 (2H, t, J=8Hz), 3.69 (2H, t, J=8Hz), 7.30 (1H, d, J=8Hz), 7.69 (1H, br s), 8.19 (1H, d, J=8Hz)

(3) 3-Acetoxyacetylamino-2,6-dichlorotoluene

mp : 111-112°C
NMR ($CDCl_3$, δ) : 2.25 (3H, s), 2.50 (3H, s), 4.73 (2H, s), 7.31, 8.27 (each 1H, d, J=9Hz), 8.52 1H, br s)

(4) 2,6-Dichloro-3-(phthalimidoacetyl)aminotoluene

mp : 245-246°C
NMR ($CDCl_3$, δ) : 2.48 (3H, s), 4.59 (2H, s), 7.27 (1H, d, J=9Hz), 7.70-7.96 (4H), 8.00 (1H, br s), 8.12 (1H, d, J=9Hz)

Preparation 7

The following compounds were obtained according to a similar manner to that of Example 23 mentioned below.

(1) 3-(N-Acetyl-N-methylamino)-2,6-dichlorotoluene

mp : 118-119°C
NMR ($CDCl_3$, δ) : 1.80 (3H, s), 2.53 (3H, s), 3.18 (3H, s), 7.10 (1H, d, J=9Hz), 7.38 (1H, d, J=9Hz)

(2) 3-(N-Acetoxyacetyl-N-methylamino)-2,6-dichlorotoluene

mp : 107-108°C

NMR ($CDCl_3$, δ) : 2.13 (3H, s), 2.55 (3H, s), 3.20 (3H, s), 4.16, 4.44 (each 1H, d, J=15Hz), 7.19, 7.40 (each 1H, d, J=9Hz)

(3) 2,6-Dichloro-3-[N-(phthalimidoacetyl)-N-methylamino]-toluene

mp : 193-194°C
NMR ($CDCl_3$, δ) : 2.58 (3H, s), 3.21 (3H, s), 4.10 (2H, s), 7.30 (1H, d, J=9Hz), 7.42 (1H, d, J=9Hz), 7.65-7.91 (4H)

Preparation 8

To a solution of 3-(4-chlorobutyryl)amino-2,6-dichlorotoluene (2.80 g) in N,N-dimethylformamide (30 ml) was added sodium hydride (60% oil dispersion, 440 mg) in one portion at 5°C. The mixture was stirred for 10 minutes at 5°C and then for 2 hours at 60°C. The cooled mixture was poured into ice water. The separated oil was extracted with dichloromethane. The organic layer was washed with water three times, dried, and concentrated in vacuo. The residue was purified by flash chromatography on silica gel to give 1-(2,4-dichloro-3-methylphenyl)-2-pyrrolidinone (1.95 g).

mp : 77-82°C
NMR ($CDCl_3$, δ) : 2.25 (2H, m), 2.50 (3H, s), 2.58 (2H, t, J=8Hz), 3.75 (2H, t, J=8Hz), 7.08 (1H, d, J=8Hz), 7.33 (1H, d, J=8Hz)

Preparation 9

A mixture of 4,6-dichloro-3,5-dimethyl-2-benzothiazolinone (1.30 g), N-bromosuccinimide (1.03 g), 2,2'-azobis-(2,4-dimethyl-4-methoxyvaleronitrile) (65 mg) and dichloromethane (26 ml) was heated under reflux for 2 hours. N-Bromosuccinimide (500 mg) was added therein and the mixture was heated under reflux for additional 4 hours. The reaction mixture was washed with water twice and brine, dried over magnesium sulfate and evaporated in vacuo. The residue was crystallized from diethyl ether to give 5-bromomethyl-4,6-dichloro-3-methyl-2-benzothiazolinone (1.20 g) as crystals.

mp : 142-143°C
NMR ($CDCl_3$, δ) : 3.89 (3H, s), 4.82 (2H, s), 7.40 (1H, s)

Preparation 10

The following compounds were obtained according to a similar manner to that of Preparation 9.

(1) 3-Bromomethyl-2,4-dichloro-N-trifluoroacetyl-N-methylaniline

NMR ($CDCl_3$, δ) : 3.31 (3H, s), 4.78 (2H, s), 7.23 (1H, d, J=9Hz), 7.43 (1H, d, J=9Hz)

(2) N-Acetyl-3-bromomethyl-2,4-dichloro-N-methylaniline

mp : 123°C (dec.)
NMR ($CDCl_3$, δ) : 1.81 (3H, s), 3.19 (3H, s), 4.79 (2H, s), 7.22 (1H, d, J=9Hz), 7.43 (1H, d, J=9Hz)

(3) 1-(3-Bromomethyl-2,4-dichlorophenyl)-2-pyrrolidinone

mp : 106-109°C
NMR ($CDCl_3$, δ) : 2.17-2.38 (2H, m), 2.59 (2H, t, J=8Hz), 3.79 (2H, t, J=6Hz), 4.77 (2H, s), 7.21, 7.40 (each 1H, d, J=9Hz)

(4) N-Acetoxyacetyl-3-bromomethyl-2,4-dichloro-N-methylaniline

mp : 92-93°C
NMR ($CDCl_3$, δ) : 2.12 (3H, s), 3.21 (3H, s), 4.16, 4.44 (each 1H, d, J=15Hz), 4.79 (2H, s), 7.31, 7.48 (each 1H, d, J=9Hz)

(5) 3-Bromomethyl-2,4-dichloro-N-methyl-N-(phthalimidoacetyl)aniline

mp : 211°C (dec.)
NMR ($CDCl_3$, δ) : 3.24 (3H, s), 4.09 (2H, s), 4.81 (2H, s), 7.44 (1H, d, J=9Hz), 7.51 (1H, d, J=9Hz), 7.68-7.91 (4H)

(6) N,N-Di-(tert-butoxycarbonyl)-2,4-dichloro-3-bromomethylaniline

NMR ($CDCl_3$, δ) : 1.40 (18H, s), 4.79 (2H, s), 7.13 (1H, d, J=9Hz), 7.35 (1H, d, J=9Hz)

(7) 5-Bromomethyl-4,6-dichloro-3-ethyl-2-benzothiazolinone

mp : 120-126°C
NMR ($CDCl_3$, δ) : 1.41 (3H, t, J=7.5Hz), 4.50 (2H, q, J=7.5Hz), 4.85 (2H, s), 7.42 (1H, s)

(8) 2H-6-Bromomethyl-5,7-dichloro-4-methyl-1,4-benzothiazin-3(4H)-one

NMR ($CDCl_3$, δ) : 3.38 (2H, s), 3.42 (3H, s), 4.78 (2H, s), 7.44 (1H, s)

Preparation 11

To a solution of 2,4-dichloro-1-isopropoxybenzene (3.53 g) in tetrahydrofuran (35 ml) was added n-butyl lithium solution (1.6 Mol solution in n-hexane, 11 ml) through a syringe at -78°C. After stirring for one hour at -78°C, the mixture was poured into dry diethyl ether (50 ml) containing pulverized dry ice in a few minutes. After warming to ambient temperature, the mixture was concentrated in vacuo. The residue was partitioned between diethyl ether (50 ml) and aqueous 10% sodium hydroxide solution (100 ml). The aqueous layer was adjusted to pH 2 with 10% hydrochloric acid. The separated oil was extracted with dichloromethane. The extract was washed with water, dried, and evaporated under reduced pressure. The residue was crystallized from n-hexane to give 2,6-dichloro-3-isopropoxybenzoic acid (3.0 g).

mp : 133-138°C
NMR ($CDCl_3$, δ) : 1.39 (6H, d, J=6Hz), 4.55 (1H, m), 6.95 (1H, d, J=10Hz), 7.29 (1H, d, J=10Hz)

Preparation 12

To a solution of 2,6-dichloro-3-isopropoxybenzoic acid (2.49 g) in tetrahydrofuran (30 ml) was added borane-methyl sulfide complex (2 ml, 10 Mol solution) through a syringe at ambient temperature. The mixture was refluxed for half an hour, cooled, and quenched with aqueous saturated ammonium chloride solution. The organic layer was dried and concentrated in vacuo. The residue was purified by flash chromatography on silica gel to give 2,6-dichloro-3-isopropoxybenzyl alcohol as a colorless oil (1.66 g).

NMR ($CDCl_3$, δ) : 1.38 (6H, d, J=5Hz), 2.15 (1H, t, J=7.5Hz), 4.52 (1H, m), 4.97 (2H, d, J=7.5Hz), 6.87 (1H, d, J=10Hz), 7.25 (1H, d, J=10Hz)

Preparation 13

The following compounds were obtained according to a similar manner to that of Preparation 12.

(1) 2,6-Dichloro-3-methoxybenzyl alcohol

mp : 99-101°C
NMR ($CDCl_3$, δ) : 2.12 (1H, br s), 3.90 (3H, s), 4.98 (2H, s), 6.86 (1H, d, J=9Hz), 7.30 (1H, d, J=9Hz)

(2) 2,4,6-Trichloro-3-nitrobenzyl alcohol

mp : 121-124°C
NMR ($CDCl_3$, δ) : 2.08 (1H, t, J=6Hz), 4.98 (2H, d, J=6Hz), 7.57 (1H, s)

(3) 2,6-Dichloro-3-nitrobenzyl alcohol

mp : 99-100°C
NMR ($CDCl_3$, δ) : 5.03 (2H, s), 7.50 (1H, d, J=8Hz), 7.72 (1H, d, J=8Hz)

Preparation 14

To a mixture of 2,6-dichloro-3-isopropoxybenzyl alcohol (1.65 g) and triethylamine (808 mg) in dichloromethane was added methanesulfonyl chloride (884 mg) in a few minutes at 5°C. After stirring for half an hour at 5°C, the mixture was washed with diluted hydrochloric acid and then with aqueous sodium bicarbonate solution, dried, and concentrated in vacuo. The residue was crystallized from n-hexane to give 2,6-dichloro-3-isopropoxybenzyl methanesulfonate (2.07 g).

mp : 78-81°C
NMR ($CDCl_3$, δ) : 1.49 (6H, d, J=5Hz), 3.10 (3H, s), 4.56 (1H, m), 5.54 (2H, s), 6.98 (1H, d, J=10Hz), 7.31 (1H, d, J=10Hz)

Preparation 15

The following compounds were obtained according to a similar manner to that of Preparation 14.

(1) 2,6-Dichloro-3-methoxybenzyl methanesulfonate

NMR ($CDCl_3$, δ) : 3.10 (3H, s), 3.91 (3H, s), 5.53 (2H, s), 6.98 (1H, d, J=9Hz), 7.46 (1H, d, J=9Hz)

(2) 2,4,6-Trichloro-3-nitrobenzyl methanesulfonate

mp : 113-114°C
NMR($CDCl_3$, δ) : 3.12 (3H, s), 5.50 (2H, s), 7.63 (1H, s)

(3) 2,6-Dichloro-3-nitrobenzyl methanesulfonate

mp : 78-80°C
NMR ($CDCl_3$, δ) : 3.13 (3H, s), 5.60 (2H, s), 7.57 (1H, d, J=8Hz), 7.85 (1H, d, J=8Hz)

Preparation 16

A mixture of 8-hydroxy-2-methylimidazo[1,2-a]pyridine (207 mg), 2,6-dichloro-3-nitrobenzyl bromide (400 mg) and potassium carbonate (580 mg) in N,N-dimethylformamide (8 ml) was stirred for 2 hours at 60°C. The mixture was cooled and diluted with water. The separated oil was extracted with dichloromethane. The organic layer was washed with water, dried, and concentrated in vacuo. The residue was purified by flash chromatography on silica gel to give 8-(2,6-dichloro-3-nitrobenzyloxy)-2-methylimidazo[1,2-a]pyridine (120 mg) as crystals.

mp : 183-185°C
NMR ($CDCl_3$, δ) : 2.43 (3H, s), 5.50 (2H, s), 6.59 (1H, d, J=7.5Hz), 6.66 (1H, t, J=7.5Hz), 7.32 (1H, s), 7.52 (1H, d, J=9Hz), 7.75 (1H, d, J=7.5Hz), 7.79 (1H, d, J=9Hz)

Preparation 17

A mixture of 8-hydroxy-2-methylimidazo[1,2-a]pyridine (296 mg), 1,3-dichloro-2-(2-mesyloxyethyl)benzene (645 mg) and potassium carbonate (828 mg) in N,N-dimethylformamide (12 ml) was stirred for 6 hours at 70°C. The mixture was treated according to a similar manner to that of Preparation 16 to give 8-[2-(2,6-dichlorophenyl)ethyloxy]-2-methylimidazo[1,2-a]pyridine (150 mg).

mp : 92-94°C
NMR ($CDCl_3$, δ) : 2.48 (3H, s), 3.69 (2H, m), 4.29 (2H, m), 6.49 (1H, d, J=7.5Hz), 6.61 (1H, t, J=7.5Hz), 7.15 (1H, dd, J=7Hz and 5Hz), 7.29-7.35 (3H, m), 7.69 (1H, d, J=7.5Hz)

Preparation 18

The following compounds were obtained according to similar manners to those of Preparation 16 or 17.

(1) 2-Amino-3-(2,6-dichlorobenzyloxy)-6-methylpyridine

mp : 140-141°C
NMR ($CDCl_3$, δ) : 2.35 (3H, s), 4.61 (2H, br s), 5.28 (2H, s), 6.49 (1H, d, J=8Hz), 7.08 (1H, d, J=8Hz), 7.20-7.41 (3H)

(2) 8-[1-(2,6-Dichlorophenyl)ethoxy]-2-methylimidazo[1,2-a]pyridine

mp : 173-174°C
NMR ($CDCl_3$, δ) : 1.93 (3H, d, J=7Hz), 2.48 (3H, s), 6.06-6.22 (2H), 6.42 (1H, t, J=7Hz), 7.06-7.32 (4H), 7.59 (1H, d, J=7Hz)

(3) 8-[2,6-Dichloro-3-(N-methyl-N-acetylamino)benzyloxy]-2-methylimidazo[1,2-a]pyridine

mp : 159-161°C
NMR ($CDCl_3$, δ) : 1.86 (3H, s), 2.44 (3H, s), 3.20 (3H, s), 5.50 (2H, s), 6.55-6.75 (2H), 7.29 (1H, d, J=9Hz), 7.33 (1H, s), 7.46 (1H, d, J=9Hz), 7.74 (1H, d, J=7Hz)

(4) 8-[2,6-Dichloro-3-(N-methyl-N-trifluoroacetylamino)benzyloxy]-2-methylimidazo[1,2-a]pyridine

NMR ($CDCl_3$, δ) : 2.42 (3H, s), 3.31 (3H, s), 5.48 (2H, s), 6.57-6.70 (2H, m), 7.30 (1H, d, J=10Hz), 7.33 (1H, s), 7.46 (1H, d, J=10Hz), 7.74 (1H, dd, J=7.5Hz and 2Hz)

(5) 8-[2,6-Dichloro-3-(N-methyl-N-tert-butoxycarbonylamino)benzyloxy]-2-methylimidazo[1,2-a]pyridine

(6) 8-[2,6-Dichloro-3-(N-methyl-N-mesylamino)benzyloxy]-2-methylimidazo[1,2-a]pyridine

NMR ($CDCl_3$, δ) : 2.42 (3H, s), 3.04 (3H, s), 3.29 (3H, s), 5.45 (2H, s), 6.58-6.72 (2H, m), 7.32 (1H, s), 7.42 (1H, d, J=10Hz), 7.49 (1H, d, J=10Hz), 7.74 (1H, dd, J=7.5Hz and 1.5Hz)

(7) 8-[2,6-Dichloro-3-(2-pyrrolidinon-1-yl)benzyloxy]-2-methylimidazo[1,2-a]pyridine

mp : 190-191°C
NMR ($CDCl_3$, δ) : 2.13-2.35 (2H, m), 2.42 (3H, s), 2.59 (2H, t, J=9Hz), 3.76 (2H, t, J=9Hz), 5.43 (2H, s), 6.54-6.75 (2H, m), 7.21-7.48 (3H, m), 7.73 (1H, d, J=6Hz)

(8) 8-(2,6-Dichloro-3-methoxybenzyloxy)-2-methylimidazo[1,2-a]pyridine

mp : 179-180°C
NMR ($CDCl_3$, δ) : 2.42 (3H, s), 3.91 (3H, s), 5.46 (2H, s), 6.55-6.72 (2H), 6.92 (1H, d, J=9Hz), 7.24-7.36 (2H), 7.71 (1H, d, J=7Hz)

(9) 8-(2,6-Dichloro-3-isopropoxybenzyloxy)-2-methylimidazo[1,2-a]pyridine

NMR ($CDCl_3$, δ) : 1.40 (6H, d, J=5Hz), 2.45 (3H, s), 4.57 (1H, m), 5.45 (2H, s), 6.59-6.70 (2H, m), 6.92 (1H, d, J=10Hz), 7.25-7.31 (2H, m), 7.72 (1H, dd, J=7.5Hz and 1.5Hz)

(10) 8-(2,4,6-Trichloro-3-nitrobenzyloxy)-2-methylimidazo[1,2-a]pyridine

mp : 184-186°C
NMR ($CDCl_3$, δ) : 2.44 (3H, s), 5.47 (2H, s), 6.57 (1H, d, J=8Hz), 6.67 (1H, t, J=8Hz), 7.33 (1H, s), 7.58 (1H, s), 7.76 (1H, d, J=8Hz)

(11) 8-[2-Chloro-5-(N-methyl-N-acetylamino)benzyloxy]-2-methylimidazo[1,2-a]pyridine

NMR ($CDCl_3$, δ) : 2.49 (3H, s), 3.21 (3H, s), 5.43 (2H, s), 6.40 (1H, d, J=7.5Hz), 6.58 (1H, t, J=7.5Hz), 7.10 (1H, dd, J=10Hz and 1.5Hz), 7.37 (1H, s), 7.46 (1H, d, J=10Hz), 7.49 (1H, d, J=1.5Hz), 7.74 (1H, d, J=7.5Hz)

(12) 8-[3-(N-Acetoxyacetyl-N-methylamino)-2,6-dichlorobenzyloxy]-2-methylimidazo[1,2-a]pyridine

mp : 118-119°C
NMR ($CDCl_3$, δ) : 2.14 (3H, s), 2.42 (3H, s), 3.21 (3H, s), 4.19, 4.48 (each 1H, d, J=15Hz), 5.49 (1H, s), 6.54-6.72 (2H), 7.32 (1H, s), 7.40-7.49 (each 1H, d, J=9Hz), 7.74 (1H, d, J=7Hz)

(13) 8-[2,6-Dichloro-3-(N-methyl-N-phthalimidoacetylamino)benzyloxy]-2-methylimidazo[1,2-a]pyridine

mp : 221-223°C
NMR ($CDCl_3$, δ) : 2.42 (3H, s), 3.22 (3H, s), 4.11 (2H, s), 5.52 (2H, s), 6.59-6.73 (2H), 7.32 (1H, s), 7.52 (2H, s), 7.67-7.92 (4H)

(14) 2-Methyl-8-(2-trifluoromethylbenzyloxy)imidazo[1,2-a]pyridine

mp : 131-132°C
NMR ($CDCl_3$, δ) : 2.48 (3H, s), 5.38 (2H, s), 6.35 (1H, d, J=7Hz), 6.55 (1H, t, J=7Hz), 7.32 (1H, s), 7.61 (5H, s), 7.69 (1H, d, J=7Hz)

(15) 8-(2-Methoxycarbonylbenzyloxy)-2-methylimidazo[1,2-a]pyridine

mp : 88-89°C
NMR ($CDCl_3$, δ) : 2.49 (3H, s), 3.92 (3H, s), 5.77 (2H, s), 6.38 (1H, d, J=7.5Hz), 6.55 (1H, d, J=7.5Hz), 7.33 (1H, s), 7.37 (1H, t, J=7.5Hz), 7.53 (1H, t, J=7.5Hz), 7.67 (1H, d, J=7.5Hz), 7.88 (1H, d, J=7.5Hz), 8.05 (1H, d, J=7.5Hz)

(16) 8-(2-Phenylbenzyloxy)-2-methylimidazo[1,2a]pyridine

mp : 90-92°C
NMR ($CDCl_3$, δ) : 2.48 (3H, s), 5.19 (2H, s), 6.14 (1H, d, J=7.5Hz), 6.48 (1H, t, J=7.5Hz), 7.31-7.45 (9H, m), 7.63 (1H, d, J=7.5Hz), 7.75 (1H, m)

(17) 8-(2,6-Difluorobenzyloxy)-2-methylimidazo[1,2-a]pyridine

mp : 114-116°C
NMR ($CDCl_3$, δ) : 2.45 (3H, s), 5.31 (2H, s), 6.56-6.67 (2H, m), 6.86-6.99 (2H, m), 7.25-7.41 (1H, m), 7.32 (1H, s), 7.70 (1H, d, J=7.5Hz)

(18) 8-(2,6-Dibromobenzyloxy)-2-methylimidazo[1,2-a]pyridine

mp : 159-162°C
NMR ($CDCl_3$, δ) : 2.43 (3H, s), 5.49 (2H, s), 6.59 (1H, d, J=7Hz), 6.67 (1H, t, J=7Hz), 7.08 (1H, t, J=8Hz), 7.30 (1H, s), 7.58 (2H, d, J=8Hz), 7.71 (1H, d, J=7Hz)

(19) 8-(2-Chloro-6-fluorobenzyloxy)-2-methylimidazo[1,2-a]pyridine

mp : 150-151°C
NMR ($CDCl_3$, δ) : 2.44 (3H, s), 5.38 (2H, s), 6.59 (1H, d, J=7Hz), 6.63 (1H, t, J=7Hz), 7.02 (1H, t, J=8Hz), 7.20-7.38 (3H), 7.70 (1H, d, J=7Hz)

(20) 8-(4-Bromo-2-fluorobenzyloxy)-2-methylimidazo[1,2-a]pyridine

mp : 150-151°C
NMR ($CDCl_3$, δ) : 2.48 (3H, s), 5.31 (2H, s), 6.42 (1H, d, J=7.5Hz), 6.58 (1H, t, J=7.5Hz), 7.23-7.33 (3H, m), 7.49 (1H, t, J=7.5Hz), 7.69 (1H, d, J=7.5Hz)

(21) 8-(2-Chloro-5-nitrobenzyloxy)-2-methylimidazo[1,2-a]pyridine

mp : 134-135°C
NMR ($CDCl_3$, δ) : 2.49 (3H, s), 5.48 (2H, s), 6.41 (1H, d, J=7Hz), 6.60 (1H, t, J=7Hz), 7.36 (1H, s), 7.60 (1H, d, J=9Hz), 7.74 (1H, d, J=7Hz), 8.16 (1H, dd, J=9Hz and 2Hz), 8.55 (1H, d, J=2Hz)

(22) 8-[2-Chloro-6-(N-methyl-N-acetylamino)benzyloxy]-2-methylimidazo[1,2-a]pyridine

NMR ($CDCl_3$, δ) : 1.90 (3H, s), 2.41 (3H, s), 3.23 (3H, s), 5.19 (1H, d, J=10Hz), 5.26 (1H, d, J=10Hz), 6.55 (1H, d, J=7.5Hz), 6.67 (1H, t, J=7.5Hz), 7.18 (1H, dd, J=7.5Hz and 2Hz), 7.33 (1H, s), 7.48-7.54 (2H, m), 7.74 (1H, dd, J=7.5Hz and 2Hz)

(23) 8-(2-Chloro-6-nitrobenzyloxy)-2-methylimidazo[1,2-a]pyridine

mp : 162-163°C
NMR ($CDCl_3$, δ) : 2.41 (3H, s), 5.62 (2H, s), 6.51-6.69 (2H), 7.30 (1H, s), 7.48 (1H, t, J=8Hz), 7.63-7.75 (2H), 7.85 (1H, d, J=7Hz)

(24) 8-[3-(N,N-Di-tert-butoxycarbonylamino)-2,6-dichlorobenzyloxy]-2-methylimidazo[1,2-a]pyridine

mp : 180-181°C
NMR ($CDCl_3$, δ) : 1.41 (18H, s), 2.42 (3H, s), 5.53 (2H, s), 6.51-6.68 (2H), 7.20 (1H, d, J=9Hz), 7.30 (1H, s), 7.38 (1H, d, J=9Hz), 7.70 (1H, d, J=7Hz)

(25) 2-Methyl-8-(2,4,6-trichlorobenzyloxy)imidazo[1,2-a]pyridine

mp : 187-189°C
NMR ($CDCl_3$, δ) : 2.42 (3H, s), 5.40 (2H, s), 6.58 (1H, d, J=7Hz), 6.67 (1H, t, J=7Hz), 7.31 (1H, s), 7.49 (2H, s), 7.71 (1H, d, J=7Hz)

(26) 8-(2,3,6-Trichlorobenzyloxy)-2-methylimidazo[1,2-a]pyridine

mp : 148-149°C
NMR ($CDCl_3$, δ) : 2.42 (3H, s), 5.48 (2H, s), 6.52-6.71 (2H), 7.30 (1H, d, J=9Hz), 7.31 (1H, s), 7.45 (1H, d, J=9Hz), 7.72 (1H, d, J=7Hz)

(27) 8-(2,6-Dibromo-4-methoxycarbonylbenzyloxy)-2-methylimidazo[1,2-a]pyridine

mp : 153-154°C
NMR ($CDCl_3$, δ) : 2.42 (3H, s), 3.97 (3H, s), 5.51 (2H, s), 6.58 (1H, d, J=7Hz), 6.68 (1H, t, J=7Hz), 7.31 (1H, s), 7.74 (1H, d, J=7Hz), 8.21 (2H, s)

(28) 8-(4-Benzoyl-2-chlorobenzyloxy)-2-methylimidazo[1,2-a]pyridine

NMR ($CDCl_3$, δ) : 2.49 (3H, s), 5.49 (2H, s), 6.40 (1H, d, J=8Hz), 6.60 (1H, d, J=8Hz), 7.35 (1H, s), 7.44-7.88 (9H)

(29) 8-(2,6-Dichloro-4-benzoylbenzyloxy)-2-methylimidazo[1,2-a]pyridine

NMR ($CDCl_3$, δ) : 2.45 (3H, s), 5.50 (2H, s), 6.57-6.72 (2H), 7.28-7.92 (9H)

(30) 8-[4-(2-Cyanophenyl)benzyloxy]-2-methylimidazo[1,2-a]pyridine

NMR ($CDCl_3$, δ) : 2.49 (3H, s), 5.38 (2H, s), 6.44 (1H, d, J=8Hz), 6.58 (1H, t, J=8Hz), 7.32 (1H, s), 7.38-7.82 (9H)

(31) 8-(6-Chloropiperonyl)methoxy-2-methylimidazo[1,2-a]pyridine

mp : 141-142°C
NMR ($CDCl_3$, δ) : 2.49 (3H, s), 5.31 (2H, s), 5.96 (2H, s), 6.38 (1H, d, J=7Hz), 6.58 (1H, t, J=7Hz), 6.86 (1H, s), 7.11 (1H, s), 7.31 (1H, s), 7.68 (1H, d, J=7Hz)

(32) 8-(2-Bromothiophen-4-yl)methoxy-2-methylimidazo[1,2-a]pyridine

mp : 127-128°C
NMR ($CDCl_3$, δ) : 2.46 (3H, s), 5.42 (2H, s), 6.48 (1H, d, J=7Hz), 6.59 (1H, t, J=7Hz), 7.09 (1H, s), 7.16-7.35 (2H), 7.69 (1H, d, J=7Hz)

(33) 8-(3-Chlorobenzofuran-2-yl)methoxy-2-methylimidazo[1,2-a]pyridine

mp : 141-143°C
NMR ($CDCl_3$, δ) : 2.45 (3H, s), 5.44 (2H, s), 6.56-6.66 (2H, m), 7.29-7.71 (6H, m)

(34) 8-[3-(4-Chlorophenyl)-5-methylbenzofuran-2-yl]methoxy-2-methylimidazo[1,2-a]pyridine

mp : 140-141°C
NMR ($CDCl_3$, δ) : 2.45 (6H, s), 5.33 (2H, s), 6.49 (1H, d, J=7Hz), 6.58 (1H, t, J=7Hz), 7.12-7.56 (8H), 7.70 (1H, d, J-7Hz)

(35) 8-(4,6-Dichloro-3-methyl-2-benzothiazolinon-5-yl)methoxy-2-methylimidazo[1,2-a]pyridine

mp : 236-237°C
NMR ($CDCl_3$, δ) : 2.42 (3H, s), 3.87 (3H, s), 5.50 (2H, s), 6.56-6.72 (2H, m), 7.32 (1H, s), 7.42 (1H, s), 7.72 (1H, d, J=7Hz)

(36) 8-(4,6-Dichloro-3-ethyl-2-benzothiazolinon-5-yl)methoxy-2-methylimidazo[1,2-a]pyridine

mp : 202-205°C
NMR ($CDCl_3$, δ) : 1.41 (3H, t, J=7.5Hz), 2.45 (3H, s), 4.49 (2H, g, J=7.5Hz), 5.53 (2H, s), 6.59-6.72 (2H, m), 7.31 (1H, s), 7.44 (1H, s), 7.74 (1H, dd, J=7.5Hz and 1.5Hz)

(37) 8-(5,7-Dichloro-4-methyl-1,4-benzothiazin-3(4H)-on-6-yl)methoxy-2-methylimidazo[1,2-a]pyridine

NMR ($CDCl_3$, δ) : 2.44 (3H, s), 3.40 (2H, s), 3.43 (3H, s), 5.45 (2H, s), 6.58 (1H, d, J=8Hz), 6.67 (1H, t, J=8Hz), 7.32 (1H, s), 7.47 (1H, s), 7.73 (1H, d, J=8Hz)

Preparation 19

A mixture of 2-amino-3-(2,6-dichlorobenzyloxy)-pyridine (1.345 g) and 3-bromo-1,1,1-trifluoroacetone (1.147 g) in ethanol (26 ml) was refluxed for 5 hours, cooled, and concentrated in vacuo. The residue was partitioned between ethyl acetate and aqueous sodium bicarbonate solution. The organic layer was washed with water, dried, and concentrated under reduced pressure to give a solid, which was purified by flash chromatography on silica gel to give 8-(2,6-dichlorobenzyloxy)-2-trifluoromethylimidazo[1,2-a]pyridine as a white solid (803 mg).

mp : 184-185°C
NMR ($CDCl_3$, δ) : 5.50 (2H, s), 6.75 (1H, d, J=7.5Hz), 6.85 (1H, t, J=7.5Hz), 7.23-7.38 (3H, m), 7.83 (1H, d, J=7.5Hz), 7.88 (1H, s)

Preparation 20

The following compounds were obtained according to a similar manner to that of Preparation 19.

(1) 8-(2,6-Dichlorobenzyloxy)-2-ethylimidazo[1,2-a]pyridine

mp : 153-154°C
NMR ($CDCl_3$, δ) : 1.30 (3H, t, J=7Hz), 2.84 (2H, q, J=7Hz), 5.46 (2H, s), 6.55-6.71 (2H), 7.19-7.42 (4H), 7.73

(1H, d, J=7Hz)

(2) Ethyl 8-(2,6-dichlorobenzyloxy)imidazo[1,2-a]pyridine-2-carboxylate

mp : 176-178°C
NMR ($CDCl_3$, δ) : 1.40 (3H, t, J=7Hz), 4.42 (2H, q, J=7Hz), 5.49 (2H, s), 6.69 (1H, d, J=7Hz), 6.81 (1H, t, J=7Hz), 7.21-7.43 (3H), 7.81 (1H, d, J=7Hz), 8.18 (1H, s)

(3) 8-(2,6-Dichlorobenzyloxy)-2,5-dimethylimidazo[1,2-a]pyridine

mp : 140-141°C
NMR ($CDCl_3$, δ) : 2.47 (6H, s), 5.44 (2H, s), 6.45 (1H, d, J=7Hz), 6.60 (1H, d, J=7Hz), 7.15-7.40 (4H)

(4) 8-Amino-2,7-dimethylimidazo[1,2-a]pyridine dihydrochloride

mp : >250°C
NMR (DMSO-$d_6$, δ) : 2.25 (3H, s), 2.46 (3H, s), 6.82 (2H, br s), 7.10 (1H, d, J=7Hz), 7.90 (1H, s), 8.03 (1H, d, J=7Hz)

Preparation 21

8-(2,6-Dichlorophenyl)methylamino-2,7-dimethylimidazo[1,2-a]pyridine (100 mg) was obtained by reacting 8-amino-2,7-dimethylimidazo[1,2-a]pyridine (100 mg) with 2,6-dichlorobenzaldehyde (191 mg) according to a similar manner to that of Example 10 mentioned below.

mp : 87-88°C
NMR ($CDCl_3$, δ) : 2.26 (3H, s), 2.40 (3H, s), 4.46 (1H, br s), 4.99 (2H, d, J=5Hz), 6.41 (1H, d, J=7Hz), 7.07-7.40 (4H), 7.53 (1H, d, J=7Hz)

Preparation 22

8-(2,6-Dichlorophenyl)methylamino-2-methylimidazo[1,2-a]pyridine was obtained according to a similar manner to that of Preparation 21.

mp : 119-121°C
NMR ($CDCl_3$, δ) : 2.38 (3H, s), 4.69 (2H, d, J=4Hz), 5.19 (1H, t, J=4Hz), 6.29 (1H, d, J=7.5Hz), 6.63 (1H, t, J=7.5Hz), 7.15-7.36 (3H, m), 7.32 (1H, s), 7.47 (1H, d, J=7.5Hz)

Preparation 23

To a suspension of sodium hydride (60% oil dispersion, 17 mg) was added 8-acetylamino-2-methylimidazo[1,2-a]pyridine (73 mg), and the mixture was stirred for 30 minutes, 2,6-dichlorobenzyl bromide (97 mg) was added thereto, and the mixture was stirred for 1 hour. Water was added thereto, and the mixture was extracted with methylene chloride three times. The combined organic layer was washed with water four times and brine, dried over magnesium sulfate, and concentrated in vacuo. The residue was crystallized with diethyl ether to give 8-[N-(2,6-dichlorophenyl)methyl-N-acetylamino]-2-methylimidazo[1,2-a]pyridine (85 mg).

mp : 177-178°C
NMR ($CDCl_3$, δ) : 1.95 (3H, s), 2.48 (3H, s), 4.92 (1H, br d, J=15Hz), 6.00 (1H, br d, J=15Hz), 6.36 (1H, d, J=7Hz), 6.45 (1H, t, J=7Hz), 7.00-7.21 (3H), 7.38 (1H, s), 7.98 (1H, d, J=7Hz)

Preparation 24

To a solution of 8-[2,6-dichloro-3-(2-pyrrolidon-1-yl)benzyloxy]-2-methylimidazo[1,2-a]pyridine (120 mg) in tetrahydrofuran (3 ml) was added lithium aluminum hydride (19 mg) under ice-cooling, and the mixture was stirred for 2 hours. A saturated aqueous ammonium chloride solution was added thereto, and insoluble material was filtered off. The filtrate was concentrated, and the residue was purified by preparative thin-layer chromatography (5% solution of methanol in methylene chloride) to give 8-[2,6-dichloro-3-(1-pyrrolidinyl)benzyloxy]-2-methylimidazo[1,2-a]pyridine (23 mg).

NMR ($CDCl_3$, δ) : 1.86-2.01 (4H, m), 2.41 (3H, s), 3.26-3.40 (4H, m), 5.44 (2H, s), 6.57-6.72 (2H, m), 6.90, 7.19 (each 1H, d, J=9Hz), 7.31 (1H, s), 7.72 (1H, d, J=7Hz)

Preparation 25

8-(3-Amino-2,6-dichlorobenzyloxy)-2-methylimidazo[1,2-a]pyridine was obtained according to a similar manner to that of Example 4 mentioned below.

NMR (DMSO-$d_6$, δ) : 2.27 (3H, s), 5.30 (2H, s), 5.70 (2H, s), 6.64-7.00 (3H), 7.24 (1H, d, J=8Hz), 7.65 (1H, s), 8.09 (1H, d, J=7Hz)

Preparation 26

8-(2,6-Dichloro-3-methylaminobenzyloxy)-2-methylimidazo[1,2-a]pyridine was obtained according to a similar manner to that of Example 7 mentioned below.

mp : 167-168°C
NMR ($CDCl_3$, δ) : 2.40 (3H, s), 2.91 (3H, d, J=5Hz), 4.41 (1H, br d, J=5Hz), 5.48 (2H, s), 6.56-6.78 (3H), 7.18 (1H, d, J=9Hz), 7.33 (1H, br s), 7.80 (1H, br d, J=6Hz)

Preparation 27

8-[2,6-Dichloro-3-(N-methyl-N-acetylamino)benzyloxy]-2-methylimidazo[1,2-a]pyridine was obtained according to a similar manner to that of Example 14 mentioned below.

mp : 159-161°C
NMR ($CDCL_3$, δ) : 1.86 (3H, s), 2.44 (3H, s), 3.20 (3H, s), 5.50 (2H, s), 6.55-6.75 (2H), 7.29 (1H, d, J=9Hz), 7.33 (1H, s), 7.46 (1H, d, J=9Hz), 7.74 (1H, d, J=7Hz)

Preparation 28

8-[2,6-Dichloro-3-(N-methoxycarbonyl-N-methylamino)-benzyloxy]-2-methylimidazo[1,2-a]pyridine was obtained according to a similar manner to that of Example 18 mentioned below.

NMR ($CDCl_3$, δ) : 2.40 (3H, s), 3.21 (3H, s), 3.68 (3H, s), 5.45 (2H, s), 6.59-6.82 (2H), 7.21-7.49 (3H), 7.81 (1H, d, J=6Hz)

Preparation 29

Pivaloyl chloride (0.22 ml) was added dropwise to a mixture of acetylsarcosine (264 mg), N-methylmorpholine (0.22 ml) and N-methylpyrrolidone (30 ml) under a dry ice-tetrachloromethane bath cooling. This mixture was stirred for 10 minutes under ice-cooling, and 8-[3-amino-2,6-dichlorobenzyloxy]-2-methylimidazo[1,2-a]pyridine (500 mg) was added thereto under a dry ice-tetrachloromethane bath cooling. The mixture was stirred for 22 hours at ambient temperature. The mixture was partitioned between ethyl acetate and a saturated aqueous sodium bicarbonate solution, and the aqueous layer was extracted with ethyl acetate twice. The combined organic layer was dried over magnesium sulfate and concentrated in vacuo, and the residue was purified by flash column chromatography (methylene chloride:methanol = 50:1, V/V) to give 8-[3-(acetylsarcosylamino)-2,6-dichlorobenzyloxy]-2-methylimidazo[1,2-a]pyridine (260 mg).

NMR ($CDCl_3$, δ) : 2.18 (3H, s), 2.42 (3H, s), 3.09 (0.3H, s), 3.19 (2.7H, s), 4.19 (2H, s), 5.45 (2H, s), 6.55-6.72 (2H), 7.30 (1H, s), 7.34 (1H, d, J=9Hz), 7.72 (1H, d, J=8Hz), 8.38 (1H, d, J=9Hz), 8.81 (1H, br s)

Preparation 30

8-[2,6-Dichloro-3-(N-ethyl-N-methylamino)benzyloxy]-2-methylimidazo[1,2-a]pyridine was obtained according to a similar manner to that of Example 26 mentioned below.

NMR (DMSO-$d_6$, δ) : 1.10 (3H, t, J=7Hz), 2.43 (3H, s), 2.75 (3H, s), 3.05 (2H, q, J=7Hz), 5.59 (2H, s), 7.28-7.62

(3H), 7.71 (1H, d, J=7Hz), 8.16 (1H, br s), 8.55 (1H, d, J=7Hz)

Preparation 31

(1) 3,5'-Dichlorobenzanilide was obtained according to a similar manner to that of Example 15 from 3,5-dichloroaniline and benzoyl chloride.

mp : 148-149°C
NMR ($CDCl_3$, δ) : 7.15 (1H, t, J=0.5Hz), 7.44-7.60 (3H, m), 7.51 (2H, d, J=0.5Hz), 7.76-7.97 (3H, m)

(2) 3',5'-Dichloro-N-methylbenzanilide was obtained according to a similar manner to that of Example 23.

NMR ($CDCl_3$, δ) : 3.46 (3H, s), 6.94 (2H, d, J=0.5Hz), 7.13 (1H, t, J=0.5Hz), 7.20-7.41 (5H, m)

(3) To a stirred solution of 3',5'-dichloro-N-methylbenzanilide (2.317 g) in tetrahydrofuran was added lithium aluminum hydride (380 mg) in an ice-bath and the resulting suspension was stirred at the same temperature for one hour. The reaction mixture was quenched with saturated ammonium chloride and filtered through celite pad. The inorganic material on the celite was washed with ethyl acetate. Methanol was added to the filtrate and stirred at ambient temperature for half an hour. The organic layer was washed with water, dried over anhydrous magnesium sulfate and concentrated in vacuo. The residue was purified by flash chromatography eluting with ethyl acetate - n-hexane (1:9, V/V) to afford 3,5-dichloro-N-methylaniline (1.38 g) as a colorless oil.

NMR ($CDCl_3$, δ) : 2.81 (3H, s), 3.87 (1H, br s), 6.94 (2H, d, J=0.5Hz), 6.66 (1H, t, J=0.5Hz)

(4) The mixture of 3,5-dichloro-N-methylaniline (345 mg) and excess amount of benzyl bromide and triethylamine in acetonitrile (7 ml) was refluxed for 4 hours. The reaction mixture was separated with ethyl acetate and water, and the organic layer was washed with water, dried and concentrated in vacuo to give N-benzyl-3,5-dichloro-N-methylaniline (532 mg).

NMR ($CDCl_3$, δ) : 3.01 (3H, s), 4.50 (2H, s), 6.57 (2H, d, J=0.5Hz), 6.67 (2H, t, J=0.5Hz), 7.10-7.46 (5H, m)

(5) Phosphoryl chloride (12.7 ml) was dropwise added to N,N-dimethylformamide (70 ml), and the mixture was stirred for 30 minutes at ambient temperature. A solution of N-benzyl-3,5-dichloro-N-methylaniline (7.26 g) in N,N-dimethylformamide (30 ml) was dropwise added thereto, and the mixture was stirred for 30 minutes at ambient temperature and then for 30 minutes at 50°C. The reaction mixture was neutralized with 1N aqueous sodium hydroxide solution and extracted with ethyl acetate. The organic solution was washed with water and brine, dried over magnesium sulfate and concentrated in vacuo. The residue was purified by a flash chromatography (ethyl acetate : n-hexane = 1:6, V/V) to give N-benzyl-3,5-dichloro-4-formyl-N-methylaniline (5.70 g).

mp : 66-70°C
NMR ($CDCl_3$, δ) : 3.10 (3H, s), 4.60 (2H, s), 6.65 (2H s), 6.98-7.42 (5H, m), 10.30 (1H, s)

(6) To a solution of N-benzyl-3,5-dichloro-4-formyl-N-methylaniline (820 mg) in ethyl acetate (10 ml) was added palladium hydroxide (80 mg) under nitrogen atmosphere. This mixture was stirred under hydrogen atmosphere under atmospheric pressure at ambient temperature for one and half an hour. The precipitate was dissolved into chloroform and filtered through celite and the filtrate was concentrated in vacuo. The residual solid was suspended in diisopropyl ether and warmed at 90°C. After being stirred and cooled, the solid was collected by filtration to afford 3,5-dichloro-4-formyl-N-methylaniline (470 mg) as a pale brown solid.

mp : 172-174°C
NMR ($CDCl_3$, δ) : 2.90 (3H, t, J=5Hz), 4.50 (1H, br s), 6.50 (2H, s), 10.32 (1H, s)

(7) To a solution of 3,5-dichloro-4-formyl-N-methylaniline (242 mg) in methanol (3 ml) and tetrahydrofuran (3 ml) was added sodium borohydride (45 mg) and the mixture was stirred for 30 minutes at ambient temperature. The reaction mixture was quenched with aqueous saturated ammonium chloride solution and ethyl acetate was added thereto. The separated organic layer was washed with aqueous saturated ammonium chloride solution, dried and concentrated in vacuo to give 3,5-dichloro-4-hydroxymethyl-N-methylaniline (246 mg).

mp : 108-111°C
NMR ($CDCl_3$, δ) : 1.85 (1H, t, J=6Hz), 2.81 (3H, d, J=5Hz), 3.91 (1H, br s), 4.84 (2H, d, J=6Hz), 6.51 (2H, s)

(8) 3,5-Dichloro-4-hydroxymethyl-N-methylacetanilide was obtained according to a similar manner to that of Example 14.

NMR ($CDCl_3$, δ) : 1.97 (3H, br s), 2.18 (1H, t, J=7Hz), 3.26 (3H, s), 4.96 (2H, d, J=7Hz), 7.23 (2H, s)

(9) 8-[2,6-Dichloro-4-(N-acetyl-N-methylamino)benzyloxy]-2-methylimidazo[1,2-a]pyridine was obtained according to a similar manner to that of Preparation 14 and then Preparation 17.

NMR ($CDCl_3$, δ) : 2.04 (3H, br s), 2.44 (3H, s), 3.27 (3H, s), 5.42 (2H, s), 6.60 (1H, dd, J=7Hz and 0.5Hz), 6.68 (1H, t, J=7Hz), 7.24 (2H, s), 7.33 (1H, s), 7.73 (1H, dd, J=7Hz and 0.5Hz)

Preparation 32

8-[3-(N-Glycyl-N-methylamino)-2,6-dichlorobenzyloxy]-2-methylimidazo[1,2-a]pyridine was obtained according to a similar manner to that of Example 34.

mp : 144-147°C
NMR ($CDCl_3$, δ) : 2.42 (3H, s), 3.00 (1H, d, J=17Hz), 3.12 (1H, d, J=17Hz), 3.22 (3H, s), 5.49 (2H, s), 6.56-6.72 (2H), 7.25 (1H, d, J=9Hz), 7.31 (1H, s), 7.45 (1H, d, J=9Hz), 7.72 (1H, d, J=7Hz)

Preparation 33

8-[2,6-Dichloro-3-[N-[N-(N,N-dimethylglycyl)glycyl]-N-methylamino]benzyloxy]-2-methylimidazo[1,2-a]pyridine was obtained according to a similar manner to that of Example 69 mentioned below.

NMR ($CDCl_3$, δ) : 2.31 (6H, s), 2.42 (3H, s), 2.94 (2H, s), 3.25 (3H, s), 3.56 (1H, dd, J=18Hz and 5Hz), 3.86 (1H, dd, J=18Hz and 5Hz), 5.48 (2H, s), 6.59-6.72 (2H), 7.31 (1H, s), 7.33 (1H, d, J=9Hz), 7.49 (1H, d, J=9Hz), 7.72 (1H, d, J=7Hz), 7.89 (1H, br s)

Example 1

To a solution of 8-(2,6-dichloro-3-nitrobenzyloxy)-2-methylimidazo[1,2-a]pyridine (85 mg) in a mixture of ethanol (1 ml) and 1,4-dioxane (1 ml) was added in one portion N-bromosuccinimide (43 mg) at ambient temperature. After stirring for one hour at the same temperature, the mixture was filtered to give 3-bromo-8-(2,6-dichloro-3-nitrobenzyloxy)-2-methylimidazo[1,2-a]pyridine (85 mg) as a yellow solid.

mp : 217-219°C
NMR (DMSO-$d_6$, δ) : 2.31 (3H, s), 5.50 (2H, s) 7.0-7.04 (2H, m), 7.89-7.98 (2H, m), 8.23 (1H, d, J=9Hz)

Example 2

To a solution of 8-(2,6-dichlorobenzyloxy)-2-methylimidazo[1,2-a]pyridine (100 mg) in ethanol (2 ml) was added in one portion N-chlorosuccinimide (65.3 mg) at ambient temperature. After stirring for 1 hour at the same temperature, water was added thereto, and the mixture was extracted with methylene chloride. The organic layer was washed with brine, dried and concentrated in vacuo. The residue was subjected to a column chromatography on silica gel eluting with 1% solution of methanol in methylene chloride. The desired residue was recrystallized with a mixture of benzene and n-hexane to give 3-chloro-8-(2,6-dichlorobenzyloxy)-2-methylimidazo-[1,2-a]pyridine (63 mg).

mp : 185-186°C
NMR ($CDCl_3$, δ) : 2.43 (3H, s), 5.48 (2H, s), 6.69 (1H, d, J=7Hz), 6.81 (1H, t, J=7Hz), 7.19-7.40 (3H, m), 7.69 (1H, d, J=7Hz)

Example 3

The following compounds were obtained according to similar manners to those of Examples 1 or 2.

(1) 3-Bromo-8-(2,6-dichlorobenzyloxy)-2-methylimidazo[1,2-a]pyridine

mp : 173-174°C
NMR ($CDCl_3$, δ) : 2.44 (3H, s), 5.48 (2H, s), 6.70 (1H, d, J=7Hz), 6.82 (1H, t, J=7Hz), 7.19-7.41 (3H, m), 7.75 (1H, d, J=7Hz)

(2) 3-Chloro-8-(2,6-dichlorobenzyloxy)-2-trifluoromethylimidazo[1,2-a]pyridine

mp : 213-5°C
NMR ($CDCl_3$-$CD_3OD$, δ) : 5.53 (2H, s), 6.98 (1H, d, J=7.5Hz), 7.12 (1H, t, J=7.5Hz), 7.31-7.46 (3H, m), 7.93 (1H, d, J=7.5Hz)

(3) 3-Chloro-8-(2,6-dichlorobenzyloxy)-2-ethylimidazo[1,2-a]pyridine

mp : 169-170°C
NMR ($CDCl_3$, δ) : 1.30 (3H, t, J=7Hz), 2.81 (2H, q, J=7Hz), 5.49 (2H, s), 6.69 (1H, d, J=7Hz), 6.72 (1H, t, J=7Hz), 7.19-7.41 (3H, m), 7.71 (1H, d, J=7Hz)

(4) Ethyl 3-Chloro-8-(2,6-dichlorobenzyloxy)imidazo[1,2-a]pyridine-2-carboxylate

mp : 207-208°C
NMR ($CDCl_3$, δ) : 1.41 (3H, t, J=7Hz), 4.47 (2H, q, J=7Hz), 5.50 (2H, s), 6.77 (1H, d, J=7Hz), 6.96 (1H, t, J=7Hz), 7.21-7.44 (3H, m), 7.82 (1H, d, J=7Hz)

(5) 3-Chloro-8-(2,6-dichlorobenzyloxy)-2,5-dimethylimidazo[1,2-a]pyridine

mp : 181-182°C
NMR ($CDCl_3$, δ) : 2.40 (3H, s), 2.89 (3H, s), 5.40 (2H, s), 6.47 (1H, d, J=7Hz), 6.53 (1H, d, J=7Hz), 7.19-7.39 (3H, m)

(6) 3-Bromo-8-[1-(2,6-dichlorophenyl)ethoxy]-2-methylimidazo[1,2-a]pyridine

mp : 135-136°C
NMR ($CDCl_3$, δ) : 1.93 (3H, d, J=7Hz), 2.49 (3H, s), 6.11-6.27 (2H), 6.60 (1H, t, J=7Hz), 7.13 (1H), 7.22-7.32 (2H), 7.61 (1H, d, J=7Hz)

(7) 3-Chloro-8-(2,6-dichlorobenzylamino)-2,7-dimethylimidazo[1,2-a]pyridine

mp : 144-145°C
NMR ($CDCl_3$, δ) : 2.38 (3H, s), 2.40 (3H, s), 4.44 (1H, br s), 5.00 (2H, d, J=5Hz), 6.59 (1H, d, J=7Hz), 7.09-7.42 (3H, m), 7.51 (1H, d, J=7Hz)

(8) 3-Bromo-8-[N-(2,6-dichlorobenzyl)-N-acetylamino]-2-methylimidazo[1,2-a]pyridine

mp : 141-142°C
NMR ($CDCl_3$, δ) : 1.90 (3H, s), 2.49 (3H, s), 4.90 (1H, br d, J=14Hz), 5.99 (1H, br d, J=14Hz), 6.45 (1H, d, J=7Hz), 6.61 (1H, t, J=7Hz), 7.01-7.21 (3H, m), 7.98 (1H, d, J=7Hz)

(9) 3-Chloro-8-[2-(2,6-dichlorophenyl)ethyloxy]-2-methylimidazo[1,2-a]pyridine

mp : 131-134°C
NMR ($CDCl_3$, δ) : 2.48 (3H, s), 3.66 (2H, m), 4.33 (2H, m), 6.58 (1H, d, J=7.5Hz), 6.78 (1H, t, J=7.5Hz), 7.15 (1H, dd, J=7Hz and 5Hz), 7.32 (2H, d, J=7.5Hz), 7.66 (1H, d, J=7.5Hz)

(10) 3-Chloro-8-(2,6-dichloro-3-nitrobenzyloxy)-2-methylimidazo[1,2-a]pyridine

mp : 203-205°C
NMR ($CDCl_3$, δ) : 2.43 (3H, s), 5.55 (2H, s), 6.70 (1H, d, J=7.5Hz), 6.85 (1H, t, J=7.5Hz), 7.54 (1H, d, J=9Hz),

7.74 (1H, d, J=7.5Hz), 7.82 (1H, d, J=9Hz)

(11) 3-Chloro-8-[2,6-dichloro-3-(N-methyl-N-acetylamino)benzyloxy]-2-methylimidazo[1,2-a]pyridine

mp : 212-213°C
NMR ($CDCl_3$, δ) : 1.87 (3H, s), 2.48 (3H, s), 3.20 (3H, s), 5.51 (2H, s), 6.74 (1H, br d, J=7Hz), 6.90 (1H, br t, J=7Hz), 7.29 (1H, d, J=9Hz), 7.45 (1H, d, J=9Hz), 7.76 (1H, d, J=7Hz)

(12) 3-Bromo-8-[2,6-dichloro-3-(N-methyl-N-trifluoroacetylamino)benzyloxy]-2-methylimidazo[1,2-a]pyridine

mp : 175-176°C
NMR ($CDCl_3$, δ) : 2.45 (3H, s), 3.33 (3H, s), 5.52 (2H, s), 6.71 (1H, d, J=7.5Hz), 6.85 (1H, t, J=7.5Hz), 7.33 (1H, d, J=8Hz), 7.46 (1H, d, J=8Hz), 7.79 (1H, dd, J=7.5Hz and 1.5Hz)

(13) 3-Bromo-8-[2,6-dichloro-3-(N-methoxycarbonyl-N-methylamino)benzyloxy]-2-methylimidazo[1,2-a]pyridine

mp : 178-179°C
NMR ($CDCl_3$, δ) : 2.48 (3H, s), 3.22 (3H, s), 3.65 (3H, s), 5.49 (2H, s), 6.78 (1H, br d, J=7Hz), 6.90 (1H, br t, J=7Hz), 7.25 (1H, d, J=9Hz), 7.39 (1H, d, J=9Hz), 7.79 (1H, d, J=7Hz)

(14) 3-Bromo-8-[2,6-dichloro-3-(N-methyl-N-tert-butoxycarbonylamino)benzyloxy]-2-methylimidazo[1,2-a]pyridine

mp : 122-123°C
Mass (M+1) : 516

(15) 3-Bromo-8-[2,6-dichloro-3-(N-methyl-N-mesylamino)benzyloxy]-2-methylimidazo[1,2-a]pyridine

mp : 161-164°C
NMR ($CDCl_3$, δ) : 2.45 (3H, s), 3.07 (3H, s), 3.30 (3H, s), 5.48 (2H, m), 6.71 (1H, d, J=7.5Hz), 6.85 (1H, t, J=7.5Hz), 7.52 (1H, d, J=10Hz), 7.60 (1H, d, J=10Hz), 7.77 (1H, d, J=7.5Hz and 1.5Hz)

(16) 3-Bromo-8-[2,6-dichloro-3-(N-ethyl-N-methylaminobenzyloxy]-2-methylimidazo[1,2-a]pyridine dihydrochloride

mp : 128-130°C
NMR ($CDCl_3$ + $CD_3OD$, δ) : 1.30 (3H, t, J=7Hz), 2.64 (3H, s), 3.39 (3H, s), 3.80 (2H, q, J=7Hz), 5.69 (2H, s), 7.49 (2H, d, J=4Hz), 7.65 (1H, d, J=9Hz), 8.01-8.21 (2H)

(17) 3-Bromo-8-[2,6-dichloro-3-(2-pyrrolidinon-1-yl)benzyloxy]-2-methylimidazo[1,2-a]pyridine

mp : 219-220°C
NMR ($CDCl_3$, δ) : 2.16-2.35 (2H, m), 2.42 (3H, s), 2.58 (2H, t, J=8Hz), 3.78 (2H, t, J=7Hz), 5.47 (2H, s), 6.71 (1H, d, J=7Hz), 6.85 (1H, t, J=7Hz), 7.28, 7.42 (each 1H, d, J=9Hz), 7.76 (1H, d, J=7Hz)

(18) 3-Bromo-8-[2,6-dichloro-3-(1-pyrrolidinyl)benzyloxy]-2-methylimidazo[1,2-a]pyridine

mp : 113-114°C
NMR ($CDCl_3$, δ) : 1.88-2.02 (4H, m), 2.42 (3H, s), 3.25-3.41 (4H, m), 5.50 (2H, s), 6.71 (1H, d, J=7Hz), 6.82 (1H, t, J=7Hz), 6.90, 7.20 (each 1H, d, J=9Hz), 7.72 (1H, d, J=7Hz)

(19) 3-Bromo-8-(2,6-dichloro-3-methoxybenzyloxy)-2-methylimidazo[1,2-a]pyridine

mp : 175-176°C
NMR ($CDCl_3$, δ) : 2.43 (3H, s), 3.91 (3H, s), 5.48 (2H, s), 6.70 (1H, d, J=7Hz), 6.82 (1H, t, J=7Hz), 6.92 (1H, d, J=9Hz), 7.31 (1H, d, J=9Hz), 7.72 (1H, d, J=7Hz)

(20) 3-Bromo-8-(2,6-dichloro-3-isopropoxybenzyloxy)-2-methylimidazo[1,2-a]pyridine

mp : 115-117°C
NMR ($CDCl_3$, δ) : 1.40 (6H, d, J=5Hz), 2.44 (3H, s), 4.53 (1H, m), 5.45 (2H, s), 6.70 (1H, d, J=7.5Hz), 6.81 (1H, t, J=7.5Hz), 6.93 (1H, d, J=10Hz), 7.27 (1H, d, J=10Hz), 7.73 (1H, dd, J=7.5Hz and 1.5Hz)

(21) 3-Bromo-8-(2,4,6-trichloro-3-nitrobenzyloxy)-2-methylimidazo[1,2-a]pyridine

mp : 167-168°C
NMR ($CDCl_3$, δ) : 2.45 (3H, s), 5.48 (2H, s), 6.68 (1H, d, J=8Hz), 6.83 (1H, t, J=8Hz), 7.60 (1H, s), 7.78 (1H, d, J=8Hz)

(22) 3-Bromo-8-[2-chloro-5-(N-methyl-N-acetylamino)benzyloxy]-2-methylimidazo[1,2-a]pyridine

mp : 141-145°C
NMR ($CDCl_3$, δ) : 1.78 (3H, s), 2.50 (3H, s), 3.20 (3H s), 5.45 (2H, s), 6.50 (1H, d, J=7.5Hz), 6.77 (1H, t, J=7.5Hz), 7.13 (1H, dd, J=10Hz and 1.5Hz), 7.42-7.52 (2H, m), 7.75 (1H, d, J=10Hz)

(23) 3-Bromo-8-[3-(N-acetoxyacetyl-N-methylamino)-2,6-dichlorobenzyloxy]-2-methylimidazo[1,2-a]pyridine

NMR ($CDCl_3$, δ) : 2.14 (3H, s), 2.43 (3H, s), 3.21 (2H, s), 4.18, 4.49 (each 1H, d, J=15Hz), 5.50 (2H, s), 6.70 (1H, d, J=7Hz), 6.83 (1H, t, J=7Hz), 7.39, 7.49 (each 1H, d, J=9Hz), 7.78 (1H, d, J=7Hz)

(24) 3-Bromo-8-[2,6-dichloro-3-(N-methyl-N-phthalimidoacetylamino)benzyloxy]-2-methylimidazo[1,2-a]pyridine

mp : 229-230°C
NMR ($CDCl_3$, δ) : 2.43 (3H, s), 3.26 (3H, s), 4.12 (2H, s), 5.53 (2H, s), 6.72 (1H, d, J=7Hz), 6.87 (1H, d, J=7Hz), 7.52 (2H, s), 7.68-7.92 (5H)

(25) 8-[3-(Acetylsarcosyl)amino-2,6-dichlorobenzyloxy]-3-bromo-2-methylimidazo[1,2-a]pyridine

NMR ($CDCl_3$, δ) : 2.20 (3H, s), 2.42 (3H, s), 3.19 (3H, s), 4.19 (2H, s), 5.48 (2H, s), 6.70 (1H, d, J=8Hz), 6.83 (1H, t, J=8Hz), 7.35 (1H, d, J=9Hz), 7.75 (1H, d, J=8Hz), 8.37 (1H, d, J=9Hz), 8.82 (1H, br s)

(26) 3-Chloro-8-(2-trifluromethylbenzyloxy)-2-methylimidazo[1,2-a]pyridine

mp : 164-165°C
NMR ($CDCl_3$, δ) : 2.48 (3H, s), 5.40 (2H, s), 6.44 (1H, d, J=7Hz), 6.71 (1H, t, J=7Hz), 7.55-7.71 (5H)

(27) 3-Chloro-8-(2-methoxycarbonylbenzyloxy)-2-methylimidazo[1,2-a]pyridine

mp : 141-144°C
NMR ($CDCl_3$, δ) : 2.48 (3H, s), 3.92 (3H, s), 5.79 (2H, s), 6.49 (1H, d, J=7.5Hz), 6.72 (1H, t, J=7.5Hz), 7.39 (1H, t, J=7.5Hz), 7.55 (1H, t, J=7.5Hz), 7.67 (1H, d, J=7.5Hz), 7.85 (1H, d, J=7.5Hz), 8.08 (1H, d, J=7.5Hz)

(28) 8-(2-Phenylbenzyloxy)-3-chloro-2-methylimidazo[1,2-a]pyridine

mp : 121-122°C
NMR ($CDCl_3$, δ) : 2.45 (3H, s), 5.19 (2H, s), 6.21 (1H, d, J=7.5Hz), 6.63 (1H, t, J=7.5Hz), 7.30-7.41 (8H, m), 7.61 (1H, d, J=7.5Hz), 7.72 (1H, m)

(29) 3-Chloro-8-(2,6-difluorobenzyloxy)-2-methylimidazo[1,2-a]pyridine

mp : 168-170°C
NMR ($CDCl_3$, δ) : 2.45 (3H, s), 5.33 (2H, s), 6.68 (1H, d, J=7.5Hz), 6.80 (1H, t, J=7.5Hz), 6.87-7.00 (2H, m), 7.28-7.41 (1H, m), 7.70 (1H, d, J=7.5Hz)

(30) 3-Chloro-8-(2,6-dibromobenzyloxy)-2-methylimidazo[1,2-a]pyridine

mp : 157-158°C
NMR ($CDCl_3$, δ) : 2.44 (3H, s), 5.51 (2H, s), 6.69 (1H, d, J=7Hz), 6.82 (1H, t, J=7Hz), 7.08 (1H, t, J=8Hz), 7.57 (2H, d, J=8Hz), 7.70 (1H, d, J=7Hz)

(31) 3-Bromo-8-(2-chloro-6-fluorobenzyloxy)-2-methylimidazo[1,2-a]pyridine

mp : 130-131°C
NMR ($CDCl_3$, δ) : 2.43 (3H, s), 5.38 (2H, s), 6.70 (1H, d, J=7Hz), 6.81 (1H, t, J=7Hz), 7.02 (1H, t, J=8Hz), 7.20-7.38 (2H), 7.73 (1H, d, J=7Hz)

(32) 8-(4-Bromo-2-fluorobenzyloxy)-3-chloro-2-methylimidazo[1,2-a]pyridine

mp : 182-123°C
NMR ($CDCl_3$, δ) : 2.47 (3H, s), 5.32 (2H, s), 6.51 (1H, d, J=7.5Hz), 6.75 (1H, t, J=7.5Hz), 7.29 (2H, d, J=7.5Hz), 7.48 (1H, t, J=7.5Hz), 7.66 (1H, d, J=7.5Hz)

(33) 3-Bromo-8-(2-chloro-5-nitrobenzyloxy)-2-methylimidazo[1,2-a]pyridine

mp : 155-156°C
NMR ($CDCl_3$, δ) : 2.50 (3H, s), 5.48 (2H, s), 6.53 (1H, d, J=7Hz), 6.78 (1H, t, J=7Hz), 7.60 (1H, d, J=7Hz), 7.78 (1H, d, J=7Hz), 8.15 (1H, dd, J=9Hz and 2Hz), 8.52 (1H, d, J=2Hz)

(34) 3-Bromo-8-[2-chloro-6-(N-methyl-N-acetylamino)benzyloxy]-2-methylimidazo[1,2-a]pyridine

mp : 135-136°C
NMR ($CDCl_3$, δ) : 1.89 (3H, s), 2.41 (3H, s), 3.23 (3H, s), 5.21 (1H, d, J=10Hz), 5.26 (1H, d, J=10Hz), 6.68 (1H, d, J=7.5Hz), 6.82 (1H, t, J=7.5Hz), 7.18 (1H, d, J=7.5Hz), 7.38-7.53 (2H, m), 7.74 (1H, d, J=7.5Hz)

(35) 3-Bromo-8-(2-chloro-6-nitrobenzyloxy)-2-methylimidazo[1,2-a]pyridine

mp : 157-158°C
NMR ($CDCl_3$, δ) : 2.42 (3H, s), 5.66 (2H, s), 6.67 (1H, d, J=7Hz), 6.82 (1H, t, J=7Hz), 7.49 (1H, t, J=8Hz), 7.72 (2H, t, J=8Hz), 7.85 (1H, d, J=7Hz)

(36) 3-Bromo-8-[3-(N,N-di-tert-butoxycarbonylamino)-2,6-dichlorobenzyloxy]-2-methylimidazo[1,2-a]pyridine

mp : 154-155°C
NMR ($CDCl_3$, δ) : 1.40 (18H, s), 2.43 (3H, s) , 5.58 (2H, s), 6.68 (1H, d, J=7Hz), 6.79 (1H, t, J=7Hz), 7.20 (1H, d, J=8Hz), 7.38 (1H, d, J=8Hz), 7.72 (1H, d, J=7Hz)

(37) 3-Bromo-8-(2,4,6-trichlorobenzyloxy)-2-methylimidazo[1,2-a]pyridine

mp : 170-172°C
NMR ($CDCl_3$, δ) : 2.44 (3H, s), 5.42 (2H, s), 6.68 (1H, d, J=7Hz), 6.81 (1H, t, J=7Hz), 7.49 (2H, s), 7.76 (1H, d, J=7Hz)

(38) 3-Bromo-8-(2,3,6-trichlorobenzyloxy)-2-methylimidazo[1,2-a]pyridine

mp : 183-184°C
NMR ($CDCl_3$, δ) : 2.44 (3H, s), 5.50 (2H, s), 6.70 (1H, d, J=7Hz), 6.83 (1H, t, J=7Hz), 7.31 (1H, d, J=9Hz), 7.46 (1H, d, J=9Hz), 7.76 (1H, d, J=7Hz)

(39) 3-Chloro-8-(2,4,6-trimethylbenzyloxy)-2-methylimidazo[1,2-a]pyridine

mp : 154-155°C
NMR ($CDCl_3$, δ) : 2.29 (3H, s), 2.38 (6H, s), 2.43 (3H, s), 5.21 (2H, s), 6.63 (1H, d, J=7Hz), 6.75-6.90 (3H), 7.69 (1H, d, J=7Hz)

(40) 3-Bromo-8-(2,6-dibromo-4-methoxycarbonylbenzyloxy)-2-methylimidazo[1,2-a]pyridine

mp : 189-190°C
NMR ($CDCl_3$, δ) : 2.44 (3H, s), 3.96 (3H, s), 5.52 (2H, s), 6.69 (1H, d, J=7Hz), 6.82 (1H, t, J=7Hz), 7.76 (1H, d, J=7Hz), 8.21 (2H, s)

(41) 3-Bromo-8-(4-benzoyl-2-chlorobenzyloxy)-2-methylimidazo[1,2-a]pyridine

NMR ($CDCl_3$, δ) : 2.50 (3H, s), 5.50 (2H, s), 6.52 (1H, d, J=8Hz), 6.78 (1H, t, J=8Hz), 7.45-7.90 (9H)

(42) 3-Bromo-8-(2,6-dichloro-4-benzoylbenzyloxy)-2-methylimidazo[1,2-a]pyridine

mp : 129-130°C
NMH ($CDCl_3$, δ) : 2.46 (3H, s), 5.53 (2H, s), 6.71 (1H, d, J=7Hz), 6.83 (1H, t, J=7Hz), 7.34 (1H, d, J=9Hz), 7.42-7.91 (7H)

(43) 3-Bromo-8-[4-(2-cyanophenyl)benzyloxy]-2-methylimidazo[1,2-a]pyridine

mp : 138-140°C
NMR ($CDCl_3$, δ) : 2.49 (3H, s), 5.40 (2H, s), 6.55 (1H, d, J=8Hz), 6.75 (1H, t, J=8Hz), 7.40-7.81 (9H)

(44) 3-Bromo-8-(6-chloropiperonyl)methoxy-2-methylimidazo[1,2-a]pyridine

mp : 185-186°C
NMR ($CDCl_3$, δ) : 2.49 (3H, s), 5.32 (2H, s), 5.96 (2H, s), 6.49 (1H, d, J=7Hz), 6.74 (1H, t, J=7Hz), 6.88 (1H, s), 7.09 (1H, s), 7.70 (1H, d, J=7Hz)

(45) 8-(2-Bromothiophen-4-yl)methoxy-3-chloro-2-methylimidazo[1,2-a]pyridine

mp : 124-125°C
NMR ($CDCl_3$, δ) : 2.48 (3H, s), 5.45 (2H, s), 6.56 (1H, d, J=7Hz), 6.75 (1H, t, J=7Hz), 7.09 (1H, s), 7.20 (1H, s), 7.69 (2H, d, J=7Hz)

(46) 3-Chloro-8-(3-chlorobenzofuran-2-yl)methoxy-2-methylimidazo[1,2-a]pyridine

mp : 132-134°C
NMR ($CDCl_3$, δ) : 2.46 (3H, s), 5.48 (2H, s), 6.71-6.82 (2H, m), 7.31-7.71 (5H, m)

(47) 3-Bromo-8-[3-(4-chlorophenyl)-5-methylbenzofuran-2-yl]methoxy-2-methylimidazo[1,2-a]pyridine

mp : 167-170°C
NMR ($CDCl_3$, δ) : 2.44 (6H, s), 5.38 (2H, s), 6.59 (1H, d, J=7Hz), 6.72 (1H, t, J=7Hz), 7.18 (1H, br d, J=8Hz), 7.32-7.52 (6H), 7.71 (1H, d, J=7Hz)

(48) 3-Bromo-8-(4,6-dichloro-3-methyl-2-benzothiazolinon-5-yl)methoxy-2-methylimidazo[1,2-a]pyridine

mp : 225-227°C
NMR ($CDCl_3$, δ) : 2.45 (3H, s), 3.87 (3H, s), 5.51 (2H, s), 6.71 (1H, d, J=7Hz), 6.83 (1H, t, J=7Hz), 7.42 (1H, s), 7.78 (1H, d, J=7Hz)

(49) 3-Bromo-8-(4,6-dichloro-3-ethyl-2-benzothiazolinon-5-yl)methoxy-2-methylimidazo[1,2-a]pyridine

mp : 219-220°C
NMR (DMSO-$d_6$, δ) : 1.35 (3H, t, J=7.5Hz), 2.35 (3H, s), 4.43 (2H, q, J=7.5Hz), 5.52 (2H, s), 6.93-7.02 (2H, m), 7.83-7.89 (2H, m)

(50) 3-Bromo-8-(5,7-dichloro-4-methyl-1,4-benzothiazin-3(4H)-on-6-yl)methoxy-2-methylimidazo[1,2-a]pyridine

NMR ($CDCl_3$, δ) : 2.44 (3H, s), 3.39 (2H, s), 3.41 (3H, s), 5.47 (2H, s), 6.70 (1H, d, J=8Hz), 6.84 (1H, t, J=8Hz), 7.48 (1H, s), 7.77 (1H, d, J=8Hz)

Example 4

A suspension of 3-bromo-8-(2,6-dichloro-3-nitrobenzyloxy)-2-methylimidazo[1,2-a]pyridine (215 mg) and iron (powder, 84 mg) in a mixture of conc. hydrochloric acid (1 ml) and methanol (1 ml) was refluxed for half an hour. The cooled mixture was poured into an ice water (15 ml). The precipitates were collected and washed with water to give 8-(3-amino-2,6-dichlorobenzyloxy)-3-bromo-2-methylimidazo[1,2-a]pyridine dihydrochloride (140 mg) as an off-white solid.

mp : 181-183°C
NMR (DMSO-$d_6$, δ) : 2.42 (3H, s), 5.48 (2H, s), 6.95 (1H, d, J=7.5Hz), 7.24 (1H, d, J=7.5Hz), 7.42 (1H, t, J=7.5Hz), 7.62 (1H, d, J=7.5Hz), 8.25 (1H, d, J=7.5Hz)

Example 5

To a mixture of 3-bromo-8-(2-chloro-6-nitrobenzyloxy)-2-methylimidazo[1,2-a]pyridine (300 mg), nickel(II) chloride hexahydrate (360 mg) and methanol (9 ml) was added sodium borohydride (114 mg) portionwise under an ice-water bath cooling for 20 minutes. After stirring for 30 minutes, the mixture was concentrated in vacuo and diluted with water. The pH value of the mixture was adjusted to 3 and the separated crystals were collected by filtration. The crystals were dissolved in dichloromethane, and the solution was washed with saturated aqueous solution of sodium hydrogencarbonate, dried over magnesium sulfate and evaporated in vacuo. The residue was purified by silica gel column chromatography (3% solution of methanol in dichloromethane) and preparative thin-layer chromatography (3% solution of methanol in dichloromethane) followed by crystallization from diethyl ether to give 8-(2-amino-6-chlorobenzyloxy)-3-bromo-2-methylimidazo[1,2-a]pyridine (40 mg).

mp : 177-178°C
NMR ($CDCl_3$, δ) : 2.44 (3H, s), 4.64 (2H, br s), 5.51 (2H, s), 6.57 (1H, d, J=7Hz), 6.71-6.85 (3H), 7.02 (1H, t, J=7Hz), 7.71 (1H, m)

Example 6

The following compounds were obtained according to similar manners to those of Examples 4 or 5.

(1) 8-(3-Amino-2,4,6-trichlorobenzyloxy)-3-bromo-2-methylimidazo[1,2-a]pyridine

mp : 217-219°C
NMR (DMSO-$d_6$, δ) : 2.30 (3H, s), 5.35 (2H, s), 6.99 (2H, d, J=8Hz), 7.58 (1H, s), 7.92 (1H, t, J=8Hz)

(2) 8-(3-Amino-2,6-dichlorobenzyloxy)-3-bromo-2-methylimidazo[1,2-a]pyridine

NMR (DMSO-$d_6$, δ) : 2.29 (3H, s), 5.31 (2H, s), 5.70 (2H, s, $D_2O$ exchangeable), 6.84-7.04 (3H), 7.23 (1H, d, J=8Hz), 7.90 (1H, d, J=5Hz)

(3) 8-(5-Amino-2-chlorobenzyloxy)-3-bromo-2-methylimidazo[1,2-a]pyridine dihydrochloride

mp : >250°C (dec.)
NMR (DMSO-$d_6$, δ) : 2.45 (3H, s), 4.21 (2H, br s), 5.41 (2H, s), 7.03 (1H, d, J=9Hz), 7.29-7.52 (4H), 8.22 (1H, d, J=6Hz)

Example 7

To a suspension of 3-bromo-8-[2,6-dichloro-3-(N-methyl-N-trifluoroacetylamino)benzyloxy]-2-methylimidazo [1,2-a]pyridine (560 mg) in methanol (6 ml) was added 28% sodium methoxide in methanol (1.93 g). The mixture was refluxed for one hour and cooled. The precipitated solid was filtered, washed with methanol, and dried to give 3-bromo-8-(2,6-dichloro-3-methylaminobenzyloxy)-2-methylimidazo[1,2-a]pyridine (350 mg) as crystals.

mp : 184-187°C
NMR ($CDCl_3$, δ) : 2.44 (3H, s), 2.91 (2H, d, J=6Hz), 4.46 (1H, m), 5.46 (2H, s), 6.69 (1H, d, J=8.5Hz), 6.71 (1H, d, J=7.5Hz), 6.83 (1H, t, J=7.5Hz), 7.24 (1H, d, J=8.5Hz), 7.73 (1H, d, J=7.5Hz)

Example 8

The following compounds were obtained according to a similar manner to that of Example 7.

(1) 3-Bromo-8-(2-chloro-5-methylaminobenzyloxy)-2-methylimidazo[1,2-a]pyridine

mp : 156-159°C
NMR ($CDCl_3$, δ) : 2.48 (3H, s), 2.76 (3H, s), 5.38 (2H, s), 6.43-6.52 (2H, m), 6.73 (1H, t, J=7.5Hz), 6.85 (1H, d, J=2Hz), 7.19 (1H, d, J=9Hz), 7.69 (1H, d, J=7.5Hz)

(2) 3-Chloro-8-(2,6-dichloro-3-methylaminobenzyloxy)-2-methylimidazo[1,2-a]pyridine

mp : 187-188°C
NMR ($CDCl_3$, δ) : 2.43 (3H, s), 2.90 (3H, d, J=6Hz), 4.46 (1H, br q, J=6Hz), 5.45 (2H, s), 6.62 (1H, d, J=9Hz), 6.68 (1H, d, J=8Hz), 6.82 (1H, t, J=8Hz), 7.24 (1H, d, J=9Hz), 7.69 (1H, d, J=8Hz)

Example 9

A mixture of 8-(3-amino-2,6-dichlorobenzyloxy)-3-bromo-2-methylimidazo[1,2-a]pyridine dihydrochloride (100 mg) and formaldehyde (200 mg, 37% wt % solution in water) in 90% aqueous formic acid (1 ml) was refluxed for 40 minutes. The cooled mixture was concentrated in vacuo. The residue was partitioned between ethyl acetate and aqueous sodium bicarbonate solution. The organic layer was dried and concentrated in vacuo to give an oil, which was purified by preparation thin-layer chromatography on silica gel (ethyl acetate:n-hexane = 2:1, V/V) to give 3-bromo-8-(2,6-dichloro-3-dimethylaminobenzyloxy)-2-methylimidazo[1,2-a]pyridine as a white solid (31 mg).

mp : 120-122°C
NMR (DMSO-$d_6$, δ) : 2.29 (3H, s), 2.76 (6H, s), 5.41 (2H, s), 6.97-7.03 (2H, m), 7.33 (1H, d, J=9Hz), 7.52 (1H, d, J=9Hz), 7.90 (1H, m)

Example 10

To a mixture of 8-(3-amino-2,6-dichlorobenzyloxy)-3-bromo-2-methylimidazo[1,2-a]pyridine dihydrochloride (100 mg) and acetone (116 mg) in 3 M HCl solution in ethanol (2 ml) was added sodium cyanoborohydride (25 mg) in one portion. The mixture was stirred for 2 hours at ambient temperature and then concentrated in vacuo. The residue was partitioned between ethyl acetate and aqueous sodium bicarbonate solution. The organic layer was dried and concentrated in vacuo to give an oil, which was purified by preparative thin-layer chromatography on silica gel (ethyl acetate:n-hexane = 1:2, V/V), to give 3-bromo-8-(2,6-dichloro-3-isopropylaminobenzyloxy)-2-methylimidazo[1,2-a]pyridine as a white solid (50 mg).

mp : 133-134°C
NMR ($CDCl_3$, δ) : 1.26 (6H, d, J=5Hz), 2.45 (3H, s), 3.66 (1H, m), 4.22 (1H, d, J=7Hz), 5.42 (2H, s), 6.63 (1H, d, J=7.5Hz), 6.70 (1H, d, J=7.5Hz), 6.83 (1H, t, J=7.5Hz), 7.19 (1H, d, J=7.5Hz), 7.73 (1H, d, J=7.5Hz)

Example 11

The following compounds were obtained according to a similar manner to that of Example 10.

(1) 8-(3-Benzylamino-2,6-dichlorobenzyloxy)-3-bromo-2-methylimidazo[1,2-a]pyridine

mp : 126-127°C
NMR ($CDCl_3$, δ) : 2.45 (3H, s), 4.43 (2H, d, J=5Hz), 4.89 (1H, t, J=5Hz), 5.46 (2H, s), 6.58 (1H, d, J=7.5Hz), 6.71 (1H, d, J=7.5Hz), 6.83 (1H, t, J=7.5Hz), 7.15 (1H, d, J=7.5Hz), 7.29-7.43 (5H, m), 7.72 (1H, d, J=7.5Hz)

(2) 3-Bromo-8-[2,6-dichloro-3-(4-pyridylmethyl)aminobenzyloxy]-2-methylimidazo[1,2-a]pyridine

mp : 214-215°C

NMR ($CDCl_3$, δ) : 2.44 (3H, s), 4.47 (2H, d, J=6Hz), 5.05 (1H, t, J=5Hz), 5.46 (2H, s), 6.41 (1H, d, J=7.5Hz), 6.71 (1H, d, J=7.5Hz), 6.83 (1H, t, J=7.5Hz), 7.12 (1H, d, J=7.5Hz), 7.25-7.29 (2H, m), 7.74 (1H, d, J=7.5Hz), 8.59 (2H, m)

Example 12

To a suspension of 8-(3-amino-2,6-dichlorobenzyloxy)-3-bromo-2-methylimidazo[1,2-a]pyridine dihydrochloride (100 mg) in a mixture of pyridine (0.5 ml) and N,N-diethylformamide (1.5 ml) was added methanesulfonyl chloride (27 mg) in one portion. The mixture was stirred at 60-70°C for one and half hours, cooled, and poured into ice water. The separated oil was extracted with dichloromethane. The extract was washed with water, dried, and concentrated in vacuo to give a brown oil, which was purified by preparative thin-layer chromatography on silica gel (5% solution of methanol in dichloromethane) to give 3-bromo-8-[2,6-dichloro-3-[(N,N-diethylaminomethylene)amino]benzyloxy]-2-methylimidazo[1,2-a]pyridine (65 mg) as crystals.

mp : 163-165°C

NMR ($CDCl_3$, δ) : 1.25 (3H, t, J=7Hz), 2.44 (2H, s), 3.18-3.61 (4H, m), 5.49 (2H, s), 6.68-6.85 (3H, m), 7.23 (1H, d, J=7.5Hz), 7.38 (1H, s), 7.71 (1H, d, J=7.5Hz)

Example 13

The following compounds were obtained according to a similar manner to that of Example 12.

(1) 3-Bromo-8-[2,6-dichloro-3-[(N,N-dimethylaminomethylene)amino]benzyloxy]-2-methylimidazo[1,2-a]pyridine

mp : 165-167°C

NMR (DMSO-$d_6$, δ) : 2.29 (3H, s), 2.97 (3H, s), 3.06 (3H, s), 5.40 (2H, s), 6.95-7.02 (2H, m), 7.14 (1H, d, J=7.5Hz), 7.40 (1H, d, J=7.5Hz), 7.78 (1H, s), 7.91 (1H, m)

(2) 3-Bromo-8-[2,6-dichloro-3-(1-methyl-2-pyrrolidinylideneamino)benzyloxy]-2-methylimidazo[1,2-a]pyridine

mp : 178-179°C

NMR ($CDCl_3$, δ) : 1.97 (2H, m), 2.32 (2H, t, J=7.5Hz), 2.45 (3H, s), 2.98 (3H, s), 3.40 (2H, t, J=7.5Hz), 5.47 (2H, s), 6.69-6.82 (2H, m), 6.83 (1H, d, J=7.5Hz), 7.19 (1H, d, J=7.5Hz), 7.72 (1H, d, J=7.5Hz)

Example 14

A mixture of 8-(3-amino-2,6-dichlorobenzyloxy)-3-bromo-2-methylimidazo[1,2-a]pyridine dihydrochloride (100 mg) and acetic anhydride (100 mg) in dry pyridine (2 ml) was stirred at 90°C for one hour. The cooled mixture was poured into an ice water (10 ml). The precipitated solid was collected, washed with water, and dried under reduced pressure to give 8-(3-acetylamino-2,6-dichlorobenzyloxy)-3-bromo-2-methylimidazo[1,2-a]pyridine (65 mg) as a solid.

mp : 210-212°C

NMR (DMSO-$d_6$, δ) : 2.13 (3H, s), 2.29 (3H, s), 5.44 (2H, s), 6.99-7.04 (2H, m), 7.59 (1H, d, J=7.5Hz), 7.84 (1H, d, J=7.5Hz), 7.92 (1H, m), 9.72 (1H, s)

Example 15

To a suspension of 8-(3-amino-2,6-dichlorobenzyloxy)-3-bromo-2-methylimidazo[1,2-a]pyridine dihydrochloride (200 mg), pyridine (1 ml) and N-methylpyrrolidone (3 ml) was added propionyl chloride (78 mg) and the mixture was stirred at 60°C for 1 hour. To the cooled mixture was added water, and the precipitate was collected by filtration and dissolved in chloroform. This organic solution was washed with water and brine respectively, dried with magnesium sulfate and concentrated in vacuo. The residue was collected by filtration and washed with ethyl acetate to give 3-bromo-8-(2,6-dichloro-3-propionylaminobenzyloxy)-2-methylimidazo[1,2-a]pyridine (158 mg).

mp : 205-207°C
NMR ($CDCl_3$, δ) : 1.28 (3H, t, J=7Hz), 2.44 (3H, s), 2.48 (2H, q, J=7Hz), 5.49 (2H, s), 6.70 (1H, d, J=7Hz), 6.82 (1H, t, J=7Hz), 7.37 (1H, d, J=9Hz), 7.69 (1H, br s), 7.76 (1H, d, J=7Hz), 8.42 (1H, d, J=9Hz)

Example 16

The following compounds were obtained according to similar manners to those of Examples 14 or 15.

(1) 3-Bromo-8-[2,6-dichloro-3-(4-methylbenzoylamino)benzyloxy]-2-methylimidazo[1,2-a]pyridine

mp : 222-223°C
NMR ($CDCl_3$, δ) : 2.43 (6H, s), 5.51 (2H, s), 6.71 (1H, d, J=7Hz), 6.82 (1H, t, J=7Hz), 7.31 (2H, d, J=9Hz), 7.41 (1H, d, J=9Hz), 7.70-7.85 (3H), 8.44 (1H, br s), 8.60 (1H, d, J=9Hz)

(2) 3-Bromo-8-[2,6-dichloro-3-(4-methoxybenzoylamino)benzyloxy]-2-methylimidazo[1,2-a]pyridine

mp : 211-212°C
NMR ($CDCl_3$, δ) : 2.44 (3H, s), 3.89 (3H, s), 5.51 (2H, s), 6.70 (1H, d, J=7Hz), 6.81 (1H, t, J=7Hz), 7.00 (2H, d, J=9Hz), 7.41 (1H, d, J=9Hz), 7.75 (1H, d, J=7Hz), 7.88 (2H, d, J=9Hz), 8.40 (1H, br s), 8.59 (1H, d, J=9Hz)

(3) 8-(3-Benzoylamino-2,6-dichlorobenzyloxy)-3-bromo-2-methylimidazo[1,2-a]pyridine

mp : 208-210°C
NMR ($CDCl_3$, δ) : 2.44 (3H, s), 5.52 (2H, s), 6.71 (1H, d, J=7Hz), 6.83 (1H, t, J=7Hz), 7.40-7.66 (4H), 7.75 (1H, d, J=7Hz), 7.87-7.96 (2H), 8.49 (1H, br s), 8.60 (1H, d, J=9Hz)

(4) 3-Bromo-8-[2,6-dichloro-3-(4-methoxycarbonylbenzoylamino)benzyloxy]-2-methylimidazo[1,2-a]pyridine

mp : 238-239°C
NMR (DMSO-$d_6$, δ) : 2.31 (3H, s), 3.92 (3H, s), 5.49 (2H, s), 6.97-7.08 (2H, m), 7.69 (1H, d, J=8.5Hz), 7.77 (1H, d, J=8.5Hz), 7.95 (1H, dd, J=7.5Hz and 2Hz), 8.13 (4H, s)

(5) 3-Bromo-8-[3-(4-chlorobenzoylamino)-2,6-dichlorobenzyloxy]-2-methylimidazo[1,2-a]pyridine

mp : 201-213°C
NMR ($CDCl_3$, δ) : 2.45 (3H, s), 5.52 (2H, s), 6.72 (1H, d, J=7.5Hz), 6.84 (1H, t, J=7.5Hz), 7.43 (1H, d, J=8.5Hz), 7.52 (2H, d, J=8.5Hz), 7.76 (1H, d, J=8.5Hz), 7.86 (2H, d, J=8.5Hz), 8.41 (1H, br s), 8.57 (1H, d, J=8.5Hz)

(6) 8-(3-Diacetylamino-2,4,6-trichlorobenzyloxy)-3-bromo-2-methylimidazo[1,2-a]pyridine

mp : 141-142°C
NMR ($CDCl_3$, δ) : 2.34 (6H, s), 2.45 (3H, s), 5.48 (2H, s), 6.69 (1H, d, J=8Hz), 6.84 (1H, t, J=8Hz), 7.61 (1H, s), 7.77 (1H, d, J=8Hz)

(7) 3-Bromo-8-[2-chloro-5-(N-methyl-N-trifluoroacetylamino)benzyloxy]-2-methylimidazo[1,2-a]pyridine

mp : 142-143°C
NMR ($CDCl_3$, δ) : 2.50 (3H, s), 3.31 (3H, s), 5.48 (2H, s), 6.46 (1H, d, J=7.5Hz), 6.75 (1H, d, J=7.5Hz), 7.18 (1H, dd, J=9Hz and 2Hz), 7.46-7.53 (1H, m), 7.76 (1H, d, J=7.5Hz)

(8) 3-Bromo-8-[2-chloro-5-(N-methyl-N-propionylamino)benzyloxy]-2-methylimidazo[1,2-a]pyridine

mp : 133-135°C
NMR ($CDCl_3$, δ) : 0.96 (3H, t, J=7.5Hz), 1.97 (2H, q, J=7.5Hz), 2.50 (3H, s), 3.19 (3H, s), 5.45 (2H, s), 6.50 (1H, d, J=7.5Hz), 6.76 (1H, t, J=7.5Hz), 7.11 (1H, dd, J=9Hz and 2Hz), 7.45 (1H, s), 7.48 (1H, d, J=9Hz), 7.75 (1H, d, J=7.5Hz)

(9) 3-Bromo-8-[2,6-dichloro-3-(phthalimidoacetylamino)-benzyloxy]-2-methylimidazo[1,2-a]pyridine

mp : 228-230°C
NMR ($CDCl_3$, δ) : 2.42 (1.2H, s), 2.46 (1.8H, s), 4.60 (1.8H, s), 4.71 (1.2H, s), 5.47 (1.8Hz), 5.54 (1.2H, s), 6.68 (1H, d, J=8Hz), 6.81 (1H, t, J=8Hz), 7.32 (0.6H, d, J=8Hz), 7.56 (0.4H, d, J=5Hz), 7.70-7.98 (5.6H), 8.28-8.40 (1.4H)

(10) 3-Bromo-8-[2,6-dichloro-3-(2-phthalimidopropionylamino)benzyloxy]-2-methylimidazo[1,2-a]pyridine

NMR (DMSO-$d_6$, δ) : 1.62 (3H, d, J=6Hz), 2.28 (3H, s), 5.01 (1H, q, J=6Hz), 5.43 (2H, s), 6.93-7.04 (2H), 7.60 (2H, s), 7.82-7.97 (5H), 9.92 (1H, s)

(11) 3-Bromo-8-[2,6-dichloro-3-(3-phthalimidopropionylamino)benzyloxy]-2-methylimidazo[1,2-a]pyridine

mp : 207-209°C
NMR ($CDCl_3$, δ) : 2.41 (3H, s), 2.90 (2H, t, J=6Hz), 4.12 (2H, t, J=6Hz), 5.45 (2H, s), 6.69 (1H, d, J=7Hz), 6.82 (1H, t, J=7Hz), 7.35 (1H, d, J=9Hz), 7.61-7.94 (6H), 8.31 (1H, d, J=7Hz)

(12) 3-Chloro-8-[2,6-dichloro-3-[N-methyl-N-(phthalimidoacetyl)amino]benzyloxy]-2-methylimidazo[1,2-a]pyridine

mp : 139-141°C
NMR ($CDCl_3$, δ) : 2.42 (3H, s), 3.25 (3H, s), 4.12 (2H, s), 5.53 (2H, s), 6.71 (1H, d, J=7Hz), 6.86 (1H, d, J=7Hz), 7.52 (2H, s), 7.68-7.92 (5H)

Example 17

To a solution of 3-bromo-8-(2,6-dichloro-3-methylaminobenzyloxy-2-methylimidazo[1,2-a]pyridine (70 mg) in formic acid (1 ml) was added acetic anhydride (35 mg) at ambient temperature. The mixture was stirred for half an hour at the same temperature and then concentrated in vacuo. The residue was partitioned between aqueous sodium bicarbonate solution and ethyl acetate. The organic layer was dried and evaporated under reduced pressure to give 3-bromo-8-[2,6-dichloro-3-(N-methyl-N-formylamino)benzyloxy]-2-methylimidazo[1,2-a]pyridine (29 mg).

mp : 207-209°C
NMR ($CDCl_3$, δ) : 2.45 (3H, s), 3.23 (3H, s), 5.50 (2H, s), 6.71 (1H, d, J=7.5Hz), 6.85 (1H, t, J=7.5Hz), 7.26 (1H, d, J=10Hz), 7.45 (1H, d, J=10Hz), 7.76 (1H, d, J=7.5Hz), 8.15 (1H, s)

Example 18

To a solution of 3-bromo-8-(2,6-dichloro-3-methylaminobenzyloxy)-2-methylimidazo[1,2-a]pyridine (68 mg) and pyridine (0.5 ml) in dichloromethane (2 ml) was added 4-nitrophenyl chloroformate (40 mg) at ambient temperature. After stirring for 1 hour, the mixture was partitioned between dichloromethane and water. The aqueous layer was extracted with dichloromethane twice. The organic layers were combined, washed with water twice and brine, dried over magnesium sulfate and evaporated in vacuo. The residue was crystallized from diethyl ether to give 3-bromo-8-[2,6-dichloro-3-[N-methyl-N-(4-nitrophenoxycarbonyl)amino]benzyloxy]-2-methylimidazo[1,2-a]pyridine (84 mg) as crystals.

mp : 229-230°C
NMR ($CDCl_3$-$CD_3OD$, δ) : 2.41 (3H, s), 3.32 (3H, s), 5.49 (1H, d, J=10Hz), 5.57 (1H, d, J=10Hz), 6.73 (1H, d, J=7Hz), 6.88 (1H, t, J=7Hz), 7.19-7.57 (4H), 7.78 (1H, d, J=7Hz), 8.20 (2H, d, J=10Hz)

Example 19

To a solution of 3-bromo-8-(2-chloro-5-methylaminobenzyloxy)-2-methylimidazo[1,2-a]pyridine (60 mg), pyridine (16 mg) and 4-dimethylaminopyridine (10 mg) in methylene chloride (2 ml) was added mesyl chloride (22 mg) in one portion at 5°C, and the mixture was stirred for 9 hours at ambient temperature. The precipitate was filtered off and the residue was washed with methylene chloride and diethyl ether respectively. The filtrate was concentrated in vacuo and the residue was purified by preparative thin-layer chromatography on silica gel (methanol:methylene chloride = 1:10, V/V) to give 3-bromo-8-[2-chloro-5-(N-methyl-N-methylsulfonylaminobenzyloxy]-2-methylimidazo[1,2-a]pyridine (25

mg).

mp : 158-162°C

NMR ($CDCl_3$, δ) : 2.49 (3H, s), 2.74 (3H, s), 3.26 (3H, s), 5.44 (2H, s), 6.53 (1H, d, J=7.5Hz), 6.78 (1H, t, J=7.5Hz), 7.32-7.46 (2H, m), 7.60 (1H, d, J=2Hz), 7.73 (1H, d, J=7.5Hz)

Example 20

To a mixture of 8-(3-amino-2,6-dichlorobensyloxy)-3-bromo-2-methylimidazo[1,2-a]pyridine dihydrochloride (100 mg), pyridine (0.5 ml) and N-methylpyrrolidone (1.5 ml) was added ethyl isocyanate (0.10 ml). The mixture was stirred at 60°C for 6 hours. The insoluble material was filtered off and washed with water. The filtrate and washings were combined and separated precipitate was collected by filtration. The precipitate was purified by preparative thin-layer chromatography (20% solution of methanol in dichloromethane) followed by recrystallization from n-hexane to give 3-bromo-8-[2,6-dichloro-3-(N'-ethylureido)benzyloxy]-2-methylimidazo[1,2-a]pyridine (13 mg) as crystals.

mp : 238-239°C

NMR ($CDCl_3$, δ) : 1.00 (3H, t, J=7Hz), 2.38 (3H, s), 3.16-3.31 (2H), 5.32 (2H, s), 6.67 (1H, d, J=7Hz), 6.86 (1H, t, J=7Hz), 7.19-7.32 (2H), 7.71 (1H, d, J=7Hz), 7.90 (1H, br s), 8.34 (1H, d, J=9Hz)

Example 21

A mixture of 3-bromo-8-[2,6-dichloro-3-[N-methyl-N-(4-nitrophenyloxycarbonyl)amino]benzyloxy]-2-methylimidazo[1,2-a]pyridine (63 mg) and 30% solution of methylamine in methanol (2 ml) was heated under reflux for 3 hours. After addition of 30% solution of methylamine in methanol (1 ml), the mixture was heated under reflux for additional 1 hour. The mixture was evaporated in vacuo and the residue was extracted with ethyl acetate. The extract was evaporated in vacuo and the residue was purified by preparative thin-layer chromatography (5% solution of methanol in dichloromethane) followed by crystallization from diethyl ether to give 3-bromo-8-[2,6-dichloro-3-(N-methyl-N'-methylureido)benzyloxy]-2-methylimidazo[1,2-a]pyridine (28 mg) as crystals.

mp : 192-193°C

NMR ($CDCl_3$, δ) : 2.42 (3H, s), 2.79 (3H, d, J=5Hz), 3.20 (3H, s), 4.20 (1H, br d, J=5Hz), 5.49 (2H, s), 6.71 (1H, d, J=7Hz), 6.85 (1H, t, J=7Hz), 7.32 (1H, d, J=9Hz), 7.42 (1H, d, J=9Hz), 7.78 (1H, d, J=7Hz)

Example 22

The following compounds were obtained according to similar manners to those of Examples 20 or 21.

(1) 3-Bromo-8-[2,6-dichloro-3-(N'-phenylureido)benzyloxy]-2-methylimidazo[1,2-a]pyridine

mp : >250°C

NMR (DMSO-$d_6$, δ) : 2.30 (3H, s), 5.45 (2H, s), 6.91-7.10 (3H), 7.31 (2H, t, J=9Hz), 7.41-7.62 (3H), 7.92 (1H, m), 8.34 (1H, m), 8.52 (1H, br s), 9.50 (1H, br s)

(2) 3-Bromo-8-[2,6-dichloro-3-(N-methyl-N'-trichloroacetylureido)benzyloxy]-2-methylimidazo[1,2-a]pyridine

mp : 160-164°C (dec.)

NMR ($CDCl_3$, δ) : 2.43 (3H, s), 3.33 (3H, s), 5.54 (2H, s), 6.72 (1H, d, J=7.5Hz), 6.85 (1H, t, J=7.5Hz), 7.41 (1H, d, J=10Hz), 7.52 (1H, d, J=10Hz), 7.81 (1H, d, J=7.5Hz)

Example 23

To a solution of 8-(3-acetylamino-2,6-dichlorobenzyloxy)-3-bromo-2-methylimidazo[1,2-a]pyridine (222 mg) in N,N-dimethylformamide (2 ml) was added sodium hydride (24 mg, 60% oil dispersion) in one portion at ambient temperature. The mixture was stirred for half an hour at the same temperature and iodomethane (142 mg) was added thereto. After stirring for half an hour, the mixture was poured into water. The separated oil was extracted with ethyl acetate. The extracts were washed with water, dried, and evaporated under reduced pressure. The residue was purified by flash chromatography on silica gel to give 3-bromo-8-[2,6-dichloro-3-(N-acetyl-N-methylamino)benzyloxy]-2-methylimidazo[1,2-a]pyridine (135 mg) as a solid.

mp : 201-204°C
NMR ($CDCl_3$, δ) : 1.88 (3H, s), 2.46 (3H, s), 3.19 (3H, s), 5.52 (2H, s), 6.72 (1H, d, J=7.5Hz), 6.87 (1H, t, J=7.5Hz), 7.31 (1H, d, J=8Hz), 7.48 (1H, d, J=8Hz), 7.80 (1H, d, J=7.5Hz)

Example 24

The following compounds were obtained according to a similar manner to that of Example 23.

(1) 3-Bromo-8-[2,6-dichloro-3-(N-ethyl-N-acetylamino)benzyloxy]-2-methylimidazo[1,2-a]pyridine

mp : 161°C
NMR ($CDCl_3$, δ) : 1.15 (3H, t, J=7.5Hz), 1.83 (3H, s), 2.45 (3H, s), 3.30 (1H, m), 4.12 (1H, m), 5.51 (2H, s), 6.71 (1H, d, J=7.5Hz), 6.85 (1H, t, J=7.5Hz), 7.25 (1H, d, J=7.5Hz), 7.46 (1H, d, J=7.5Hz), 7.78 (1H, d, J=7.5Hz)

(2) 3-Bromo-8-[2,6-dichloro-3-(N-propyl-N-acetylamino)benzyloxy]-2-methylimidazo[1,2-a]pyridine

mp : 142-144°C
NMR ($CDCl_3$, δ) : 0.91 (3H, t, J=7Hz), 1.41-1.68 (2H), 1.81 (3H, s), 2.45 (3H, s), 3.14 (1H, dt, J=9Hz and 7Hz), 4.03 (1H, dt, J=9Hz and 7Hz), 5.51 (2H, s), 6.70 (1H, d, J=7Hz), 6.85 (1H, t, J=7Hz), 7.24 (1H, d, J=9Hz), 7.44 (1H, d, J=9Hz), 7.79 (1H, d, J=7Hz)

(3) 3-Bromo-8-[2,6-dichloro-3-[N-methyl-N-(4-methoxybenzoyl)amino]benzyloxy]-2-methylimidazo[1,2-a]pyridine

mp : 168-169°C
NMR ($CDCl_3$, δ) : 2.45 (3H, s), 3.35 (3H, s), 3.78 (3H, s), 5.45 (2H s), 6.59-6.86 (4H), 7.03-7.40 (4H), 7.76 (1H, d, J=7Hz)

(4) 3-Bromo-8-[2,6-dichloro-3-(N-methyl-N-propionylamino)benzyloxy]-2-methylimidazo[1,2-a]pyridine

mp : 170-171°C
NMR ($CDCl_3$, δ) : 1.08 (3H, t, J=7Hz), 2.01 (2H, q, J=7Hz), 2.43 (3H, s), 3.20 (3H, s), 5.50 (2H, s), 6.70 (1H, d, J=7Hz), 6.84 (1H, t, J=7Hz), 7.28 (1H, d, J=9Hz), 7.44 (1H, d, J=9Hz), 7.78 (1H, d, J=7Hz)

(5) 3-Bromo-8-[2,4,6-trichloro-3-(N-methyl-N-acetylamino)benzyloxy]-2-methylimidazo[1,2-a]pyridine

NMR ($CDCl_3$, δ) : 1.84 (2.7H, s), 2.30 (0.3H, s), 2.44 (3H, s), 3.15 (2.7H, s), 3.28 (0.3H, s), 5.41 (0.2H, s), 5.47 (1.8H, s), 6.70 (1H, d, J=8Hz), 6.84 (1H, t, J=8Hz), 7.53 (0.1H, s), 7.59 (0.9H, s), 7.77 (1H, d, J=8Hz)

(6) 3-Bromo-8-[2,6-dichloro-3-(N-methyl-N-phthalimidoacetylamino)benzyloxy]-2-methylimidazo[1,2-a]pyridine

mp : 229-230°C

(7) 8-[3-[N-(Acetylsarcosyl)-N-methylamino]-2,6-dichlorobenzyloxy]-3-bromo-2-methylimidazo[1,2-a]pyridine

NMR ($CDCl_3$, δ) : 1.98 (0.5H, s), 2.11 (2.5H, s), 2.43 (3H, s), 2.90 (0.5H, s), 3.07 (2.5H, s), 3.21 (2.5H, s), 3.24 (0.5H, s), 3.30 (1H, d, J=16Hz), 4.27 (1H, d, J=16Hz), 5.50 (2H, s), 6.70 (1H, d, J=8Hz), 6.84 (1H, t, J=8Hz), 7.33 (0.17H, d, J=8Hz), 7.48 (1.6H, s), 7.51 (0.17H, d, J=8Hz), 7.78 (1H, d, J=8Hz)

(8) 3-Bromo-8-[2,6-dichloro-3-(N-ethyl-N-(phthalimidoacetylamino)benzyloxy]-2-methylimidazo[1,2-a]pyridine

mp : 185-188°C
NMR (DMSO-$d_6$, δ) : 1.08 (3H, t, J=7Hz), 2.29 (3H, s), 3.88 (1H, d, J=16Hz), 4.11 (1H, d, J=16Hz), 5.50 (2H, s), 6.95-7.09 (3H), 7.80-8.00 (6H)

(9) 3-Bromo-8-[2,6-dichloro-3-[N-methyl-N-(2-phthalimidopropionyl)amino]benzyloxy]-2-methylimidazo[1,2-a]py-

ridine

NMR ($CDCl_3$, δ) : 1.51-1.65 (6H), 2.41 (0.9H, s), 2.42 (2.1H, s), 3.22 (3H, s), 4.78 (0.3H, d, J=10Hz), 4.83 (1H, q, J=6Hz), 5.17 (0.3H, d, J=10Hz), 5.51 (1.4H, s), 6.46 (0.3H, d, J=8Hz), 6.70-6.90 (1.7H), 7.00 (1H, d, J=8Hz), 7.08 (1H, d, J=8Hz), 7.31 (0.3H, d, J=8Hz), 7.48 (0.3H, d, J=8Hz), 7.67-7.82 (5H)

(10) 3-Bromo-8-[2,6-dichloro-3-[N-methyl-N-(3-phthalimidopropionyl)amino]benzyloxy]-2-methylimidazo[1,2-a]pyridine

mp : 213-215°C
NMR ($CDCl_3$, δ) : 2.28-2.58 (2H), 2.41 (3H, s), 3.20 (3H, s), 3.82-4.12 (2H), 5.41 (1H, d, J=9Hz), 5.50 (1H, d, J=9Hz), 6.69 (2H, d, J=7Hz), 6.84 (1H, t, J=7Hz), 7.31 (1H, d, J=9Hz), 7.43 (1H, d, J=9Hz), 7.61-7.90 (5H)

Example 25

To a solution of 3-bromo-8-(2,6-dichloro-3-acetylaminobenzyloxy)-2-methylimidazo[1,2-a]pyridine (222 mg) in N,N-dimethylformamide (2 ml) was added sodium hydride (24 mg, 60% oil dispersion) at ambient temperature. The mixture was stirred for half an hour at the same temperature and then ethyl bromoacetate (100 mg) was added thereto in one portion. The mixture was stirred for 2 hours at the same temperature and poured into water. The separated oil was extracted with dichloromethane. The extract was washed with water, dried, and concentrated in vacuo. The residue was purified by flash chromatography on silica gel to give an oil, which was dissolved in a mixture of ethanol (10 ml) and 1N sodium hydroxide solution (2 ml). The solution was refluxed for one and half hour. The organic solvent was removed under reduced pressure. The aqueous layer was adjusted to pH 4 with diluted hydrochloric acid to give 3-bromo-8-[2,6-dichloro-3-(N-carboxymethyl-N-acetylamino)benzyloxy]-2-methylimidazo[1,2-a]pyridine (150 mg) as a white solid.

mp : 225-227°C (dec.)
NMR (DMSO-$d_6$, δ) : 1.79 (3H, s), 2.31 (3H, s), 3.78 (1H, d, J=17Hz), 4.64 (1H, d, J=17Hz), 5.45 (2H, s), 6.96-7.06 (2H, m), 7.76 (2H, s), 7.95 (1H, m)

Example 26

To a solution of 3-bromo-8-[2-chloro-6-(N-acetyl-N-methylamino)benzyloxy]-2-methylimidazo[1,2-a]pyridine (103 mg) in tetrahydrofuran (2 ml) was added lithium aluminum hydride (16 mg) in several portions at 5°C. After the addition, the mixture was stirred for 2 hours at 5°C and then quenched with aqueous saturated ammonium chloride solution. The separated organic layer was washed with aqueous saturated ammonium chloride solution, dried, and concentrated in vacuo. The residue was purified by preparative thin-layer chromatography (ethyl acetate:n-hexane = 1:2, V/V) to give 3-bromo-8-[2-chloro-6-(N-ethyl-N-methylamino)benzyloxy]-2-methylimidazo[1,2-a]pyridine (41 mg).

mp : 124-126°C
NMR ($CDCl_3$, δ) : 1.02 (3H, t, J=7.5Hz), 2.44 (3H, s), 2.71 (3H, s), 3.03 (2H, q, J=7.5Hz), 5.41 (2H, s), 6.73 (1H, d, J=7.5Hz), 6.81 (1H, t, J=7.5Hz), 7.01-7.28 (3H, m), 7.71 (1H, dd, J=7Hz and 1.5Hz)

Example 27

A mixture of 3-bromo-8-[3-(N-acetoxyacetyl-N-methylamino)-2,6-dichlorobenzyloxy]-2-methylimidazo[1,2-a]pyridine (1.42 g), potassium carbonate (761 mg), methanol (7 ml) and tetrahydrofuran (7 ml) was stirred at ambient temperature for 1 hour. The mixture was partitioned between dichloromethane and water. The aqueous layer was extracted with dichloromethane twice. The combined organic layers were washed with brine, dried over magnesium sulfate and evaporated in vacuo. The residue was crystallized from ethanol to give 3-bromo-8-[2,6-dichloro-3-(N-glycoloyl-N-methylamino)benzyloxy]-2-methylimidazo[1,2-a]pyridine (1.16 g) as crystals.

mp : 217-218°C
NMR ($CDCl_3$, δ) : 2.45 (3H, s), 3.19-3.32 (4H), 3.69, 3.82 (each 1H, d, J=15Hz), 5.50 (2H, s), 6.70 (1H, d, J=7Hz), 6.83 (1H, t, J=7Hz), 7.29 (1H, d, J=9Hz), 7.49 (1H, d, J=9Hz), 7.78 (1H, d, J=7Hz)

Example 28

3-Bromo-8-[2,6-dichloro-3-[N-(glycoloylglycyl)-N-methylamino]benzyloxy]-2-methylimidazo[1,2-a]pyridine was obtained according to a similar manner to that of Example 27.

mp : 163-165°C
NMR ($CDCl_3$, δ) : 2.40 (3H, s), 3.21 (3H, s), 3.29 (1H, dd, J=17Hz and 4Hz), 3.94 (2H, s), 4.32 (1H, dd, J=17Hz and 9Hz), 5.31 (1H, d, J=10Hz), 5.59 (1H, d, J=10Hz), 6.30 (1H, br s), 6.73 (1H, d, J=7Hz), 6.90 (1H, t, J=7Hz), 7.10 (1H, br s), 7.32 (1H, d, J=9Hz), 7.49 (1H, d, J=9Hz), 7.29 (1H, d, J=7Hz)

Example 29

To a suspension of sodium hydride (60% in oil, 9 mg) in N,N-dimethylformamide (1 ml) was added 3-bromo-8-[2,6-dichloro-3-(N-glycoloyl-N-methylamino)benzyloxy]-2-methylimidaso[1,2-a]pyridine (100 mg). After stirring for 10 minutes, methyl iodide (36 mg) was added thereto and the mixture was stirred at ambient temperature for 1 hour. To this mixture were added methyl iodide (36 mg) and N,N-dimethylformamide (1 ml). The mixture was stirred at 60°C for additional 2 hours. The reaction mixture was poured into water and extracted with ethyl acetate 3 times. The combined organic layers were washed with water 4 times and brine, dried over magnesium sulfate and evaporated in vacuo. The residue was purified by preparative thin-layer chromatography (dichloromethane: methanol = 20:1, V/V) followed by crystallization from ethanol-diethyl ether to give 3-bromo-8-[3-(N-methoxyacetyl-N-methylamino)-2,6-dichlorobenzyloxy]-2-methylimidazo[1,2-a]pyridine (20 mg) as crystals.

mp : 145-146°C
NMR ($CDCl_3$, δ) : 2.43 (3H, s), 3.21 (3H, s), 3.35 (3H, s), 3.68, 3.32 (each 1H, d, J=15Hz), 5.50 (2H, s), 6.70 (1H, d, J=7Hz), 6.85 (1H, t, J=7Hz), 7.29, 7.46 (each 1H, d, J=9Hz), 7.78 (1H, d, J=7Hz)

Example 30

To a solution of 3-bromo-8-[2,6-dichloro-3-[N-(glycoloylglycyl)-N-methylamino]benzyloxy]-2-methylimidazo[1,2-a]pyridine (200 mg) and triethylamine (76 mg) in methylene chloride (2 ml) was dropwise added mesyl chloride (52 mg) under ice-cooling, and the mixture was stirred for 1 hour at the same temperature. The mixture was washed with water twice and brine, dried over magnesium sulfate and concentrated in vacuo to give 3-bromo-8-[2,6-dichloro-3-[N-(mesyloxyacetylglycyl)-N-methylamino]benzyloxy]-2-methylimidazo[1,2-a]pyridine (221 mg).

NMR ($CDCl_3$, δ) : 2.48 (3H, s), 3.19 (3H, s), 3.28 (3H, s), 3.61 (1H, dd, J=17Hz and 5Hz), 4.86 (1H, dd, J=17Hz and 5Hz), 4.18 (2H, s), 5.50 (2H, s), 6.78 (1H, d, J=7Hz), 6.91 (1H, t, J=7Hz), 7.25 (1H, br s), 7.32 (1H, d, J=9Hz), 7.51 (1H, d, J=9Hz), 7.80 (1H, d, J=7Hz)

Example 31

To a solution of 3-bromo-8-[2,6-dichloro-3-[N-(N-mesyloxyacetylglycyl)-N-methylamino]benzyloxy]-2- methylimidazo[1,2-a]pyridine (100 mg) in methanol (2 ml) was added sodium methoxide (317 mg), and the mixture was stirred for 1 hour. Sodium methoxide (200 mg) was added thereto, and the mixture was stirred for 3 hours. To the reaction mixture was added water, and the mixture was extracted with methylene chloride 3 times. The extracts were combined, washed with brine, dried over magnesium sulfate and concentrated in vacuo. The residue was purified by preparative thin-layer chromatography (methylene chloride:methanol = 10:1, V/V) to give 3-bromo-8-[2,6-dichloro-3-[N-(methoxyacetylglycyl)-N-methylamino]benzyloxy]-2-methylimidazo[1,2-a]pyridine (60 mg).

NMR ($CDCl_3$, δ) : 2.44 (3H, s), 3.27 (3H, s), 3.59 (1H, dd, J=17Hz and 4Hz), 3.78-3.95 (3H), 5.48 (1H, d, J=9Hz), 5.52 (1H, d, J=9Hz), 6.71 (1H, d, J=7Hz), 6.87 (1H, t, J=7Hz), 7.31 (1H, d, J=9Hz), 7.39 (1H, br s), 7.49 (1H, d, J=9Hz), 7.78 (1H, d, J=7Hz)

Example 32

To a solution of 3-bromo-8-[2,6-dichloro-3-(N-glycoloyl-N-methylamino)benzyloxy]-2-methylimidazo[1,2-a]pyridine (700 mg) and triethylamine (269 mg) in methylene chloride (14 ml) was added mesyl chloride (224 mg) under ice-cooling, and the mixture was stirred for 1 hour at ambient temperature. The reaction mixture was washed with water twice and brine, dried over magnesium sulfate and concentrated in vacuo to give the residue of 3-bromo-8-[2,6-dichloro-3-(N-mesyloxyacetyl-N-methylamino)benzyloxy]-2-methylimidazo[1,2-a]pyridine. The residue was dissolved in N,N-dimethylformamide (7 ml) and potassium phthalimide (362 mg) was added thereto at ambient temperature under $N_2$ atmosphere. The mixture was stirred for 1 day, and water was added thereto. The precipitate was collected by filtration and dried to give 3-bromo-8-[2,6-dichloro-3-(N-methyl-N-phthalimidoacetylamino)benzyloxy]-2-methylimidazo[1,2-a]pyridine (629 mg).

mp : 229-230°C
NMR ($CDCl_3$, δ) : 2.43 (3H, s), 3.26 (3H, s), 4.12 (2H, s), 5.53 (2H, s), 6.72 (1H, d, J=7Hz), 6.87 (1H, t, J=7Hz), 7.52 (2H, s), 7.68-7.92 (5H, m)

Example 33

3-Bromo-8-[2,6-dichloro-3-[N-methyl-N-(phthalimidoacetylglycyl)amino]benzyloxy]-2-methylimidazo[1,2-a]pyridine was obtained according to a similar manner to that of Example 32.

mp : 148-150°C
NMR ($CDCl_3$, δ) : 2.42 (3H, s), 3.22 (3H, s), 3.53 (1H, dd, J=17Hz and 4Hz), 3.80 (1H, dd, J=17Hz and 5Hz), 4.39 (2H, s), 5.48 (2H, s), 6.70 (1H, d, J=7Hz), 6.78 (1H), 6.84 (1H, t, J=7Hz), 7.29 (1H, d, J=9Hz), 7.45 (1H, d, J=9Hz), 7.62-7.92 (5H)

Example 34

A mixture of 3-bromo-8-[2,6-dichloro-3-(N-methyl-N-phthalimidoacetylamino)benzyloxy]-2-methylimidazo[1,2-a]pyridine (450 mg) and hydrazine hydrate (41 mg) in methanol (5 ml) were refluxed for 30 minutes, and hydrazine hydrate (41 mg) was added thereto, and further, the mixture was refluxed for 1.5 hours. The resulting precipitates were filtered off and the filtrate was concentrated in vacuo. The residue was dissolved in chloroform and the solution was washed with water twice and brine, dried over magnesium sulfate and concentrated in vacuo. The residue was crystallized with diethyl ether to give 8-[3-(N-glycyl-N-methylamino)-2,6-dichlorobenzyloxy]-3-bromo-2-methylimidazo[1,2-a]pyridine (338 mg).

mp : 175-178°C
NMR ($CDCl_3$, δ) : 2.43 (3H, s), 2.92-3.20 (2H), 3.23 (3H, s), 5.49 (2H, s), 6.70 (1H, d, J=7Hz), 6.83 (1H, t, J=7Hz), 7.29, 7.47 (each 1H, d, J=9Hz), 7.79 (1H, d, J=7Hz)

Example 35

The following compounds were obtained according to a similar manner to that of Example 34.

(1) 8-[3-[N-(Glycylglycyl)-N-methylamino]-2,6-dichlorobenzyloxy]-3-bromo-2-methylimidazo[1,2-a]pyridine

NMR ($CDCl_3$, δ) : 2.41 (3H, s), 3.22 (3H, s), 3.41 (3H, s), 3.60 (1H, dd, J=17Hz and 4Hz), 3.84 (1H, dd, J=17Hz and 4Hz), 5.49 (2H, s), 6.71 (1H, d, J=7Hz), 6.87 (1H, t, J=7Hz), 7.34 (1H, d, J=9Hz)

(2) 8-[3-(N-Glycyl-N-ethylamino)-2,6-dichlorobenzyloxy]-3-bromo-2-methylimidazo[1,2-a]pyridine

mp : 169-171°C
NMR ($CDCl_3$, δ) : 1.16 (3H, t, J=7Hz), 2.45 (3H, s), 2.92-3.40 (3H), 4.18 (1H), 5.50 (2H, s), 6.70 (1H, d, J=7Hz), 6.83 (1H, t, J=7Hz), 7.22 (1H, d, J=9Hz), 7.48 (1H, d, J=9Hz), 7.78 (1H, d, J=7Hz)

(3) 3-Bromo-8-[2,6-dichloro-3-(N-DL-alanyl-N-methylamino)benzyloxy]-2-methylimidazo[1,2-a]pyridine

NMR ($CDCl_3$, δ) : 1.11 (1.8H, d, J=6Hz), 1.15 (1.2H, d, J=6Hz), 2.45 (3H, s), 3.20 (0.4H, q, J=6Hz), 3.22 (3H, s), 3.36 (0.6H, q, J=6Hz), 5.43-5.56 (2H), 6.70 (1H, d, J=8Hz), 6.84 (1H, t, J=8Hz), 7.30 (0.6Hz, d, J=8Hz), 7.33 (0.4H, d, J=8Hz), 7.45 (0.4H, d, J=8Hz), 7.48 (0.6H, d, J=8Hz), 7.77 (1H, d, J=8Hz)

(4) 8-[3-(N-β-Alanyl-N-methylamino)-2,6-dichlorobenzyloxy]-3-bromo-2-methylimidazo[1,2-a]pyridine

mp : 163-165°C

NMR ($CDCl_3$, δ) : 2.10-2.25 (2H), 2.41 (3H, s), 2.85-2.99 (2H), 3.20 (3H, s), 5.50 (2H, s), 6.72 (1H, d, J=7Hz), 6.88 (1H, t, J=7Hz), 7.32 (1H, d, J=9Hz), 7.49 (1H, d, J=9Hz), 7.78 (1H, d, J=7Hz)

(5) 3-Chloro-8-[2,6-dichloro-3-(N-glycyl-N-methylamino)benzyloxy]-2-methylimidazo[1,2-a]pyridine

mp : 184-186°C

NMR ($CDCl_3$, δ) : 2.43 (3H, s), 3.01 (1H, d, J=17Hz), 3.12 (1H, d, J=17Hz), 3.22 (3H, s), 5.50 (2H, s), 6.70 (1H, d, J=7Hz) 6.86 (1H, t, J=7Hz), 7.29 (1H, d, J=9Hz), 7.48 (1H, d, J=9Hz), 7.72 (1H, d, J=7Hz)

Example 36

To a solution of 3-bromo-8-[2,6-dichloro-3-(N-glycyl-N-methylamino)benzyloxy]-2-methylimidazo[1,2-a]pyridine (100 mg) in methylene chloride (2 ml) were added pyridine (17 mg), 4-dimethylaminopyridine (10 mg) and acetic anhydride (32 mg), and the mixture was stirred for 1.5 hours. The mixture was washed with water twice and brine, dried over magnesium sulfate and concentrated in vacuo. The residue was purified by silica gel column chromatography (methylene chloride:methanol = 5:1, V/V) to give 8-[3-[N-(acetylglycyl)-N-methylamino]-2,6-dichlorobenzyloxy]-3-bromo-2-methylimidazo[1,2-a]pyridine (74 mg).

mp : 187-189°C

NMR ($CDCl_3$, δ) : 2.01 (3H, s), 2.43 (3H, s), 3.24 (3H, s), 3.51, 3.78 (each 1H, dd, J=17Hz and 4Hz), 5.49 (2H, s), 6.45 (1H, br s), 6.71 (1H, d, J=7Hz), 6.87 (1H, t, J=7Hz), 7.30, 7.49 (each 1H, d, J=9Hz), 7.77 (1H, d, J=7Hz)

Example 37

To a mixture of 3-bromo-8-[2,6-dichloro-3-(N-glycyl-N-methylamino)benzyloxy]-2-methylimidazo[1,2-a]pyridine (100 mg), pyridine (25 mg) and methylene chloride (2 ml) was added propionyl chloride (0.02 ml) at ambient temperature and the mixture was stirred at the same temperature for 30 minutes. The mixture was partitioned between methylene chloride and water, and the organic layer was washed with water, dried over magnesium sulfate and concentrated in vacuo to give 3-bromo-8-[2,6-dichloro-3-[N-methyl-N-(propionylglycyl)amino]benzyloxy]-2-methylimidazo[1,2-a]pyridine (111 mg).

NMR ($CDCl_3$, δ) : 1.25 (3H, t, J=6Hz), 2.27 (2H, q, J=6Hz), 2.43 (2.8H, s), 3.30 (0.2H, s), 3.52 (1H, dd, J=17Hz and 4Hz), 3.80 (1H, dd, J=17Hz and 4Hz), 5.48 (2H, s), 6.43 (1H, t like), 6.73 (1H, d, J=7Hz), 6.87 (1H, t, J=7Hz), 7.30 (1H, d, J=8Hz), 7.47 (1H, d, J=8Hz), 7.76 (1H, d, J=7Hz)

Example 38

Pivaloyl chloride (0.04 ml) was added dropwise to a mixture of tert-butoxycarbonyl-L-proline (77 mg), N-methylmorpholine (0.04 ml) and dichloromethane (3 ml) under a dry ice-tetrachloromethane bath cooling. This mixture was stirred for 5 minutes under an ice-water bath cooling and cooled under a dry ice-tetrachloromethane bath cooling. To the mixture was added a solution of 8-[3-(N-glycyl-N-methylamino)-2,6-dichlorobenzyloxy]-3-bromo-2-methylimidazo[1,2-a]pyridine (139 mg) in dichloromethane (4 ml). The mixture was stirred for 30 minutes at ambient temperature and washed with saturated aqueous solution of sodium hydrogen carbonate and water. The organic layer was dried over magnesium sulfate and evaporated in vacuo. The residue was purified by preparative thin-layer chromatography (dichloromethane:methanol = 19:1, V/V) to give 3-bromo-8-[3-[N-(tert-butoxycarbonyl-L-prolylglycyl)-N-methylamino]-2,6-dichlorobenzyloxy]-2-methylimidazo[1,2-a]pyridine (72 mg) (Compound A), and 3-bromo-8-[2,6-dichloro-3-(N-methyl-N-pivaloylglycylamino)benzyloxy]-2-methylimidazo[1,2-a]pyridine (93 mg) (Compound B).

Compound A :

NMR ($CDCl_3$, δ) : 1.45 (9H, br s), 1.78-2.22 (4H), 2.43 (3H, s), 3.23 (3H, s), 3.32-3.90 (4H), 4.25 (1H, m), 5.48 (2H, s), 6.72 (1H, d, J=8Hz), 6.86 (1H, t, J=8Hz), 7.31 (1H, d, J=9Hz), 7.47 (1H, d, J=9Hz), 7.77 (1H, d, J=8Hz)

Compound B :

NMR ($CDCl_3$, δ) : 1.20 (9H, s), 2.44 (3H, s), 3.26 (3H, s), 3.49 (1H, dd, J=16Hz and 4Hz), 3.77 (1H, dd, J=16Hz and 4Hz), 5.48 (2H, s), 6.64 (1H, t, like), 6.72 (1H, d, J=8Hz), 6.85 (1H, t, J=8Hz), 7.31 (1H, d, J=9Hz), 7.47 (1H, d, J=9Hz), 7.77 (1H, d, J=8Hz)

Example 39

A mixture of 3-bromo-8-[2,6-dichloro-3-(N-glycyl-N-methylamino)benzyloxy]-2-methylimidazo[1,2-a]pyridine (150 mg), 4-pentenoic acid (33 mg), N-ethyl-N'-(3-dimethylaminopropyl)carbodiimide hydrochloride (77 mg), 1-hydroxybenzotriazole (64 mg) and N,N-dimethylformamide (1.5 ml) was stirred for 2 hours at ambient temperature. Water was added thereto, and the mixture was extracted with methylene chloride three times. The organic layers were combined, washed with water four times and brine, dried over magnesium sulfate and concentrated in vacuo. The residue was purified by silica gel column chromatography (methylene chloride:methanol = 30:1, V/V) to give 3-bromo-8-[2,6-dichloro-3-[N-(4-pentenoylglycyl)-N-methylamino]benzyloxy]-2-methylimidazo[1,2-a]pyridine (172 mg).

NMR ($CDCl_3$, δ) : 2.24-2.42 (4H), 2.44 (3H, s), 3.26 (3H, s), 3.52 (1H, dd, J=17Hz and 4Hz), 3.80 (1H, dd, J=17 and 4Hz), 4.96-5.16 (2H), 5.49 (2H, s), 5.70-5.92 (1H, m), 6.46 (1H, br s), 6.72 (1H, d, J=7Hz), 6.88 (1H, t, J=7Hz), 7.31 (1H, d, J=9Hz), 7.49 (1H, d, J=9Hz), 7.78 (1H, d, J=7Hz)

Example 40

The following compounds were obtained according to similar manners to those of Examples 36 to 39.

(1) 3-Bromo-8-[3-[N-(butyrylglycyl)-N-methylamino]-2,6-dichlorobenzyloxy]-2-methylimidazo[1,2-a]pyridine

NMR ($CDCl_3$, δ) : 0.94 (3H, t, J=7Hz), 1.65 (2H, m), 2.20 (2H, t, J=7Hz), 2.44 (3H, s), 3.26 (3H, s), 3.52 (1H, dd, J=16Hz and 4Hz), 3.80 (1H, dd, J=16Hz and 4Hz), 5.49 (2H, s), 6.41 (1H, t like), 6.72 (1H, d, J=8Hz), 6.86 (1H, t, J=8Hz), 7.31 (1H, d, J=9Hz), 7.48 (1H, d, J=9Hz), 7.77 (1H, d, J=8Hz)

(2) 3-Bromo-8-[3-[N-(isobutyrylglycyl)-N-methylamino]-2,6-dichlorobenzyloxy]-2-methylimidazo[1,2-a]pyridine

NMR ($CDCl_3$, δ) : 1.14 (6H, d, J=6Hz), 2.42 (1H, m), 2.44 (3H, s), 3.25 (3H, s), 3.50 (1H, dd, J=16Hz and 4Hz), 3.79 (1H, dd, J=16Hz and 5Hz), 5.48 (2H, s), 6.45 (1H, t like), 6.72 (1H, d, J=8Hz), 6.86 (1H, t, J=8Hz), 7.31 (1H, d, J=9Hz), 7.48 (1H, d, J=9Hz), 7.77 (1H, d, J=8Hz)

(3) 3-Bromo-8-[2,6-dichloro-3-[N-(cyclopropylcarbonylglycyl)-N-methylamino]benzyloxy]-2-methylimidazo[1,2-a]pyridine

NMR ($CDCl_3$, δ) : 0.70-1.00 (4H), 1.47 (1H, m), 2.44 (3H, s), 3.25 (3H, s), 3.54 (1H, dd, J=16Hz and 4Hz), 3.81 (1H, dd, J=16Hz and 4Hz), 5.48 (2H, s), 6.58 (1H, t like), 6.7 (1H, d, J=8Hz), 6.86 (1H, t, J=8Hz), 7.30 (1H, d, J=9Hz), 7.46 (1H, d, J=9Hz), 7.77 (1H, d, J=8Hz)

(4) 3-Bromo-8-[2,6-dichloro-3-[N-(trifluoroacetylglycyl)-N-methylamino]benzyloxy]-2-methylimidazo[1,2-a]pyridine

NMR ($CDCl_3$, δ) : 2.42 (3H, s), 3.29 (3H, s), 3.61 (1H, dd, J=18Hz and 4Hz), 3.83 (1H, dd, J=18Hz and 4Hz), 5.48 (1H, d, J=7Hz), 5.53 (1H, d, J=7Hz), 6.71 (1H, d, J=7Hz), 6.88 (1H, t, J=7Hz), 7.31 (1H, d, J=9Hz), 7.41 (1H, br s), 7.50 (1H, d, J=9Hz), 7.78 (1H, d, J=7Hz)

(5) 8-[3-[N-(Benzoylglycyl)-N-methylamino]-2,6-dichlorobenzyloxy]-3-bromo-2-methylimidazo[1,2-a]pyridine

mp : 126°C (dec.)
NMR ($CDCl_3$, δ) : 2.44 (3H, s), 3.29 (3H, s), 3.73 (1H, dd, J=18Hz and 4Hz), 3.99 (1H, dd, J=18Hz and 4Hz), 5.48 (1H, d, J=7Hz), 5.54 (1H, d, J=7Hz), 6.73 (1H, d, J=7Hz), 6.88 (1H, t, J=7Hz), 7.18 (1H, br s), 7.31-7.59 (5H), 7.72-7.88 (3H)

(6) 3-Bromo-8-[2,6-dichloro-3-[N-(cyclohexylcarbonylglycyl)-N-methylamino]benzyloxy]-2-methylimidazo[1,2-a]pyridine

NMR ($CDCl_3$, δ) : 1.14-1.94 (10H), 2.14 (1H, m), 2.44 (3H, s), 3.26 (3H, s), 3.50 (1H, dd, J=18Hz and 4Hz), 3.78 (1H, dd, J=18Hz and 5Hz), 5.48 (2H, s), 6.43 (1H, t like), 6.72 (1H, d, J=8Hz), 6.86 (1H, t, J=8Hz), 7.32 (1H, d, J=8Hz), 7.48 (1H, d, J=8Hz), 7.77 (1H, d, J=8Hz)

(7) 3-Bromo-8-[2,6-dichloro-3-[N-(phenylacetylglycyl)-N-methylamino]benzyloxy]-2-methylimidazo[1,2-a]pyridine

NMR ($CDCl_3$, δ) : 2.43 (3H, s), 3.21 (3H, s), 3.49 (1H, dd, J=17Hz and 4Hz), 3.59 (2H, s), 3.75 (1H, dd, J=17Hz and 5Hz), 5.48 (2H, s), 6.40 (1H, t like), 6.72 (1H, d, J=8Hz), 6.86 (1H, t, J=8Hz), 7.21-7.43 (6H), 7.46 (1H, d, J=8Hz), 7.77 (1H, d, J=8Hz)

(8) 3-Bromo-8-[3-[N-(tert-butoxycarbonyl-D-prolylglycyl)-N-methylamino]-2,6-dichlorobenzyloxy]-2-methylimidazo[1,2-a]pyridine

NMR ($CDCl_3$, δ) : 1.45 (9H, br s), 1.78-2.22 (4H), 2.43 (3H, s), 3.23 (3H, s), 3.32-3.90 (4H), 4.25 (1H, m), 5.48 (2H, s), 6.72 (1H, d, J=8Hz), 6.86 (1H, t, J=8Hz), 7.31 (1H, d, J=9Hz), 7.47 (1H, d, J=9Hz), 7.77 (1H, d, J=8Hz)

(9) 8-[3-[N-(Acetoxyacetylglycyl)-N-methylamino]-2,6-dichlorobenzyloxy]-3-bromo-2-methylimidazo[1,2-a]pyridine

NMR ($CDCl_3$, δ) : 2.20 (3H, s), 2.44 (3H, s), 3.26 (3H, s), 3.59 (1H, dd, J=17Hz and 4Hz), 3.82 (1H, dd, J=17Hz and 4Hz), 4.59 (2H, s), 5.46 (1H, d, J=10Hz), 5.52 (1H, d, J=10Hz), 6.71 (1H, d, J=7Hz), 6.87 (1H, t, J=7Hz), 7.11 (1H, br s), 7.31 (1H, d, J=9Hz), 7.49 (1H, d, J=9Hz), 7.78 (1H, d, J=7Hz)

(10) 8-[3-[N-(Acetylglycyl)-N-ethylamino]-2,6-dichlorobenzyloxy]-3-bromo-2-methylimidazo[1,2-a]pyridine

NMR ($CDCl_3$, δ) : 1.19 (3H, t, J=7Hz), 2.00 (3H, s), 2.42 (3H, s), 3.29 (1H), 3.48 (1H, dd, J=17Hz and 5Hz), 3.72 (1H, dd, J=17Hz and 5Hz), 4.21 (1H), 5.49 (2H, s), 6.46 (1H, br s), 6.71 (1H, d, J=7Hz), 6.87 (1H, t, J=7Hz), 7.22 (1H, d, J=9Hz), 7.48 (1H, d, J=9Hz), 7.78 (1H, d, J=7Hz)

(11) 8-[3-[N-(Acetyl-DL-alanyl)-N-methylamino]-2,6-dichlorobenzyloxy]-3-bromo-2-methylimidazo[1,2-a]pyridine

NMR ($CDCl_3$, δ) : 1.18 (1.5H, d, J=6Hz), 1.20 (1.5H, d, J=6Hz), 1.95 (1.5H, s), 1.99 (1.5H, s), 2.43 (3H, s), 3.22 (3H, s), 4.26-4.51 (1H), 5.47 (1H, s), 5.51 (1H, s), 6.15 (0.5H, d, J=8Hz), 6.43 (1H, d, J=8Hz), 6.65-6.90 (2H), 7.30 (0.5H, d, J=8Hz), 7.47 (0.5H, d, J=8Hz), 7.50 (1H, s), 7.76 (1H, d, J=8Hz)

(12) 8-[3-[N-(Acetyl-β-alanyl)-N-methylamino]-2,6-dichlorobenzyloxy]-3-bromo-2-methylimidazo[1,2-a]pyridine

NMR ($CDCl_3$, δ) : 1.94 (3H, s), 2.10-2.30 (2H), 2.42 (3H, s), 3.20 (3H, s), 3.39-3.56 (2H), 5.49 (2H, s), 6.39 (1H, br s), 6.70 (1H, d, J=7Hz), 6.85 (1H, t, J=7Hz), 7.26 (1H, d, J=9Hz), 7.45 (1H, d, J=9Hz), 7.78 (1H, d, J=7Hz)

(13) 3-Bromo-8-[2,6-dichloro-3-[N-(methoxycarbonylglycyl)-N-methylamino]benzyloxy]-2-methylimidazo[1,2-a]pyridine

NMR ($CDCl_3$, δ) : 2.42 (3H, s), 3.22 (3H, s), 3.49 (1H, dd, J=17Hz and 5Hz), 3.67 (3H, s), 3.72 (1H, dd, J=17Hz and 5Hz), 5.48 (2H, s), 5.54 (1H, br s), 6.71 (1H, d, J=7Hz), 6.82 (1H, t, J=7Hz), 7.31 (1H, d, J=9Hz), 7.49 (1H, d, J=9Hz), 7.78 (1H, d, J=7Hz)

(14) 3-Bromo-8-[2,6-dichloro-3-[N-(valerylglycyl)-N-methylamino]benzyloxy]-2-methylimidazo[1,2-a]pyridine

NMR ($CDCl_3$, δ) : 0.91 (3H, t, J=7Hz), 1.34 (2H, m), 1.61 (2H, m), 2.22 (2H, t, J=7Hz), 2.45 (3H, s), 3.26 (3H, 2), 3.52 (1H, dd, J=16Hz and 4Hz), 3.80 (1H, dd, J=16Hz and 4Hz), 5.48 (2H, s), 6.41 (1H,

t like), 6.71 (1H, d, J=8Hz), 6.86 (1H, dd, J=8Hz and 7Hz), 7.30 (1H, d, J=9Hz), 7.48 (1H, d, J=9Hz), 7.77 (1H, d, J=7Hz)

(15) 3-Bromo-8-[2,6-dichloro-3-[N-(isovalerylglycyl)-N-methylamino]benzyloxy]-2-methylimidazo[1,2-a]pyridine

NMR ($CDCl_3$, δ) : 0.96 (6H, d, J=7Hz), 2.02-2.17 (3H), 2.44 (3H, s), 3.26 (3H, s), 3.52 (1H, dd, J=16Hz and 4Hz), 3.80 (1H, dd, J=16Hz and 4Hz), 5.49 (2H, s), 6.39 (1H, t like), 6.72 (1H, d, J=8Hz), 6.86 (1H, dd, J=8Hz and 7Hz), 7.30 (1H, d, J=9Hz), 7.48 (1H, d, J=9Hz), 7.77 (1H, d, J=7Hz)

(16) 3-Bromo-8-[2,6-dichloro-3-[N-(2-pyridylacetylglycyl)-N-methylamino]benzyloxy]-2-methylimidazo[1,2-a]pyridine

NMR ($CDCl_3$, δ) : 2.45 (3H, s), 3.22 (3H, s), 3.54 (1H, dd, J=18Hz and 5Hz), 3.76 (2H, s), 3.81 (1H, dd, J=18Hz and 5Hz), 5.48 (2H, s), 6.71 (1H, d, J=7Hz), 6.85 (1H, t, J=7Hz), 7.16-7.32 (3H), 7.47 (1H, d, J=9Hz), 7.68 (1H, t, J=7Hz), 7.77 (1H, d, J=7Hz), 7.93 (1H, br t, J=5Hz)

(17) 3-Bromo-8-[2,6-dichloro-3-[N-(3-pyridylacetylglycyl)-N-methylamino]benzyloxy]-2-methylimidazo[1,2-a]pyridine

NMR ($CDCl_3$, δ) : 2.43 (3H, s), 3.22 (3H, s), 3.51 (1H, dd, J=17Hz and 5Hz), 3.58 (2H, s), 3.79 (1H, dd, J=17Hz and 5Hz), 5.48 (2H, s), 6.50 (1H, br t, J=5Hz), 6.70 (1H, d, J=7Hz), 6.85 (1H, t, J=7Hz), 7.21-7.32 (2H), 7.48 (1H, d, J=9Hz), 7.66 (1H, d, J=8Hz), 7.78 (1H, d, J=7Hz), 8.49-8.59 (2H)

(18) 3-Bromo-8-[2,6-dichloro-3-[N-(3-ethoxycarbonylpropionylglycyl)-N-methylamino]benzyloxy]-2-methylimidazo[1,2-a]pyridine

NMR ($CDCl_3$, δ) : 1.26 (3H, t, J=7Hz), 2.44 (3H, s), 2.49-2.69 (4H), 3.25 (3H, s), 3.53 (1H, dd, J=16Hz and 4Hz), 3.79 (1H, dd, J=16Hz and 4Hz), 4.13 (2H, q, J=7Hz), 5.50 (2H, s), 6.56 (1H, t like), 6.71 (1H, d, J=8Hz), 6.86 (1H, dd, J=8Hz and 6Hz), 7.30 (1H, d, J=10Hz), 7.48 (1H, d, J=10Hz), 7.77 (1H, d, J=6Hz)

(19) 3-Bromo-8-[2,6-dichloro-3-[N-(bromoacetylglycyl)-N-methylamino]benzyloxy]-2-methylimidazo[1,2-a]pyridine

NMR ($CDCl_3$, δ) : 2.44 (3H, s), 3.28 (3H, s), 3.57 (1H, dd, J=18Hz and 4Hz), 3.80 (1H, dd, J=18Hz and 4Hz), 3.88 (2H, s), 5.49 (2H, s), 6.71 (1H, d, J=7Hz), 6.88 (1H, t, J=7Hz), 7.31 (1H, d, J=9Hz), 7.39 (1H, br t, J=4Hz), 7.50 (1H, d, J=9Hz), 7.78 (1H, d, J=7Hz)

(20) 3-Bromo-8-[2,6-dichloro-3-[N-(phthalimidoacetylglycyl)-N-methylamino]benzyloxy]-2-methylimidazo[1,2-a]pyridine

mp : 211-213°C
NMR ($CDCl_3$, δ) : 2.43 (3H, s), 3.22 (3H, s), 3.56 (1H, dd, J=18Hz and 5Hz), 3.82 (1H, dd, J=18Hz and 5Hz), 4.40 (2H, s), 5.49 (2H, s), 6.69 (1H, d, J=7Hz), 6.78 (1H, br t, J=5Hz), 6.82 (1H, t, J=7Hz), 7.29 (1H, d, J=9Hz), 7.47 (1H, d, J=7Hz), 7.69-7.80 (3H), 7.82-7.92 (2H)

(21) 3-Bromo-8-[2,6-dichloro-3-[N-(4-nitrophenoxycarbonylglycyl)-N-methylamino]benzyloxy]-2-methylimidazo[1,2-a]pyridine

mp : 166-168°C
NMR ($CDCl_3$, δ) : 2.43 (3H, s), 3.29 (3H, s), 3.60 (1H, dd, J=17Hz and 5Hz), 3.81 (1H, dd, J=17Hz and 5Hz), 5.50 (2H, s), 6.05 (1H, br t, J=5Hz), 6.71 (1H, d, J=7Hz), 6.87 (1H, t, J=7Hz), 7.22-7.38 (3H), 7.49 (1H, d, J=9Hz), 7.79 (1H, d, J=7Hz), 8.22 (2H, d, J=9Hz)

(22) 3-Chloro-8-[2,6-dichloro-3-[N-(phenylacetylgylcyl)-N-methylamino]benzyloxy]-2-methylimidazo[1,2-a]pyridine

mp : 198-200°C

NMR ($CDCl_3$, δ) : 2.42 (3H, s), 3.21 (3H, s), 3.49 (1H, dd, J=15Hz and 4Hz), 3.60 (2H, s), 3.76 (1H, dd, J=15Hz and 4Hz), 5.46 (2H, s), 6.40 (1H, t like), 6.69 (1H, d, J=8Hz), 6.84 (1H, dd, J=8Hz and 7Hz), 7.20-7.50 (7H), 7.71 (1H, d, J=6Hz)

(23) 3-Chloro-8-[2,6-dichloro-3-[N-(butyrylglycyl)-N-methylamino]benzyloxy]-2-methylimidazo[1,2-a]pyridine

mp : 103-104°C
NMR ($CDCl_3$, δ) : 0.93 (3H, t, J=7Hz), 1.54-1.77 (2H), 2.20 (2H, t, J=7Hz), 2.42 (3H, s), 3.23 (3H, s), 3.51 (1H, dd, J=17Hz and 4Hz), 3.79 (1H, dd, J=17Hz and 4Hz), 5.49 (2H, s), 6.41 (1H, br t, J=4Hz), 6.70 (1H, d, J=7Hz), 6.86 (1H, t, J=7Hz), 7.31 (1H, d, J=9Hz), 7.47 (1H, d, J=9Hz), 7.71 (1H, d, J=7Hz)

Example 41

To a suspension of 3-bromo-8-[2,6-dichloro-3-(N-glycyl-N-methylamino)benzyloxy]-2-methylimidazo[1,2-a]pyridine (150 mg) in formic acid (1 ml) was added acetic anhydride (65 mg), and the mixture was stirred for 1.5 hours. Acetic anhydride (40 mg) was added thereto, and the mixture was stirred for 3 hours. The mixture was concentrated in vacuo, and a saturated aqueous solution of sodium bicarbonate was added thereto, and the mixture was extracted with methylene chloride three times. The organic layers were combined, washed with brine, dried over magnesium sulfate, and concentrated in vacuo. The residue was purified by preparative thin-layer chromatography (methylene chloride:methanol = 5:1, V/V) to give 3-bromo-8-[2,6-dichloro-3-[N-(formylglycyl)-N-methylamino]benzyloxy]-2-methylimidazo[1,2-a]pyridine (102 mg).

mp : 211-212°C
NMR ($CDCl_3$, δ) : 2.43 (3H, s), 3.26 (3H, s), 3.58 (1H, dd, J=17Hz and 5Hz), 3.82 (1H, dd, J=17Hz and 5Hz), 5.47 (1H, d, J=7Hz), 5.52 (1H, d, J=7Hz), 6.61 (1H, br s), 6.71 (1H, d, J=7Hz), 6.87 (1H, t, J=7Hz), 7.31 (1H, d, J=9Hz), 7.50 (1H, d, J=9Hz), 7.78 (1H, d, J=7Hz), 8.20 (1H, s)

Example 42

To a solution of 3-bromo-8-[2,6-dichloro-3-(N-glycyl-N-methylamino)benzyloxy]-2-methylimidazo[1,2-a]pyridine (98 mg) in methylene chloride (2 ml) was added methyl isocyanate (0.02 ml), and the mixture was stirred for 1 hour at ambient temperature. The mixture was concentrated in vacuo to give 3-bromo-8-[2,6-dichloro-3-[N-methyl-N-(N'-methylureidoacetyl)amino]benzyloxy]-2-methylimidazo[1,2-a]pyridine (9.5 mg).

NMR ($CDCl_3$, δ) : 2.44 (3H, s), 2.74 (3H, d, J=5Hz), 3.23 (3H, s), 3.58 (1H, dd, J=16Hz and 5Hz), 4.76 (1H, br s), 5.35 (1H, t like), 5.49 (2H, s), 6.74 (1H, d, J=8Hz), 6.88 (1H, t, J=8Hz), 7.35 (1H, d, J=8Hz), 7.48 (1H, d, J=8Hz), 7.78 (1H, d, J=8Hz)

Example 43

A mixture of 3-bromo-8-[2,6-dichloro-3-[N-(4-nitrophenoxycarbonylglycyl)-N-methylamino]benzyloxy]-2-methylimidazo[1,2-a]pyridine (100 mg), 3-aminopyridine (18 mg) and dioxane (1 ml) was refluxed for 1.5 hours under nitrogen atmosphere. The reaction mixture was washed with water four times and brine, dried over magnesium sulfate, and concentrated in vacuo. The residue was purified by preparative thin-layer chromatography (methylene chloride:methanol = 10:1, V/V) to give 3-bromo-8-[2,6-dichloro-3-[N-[N'-(3-pyridyl)ureidoacetyl]-N-methylamino]benzyloxy]-2-methylimidazo[1,2-a]pyridine (58 mg).

NMR ($CDCl_3$, δ) : 2.40 (3H, s), 3.22 (3H, s), 3.82 (2H, d, J=5Hz), 5.42 (1H, d, J=10Hz), 5.52 (1H, d, J=10Hz), 6.07 (1H, br t, J=5Hz), 6.72 (1H, d, J=7Hz), 6.89 (1H, t, J=7Hz), 7.11 (1H, dd, J=9Hz and 5Hz), 7.34 (1H, d, J=9Hz), 7.41 (1H, d, J=9Hz), 7.79 (1H, d, J=7Hz), 7.86 (1H, d, J=9Hz), 8.18 (1H, d, J=5Hz), 8.31 (1H, br s), 8.41 (1H, br s)

Example 44

The following compounds were obtained according to similar manners to those of Examples 42 or 43.

(1) 3-Bromo-8-[2,6-dichloro-3-[N-(N'-ethylureidoacetyl)-N-methylamino]benzyloxy]-2-methylimidazo[1,2-a]pyridine

mp : 126-127°C

NMR ($CDCl_3$, δ) : 1.20 (3H, t, J=7Hz), 2.42 (3H, s), 3.09-3.33 (5H), 3.56 (1H, dd, J=17Hz and 5Hz), 3.80 (1H, dd, J=17Hz and 5Hz), 4.71 (1H, br t, J=5Hz), 5.32 (1H, br t, J=5Hz), 5.49 (2H, s), 6.71 (1H, d, J=7Hz), 6.88 (1H, t, J=7Hz), 7.31 (1H, d, J=9Hz), 7.46 (1H, d, J=9Hz), 7.78 (1H, d, J=7Hz)

(2) 3-Bromo-8-[2,6-dichloro-3-[N-methyl-(N'-phenylureidoacetyl)amino]benzyloxy]-2-methylimidazo[1,2-a]pyridine

mp : 144-147°C

NMR ($CDCl_3$, δ) : 2.42 (3H, s), 3.22 (3H, s), 3.70 (1H, dd, J=17Hz and 4Hz), 3.86 (1H, dd, J=17Hz and 4Hz), 5.49 (2H, s), 5.79 (1H, br t, J=4Hz), 6.71 (1H, d, J=7Hz), 6.86 (1H, t, J=7Hz), 7.02 (1H), 7.19-7.30 (5H), 7.32 (1H, d, J=9Hz), 7.42 (1H, d, J=9Hz), 7.78 (1H, d, J=7Hz)

(3) 3-Bromo-8-[2,6-dichloro-3-[N-(N'-cyclohexylureidoacetyl)-N-methylamino]benzyloxy]-2-methylimidazo[1,2-a]pyridine

NMR ($CDCl_3$, δ) : 0.98-2.01 (10H), 2.43 (3H, s), 3.22 (3H, s), 3.42 (1H, m), 3.52 (1H, dd, J=17Hz and 5Hz), 3.80 (1H, dd, J=17Hz and 5Hz), 4.56 (1H, d, J=8Hz), 5.26 (1H, br t, J=5Hz), 5.48 (2H, s), 6.71 (1H, d, J=8Hz), 6.87 (1H, t, J=8Hz), 7.32 (1H, d, J=9Hz), 7.48 (1H, d, J=9Hz), 7.78 (1H, d, J=8Hz)

(4) 3-Bromo-8-[2,6-dichloro-3-[N-[N'-(α-naphthyl)ureidoacetyl]-N-methylamino]benzyloxy]-2-methylimidazo[1,2-a]pyridine

mp : 148-151°C

NMR ($CDCl_3$, δ) : 2.41 (3H, s), 3.16 (3H, s), 3.61 (1H, dd, J=18Hz and 4Hz), 3.87 (1H, dd, J=18Hz and 4Hz), 5.42 (2H, s), 6.01 (1H, br t, J=4Hz), 6.69 (1H, d, J=8Hz), 6.85 (1H, t, J=8Hz), 7.18 (1H, br s), 7.25 (1H, d, J=9Hz), 7.40 (1H, d, J=9Hz), 7.42-7.56 (4H), 7.62-7.92 (3H), 7.99-8.10 (1H, m)

(5) 3-Bromo-8-[2,6-dichloro-3-[N-(N'-benzylureidoacetyl)-N-methylamino]benzyloxy]-2-methylimidazo[1,2-a]pyridine

NMR ($CDCl_3$, δ) : 2.42 (3H, s), 3.11 (3H, s), 3.57 (1H, dd, J=17Hz and 5Hz), 3.82 (1H, dd, J=17Hz and 5Hz), 4.32 (2H, d, J=5Hz), 5.30 (1H, br t, J=5Hz), 5.48 (2H, s), 5.58 (1H, br t, J=5Hz), 6.71 (1H, d, J=7Hz), 6.86 (1H, t, J=7Hz), 7.18-7.39 (6H), 7.46 (1H, d, J=9Hz), 7.78 (1H, d, J=7Hz)

(6) 3-Bromo-8-[2,6-dichloro-3-[N-[N'-(m-tolyl)ureidoacetyl]-N-methylamino]benzyloxy]-2-methylimidazo[1,2-a]pyridine

NMR ($CDCl_3$, δ) : 2.29 (3H, s), 2.42 (3H, s), 3.22 (3H, s), 3.65 (1H, dd, J=17Hz and 5Hz), 3.85 (1H, dd, J=17Hz and 5Hz), 5.47 (2H, s), 5.96 (1H, br t, J=5Hz), 6.71 (1H, d, J=7Hz), 6.80-6.91 (2H), 7.00-7.39 (4H), 7.32 (1H, d, J=9Hz), 7.43 (1H, d, J=9Hz), 7.78 (1H, d, J=7Hz)

(7) 3-Bromo-8-[2,6-dichloro-3-[N-[N'-(p-tolyl)ureidoacetyl]-N-methylamino]benzyloxy]-2-methylimidazo[1,2-a]pyridine

NMR ($CDCl_3$, δ) : 2.30 (3H, s), 2.42 (3H, s), 3.21 (3H, s), 3.62 (1H, dd, J=17Hz and 5Hz), 3.85 (1H, dd, J=17Hz and 5Hz), 5.47 (2H, s), 5.91 (1H, br t, J=5Hz), 6.71 (1H, d, J=7Hz), 6.86 (1H, t, J=7Hz), 7.01-7.21 (5H), 7.31 (1H, d, J=9Hz), 7.43 (1H, d, J=9Hz), 7.78 (1H, d, J=7Hz)

(8) 3-Bromo-8-[2,6-dichloro-3-[N-[N'-(4-methoxyphenyl)ureidoacetyl]-N-methylamino]benzyloxy]-2-methylimidazo[1,2-a]pyridine

NMR ($CDCl_3$, δ) : 2.42 (3H, s), 3.21 (3H, s), 3.62 (1H, dd, J=17Hz and 5Hz), 3.78 (3H, s), 3.83 (1H, dd, J=17Hz and 5Hz), 5.48 (2H, s), 5.78 (1H, br t, J=5Hz), 6.68-6.96 (5H), 7.12-7.25 (2H), 7.32 (1H, d,

J=9Hz), 7.45 (1H, d, J=9Hz), 7.78 (1H, d, J=7Hz)

(9) 3-Bromo-8-[2,6-dichloro-3-[N-[N'-(4-trifluoromethylphenyl)ureidoacetyl]-N-methylamino]benzyloxy]-2-methylimidazo[1,2-a]pyridine

NMR ($CDCl_3$, δ) : 2.41 (3H, s), 3.22 (3H, s), 3.72 (1H, dd, J=17Hz and 5Hz), 3.86 (1H, dd, J=17Hz and 5Hz), 5.43 (1H, d, J=9Hz), 5.51 (1H, d, J=9Hz), 6.18 (1H, br t, J=5Hz), 6.72 (1H, d, J=7Hz), 6.89 (1H, t, J=7Hz), 7.26-7.45 (6H), 7.79 (1H, d, J=7Hz), 8.42 (1H, br s)

(10) 3-Bromo-8-[2,6-dichloro-3-[N-[N'-(4-fluorophenyl)ureidoacetyl]-N-methylamino]benzyloxy]-2-methylimidazo[1,2-a]pyridine

NMR ($CDCl_3$, δ) : 2.42 (3H, s), 3.21 (3H, s), 3.69 (1H, dd, J=17Hz and 5Hz), 3.83 (1H, dd, J=17Hz and 5Hz), 5.48 (2H, s), 5.91 (1H, br t, J=5Hz), 6.71 (1H, d, J=7Hz), 6.81-7.00 (3H), 7.16-7.29 (2H), 7.34 (1H, d, J=9Hz), 7.42 (1H, d, J=9Hz), 7.58 (1H, br s), 7.78 (1H, d, J=7Hz)

(11) 3-Bromo-8-[2,6-dichloro-3-[N-(N'-n-propylureidoacetyl)-N-methylamino]benzyloxy]-2-methylimidazo [1,2-a]pyridine

NMR ($CDCl_3$, δ) : 0.90 (3H, t, J=7Hz), 1.39-1.59 (2H), 2.43 (3H, s), 3.10 (2H, q, J=6Hz), 3.22 (3H, s), 3.54 (1H, dd, J=17Hz and 4Hz), 3.80 (1H, dd, J=17Hz and 4Hz), 4.73 (1H, br t, J=6Hz), 5.32 (1H, br t, J=4Hz), 5.49 (2H, s), 6.71 (1H, d, J=7Hz), 6.88 (1H, t, J=7Hz), 7.32 (1H, d, J=9Hz), 7.48 (1H, d, J=9Hz), 7.77 (1H, d, J=7Hz)

(12) 3-Bromo-8-[2,6-dichloro-3-[N-(N'-isopropylureidoacetyl)-N-methylamino]benzyloxy]-2-methylimidazo[1,2-a]pyridine

NMR ($CDCl_3$, δ) : 1.12 (6H, d, J=7Hz), 2.45 (3H, s), 3.52 (1H, dd, J=17Hz and 4Hz), 3.70-3.88 (2H), 4.51 (1H, br d, J=7Hz), 5.23 (1H, br t, J=4Hz), 5.49 (2H, s), 6.71 (1H, d, J=7Hz), 6.87 (1H, t, J=7Hz), 7.32 (1H, d, J=9Hz), 7.48 (1H, d, J=9Hz), 7.78 (1H, d, J=7Hz)

(13) 3-Bromo-8-[2,6-dichloro-3-[N-(N'-allylureidoacetyl)-N-methylamino]benzyloxy]-2-methylimidazo[1,2-a]pyridine

NMR ($CDCl_3$, δ) : 2.43 (3H, s), 3.22 (3H, s), 3.57 (1H, dd, J=17Hz and 5Hz), 3.70-3.89 (3H), 4.94 (1H, br t, J=5Hz), 5.04-5.28 (2H), 5.42-5.55 (3H), 5.83 (1H, m), 6.71 (1H, d, J=7Hz), 6.87 (1H, t, J=7Hz), 7.31 (1H, d, J=9Hz), 7.48 (1H, d, J=9Hz), 7.78 (1H, d, J=7Hz)

(14) 3-Bromo-8-[2,6-dichloro-3-[N-[N'-(4-pyridyl)ureidoacetyl]-N-methylamino]benzyloxy]-2-methylimidazo[1,2-a]pyridine

NMR ($CDCl_3$, δ) : 2.41 (3H, s), 3.20 (3H, s), 3.80 (2H, d, J=5Hz), 5.41 (1H, d, J=10Hz), 5.51 (1H, d, J=10Hz), 6.19 (1H, br t, J=5Hz), 6.76 (1H, d, J=7Hz), 6.90 (1H, t, J=7Hz), 7.19 (2H, d, J=6Hz), 7.32 (1H, d, J=9Hz), 7.40 (1H, d, J=9Hz), 7.79 (1H, d, J=7Hz), 8.28 (2H, d, J=6Hz), 8.95 (1H, br s)

(15) 3-Chloro-8-[2,6-dichloro-3-[N-(N'-phenylureidoacetyl)-N-methylamino]benzyloxy]-2-methylimidazo1,2-a]pyridine

mp : 230-231°C

NMR ($CDCl_3$, δ) : 2.42 (3H, s), 3.23 (3H, s), 3.69 (1H, dd, J=16Hz and 4Hz), 3.85 (1H, dd, J=16Hz and 4Hz), 5.49 (2H, s), 5.93 (1H, t like), 6.71 (1H, d, J=8Hz), 6.86 (1H, dd, J=8Hz and 7Hz), 7.20-7.37 (7H), 7.44 (1H, d, J=9Hz), 7.74 (1H, d, J=7Hz)

(16) 3-Chloro-8-[2,6-dichloro-3-[N-(N'-cyclohexylureidoacetyl)-N-methylamino]benzyloxy]-2-methylimidazo[1,2-a]pyridine

mp : 137-140°C

NMR ($CDCl_3$, δ) : 0.98-1.47 (6H), 1.50-1.78 (2H), 1.81-2.00 (2H), 2.43 (3H, s), 3.23 (3H, s), 3.43 (1H, m), 3.52

(1H, dd, J=16Hz and 4Hz), 3.79 (1H, dd, J=16Hz and 4Hz), 4.50 (1H, d, J=10Hz), 5.21 (1H, t like), 5.48 (2H, s), 6.70 (1H, d, J=8Hz), 6.86 (1H, dd, J=8Hz and 7Hz), 7.34 (1H, d, J=8Hz), 7.47 (1H, d, J=8Hz), 7.72 (1H, d, J=7Hz)

(17) 3-Chloro-8-[2,6-dichloro-3-[N-(N'-ethylureidoacetyl)-N-methylamino]benzyloxy]-2-methylimidazo[1,2-a]pyridine

NMR ($CDCl_3$, δ) : 1.11 (3H, t, J=6Hz), 2.42 (3H, s), 3.09-3.28 (5H), 3.53 (1H, dd, J=17Hz and 5Hz), 3.80 (1H, dd, J=17Hz and 5Hz), 4.70 (1H, br t, J=5Hz), 5.32 (1H, br t, J=5Hz), 5.49 (2H, s), 6.70 (1H, d, J=7Hz), 6.87 (1H, t, J=7Hz), 7.32 (1H, d, J=9Hz), 7.48 (1H, d, J=9Hz), 7.71 (1H, d, J=7Hz)

Example 45

To a mixture of 8-[3-(N-glycyl-N-methylamino)-2,6-dichlorobenzyloxy]-3-bromo-2-methylimidazo[1,2-a]pyridine (100 mg), acetic acid (0.5 ml) and water (1.0 ml) was added an aqueous solution (1 ml) of sodium cyanate (138 mg) at 40°C. The mixture was stirred at the same temperature for 1 hour and evaporated in vacuo. The residue was partitioned into dichloromethane and a saturated aqueous solution of sodium hydrogen carbonate and the organic layer was separated. The aqueous layer was extracted with dichloromethane twice. The combined organic layers were washed with brine, dried over magnesium sulfate and evaporated in vacuo. The residue was purified by preparative thin-layer chromatography (dichloromethane:methanol = 10:1, V/V) followed by crystallization from ethyl acetate - diethyl ether to give 3-bromo-8-[2,6-dichloro-3-[N-methyl-N-(ureidoacetyl)amino]benzyloxy]-2-methylimidazo[1,2-a]pyridine (72 mg) as crystals.

mp : 147-149°C
NMR ($CDCl_3$, δ) : 2.44 (3H, s), 3.23 (3H, s), 3.52 (1H, dd, J=17Hz and 5Hz), 3.80 (1H, dd, J=17Hz and 5Hz), 4.76 (2H, br s), 5.49 (2H, s), 5.82 (1H, t, J=5Hz), 6.71 (1H, d, J=7Hz), 6.87 (1H, t, J=7Hz), 7.33 (1H, d, J=9Hz), 7.49 (1H, d, J=9Hz), 7.79 (1H, d, J=7Hz)

Example 46

To a solution of 3-bromo-8-[2,6-dichloro-3-(N-glycyl-N-methylamino)benzyloxy]-2-methylimidazo[1,2-a] pyridine (150 mg) in methylene chloride (3 ml) was added phenylisothiocyanate (86 mg), and the mixture was refluxed for 1 hour under nitrogen atmosphere. The mixture was concentrated in vacuo, and the residue was purified by silica gel column chromatography (methylene chloride:methanol = 40:1, V/V) to give 3-bromo-8-[2,6-dichloro-3-[N-(N'-phenylthioureidoacetyl)-N-methylamino]benzyloxy]-2-methylimidazo[1,2-a]pyridine (190 mg).

NMR ($CDCl_3$, δ) : 2.42 (3H, s), 3.21 (3H, s), 3.82 (1H, dd, J=18Hz and 4Hz), 4.21 (1H, dd, J=18Hz and 5Hz), 5.49 (2H, s), 6.71 (1H, d, J=8Hz), 6.88 (1H, t, J=8Hz), 7.16 (1H, br s), 7.21-7.56 (7H), 7.71-7.87 (2H)

Example 47

3-Chloro-8-[2,6-dichloro-3-[N-(N'-phenylthioureidoacetyl)-N-methylamino]benzyloxy]-2-methylimidazo[1,2-a]pyridine was obtained according to a similar manner to that of Example 46.

mp : 135-137°C
NMR ($CDCl_3$, δ) : 2.43 (3H, s), 3.22 (3H, s), 3.85 (1H, dd, J=16Hz and 4Hz), 4.22 (1H, dd, J=16Hz and 4Hz), 5.51 (2H, s), 6.70 (1H, d, J=8Hz), 6.87 (1H, dd, J=8Hz and 7Hz), 7.16 (1H, t like), 7.21-7.54 (6H), 7.68-7.80 (2H)

Example 48

3-Bromo-8-[3-[N-(tert-butoxycarbonyl-L-prolylglycyl)-N-methylamino]-2,6-dichlorobenzyloxy]-2-methylimidazo[1,2-a]pyridine (67 mg) was dissolved in 4N solution of hydrogen chloride in ethyl acetate (3 ml). The solution was evaporated in vacuo to give 3-bromo-8-[2,6-dichloro-3-[N-(L-prolylglycyl)-N-methylamino]benzyloxy]-2- methylimidazo[1,2-a]pyridine dihydrochloride (64 mg) as colorless glass.

NMR ($CDCl_3$-$CD_3OD$, δ) : 2.00-2.20 (4H), 2.56 (3H, s), 3.26 (2.5H, s), 3.31-3.97 (4.5H), 4.38 (1H, t like), 5.70 (2H, s), 7.41-7.71 (4H), 8.23 (1H, d, J=8Hz)

Example 49

3-Bromo-8-[2,6-dichloro-3-[N-(D-prolylglycyl)-N-methylamino]benzyloxy]-2-methylimidazo[1,2-a]pyridine dihydrochloride was obtained according to a similar manner to that of Example 48.

NMR ($CDCl_3$-$CD_3OD$, δ) : 2.00-2.20 (4H), 2.54 (3H, s), 3.26 (2.5H, s), 3.31-3.95 (4.5H), 4.38 (1H, t like), 5.72 (2H, s), 7.49-7.71 (4H), 8.29 (1H, d, J=8Hz)

Example 50

A mixture of 3-bromo-8-[2,6-dichloro-3-[N-(L-prolylglycyl)-N-methylamino]benzyloxY]-2-methylimidazo[1,2-a]pyridine dihydrochloride (40 mg), acetic anhydride (0.01 ml), pyridine (0.03 ml) and methylene chloride (2 ml) was stirred for 2 hours at ambient temperature. The mixture was washed with water twice, dried over magnesium sulfate, and concentrated in vacuo to give 8-[3-[N-(acetyl-L-prolylglycyl)-N-methylamino]-2,6-dichlorobenzyloxy]-3-bromo-2-methylimidazo[1,2-a]pyridine (31 mg).

NMR ($CDCl_3$, δ) : 1.82-2.37 (7H), 2.44 (3H, s), 3.24 (3H, s), 3.34-3.89 (4H), 4.58 (1H, br s), 5.48 (2H, s), 6.71 (1H, d, J=8Hz), 6.85 (1H, t, J=8Hz), 7.29-7.53 (2H), 7.77 (1H, d, J=8Hz)

Example 51

The following compounds were obtained according to a similar manner to that of Example 50.

(1) 8-[3-[N-(Acetyl-D-prolylglycyl)-N-methylamino]-2,6-dichlorobenzyloxy]-3-bromo-2-methylimidazo[1,2-a]pyridine

NMR ($CDCl_3$, δ) : 1.82-2.37 (7H), 2.44 (3H, s), 3.24 (3H, s), 3.34-3.89 (4H), 4.58 (1H, br s), 5.48 (2H, s), 6.71 (1H, d, J=8Hz), 6.85 (1H, t, J=8Hz), 7.29-7.53 (2H), 7.77 (1H, d, J=8Hz)

(2) 8-[3-[N-(Acetylglycylglycyl)-N-methylamino]-2,6-dichlorobenzyloxy]-3-bromo-2-methylimidazo[1,2-a]pyridine

mp : 220-222°C

NMR ($CDCl_3$, δ) : 2.04 (3H, s), 2.41 (3H, s), 3.22 (3H, s), 3.53 (1H, dd, J=17Hz and 5Hz), 3.84 (1H, dd, J=17Hz and 5Hz), 3.38-4.00 (2H), 5.51 (2H, s), 6.72 (1H, d, J=7Hz), 6.90 (1H, t, J=7Hz), 7.40 (1H, d, J=9Hz), 7.52 (1H, d, J=9Hz), 7.79 (1H,, d, J=7Hz)

Example 52

The following compounds were obtained according to similar manners to those of Examples 9 or 10.

(1) 3-Bromo-8-[2,6-dichloro-3-[N-(N,N-dimethylglycyl)-N-methylamino]benzyloxy]-2-methylimidazo[1,2-a]pyridine

NMR ($CDCl_3$, δ) : 2.27 (6H, s), 2.45 (3H, s), 2.72 (1H, d, J=15Hz), 2.91 (1H, d, J=15Hz), 3.20 (3H, s), 5.49 (1H, d, J=7Hz), 5.55 (1H, d, J=7Hz), 6.71 (1H, d, J=7Hz), 6.85 (1H, t, J=7Hz), 7.79 (1H, d, J=9Hz), 7.45 (1H, d, J=9Hz), 7.78 (1H, d, J=7Hz)

(2) 3-Bromo-8-[2,6-dichloro-3-[N-(isopropylglycyl)-N-methylamino]benzyloxy]-2-methylimidazo[1,2-a]pyridine

NMR ($CDCl_3$, δ) : 0.98-1.09 (6H), 2.43 (3H, s), 2.72 (1H), 3.00 (1H, d, J=16Hz), 3.18 (1H, d, J=16Hz), 3.22 (3H, s), 5.51 (2H, s), 6.71 (1H, d, J=7Hz), 6.85 (1H, t, J=7Hz), 7.30 (1H, d, J=9Hz), 7.48 (1H, d, J=9Hz), 7.78 (1H, d, J=7Hz)

(3) 3-Bromo-8-[2,6-dichloro-3-[N-(isopropylglycylglycyl)-N-methylamino]benzyloxy]-2-methylimidazo[1,2-a]pyridine

NMR ($CDCl_3$, δ) : 1.10 (6H, d, J=6Hz), 2.43 (3H, s), 3.26 (3H, s), 3.29 (2H, s), 3.56 (1H, dd, J=17Hz and 5Hz), 3.82 (1H, dd, J=17Hz and 5Hz), 5.49 (2H, s), 6.71 (1H, d, J=7Hz), 6.86 (1H, t, J=7Hz), 7.32 (1H, d, J=9Hz), 7.48 (1H, d, J=9Hz), 7.76 (1H, d, J=7Hz), 8.08 (1H, br t, J=5Hz)

(4) 3-Bromo-8-[2,6-dichloro-3-[N-[(N,N-dimethylglycyl)glycyl]-N-methylamino]benzyloxy]-2-methylimidazo[1,2-a]pyridine

NMR ($CDCl_3$, δ) : 2.32 (6H, s), 2.43 (3H, s), 2.96 (2H, s), 3.25 (3H, s), 3.55 (1H, dd, J=18Hz and 4Hz), 3.85 (1H, dd, J=18Hz and 4Hz), 5.50 (2H, s), 6.71 (1H, d, J=7Hz), 6.86 (1H, t, J=7Hz), 7.32 (1H, d, J=9Hz), 7.49 (1H, d, J=9Hz), 7.78 (1H, d, J=7Hz), 7.89 (1H, br t, J=4Hz)

(5) 3-Bromo-8-[2,6-dichloro-3-[N-(benzylglycylglycyl)-N-methylamino]benzyloxy]-2-methylimidazo[1,2-a]pyridine

NMR ($CDCl_3$, δ) : 2.43 (3H, s), 3.28 (3H, s), 3.31 (2H, s), 3.58 (1H, dd, J=17Hz and 5Hz), 3.80 (2H, s), 3.84 (1H, dd, J=17Hz and 5Hz), 5.50 (2H, s), 6.71 (1H, d, J=7Hz), 6.86 (1H, t, J=7Hz), 7.19-7.40 (7H), 7.48 (1H, d, J=9Hz), 7.27 (1H, d, J=7Hz), 7.97 (1H, br t, J=5Hz)

Example 53

A mixture of 3-bromo-8-[2,6-dichloro-3-[N-(bromoacetylglycyl)-N-methylamino]benzyloxy]-2-methylimidazo[1,2-a]pyridine (150 mg) and 30% solution of methylamine in methanol (2 ml) was stirred for 1 hour at ambient temperature. The mixture was concentrated in vacuo, and the residue was purified by silica gel column chromatography (methylene chloride:methanol = 10:1, V/V) to give 3-bromo-8-[2,6-dichloro-3-[N-(sarcosylglycyl)-N-methylamino]benzyloxy]-2-methylimidazo[1,2-a]pyridine (103 mg).

NMR ($CDCl_3$, δ) : 2.45 (3H, s), 2.48 (3H, s), 3.26 (5H, s), 3.59 (1H, dd, J=17Hz and 5Hz), 3.83 (1H, dd, J=17.5Hz), 5.49 (2H, s), 6.71 (1H, d, J=7Hz), 6.86 (1H, t, J=7Hz), 7.32 (1H, d, J=9Hz), 7.49 (1H, d, J=9Hz), 7.78 (1H, d, J=7Hz), 7.89 (1H, br t, J=5Hz)

Example 54

The following compounds were obtained according to a similar manner to that of Example 53.

(1) 3-Bromo-8-[2,6-dichloro-3-[N-(ethylglycylglycyl)-N-methylamino]benzyloxy]-2- methylimidazo[1,2-a]pyridine

NMR ($CDCl_3$, δ) : 1.12 (3H, t, J=6Hz), 2.43 (3H, s), 2.69 (2H, g, J=6Hz), 3.26 (3H, s), 3.30 (2H, s), 3.58 (1H, dd, J=17Hz and 5Hz), 3.82 (1H, dd, J=17Hz and 5Hz), 5.49 (2H, s), 6.71 (1H, d, J=7Hz), 6.86 (1H, t, J=7Hz), 7.32 (1H, d, J=9Hz), 7.49 (1H, d, J=9Hz), 7.78 (1H, d, J=7Hz), 7.99 (1H, br t, J=5Hz)

(2) 3-Bromo-8-[2,6-dichloro-3-[N-[(N-phenylglycyl)glycyl]-N-methylamino]benzyloxy]-2-methylimidazo [1,2-a]pyridine

NMR ($CDCl_3$, δ) : 2.42 (3H, s), 3.21 (3H, s), 3.60 (1H, dd, J=18Hz and 5Hz), 3.80 (2H, s), 3.83 (1H, dd, J=18Hz and 5Hz), 4.39 (1H, br s), 5.49 (2H, s), 6.60 (2H, d, J=6Hz), 6.68-6.90 (3H), 7.12-7.41 (4H), 7.48 (1H, d, J=9Hz), 7.78 (1H, d, J=7Hz)

(3) 3-Bromo-8-[2,6-dichloro-3-[N-(morpholinoacetylglycyl)-N-methylamino]benzyloxy]-2-methylimidazo[1,2-a]pyridine

NMR ($CDCl_3$, δ) : 2.45 (3H, s), 2.50-2.62 (4H), 3.02 (2H, s), 3.26 (3H, s), 3.57 (1H, dd, J=18Hz and 5Hz), 3.70-3.92 (5H), 5.50 (2H, s), 6.71 (1H, d, J=7Hz), 6.87 (1H, t, J=7Hz), 7.32 (1H, d, J=9Hz), 7.49 (1H, d, J=9Hz), 7.78 (1H, d, J=7Hz), 7.92 (1H, br t, J=5Hz)

Example 55

To a mixture of 3-bromo-8-(2,6-dichloro-4-benzoylbenzyloxy)-2-methylimidazo[1,2-a]pyridine (80 mg), sodium borohydride (18.5 mg) and ethanol (2 ml) was stirred at ambient temperature for 1 hour. The reaction mixture was partitioned between dichloromethane and water and the aqueous layer was extracted with dichloromethane twice. The combined organic layers were washed with brine, dried over magnesium sulfate and evaporated in vacuo. The residue was crystallized from diethyl ether to give 3-bromo-8-[2,6-dichloro-4-(α-hydroxybenzyl)benzyloxy]-2-methylimidazo[1,2-a]pyridine (73 mg) as crystals.

mp : 178-179°C

NMR ($CDCl_3$, δ) : 2.40 (3H, s), 2.73 (1H, br s), 5.45 (2H, dd, J=10Hz and 9Hz), 6.28 (1H, s), 6.69 (1H, d, J=7Hz), 6.80 (1H, t, J=7Hz), 7.23-7.48 (6H), 7.60-7.80 (2H)

Example 56

3-Bromo-8-[2,6-dichloro-3-[N-(mesylglycyl)-N-methylamino]benzyloxy]-2-methylimidazo[1,2-a]pyridine was obtained according to a similar manner to that of Example 19.

NMR ($CDCl_3$, δ) : 2.45 (3H, s), 2.97 (2.7Hz, s), 3.02 (0.3H, s), 3.27 (2.7H, s), 3.30 (0.3H, s), 3.50 (1H, dd, J=16Hz and 5Hz), 3.67 (1H, dd, J=16Hz and 5Hz), 5.18 (1H, t like), 5.51 (2H, s), 6.71 (1H, d, J=8Hz), 6.87 (1H, t, J=8Hz), 7.32 (1H, d, J=8Hz), 7.51 (1H, d, J=8Hz), 7.78 (1H, d, J=8Hz)

Example 57

To a mixture of 3-bromo-8-(2,6-dibromo-4-methoxycarbonylbenzyloxy)-2-methylimidazo[1,2-a]pyridine (550 mg), methanol (10 ml) and tetrahydrofuran (5 ml) was added 1N aqueous solution of sodium hydroxide (1.135 ml), and the mixture was stirred for 1 hour at 60°C. The reaction mixture was adjusted pH 4 with 1N hydrochloric acid, and water was added thereto. The precipitate was collected by filtration to give 3-bromo-8-(2,6-dibromo-4-carboxybenzyloxy)-2-methylimidazo[1,2-a]pyridine (518 mg).

mp : 241-242°C

NMR (DMSO-$d_6$, δ) : 2.41 (3H, s), 5.62 (2H, s), 7.54 (1H, t, J=7Hz), 7.78 (1H, d, J=7Hz), 8.19 (2H, s), 8.32 (1H, d, J=7Hz)

Example 58

The following compounds were obtained according to a similar manner to that of Example 57.

(1) 3-Chloro-8-(2,6-dichlorobenzyloxy)imidazo[1,2-a]pyridine-2-carboxylic acid

mp : 212-213°C

NMR ($CDCl_3$ + $CD_3OD$, δ) : 5.50 (2H, s), 6.82 (1H, d, J=7Hz), 7.02 (1H, t, J=7Hz), 7.22-7.48 (3H), 7.86 (1H, d, J=7Hz)

(2) 3-Bromo-8-[2,6-dichloro-3-[N-(3-carboxypropionylglycyl)-N-methylamino]benzyloxy]-2-methylimidazo[1,2-a]pyridine

NMR ($CDCl_3$-$CD_3OD$, δ) : 2.30 (3H, s), 2.44 (4H, s), 3.12 (3H, s), 3.68-3.86 (2H), 5.40 (2H, s), 6.69 (1H, d, J=8Hz), 6.81 (1H, dd, J=8Hz and 6Hz), 7.46 (2H, s), 7.69 (1H, d, J=6Hz)

Example 59

To a mixture of 3-bromo-8-(2,6-dibromo-4-carboxybenzyloxy)-2-methylimidazo[1,2-a]pyridine (100 mg), methylene chloride (2 ml) and N,N-dimethylformamide (1 drop) was added oxalyl chloride (49 mg), and the mixture was stirred for 30 minutes and evaporated. The residue was dissolved in methylene chloride, triethylamine (0.5 ml) and 1-methylpiperazine (23 mg) were added thereto, and the mixture was stirred for 1 hour. Water was added thereto, the mixture was extracted with methylene chloride 3 times. The combined organic layer was washed with brine, dried over magnesium sulfate, and concentrated in vacuo. The residue was purified by silica gel column chromatography (5% solution of methanol in methylene chloride) to give 3-bromo-8-[2,6-dibromo-4-(N-methylpiperazinylcarbonyl)benzyloxy]-2-methylimidazo[1,2-a]pyridine (102 mg).

mp : 188-190°C

NMR ($CDCl_3$, δ) : 2.45 (3H, s), 2.48 (3H, s), 2.49-2.79 (4H), 3.56-4.02 (4H), 5.50 (2H, s), 6.70 (1H, d, J=7Hz), 6.85 (1H, t, J=7Hz), 7.61 (2H, s), 7.77 (1H, d, J=7Hz)

Example 60

To a suspension of 3-bromo-8-(2,6-dichloro-3-nitrobenzyloxy)-2-methylimidazo[1,2-a]pyridine (200 mg) in ethanol (2 ml) was added hydrogen chloride in ethanol (3.5 Mol solution, 1 ml). The solution was concentrated to half volume under reduced pressure. The separated precipitates were collected by filtration and washed with ethanol to give 3-bromo-8-(2,6-dichloro-3-nitrobenzyloxy)-2-methylimidazo[1,2-a]pyridine hydrochloride (175 mg) as an off-white solid.

mp : 195-197°C
NMR (DMSO-$d_6$, δ) : 2.39 (3H, s), 5.60 (2H, s), 7.39 (1H, t, J=7.5Hz), 7.56 (1H, d, J=7.5Hz), 7.92 (1H, d, J=7.5Hz), 8.25 (2H, d, J=7.5Hz)

Example 61

The following compounds were obtained according to a similar manner to that of Example 60.

(1) 3-Bromo-8-[2,6-dichloro-3-(N-acetyl-N-methylamino)benzyloxy]-2-methylimidazo[1,2-a]pyridine hydrochloride

mp : 148-150°C
NMR ($CDCl_3$ + $CD_3OD$, δ) : 1.88 (3H, s), 2.68 (3H, s), 3.22 (3H, s), 5.62 (1H, d, J=9Hz), 5.70 (1H, d, J=9Hz), 7.32-7.61 (4H), 8.09 (1H, d, J=6Hz)

(2) 3-Chloro-8-[2,6-dichloro-3-(N-acetyl-N-methylamino)benzyloxy]-2-methylimidazo[1,2-a]pyridine hydrochloride

mp : 145-149°C
NMR ($CDCl_3$ + $CD_3OD$, δ) : 1.89 (3H, s), 2.70 (3H, s), 3.22 (3H, s), 5.61 (1H, d, J=10Hz), 5.70 (1H, d, J=10Hz), 7.25-7.59 (4H), 8.01 (1H, d, J=7Hz)

(3) 3-Bromo-8-[2,6-dichloro-3-(N-propionyl-N-methylamino)benzyloxy]-2-methylimidazo[1,2-a]pyridine hydrochloride

mp : 146-148°C
NMR ($CDCl_3$, δ) : 1.08 (3H, t, J=7Hz), 2.09 (2H, q, J=7Hz), 2.74 (3H, s), 3.26 (3H, s), 5.66 (2H, br t, J=12Hz), 7.16-7.58 (4H), 8.00 (1H)

(4) 3-Bromo-8-[2,4,6-trichloro-3-(N-acetyl-N-methylamino)benzyloxy]-2-methylimidazo[1,2-a]pyridine hydrochloride

mp : 116-120°C
NMR ($CDCl_3$, δ) : 1.90 (2.6H, s), 2.29 (0.4H, s), 2.70 (2.6H, s), 2.72 (0.4H, s), 3.22 (2.6H, s), 3.43 (0.4H, s), 5.50-5.70 (2H), 7.18 (1H, d, J=8Hz), 7.30 (1H, t, J=8Hz), 7.52 (0.13H, s), 7.62 (0.87H, s), 7.98 (1H, d, J=8Hz)

(5) 8-[3-[N-(Acetylglycyl)-N-methylamino]-2,6-dichlorobenzyloxy]-3-bromo-2-methylimidazo[1,2-a]pyridine hydrochloride

mp : 175-176°C
NMR (DMSO-$d_6$, δ) : 1.83, 2.40, 3.12 (each 3H, s), 3.37, 3.67 (each 1H, dd, J=16Hz and 5Hz), 5.59 (2H, s), 7.31-7.83 (4H), 8.10 (1H, t, J=5Hz), 8.25 (1H, d, J=7Hz)

(6) 3-Bromo-8-[2,6-dichloro-3-[N-methyl-N-(propionylglycyl)amino]benzyloxy]-2-methylimidazo[1,2-a]pyridine hydrochloride

NMR ($CDCl_3$, δ) : 1.13 (3H, t, J=6Hz), 2.23 (2H, q, J=6Hz), 2.71 (3H, s), 3.33 (2.8H, s), 3.47 (0.2H, s), 3.66 (1H, dd, J=16Hz and 4Hz), 3.80 (1H, dd, J=16Hz and 4Hz), 5.61 (1H, d, J=10Hz), 5.68 (1H, d, J=10Hz), 6.74 (1H, t like), 7.20-7.44 (3H), 7.50 (1H, d, J=8Hz), 8.00 (1H, d, J=7Hz)

(7) 3-Bromo-8-[3-[N-(butyrylglycyl)-N-methylamino]-2,6-dichlorobenzyloxy]-2-methylimidazo[1,2-a]pyridine hydrochloride

NMR (DMSO-$d_6$, δ) : 0.84 (3H, t, J=7Hz), 1.49 (2H, m), 2.10 (2H, t, J=7Hz), 2.40 (3H, s), 3.13 (2.4H, s), 3.30 (0.6H, s), 3.36 (1H, dd, J=16Hz and 5Hz), 3.68 (1H, dd, J=16Hz and 5Hz), 5.60 (2H, s), 7.37-7.86 (4H), 8.03 (1H, t like), 8.28 (1H, d, J=6Hz)

18) 3-Bromo-8-[3-[N-(isobutyrylglycyl)-N-methylamino]-2,6-dichlorobenzyloxy]-2-methylimidazo[1,2-a]pyridine hydrochloride

NMR (DMSO-$d_6$, δ) : 0.97 (6H, d, J=6Hz), 2.40 (3H, s), 2.45 (1H, m), 3.12 (2.3H, s), 3.28 (0.7H, s), 3.34 (1H, dd, J=16Hz and 5Hz), 3.67 (1H, dd, J=16Hz and 5Hz), 5.58 (2H, s), 7.36-7.84 (4H), 7.99 (1H, t like), 8.27 (1H, d, J=7Hz)

(9) 3-Bromo-8-[2,6-dichloro-3-[N-(cyclopropylcarbonylglycyl)-N-methylamino]benzyloxy]-2-methylimidazo[1,2-a]pyridine hydrochloride

NMR (DMSO-$d_6$, δ) : 0.53-0.72 (4H), 1.68 (1H, m), 2.39 (3H, s), 3.12 (2.5H, s), 3.28 (0.5H, s), 3.40 (1H, dd, J=16Hz and 5Hz), 3.70 (1H, dd, J=16Hz and 5Hz), 5.59 (2H, s), 7.33-7.89 (4H), 8.26 (1H, d, J=7Hz), 8.34 (1H, t, J=6Hz)

(10) 3-Bromo-8-[2,6-dichloro-3-[N-(trifluoroacetylglycyl)-N-methylamino]benzyloxy]-2-methylimidazo[1,2-a]pyridine hydrochloride

mp : 194-195°C
NMR (DMSO-$d_6$, δ) : 2.39 (3H, s), 3.14 (3H, s), 3.51 (1H, dd, J=17Hz and 5Hz), 3.78 (1H, dd, J=17Hz and 5Hz), 5.58 (2H, s), 7.39 (1H, t, J=6Hz), 7.59 (1H), 7.81 (2H, s), 8.25 (1H, d, J=6Hz), 9.71 (1H, t, J=6Hz)

(11) 8-[3-[N-(Benzoylglycyl)-N-methylamino]-2,6-dichlorobenzyloxy]-3-bromo-2-methylimidazo[1,2-a]pyridine hydrochloride

mp : 138°C (dec.)
NMR (DMSO-$d_6$, δ) : 2.39 (3H, s), 3.15 (3H, s), 3.58 (1H, dd, J=16Hz and 6Hz), 3.89 (1H, dd, J=16Hz and 6Hz), 5.59 (2H, s), 7.34-7.64 (5H), 7.77-7.93 (4H), 8.28 (1H, d, J=6Hz), 8.72 (1H, t, J=6Hz)

(12) 3-Bromo-8-[2,6-dichloro-3-[N-(cyclohexylcarbonylglycyl)-N-methylamino]benzyloxy]-2-methylimidazo[1,2-a]pyridine hydrochloride

NMR (DMSO-$d_6$, δ) : 1.02-1.79 (10H), 2.18 (1H, m), 2.39 (3H, s), 3.11 (2.6H, s), 3.27 (0.4H, s), 3.31 (1H, dd, J=16Hz and 5Hz), 3.65 (1H, dd, J=16Hz and 5Hz), 5.58 (2H, s), 7.31-7.82 (4H), 7.92 (1H, t, J=5Hz), 8.26 (1H, d, J=7Hz)

(13) 3-Bromo-8-[2,6-dichloro-3-[N-(phenylacetylglycyl)-N-methylamino]benzyloxy]-2-methylimidazo[1,2-a]pyridine hydrochloride

NMR (DMSO-$d_6$, δ) : 2.38 (3H, s), 3.13 (2.5H, s), 3.27 (0.5H, s), 3.30-3.76 (4H), 5.56 (2H, s), 7.14-7.85 (9H), 8.24 (1H, d, J=7Hz), 8.32 (1H, t, J=5Hz)

(14) 8-[3-[N-(Acetoxyacetylglycyl)-N-methylamino]-2,6-dichlorobenzyloxy]-3-bromo-2-methylimidazo[1,2-a]pyridine hydrochloride

NMR ($CDCl_3$-$CD_3OD$, δ) : 2.21 (3H, s), 2.61 (3H, s), 3.25 (3H, s), 3.59-3.88 (2H), 4.09 (1H, br s), 4.59 (1H, br s), 5.69 (2H, br s), 7.35-7.68 (4H), 8.11 (1H, br s)

(15) 3-Bromo-8-[2,6-dichloro-3-[N-(glycoloylglycyl)-N-methylamino]benzyloxy]-2-methylimidazo[1,2-a]pyridine hydrochloride

NMR ($CDCl_3$-$CD_3OD$, δ) : 2.59 (3H, s), 3.28 (3H, s), 3.78 (2H, s), 4.09 (2H, s), 5.54-5.81 (2H), 7.47-7.68 (4H), 8.14 (1H, br s)

(16) 3-Bromo-8-[2,6-dichloro-3-[N-(methoxyacetylglycyl)acetyl-N-methylamino]benzyloxy]-2-methylimidazo[1,2-a]pyridine hydrochloride

mp : 175-177°C
NMR (DMSO-$d_6$, δ) : 2.40 (3H, s), 3.12 (3H, s), 3.32 (3H, s), 3.42 (1H, dd, J=17Hz and 5Hz), 3.70 (1H, dd, J=17Hz and 5Hz), 3.82 (2H, s), 5.59 (2H, s), 7.34-7.79 (5H), 8.26 (1H, d, J=7Hz)

(17) 3-Bromo-8-[2,6-dichloro-3-[N-methyl-N-(phthalimidoacetylglycyl)amino]benzyloxy]-2-methylimidazo[1,2-a]pyridine hydrochloride

NMR ($CDCl_3$-$CD_3OD$, δ) : 2.57 (3H, s), 3.26 (3H, s), 3.65 (1H, d, J=15Hz), 3.78 (1H, d, J=15Hz), 4.32 (1H, d, J=15Hz), 4.46 (1H, d, J=15Hz), 5.66 (2H, s), 7.35-7.64 (4H), 7.70-7.92 (4H), 8.08 (1H, d, J=5Hz)

(18) 8-[3-[N-(Acetylglycylglycyl)-N-methylamino]-2,6-dichlorobenzyloxy]-3-bromo-2-methylimidazo[1,2-a]pyridine hydrochloride

NMR ($CDCl_3$ + $CD_3OD$, δ) : 2.01 (3H, s), 2.52 (3H, s), 3.22 (3H, s), 3.56-3.70 (2H), 3.84 (2H, s), 5.70 (2H, br s), 7.51-7.72 (5H), 8.29 (1H, d, J=6Hz)

(19) 8-[3-[N-(Acetylglycyl)-N-ethylamino]-2,6-dichlorobenzyloxy]-3-bromo-2-methylimidazo[1,2-a]pyridine hydrochloride

NMR ($CDCl_3$, δ) : 1.20 (3H, t, J=7Hz), 2.00 (3H, s), 2.71 (3H, s), 3.43-3.88 (3H), 4.14 (1H), 5.68 (2H, s), 6.88 (1H, br s), 7.18-7.58 (3H), 8.00 (1H, br s)

(20) 8-[3-[N-(Acetyl-DL-alanyl)-N-methylamino]-2,6-dichlorobenzyloxy]-3-bromo-2-methylimidazo[1,2-a]pyridine hydrochloride

NMR ($CDCl_3$, δ) : 1.11 (3H, d, J=6Hz), 1.94 (3H, s), 2.68 (3H, s), 3.30 (3H, s), 4.36 (1H, m), 5.53-5.76 (2H), 6.60-6.80 (1H), 7.13-7.60 (4H), 7.93-8.06 (1H)

(21) 8-[3-[N-(Acetyl-β-alanyl)-N-methylamino]-2,6-dichlorobenzyloxy]-3-bromo-2-methylimidazo[1,2-a]pyridine hydrochloride

mp : 188-190°C
NMR ($CDCl_3$ + $CD_3OD$, δ) : 2.01 (3H, s), 2.12-2.72 (2H), 3.63 (3H, s), 3.20 (3H, s), 3.30-3.68 (2H), 5.58 (1H, d, J=10Hz), 5.73 (1H, d, J=10Hz), 7.32-7.61 (4H), 8.11 (1H, d, J=5Hz)

(22) 3-Bromo-8-[2,6-dichloro-3-[N-(N,N-dimethylglycyl)-N-methylamino]benzyloxy]-2-methylimidazo[1,2-a]pyridine dihydrochloride

mp : 162-164°C
NMR ($CDCl_3$ + $CD_3OD$, δ) : 2.66 (3H, s), 3.00-3.19 (6H), 3.28 (3H, s), 4.01 (1H, d, J=17Hz), 4.36 (1H, d, J=17Hz), 5.49 (1H, d, J=9Hz), 5.75 (1H, d, J=9Hz), 7.32-7.56 (2H), 7.61 (1H, d, J=9Hz), 7.75 (1H, d, J=9Hz), 8.09 (1H, d, J=5Hz)

(23) 3-Bromo-8-[2,6-dichloro-3-[N-(isopropylglycyl)-N-methylamino]benzyloxy]-2-methylimidazo[1,2-a]pyridine dihydrochloride

NMR ($CDCl_3$ + $CD_3OD$, δ) : 1.41 (6H, br s), 2.67 (3H, br s), 3.29 (3H, br s), 3.60-3.98 (3H), 5.51 (1H, br s), 5.76 (1H, br s), 7.32-8.14 (5H)

(24) 3-Bromo-8-[2,6-dichloro-3-[N-(methoxycarbonylglycyl)-N-methylamino]benzyloxy]-2-methylimidazo[1,2-a]pyridine hydrochloride

mp : 176-177°C
NMR ($CDCl_3$ + $CD_3OD$, δ) : 2.68 (3H, s), 3.29 (3H, s), 3.50-3.79 (5H), 5.68 (2H, s), 7.31-7.60 (4H), 8.02 (1H,

d, J=6Hz)

(25) 3-Bromo-8-[2,6-dichloro-3-[N-(mesylglycyl)-N-methylamino]benzyloxy]-2-methylimidazo[1,2-a]pyridine hydrochloride

mp : 174-176°C
NMR ($CDCl_3$-$CD_3OD$, δ) : 2.63 (3H, s), 3.03 (3H, s), 3.27 (3H, s), 3.60 (1H, d, J=16Hz), 3.70 (1H, d, J=16Hz), 5.62 (1H, d, J=12Hz), 5.70 (1H, d, J=12Hz), 7.33-7.51 (3H), 7.58 (1H, d, J=8Hz), 8.06 (1H, d, J=6Hz)

(26) 3-Bromo-8-[2,6-dichloro-3-[N-methyl-N-(ureidoacetyl)amino]benzyloxy]-2-methylimidazo[1,2-a]pyridine hydrochloride

NMR (DMSO-$d_6$, δ) : 2.40 (3H, s), 3.12 (3H, s), 3.39 (1H, d, J=17Hz), 3.61 (1H, d, J=17Hz), 5.57 (1H, d, J=8Hz), 5.67 (1H, d, J=8Hz), 7.49 (1H, t, J=7Hz), 7.69 (1H, d, J=7Hz), 7.81 (2H, s), 8.31 (1H, d, J=7Hz)

(27) 3-Bromo-8-[2,6-dichloro-3-[N-methyl-N-(N'-methylureidoacetyl)amino]benzyloxy]-2-methylimidazo[1,2-a]pyridine hydrochloride

mp : 153-155°C
NMR ($CDCl_3$, δ) : 2.63 (3H, s), 2.74 (3H, s), 3.23 (3H, s), 3.85 (1H, d, J=16Hz), 3.98 (1H, d, J=16Hz), 5.57 (1H, d, J=10Hz), 5.67 (1H, d, J=10Hz), 7.30-7.60 (4H), 8.04 (1H, d, J=7Hz)

(28) 3-Bromo-8-[2,6-dichloro-3-[N-(N'-ethylureidoacetyl)-N-methylamino]benzyloxy]-2-methylimidazo[1,2-a]pyridine hydrochloride

NMR (DMSO-$d_6$, δ) : 0.96 (3H, t, J=7Hz), 2.41 (3H, s), 2.98 (2H, q, J=7Hz), 3.11 (3H, s), 3.30 (1H, d, J=17Hz), 3.61 (1H, d, J=17Hz), 5.56 (1H, d, J=9Hz), 5.67 (1H, d, J=9Hz), 7.50 (1H, t, J=7Hz), 7.70 (1H, d, J=7Hz), 7.81 (2H, s), 8.31 (1H, d, J=7Hz)

its dihydrochloride

mp : 173-175°C

(29) 3-Bromo-8-[2,6-dichloro-3-[N-methyl-N-(N'-phenylureidoacetyl)amino]benzyloxy]-2-methylimidazo[1,2-a]pyridine hydrochloride

NMR (DMSO-$d_6$, δ) : 2.38 (3H, s), 3.16 (2.5H, s), 3.29 (0.5H, s), 3.42 (1H, d, J=16Hz), 3.70 (1H, d, J=16Hz), 5.58 (2H, s), 6.45 (1H, br s), 6.89 (1H, t, J=7Hz), 7.13-7.90 (8H), 8.26 (1H, d, J=7Hz), 8.97 (1H, s)

(30) 3-Bromo-8-[2,6-dichloro-3-[N-(4-pentenoylglycyl)-N-methylamino]benzyloxy]-2-methylimidazo[1,2-a]pyridine hydrochloride

NMR ($CDCl_3$-$CD_3OD$, δ) : 2.20-2.32 (4H), 2.46 (3H, s), 3.17 (3H, s), 3.56 (1H, s), 3.59 (1H, s), 4.83-5.06 (2H), 5.56-5.84 (3H), 7.42-7.62 (4H), 8.19 (1H, d, J=6Hz)

(31) 3-Bromo-8-[2,6-dichloro-3-[N-(valerylglycyl)-N-methylamino]benzyloxy]-2-methylimidazo[1,2-a]pyridine hydrochloride

NMR ($CDCl_3$, δ) : 0.90 (3H, t, J=7Hz), 1.32 (2H, m), 1.60 (2H, m), 2.22 (2H, t, J=7Hz), 2.72 (3H, s), 3.33 (3H, s), 3.58-3.87 (2H), 5.60 (1H, d, J=11Hz), 5.68 (1H, d, J=11Hz), 6.76 (1H, br s), 7.21-7.45 (3H), 7.50 (1H, d, J=9Hz), 8.00 (1H, d, J=6Hz)

(32) 3-Bromo-8-[2,6-dichloro-3-[N-(isovalerylglycyl)-N-methylamino]benzyloxy]-2-methylimidazo[1,2-a]pyridine hydrochloride

NMR ($CDCl_3$, δ) : 0.93 (6H, d, J=6Hz), 2.00-2.20 (3H), 2.73 (3H, s), 3.33 (3H, s), 3.60-3.88 (2H), 5.65 (2H, s), 6.77 (1H, br s), 7.20-7.44 (3H), 7.50 (1H, d, J=9Hz), 8.00 (1H, d, J=6Hz)

(33) 3-Bromo-8-[2,6-dichloro-3-[N-(2-pyridylacetylglycyl)-N-methylamino]benzyloxy]-2-methylimidazo[1,2-a]pyridine dihydrochloride

NMR ($CDCl_3$-$CD_3OD$, δ) : 2.53 (3H, s), 3.25 (3H, s), 3.35 (2H, s), 3.62 (1H, d, J=17Hz), 3.82 (1H, d, J=17Hz), 5.72 (2H, s), 7.48-7.72 (4H), 7.87-8.03 (2H), 8.29 (1H, d, J=6Hz), 8.48 (1H, t, J=7Hz), 8.75 (1H, d, J=6Hz)

(34) 3-Bromo-8-[2,6-dichloro-3-[N-(3-pyridylacetylglycyl)-N-methylamino]benzyloxy]-2-methylimidazo[1,2-a]pyridine dihydrochloride

NMR ($CDCl_3$-$CD_3OD$, δ) : 2.56 (3H, s), 3.25 (3H, s), 3.60 (1H, d, J=17Hz), 3.80 (1H, d, J=17Hz), 3.93 (2H, s), 5.72 (2H, s), 7.52-7.73 (4H), 8.07 (1H, dd, J=7Hz and 5Hz), 8.29 (1H, d, J=7Hz), 8.60 (1H, d, J=7Hz), 8.75 (1H, d, J=5Hz), 8.89 (1H, br s)

(35) 3-Bromo-8-[2,6-dichloro-3-[N-[(3-ethoxycarbonylpropionyl)glycyl]-N-methylamino]benzyloxy]-2-methylimidazo[1,2-a]pyridine hydrochloride

NMR ($CDCl_3$, δ) : 1.23 (3H, t, J=7Hz), 2.44-2.66 (4H), 2.70 (3H, s), 3.30 (3H, s), 3.63 (1H, dd, J=15Hz and 4Hz), 3.79 (1H, dd, J=15Hz and 4Hz), 4.10 (2H, q, J=7Hz), 5.58 (1H, d, J=11Hz), 5.67 (1H, d, J=11Hz), 6.89 (1H, br s), 7.09-7.43 (3H), 7.51 (1H, d, J=9Hz), 7.96 (1H, d, J=5Hz)

(36) 3-Bromo-8-[2,6-dichloro-3-[N-(sarcosylglycyl)-N-methylamino]benzyloxy]-2-methylimidazo[1,2-a]pyridine dihydrochloride

NMR ($CDCl_3$-$CD_3OD$, δ) : 2.28 (3H, s), 2.46 (3H, s), 2.98 (3H, s), 3.34 (1H, d, J=16Hz), 3.52 (2H, s), 3.59 (1H, d, J=16Hz), 5.43 (2H, s), 7.22-7.42 (4H), 8.00 (1H, d, J=6Hz)

(37) 3-Bromo-8-[2,6-dichloro-3-[N-[(ethylglycyl)glycyl]-N-methylamino]benzyloxy]-2-methylimidazo[1,2-a]pyridine dihydrochloride

NMR ($CDCl_3$-$CD_3OD$, δ) : 1.38 (3H, t, J=6Hz), 2.56 (3H, s), 3.10 (2H, q, J=6Hz), 3.27 (3H, s), 3.61 (1H, d, J=16Hz), 3.82 (2H, s), 3.88 (1H, d, J=16Hz), 5.72 (2H, s), 7.50-7.74 (4H), 8.29 (1H, d, J=6Hz)

(38) 3-Bromo-8-[2,6-dichloro-3-[N-[(N-phenylglycy1)glycyl]-N-methylamino]benzyloxy]-2-methylimidazo[1,2-a]pyridine dihydrochloride

NMR ($CDCl_3$-$CD_3OD$, δ) : 2.59 (3H, s), 3.26 (3H, s), 3.61-3.78 (2H), 4.00 (2H, s), 5.71 (2H, br s), 7.02-7.20 (3H), 7.30-7.41 (2H), 7.53-7.70 (4H), 8.26 (1H, d, J=6Hz)

(39) 3-Bromo-8-[2,6-dichloro-3-[N-(morpholinoacetylglycyl)-N-methylamino]benzyloxy]-2-methylimidazo[1,2-a]pyridine dihydrochloride

NMR ($CDCl_3$-$CD_3OD$, δ) : 2.53 (3H, s), 3.21 (3H, s), 3.30-3.48 (4H), 3.59 (1H, d, J=16Hz), 3.81 (1H, d, J=16Hz), 3.85-4.07 (6H), 5.69 (2H, s), 7.50-7.69 (4H), 8.24 (1H, d, J=6Hz)

(40) 3-Bromo-8-[2,6-dichloro-3-[N-(isopropylglycylglycyl)-N-methylamino]benzyloxy]-2-methylimidazo[1,2-a]pyridine dihydrochloride

NMR ($CDCl_3$-$CD_3OD$, δ) : 1.38 (6H, d, J=6Hz), 2.56 (3H, s), 3.28 (3H, s), 3.81-3.48 (3H), 3.82 (2H, s), 5.72 (2H, s), 7.49-7.73 (4H), 8.30 (1H, d, J=6Hz)

(41) 3-Bromo-8-[2,6-dichloro-3-[N-[(N,N-dimethylglycyl)glycyl]-N-methylamino]benzyloxy]-2-methylimidazo[1,2-a]pyridine dihydrochloride

NMR ($CDCl_3$-$CD_3OD$, δ) : 2.29 (3H, s), 2.68 (6H, s), 2.97 (3H, s), 3.35 (1H, d, J=17Hz), 3.58 (1H, d, J=17Hz), 3.71 (2H, s), 5.42 (2H, s), 7.25-7.44 (4H), 7.99 (1H, d, J=6Hz)

(42) 3-Bromo-8-[2,6-dichloro-3-[N-(benzylglycylglycyl)-N-methylamino]benzyloxy]-2-methylimidazo[1,2-a]pyridine dihydrochloride

NMR ($CDCl_3$-$CD_3OD$, δ) : 2.58 (3H, s), 3.25 (3H, s), 3.62 (1H, d, J=17Hz), 3.70-3.92 (3H), 4.22 (2H, s), 5.72 (2H, s), 7.40-7.75 (9H), 8.30 (1H, d, J=6Hz)

(43) 3-Bromo-8-[2,6-dichloro-3-[N-(N'-cyclohexylureidoacetyl)-N-methylamino]benzyloxy]-2-methylimidazo[1,2-a]pyridine hydrochloride

NMR ($CDCl_3$-$CD_3OD$, δ) : 1.03-1.92 (10H), 2.58 (3H, s), 3.27 (3H, s), 3.44 (1H, m), 3.60 (1H, d, J=17Hz), 3.71 (1H, d, J=17Hz), 5.68 (1H, d, J=10Hz), 5.79 (1H, d, J=10Hz), 7.53-7.72 (4H), 8.79 (1H, d, J=6Hz)

(44) 3-Bromo-8-[2,6-dichloro-3-[N-[N'-(α-naphthyl)ureidoacetyl]-N-methylamino]benzyloxy]-2-methylimidazo[1,2-a]pyridine hydrochloride

NMR ($CDCl_3$-$CD_3OD$, δ) : 1.99 (3H, s), 3.00 (3H, s), 3.45 (1H, d, J=17Hz), 3.62 (1H, d, J=17Hz), 5.40 (2H, s), 7.02-7.50 (10H), 7.69 (1H, d, J=9Hz), 7.91 (1H, d, J=5Hz)

(45) 3-Bromo-8-[2,6-dichloro-3-[N-(N'-benzylureidoacetyl)-N-methylamino]benzyloxy]-2-methylimidazo[1,2-a]pyridine hydrochloride

NMR ($CDCl_3$-$CD_3OD$, δ) : 2.48 (3H, s), 3.28 (3H, s), 3.70 (2H, s), 4.30 (2H, s), 5.68 (1H, d, J=10Hz), 6.79 (1H, d, J=10Hz), 7.16-7.35 (5H), 7.54-7.72 (4H), 8.29 (1H, d, J=6Hz)

(46) 3-Bromo-8-[2,6-dichloro-3-[N-[N'-(m-tolyl)ureidoacetyl]-N-methylamino]benzyloxy]-2-methylimidazo[1,2-a]pyridine hydrochloride

NMR ($CDCl_3$-$CD_3OD$, δ) : 2.30 (3H, s), 2.52 (3H, s), 3.29 (3H, s), 3.72 (2H, s), 5.67 (1H, d, J=10Hz), 5.79 (1H, d, J=10Hz), 6.82 (1H, m), 7.09-7.19 (3H), 7.51-7.71 (4H), 8.26 (1H, d, J=5Hz)

(47) 3-Bromo-8-[2,6-dichloro-3-[N-[N'-(p-tolyl)ureidoacetyl]-N-methylamino]benzyloxy]-2-methylimidazo[1,2-a]pyridine hydrochloride

NMR ($CDCl_3$-$CD_3OD$, δ) : 2.18 (3H, s), 2.42 (3H, s), 3.19 (3H, s), 3.62 (2H, s), 5.59 (1H, br d, J=10Hz), 5.69 (1H, br d, J=10Hz), 6.93 (2H, d, J=9Hz), 7.10 (2H, d, J=9Hz), 7.43-7.62 (4H), 8.18 (1H, d, J=6Hz)

(48) 3-Bromo-8-[2,6-dichloro-3-[N-[N'-(4-methoxyphenyl)ureidoacetyl]-N-methylamino]benzyloxy]-2-methylimidazo[1,2-a]pyridine hydrochloride

NMR ($CDCl_3$-$CD_3OD$, δ) : 2.52 (3H, s), 3.29 (3H, s), 3.72 (2H, s), 3.79 (3H, s), 5.69 (1H, d, J=10Hz), 5.79 (1H, d, J=10Hz), 6.80 (2H, d, J=10Hz), 7.22 (2H, d, J=10Hz), 7.52-7.73 (4H), 8.29 (1H, d, J=6Hz)

(49) 3-Bromo-8-[2,6-dichloro-3-[N-[N'-(4-trifluoromethylphenyl)ureidoacetyl]-N-methylamino]benzyloxy]-2-methylimidazo[1,2-a]pyridine hydrochloride

NMR ($CDCl_3$-$CD_3OD$, δ) : 2.54 (3H, s), 3.29 (3H, s), 3.66 (1H, d, J=17Hz), 3.76 (1H, d, J=17Hz), 5.68 (1H, d, J=10Hz), 5.76 (1H, d, J=10Hz), 7.49-7.71 (8H), 8.28 (1H, d, J=6Hz)

(50) 3-Bromo-8-[2,6-dichloro-3-[N-[N'-(4-fluorophenyl)ureidoacetyl]-N-methylamino]benzyloxy]-2-methylimidazo[1,2-a]pyridine hydrochloride

NMR ($CDCl_3$-$CD_3OD$, δ) : 2.52 (3H, s), 3.28 (3H, s), 3.68 (1H, d, J=16Hz), 3.78 (1H, d, J=16Hz), 5.70 (1H, d,

J=10Hz), 5.79 (1H, d, J=10Hz), 6.89-7.02 (2H), 7.22-7.36 (2H), 7.55-7.75 (4H), 8.80 (1H, d, J=5Hz)

(51) 3-Bromo-8-[2,6-dichloro-3-[N-(N'-n-propylureidoacetyl)-N-methylamino]benzyloxy]-2-methylimidazo[1,2-a]pyridine hydrochloride

mp : 168-171°C
NMR ($CDCl_3$-$CD_3OD$, δ) : 0.92 (3H, t, J=7Hz), 1.40-1.60 (2H), 2.55 (3H, s), 3.08 (2H, t, J=6Hz), 3.22 (3H, s), 3.58 (1H, d, J=17Hz), 3.71 (1H, d, J=17Hz), 5.69 (1H, d, J=10Hz), 5.79 (1H, d, J=10Hz), 7.53-7.77 (4H), 8.31 (1H, d, J=6Hz)

(52) 3-Bromo-8-[2,6-dichloro-3-[N-(N'-isopropylureidoacetyl)-N-methylamino]benzyloxy]-2-methylimidazo[1,2-a]pyridine hydrochloride

NMR ($CDCl_3$-$CD_3OD$, δ) : 1.12 (6H, d, J=6Hz), 2.56 (3H, s), 3.25 (3H, s), 3.57-3.32 (3H), 5.65 (1H, d, J=10Hz), 5.78 (1H, d, J=10Hz), 7.51-7.71 (4H), 8.28 (1H, d, J=6Hz)

(53) 3-Bromo-8-[2,6-dichloro-3-[N-(N'-allylureidoacetyl)-N-methylamino]benzyloxy]-2-methylimidazo[1,2-a]pyridine hydrochloride

NMR ($CDCl_3$-$CD_3OD$, δ) : 2.51 (3H, s), 3.20 (3H, s), 3.57-3.79 (4H), 5.00-5.21 (2H), 5.57-2.90 (3H), 7.50-7.68 (4H), 8.22 (1H, d, J=6Hz)

(54) 3-Bromo-8-[2,6-dichloro-3-[N-[N'-(3-pyridyl)ureidoacetyl]-N-methylamino]benzyloxy]-2-methylimidazo[1,2-a]pyridine dihydrochloride

NMR ($CDCl_3$-$CD_3OD$, δ) : 2.48 (3H, s), 3.19 (3H, s), 3.52 (1H, d, J=17Hz), 3.73 (1H, d, J=17Hz), 5.62 (2H, s), 7.44-7.62 (4H), 7.81 (1H, m), 8.15-8.29 (3H), 9.12 (1H, s)

(55) 3-Bromo-8-[2,6-dichloro-3-[N-[N'-(4-pyridyl)ureidoacetyl]-N-methylamino]benzyloxy]-2-methylimidazo[1,2-a]pyridine dihydrochloride

NMR ($CDCl_3$-$CD_3OD$, δ) : 2.55 (3H, s), 3.28 (3H, s), 3.62 (1H, d, J=17Hz), 3.84 (1H, d, J=17Hz), 5.72 (2H, s), 7.50-7.72 (4H), 7.98 (2H, d, J=6Hz), 8.26 (1H, d, J=6Hz), 8.39 (2H, d, J=6Hz)

156) 3-Bromo-8-[2,6-dichloro-3-[N-(N'-phenylthioureidoacetyl)-N-methylamino]benzyloxy]-2-methylimidazo[1,2-a]pyridine hydrochloride

NMR ($CDCl_3$-$CD_3OD$, δ) : 2.58 (3H, s), 3.26 (3H, s), 3.92 (1H, d, J=17Hz), 4.18 (1H, d, J=17Hz), 5.74 (2H, s), 7.20-7.72 (9H), 8.27 (1H, d, J=6Hz)

(57) 3-Chloro-8-[2,6-dichloro-3-[N-(N'-phenylureidoacetyl)-N-methylamino]benzyloxy]-2-methylimidazo[1,2-a]pyridine hydrochloride

mp : 157-162°C
NMR (DMSO-$d_6$, δ) : 2.40 (3H, s), 3.16 (3H, s), 3.42 (1H, d, J=16Hz), 3.70 (1H, d, J=16Hz), 5.60 (2H, s), 6.48 (1H, br s), 6.89 (1H, t, J=8Hz), 7.12-7.90 (8H), 8.29 (1H, d, J=6Hz), 9.00 (1H, s)

(58) 3-Chloro-8-[2,6-dichloro-3-[N-(N'-cyclohexylureidoacetyl)-N-methylamino]benzyloxy]-2-methylimidazo[1,2-a]pyridine hydrochloride

mp : 170-172°C
NMR (DMSO-$d_6$, δ) : 0.91-1.80 (10H), 2.43 (3H, s), 3.12 (3H, s), 3.20-3.40 (2H), 3.62 (1H, d, J=16Hz), 5.58 (1H, d, J=10Hz), 5.66 (1H, dd, J=10Hz), 7.45-7.85 (4H), 8.37 (1H, d, J=6Hz)

(59) 3-Chloro-8-[2,6-dichloro-3-[N-(N'-ethylureidoacetyl)-N-methylamino]benzyloxy]-2-methylimidazo[1,2-a]pyridine hydrochloride

NMR ($CDCl_3$-$CD_3OD$, δ) : 1.10 (3H, t, J=6Hz), 2.57 (3H, s), 3.15 (2H, q, J=6Hz), 3.27 (3H, s), 3.66 (2H, s), 5.68 (1H, d, J=10Hz), 5.79 (1H, d, J=10Hz), 7.54-7.72 (4H), 8.29 (1H, d, J=6Hz)

(60) 3-Chloro-8-[2,6-dichloro-3-[N-(phenylacetylglycyl)-N-methylamino]benzyloxy]-2-methylimidazo[1,2-a]pyridine hydrochloride

NMR (DMSO-$d_6$, δ) : 2.39 (3H, s), 3.12 (2.2H, s), 3.27 (0.8H, s), 3.48 (1H, dd, J=16Hz and 6Hz), 3.47 (2H, s), 3.69 (1H, dd, J=16Hz and 6Hz), 5.58 (2H, s), 7.14-7.84 (9H), 8.23-8.37 (2H)

(61) 3-Chloro-8-[2,6-dichloro-3-[N-(butyrylglycyl)-N-methylamino]benzyloxy]-2-methylimidazo[1,2-a]pyridine hydrochloride

NMR ($CDCl_3$-$CD_3OD$, δ) : 0.98 (3H, t, J=6Hz), 1.53-1.75 (2H), 2.23 (2H, t, J=6Hz), 2.57 (3H, s), 3.27 (3H, s), 3.68 (1H, s), 3.70 (1H, s), 5.70 (1H, d, J=10Hz), 5.78 (1H, d, J=10Hz), 7.56-7.75 (4H), 8.30 (1H, d, J=6Hz)

(62) 3-Chloro-8-[2,6-dichloro-3-[N-(N'-phenylthioureidoacetyl)-N-methylamino]benzyloxy]-2-methylimidazo[1,2-a]pyridine hydrochloride

mp : 150-155°C
NMR (DMSO-$d_6$, δ) : 2.40 (3H, s), 3.18 (3H, s), 3.80 (1H, dd, J=16Hz and 4Hz), 4.23 (1H, dd, J=16Hz and 4Hz), 5.61 (2H, s), 7.11 (1H, t, J=8Hz), 7.27-7.92 (8H), 7.97 (1H, br s), 8.28 (1H, d, J=6Hz), 10.15 (1H, br s)

(63) 3-Bromo-8-[2,6-dichloro-3-[N-(glycylglycyl)-N-methylamino]benzyloxy]-2-methylimidazo[1,2-a]pyridine dihydrochloride

NMR ($CDCl_3$-$CD_3OD$, δ) : 2.56 (3H, s), 3.25 (3H, s), 3.69 (1H, d, J=17Hz), 3.72 (2H, s), 3.86 (1H, d, J=17Hz), 5.72 (2H, s), 7.50-7.78 (4H), 8.31 (1H, d, J=6Hz)

Example 62

The following compounds were obtained according to similar manners to those of Examples 1 or 2 using N-iodosuccinimide instead of N-bromosuccinimide or N-chlorosuccinimide.

(1) 8-[2,6-Dichloro-3-[N-[N-(N,N-dimethylglycyl)glycyl]-N-methylamino]benzyloxy]-3-iodo-2-methylimidazo[1,2-a]pyridine

NMR ($CDCl_3$, δ) : 2.31 (6H, s), 2.48 (3H, s), 2.96 (2H, s), 3.25 (3H, s), 3.55 (1H, dd, J=18Hz and 5Hz), 3.85 (1H, dd, J=18Hz and 5Hz), 5.50 (2H, s), 6.72 (1H, d, J=7Hz), 6.86 (1H, t, J=7Hz), 7.32 (1H, d, J=9Hz), 7.48 (1H, d, J=9Hz), 7.79 (1H, d, J=7Hz), 7.89 (1H, br s)

(2) 8-[2,6-Dichloro-3-(N-acetyl-N-methylamino)benzyloxy]-3-iodo-2-methylimidazo[1,2,a]pyridine

mp : 190-192°C
NMR ($CDCl_3$, δ) : 1.84 (3H, s), 2.49 (3H, s), 3.20 (3H, s), 5.50 (2H, s), 6.71 (1H, d, J=7Hz), 6.86 (1H, t, J=7Hz), 7.30 (1H, d, J=9Hz), 7.47 (1H, d, J=9Hz), 7.80 (1H, d, J=7Hz)

Example 63

The following compounds were obtained according to similar manners to those of Example 1 or 2.

(1) 3-Bromo-8-[2,6-dichloro-4-(N-acetyl-N-methylamino)benzyloxy]-2-methylimidazo[1,2-a]pyridine

mp : 82-88°C
NMR ($CDCl_3$, δ) : 2.06 (3H, br s), 2.45 (3H, s), 3.28 (3H, s), 5.45 (2H, s), 6.73 (1H, d, J=7.5Hz), 6.86 (1H, t, J=7.5Hz), 7.25 (2H, s), 7.77 (1H, d, J=7.5Hz)

(2) 3-Chloro-8-[2,6-dichloro-4-(N-acetyl-N-methylamino)benzyloxy]-2-methylimidazo[1,2-a]pyridine

mp : 74-78°C
NMR ($CDCl_3$, δ) : 2.04 (3H, br s), 2.44 (3H, s), 3.28 (3H, s), 5.45 (2H, s), 6.70 (1H, d, J=7Hz), 6.84 (1H, t, J=7.0Hz), 7.25 (2H, s), 7.71 (1H, d, J=7Hz)

Example 64

The following compounds were obtained according to similar manners to those of Example 42 or 43.

(1) 3-Bromo-8-[2,6-dichloro-3-[N-[N'-(3-methoxyphenyl)ureidoacetyl]-N-methylamino]benzyloxy]-2-methylimidazo[1,2-a]pyridine

NMR ($CDCl_3$, δ) : 2.41 (3H, s), 3.22 (3H, s), 3.61-3.91 (5H), 5.47 (2H, s), 5.95 (1H, br t, J=4Hz), 6.58 (1H, dd, J=7Hz and 1Hz), 6.58-6.91 (3H), 7.01 (1H, t, J=1Hz), 7.13 (1H, t, J=7Hz), 7.32 (1H, d, J=9Hz), 7.42 (1H, d, J=9Hz), 7.78 (1H, d, J=7Hz)

(2) 3-Bromo-8-[3-[N-[N'-(3-chlorophenyl)ureidoacetyl]-N-methylamino]-2,6-dichlorobenzyloxy]-2-methylimidazo[1,2-a]pyridine

mp : 151-152°C
NMR ($CDCl_3$, δ) : 2.42 (3H, s), 3.21 (3H, s), 3.88 (1H, dd, J=18Hz and 4Hz), 4.21 (1H, dd, J=18Hz and 4Hz), 5.50 (2H, s), 6.71 (1H, d, J=7Hz), 6.88 (1H, t, J=7Hz), 7.18-7.42 (6H), 7.50 (1H, d, J=9Hz), 7.78 (1H, d, J=7Hz), 8.01 (1H, br s)

(3) 3-Bromo-8-[2,6-dichloro-3-[N-[N'-(3-trifluoromethylphenyl)ureidoacetyl]-N-methylamino]benzyloxy]-2-methylimidazo[1,2-a]pyridine

NMR ($CDCl_3$, δ) : 2.40 (3H, s), 3.22 (3H, s), 3.82 (2H, br d, J=5Hz), 5.42 (1H, d, J=10Hz), 5.52 (1H, d, J=10Hz), 6.07 (1H, br t, J=5Hz), 6.73 (1H, d, J=7Hz), 6.89 (1H, t, J=7Hz), 7.11-7.46 (5H), 7.65 (1H, br s), 7.79 (1H, d, J=7Hz), 8.22 (1H, br s)

(4) 8-[3-[N-[N'-(3-Acetylphenyl)ureidoacetyl]-N-methylamino]-2,6-dichlorobenzyloxy]-3-bromo-2-methylimidazo[1,2-a]pyridine

NMR ($CDCl_3$, δ) : 2.40 (3H, s), 2.54 (3H, s), 3.37 (3H, s), 3.76 (1H, dd, J=18Hz and 5Hz), 3.89 (1H, dd, J=18Hz and 5Hz), 5.44 (1H, d, J=10Hz), 5.52 (1H, d, J=10Hz), 6.09 (1H, br t, J=5Hz), 6.72 (1H, d, J=7Hz), 6.88 (1H, t, J=7Hz), 7.28 (1H, t, J=7Hz), 7.39 (1H, d, J=9Hz), 7.43 (1H, d, J=9Hz), 7.54 (2H, d, J=7Hz), 7.79 (1H, d, J=7Hz), 7.86 (1H, br s), 8.00 (1H, br s)

(5) 3-Bromo-8-[3-[N-[N'-(3-cyanophenyl)ureidoacetyl]-N-methylamino]-2,6-dichlorobenzyloxy]-2-methylimidazo[1,2-a]pyridine

NMR ($CDCl_3$, δ) : 2.40 (3H, s), 3.21 (3H, s), 3.81 (2H, d, J=5Hz), 5.42 (1H, d, J=10Hz), 5.53 (1H, d, J=10Hz), 6.08 (1H, br t, J=5Hz), 6.76 (1H, d, J=7Hz), 6.90 (1H, t, J=7Hz), 7.11-7.26 (2H), 7.31-7.42 (3H), 7.66 (1H, br s), 7.80 (1H, d, J=7Hz), 8.63 (1H, br s)

(6) 3-Bromo-8-[2,6-dichloro-3-[N-[N'-(o-tolyl)ureidoacetyl]-N-methylamino]benzyloxy]-2-methylimidazo[1,2-a]pyridine

NMR ($CDCl_3$, δ) : 2.25 (3H, s), 2.42 (3H, s), 3.21 (3H, s), 3.58 (1H, dd, J=18Hz and 4Hz), 3.85 (1H, dd, J=18Hz and 5Hz), 5.48 (2H, s), 5.72 (1H, br s), 6.37 (1H, br s), 6.71 (1H, d, J=7Hz), 6.87 (1H, t, J=7Hz), 7.05-7.51 (6H), 7.78 (1H, d, J=7Hz)

(7) 3-Bromo-8-[2,6-dichloro-3-[N-[N'-(3-fluorophenyl)ureidoacetyl]-N-methylamino]benzyloxy]-2-methylimidazo[1,2-a]pyridine

NMR ($CDCl_3$, δ) : 2.41 (3H, s), 3.21 (3H, s), 3.79 (2H, br t, J=5Hz), 5.43 (1H, d, J=10Hz), 5.51 (1H, d, J=10Hz),

6.02 (1H, br t, J=5Hz), 6.58-6.94 (4H), 7.03-7.28 (2H), 7.34 (1H, d, J=9Hz), 7.41 (1H, d, J=9Hz), 7.78 (1H, d, J=7Hz), 7.98 (1H, br s)

(8) 3-Bromo-8-[3-[N-[N'-(3-ethylphenyl)ureidoacetyl]-N-methylamino]-2,6-dichlorobenzyloxy]-2-methylimidazo[1,2-a]pyridine

NMR ($CDCl_3$, δ) : 1.20 (3H, t, J=7Hz), 2.42 (3H, s), 2.60 (2H, q, J=7Hz), 3.22 (3H, s), 3.68 (1H, dd, J=18Hz and 5Hz), 3.87 (1H, dd, J=18Hz and 5Hz), 5.48 (2H, s), 5.91 (1H, br t, J=5Hz), 6.71 (1H, d, J=7Hz), 6.88 (1H, t, J=7Hz), 7.01-7.26 (4H), 7.32 (1H, d, J=9Hz), 7.45 (1H, d, J=9Hz), 7.78 (1H, d, J=7Hz)

(9) 8-[3-[N-(N'-Benzoylureidoacetyl)-N-methylamino]-2,6-dichlorobenzyloxy]-3-bromo-2-methylimidazo[1,2-a]pyridine

mp : 187-190°C (dec.)
NMR ($CDCl_3$, δ) : 2.42 (3H, s), 3.28 (3H, s), 3.71 (1H, dd, J=18Hz and 5Hz), 3.96 (1H, dd, J=18Hz and 5Hz), 5.50 (2H, s), 6.71 (1H, d, J=7Hz), 6.86 (1H, t, J=7Hz), 7.36 (1H, d, J=9Hz), 7.40-7.62 (4H), 7.77 (1H, d, J=7Hz), 7.83 (2H, d, J=8Hz), 8.45 (1H, br s), 9.23 (1H, br t, J=5Hz)

(10) 3-Bromo-8-[2,6-dichloro-3-[N-methyl-N-[N'-(3-nitrophenyl)ureidoacetyl]amino]benzyloxy]-2-methylimidazo[1,2-a]pyridine

mp : 160-168°C (dec.)
NMR ($CDCl_3$, δ) : 2.40 (3H, s), 3.25 (3H, s), 3.83 (2H, d, J=5Hz), 5.42 (1H, d, J=10Hz), 5.52 (1H, d, J=10Hz), 6.16 (1H, br t, J=5Hz), 6.77 (1H, d, J=7Hz), 6.90 (1H, t, J=7Hz), 7.23 (1H, t, J=8Hz), 7.40 (2H, s), 7.52 (1H, d, J=8Hz), 7.70 (1H, dd, J=8Hz and 1Hz), 7.79 (1H, d, J=7Hz), 8.14 (1H, t, J=1Hz), 8.79 (1H, s)

(11) 3-Bromo-8-[2,6-dichloro-3-[N-[N'-(3-ethoxycarbonylphenyl)ureidoacetyl]-N-methylamino]benzyloxy]-2-methylimidazo[1,2-a]pyridine

NMR ($CDCl_3$, δ) : 1.35 (3H, t, J=7Hz), 2.41 (3H, s), 3.25 (3H, s), 3.80 (2H, dd, J=7Hz and 5Hz), 4.32 (2H, q, J=7Hz), 5.44 (1H, d, J=10Hz), 5.51 (1H, d, J=10Hz), 6.01 (1H, br t, J=5Hz), 6.72 (1H, d, J=7Hz), 6.88 (1H, t, J=7Hz), 7.36 (1H, d, J=9Hz), 7.42 (1H, d, J=9Hz), 7.56-7.69 (2H), 7.72-7.89 (3H)

(12) 3-Bromo-8-[2,6-dichloro-3-[N-(N'-ethoxycarbonylmethylureidoacetyl)-N-methylamino]benzyloxy]-2-methylimidazo[1,2-a]pyridine

mp : 194-196°C
NMR ($CDCl_3$, δ) : 1.28 (3H, t, J=7Hz), 2.42 (3H, s), 3.22 (3H, s), 3.57 (1H, dd, J=18Hz and 5Hz), 3.80 (1H, dd, J=18Hz and 5Hz), 3.92 (2H, d, J=5Hz), 4.20 (2H, q, J=7Hz), 5.38 (1H, br t, J=5Hz), 5.49 (2H, s), 5.62 (1H, br t, J=5Hz), 6.71 (1H, d, J=7Hz), 6.86 (1H, t, J=7Hz), 7.32 (1H, d, J=9Hz), 7.47 (1H, d, J=9Hz), 7.78 (1H, d, J=7Hz)

Example 65

A mixture of 2-thiophenecarboxylic acid (60 mg), triethylamine (52 mg) and diphenylphosphoryl azide (135 mg) in dry toluene (0.6 ml) was refluxed. After 1 hour, to the cooled mixture was added a solution of 8-[3-(N-glycyl-N-methylamino)-2,6-dichlorobenzyloxy]-3-bromo-2-methylimidazo[1,2-a]pyridine (200 mg) in dry dichloromethane (1 ml), and the mixture was stirred at ambient temperature. After 1 hour, to the mixture was added 2-thiophenecarboxylic acid (60 mg), triethylamine (52 mg) and diphenylphosphoryl azide (135 mg). The mixture was refluxed for 1 hour. The reaction mixture was washed with water twice and brine. The organic layer was dried over magnesium sulfate and evaporated in vacuo. The residue was purified by silica gel column chromatography (ethyl acetate:methanol = 50:1, V/V) followed by preparative thin layer chromatography (dichloromethane: methanol = 10:1, V/V) to give 3-bromo-8-[2,6-dichloro-3-[N-[N'-(2-thienyl)ureidoacetyl]-N-methylamino]benzyloxy]-2-methylimidazo[1,2-a]pyridine (201 mg) as amorphous.

NMR ($CDCl_3$, δ) : 2.42 (3H, s), 3.29 (3H, s), 3.71 (1H, dd, J=18Hz and 4Hz), 3.97 (1H, dd, J=18Hz and 5Hz), 5.50 (2H, s), 6.71 (1H, d, J=7Hz), 6.88 (1H, t, J=7Hz), 7.00 (1H, br s), 7.09 (1H, dd, J=5Hz and 4Hz), 7.34 (1H, d, J=9Hz), 7.42-7.60 (3H), 7.78 (1H, d, J=7Hz)

Example 66

3-Bromo-8-[3-[N-[N'-(3-carboxyphenyl)ureidoacetyl]-N-methylamino]-2,6-dichlorobenzyloxy]-2-methylimidazo[1,2-a]pyridine was obtained according to a similar manner to that of Example 57.

mp : 248-250°C
NMR (DMSO-$d_6$, δ) : 2.30 (3H, s), 3.18 (3H, s), 3.43 (1H, dd, J=18Hz and 5Hz), 3.69 (1H, dd, J=18Hz and 5Hz). 5.49 (2H, s), 6.42 (1H, br t. J=5Hz), 6.94-7.07 (2H), 7.32 (1H, t, J=7Hz), 7.42-7.62 (2H), 7.30 (2H, s), 7.93 (1H, m), 8.02 (1H, br s), 9.09 (1H, s)

Example 67

To a solution of 3-bromo-8-[3-[N-[N'-(3-carboxyphenyl)ureidoacetyl]-N-methylamino]-2,6-dichlorobenzyloxy]-2-methylimidazo[1,2-a]pyridine (200 mg) and dimethylamine hydrochloride (31 mg) in N,N-dimethylformamide (2 ml) were added N-ethyl-N'-(3-dimethylaminopropyl)carbodiimide (64 mg) and 1-hydroxybenzotriazole (64 mg), and the mixture was stirred for 1 hour at ambient temperature. Water was added thereto, and the mixture was extracted with ethyl acetate three times. The organic layer was washed with water four times and brine, dried over magnesium sulfate and concentrated in vacuo. The residue was purified by a silica gel column chromatography (methylene chloride:methanol = 20:1, V/V) to give 3-bromo-8-[3-[N-[N'-[3-(N,N-dimethylcarbamoyl)phenyl]ureidoacetyl]-N-methylamino]-2,6-dichlorobenzyloxy]-2-methylimidazo[1,2-a]pyridine (189 mg).

NMR ($CDCl_3$, δ) : 2.41 (3H, s), 2.95 (3H, br s), 3.09 (3H, br s), 3.22 (3H, s), 3.64 (1H, dd, J=17Hz and 5Hz), 3.82 (1H, dd, J=17Hz and 5Hz), 5.48 (2H, s), 6.06 (1H, br t, J=5Hz), 6.72 (1H, d, 7Hz), 6.88 (1H, t, J=7Hz), 6.99 (1H, d, J=8Hz), 7.20 (1H, t, J=8Hz), 7.30-7.49 (4H), 7.78 (1H, d, J=7Hz), 8.11 (1H, s)

Example 68

3-Bromo-8-[2,6-dichloro-3-[N-[N-[3-(N-methylcarbamoyl)propionyl]glycyl]-N-methylamino]benzyloxy]-2-methylimidazo[1,2-a]pyridine was obtained according to a similar manner to that of Example 67.

NMR ($CDCl_3$, δ) : 2.40-2.63 (7H), 2.79 (3H, d, J=5Hz), 3.25 (3H, s), 3.52, 3.80 (each 1H, dd, J=18Hz and 5Hz), 5.48 (2H, s), 6.01 (1H, br s), 6.62-6.76 (2H), 6.86 (1H, t, J=7Hz), 7.31, 7.48 (each 1H, d, J=9Hz), 7.78 (1H, d, J=7Hz)

Example 69

To a mixture of 8-[3-(N-glycyl-N-methylamino)-2,6-dichlorobenzyloxy]-3-chloro-2-methylimidazo[1,2-a]pyridine (200 mg), triethylamine (0.1 ml) and dichloromethane (2 ml) was added bromoacetyl chloride (0.042 ml) in a dry ice-acetone bath. After 20 minutes, to the mixture was added 50% aqueous solution of dimethylamine (0.42 ml). The mixture was stirred for 1 hour at ambient temperature. The reaction mixture was washed with aqueous sodium bicarbonate solution, water and brine. The organic layer was dried over magnesium sulfate and evaporated in vacuo. The residue was purified by a silica gel column chromatography (dichloromethane:methanol = 20:1, V/V) to yield colorless crystals (191 mg) of 3-chloro-8-[2,6-dichloro-3-[N-[N-(N,N-dimethylglycyl)glycyl]-N-methylamino]benzyloxy]-2-methylimidazo[1,2-a]pyridine.

mp : 171-172°C
NMR ($CDCl_3$, δ) : 2.31 (6H, s), 2.44 (3H, s), 2.96 (2H, s), 3.23 (3H, s), 3.56 (1H, dd, J=18Hz, and 4Hz), 3.85 (1H, dd, J=18Hz and 4Hz), 5.49 (2H, s), 6.70 (1H, d, J=7Hz), 6.86 (1H, t, J=7Hz), 7.32 (1H, d, J=9Hz), 7.49 (1H, d, J=9Hz), 7.72 (1H, d, J=7Hz), 7.89 (1H, br s)

Example 70

The following compounds were obtained according to similar manners to those of Examples 53 or 69.

(1) 3-Bromo-8-[2,6-dichloro-3-[N-[N-(N-ethyl-N-methylglycyl)glycyl]-N-methylamino]benzyloxy]-2-methyl imidazo[1,2-a]pyridine

mp : 168-170°C

NMR ($CDCl_3$, δ) : 1.09 (3H, t, J=7Hz), 2.30 (3H, s), 2.43 (3H, s), 2.50 (2H, q, J=7Hz), 2.99 (2H, s), 3.25 (3H, s), 3.56 (1H, dd, J=17Hz and 5Hz), 3.85 (1H, dd, J=17Hz and 5Hz), 5.49 (2H, s), 6.71 (1H, d, J=7Hz), 6.87 (1H, t, J=7Hz), 7.32 (1H, d, J=9Hz), 7.48 (1H, d, J=9Hz), 7.78 (1H, d, J=7Hz), 8.01 (1H, br s)

(2) 3-Bromo-8-[3-[N-[N-(N-cyclopropylglycyl)glycyl]-N-methylamino]-2,6-dichlorobenzyloxy]-2-methylimidazo[1,2-a]pyridine

NMR ($CDCl_3$, δ) : 0.39-0.53 (4H), 2.22 (1H, m), 2.42 (3H), 3.25 (3H, s), 3.38 (2H, s), 3.55 (1H, dd, J=18Hz and 4Hz), 3.82 (1H, dd, J=18Hz and 4Hz), 5.49 (2H, s), 6.71 (1H, d, J=7Hz), 6.87 (1H, t, J=7Hz), 7.31 (1H, d, J=9Hz), 7.49 (1H, d, J=9Hz), 7.68 (1H, br s), 7.78 (1H, d, J=7Hz)

(3) 3-Bromo-8-[3-[N-[N-(N-cyclohexylglycyl)glycyl]-N-methylamino]-2,6-dichlorobenzyloxy]-2-methylimidazo[1,2-a]pyridine

mp : 182-184°C

NMR ($CDCl_3$, δ) : 0.98-1.31 (5H), 1.52-1.95 (5H), 2.39 (1H, m), 2.43 (3H, s), 3.24 (3H, s), 3.30 (2H, s), 3.56 (1H, ad, J=18Hz and 4Hz), 3.82 (1H, ad, J=18Hz and 4Hz), 5.49 (2H, s), 6.71 (1H, a, J=7Hz), 6.86 (1H, t, J=7Hz), 7.31 (1H, d, J=9Hz), 7.47 (1H d, J=9Hz), 7.78 (1H, d, J=7Hz), 8.11 (1H, br s)

(4) 3-Bromo-8-[2,6-dichloro-3-[N-methyl-N-[N-(1-pyrrolidinylacetyl)glycyl]amino]benzyloxy]-2- methylimidazo[1,2-a]pyridine

NMR ($CDCl_3$, δ) : 1.72-1.90 (4H), 2.42 (3H, s), 2.57-2.70 (4H), 3.16 (2H, s), 3.25 (3H, s), 3.57 (1H, dd, J=18Hz and 5Hz), 3.87 (1H, dd, J=18Hz and 5Hz), 5.50 (2H, s), 6.71 (1H, d, J=7Hz), 6.87 (1H, t, J=7Hz), 7.32 (1H, d, J=9Hz), 7.49 (1H, d, J=9Hz), 7.78 (1H, d, J=7Hz), 7.89 (1H, br s)

(5) 3-Bromo-8-[2,6-dichloro-3-[N-methyl-N-[N-(piperidinoacetyl)glycyl]amino]benzyloxy]-2-methylimidazo[1,2-a]pyridine

NMR ($CDCl_3$, δ) : 1.38-1.72 (6H), 2.39-2.55 (7H), 2.94 (2H, s), 3.27 (3H, s), 3.55 (1H, dd, J=18Hz and 4Hz), 3.83 (1H, dd, J=18Hz and 4Hz), 5.50 (2H, s), 6.71 (1H, d, J=7Hz), 6.87 (1H, t, J=7Hz), 7.31 (1H, d, J=9Hz), 7.49 (1H, d, J=9Hz), 7.78 (1H, d, J=7Hz), 8.08 (1H, br s)

(6) 3-Bromo-8-[2,6-dichloro-3-[N-methyl-N-[N-[[4-(4-pyridyl)-1-piperazinyl]acetyl]glycyl]amino]benzyloxy]-2-methylimidazo[1,2-a]pyridine

NMR ($CDCl_3$, δ) : 2.43 (3H, s), 2.61-2.78 (4H), 3.09 (2H, s), 3.26 (3H, s), 3.34-3.49 (4H), 3.58 (1H, dd, J=18Hz and 4Hz), 3.88 (1H, dd, J=18Hz and 4Hz), 5.48 (1H, d, J=10Hz), 5.52 (1H, d, J=10Hz), 6.61-6.78 (3H), 6.86 (1H, t, J=7Hz), 7.32 (1H, d, J=9Hz), 7.50 (1H, d, J=9Hz), 7.78 (1H, d, J=7Hz), 7.92 (1H, br t, J=4Hz), 8.29 (2H, br d, J=6Hz)

(7) 3-Bromo-8-[2,6-dichloro-3-[N-[N-(N-isopropyl-N-methylglycyl)glycyl]-N-methylamino]benzyloxy]-2-methylimidazo[1,2-a]pyridine

mp : 150-151°C

NMR ($CDCl_3$, δ) : 1.02 (6H, d, J=6Hz), 2.38 (3H, s), 2.42 (3H, s), 2.85 (1H, m), 3.00 (2H, s), 3.25 (3H, s), 3.55 (1H, dd, J=18Hz and 5Hz), 3.85 (1H, dd, J=18Hz and 5Hz), 5.49 (2H, s), 6.71 (1H, d, J=7Hz), 6.86 (1H, t, J=7Hz), 7.32 (1H, d, J=9Hz), 7.49 (1H, d, J=9Hz), 7.76 (1H, d, J=7Hz), 8.12 (1H, br s)

(8) 3-Bromo-8-[3-[N-[N-(N-cyclohexyl-N-methylglycyl)glycyl]-N-methylamino]-2,6-dichlorobenzyloxy]-2-methylimidazo[1,2-a]pyridine

NMR ($CDCl_3$, δ) : 1.00-1.33 (6H), 1.70-1.90 (4H), 2.31 (3H, s), 2.38 (1H, m), 2.42 (3H, s), 3.02 (2H, s), 3.25 (3H, s), 3.56 (1H, dd, J=18Hz and 5Hz), 3.83 (1H, dd, J=18Hz and 5Hz), 5.49 (2H, s), 6.71 (1H, d, J=7Hz), 6.86 (1H, t, J=7Hz), 7.32 (1H, d, J=9Hz), 7.48 (1H, d, J=9Hz), 7.78 (1H, d, J=7Hz), 8.19 (1H, br s)

(9) 3-Bromo-8-[3-[N-[N-(N-cycloheptylglycyl)glycyl]-N-methylamino]-2,6-dichlorobenzyloxy]-2-methylimidazo[1,2-a]pyridine

NMR ($CDCl_3$, δ) : 1.29-1.92 (12H), 2.43 (3H, s), 2.62 (1H, m), 3.24 (3H, s), 3.28 (2H, s), 3.58 (1H, dd, J=18Hz and 5Hz), 3.82 (1H, dd, J=18Hz and 5Hz), 5.49 (2H, s), 6.71 (1H, d, J=7Hz), 6.86 (1H, t, J=7Hz), 7.31 (1H, d, J=9Hz), 7.48 (1H, d, J=9Hz), 7.78 (1H, d, J=7Hz), 8.10 (1H, br s)

(10) 3-Bromo-8-[3-[N-[N-(N-cyclopentylglycyl)glycyl]-N-methylamino]-2,6-dichlorobenzyloxy]-2-methylimidazo[1,2-a]pyridine

NMR ($CDCl_3$, δ) : 1.23-1.90 (8H), 2.42 (3H, s), 3.09 (1H, m), 3.24 (3H, s), 3.28 (2H, s), 3.56 (1H, dd, J=18Hz and 5Hz), 3.82 (1H, dd, J=18Hz and 5Hz), 5.49 (2H, s), 6.72 (1H, d, J=7Hz), 6.86 (1H, t, J=7Hz), 7.31 (1H, d, J=9Hz), 7.49 (1H, d, J=9Hz), 7.78 (1H, d, J=7Hz), 8.00 (1H, br s)

(11) 3-Bromo-8-[3-[N-[N-(N-cyclooctylglycyl)glycyl]-N-methylamino]-2,6-dichlorobenzyloxy]-2-methylimidazo[1,2-a]pyridine

NMR ($CDCl_3$, δ) : 1.35-1.85 (14H), 2.42 (3H, s), 2.65 (1H, m), 3.27 (5H, s), 3.58 (1H, dd, J=18Hz and 5Hz), 3.82 (1H, dd, J=18Hz and 5Hz), 5.50 (2H, s), 6.71 (1H, d, J=7Hz), 6.88 (1H, t, J=7Hz), 7.32 (1H, d, J=9Hz), 7.49 (1H, d, J=9Hz), 7.79 (1H, d, J=7Hz), 8.10 (1H, br s)

(12) 3-Bromo-8-[3-[N-[N-(N-cycloheptyl-N-methylglycyl)glycyl]-N-methylamino]-2,6-dichlorobenzyloxy]-2-methylimidazo[1,2-a]pyridine

NMR ($CDCl_3$, δ) : 1.28-1.92 (12H), 2.29 (3H, s), 2.15 (3H, s), 2.60 (1H, m), 3.01 (2H, s), 3.25 (3H, s), 3.56 (1H, dd, J=18Hz and 5Hz), 3.85 (1H, dd, J=18Hz and 5Hz), 5.50 (2H, s), 6.72 (1H, d, J=7Hz), 6.88 (1H, t, J=7Hz), 7.32 (1H, d, J=9Hz), 7.49 (1H, d, J=9Hz), 7.79 (1H, d, J=7Hz), 8.18 (1H, br s)

(13) 3-Bromo-8-[2,6-dichloro-3-[N-[N-(hexamethyleneiminoacetyl)glycyl]-N-methylamino]benzyloxy]-2-methylimidazo[1,2-a]pyridine

NMR ($CDCl_3$, δ) : 1.58-1.78 (8H), 2.42 (3H, s), 2.63-2.73 (4H), 3.13 (2H, s), 3.28 (3H, s), 3.58 (1H, dd, J=18Hz and 5Hz), 3.83 (1H, dd, J=18Hz and 5Hz), 5.50 (2H, s), 6.71 (1H, d, J=7Hz), 6.86 (1H, t, J=7Hz), 7.32 (1H, d, J=9Hz), 7.49 (1H, d, J=9Hz), 7.78 (1H, d, J=7Hz), 8.12 (1H, br s)

(14) 8-[3-[N-[N-[N-(1-Adamantyl)glycyl]glycyl]-N-methylamino]-2,6-dichlorobenzyloxy]-3-bromo-2-methylimidazo[1,2-a]pyridine

NMR ($CDCl_3$, δ) : 1.49-1.74 (12H), 1.99-2.12 (3H), 2.43 (3H, s), 3.25 (5H, s), 3.56 (1H, dd, J=18Hz and 5Hz), 3.82 (1H, dd, J=18Hz and 5Hz), 5.50 (2H, s), 6.71 (1H, d, J=7Hz), 6.86 (1H, t, J=7Hz), 7.32 (1H, d, J=9Hz), 7.49 (1H, d, J=9Hz), 7.78 (1H, d, J=7Hz), 8.25 (1H, br s)

(15) 3-Bromo-8-[2,6-dichloro-3-[N-methyl-N-[N-[N-(piperidino)glycyl]glycyl]amino]benzyloxy]-2-methylimidazo[1,2-a]pyridine

mp : 242-243°C

NMR ($CDCl_3$-$CD_3OD$, δ) : 1.50-1.90 (4H), 2.06-2.34 (2H), 2.42 (3H, s), 2.66 (1H, m), 3.22 (3H, s), 3.52-3.87 (5H), 4.53 (2H, s), 5.50 (2H, s), 6.73 (1H, d, J=7Hz), 6.89 (1H, t, J=7Hz), 7.44 (1H, d, J=9Hz), 7.52 (1H, d, J=9Hz), 7.79 (1H, d, J=7Hz)

(16) 3-Bromo-8-[2,6-dichloro-3-[N-[N-[N-(2-furylmethyl)glycyl]glycyl]-N-methylamino]benzyloxy]-2-methylimidazo[1,2-a]pyridine

NMR ($CDCl_3$, δ) : 2.42 (3H, s), 3.25 (3H, s), 3.30 (2H, s), 3.56 (1H, dd, J=18Hz and 5Hz), 3.72-3.91 (3H), 5.50 (2H, s), 6.20 (1H, d, J=2Hz), 6.30 (1H, m), 6.71 (1H, d, J=7Hz), 6.86 (1H, t, J=7Hz), 7.29-7.39 (2H), 7.48 (1H, d, J=9Hz), 7.77 (1H, d, J=7Hz), 7.88 (1H, br s)

(17) 3-Bromo-8-[2,6-dichloro-3-[N-[N-(N,N-diethylglycyl)glycyl]-N-methylamino]benzyloxy]-2-methylimidazo[1,2-a]pyridine

mp : 159-160°C
NMR ($CDCl_3$, δ) : 1.06 (6H, t, J=7Hz), 2.43 (3H, s), 2.58 (4H, q, J=7Hz), 3.02 (2H, s), 3.26 (3H, s), 3.55 (1H, dd, J=18Hz and 4Hz), 3.84 (1H, dd, J=18Hz and 4Hz), 5.49 (2H, s), 6.71 (1H, d, J=7Hz), 6.86 (1H, t, J=7Hz), 7.31 (1H, d, J=9Hz), 7.48 (1H, d, J=9Hz), 7.76 (1H, d, J=7Hz), 8.18 (1H, br s)

(18) 3-Bromo-8-[2,6-dichloro-3-[N-[N-(1-imidazolylacetyl)glycyl]-N-methylamino]benzyloxy]-2-methylimidazo[1,2-a]pyridine

NMR ($CDCl_3$, δ) : 2.42 (3H, s), 3.21 (3H, s), 3.52 (1H, dd, J=18Hz and 4Hz), 3.79 (1H, dd, J=18Hz and 5Hz), 4.68 (2H, s), 5.49 (2H, s), 6.49 (1H, br s), 6.71 (1H, d, J=7Hz), 6.86 (1H, t, J=7Hz), 7.00 (1H, s), 7.19 (1H, s), 7.30 (1H, d, J=9Hz), 7.49 (1H, d, J=9Hz), 7.55 (1H, s), 7.78 (1H, d, J=7Hz)

Example 71

To a solution of 3-bromo-8-[2,6-dichloro-3-[N-(N-bromoacetylglycyl)-N-methylamino]benzyloxy]-2-methylimidazo[1,2-a]pyridine (200 mg) in N,N-dimethylformamide (2 ml) was added sodium methanethiolate (35 mg), and the mixture was stirred for 5 hours at ambient temperature under nitrogen atmosphere. The reaction mixture was concentrated in vacuo, the residue was dissolved in methylene chloride. The solution was washed with water four times and brine, dried over magnesium sulfate and concentrated in vacuo. The residue was purified by a silica gel column chromatography (methylene chloride:methanol = 40:1, V/V) to give 3-bromo-8-[2,6-dichloro-3-[N-methyl-N-[N-(methylthioacetyl)glycyl]amino]benzyloxy]-2-methylimidazo[1,2-a]pyridine (105 mg).

NMR ($CDCl_3$, δ) : 2.19 (3H, s), 2.43 (3H, s), 3.21 (2H, s), 3.28 (3H, s), 3.59 (1H, dd, J=18Hz and 4Hz), 3.84 (1H, dd, J=18Hz and 5Hz), 5.50 (2H, s), 6.72 (1H, d, J=7Hz), 6.86 (1H, t, J=7Hz), 7.32 (1H, d, J=9Hz), 7.49 (1H, d, J=9Hz), 7.61 (1H, br s), 7.78 (1H, d, J=7Hz)

Example 72

The following compounds were obtained according to similar manners to those of Examples 36 to 39.

(1) 3-Bromo-8-[2,6-dichloro-3-[N-(N-hexanoylglycyl)-N-methylamino]benzyloxy]-2-methylimidazo[1,2-a]pyridine

mp : 131-132°C
NMR ($CDCl_3$, δ) : 0.89 (3H, t, J=6Hz), 1.19-1.40 (4H), 1.51-1.71 (2H), 2.20 (2H, t, J=6Hz), 2.42 (3H, s), 3.25 (3H, s), 3.51 (1H, dd, J=18Hz and 4Hz), 3.79 (1H, dd, J=18Hz and 5Hz), 5.49 (2H, s), 6.40 (1H, br s), 6.71 (1H, d, J=7Hz), 6.86 (1H, t, J=7Hz), 7.30 (1H, d, J=9Hz), 7.48 (1H, d, J=9Hz), 7.78 (1H, d, J=7Hz)

(2) 3-Bromo-8-[2,6-dichloro-3-[N-methyl-N-[N-(3-phenylpropionyl)glycyl]amino]benzyloxy]-2-methylimidazo[1,2-a]pyridine

NMR ($CDCl_3$, δ) : 2.42 (3H, s), 2.88-3.02 (4H), 3.23 (3H, s), 3.50 (1H, dd, J=18Hz and 4Hz), 3.79 (1H, dd, J=18Hz and 5Hz), 5.49 (2H, s), 6.45 (1H, br s), 6.71 (1H, d, J=7Hz), 6.88 (1H, t, J=7Hz), 7.11-7.33 (5H), 7.46 (1H, d, J=9Hz), 7.78 (1H, d, J=7Hz)

(3) 3-Bromo-8-[2,6-dichloro-3-[N-[N-(N-methyl-N-phenylglycyl)glycyl]-N-methylamino]benzyloxy]-2-methylimidazo[1,2-a]pyridine

NMR ($CDCl_3$, δ) : 2.43 (3H, s), 3.08 (3H, s), 3.22 (3H, s), 3.56 (1H, dd, J=18Hz and 5Hz), 3.82 (1H, dd, J=18Hz and 5Hz), 3.90 (2H, s), 5.50 (2H, s), 6.69-6.91 (5H), 7.20-7.40 (3H), 7.48 (1H, d, J=9Hz), 7.78 (1H, d, J=7Hz)

Example 73

The following compounds were obtained according to a similar manner to that of Example 60.

(1) 3-Bromo-8-[2,6-dichloro-3-[N-methyl-N-[N-(methylthioacetyl)glycyl]amino]benzyloxy]-2-methylimidazo[1,2-a]pyridine hydrochloride

NMR ($CDCl_3$-$CD_3OD$, δ) : 2.19 (3H, s), 2.53 (3H, s), 3.21 (2H, s), 3.27 (3H, s), 3.66 (1H, d, J=18Hz), 3.79 (1H, d, J=18Hz), 5.72 (2H, s), 7.49-7.73 (4H), 8.29 (1H, d, J=6Hz)

(2) 3-Bromo-8-[2,6-dichloro-3-[N-[N-(1-imidazolylacetyl)glycyl]-N-methylamino]benzyloxy]-2-methylimidazo[1,2-a]pyridine dihydrochloride

NMR ($CDCl_3$-$CD_3OD$, δ) : 2.52 (3H, s), 3.26 (3H, s), 3.63 (1H, d, J=18Hz), 3.83 (1H, d, J=18Hz), 5.12 (2H, s), 5.71 (2H, s), 7.48-7.72 (6H), 8.29 (1H, d, J=6Hz), 9.00 (1H, s)

(3) 3-Bromo-8-[2,6-dichloro-3-[N-(N-hexanoylglycyl)-N-methylamino]benzyloxy]-2-methylimidazo[1,2-a]pyridine hydrochloride

NMR ($CDCl_3$-$CD_3OD$, δ) : 0.90 (3H, t, J=6Hz), 1.21-1.42 (4H), 1.52-1.71 (2H), 2.25 (2H, t, J=6Hz), 2.56 (3H, s), 3.27 (3H, s), 3.62 (1H, d, J=16Hz), 3.74 (1H, d, J=16Hz), 5.70 (1H, d, J=10Hz), 5.78 (1H, d, J=10Hz), 7.53-7.76 (4H), 8.30 (1H, d, J=6Hz)

(4) 3-Bromo-8-[2,6-dichloro-3-[N-methyl-N-[N-(3-phenylpropionyl)glycyl]amino]benzyloxy]-2-methylimidazo[1,2-a]pyridine hydrochloride

NMR ($CDCl_3$-$CD_3OD$, δ) : 2.53 (3H, s), 2.83-3.00 (4H), 3.22 (3H, s), 3.18 (2H, s), 5.71 (2H, s), 7.10-7.30 (5H), 7.55-7.76 (4H), 8.30 (1H, d, J=6Hz)

(5) 3-Bromo-8-[2,6-dichloro-3-[N-[N-(N,N-diethylglycyl)glycyl]-N-methylamino]benzyloxy]-2-methylimidazo[1,2-a]pyridine dihydrochloride

NMR ($CDCl_3$-$CD_3OD$, δ) : 1.40 (6H, t, J=7Hz), 2.63 (3H, s), 3.25 (3H, s), 3.28-3.49 (4H), 3.60-4.09 (4H), 5.61 (1H, d, J=10Hz), 5.70 (1H, d, J=10Hz), 7.40-7.62 (4H), 8.11 (1H, d, J=6Hz)

(6) 3-Bromo-8-[2,6-dichloro-3-[N-[N-(N-ethyl-N-methylglycyl)glycyl]-N-methylamino]benzyloxy]-2-methylimidazo[1,2-a]pyridine dihydrochloride

NMR ($CDCl_3$-$CD_3OD$, δ) : 1.40 (3H, t, J=7Hz), 2.45-2.90 (5H), 2.95 (3H, s), 3.21 (3H s), 3.61-4.01 (4H), 5.63 (3H, br s), 7.38-7.64 (4H), 8.08 (1H, d, J=6Hz)

(7) 3-Bromo-8-[3-[N-[N-(N-cyclopropylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 0.84-1.03 (4H), 2.59 (3H, s), 2.79 (1H, m), 3.25 (3H, s), 3.58-3.71 (2H), 3.80-4.00 (3H), 5.73 (2H, s), 7.56-7.75 (4H), 8.30 (1H, d, J=6Hz)

(8) 3-Bromo-8-[3-[N-[N-(N-cyclohexylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.16-1.51 (5H), 1.68-2.20 (5H), 2.58 (3H, s), 3.08 (1H, m), 3.26 (3H, s), 3.56-3.71 (2H), 3.80-3.92 (3H), 5.72 (2H, s), 7.48-7.74 (4H), 8.30 (1H, d, J=6Hz)

(9) 3-Bromo-8-[2,6-dichloro-3-[N-methyl-N-[N-(1-Pyrrolidinylacetyl)glycyl]amino]benzyloxy]-2-methylimidazo[1,2-a]pyridine dihydrochloride

NMR ($CDCl_3$-$CD_3OD$, δ) : 1.97-2.29 (4H), 2.58 (3H, s), 3.09-3.30 (5H), 3.53-3.91 (4H), 4.09 (2H, s), 5.71 (2H, s), 7.53-7.76 (4H), 8.30 (1H, d, J=6Hz)

(10) 3-Bromo-8-[2,6-dichloro-3-[N-methyl-N-[N-(piperidinoacetyl)glycyl]amino]benzyloxy]-2-methylimidazo[1,2-a]pyridine dihydrochloride

NMR ($CDCl_3$-$CD_3OD$, δ) : 1.42-2.09 (6H), 2.58 (3H, s), 2.98-3.19 (2H), 3.28 (3H, s), 3.50-3.71 (4H), 3.80-4.04 (2H), 5.72 (2H, s), 7.55-7.76 (4H), 8.30 (1H, d, J=6Hz)

(11) 3-Bromo-8-[2,6-dichloro-3-[N-methyl-N-[N-[[4-(4-pyridyl)-1-piperazinyl]acetyl]glycyl]amino]benzyloxy]-2-methylimidazo[1,2-a]pyridine tetrahydrochloride

NMR ($CDCl_3$-$CD_3OD$, δ) : 2.59 (3H, s), 3.28 (3H, s), 3.58-3.72 (5H), 3.89 (1H, d, J=18Hz), 4.02-4.25 (4H), 5.72 (2H, s), 7.30 (2H, d, J=7Hz), 7.55-7.76 (4H), 8.21 (2H, d, J=7Hz), 8.30 (1H, d, J=6Hz)

(12) 3-Bromo-8-[2,6-dichloro-3-[N-[N-(N-isopropyl-N-methylglycyl)glycyl]-N-methylamino]benzyloxy]-2-methylimidazo[1,2-a]pyridine dihydrochloride

NMR ($CDCl_3$-$CD_3OD$, δ) : 1.40 (6H, d, J=6Hz), 2.56 (3H, s), 2.85 (3H, s), 3.25 (3H, s), 3.59-3.71 (3H), 3.90 (1H, d, J=18Hz), 4.01 (1H, m), 5.72 (2H, s), 7.48-7.78 (4H), 8.30 (1H, d, J=6Hz)

(13) 3-Bromo-8-[3-[N-[N-(N-cyclohexyl-N-methylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.43-1.90 (8H), 2.01-2.19 (2H), 2.56 (3H, s), 3.26 (3H, s), 3.31-3.41 (4H), 3.62 (1H, d, J=18Hz), 3.80-3.96 (3H), 5.72 (2H, s), 7.50-7.72 (4H), 8.29 (1H, d, J=6Hz)

(14) 3-Bromo-8-[3-[N-[N-(N-cycloheptylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.25-2.20 (12H), 2.59 (3H, s), 2.89 (3H, s), 3.26 (3H, s), 3.31-3.41 (2H), 3.62 (1H, d, J=18Hz), 3.90 (1H, d, J=18Hz), 4.09 (1H, m), 5.73 (2H, s), 7.56-7.75 (4H), 8.30 (1H, d, J=6Hz)

(15) 3-Bromo-8-[3-[N-[N-(N-cyclopentylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.59-1.93 (6H), 2.00-2.23 (2H), 2.56 (3H, s), 3.26 (3H, s), 3.49-3.67 (2H), 3.80-3.92 (3H), 5.72 (2H, s), 7.50-7.74 (4H), 8.30 (1H, d, J=6Hz)

(16) 3-Bromo-8-[3-[N-[N-(N-cyclooctylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.31-2.10 (14H), 2.63 (3H, s), 3.23 (3H, s), 3.30 (1H, m), 3.71 (1H, d, J=18Hz), 3.81 (2H, s), 3.90 (1H, d, J=18Hz), 5.65 (2H, s), 7.40-7.66 (4H), 8.09 (1H, d, J=6Hz)

(17) 3-Bromo-8-[3-[N-[N-(N-cycloheptyl-N-methylglycyl)glycyl]-N-methylamino]-2,6-dichlorobenzyloxy]-2-methylimidazo[1,2-a]pyridine dihydrochloride

NMR ($CDCl_3$-$CD_3OD$, δ) : 1.42-1.90 (10H), 2.05-2.30 (2H), 2.65 (3H, s), 2.88 (3H, s), 3.24 (3H, s), 3.50-3.72 (2H), 3.80-4.10 (3H), 5.61 (1H, d, J=10Hz), 5.71 (1H, d, J=10Hz), 7.42-7.62 (4H), 8.10 (1H, d, J=6Hz)

(18) 3-Bromo-8-[2,6-dichloro-3-[N-[N-(hexamethyleneiminoacetyl)glycyl]-N-methylamino]benzyloxy]-2-methylimidazo[1,2-a]pyridine dihydrochloride

NMR ($CDCl_3$-$CD_3OD$, δ) : 1.42-2.20 (8H), 2.66 (3H, s), 3.22 (3H, s), 3.30-3.82 (6H), 3.90-4.08 (2H), 5.56 (1H, d, J=10Hz), 5.69 (1H, d, J=10Hz), 7.20-7.50 (3H), 7.59 (1H, d, J=9Hz), 8.00 (1H, d, J=6Hz)

(19) 8-[3-[N-[N-[N-(1-Adamantyl)glycyl]glycyl]-N-methylamino]-2,6-dichlorobenzyloxy]-3-bromo-2-methylimidazo[1,2-a]pyridine dihydrochloride

NMR ($CDCl_3$-$CD_3OD$, δ) : 1.56-2.35 (15H), 2.65 (3H, s), 3.22 (3H, s), 3.66-3.99 (4H), 5.60 (1H, d, J=10Hz), 5.70 (1H, d, J=10Hz), 7.31-7.54 (3H), 7.62 (1H, d, J=9Hz), 8.02 (1H, d, J=6Hz)

(20) 3-Bromo-8-[2,6-dichloro-3-[N-methyl-N-[N-[N-(piperidino)glycyl]glycyl]amino]benzyloxy]-2-methylimidazo[1,2-a]pyridine trihydrochloride

NMR ($CDCl_3$-$CD_3OD$, δ) : 1.52-2.40 (6H), 2.68 (3H, s), 3.22 (3H, s), 3.51-3.99 (6H), 4.59 (1H, d, J=11Hz), 4.70 (1H, d, J=11Hz), 5.67 (2H, s), 7.31-7.50 (2H), 7.55 (1H, d, J=9Hz), 7.67 (1H, d, J=9Hz), 8.06 (1H, d, J=6Hz)

(21) 3-Bromo-8-[2,6-dichloro-3-[N-[N-[N-(2-furylmethyl)glycyl]glycyl]-N-methylamino]benzyloxy]-2-methylimidazo[1,2-a]pyridine dihydrochloride

NMR ($CDCl_3$-$CD_3OD$, δ) : 2.62 (3H, s), 3.22 (3H, s), 3.70-3.99 (4H), 4.30 (2H, s), 5.60 (1H, d, J=10Hz), 5.70 (1H, d, J=10Hz), 6.39 (1H, m), 6.69 (1H, d, J=3Hz), 7.37-7.62 (5H), 8.08 (1H, d, J=6Hz)

(22) 3-Bromo-8-[2,6-dichloro-3-[N-[N'-(3-methoxyphenyl)ureidoacetyl]-N-methylamino]benzyloxy]-2-methylimidazo[1,2-a]pyridine hydrochloride

NMR ($CDCl_3$-$CD_3OD$, δ) : 2.51 (3H, s), 3.28 (3H, s), 3.71 (2H, s), 3.76 (3H, s), 5.70 (1H, d, J=10Hz), 5.79 (1H, d, J=10Hz), 6.56 (1H, dd, J=9Hz and 1Hz), 6.81 (1H, dd, J=9Hz and 1Hz), 7.06 (1H, t, J=1Hz), 7.12 (1H, t, J=9Hz), 7.50-7.78 (4H), 8.30 (1H, d, J=6Hz)

(23) 3-Bromo-8-[3-[N-[N'-(3-chlorophenyl)ureidoacetyl]-N-methylamino]-2,6-dichlorobenzyloxy]-2-methylimidazo[1,2-a]pyridine hydrochloride

NMR ($CDCl_3$-$CD_3OD$, δ) : 2.55 (3H, s), 3.29 (3H, s), 3.95 (1H, d, J=18Hz), 4.16 (1H, d, J=18Hz), 5.77 (2H, s), 7.18 (1H, m), 7.31 (2H, d, J=5Hz), 7.50-7.72 (5H), 8.29 (1H, d, J=6Hz)

(24) 3-Bromo-8-[2,6-dichloro-3-[N-[N'-(3-trifluoromethylphenyl)ureidoacetyl]-N-methylamino]benzyloxy]-2-methylimidazo[1,2-a]pyridine hydrochloride

NMR ($CDCl_3$-$CD_3OD$, δ) : 2.52 (3H, s), 3.30 (3H, s), 3.72 (2H, s), 5.69 (1H, d, J=10Hz), 5.79 (1H, d, J=10Hz), 7.23 (1H, m), 7.39 (2H, d, J=5Hz), 7.50-7.73 (4H), 7.89 (1H, br s), 8.28 (1H, d, J=6Hz)

(25) 8-[3-[N-[N'-(3-Acetylphenyl)ureidoacetyl]-N-methylamino]-2,6-dichlorobenzyloxy]-3-bromo-2-methylimidazo[1,2-a]pyridine hydrochloride

NMR ($CDCl_3$-$CD_3OD$, δ) : 2.54 (3H, s), 2.60 (3H, s), 3.30 (3H, s), 3.72 (2H, br s), 5.70 (1H, d, J=10Hz), 5.80 (1H, d, J=10Hz), 7.38 (1H, t, J=9Hz), 7.49-7.72 (6H), 8.04 (1H, br s), 8.29 (1H, d, J=6Hz)

(26) 3-Bromo-8-[3-[N-[N'-(3-cyanophenyl)ureidoacetyl]-N-methylamino]-2,6-dichlorobenzyloxy]-2-methylimidazo[1,2-a]pyridine hydrochloride

NMR ($CDCl_3$-$CD_3OD$, δ) : 2.58 (3H, s), 3.29 (3H, s), 3.72 (2H, s), 5.69 (1H, d, J=10Hz), 5.79 (1H, d, J=10Hz), 7.25-7.72 (7H), 7.97 (1H, br s), 8.28 (1H, d, J=7Hz)

(27) 3-Bromo-8-[2,6-dichloro-3-[N-[N'-(o-tolyl)ureidoacetyl]-N-methylamino]benzyloxy]-2-methylimidazo[1,2-a]pyridine hydrochloride

NMR ($CDCl_3$-$CD_3OD$, δ) : 2.25 (3H, s), 2.49 (3H, s), 3.28 (3H, s), 3.73 (2H, s), 5.68 (1H, d, J=10Hz), 5.78 (1H, d, J=10Hz), 6.95-7.20 (3H), 7.40-7.71 (5H), 8.29 (1H, d, J=6Hz)

(28) 3-Bromo-8-[3-[N-[N'-(3-fluorophenyl)ureidoacetyl]-N-methylamino]-2,6-dichlorobenzyloxy]-2-methylimidazo[1,2-a]pyridine hydrochloride

NMR ($CDCl_3$-$CD_3OD$, δ) : 2.55 (3H, s), 3.29 (3H, s), 3.71 (2H, s), 5.67 (1H, d, J=10Hz), 5.78 (1H, d, J=10Hz), 6.68 (1H, dt, J=8Hz and 1Hz), 6.93 (1H, br d, J=8Hz), 7.20 (1H, dt, J=8Hz and 6Hz), 7.32 (1H, dt, J=10Hz and 1Hz), 7.48-7.71 (4H), 8.24 (1H, d, J=6Hz)

(29) 3-Bromo-8-[3-[N-[N'-(3-ethylphenyl)ureidoacetyl]-N-methylamino]-2,6-dichlorobenzyloxy]-2-methylimidazo[1,2-a]pyridine hydrochloride

NMR ($CDCl_3$-$CD_3OD$, δ) : 1.21 (3H, t, J=7Hz), 2.52 (3H, s), 2.60 (2H, q, J=7Hz), 3.28 (3H, s), 3.71 (2H, s), 5.65 (1H, d, J=10Hz), 5.79 (1H, d, J=10Hz), 6.87 (1H, m), 7.10-7.22 (3H), 7.52-7.71 (4H), 8.25 (1H, d, J=6Hz)

(30) 8-[3-[N-(N'-Benzoylureidoacetyl)-N-methylamino]-2,6-dichlorobenzyloxy]-3-bromo-2-methylimidazo[1,2-a]pyridine hydrochloride

NMR ($CDCl_3$-$CD_3OD$, δ) : 2.62 (3H, s), 3.30 (3H, s), 3.75 (1H, d, J=18Hz), 3.95 (1H, d, J=18Hz), 5.68 (2H, s), 7.38-7.66 (7H), 7.89 (2H, d, J=8Hz), 8.09 (1H, d, J=6Hz)

(31) 3-Bromo-8-[2,6-dichloro-3-[N-methyl-N-[N'-(3-nitrophenyl)ureidoacetyl]amino]benzyloxy]-2-methylimidazo[1,2-a]pyridine hydrochloride

NMR ($CDCl_3$-$CD_3OD$, δ) : 2.63 (3H, s), 3.25 (3H, s), 3.92 (2H, s), 5.59 (1H, d, J=10Hz), 5.68 (1H, d, J=10Hz), 7.29-7.58 (5H), 7.70 (1H, dd, J=7Hz and 1Hz), 7.79 (1H, d, J=7Hz), 8.10 (1H, d, J=6Hz), 8.19 (1H, br s)

(32) 3-Bromo-8-[2,6-dichloro-3-[N-[N'-(3-ethoxycarbonylphenyl)ureidoacetyl]-N-methylamino]benzyloxy]-2-methylimidazo[1,2-a]pyridine hydrochloride

NMR ($CDCl_3$-$CD_3OD$, δ) : 1.30 (3H, t, J=7Hz), 2.62 (3H, s), 3.21 (3H, s), 3.99 (2H, s), 4.20 (2H, q, J=7Hz), 5.58 (2H, s), 7.12-7.52 (6H), 7.62-7.80 (2H), 8.01 (1H, d, J=6Hz)

(33) 3-Bromo-8-[2,6-dichloro-3-[N-(N'-ethoxycarbonylmethylureidoacetyl)-N-methylamino]benzyloxy]-2-methylimidazo[1,2-a]pyridine hydrochloride

NMR ($CDCl_3$, δ) : 2.57 (3H, s), 3.28 (3H, s), 3.68 (2H, s), 3.89 (2H, s), 5.68 (1H, d, J=10Hz), 5.78 (1H, d, J=10Hz), 7.57-7.72 (4H), 8.29 (1H, d, J=6Hz)

(34) 3-Bromo-8-[3-[N-[N'-[3-(N,N-dimethylcarbamoyl)phenyl]ureidoacetyl]-N-methylamino]-2,6-dichlorobenzyloxy]-2-methylimidazo[1,2-a]pyridine hydrochloride

NMR ($CDCl_3$-$CD_3OD$, δ) : 2.56 (3H, s), 3.01 (3H, br s), 3.11 (3H, br s), 3.30 (3H, s), 3.73 (2H, s), 5.68 (1H, d, J=10Hz), 5.79 (1H, d, J=10Hz), 7.00 (1H, d, J=8Hz), 7.30 (1H, t, J=8Hz), 7.38-7.70 (6H), 8.24 (1H, d, J=6Hz)

(35) 3-Bromo-8-[2,6-dichloro-3-[N-[N-[3-(N-methylcarbamoyl)propionyl]glycyl]-N-methylamino]benzyloxy]-2-methylimidazo[1,2-a]pyridine hydrochloride

NMR ($CDCl_3$-$CD_3OD$, δ) : 2.25-2.70 (5H), 2.80 (3H, s), 3.26 (3H, s), 3.72 (1H, d, J=18Hz), 3.89 (1H, d, J=18Hz), 5.60 (1H, d, J=10Hz), 5.70 (1H, d, J=10Hz), 7.38-7.61 (4H), 8.09 (1H, d, J=6Hz)

(36) 3-Bromo-8-[2,6-dichloro-3-[N-[N-(N-methyl-N-phenylglycyl)glycyl]-N-methylamino]benzyloxy]-2-methylimidazo[1,2-a]pyridine dihydrochloride

NMR ($CDCl_3$-$CD_3OD$, δ) : 2.69 (3H, s), 3.21 (3H, s), 3.29 (3H, s), 3.64 (1H, d, J=17Hz), 3.81 (1H, d, J=17Hz), 4.20 (1H, d, J=16Hz), 4.32 (1H, d, J=16Hz), 5.62 (2H, s), 7.20-7.61 (9H), 8.06 (1H, d, J=6Hz)

(37) 3-Bromo-8-[2,6-dichloro-3-[N-[N'-(2-thienyl)ureidoacetyl]-N-methylamino]benzyloxy]-2-methylimidazo[1,2-a]pyridine hydrochloride

NMR ($CDCl_3$-$CD_3OD$, δ) : 2.63 (3H, s), 3.30 (3H, s), 3.81 (1H, d, J=17Hz), 3.93 (1H, d, J=17Hz), 5.62 (1H, d, J=10Hz), 5.72 (1H, d, J=10Hz), 7.09 (1H, dd, J=5Hz and 4Hz), 7.45-7.59 (5H), 7.69 (1H, d, J=4Hz), 8.09 (1H, dd, J=5Hz and 1Hz)

(38) 3-Bromo-8-[3-[N-[N'-(3-carboxyphenyl)ureidoacetyl]-N-methylamino]-2,6-dichlorobenzyloxy]-2-methylimidazo[1,2-a]pyridine hydrochloride

NMR ($CDCl_3$-$CD_3OD$, δ) : 2.52 (3H, s), 3.29 (3H, s), 3.75 (2H, s), 5.68 (1H, d, J=10Hz), 5.79 (1H, d, J=10Hz), 7.32 (1H, t, J=8Hz), 7.49-7.72 (6H), 8.05 (1H, br s), 8.28 (1H, d, J=6Hz)

(39) 3-Chloro-8-[2,6-dichloro-3-[N-[N-(N,N-dimethylglycyl)glycyl]-N-methylamino]benzyloxy]-2-methylimidazo[1,2-a]pyridine dihydrochloride

NMR ($CDCl_3$-$CD_3OD$, δ) : 2.65 (3H, s), 2.96 (3H, s), 2.99 (3H, s), 3.22 (3H, s), 3.62-4.04 (4H), 5.68 (2H, s), 7.38-7.68 (4H), 8.08 (1H, d, J=6Hz)

(40) 8-[2,6-Dichloro-3-[N-[N-(N,N-dimethylglycyl)glycyl]-N-methylamino]benzyloxy]-3-iodo-2-methylimidazo[1,2-a]pyridine dihydrochloride

NMR ($CDCl_3$-$CD_3OD$, δ) : 2.58 (3H, s), 2.97 (6H, s), 3.27 (3H, s), 3.61 (1H, d, J=18Hz), 3.88 (1H, d, J=18Hz), 4.01 (2H, s), 5.72 (2H, s), 7.50-7.75 (4H), 8.28 (1H, d, J=6Hz)

(41) 3-Bromo-8-[2,6-dichloro-4-(N-acetyl-N-methylamino)benzyloxy]-2-methylimidazo[1,2-a]pyridine hydrochloride

mp : 118-121°C

NMR ($DMSO-d_6$, δ) : 2.03 (3H, br s), 2.38 (3H, s), 3.25 (3H, s), 5.51 (2H, s), 7.36 (1H, t, J=7Hz), 7.50 (1H, d, J=7Hz), 7.71 (2H, s), 8.20 (1H, d, J=7Hz)

(42) 3-Chloro-8-[2,6-dichloro-4-(N-acetyl-N-methylamino)benzyloxy]-2-methylimidazo[1,2-a]pyridine hydrochloride

NMR ($DMSO-d_6$, δ) : 2.01 (3H, br s), 2.38 (3H, s), 3.25 (3H, s), 5.50 (2H, s), 7.35 (1H, t, J=7Hz), 7.50 (1H, d, J=7Hz), 7.72 (2H, s), 8.22 (1H, d, J=7Hz)

Preparation 34

To a stirred two-phase solution of 3-nitrobenzoyl chloride (9.3 g) in a mixture of diethyl ether (50 ml) and saturated sodium bicarbonate solution (50 ml) was added 3-aminomethylpyridine (5.4 g) in an ice-cooled bath. The mixture was stirred vigorously at ambient temperature for 30 minutes. The reaction mixture was filtered, and the resulting solid was washed with water. The solid was further solidified with diisopropyl alcohol-water to afford 3-nitro-N-(3-pyridylmethyl)benzamide (5.91 g) as a pale yellow amorphous solid.

NMR ($CDCl_3$, δ) : 4.70 (2H, d, J=5Hz), 7.05 (1H, br s), 7.30 (1H, dd, J=7, 5Hz), 7.68 (1H, t, J=9Hz), 7.76 (1H, dt, J=8, 0.5Hz), 8.22 (1H, d, J=8Hz), 8.39 (1H, m), 8.54 (1H, dd, J=5, 0.5Hz), 8.60 (1H, d, J=0.5Hz), 8.65 (1H, t, J=0.5Hz)

Preparation 35

To a solution of N,N-bis(2-methoxyethyl)amine (2.40 g) and triethylamine (2.27 g) in dichloromethane (30 ml) was added 3-nitrobenzoyl chloride (2.78 g) in an ice-water bath. The mixture was stirred at ambient temperature for 1 hour. The reaction mixture was washed with saturated sodium bicarbonate solution, water and brine, dried over anhydrous magnesium sulfate, and evaporated in vacuo. The residue was purified with column chromatography eluting with dichloromethane-methanol to give N,N-bis(2-methoxyethyl)-3-nitrobenzamide (4.12 g) as an oil.

NMR ($CDCl_3$, δ) : 3.22-3.88 (14H), 7.59 (1H, t, J=8Hz), 7.80 (1H, dt, J=8, 1Hz), 8.26 (1H, dt, J=8, 1Hz), 8.39 (1H, t, J=1Hz)

Preparation 36

The following compounds were obtained according to similar manners to those of Preparations 34 or 35.

(1) 3-Nitro-N-(4-pyridyl)benzamide

mp : >250°C
NMR (DMSO-$d_6$, δ) : 7.80 (2H, d, J=6Hz), 7.89 (1H, t, J=7Hz), 8.38-8.58 (4H), 8.80 (1H, t, J=1Hz)

(2) 4-Methyl-1-(3-nitrobenzoyl)piperazine

mp : 97-98°C
NMR ($CDCl_3$, δ) : 2.31-2.66 (7H), 3.38-3.97 (4H), 7.62 (1H, dt, J=8, 1Hz), 7.78 (1H, dt, J=1, 8Hz), 8.25-8.34 (2H)

(3) 1-(3-Nitrobenzoyl)pyrrolidine

mp : 67°C
NMR ($CDCl_3$, δ) : 1.85-2.10 (4H, m), 3.45 (2H, t, J=6Hz), 3.69 (2H, t, J=6Hz), 7.61 (1H, t, J=8Hz), 7.89 (1H, dif-ddd, J=8Hz), 8.29 (1H, dif-ddd, J=8Hz), 8.40 (1H, dif-dd)

Preparation 37

To a stirred solution of 3-nitro-N-(3-pyridylmethyl)benzamide (2.00 g) in tetrahydrofuran (40 ml) was added potassium tert-butoxide (917 mg) in one portion in an ice-cooled bath. The stirring was continued for 40 minutes and then iodomethane (0.53 ml) was added thereto. The reaction mixture was stirred at 0°C for one hour, then at ambient temperature for five hours. Saturated sodium bicarbonate solution was added thereto and the mixture was extracted with ethyl acetate. The organic layer was washed with water and saturated sodium chloride solution. After dried over anhydrous magnesium sulfate and filtered, the solvent was removed in vacuo and the residue was purified by flash chromatography (methanol-chloroform 3%, V/V) to afford 3-nitro-N-methyl-N-(3-pyridylmethyl)benzamide (1.8 g) as an yellow oil.

NMR ($CDCl_3$, δ) : 2.80-3.22 (3H, m), 4.40-4.93 (2H, m), 7.30-7.42 (1H, m), 7.44-7.90 (3H, m), 8.24-8.37 (2H, m), 8.40-8.75 (2H, m)

Preparation 38

Ethyl 4-[N-(3-pyridylmethyl)acetamido]cinnamate was obtained by reacting ethyl 4-acetamidocinnamate with 3-pyridylmethyl chloride according to a similar manner to that of Preparation 37.

NMR ($CDCl_3$, δ) : 1.34 (3H, t, J=7Hz), 1.92 (3H, s), 4.29 (2H, q, J=7Hz), 4.90 (2H, s), 6.41 (1H, d, J=15Hz), 7.02 (2H, d, J=7Hz), 7.24 (1H, m), 7.51 (2H, d, J=7Hz), 7.60-7.70 (2H), 8.38 (1H, br s), 8.51 (1H, d, J=3Hz)

Preparation 39

A mixture of N,N-bis(2-methoxyethyl)-3-nitrobenzamide (4.11 g) and palladium on charcoal (411 mg) in ethyl acetate (41 ml) was hydrogenated under 1 atmospheric pressure of hydrogen for 1 hour at ambient temperature. The catalyst was removed by filtration and washed with ethyl acetate, and the volatiles were removed in vacuo. The residue was purified with column chromatography eluting with dichloromethane-methanol to give 3-amino-N,N-bis(2-methoxyethyl)benzamide (3.62 g) as an oil.

NMR ($CDCl_3$, δ) : 3.19-3.86 (16H), 6.62-6.79 (3H), 7.16 (1H, dt, J=8, 1Hz)

Preparation 40

The following compounds were obtained according to a similar manner to that of Preparation 39.

(1) 3-Amino-N-methyl-N-(3-pyridylmethyl)benzamide

NMR ($CDCl_3$, δ) : 2.87 (3H, br s), 3.75 (1H, or 2H, br s), 4.41-4.88 (2H, m), 6.55-6.84 (3H, m), 7.03-7.40 (2H, m), 7.42-7.84 (1H, m), 8.35-8.70 (2H, m)

(2) 3-Amino-N-(4-pyridyl)benzamide

mp : 232-234°C
NMR (DMSO-$d_6$, δ) : 5.39 (2H, br s), 6.79 (1H, br d, J=8Hz), 7.02-7.11 (2H), 7.19 (1H, t, J=8Hz), 7.78 (2H, d, J=7Hz), 8.46 (2H, d, J=7Hz)

(3) 1-(3-Aminobenzoyl)-4-methylpiperazine

mp : 114-116°C
NMR ($CDCl_3$, δ) : 2.28-2.60 (7H), 3.38-3.90 (6H), 6.68-6.79 (3H), 7.68 (1H, t, J=8Hz)

(4) 1-(3-Aminobenzoyl)pyrrolidine

NMR ($CDCl_3$, δ) : 1.75-2.05 (4H, m), 3.40 (2H, t, J=6Hz), 3.60 (2H, t, J=6Hz), 3.72 (2H, br s), 6.71 (1H, dif-ddd, J=8Hz), 6.78-6.89 (2H, m), 7.10 (1H, t, J=8Hz)

(5) 3-Amino-N-(3-pyridylmethyl)benzamide

NMR ($CDCl_3$-$CD_3OD$, δ) : 4.60 (2H, s), 6.82 (1H, dt, J=8, 1Hz), 7.10-7.39 (4H), 7.79 (1H, dt, J=9, 1Hz), 8.45 (1H, dd, J=5, 1Hz), 8.52 (1H, d, J=1Hz)

Preparation 41

To a stirred solution of 3-amino-N,N-bis(2-methoxyethyl)benzamide (1.01 g) in 1,4-dioxane (10 ml) was added 1N sodium hydroxide solution (5.2 ml) and phenyl chloroformate (0.55 ml) successively in an ice-cooled bath. The bath was removed and the reaction mixture was stirred vigorously for 1 hour, during which time phenyl chloroformate (0.25 ml) was further added. The mixture was extracted with dichloromethane and the organic layer was washed with water twice and brine, dried over anhydrous magnesium sulfate, and evaporated in vacuo. The residue was crystallized from diisopropyl ether to give phenyl 3-[N,N-bis(2-methoxyethyl)carbamoyl]phenylcarbamate (1.30 g) as a colorless powder.

mp : 116-118°C
NMR ($CDCl_3$, δ) : 3.19-3.82 (14H), 7.10-7.57 (10H)

Preparation 42

The following compounds were obtained according to a similar manner to that of Preparation 41.

(1) Phenyl 3-[N-(4-pyridyl)carbamoyl]phenylcarbamate

mp : 204-206°C
NMR (DMSO-$d_6$, δ) : 5.39 (1H, br s), 6.71-6.82 (2H), 7.02-7.33 (4H), 7.40-7.81 (4H), 8.09 (1H, br s), 8.41-8.51 (2H), 9.32 (1H, br s)

(2) Phenyl 3-(4-methyl-1-piperazinylcarbonyl)phenylcarbamate

mp : 152-154°C
NMR ($CDCl_3$, δ) : 2.27-2.56 (7H), 3.38-3.91 (4H), 7.10-7.60 (9H)

(3) Phenyl 3-(1-pyrrolidinylcarbonyl)phenylcarbamate

mp : 135-140°C
NMR ($CDCl_3$, δ) : 1.74-2.00 (4H, m), 3.45 (2H, t, J=6Hz), 3.63 (2H, t, J=6Hz), 7.07-7.53 (9H, m), 7.72 (1H, br s)

(4) Phenyl 3-[N-methyl-N-(3-pyridylmethyl)carbamoyl]phenylcarbamate

NMR ($CDCl_3$, δ): 2.90-3.08 (3H), 4.58 (0.5H, br s), 4.76 (1.5H, br s), 7.15-7.80 (13H), 8.58 (1H, d, J=5Hz)

(5) Phenyl 3-[N-(3-pyridylmethyl)carbamoyl]phenylcarbamate

mp : 185-188°C
NMR ($CDCl_3$-$CD_3OD$, δ) : 4.62 (2H, s), 7.11-7.49 (7H), 7.56 (1H, dt, J=8, 1Hz), 7.63-7.80 (2H), 7.84 (1H, t, J=1Hz), 8.49 (1H, dd, J=5, 1Hz), 8.55 (1H, d, J=1Hz)

(6) Phenyl 3-(N,N-dimethylamino)phenylcarbamate

mp : 226-228°C
NMR ($CDCl_3$, δ) : 2.93 (6H, s), 6.49 (1H, d, J=7Hz), 6.65 (1H, d, J=7Hz), 6.87 (1H, br s), 7.03 (1H, br s), 7.12-7.29 (4H), 7.32-7.42 (2H)

Preparation 43

(1) Ethyl 4-(phenoxycarbonylamino)cinnamate was obtained by reacting ethyl 4-aminocinnamate with phenyl chloroformate according to a similar manner to that of Preparation 42.

mp : 136-138°C
NMR ($CDCl_3$, δ) : 1.33 (3H, t, J=7Hz), 4.27 (2H, q, J=7Hz), 6.39 (1H, d, J=15Hz), 7.09 (1H, br s), 7.15-7.58 (9H), 7.65 (1H, d, J=15Hz)

(2) A solution of ethyl 4-(phenoxycarbonylamino)cinnamate (500 mg) 3-aminopyridine (154 mg) and triethylamine (325 mg) in N,N-dimethylformamide (5 ml) was stirred for 2 hours at 80°C. Water was added thereto, and the resulting precipitate was collected by filtration to give ethyl 4-[3-(3-pyridyl)ureido]cinnamate (307 mg) as a colorless powder.

mp : 188-189°C
NMR (DMSO-$d_6$, δ) : 1.26 (3H, t, J=7Hz), 4.19 (2H, q, J=7Hz), 6.50 (1H, d, J=15Hz), 7.34 (1H, dd, J=9, 5Hz), 7.46-7.72 (5H), 7.96 (1H, dt, J=9, 1Hz), 8.21 (1H, dd, J=9, 1Hz), 8.62 (1H, d, J=1Hz), 8.98 (1H, br s), 9.10 (1H, m)

Preparation 44

To a mixture of ethyl 4-aminocinnamate (300 mg), triethylamine (167 mg) and dichloromethane (3 ml) was added a solution of propionyl chloride (182 mg) in dichloromethane (1 ml) in an ice-water bath, and the mixture was stirred for 1 hour at the same temperature. To the reaction mixture was added 4 drops of N,N-dimethylpropanediamine, and the mixture was further stirred for 5 minutes. The reaction mixture was washed with water, dried over magnesium sulfate, and evaporated in vacuo. The residue was crystallized from diisopropyl ether to give ethyl 4-propionamidocinnamate (341 mg) as a colorless powder.

mp : 138°C
NMR ($CDCl_3$, δ) : 1.26 (3H, t, J=8Hz), 1.34 (3H, t, J=8Hz), 2.42 (2H, q, J=8Hz), 4.26 (2H, q, J=8Hz), 6.37 (1H, d, J=16Hz), 7.21 (1H, br s), 7.49 (2H, d, J=8Hz), 7.58 (2H, d, J=8Hz), 7.68 (1H, d, J=16Hz)

Preparation 45

To a solution of ethyl 4-aminocinnamate (2.00 g) and methoxyacetic acid (1.04 ml) in N,N-dimethylformamide (20 ml) were added 1-ethyl-3-(3-dimethylaminopropyl)carbodiimide hydrochloride (2.61 g) and 1-hydroxybenzotriazole (2.12 g) at ambient temperature, and the mixture was stirred for 1 hour at the same temperature. The reaction mixture was poured into water, and extracted with dichloromethane. The organic layer was washed with aqueous sodium bicarbonate solution and water, dried over magnesium sulfate, and evaporated in vacuo. The residue was crystallized from diisopropyl ether to give ethyl 4-(methoxyacetamido)cinnamate (2.34 g) as a pale yellow powder.

mp : 92.2°C

NMR ($CDCl_3$, δ) : 1.34 (3H, t, J=7.5Hz), 3.52 (3H, s), 4.03 (2H, s), 4.26 (2H, q, J=7.5Hz), 6.88 (1H, d, J=16Hz), 7.50 (2H, d, J=9Hz), 7.62 (2H, d, J=9Hz), 7.65 (1H, d, J=16Hz), 8.34 (1H, br s)

Preparation 46

The following compounds were obtained according to similar manners to those of Preparations 44 or 45.

(1) Ethyl 4-(4-bromobutyramido)cinnamate

mp : 119-124°C

NMR ($CDCl_3$, δ) : 1.32 (3H, t, J=7.5Hz), 2.21 (2H, quint, J=6Hz), 2.59 (2H, t, J=6Hz), 3.66 (2H, t, J=6Hz), 4.25 (2H, q, J=7.5Hz), 6.34 (1H, d, J=16Hz), 7.47 (2H, d, J=8Hz), 7.55 (2H, d, J=8Hz), 7.61 (1H, d, J=16Hz)

(2) Ethyl 4-(methoxyacetamido)cinnamate

mp : 87-92°C

NMR ($CDCl_3$, δ) : 1.34 (3H, t, J=7.5Hz), 3.53 (3H, s), 4.03 (2H, s), 4.26 (2H, q, J=7.5Hz), 6.37 (1H, d, J=16Hz), 7.50 (2H, d, J=8Hz), 7.63 (2H, d, J=8Hz), 7.65 (1H, d, J=16Hz), 8.35 (1H, br s)

(3) Ethyl 4-(isonicotinoylamino)cinnamate

mp : 179-188°C

NMR ($CDCl_3$, δ) : 1.34 (3H, t, J=7.5Hz), 4.26 (2H, q, J=7.5Hz), 6.40 (1H, d, J=16Hz), 7.52 (2H, d, J=9Hz), 7.57 (1H, d, J=16Hz), 7.65-7.78 (4H), 8.19 (1H, br s), 8.31 (2H, dd, J=6, 0.5Hz)

(4) Ethyl 4-(morpholinocarbonylamino)cinnamate

mp : 170-173°C

NMR ($CDCl_3$, δ) ; 1.33 (3H, t, J=7Hz), 3.43-3.56 (4H), 3.70-3.81 (4H), 4.28 (2H, q, J=7Hz), 6.35 (1H, d, J=15Hz), 6.49 (1H, br s), 7.40 (2H, d, J=9Hz), 7.48 (2H, d, J=9Hz), 7.63 (1H, d, J=15Hz)

(5) Ethyl 4-(5-bromovaleramido)cinnamate

mp : 124.0°C-134.7°C

NMR ($CDCl_3$, δ) : 1.33 (3H, t, J=7.5Hz), 1.79-2.06 (4H, m), 2.42 (2H, t, J=6Hz), 3.44 (2H, t, J=6Hz), 4.26 (2H, q, J=7.5Hz), 6.36 (1H, d, J=16Hz), 7.30 (1H, br s), 7.49 (2H, d, J=8Hz), 7.57 (2H, d, J=8Hz), 7.64 (1H, d, J=16Hz)

Preparation 47

To a solution of methyl 4-carboxycinnamate (160 mg) in methylene chloride was added methylamine hydrochloride (58 mg) and 1-ethyl-3-(3-dimethylaminopropyl)carbodiimide (140 mg) at ambient temperature, and the mixture was stirred for 2 hours. To this suspension was added 1-hydroxybenzotriazole (137 mg) and dimethylformamide (2 ml), and the mixture was stirred for 14 hours at same temperature. The reaction mixture was poured into water, and extracted with dichloromethane. The organic layer was washed with aqueous sodium bicarbonate solution and water, dried over magnesium sulfate, and evaporated in vacuo. The residue was crystallized from diisopropyl ether to give methyl 4-(methylcarbamoyl)cinnamate (82 mg) as a colorless powder.

mp : 210.5°C
NMR (DMSO-$d_6$, δ) : 2.79 (3H, d, J=5Hz), 3.74 (3H, s), 6.74 (1H, d, J=16Hz), 7.69 (1H, d, J=16Hz), 7.80 (2H, d, J=8Hz), 7.87 (2H, d, J=8Hz), 8.51 (1H, q-like)

Preparation 48

To a stirred suspension of methyl 4-carboxycinnamate (400 mg) in thionyl chloride (1.4 ml) was added one drop of N,N-dimethylformamide. The mixture was refluxed for 20 minutes. The solvent was removed in vacuo. To the residue was added toluene (2 ml) and the mixture was evaporated in vacuo twice. The residue was dissolved with dichloromethane (4 ml), and 4-aminopyridine (201 mg) and triethylamine (0.81 ml) were added thereto in an ice-water bath. After 10 minutes the mixture was stirred at ambient temperature. After 3 hours, to the reaction mixture was added water and the mixture was extracted with dichloromethane-methanol (5:1, V/V). The organic layer was washed with saturated sodium bicarbonate solution, water and brine, and dried over anhydrous magnesium sulfate. The solvent was evaporated in vacuo. The residue was crystallized from ethyl acetate to give methyl 4-[N-(4-pyridyl)carbamoyl]cinnamate (555 mg) as a colorless powder.

mp : 209-211°C
NMR (DMSO-$d_6$, δ) : 3.76 (3H, s), 6.82 (1H, d, J=15Hz), 7.69-7.83 (3H), 7.92 (2H, d, J=9Hz), 8.01 (2H, d, J=9Hz), 8.50 (2H, d, J=7Hz)

Preparation 49

The following compounds were obtained according to similar manners to those of Preparations 47 or 48.

(1) Methyl 4-(N,N-dimethylcarbamoyl)cinnamate

mp : 130°C
NMR ($CDCl_3$, δ) : 3.00 (3H, s), 3.12 (3H, s), 3.83 (3H, s), 6.49 (1H, d, J=16Hz), 7.45 (2H, d, J=8Hz), 7.58 (2H, d, J=8Hz), 7.70 (1H, d, J=16Hz)

(2) Methyl 4-[N-(2-methoxyethyl)carbamoyl]cinnamate

mp : 122-124°C
NMR ($CDCl_3$, δ) : 3.40 (3H, s), 3.53-3.72 (4H), 3.83 (3H, s), 6.45-6.60 (3H), 7.58 (2H, d, J=8Hz), 7.71 (1H, d, J=15Hz), 7.80 (2H, d, J=8Hz)

(3) Methyl 4-[N,N-bis(2-methoxyethyl)carbamoyl]cinnamate

NMR ($CDCl_3$, δ) : 3.21-3.86 (17H), 6.48 (1H, d, J=15Hz), 7.44 (1H, d, J=9Hz), 7.57 (1H, d, J=9Hz), 7.70 (1H, d, J=15Hz)

Preparation 50

To a solution of ethyl 4-aminocinnamate (150 mg) in methylene chloride were added methyl isocyanate (0.06 ml) under nitrogen atmosphere at ambient temperature, and the mixture was stirred for 2 hours at the same temperature. The reaction mixture was poured into the mixture of ethyl acetate and water. The organic layer was washed with water twice, dried over magnesium sulfate, and concentrated in vacuo. The residue was crystallized from diisopropyl ether to give ethyl 4-(3-methylureido)cinnamate (136 mg).

mp : 166°C
NMR (DMSO-$d_6$, δ) : 1.25 (3H, t, J=7.5Hz), 2.64 (3H, d, J=5Hz), 4.17 (2H, q, J=7.5Hz), 6.12 (1H, q, J=5Hz), 6.43 (1H, d, J=16Hz), 7.45 (2H, d, J=8Hz), 7.56 (1H, d, J=16Hz), 7.59 (2H, d, J=8Hz), 8.81 (1H, s)

Preparation 51

To a stirred solution of ethyl 4-(4-bromobutyramido)cinnamate (420 mg) in N,N-dimethylformamide (5 ml) was added potassium carbonate (552 mg) at ambient temperature and the resulting mixture was warmed at 50°C for three hours. The reaction mixture was diluted with ethyl acetate and washed with water and brine. The organic phase was

dried over anhydrous magnesium sulfate, and concentrated in vacuo. The residue was purified by flash column chromatography eluting with chloroform to afford ethyl 4-(2-oxo-1-pyrrolidinyl)cinnamate (281 mg) as a pale yellow solid.

mp : 134°C
NMR ($CDCl_3$, δ) : 1.34 (3H, t, J=7.7Hz), 2.19 (2H, quint, J=7.7Hz), 2.63 (2H, t, J=7.7Hz), 3.88 (2H, t, J=7.7Hz), 4.26 (2H, q, J=7.7Hz), 6.38 (1H, d, J=16Hz), 7.53 (2H, d, J=8Hz), 7.64 (1H, d, J=16Hz), 7.68 (2H, d, J=8Hz)

Preparation 52

Ethyl 4-(2-oxopiperidino)cinnamate was obtained according to a similar manner to that of Preparation 51.

mp : 120.2°C
NMR ($CDCl_3$, δ) : 1.34 (3H, t, J=7.5Hz), 1.83-2.05 (4H, m), 2.56 (2H, dif-t), 3.65 (2H, dif-d), 4.26 (2H, q, J=7.5Hz), 6.40 (1H, d, J=16Hz), 7.29 (2H, d, J=8Hz), 7.54 (2H, d, J=8Hz), 7.65 (1H, d, J=16Hz)

Preparation 53

A solution of ethyl 4-aminocinnamate (1 g) and 2,5-dimethoxytetrahydrofuran (0.677 ml) in toluene (3 ml) and acetic acid (3 ml) was refluxed for 5 hours with removing methanol. After cooling, the mixture was washed with water twice and saturated sodium bicarbonate solution, dried over anhydrous magnesium sulfate. The solvent was evaporated in vacuo. The residue was purified with column chromatography eluting with n-hexane - ethyl acetate to give ethyl 4-(1-pyrrolyl)cinnamate (740 mg) as colorless crystals.

mp : 86-87°C
NMR ($CDCl_3$, δ) : 1.37 (3H, t, J=7Hz), 4.29 (2H, q, J=7Hz), 6.32-6.49 (2H), 7.12 (2H, t, J=1Hz), 7.41 (2H, d, J=9Hz), 7.60 (2H, d, J=9Hz), 7.69 (1H, d, J=16Hz)

Preparation 54

Ethyl 4-(N-methyl-2-methoxyacetamido)cinnamate was obtained according to a similar manner to that of Preparation 37.

NMR ($CDCl_3$, δ) : 1.35 (3H, t, J=7.5Hz), 3.29 (3H, s), 3.35 (3H, s), 3.85 (2H, br s), 4.27 (2H, q, J=7.5Hz), 6.46 (1H, d, J=16Hz), 7.24 (2H, d, J=8Hz), 7.57 (2H, d, J=8Hz), 7.67 (1H, d, J=16Hz)

Preparation 55

To a solution of ethyl 4-propionamidocinnamate (160 mg) in ethanol (5 ml) was added 1N aqueous sodium hydroxide solution (1.5 ml) at ambient temperature. The mixture was stirred at same temperature for 14 hours, and then at 40°C for 2 hours. 1N-hydrochloric acid (1.5 ml) was added to the reaction mixture and evaporated in vacuo. The residue was diluted with 10% methanol-dichloromethane, washed with water, dried over magnesium sulfate and evaporated in vacuo. The residue was crystallized from diisopropyl ether to give 4-propionamidocinnamic acid (115 mg) as a colorless powder.

mp : 243°C
NMR (DMSO-$d_6$, δ) : 1.08 (3H, t, J=8Hz), 2.34 (2H, q, J=8Hz), 6.39 (1H, d, J=16Hz), 7.51 (1H, d, J=16Hz), 7.62 (4H, s-like), 10.07 (1H, s)

Preparation 56

The following compounds were obtained according to a similar manner to that of Preparation 55.

(1) 4-(Methylcarbamoyl)cinnamic acid

mp : >250°C
NMR (DMSO-$d_6$, δ) : 2.78 (3H, d, J=5Hz), 6.62 (1H, d, J=16Hz), 7.61 (1H, d, J=16Hz), 7.77 (2H, d, J=8Hz), 7.85 (2H, d, J=8Hz), 8.51 (1H, q-like)

(2) 4-(N,N-Dimethylcarbamoyl)cinnamic acid

mp : 82°C
NMR (DMSO-$d_6$, δ) : 2.93 (3H, s), 2.97 (3H, s), 6.59 (1H, d, J=16Hz), 7.43 (2H, d, J=8Hz), 7.61 (1H, d, J=16Hz), 7.75 (2H, d, J=8Hz)

(3) 4-(3-Methylureido)cinnamic acid

mp : 234°C
NMR (DMSO-$d_6$, δ) : 2.64 (3H, d, J=5Hz), 6.12 (1H, q, J=5Hz), 6.33 (1H, d, J=16Hz), 7.44 (2H, d, J=8Hz), 7.51 (1H, d, J=16Hz), 7.55 (2H, d, J=8Hz), 8.78 (1H, s)

(4) 4-[N-(2-Methoxyethyl)carbamoyl]cinnamic acid

mp : 207-209°C
NMR (DMSO-$d_6$, δ) : 3.20-3.50 (7H), 6.63 (1H, d, J=15Hz), 7.62 (1H, d, J=15Hz), 7.79 (2H, d, J=8Hz), 7.89 (2H, d, J=8Hz), 8.61 (1H, br s)

(5) 4-[N,N-Bis(2-methoxyethyl)carbamoyl]cinnamic acid

NMR ($CDCl_3$, δ) : 3.21-3.86 (17H), 6.48 (1H, d, J=15Hz), 7.44 (2H, d, J=9Hz), 7.57 (2H, d, J=9Hz), 7.70 (1H, d, J=15Hz)

(6) 4-[N-(4-Pyridyl)carbamoyl]cinnamic acid

mp : >250°C
NMR (DMSO-$d_6$, δ) : 6.69 (1H, d, J=16Hz), 7.52-8.08 (7H), 8.49 (2H, d, J=6Hz)

(7) 4-(2-Oxo-1-pyrrolidinyl)cinnamic acid

mp : >250°C
NMR (DMSO-$d_6$, δ) : 2.06 (2H, quint, J=8Hz), 3.86 (2H, t, J=8Hz), 6.46 (1H, d, J=16Hz), 7.55 (1H, d, J=16Hz), 7.65-7.76 (4H, m)

(8) 4-(Methoxyacetamido)cinnamic acid

mp : 201.5-229°C
NMR ($CDCl_3$, δ) : 4.02 (2H, s), 6.43 (1H, d, J=16Hz), 7.52 (1H, d, J=16Hz), 7.63 (2H, d, J=8Hz), 7.74 (2H, d, J=8Hz), 9.97 (1H, s)

(9) 4-[N-(3-Pyridylmethyl)acetamido]cinnamic acid

mp : 184-186°C
NMR (DMSO-$d_6$, δ) : 1.90 (3H, s), 4.91 (2H, s), 6.52 (1H, d, J=15Hz), 7.21-7.39 (3H), 7.50-7.79 (4H), 8.39 (1H, d, J=2Hz), 8.43 (1H, dd, J=5, 2Hz)

(10) 4-(Isonicotinoylamino)cinnamic acid

mp : 283-290°C
NMR (DMSO-$d_6$, δ) : 6.46 (1H, d, J=16.2Hz), 7.53 (1H, d, J=16.2Hz), 7.68 (2H, d, J=9Hz), 7.79-7.91 (4H), 8.80 (2H, dd, J=6, 0.5Hz)

(11) 4-[3-(3-Pyridyl)ureido]cinnamic acid

mp : 219-221°C
NMR (DMSO-$d_6$, δ) : 6.40 (1H, d, J=15Hz), 7.37 (1H, dd, J=9, 5Hz), 7.47-7.70 (5H), 7.98 (1H, dt, J=9, 1Hz), 8.21 (1H, br d, J=5Hz), 8.62 (1H, d, J=1Hz), 9.03 (1H, s), 9.16 (1H, s)

(12) 4-(Morpholinocarbonylamino)cinnamic acid

mp : 219-221°C
NMR (DMSO-$d_6$, δ) : 3.39-3.49 (4H), 3.58-3.68 (4H), 6.37 (1H, d, J=15Hz), 7.46-7.60 (5H), 8.76 (1H, br s)

(13) 4-(2-Oxopiperidino)cinnamic acid

mp : 235.7-243.2°C
NMR (DMSO-$d_6$, δ) : 1.73-1.97 (4H, m), 2.39 (2H, dif-t), 3.61 (2H, dif-t), 6.51 (1H, d, J=16Hz), 7.34 (2H, d, J=8Hz), 7.59 (1H, d, J=16Hz), 7.70 (2H, d, J=8Hz)

(14) 4-(N-Methyl-2-methoxyacetamido)cinnamic acid

mp : 182.1°C
NMR (DMSO-$d_6$, δ) : 3.17 (3H, s), 3.20 (3H, s), 3.87 (2H, br s), 6.57 (1H, d, J=15Hz), 7.39 (2H, d, J=9Hz), 7.60 (1H, d, J=15Hz), 7.74 (2H, d, J=9Hz)

(15) 4-(1-Pyrrolyl)cinnamic acid

mp : 236-240°C
NMR (DMSO-$d_6$, δ) : 6.30 (2H, t, J=1Hz), 6.54 (1H, d, J=16Hz), 7.48 (2H, t, J=1Hz), 7.53-7.71 (3H), 7.80 (2H, d, J=9Hz)

Preparation 57

To a solution of ethyl 4-aminocinnamate (150 mg) and triethylamine (94 mg) in methylene chloride (3 ml) was added mesyl chloride (0.08 ml) under ice-cooling under nitrogen atmosphere, and the mixture was stirred at ambient temperature for 2 hours. The reaction mixture was poured into water, and extracted with methylene chloride twice. The combined organic layer was washed with water, dried over magnesium sulfate and concentrated to give a residue including ethyl 4-mesylaminocinnamate and ethyl 4-(N,N-dimesylamino)cinnamate. The residue was dissolved in ethanol, and 1N aqueous sodium hydroxide solution (1.5 ml) was added thereto at 40°C. The mixture was stirred at ambient temperature for 2 days, and 1N hydrochloric acid (1.5 ml) was added thereto. The mixture was concentrated in vacuo, and the residue was partitioned between 10% methanol-methylene chloride and water. The organic layer was washed with water, dried over magnesium sulfate and concentrated in vacuo. The residue was purified by preparative thin-layer chromatography (methylene chloride - methanol, 10:1, V/V) to give 4-mesylaminocinnamic acid (49.3 mg).

mp : 218°C
NMR (DMSO-$d_6$, δ) : 3.05 (3H, s), 6.44 (1H, d, J=16Hz), 7.21 (2H, d, J=8Hz), 7.53 (1H, d, J=16Hz), 7.66 (2H, d, J=8Hz)

Preparation 58

4-(N,N-Dimethylcarbamoyl)benzaldehyde was obtained by reacting 4-formylbenzoic acid with dimethylamine hydrochloride according to a similar manner to that of Preparation 47.

mp : 60-67°C
NMR ($CDCl_3$, δ) : 2.97 (3H, s), 3.15 (3H, s), 7.59 (2H, d, J=7.5Hz), 7.94 (2H, d, J=7.5Hz), 10.3 (1H, s)

Preparation 59

A mixture of 2-acetylamino-5-formylpyridine (241 mg) and malonic acid (168 mg) in pyridine (0.12 ml) and ethanol (0.36 ml) was refluxed for 2 hours. After cooling the mixture, the precipitate was collected by filtration, and washed ethyl acetate to give (E)-3-(6-acetylamino-3-pyridyl)acrylic acid (248 mg) as a colorless powder.

mp : 291-292°C
NMR (DMSO-$d_6$, δ) : 2.10 (3H, s), 6.55 (1H, d, J=16Hz), 7.58 (1H, d, J=16Hz), 8.07-8.21 (2H), 8.59 (1H, br s)

Preparation 60

The following compounds were obtained according to a similar manner to that of Preparation 59.

(1) (E)-3-(6-Ethoxycarbonyl-3-pyridyl)acrylic acid (from ethyl 5-formyl-2-pyridinecarboxylate)

mp : 201-202°C
NMR (DMSO-$d_6$, δ) : 1.33 (3H, t, J=7Hz), 4.36 (2H, q, J=7Hz), 6.80 (1H, d, J=16Hz), 7.69 (1H, d, J=16Hz), 8.07 (1H, d, J=9Hz), 8.33 (1H, dd, J=9, 2Hz), 9.00 (1H, d, J=2Hz)

(2) 4-(N,N-Dimethylcarbamoyl)cinnamic acid

NMR ($CDCl_3$, δ) : 2.99 (3H, s), 3.11 (3H, s), 6.49 (1H, d, J=15Hz), 7.46 (2H, d, J=8Hz), 7.59 (2H, d, J=8Hz), 7.76 (1H, d, J=15Hz)

Preparation 61

To a suspension of sodium hydride (60% active, 124 mg) in dimethylformamide (2 ml) at ambient temperature was added ethyl 4-hydroxycinnamate (500 mg) under nitrogen, and the mixture was stirred for 1 hour. 2-Bromoethyl acetate (522 mg) was added to the mixture at same temperature, and the mixture was allowed to stand for 19 hours. The reaction mixture was poured into water and extracted with ethyl acetate. The organic layer was washed with water and brine, dried over magnesium sulfate and evaporated in vacuo. The residue was chromatographed on silica gel eluting with chloroform to give ethyl 4-(2-acetoxyethoxy)cinnamate (716 mg) as an oil.

NMR ($CDCl_3$, δ) : 1.33 (3H, t, J=7.5Hz), 2.11 (3H, s), 4.19 (2H, t, J=6Hz), 4.25 (2H, q, J=7.5Hz), 4.44 (2H, t, J=6Hz), 6.31 (1H, d, J=16Hz), 6.94 (2H, d, J=8Hz), 7.49 (2H, d, J=8Hz), 7.64 (1H, d, J=16Hz)

Preparation 62

4-(2-Hydroxyethoxy)cinnamic acid was obtained from ethyl 4-(2-acetoxyethoxy)cinnamate according to a similar manner to that of Preparation 55.

mp : 194°C
NMR (DMSO-$d_6$, δ) : 3.64-3.79 (2H, br peak), 4.02 (2H, t, J=6Hz), 4.90 (1H, br peak), 6.37 (1H, d, J=16Hz), 6.98 (2H, d, J=8Hz), 7.54 (1H, d, J=16Hz), 7.63 (2H, d, J=8Hz)

Example 74

The following compounds were obtained according to similar manners to those of Examples 53 or 69.

(1) 3-Bromo-8-[2,6-dichloro-3-[N-methyl-N-[(thiomorpholinoacetyl)glycyl]amino]benzyloxy]-2-methylimidazo[1,2-a]pyridine

NMR ($CDCl_3$, δ) : 2.44 (3H, s), 2.66-2.88 (8H), 3.01 (2H, s), 3.28 (3H, s), 3.55 (1H, dd, J=18, 5Hz), 3.82 (1H, dd, J=18, 5Hz), 5.50 (2H, s), 6.71 (1H, d, J=7Hz), 6.87 (1H, t, J=7Hz), 7.32 (1H, d, J=9Hz), 7.50 (1H, d, J=9Hz), 7.78 (1H, d, J=7Hz), 7.90 (1H, br s)

(2) 3-Bromo-8-[2,6-dichloro-3-[N-[(N,N-dimethyl-β-alanyl)glycyl]-N-methylamino]benzyloxy]-2-methylimidazo[1,2-a]pyridine

NMR ($CDCl_3$, δ) : 2.39-2.56 (11H), 2.78 (2H, t, J=6Hz), 3.24 (3H, s), 3.55 (1H, dd, J=17, 4Hz), 3.82 (1H, dd, J=17, 5Hz), 5.50 (2H, s), 6.71 (1H, d, J=7Hz), 6.87 (1H, t, J=7Hz), 7.34 (1H, d, J=9Hz), 7.49 (1H, d, J=9Hz), 7.79 (1H, d, J=7Hz), 8.49 (1H, br s)

Example 75

The following compounds were obtained according to similar manners to those of Examples 36 to 39.

(1) 3-Bromo-8-[2,6-dichloro-3-[N-methyl-N-[[(2-pyrimidinylthio)acetyl]glycyl]amino]benzyloxy]-2-methylimidazo[1,2-a]pyridine

mp : 212-212.5°C
NMR ($CDCl_3$, δ) : 2.42 (3H, s), 3.21 (3H, s), 3.52 (1H, dd, J=18, 5Hz), 3.70-3.89 (3H), 5.49 (2H, s), 6.71 (1H, d, 3=7Hz), 6.87 (1H, t, J=7Hz), 7.07 (1H, t, J=5Hz), 7.28 (1H, d, J=9Hz), 7.47 (1H, d, J=9Hz), 7.77 (1H, d, J=7Hz), 8.03 (1H, br s), 8.62 (2H, d, J=5Hz)

(2) 3-Bromo-8-[2,6-dichloro-3-[N-methyl-N-[(phenoxyacetyl)glycyl]amino]benzyloxy]-2-methylimidazo[1,2-a]pyridine

NMR ($CDCl_3$, δ) : 2.43 (3H, s), 3.28 (3H, s), 3.62 (1H, dd, J=17, 5Hz), 3.89 (1H, dd, J=17, 5Hz), 4.50 (2H, s), 5.50 (2H, s), 6.71 (1H, d, J=7Hz), 6.80-7.09 (4H), 7.22-7.39 (3H), 7.46-7.60 (2H), 7.78 (1H, d, J=7Hz)

(3) 3-Bromo-8-[2,6-dichloro-3-[N-[(heptafluorobutanoyl)glycyl]-N-methylamino]benzyloxy]-2-methylimidazo[1,2-a]pyridine

NMR ($CDCl_3$, δ) : 2.44 (3H, s), 3.29 (3H, s), 3.62 (1H, dd, J=17, 4Hz), 3.87 (1H, dd, J=17, 5Hz), 5.51 (2H, s), 6.71 (1H, d, J=7Hz), 6.87 (1H, t, J=7Hz), 7.32 (1H, d, J=9Hz), 7.41-7.55 (2H), 7.78 (1H, d, J=7Hz)

(4) 3-Bromo-8-[2,6-dichloro-3-[N-(n-heptanoylglycyl)-N-methylamino]benzyloxy]-2-methylimidazo[1,2-a]pyridine

NMR ($CDCl_3$, δ) : 0.88 (3H, t, J=7Hz), 1.16-1.40 (6H), 1.50-1.77 (2H), 2.21 (2H, t, J=7Hz), 2.42 (3H, s), 3.25 (3H, s), 3.52 (1H, dd, J=18, 4Hz), 3.80 (1H, dd, J=18, 5Hz), 5.49 (2H, s), 6.41 (1H, br t, J=5Hz), 6.72 (1H, d, J=7Hz), 6.85 (1H, t, J=7Hz), 7.31 (1H, d, J=9Hz), 7.48 (1H, d, J=9Hz), 7.78 (1H, d, J=7Hz)

(5) 3-Bromo-8-[2,6-dichloro-3-[N-methyl-N-(cinnamoylglycyl)amino]benzyloxy]-2-methylimidazo[1,2-a]pyridine

NMR ($CDCl_3$, δ) : 2.43 (3H, s), 3.29 (3H, s), 3.69 (1H, dd, J=17, 5Hz), 3.92 (1H, dd, J=17, 5Hz), 5.50 (2H, s), 6.49 (1H, d, J=15Hz), 6.62 (1H, br s), 6.72 (1H, d, J=7Hz), 6.86 (1H, t, J=7Hz), 7.30-7.64 (8H), 7.78 (1H, d, J=7Hz)

(6) 3-Bromo-8-[2,6-dichloro-3-[N-methyl-N-[(trans-3-pentenoyl)glycyl]amino]benzyloxy]-2-methylimidazo[1,2-a]pyridine

NMR ($CDCl_3$, δ) : 1.72 (3H, d, J=5Hz), 2.43 (3H, s), 2.95 (2H, d, J=5Hz), 3.25 (3H, s), 3.51 (1H, dd, J=18, 4Hz), 3.79 (1H, dd, J=18, 5Hz), 5.43-5.79 (4H), 6.60 (1H, br s), 6.71 (1H, d, J=7Hz), 6.87 (1H, t, J=7Hz), 7.30 (1H, d, J=9Hz), 7.49 (1H, d, J=9Hz), 7.78 (1H, d, J=7Hz)

(7) 3-Bromo-8-[3-[N-[(3-butenoyl)glycyl]-N-methylamino]-2,6-dichlorobenzyloxy]-2-methylimidazo[1,2-a]pyridine

NMR ($CDCl_3$, δ) : 2.43 (3H, s), 3.02 (2H, d, J=7Hz), 3.25 (3H, s), 3.52 (1H, dd, J=18, 4Hz), 3.79 (1H, dd, J=18, 5Hz), 5.19-5.32 (2H), 5.49 (2H, s), 6.94 (1H, m), 6.59 (1H, br s), 6.72 (1H, d, J=7Hz), 6.87 (1H, t, J=7Hz), 7.30 (1H, d, J=9Hz), 7.48 (1H, d, J=9Hz), 7.78 (1H, d, J=7Hz)

(8) 3-Bromo-8-[2,6-dichloro-3-[N-methyl-N-[(4-phenylbutanoyl)glycyl]amino]benzyloxy]-2-methylimidazo[1,2-a]pyridine

NMR ($CDCl_3$, δ) : 1.86-2.06 (2H), 2.22 (2H, t, J=8Hz), 2.42 (3H, s), 2.63 (2H, t, J=8Hz), 3.25 (3H, s), 3.51 (1H, dd, J=17, 5Hz), 3.79 (1H, dd, J=17, 5Hz), 5.49 (2H, s), 6.39 (1H, br s), 6.71 (1H, d, J=7Hz), 6.86 (1H, t, J=7Hz), 7.11-7.34 (6H), 7.48 (1H, d, J=9Hz), 7.78 (1H, d, J=7Hz)

(9) 3-Bromo-8-[2,6-dichloro-3-[N-methyl-N-[4-(methylcarbamoyl)cinnamoylglycyl]amino]benzyloxy]-2- methylimidazo[1,2-a]pyridine

NMR ($CDCl_3$, δ) : 2.45 (3H, s), 3.03 (3H, d, J=5Hz), 3.28 (3H, s), 3.66 (1H, dd, J=4, 18Hz), 3.92 (1H, dd, J=4, 18Hz), 5.46 (1H, d, J=10Hz), 5.53 (1H, d, J=10Hz), 6.16 (1H, q-like), 6.53 (1H, d, J=16Hz), 6.62-6.78 (2H, m), 6.85 (1H, t, J=7.5Hz), 7.33 (1H, d, J=8Hz), 7.45-7.68 (4H, m), 7.70-7.82 (3H, m)

(10) 3-Bromo-8-[2,6-dichloro-3-[N-[4-(dimethylcarbamoyl)cinnamoylglycyl]-N-methylamino]benzyloxy]-2-methylimidazo[1,2-a]pyridine

NMR ($CDCl_3$, δ) : 2.45 (3H, s), 2.99 (3H, br s), 3.10 (3H, br s), 3.29 (3H, s), 3.69 (1H, dd, J=17, 4Hz), 3.91 (1H, dd, J=17, 5Hz), 5.47 (1H, d, J=10Hz), 5.52 (1H, d, J=10Hz), 6.50 (1H, d, J=15Hz), 6.65 (1H, br t, J=4Hz), 6.71 (1H, d, J=7Hz), 6.86 (1H, t, J=7Hz), 7.29-7.62 (7H), 7.78 (1H, d, J=7Hz)

(11) 3-Bromo-8-[2,6-dichloro-3-[N-[4-(2-methoxyethylcarbamoyl)cinnamoylglycyl]-N-methylamino]benzyloxy]-2-methylimidazo[1,2-a]pyridine

NMR ($CDCl_3$, δ) : 2.44 (3H, s), 3.29 (3H, s), 3.40 (3H, s), 3.52-3.77 (5H), 3.91 (1H, dd, J=17, 5Hz), 5.50 (2H, s), 6.48-6.61 (2H), 6.63-6.79 (2H), 6.88 (1H, t, J=7Hz), 7.33 (1H, d, J=9Hz), 7.46-7.65 (4H), 7.72-7.83 (3H)

(12) 8-[3-[N-[4-[N,N-Bis(2-methoxyethyl)carbamoyl]cinnamoylglycyl]-N-methylamino]-2,6-dichlorobenzyloxy]-3-bromo-2-methylimidazo[1,2-a]pyridine

NMR ($CDCl_3$, δ) : 2.44 (3H, s), 3.21-3.82 (15H), 3.92 (1H, dd, J=17, 5Hz), 5.48 (1H, d, J=10Hz), 5.56 (1H, d, J=10Hz), 6.50 (1H, d, J=15Hz), 6.65 (1H, br t, J=4Hz), 6.73 (1H, d, J=7Hz), 6.88 (1H, t, J=7Hz), 7.30-7.62 (7H), 7.78 (1H, d, J=7Hz)

(13) 3-Bromo-8-[2,6-dichloro-3-[N-methyl-N-[4-(4-pyridylcarbamoyl)cinnamoylglycyl]amino]benzyloxy]-2-methylimidazo[1,2-a]pyridine

NMR ($CDCl_3$, δ) : 2.40 (3H, s), 3.25 (3H, s), 3.65 (1H, dd, J=17,4Hz), 3.90 (1H, dd, J=17, 5Hz), 5.42 (1H, d, J=10Hz), 5.51 (1H, d, J=10Hz), 6.51 (1H, d, J=15Hz), 6.69-6.80 (2H), 6.87 (1H, t, J=7Hz), 7.30 (1H, d, J=9Hz), 7.41-7.69 (6H), 7.78 (1H, d, J=7Hz), 7.87 (2H, d, J=8Hz), 8.43-8.59 (3H)

(14) 3-Bromo-8-[2,6-dichloro-3-[N-methyl-N-[4-(2-oxo-1-pyrrolidinyl)cinnamoylglycyl]amino]benzyloxy]-2-methylimidazo[1,2-a]pyridine

NMR ($CDCl_3$, δ) : 2.15 (1H, quint, J=7.5Hz), 2.41 (3H, s), 2.63 (2H, t, J=7.5Hz), 3.26 (3H, s), 3.65 (1H, dd, J=4, 18Hz), 3.80-3.99 (3H, m), 5.43-5.57 (2H, m), 6.43 (1H, d, J=16Hz), 6.60 (1H, t-like), 6.73 (1H, d, J=8Hz), 6.85 (1H, t, J=8Hz), 7.32 (1H, d, J=8Hz), 7.45-7.61 (4H, m), 7.67 (2H, d, J=8Hz), 7.78 (1H, d, J=6Hz)

(15) 3-Bromo-8-[2,6-dichloro-3-[N-[4-(methoxyacetamido)cinnamoylglycyl]-N-methylamino]benzyloxy]-2-methylimidazo[1,2-a]pyridine

NMR ($CDCl_3$, δ) : 2.43 (3H, s), 3.26 (3H, s), 3.50 (3H, s), 3.65 (1H, dd, J=4, 18Hz), 3.90 (1H, dd, J=4, 18Hz), 4.01 (2H, s), 5.42-5.57 (2H, m), 6.40 (1H, d, J=16Hz), 6.59 (1H, t-like), 6.72 (1H, d, J=7.5Hz), 6.85 (1H, t, J=7.5Hz), 7.32 (1H, d, J=8Hz), 7.43-7.55 (3H, m), 7.55-7.65 (3H, m), 7.77 (1H, d, J=6Hz), 8.30 (1H, s)

(16) 3-Bromo-8-[2,6-dichloro-3-[N-methyl-N-[4-(propionamido)cinnamoylglycyl]amino]benzyloxy]-2-methylimidazo[1,2-a]pyridine

NMR ($CDCl_3$, δ) : 1.24 (3H, t, J=7.5Hz), 2.39 (2H, q, J=7.5Hz), 2.43 (3H, s), 3.26 (3H, s), 3.66 (1H, dd, J=16, 5Hz), 3.88 (1H, dd, J=16, 6Hz), 5.45 (1H, d, J=9Hz), 5.50 (1H, d, J=9Hz), 6.39 (1H, d, J=15Hz), 6.60 (1H, br t, J=5Hz), 6.73 (1H, d, J=7Hz), 6.86 (1H, t, J=7Hz), 7.32 (1H, d,

J=9Hz), 7.40-7.60 (6H, m), 7.76 (1H, d, J=7Hz)

(17) 8-[3-[N-[4-(Acetamido)cinnamoylglycyl]-N-methylamino]-2,6-dichlorobenzyloxy]-3-bromo-2-methylimidazo[1,2-a]pyridine

NMR ($CDCl_3$, δ) : 2.18 (3H, s), 2.44 (3H, s), 3.27 (3H, s), 3.66 (1H, dd, J=4, 18Hz), 3.90 (1H, dd, J=4, 18Hz), 5.41-5.55 (2H, m), 6.40 (1H, d, J=16Hz), 6.59 (1H, t-like), 6.73 (1H, d, J=7Hz), 6.87 (1H, t, J=7Hz), 7.33 (1H, d, J=8Hz), 7.39-7.61 (7H, m), 7.78 (1H, d, J=6Hz)

(18) 3-Bromo-8-[2,6-dichloro-3-[N-[4-(N-methylacetamido)cinnamoylglycyl]-N-methylamino]benzyloxy]-2-methylimidazo[1,2-a]pyridine

NMR ($CDCl_3$, δ) : 1.91 (3H, br s), 2.43 (3H, s), 3.29 (6H, s), 3.68 (1H, dd, J=17, 4Hz), 3.92 (1H, dd, J=17, 5Hz), 5.47 (1H, d, J=10Hz), 5.53 (1H, d, J=10Hz), 6.49 (1H, d, J=15Hz), 6.65 (1H, br t, J=4Hz), 6.72 (1H, d, J=7Hz), 6.88 (1H, t, J=7Hz), 7.15-7.39 (3H), 7.48-7.62 (4H), 7.79 (1H, d, J=7Hz)

(19) 3-Bromo-8-[2,6-dichloro-3-[N-methyl-N-[4-[N-(3-pyridylmethyl)acetamido]cinnamoylglycyl]amino]benzyloxy]-2-methylimidazo[1,2-a]pyridine

NMR ($CDCl_3$, δ) : 1.91 (3H, s), 2.42 (3H, s), 3.29 (3H, s), 3.67 (1H, dd, J=17, 4Hz), 3.91 (1H, dd, J=17, 5Hz), 4.90 (2H, s), 5.48 (1H, d, J=10Hz), 5.53 (1H, d, J=10Hz), 6.46 (1H, d, J=15Hz), 6.67 (1H, br t, J=4Hz), 6.72 (1H, d, J=7Hz), 6.88 (1H, t, J=7Hz), 7.01 (2H, d, J=8Hz), 7.19-7.68 (7H), 7.79 (1H, d, J=7Hz), 7.39 (1H, d, J=1Hz), 8.51 (1H, dd, J=5, 1Hz)

(20) 3-Bromo-8-[2,6-dichloro-3-[N-methyl-N-[4-(isonicotinoylamino)cinnamoylglycyl]amino]benzyloxy]-2-methylimidazo[1,2-a]pyridine

NMR ($CDCl_3$, δ) : 2.45 (3H, s), 3.29 (3H, s), 3.69 (1H, dd, J=17Hz), 3.92 (1H, d, J=17Hz), 5.45-5.58 (2H), 6.47 (1H, d, J=15Hz), 6.65 (1H, br s), 6.78 (1H, d, J=7Hz), 6.90. (1H, t, J=7Hz), 7.31-7.83 (10H), 8.71 (1H, s), 8.82 (2H, d, J=7Hz)

(21) 8-[3-[N-(4-Aminocinnamoylglycyl)-N-methylamino]-2,6-dichlorobenzyloxy]-3-bromo-2-methylimidazo[1,2-a]pyridine

NMR ($CDCl_3$, δ) : 2.43 (3H, s), 3.26 (3H, s), 3.66 (1H, dd, J=18, 4Hz), 3.66 (2H, br s), 5.45 (1H, d, J=9Hz), 5.51 (1H, d, J=9Hz), 6.26 (1H, d, J=16Hz), 6.50 (1H, br t, J=5Hz), 6.63 (2H, d, J=9Hz), 6.72 (1H, d, J=8Hz), 6.87 (1H, t, J=8Hz), 7.31 (2H, d, J=9Hz), 7.46 (1H, d, J=5Hz), 7.47 (1H, d, J=16Hz), 7.51 (1H, d, J=5Hz), 7.77 (1H, d, J=8Hz)

(22) 3-Bromo-8-[2,6-dichloro-3-[N-[4-(methanesulfonamido)cinnamoylglycyl]-N- methylamino]benzyloxy]-2-methylimidazo[1,2-a]pyridine

NMR ($CDCl_3$, δ) : 2.43 (3H, s), 3.03 (3H, s), 3.27 (3H, s), 3.65 (1H, dd, J=4, 7Hz), 3.91 (1H, dd, J=4, 17Hz), 5.42-5.58 (2H, m), 6.40 (1H, d, J=16Hz), 6.55-6.77 (3H, m), 6.86 (1H, dd, J=8, 6Hz), 7.13-7.30 (2H, m), 7.33 (1H, d, J=8Hz), 7.42-7.59 (4H, m), 7.78 (1H, d, J=6Hz)

(23) 3-Bromo-8-[2,6-dichloro-3-[N-methyl-N-[4-(3-methylureido)cinnamoylglycyl]amino]benzyloxy]-2-methylimidazo[1,2-a]pyridine

NMR ($CDCl_3$, δ) : 2.42 (3H, s), 2.80 (3H, d, J=5Hz), 3.23 (3H, s), 3.65 (1H, dd, J=4, 18Hz), 3.88 (1H, dd, J=4, 18Hz), 5.05-5.18 (1H, m), 5.39-5.53 (2H, m), 6.34 (1H, d, J=16Hz), 6.63-6.77 (2H, m), 6.88 (1H, t, J=8Hz), 7.23-7.57 (8H, m), 7.77 (1H, d, J=8Hz)

(24) 3-Bromo-8-[2,6-dichloro-3-[N-[4-(methoxycarbonyl)cinnamoylglycyl]-N-methylamino]benzyloxy]-2-methylimidazo[1,2-a]pyridine

NMR ($CDCl_3$, δ) : 2.43 (3H, s), 3.28 (3H, s), 3.69 (1H, dd, J=17, 4Hz), 3.91 (1H, dd, J=17, 5Hz), 3.93 (3H, s), 5.46 (1H, d, J=9Hz), 5.51 (1H,, d, J=9Hz), 6.56 (1H, d, J=16Hz), 6.70 (1H, m), 6.73 (1H, d, J=8Hz), 6.86 (1H, t, J=8Hz), 7.34 (1H, d, J=9Hz), 7.50 (1H, d, J=9Hz), 7.50 (1H, d, J=9Hz), 7.56 (2H, d, J=9Hz), 7.61 (1H, d, J=16Hz), 7.77 (1H, d, J=8Hz), 8.02 (2H, d, J=9Hz)

(25) 3-Bromo-8-[2,6-dichloro-3-[N-methyl-N-[4-[3-(3-pyridyl)ureido]cinnamoylglycyl]amino]benzyloxy]-2-methylimidazo[1,2-a]pyridine

NMR ($CDCl_3$-$CD_3OD$, δ) : 2.41 (3H, s), 3.25 (3H, s), 3.63 (1H, d, J=18Hz), 3.93 (1H, d, J=18Hz), 5.50 (2H, s), 6.41 (1H, d, J=15Hz), 6.77 (1H, d, J=7Hz), 6.90 (1H, t, J=7Hz), 7.22-7.59 (8H), 7.80 (1H, d, J=7Hz), 8.14-8.32 (3H)

(26) 3-Bromo-8-[2,6-dichloro-3-[N-methyl-N-[4-(morpholinocarbonylamino)cinnamoylglycyl]amino]benzyloxy]-2-methylimidazo[1,2-a]pyridine

NMR ($CDCl_3$, δ) : 2.42 (3H, s), 3.26 (3H, s), 3.43-3.56 (4H), 3.59-3.80 (5H), 3.91 (1H, dd, J=17, 5Hz), 5.50 (2H, s), 6.38 (1H, d, J=15Hz), 6.51-6.62 (2H), 6.72 (1H, d, J=7Hz), 6.88 (1H, t, J=7Hz), 7.24-7.59 (7H), 7.78 (1H, d, J=7Hz)

(27) 3-Bromo-8-[2,6-dichloro-3-[N-methyl-N-[4-(2-oxopiperidino)cinnamoylglycyl]amino]benzyloxy]-2-methylimidazo[1,2-a]pyridine

NMR ($CDCl_3$, δ) : 1.86-2.03 (4H, m), 2.44 (3H, s), 2.57 (3H, dif-t), 3.28 (3H, s), 3.57-3.75 (3H, m), 3.91 (1H, dd, J=4, 18Hz), 5.43-5.56 (2H, m), 6.43 (1H, d, J=16Hz), 6.61 (1H, t-like), 6.71 (1H, d, J=7Hz), 6.85 (1H, t, J=7Hz), 7.21-7.38 (3H, m), 7.44-7.63 (4H, m), 7.78 (1H, d, J=6Hz)

(28) 3-Bromo-8-[2,6-dichloro-3-[N-methyl-N-[4-(N-methyl-2-methoxyacetamido)cinnamoylglycyl]amino]benzyloxy]-2-methylimidazo[1,2-a]pyridine

NMR ($CDCl_3$, δ) : 2.43 (3H, s), 3.28 (3H, s), 3.30 (3H, s), 3.36 (3H, s), 3.68 (1H, dd, J=18, 4Hz), 3.83 (2H, br s), 3.91 (1H, dd, J=18, 5Hz), 5.48 (1H, d, J=9Hz), 5.52 (1H, d, J=9Hz), 6.48 (1H, d, J=15Hz), 6.66 (1H, br t, J=4Hz), 6.73 (1H, d, J=7Hz), 6.86 (1H, t, J=7Hz), 7.20 (2H, d, J=9Hz), 7.34 (1H, d, J=7Hz), 7.50 (1H, d, J=7Hz), 7.55 (2H, d, J=9Hz), 7.59 (1H, d, J=15Hz), 7.78 (1H, d, J=7Hz)

(29) 3-Bromo-8-[2,6-dichloro-3-[N-methyl-N-[4-(1-pyrrolyl)cinnamoylglycyl]amino]benzyloxy]-2-methylimidazo[1,2-a]pyridine

NMR ($CDCl_3$, δ) : 2.43 (3H, s), 3.29 (3H, s), 3.69 (1H, dd, J=18, 4Hz), 3.92 (1H, dd, J=18, 5Hz), 5.50 (2H, s), 6.38 (2H, t, J=1Hz), 6.47 (1H, d, J=16Hz), 6.65 (1H, br t, J=4Hz), 6.72 (1H, d, J=7Hz), 6.88 (1H, t, J=7Hz), 7.12 (2H, t, J=1Hz), 7.30-7.64 (7H), 7.78 (1H, d, J=7Hz)

(30) 3-Chloro-8-[2,6-dichloro-3-[N-[4-(dimethylcarbamoyl)cinnamoylglycyl]-N-methylamino]benzyloxy]-2-methylimidazo[1,2-a]pyridine

NMR ($CDCl_3$, δ) : 2.41 (3H, s), 3.00 (3H, br s), 3.12 (3H, br s), 3.29 (3H, s), 3.69 (1H, dd, J=17, 4Hz), 3.92 (1H, dd, J=17, 5Hz), 5.47 (1H, d, J=10Hz), 5.53 (1H, d, J=10Hz), 6.50 (1H, d, J=16Hz), 6.61-6.78 (2H), 6.88 (1H, t, J=7Hz), 7.30-7.64 (7H), 7.73 (1H, d, J=7Hz)

(31) 3-Chloro-8-[2,6-dichloro-3-[N-methyl-N-[4-(methylcarbamoyl)cinnamoylglycyl]amino]benzyloxy]-2-methylimidazo[1,2-a]pyridine

NMR (DMSO-$d_6$, δ) : 2.30 (3H, s), 2.79 (3H, d, J=5Hz), 3.17 (3H, s), 3.53 (1H, dd, J=4, 18Hz), 3.81 (1H, dd, J=4, 18Hz), 5.48 (2H, s), 6.86 (1H, d, J=16Hz), 6.93-7.05 (2H, m), 7.42 (1H, d, J=16Hz), 7.58-7.70 (2H, m), 7.75-7.90 (4H, m), 7.90-8.00 (1H, m), 8.35 (1H, t-like), 8.50 (1H, q-like)

(32) 3-Chloro-8-[2,6-dichloro-3-[N-[4-(2-methoxyethylcarbamoyl)cinnamoylglycyl]-N- methylamino]benzyloxy]-2-methylimidazo[1,2-a]pyridine

NMR ($CDCl_3$, δ) : 2.42 (3H, s), 3.28 (3H, s), 3.40 (3H, s), 3.54-3.60 (2H), 3.63-3.73 (3H), 3.91 (1H, dd, J=18, 5Hz), 5.49 (1H, d, J=10Hz), 5.52 (1H, d, J=10Hz), 6.50-6.60 (2H), 6.68-6.74 (2H), 6.87 (1H, t, J=7.5Hz), 7.34 (1H, d, J=8Hz), 7.50 (1H, d, J=8Hz), 7.52-7.63 (3H), 7.72 (1H, d, J=7.5Hz), 7.79 (2H, d, J=8Hz)

(33) 3-Chloro-8-[2,6-dichloro-3-[N-[4-(methoxycarbonyl)cinnamoylglycyl]-N-methylamino]benzyloxy]-2-methylimidazo[1,2-a]pyridine

NMR ($CDCl_3$, δ) : 2.44 (3H, s), 3.29 (3H, s), 3.68 (1H, dd, J=4, 18Hz), 3.85-3.96 (4H, m), 5.49 (1H, d, J=10Hz), 5.63 (1H, d, J=10Hz), 6.57 (1H, d, J=16Hz), 6.66-6.74 (2H, m), 6.86 (1H, t, J=7.7Hz), 7.34 (1H, d, J=8Hz), 7.50 (1H, d, J=8Hz), 7.53-7.64 (3H, m), 7.73 (1H, d, J=6Hz), 7.99-8.06 (2H, m)

(34) 3-Chloro-8-[2,6-dichloro-3-[N-methyl-N-[4-(2-oxo-1-pyrrolidinyl)cinnamoylglycyl]amino]benzyloxy]-2-methylimidazo[1,2-a]pyridine

NMR ($CDCl_3$, δ) : 2.19 (2H, quint, J=8Hz), 2.44 (3H, s), 2.63 (3H, t, J=8Hz), 3.29 (3H, s), 3.66 (1H, dd, J=4, 17.5Hz), 3.81-3.99 (3H, m), 5.43-5.58 (2H, m), 6.42 (1H, d, J=16Hz), 6.60 (1H, t-like), 6.70 (1H, d, J=7.5Hz), 6.86 (1H, t, J=7.5Hz), 7.33 (1H, d, J=8Hz), 7.45-7.60 (4H, m), 7.60-7.76 (3H, m)

(35) 8-[3-[N-[4-(Acetamido)cinnamoylglycyl]-N-methylamino]-2,6-dichlorobenzyloxy]-3-chloro-2- methylimidazo[1,2-a]pyridine

NMR ($CDCl_3$, δ) : 2.18 (3H, s), 2.42 (3H, s), 3.26 (3H, s), 3.65 (1H, dd, J=4, 18Hz), 3.90 (1H, dd, J=4, 18Hz), 5.41-5.56 (2H, m), 6.39 (16H, d), 6.59 (1H, t-like), 6.70 (7.5H, d), 6.85 (7.5H, t), 7.31 (8H, d), 7.39-7.59 (7H, m), 7.72 (6H, d)

(36) 3-Chloro-8-[2,6-dichloro-3-[N-methyl-N-[4-(3-methylureido)cinnamoylglycyl]amino]benzyloxy]-2-methylimidazo[1,2-a]pyridine

NMR ($CDCl_3$, δ) : 2.40 (3H, s), 2.78 (3H, d, J=4Hz), 3.21 (3H, s), 3.77 (1H, dd, J=18, 4Hz), 3.87 (1H, dd, J=18, 5Hz), 5.35 (1H, br d, J=4Hz), 5.41 (1H, d, J=10Hz), 5.49 (1H, d, J=10Hz), 6.33 (1H, d, J=16Hz), 6.71 (1H, d, J=7.5Hz), 6.79 (1H, br t, J=4Hz), 6.89 (1H, t, J=7.5Hz), 7.26-7.37 (5H), 7.40 (1H, d, J=8Hz), 7.49 (1H, d, J=16Hz), 7.67 (1H, br s), 7.74 (1H, d, J=7.5Hz)

(37) 3-Chloro-8-[2,6-dichloro-3-[N-[4-(methoxyacetamido)cinnamoylglycyl]-N-methylamino]benzyloxy]-2-methylimidazo[1,2-a]pyridine

NMR ($CDCl_3$, δ) : 2.43 (3H, s), 3.29 (3H, s), 3.51 (3H, s), 3.68 (1H, dd, J=18, 4Hz), 3.91 (1H, dd, J=18, 5Hz), 4.02 (2H, s), 5.48 (1H, d, J=10Hz), 5.52 (1H, d, J=10Hz), 6.41 (1H, d, J=15Hz), 6.60 (1H, br s), 6.71 (1H, d, J=7Hz), 6.87 (1H, t, J=7Hz), 7.32 (1H, d, J=8Hz), 7.46-7.64 (6H), 7.72 (1H, d, J=7Hz), 8.32 (1H, br s)

(38) 3-Chloro-8-[2,6-dichloro-3-[N-methyl-N-[4-(propionamido)cinnamoylglycyl]amino]benzyloxy]-2-methylimidazo[1,2-a]pyridine

NMR ($CDCl_3$, δ) : 1.25 (3H, t, J=7.5Hz), 3.34-3.45 (5H, m), 3.26 (3H, s), 3.66 (1H, dd, J=4, 18Hz), 3.90 (1H, dd, J=4, 18Hz), 5.43-5.55 (2H, m), 6.40 (1H, d, J=16Hz), 6.69 (1H, t-like), 6.71 (1H, d, J=7.5Hz), 7.30-7.36 (2H, m), 7.43-7.57 (5H, m), 7.72 (1H, d, J=6Hz)

(39) 8-[3-[N-(4-Aminocinnamoylglycyl)-N-methylamino]-2,6-dichlorobenzyloxy]-3-chloro-2-methylimidazo[1,2-a]pyridine

NMR ($CDCl_3$, δ) : 2.43 (3H, s), 3.26 (3H, s), 3.65 (1H, dd, J=4, 18Hz), 3.83-3.95 (3H, m), 5.42-5.54 (2H, m),

6.25 (1H, d, J=16Hz), 6.50 (1H, t-like), 6.63 (2H, d, J=8Hz), 6.70 (1H, d, J=7.7Hz), 6.85 (1H, t, J=7.7Hz), 7.29-7.36 (3H, m), 7.43-7.52 (2H, m), 7.71 (1H, d, J=6Hz)

(40)
3-Bromo-8-[2,6-dichloro-3-[N-[(E)-3-(6-ethoxycarbonyl-3-pyridyl)acryloylglycyl]-N-methylamino]benzyloxy]-2-methylimidazo[1,2-a]pyridine

NMR ($CDCl_3$, δ) : 1.47 (3H, t, J=7Hz), 2.44 (3H, s), 3.29 (3H, s), 3.70 (1H, dd, J=18, 4Hz), 3.92 (1H, dd, J=18, 5Hz), 4.50 (2H, q, J=7Hz), 5.51 (2H, s), 6.64 (1H, d, J=16Hz), 6.70-6.80 (2H), 6.88 (1H, t, J=7Hz), 7.35 (1H, d, J=9Hz), 7.51 (1H, d, J=9Hz), 7.62 (1H, d, J=16Hz), 7.78 (1H, d, J=7Hz), 7.94 (1H, dd, J=8, 1Hz), 8.16 (1H, d, J=8Hz), 8.88 (1H, d, J=1Hz)

(41)
8-[3-[N-[(E)-3-(6-Acetylamino-3-pyridyl)acryloylglycyl]-N-methylamino]-2,6-dichlorobenzyloxy]-3-bromo-2-methylimidazo[1,2-a]pyridine

NMR ($CDCl_3$, δ) : 2.21 (3H, s), 2.44 (3H, s), 3.29 (3H, s), 3.69 (1H, dd, J=18, 4Hz), 3.92 (1H, dd, J=18, 5Hz), 5.50 (2H, s), 6.48 (1H, d, J=16Hz), 6.65-6.78 (2H), 6.87 (1H, t, J=7Hz), 7.35 (1H, d, J=9Hz), 7.50 (1H, d, J=9Hz), 7.55 (1H, d, J=16Hz), 7.78 (1H, d, J=7Hz), 7.85 (1H, dd, J=9, 1Hz), 8.10 (1H, br s), 8.22 (1H, d, J=9Hz), 8.37 (1H, d, J=1Hz)

(42) 3-Bromo-8-[2,6-dichloro-3-[N-[4-(2-hydroxyethoxy)cinnamoylglycyl]-N-methylamino]benzyloxy]-2-methylimidazo[1,2-a]pyridine

NMR ($CDCl_3$, δ) : 2.00 (1H, t, J=5Hz), 2.43 (3H, s), 3.27 (3H, s), 3.65 (1H, dd, J=4, 18Hz), 3.83-4.04 (3H, m), 4.11 (2H, t), 5.47 (1H, d, J=10Hz), 5.53 (1H, d, J=10Hz), 6.35 (1H, d, J=16Hz), 6.56 (1H, t-like), 6.71 (1H, d, J=7Hz), 6.79-6.97 (3H, m), 7.33 (1H, d, J=8Hz), 7.40-7.61 (4H, m), 7.77 (1H, d, J=6Hz)

(43) 3-Bromo-8-[2,6-dichloro-3-[N-[3,4-dimethoxycinnamoylglycyl]-N-methylamino]benzyloxy]-2-methylimidazo[1,2-a]pyridine

NMR ($CDCl_3$, δ) : 2.44 (3H, s), 3.29 (3H, s), 3.67 (1H, dd, J=4, 18Hz), 3.84-4.00 (7H, m), 5.43-5.58 (2H, m), 6.37 (1H, d, J=16Hz), 6.59 (1H, t-like), 6.73 (1H, d, J=7.5Hz), 6.81-6.92 (2H, m), 7.00-7.15 (2H, m), 7.34 (1H, d, J=8Hz), 7.49 (1H, d, J=1Hz), 7.54 (1H, d, J=8Hz), 7.78 (1H, d, J=6Hz)

(44) 3-Bromo-8-[2,6-dichloro-3-[N-methyl-N-[3,4-(methylenedioxy)cinnamoylglycyl]amino]benzyloxy]-2-methylimidazo[1,2-a]pyridine

NMR ($CDCl_3$, δ) : 2.44 (3H, s), 3.28 (3H, s), 3.65 (1H, dd, J=4, 18Hz), 3.92 (1H, dd, J=4, 18Hz), 5.42-5.57 (2H, m), 6.00 (2H, s), 6.30 (1H, d, J=16Hz), 6.57 (1H, t-like), 6.69-6.92 (3H, m), 6.92-7.04 (2H, m), 7.33 (2H, d, J=8Hz), 7.42-7.55 (2H, m), 7.78 (1H, d, J=6Hz)

(45) 3-Bromo-8-[2,6-dichloro-3-[N-[(1-indolylcarbonyl)glycyl]-N-methylamino]benzyloxy]-2-methylimidazo[1,2-a]pyridine

NMR ($CDCl_3$, δ) : 2.44 (3H, s), 3.31 (3H, s), 3.79 (1H, dd, J=17, 4Hz), 4.01 (1H, dd, J=17, 5Hz), 5.51 (2H, s), 6.62 (1H, d, J=3Hz), 6.68-6.78 (2H), 6.88 (1H, t, J=7Hz), 7.19-7.62 (6H), 7.78 (1H, d, J=7Hz), 8.11 (1H, d, J=8Hz)

(46) 3-Bromo-8-[2,6-dichloro-3-[N-methyl-N-[(morpholinocarbonyl)glycyl]amino]benzyloxy]-2-methylimidazo[1,2-a]pyridine

NMR ($CDCl_3$, δ) : 2.42 (3H, s), 3.24 (3H, s), 3.30-3.42 (4H), 3.51 (1H, dd, J=17, 4Hz), 3.62-3.88 (5H), 5.40-5.60 (3H), 6.71 (1H, d, J=7Hz), 6.86 (1H, t, J=7Hz), 7.32 (1H, d, J=9Hz), 7.48 (1H, d, J=9Hz), 7.78 (1H, d, J=7Hz)

Example 76

A mixture of 8-[3-(N-glycyl-N-methylamino)-2,6-dichlorobenzyloxy]-3-chloro-2-methylimidazo[1,2-a]pyridine (120 mg), phenyl 3-[N,N-bis(2-methoxyethyl)carbamoyl]phenylcarbamate (110 mg) and triethylamine (57 mg) in N,N-dimethylformamide (1.2 ml) was stirred at 80°C for 1.5 hours. The mixture was extracted with dichloromethane and washed with water. After dried over magnesium sulfate, the solvent was removed in vacuo. The residue was purified by preparative thin layer chromatography eluting with dichlormethane-methanol to give 8-[3-[N-[N'-[3-[N,N-bis(2-methoxyethyl)carbamoyl]phenyl]ureidoacetyl]-N-methylamino]-2,6-dichlorobenzyloxy]-3-chloro-2-methylimidazo[1,2-a]pyridine (162 mg) as amorphous.

NMR ($CDCl_3$, δ) : 2.41 (3H, s), 3.13-3.90 (19H), 5.47 (2H, s), 5.98 (1H, br t, J=4Hz), 6.71 (1H, d, J=7Hz), 6.88 (1H, t, J=7Hz), 7.00 (1H, br d, J=7Hz), 7.14-7.49 (5H), 7.72 (1H, d, J=7Hz), 7.89 (1H, br s)

Example 77

The following compounds were obtained according to similar manners to those of Examples 42, 43, 65 or 76.

(1) 3-Bromo-8-[2,6-dichloro-3-[N-[N'-(5-isoquinolyl)ureidoacetyl]-N-methylamino]benzyloxy]-2-methylimidazo[1,2-a]pyridine

NMR ($CDCl_3$, δ) : 2.40 (3H, s), 3.19 (3H, s), 3.71 (1H, dd, J=17, 5Hz), 3.91 (1H, dd, J=17, 5Hz), 5.46 (2H, s), 6.29 (1H, br t, J=5Hz), 6.71 (1H, d, J=7Hz), 6.87 (1H, t, J=7Hz), 7.31 (1H, d, J=9Hz), 7.41 (1H, d, J=9Hz), 7.54 (1H, t, J=7Hz), 7.66-7.81 (4H), 7.99 (1H, d, J=7Hz), 8.41 (1H, d, J=5Hz), 9.21 (1H, br s)

(2) 3-Bromo-8-[2,6-dichloro-3-[N-methyl-N-[N'-(3-pyrazolyl)ureidoacetyl]amino]benzyloxy]-2-methylimidazo[1,2-a]pyridine

NMR ($CDCl_3$, δ) : 2.44 (3H, s), 3.28 (3H, s), 3.68 (1H, dd, J=17.4Hz), 3.81-3.99 (2H), 5.50 (2H, s), 5.79 (1H, d, J=3Hz), 6.71 (1H, d, J=7Hz), 6.85 (1H, t, J=7Hz), 7.34 (1H, d, J=9Hz), 7.42-7.60 (2H), 7.78 (1H, d, J=7Hz), 7.89 (1H, d, J=3Hz)

(3) 3-Bromo-8-[2,6-dichloro-3-[N-methyl-N-[N'-(4-pyrimidinyl)ureidoacetyl]amino]benzyloxy]-2-methylimidazo[1,2-a]pyridine

NMR ($CDCl_3$, δ) : 2.42 (3H, s), 3.28 (3H, s), 3.80 (1H, dd, J=17, 5Hz), 4.03 (1H, dd, J=17, 5Hz), 5.49 (2H, s), 6.71 (1H, d, J=7Hz), 6.81-6.95 (2H), 7.36 (1H, d, J=9Hz), 7.45 (1H, d, J=9Hz), 7.78 (1H, d, J=7Hz), 8.40 (1H, d, J=6Hz), 8.76-8.85 (2H), 9.35 (1H, br s)

(4) 3-Bromo-8-[2,6-dichloro-3-[N-methyl-N-[N'-(6-quinolyl)ureidoacetyl]amino]benzyloxy]-2-methylimidazo[1,2-a]pyridine

NMR ($CDCl_3$, δ) : 2.41 (3H, s), 3.24 (3H, s), 3.80 (1H, d, J=5Hz), 3.87 (1H, d, J=5Hz), 5.43 (1H, d, J=10Hz), 5.52 (1H, d, J=10Hz), 6.15 (1H, br t, J=5Hz), 6.72 (1H, d, J=7Hz), 6.88 (1H, t, J=7Hz), 7.19-7.46 (4H), 7.70-8.00 (4H), 8.35 (1H, s), 8.70 (1H, d, J=5Hz)

(5) 3-Bromo-8-[3-[N-[N'-n-butylureidoacetyl]-N-methylamino]-2,6-dichlorobenzyloxy]-2-methylimidazo[1,2-a]pyridine

NMR ($CDCl_3$, δ) : 0.90 (3H, t, J=6Hz), 1.21-1.54 (4H), 2.42 (3H, s), 3.12 (1H, q, J=6Hz), 3.23 (3H, s), 3.52 (2H, dd, J=17, 5Hz), 3.80 (1H, dd, J=17, 5Hz), 4.68 (1H, br t, J=6Hz), 5.30 (1H, br t, J=5Hz), 5.49 (2H, s), 6.71 (1H, d, J=7Hz), 6.88 (1H, t, J=7Hz), 7.33 (1H, d, J=9Hz), 7.48 (1H, d, J=9Hz), 7.78 (1H, d, J=7Hz)

(6) 3-Bromo-8-[2,6-dichloro-3-[N-methyl-N-[N'-(3-quinolyl)ureidoacetyl]amino]benzyloxy]-2-methylimidazo[1,2-a]pyridine

NMR ($CDCl_3$, δ) : 2.41 (3H, s), 3.22 (3H, s), 3.86 (1H, dd, J=17, 5Hz), 3.99 (1H, dd, J=17, 5Hz), 5.42 (1H, d,

J=10Hz), 5.52 (1H, d, J=10Hz), 6.11 (1H, br t, J=5Hz), 6.71 (1H, d, J=7Hz), 6.89 (1H, t, J=7Hz), 7.31-7.55 (4H), 7.61 (1H, d, J=9Hz), 7.79 (1H, d, J=7Hz), 7.91 (1H, d, J=9Hz), 8.35 (1H, d, J=3Hz), 8.51 (1H, d, J=3Hz), 8.80 (1H, br s)

(7)
3-Bromo-8-[2,6-dichloro-3-[N-methyl-N-[N'-[3-(4-pyridylcarbamoyl)phenyl]ureidoacetyl]amino]benzyloxy]-2-methylimidazo[1,2-a]pyridine

NMR ($CDCl_3$, δ) : 2.42 (3H, s), 3.25 (3H, s), 3.90-4.03 (2H, m), 5.11 (1H, d, J=10Hz), 5.38 (1H, d, J=10Hz), 6.54-6.69 (2H, m), 6.81 (1H, br s), 6.90 (1H, t, J=7.5Hz), 7.01 (1H, t, J=8Hz), 7.20-7.40 (3H, m), 7.50 (1H, d, J=8Hz), 7.78 (1H, d, J=6Hz), 7.94 (2H, d, J=6Hz), 8.28 (1H, br s), 8.57 (2H, d, J=6Hz)

(8)
3-Bromo-8-[2,6-dichloro-3-[N-methyl-N-[N'-[3-(1-pyrrolidinylcarbonyl)phenyl]ureidoacetyl]amino]benzyloxy]-2-methylimidazo[1,2-a]pyridine

NMR ($CDCl_3$, δ) : 1.76-1.99 (4H, m), 2.40 (3H, s), 3.20 (3H, s), 3.40 (2H, t, J=7Hz), 3.55-3.74 (3H, m), 3.81 (1H, dd, J=4, 17Hz), 5.36-5.52 (2H, m), 5.90-6.00 (1H, m), 6.72 (1H, d, J=8Hz), 6.85 (1H, t, J=8Hz), 7.10 (1H, d, J=6Hz), 7.18-7.28 (1H, m), 7.28-7.48 (4H, m), 7.77 (1H, d, J=6Hz), 7.93 (1H, br s)

(9)
3-Bromo-8-[2,6-dichloro-3-[N-methyl-N-[N'-[3-[N-methyl-N-(3-pyridylmethyl)carbamoyl]phenyl]ureidoacetyl]amino]benzyloxy]-2-methylimidazo[1,2-a]pyridine

NMR ($CDCl_3$, δ) : 2.41 (3H, s), 2.82-3.02 (3H), 3.21 (3H, s), 3.69 (1H, dd, J=18, 5Hz), 3.80 (1H, dd, J=18, 5Hz), 4.52 (0.5H, br s), 4.73 (1.5H, br s), 5.42 (1H, d, J=10Hz), 5.51 (1H, d, J=10Hz), 5.96 (1H, br t, J=5Hz), 6.72 (1H, d, J=7Hz), 6.87 (1H, t, J=7Hz), 7.01 (1H, br d, J=7Hz), 7.18-7.52 (7H), 7.78 (1H, d, J=6Hz), 8.00 (1H, m), 8.50-8.65 (2H)

(10)
3-Bromo-8-[2,6-dichloro-3-[N-methyl-N-[N'-[3-(4-methyl-1-piperazinylcarbonyl)phenyl]ureidoacetyl]amino]benzyloxy]-2-methylimidazo[1,2-a]pyridine

NMR ($CDCl_3$, δ) : 2.21-2.55 (10H), 3.21 (3H, s), 3.33-3.53 (2H), 3.60-3.90 (4H), 5.43 (1H, d, J=10Hz), 5.51 (1H, d, J=10Hz), 5.96 (1H, br t, J=5Hz), 6.72 (1H, d, J=7Hz), 6.89 (1H, t, J=7Hz), 6.98 (1H, d, J=7Hz), 7.18-7.48 (5H), 7.79 (1H, d, J=7Hz), 8.00 (1H, br s)

(11)
3-Bromo-8-[2,6-dichloro-3-[N-methyl-N-[N'-[3-(3-pyridylmethylcarbamoyl)phenyl]ureidoacetyl]amino]benzyloxy]-2-methylimidazo[1,2-a]pyridine

NMR ($CDCl_3$, δ) : 2.40 (3H, s), 3.19 (3H, s), 3.62 (1H, dd, J=17, 5Hz), 4.57 (2H, d, J=5Hz), 5.49 (2H, s), 6.21 (1H, br t, J=5Hz), 6.72 (1H, d, J=7Hz), 6.86 (1H, t, J=7Hz), 7.01 (1H, t, J=8Hz), 7.18-7.39 (5H), 7.45 (1H, d, J=9Hz), 7.62-7.80 (3H), 8.25 (1H, br s), 8.49 (1H, dd, J=5, 1Hz), 8.60 (1H, br s)

(12) 3-Bromo-8-[2,6-dichloro-3-[N-[N'-[3-(dimethylamino)phenyl]ureiaoacetyl]-N-methylamino]benzyloxy]-2-methylimidazo[1,2-a]pyridine

NMR ($CDCl_3$, δ) : 2.42 (3H, s), 2.92 (6H, s), 3.22 (3H, s), 3.63 (1H, dd, J=17, 5Hz), 3.82 (1H, dd, J=17, 5Hz), 5.47 (2H, s), 5.91 (1H, br t, J=5Hz), 6.46 (1H, dd, J=8, 2Hz), 6.69-6.80 (3H), 6.86 (1H, t, J=7Hz), 7.13 (1H, t, J=8Hz), 7.31 (1H, d, J=8Hz), 7.44 (1H, d, J=8Hz), 7.77 (1H, d, J=7Hz)

(13)
8-[3-[N-[N'-[3-[N,N-Bis(2-methoxyethyl)carbamoyl]phenyl]ureidoacetyl]-N-methylamino]-2,6-dichlorobenzyloxy]-3-bromo-2-methylimidazo[1,2-a]pyridine

NMR ($CDCl_3$, δ) : 2.41 (3H, s), 3.22-3.83 (19H, m), 5.45 (1H, d, J=10Hz), 5.53 (1H, d, J=10Hz), 5.82 (1H, t-like), 6.73 (1H, d, J=8Hz), 6.88 (1H, dd, J=6, 8Hz), 7.01 (1H, d, J=8Hz), 7.17-7.48 (5H, m), 7.58 (1H, br s), 7.78 (1H, d, J=6Hz)

(14) 3-Chloro-8-[2,6-dichloro-3-[N-methyl-N-[N'-[3-(4-pyridylcarbamoyl)phenyl]ureidoacetyl]amino]benzyloxy]-2-methylimidazo[1,2-a]pyridine

NMR ($CDCl_3$, δ) : 2.40 (3H, s), 3.25 (3H, s), 3.81-4.06 (2H, m), 5.10 (1H, br d, J=9Hz), 5.36 (1H, d, J=9Hz), 6.55-6.64 (2H, m), 6.80 (1H, br s), 6.90 (1H, t, J=7Hz), 7.01 (1H, t, J=7Hz), 7.24-7.32 (2H, m), 7.36 (1H, d, J=8Hz), 7.50 (1H, br d, J=9Hz), 7.84 (2H, d, J=8Hz), 8.31 (1H, br s), 8.58 (2H, d, J=8Hz), 9.76 (1H, br s)

Example 78

The following compounds were obtained according to a similar manner to that of Example 57.

(1) 3-Bromo-8-[3-[N-(4-carboxycinnamoylglycyl)-N-methylamino]-2,6-dichlorobenzyloxy]-2-methylimidazo[1,2-a]pyridine

mp : 230°C (dec.)

NMR (DMSO-$d_6$, δ) : 2.29 (3H, s), 3.15 (3H, s), 3.54 (1H, dd, J=16, 5Hz), 3.81 (1H, dd, J=16, 6Hz), 5.49 (2H, s), 6.90 (1H, d, J=16Hz), 7.61-7.73 (2H, m), 7.79 (1H, d, J=7Hz), 7.84 (1H, d, J=7Hz), 7.88-8.01 (3H, m), 8.41 (1H, t, J=5Hz)

(2) 3-Chloro-8-[3-[N-(4-carboxycinnamoylglycyl)-N-methylamino]-2,6-dichlorobenzyloxy]-2- methylimidazo[1,2-a]pyridine

mp : >250°C

NMR (DMSO-$d_6$, δ) : 2.30 (3H, s), 3.17 (3H, s), 3.82 (1H, dd, J=4, 18Hz), 5.50 (2H, s), 6.80-7.06 (3H, m), 7.45 (1H, d, J=16Hz), 7.63-7.74 (2H, m), 7.74-7.88 (2H, m), 7.88-8.03 (3H, m), 8.33-8.48 (1H, m)

(3) 3-Bromo-8-[3-[N-[(E)-3-(6-carboxy-3-pyridyl)acryloylglycyl]-N-methylamino]-2,6-dichlorobenzyloxy]-2-methylimidazo[1,2-a]pyridine

mp : 226-228°C (dec.)

NMR (DMSO-$d_6$, δ) : 2.30 (3H, s), 3.16 (3H, s), 3.45-3.62 (1H, overlapped with $H_2O$), 3.82 (1H, dd, J=18, 5Hz), 5.49 (2H, s), 6.93-7.12 (3H), 7.51 (1H, d, J=16Hz), 7.79 (1H, d, J=9Hz), 7.85 (1H, d, J=9Hz), 7.93 (1H, dd, J=5, 3Hz), 8.01-8.20 (2H), 8.44 (1H, br t, J=5Hz), 8.89 (1H, br s)

Example 79

The following compounds were obtained according to a similar manner to that of Example 67.

(1) 3-Bromo-8-[2,6-dichloro-3-[N-methyl-N-[N'-[3-(morpholinocarbonyl)phenyl]ureidoacetyl]amino]benzyloxy]-2-methylimidazo[1,2-a]pyridine

NMR ($CDCl_3$, δ) : 2.41 (3H, s), 3.21 (3H, s), 3.33-3.89 (10H), 5.48 (2H, s), 6.01 (1H, br t, J=5Hz), 6.72 (1H, d, J=7Hz), 6.88 (1H, t, J=7Hz), 6.98 (1H, d, J=8Hz), 7.15-7.48 (5H), 7.78 (1H, d, J=7Hz), 8.10 (1H, br s)

(2) 3-Bromo-8-[2,6-dichloro-3-[N-[4-(ethylcarbamoyl)cinnamoylglycyl]-N- methylamino]benzyloxy]-2-methylimidazo[1,2-a]pyridine

NMR ($CDCl_3$, δ) : 1.28 (3H, t, J=7Hz), 2.44 (3H, s), 3.29 (3H, s), 3.42-3.60 (2H), 3.68 (1H, dd, J=18, 4Hz),

3.91 (1H, dd, J=18, 5Hz), 5.50 (2H, s), 6.13 (1H, br t, J=5Hz), 6.52 (1H, d, J=15Hz), 6.67-6.78 (2H), 6.87 (1H, t, J=7Hz), 7.34 (1H, d, J=8Hz), 7.48-7.66 (4H), 7.71-7.81 (3H)

(3) 3-Bromo-8-[2,6-dichloro-3-[N-[4-(N-ethyl-N-methylcarbamoyl)cinnamoylglycyl]-N-methylamino]benzyloxy]-2-methylimidazo[1,2-a]pyridine

NMR ($CDCl_3$, δ) : 1.08-1.31 (3H), 2.45 (3H, s), 2.90-3.12 (3H), 3.20-3.39 (4H), 3.51-3.77 (2H), 3.91 (1H, dd, J=18, 5Hz), 5.50 (2H, s), 6.51 (1H, d, J=15Hz), 6.68 (1H, br t, J=5Hz), 6.72 (1H, d, J=7Hz), 6.88 (1H, t, J=7Hz), 7.30-7.64 (7H), 7.78 (1H, d, J=7Hz)

(4) 3-Bromo-8-[2,6-dichloro-3-[N-methyl-N-[4-(1-pyrrolidinylcarbonyl)cinnamoylglycyl]amino]benzyloxy]-2-methylimidazo[1,2-a]pyridine

NMR ($CDCl_3$, δ) : 1.80-2.07 (4H, m), 2.44 (3H, s), 3.28 (3H, s), 3.43 (2H, t, J=6Hz), 3.57-3.74 (3H, m), 3.91 (1H, dd, J=4, 18Hz), 5.42-5.56 (2H, m), 6.49 (1H, d, J=16Hz), 6.63 (1H, t-like), 6.71 (1H, d, J=7.5Hz), 6.85 (1H, t, J=7.5Hz), 7.33 (1H, d, J=8Hz), 7.44-7.66 (6H, m), 7.77 (1H, d, J=6Hz)

(5) 3-Bromo-8-[2,6-dichloro-3-[N-methyl-N-[4-(morpholinocarbonyl)cinnamoylglycyl]amino]benzyloxy]-2-methylimidazo[1,2-a]pyridine

NMR ($CDCl_3$, δ) : 2.44 (3H, s), 3.27 (3H, s), 3.35-3.87 (9H, m), 3.91 (1H, dd, J=4, 18Hz), 5.41-5.58 (2H, m), 6.50 (1H, d, J=16Hz), 6.65 (1H, t-like), 6.72 (1H, d, J=7.5Hz), 6.85 (1H, t, J=7.5Hz), 7.33 (1H, d, J=8Hz), 7.41 (2H, d, J=8Hz), 7.47-7.64 (4H, m), 7.77 (1H, d, J=6Hz)

(6) 3-Bromo-8-[2,6-dichloro-3-[N-[4-(N-(2-methoxyethyl)-N-methylcarbamoyl]cinnamoylglycyl]-N-methylamino]benzyloxy]-2-methylimidazo[1,2-a]pyridine

NMR ($CDCl_3$, δ) : 2.43 (3H, s), 3.00-3.15 (3H, m), 3.23-3.50 (8H, m), 3.60-3.78 (3H, m), 3.93 (1H, dd, J=4, 18Hz), 5.44-5.56 (2H, m), 6.50 (1H, d, J=16Hz), 6.64 (1H, t-like), 6.72 (1H, d, J=7.5Hz), 6.86 (1H, t, J=7.5Hz), 7.34 (1H, d, J=8Hz), 7.39-7.65 (6H, m), 7.78 (1H, d, J=6Hz)

(7) 3-Bromo-8-[2,6-dichloro-3-[N-[4-( dimethylcarbamoyl)cinnamoylglycyl]-N-methylamino]benzyloxy]-2-methylimidazo[1,2-a]pyridine

NMR ($CDCl_3$, δ) : 2.45 (3H, s), 2.99 (3H, br s), 3.10 (3H, br s), 3.29 (3H, s), 3.69 (1H, dd, J=17, 4Hz), 3.91 (1H, dd, J=17, 5Hz), 5.47 (1H, d, J=10Hz), 5.52 (1H, d, J=10Hz), 6.50 (1H, d, J=15Hz), 6.65 (1H, br t, J=4Hz), 6.71 (1H, d, J=7Hz), 6.86 (1H, t, J=7Hz), 7.29-7.62 (7H), 7.78 (1H, d, J=7Hz)

(8) 3-Bromo-8-[2,6-dichloro-3-[N-[4-(isopropylcarbamoyl)cinnamoylglycyl]-N-methylamino]benzyloxy]-2-methylimidazo[1,2-a]pyridine

mp : 238°C (dec.)

NMR ($CDCl_3$, δ) : 1.27 (6H, d, J=6Hz), 2.44 (3H, s), 3.28 (3H, s), 3.67 (1H, dd, J=4, 18Hz), 3.91 (1H, dd, J=4, 18Hz), 4.31 (1H, m), 5.43-5.57 (2H, m), 5.92 (1H, d, J=6Hz), 6.53 (1H, d, J=16Hz), 6.63-6.77 (2H, m), 6.86 (1H, t, J=7.5Hz), 7.34 (1H, d, J=8Hz), 7.46-7.65 (4H, m), 7.68-7.81 (1H, m)

(9) 3-Bromo-8-[2,6-dichloro-3-[N-[4-(n-propylcarbamoyl)cinnamoylglycyl]-N-methylamino]benzyloxy]-2-methylimidazo[1,2-a]pyridine

NMR ($CDCl_3$, δ) : 1.00 (3H, t, J=7.5Hz), 1.63 (2H, m), 2.44 (3H, s), 3.28 (3H, s), 3.43 (2H, q, J=7.5Hz), 3.67 (1H, dd, J=4, 18Hz), 3.92 (1H, dd, J=4, 18Hz), 5.43-5.56 (2H, m), 6.13 (1H, t-like), 6.54 (1H, d, J=16Hz), 6.64-6.77 (2H, m), 6.85 (1H, t, J=7.5Hz), 7.35 (1H, d, J=8Hz), 7.44-7.66 (4H, m), 7.69-7.81 (1H, m)

(10)
3-Bromo-8-[2,6-dichloro-3-[N-[4-(3-methoxypropylcarbamoyl)cinnamoylglycyl]-N-methylamino]benzyloxy]-2-methylimidazo[1,2-a]pyridine

NMR ($CDCl_3$, δ) : 1.90 (2H, quint, J=6Hz), 2.45 (3H, s), 3.28 (3H, s), 3.40 (3H, s), 3.51-3.75 (5H, m), 3.91 (1H, dd, J=4, 18Hz), 5.43-5.57 (2H, m), 6.53 (1H, d, J=16Hz), 6.62-6.77 (2H, m), 6.86 (1H, t, J=7.5Hz), 6.99 (1H, t-like), 7.34 (1H, d, J=8Hz), 7.45-7.67 (4H, m), 7.71-7.81 (3H, m)

(11)
3-Bromo-8-[2,6-dichloro-3-[N-[4-(2-ethoxyethylcarbamoyl)cinnamoylglycyl]-N-methylamino]benzyloxy]-2-methylimidazo[1,2-a]pyridine

NMR ($CDCl_3$-$CD_3OD$, δ) : 1.23 (3H, t, J=7Hz), 2.43 (3H, s), 3.28 (3H, s), 3.46-3.77 (7H, m), 3.93 (1H, dd, J=4, 18Hz), 5.42-5.56 (2H, m), 6.46-6.61 (2H, m), 6.65-6.78 (2H, m), 6.87 (1H, t, J=7.5Hz), 7.35 (8H, d), 7.46-7.64 (4H, m), 7.72-7.83 (3H, m)

(12)
3-Bromo-8-[2,6-dichloro-3-[N-[4-(2-hydroxyethylcarbamoyl)cinnamoylglycyl]-N-methylamino]benzyloxy]-2-methylimiazo[1,2-a]pyridine

NMR ($CDCl_3$-$CD_3OD$, δ) : 2.41 (3H, s), 3.26 (3H, s), 3.34-3.46 (1H, m), 3.57 (2H, q, J=5Hz), 3.65-3.81 (3H, m), 3.95 (1H, d, J=17.5Hz), 5.50 (2H, s), 6.58 (1H, d, J=16Hz), 6.75 (1H, d, J=7.5Hz), 6.89 (1H, t, J=7.5Hz), 7.42 (1H, d, J=8Hz), 7.48-7.63 (4H, m), 7.73-7.87 (3H, m)

(13) 3-Bromo-8-[2,6-dichloro-3-[N-[4-(diethylcarbamoyl)cinnamoylglycyl]-N-methylamino]benzyloxy]-2-methylimidazo[1,2-a]pyridine

NMR ($CDCl_3$, δ) : 1.00-1.35 (6H, m), 2.43 (3H, s), 3.26 (3H, s), 3.40-3.75 (5H, m), 3.91 (1H, dd, J=4, 18Hz), 5.47 (1H, d, J=10Hz), 5.53 (1H, d, J=10Hz), 6.48 (1H, d, J=16Hz), 6.65 (1H, t-like), 6.71 (1H, d, J=7.5Hz), 6.86 (1H, t, J=7.5Hz), 7.30-7.45 (3H, m), 7.45-7.64 (4H, m), 7.76 (6H, d)

(14)
3-Bromo-8-[2,6-dichloro-3-[N-[N'-[3-(2-methoxyethylcarbamoyl)phenyl]ureidoacetyl]-N-methylamino]benzyloxy]-2-methylimidazo[1,2-a]pyridine

NMR ($CDCl_3$, δ) : 2.42 (3H, s), 3.23 (3H, s), 3.35 (3H, s), 3.49-3.79 (5H, m), 3.87 (1H, dd, J=18, 5Hz), 5.44 (1H, d, J=10Hz), 5.52 (1H, d, J=10Hz), 6.07 (1H, t-like), 6.72 (1H, d, J=8Hz), 6.80-6.93 (2H, m), 7.15-7.60 (6H, m), 7.78 (1H, d, J=6Hz), 7.92 (1H, s)

(15)
3-Bromo-8-[2,6-dichloro-3-[N-[N'-[3-[N-(2-methoxyethyl)-N-methylcarbamoyl]phenyl]ureidoacetyl]-N-methylamino]benzyloxy]-2-methylimidazo[1,2-a]pyridine

NMR ($CDCl_3$, δ) : 2.42 (3H, s), 2.96-3.14 (3H, m), 3.20-3.49 (8H, m), 3.58-3.90 (4H, m), 5.43 (1H, d, J=10Hz), 5.51 (1H, d, J=10Hz), 5.88 (1H, t-like), 6.72 (1H, d, J=7.5Hz), 6.86 (1H, t, J=7.5Hz), 7.00 (1H, dif-dd, J=7.5Hz), 7.18-7.47 (5H, m), 7.72 (1H, s), 7.77 (1H, d, J=6Hz)

(16)
3-Bromo-8-[2,6-dichloro-3-[N-[N'-[3-[N,N-bis(2-ethoxyethyl)carbamoyl]phenyl]ureidoacetyl]-N-methylamino]benzyloxy]-2-methylimidazo[1,2-a]pyridine

NMR ($CDCl_3$, δ) : 1.07-1.28 (6H, m), 2.40 (3H, s), 3.20 (3H, s), 3.28-3.81 (14H, m), 5.45 (1H, d, J=10Hz), 5.54 (1H, d, J=10Hz), 5.84 (1H, t-like), 6.73 (1H, d, J=7.5Hz), 6.89 (1H, t, J=7.5Hz), 7.03 (1H, d, J=7.5Hz), 7.18-7.49 (5H, m), 7.58 (1H, s), 7.79 (1H, d, J=6Hz)

(17)
3-Bromo-8-[2,6-dichloro-3-[N-[N'-[3-[N,N-bis(2-hydroxyethyl)carbamoyl]phenyl]ureidoacetyl]-N-methylamino]benzyloxy]-2-methylimidazo[1,2-a]pyridine

NMR ($CDCl_3$, δ) : 2.40 (3H, s), 3.23 (3H, s), 3.26-4.01 (12H, m), 5.39-5.55 (2H, m), 6.01 (1H, t-like), 6.71 (1H, d, J=7.5Hz), 6.86 (1H, t, J=7.5Hz), 7.02 (1H, d, J=7.5Hz), 7.10-7.53 (5H, m), 7.77 (1H, d, J=6Hz), 8.36 (1H, s)

(18) 3-Chloro-8-[2,6-dichloro-3-[N-[4-(N-ethyl-N-methylcarbamoyl)cinnamoylglycyl]-N-methylamino]benzyloxy]-2-methylimidazo[1,2-a]pyridine

NMR ($CDCl_3$, δ) : 1.05-1.32 (3H, m), 2.43 (3H, s), 2.88-3.13 (3H, m), 3.20-3.37 (4H, m), 3.47-3.76 (2H, m), 3.93 (1H, dd, J=4, 18Hz), 5.41-5.57 (2H, m), 6.50 (1H, d, J=16Hz), 6.60-6.75 (2H, m), 6.86 (1H, t, J=7.5Hz), 7.30-7.46 (3H, m), 7.46-7.64 (4H, m), 7.73 (1H, d, J=6Hz)

(19) 3-Chloro-8-[2,6-dichloro-3-[N-[4-(ethylcarbamoyl)cinnamoylglycyl]-N-methylamino]benzyloxy]-2-methylimidazo[1,2-a]pyridine

NMR ($CDCl_3$, δ) : 1.24 (3H, t, J=7.5Hz), 2.41 (3H, s), 3.26 (3H, s), 3.50 (2H, quint, J=7.5Hz), 3.65 (1H, dd, J=4, 18Hz), 3.90 (1H, dd, J=4, 18Hz), 5.43-5.55 (2H, m), 6.10 (1H, t-like), 6.53 (1H, d, J=16Hz), 6.63-6.75 (2H, m), 6.85 (1H, t, J=7.5Hz), 7.33 (1H, d, J=8Hz), 7.44-7.64 (4H, m), 7.67-7.80 (3H, m)

(20) 3-Bromo-8-[2,6-dichloro-3-[N-methyl-N-[(E)-3-(6-methylcarbamoyl-3-pyridyl)acryloylglycyl]amino]benzyloxy]-2-methylimidazo[1,2-a]pyridine

NMR ($CDCl_3$, δ) : 2.45 (3H, s), 3.05 (3H, d, J=5Hz), 3.29 (3H, s), 3.70 (1H, dd, J=18, 4Hz), 3.92 (1H, dd, J=18, 5Hz), 5.49 (1H, d, J=10Hz), 5.52 (1H, d, J=10Hz), 6.61 (1H, d, J=16Hz), 6.70-6.79 (2H), 6.88 (1H, t, J=7.5Hz), 7.34 (1H, d, J=7.5Hz), 7.50 (1H, d, J=7.5Hz), 7.61 (1H, d, J=16Hz), 7.78 (1H, d, J=7.5Hz), 7.92-8.02 (2H), 8.20 (1H, d, J=7.5Hz), 8.62 (1H, br s)

(21) 3-Bromo-8-[2,6-dichloro-3-[N-[(E)-3-(6-dimethylcarbamoyl-3-pyridyl)acryloylglycyl]-N-methylamino]benzyloxy]-2-methylimidazo[1,2-a]pyridine

NMR ($CDCl_3$, δ) : 2.43 (3H, s), 3.11 (3H, s), 3.16 (3H, s), 3.29 (3H, s), 3.69 (1H, dd, J=18, 5Hz), 3.91 (1H, dd, J=18, 5Hz), 5.49 (1H, d, J=10Hz), 5.52 (1H, d, J=10Hz), 6.60 (1H, d, J=16Hz), 6.70-6.78 (2H), 6.87 (1H, t, J=7.5Hz), 7.35 (1H, d, J=8Hz), 7.50 (1H, d, J=8Hz), 7.60 (1H, d, J=16Hz), 7.69 (1H, d, J=8Hz), 7.78 (1H, d, J=7.5Hz), 7.91 (1H, dd, J=8, 2Hz), 8.69 (1H, br s)

(22) 3-Bromo-8-[2,6-dichloro-3-[N-[(E)-3-(6-ethylcarbamoyl-3-pyridyl)acryloylglycyl]-N-methylamino]benzyloxy]-2-methylimidazo[1,2-a]pyridine

NMR ($CDCl_3$, δ) : 1.29 (3H, t, J=7Hz), 2.45 (3H, s), 3.29 (3H, s), 3.48-3.59 (2H), 3.70 (1H, dd, J=18, 4Hz), 3.92 (1H, dd, J=18, 5Hz), 5.49 (1H, d, J=10Hz), 5.53 (1H, d, J=10Hz), 6.61 (1H, d, J=16Hz), 6.70-6.79 (2H), 6.87 (1H, t, J=7.5Hz), 7.36 (1H, d, J=8Hz), 7.51 (1H, d, J=8Hz), 7.62 (1H, d, J=16Hz), 7.68 (1H, d, J=7Hz), 7.92-8.01 (2H), 8.20 (1H, d, J=7.5Hz), 8.63 (1H, br s)

(23) 3-Bromo-8-[2,6-dichloro-3-[N-[(E)-3-[6-(3-methoxypropylcarbamoyl)-3-pyridyl]acryloylglycyl]-N-methylamino]benzyloxy]-2-methylimidazo[1,2-a]pyridine

NMR ($CDCl_3$, δ) : 1.91 (2H, quint, J=6.5Hz), 2.45 (3H, s), 3.28 (3H, s), 3.38 (3H, s), 3.50-3.63 (4H, m), 3.68 (1H, dd, J=4, 18Hz), 3.93 (1H, dd, J=4, 18Hz), 5.48 (1H, d, J=10Hz), 5.53 (1H, d, J=10Hz), 6.61 (1H, d, J=16Hz), 6.70-7.76 (2H, m), 6.86 (1H, t, J=7.3Hz), 7.35 (1H, d, J=8.7Hz), 7.50 (1H, d, J=8.3Hz), 7.62 (1H, d, J=16Hz), 7.78 (1H, d, J=7.5Hz), 7.95 (1H, dd, J=8.3, 2.2Hz), 8.20 (1H, d, J=8.3Hz), 8.29 (1H, t-like), 8.65 (1H, d, J=2.0Hz)

Example 80

To a suspension of 3-bromo-8-[2,6-dichloro-3-[N-methyl-N-[N'-(3-nitrophenyl)ureiaoacetyl]amino]benzyloxy]-2-methylimidazo[1,2-a]pyridine (800 mg) in ethanol (8 ml) was added tin(II) chloride (954 mg) at ambient temperature. The mixture was refluxed for 1.5 hours. After cooling, the mixture was adjusted to pH 10 with 1N sodium hydroxide solution. To this mixture was added dichloromethane (10 ml) and the precipitate was removed by filtration. The filtrate was extracted with dichloromethane twice. The organic layer was washed with saturated sodium bicarbonate, water and brine. After dried over magnesium sulfate, the solvent was removed in vacuo. The residue was purified by column chromatography eluting with dichloromethane-methanol to give 8-[3-[N-[N'-(3-aminophenyl)ureidoacetyl]-N-methylamino]-2,6-dichlorobenzyloxy]-3-bromo-2-methylimidazo[1,2-a]pyridine (539 mg) as amorphous.

NMR ($CDCl_3$, δ) : 2.42 (3H, s), 3.22 (3H, s), 3.56-3.75 (3H), 3.82 (1H, dd, J=18, 5Hz), 5.48 (2H, s), 5.92 (1H, br t, J=4Hz), 6.88 (1H, dd, J=8, 1Hz), 6.53 (1H, br d, J=7Hz), 6.72 (1H, d, J=7Hz), 6.79-6.91 (2H), 7.01 (1H, t, J=8Hz), 7.09 (1H, br s), 7.33 (1H, d, J=9Hz), 7.45 (1H, d, J=9Hz), 7.78 (1H, d, J=7Hz)

Example 81

3-Bromo-8-[2,6-dichloro-3-[N-[4-(ethoxycarbonylacetamido)cinnamoylglycyl]-N-methylamino]benzyloxy]-2-methylimidazo[1,2-a]pyridine was obtained by reacting 8-[3-[N-(4-aminocinnamoylglycyl)-N-methylamino]-2,6-dichlorobenzyloxy]-3-bromo-2-methylimidazo[1,2-a]pyridine with ethyl chloroformylacetate according to a similar manner to that of Example 37.

NMR ($CDCl_3$, δ) : 1.33 (3H, t, J=7.5Hz), 2.44 (3H, s), 3.26 (3H, s), 3.47 (2H, s), 3.65 (1H, dd, J=4, 18Hz), 3.90 (1H, dd, J=4, 18Hz), 4.26 (2H, q, J=7.5Hz), 5.42-5.55 (2H, m), 6.41 (1H, d, J=16Hz), 6.60 (1H, t-like), 6.72 (1H, d, J=7.5Hz), 6.85 (1H, t, J=7.5Hz), 7.33 (1H, d, J=8Hz), 7.43-7.64 (6H, m), 7.78 (1H, d, J=6Hz), 9.45 (1H, s)

Example 82

8-[3-[N-[4-(Benzamido)cinnamoylglycyl]-N-methylamino]-2,6-dichlorobenzyloxy]-3-bromo-2-methylimidazo[1,2-a]pyridine was obtained by reacting 8-[3-[N-(4-aminocinnamoylglycyl)-N-methylamino]-2,6-dichlorobenzyloxy]-3-bromo-2-methylimidazo[1,2-a]pyridine with bensoic acid in the presence of N-ethyl-N'-(3-dimethylaminopropyl)carbodiimide hydrochloride and 1-hydroxybenzotriazole according to a similar manner to that of Example 39.

NMR ($CDCl_3$, δ) : 2.41 (3H, s), 3.25 (3H, s), 3.66 (1H, dd, J=18, 4Hz), 3.90 (1H, dd, J=18, 5Hz), 5.49 (2H, br s), 6.42 (1H, d, J=16Hz), 6.60 (1H, br t), 6.72 (1H, d, J=7Hz), 6.86 (1H, t, J=7Hz), 7.18-7.80 (9H, m), 7.86 (2H, d, J=9Hz), 8.01 (1H, br s)

Example 83

The following compounds were obtained according to similar manners to those of Examples 81 or 82.

(1) 3-Bromo-8-[2,6-dichloro-3-[N-methyl-N-[4-(4-pyridylacetamido)cinnamoylglycyl]amino]benzyloxy]-2-methylimidazo[1,2-a]pyridine

NMR ($CDCl_3$, δ) : 2.40 (3H, s), 3.23 (3H, s), 3.64 (1H, dd, J=18, 5Hz), 3.67 (2H, br s), 3.86 (1H, dd, J=18, 4Hz), 5.42 (1H, d, J=9Hz), 5.48 (1H, d, J=9Hz), 6.39 (1H, d, J=16Hz), 6.63 (1H, br t, J=4Hz), 6.73 (1H, d, J=7.5Hz), 6.86 (1H, t, J=7.5Hz), 7.15-7.33 (3H, m), 7.35-7.57 (6H, m), 7.78 (1H, d, J=7.5Hz), 8.18 (1H br s), 8.58 (2H, dd, J=7, 2Hz)

(2) 3-Bromo-8-[2,6-dichloro-3-[N-[4-(3-methoxypropionamido)cinnamoylglycyl]-N-methylamino]benzyloxy]-2-methylimidazo[1,2-a]pyridine

NMR ($CDCl_3$, δ) 2.45 (3H, s), 2.65 (2H, t, J=6Hz), 3.28 (3H, s), 3.46 (3H, s), 3.59-3.79 (3H, m), 3.90 (1H, dd, J=4, 18Hz), 5.42-5.57 (2H, m), 6.40 (1H, d, J=16Hz), 6.60 (1H, t-like), 6.73 (1H, d, J=7Hz), 6.87 (1H, d, J=7Hz), 7.33 (1H, d, J=8Hz), 7.40-7.60 (6H, m), 7.77 (1H, d, J=6Hz), 8.39 (1H, s)

(3) 8-[3-[N-[4- (Acetamidoacetamido)cinnamoylglycyl]-N-methylamino]-2,6-dichlorobenzyloxy]-3-bromo-2-methylimidazo[1,2-a]pyridine

NMR ($CDCl_3$, δ) : 2.10 (3H, s), 2.44 (3H, s), 3.27 (3H, s), 3.64 (1H, dd, J=4, 17.5Hz), 3.92 (1H, dd, J=4, 17.5Hz), 4.06 (2H, d, J=6Hz), 5.43-5.56 (2H, m), 6.35 (1H, d, J=16Hz), 6.59 (1H, t-like), 6.66-6.92 (3H, m), 7.30-7.57 (7H, m), 7.76 (1H, d, J=6Hz), 8.86 (1H, s)

(4)
3-Bromo-8-[2,6-dichloro-3-[N-methyl-N-[4-[2-(dimethylamino)acetamido]cinnamoylglycyl]amino]benzyloxy]-2-methylimidazo[1,2-a]pyridine

NMR ($CDCl_3$, δ) : 2.39 (6H, s), 2.43 (3H, s), 3.08 (2H, s), 3.28 (3H, s), 3.68 (1H, dd, J=4, 17.5Hz), 4.92 (1H, dd, J=4, 17.5Hz), 5.42-5.56 (2H, m), 6.42 (1H, d, J=16Hz), 6.59 (1H, t-like), 6.72 (1H, d, J=7.5Hz), 6.86 (1H, t, J=7.5Hz), 7.33 (1H, d, J=8Hz), 7.40-7.68 (6H, m), 7.76 (1H, d, J=6Hz), 9.23 (1H, s)

(5)
3-Bromo-8-[2,6-dichloro-3-[N-[4-[(E)-3-(ethoxycarbonyl)acrylamido]cinnamoylglycyl]-N-methylamino]benzyloxy]-2-methylimidazo[1,2-a]pyridine

NMR ($CDCl_3$, δ) : 1.30 (3H, t, J=7.5Hz), 2.41 (3H, s), 3.25 (3H, s), 3.64 (1H, dd, J=17, 4Hz), 3.88 (1H, dd, J=17, 5Hz), 4.24 (2H, q, J=7.5Hz), 5.42 (1H, d, J=9Hz), 5.49 (1H, d, J=9Hz), 6.40 (1H, d, J=16Hz), 6.67 (1H, br t, J=4Hz), 6.73 (1H, d, J=7Hz), 6.81-6.97 (2H, m), 7.10 (1H, d, J=15Hz), 7.20 (2H, d, J=9Hz), 7.36-7.52 (3H, m), 7.58 (1H, d, J=11Hz), 7.63 (1H, d, J=7Hz), 7.78 (1H, d, J=7Hz), 8.82 (1H, br s)

(6) 3-Bromo-8-[2,6-dichloro-3-[N-[4-(hydroxyacetamido)cinnamoylglycyl]-N-methylamino]benzyloxy]-2-methylimidazo[1,2-a]pyridine

NMR ($CDCl_3$, δ) : 2.39 (3H, s), 3.24 (3H, s), 3.67 (1H, dd, J=17, 4Hz), 3.94 (1H, dd, J=17, 5Hz), 4.11 (2H, s), 5.48 (2H, s), 6.30 (1H, d, J=15Hz), 6.66-6.90 (2H, m), 6.86 (1H, t, J=7Hz), 7.25-7.60 (7H, m), 7.76 (1H, d, J=7Hz), 8.78 (1H, br s)

(7)
3-Bromo-8-[2,6-dichloro-3-[N-[4-(2-chloroethoxycarbonylamino)cinnamoylglycyl]-N-methylamino]benzyloxy]-2-methylimidazo[1,2-a]pyridine

NMR ($CDCl_3$, δ) : 2.44 (3H, s), 3.27 (3H, s), 3.57-3.80 (3H, m), 3.91 (1H, dd, J=4, 18Hz), 4.45 (2H, t, J=6Hz), 5.50 (2H, s), 6.40 (1H, d, J=16Hz), 6.60 (1H, t-like), 6.72 (1H, d, J=7.5Hz), 6.80-6.91 (2H, m), 7.22-7.60 (7H, m), 7.78 (1H, d, J=6Hz)

(8)
3-Bromo-8-[2,6-dichloro-3-[N-[N'-[3-(methoxyacetamido)phenyl]ureidoacetyl]-N-methylamino]benzyloxy]-2-methylimidazo[1,2-a]pyridine

mp : 238-239°C
NMR ($CDCl_3$-$CD_3OD$, δ) : 2.40 (3H, s), 3.25 (3H, s), 3.45-3.68 (4H, overlapped with $H_2O$), 3.89 (1H, d, J=18Hz), 4.00 (2H, s), 5.50 (2H, s), 6.78 (1H, d, J=7Hz), 6.91 (1H, t, J=7Hz), 7.08-7.38 (3H), 7.44 (1H, d, J=9Hz), 7.52 (1H, d, J=9Hz), 7.59 (1H, br s), 7.80 (1H, d, J=7Hz)

(9)
3-Bromo-8-[2,6-dichloro-3-[N-[N'-[3-(ethoxycarbonylacetamido)phenyl]ureidoacetyl]-N-methylamino]benzyloxy]-2-methylimidazo[1,2-a]pyridine

NMR ($CDCl_3$, δ) : 1.27 (3H, t, J=7Hz), 2.40 (3H, s), 3.19 (3H, s), 3.41 (2H, s), 3.59 (1H, br d, J=18Hz), 3.89 (1H, br d, J=18Hz), 4.19 (2H, q, J=7Hz), 5.40 (2H, s), 6.22 (1H, br s), 6.72 (1H, d, J=7Hz), 6.88 (1H, t, J=7Hz), 7.00-7.52 (6H), 7.78 (1H, d, J=7Hz), 8.18 (1H, br s), 9.62 (1H, br s)

(10) 3-Chloro-8-[2,6-dichloro-3-[N-[4-(3-methoxypropionamido)cinnamoylglycyl]-N-methylamino]benzyloxy]-2-methylimidazo[1,2-a]pyridine

NMR ($CDCl_3$, δ) : 2.31 (3H, s), 2.63 (2H, t, J=5Hz), 3.26 (3H, s), 3.47 (3H, s), 3.61-3.77 (3H), 3.90 (1H, dd, J=18, 5Hz), 5.48 (1H, d, J=10Hz), 5.51 (1H, d, J=10Hz), 6.39 (1H, d, J=15Hz), 6.60 (1H, br s), 6.71 (1H, d, J=7.5Hz), 6.87 (1H, t, J=7.5Hz), 7.32 (1H, d, J=8Hz), 7.41-7.58 (6H), 7.72 (1H, d, J=7.5Hz), 8.45 (1H, br s)

(11) 8-[3-[N-[4-(Acetamidoacetamido)cinnamoylglycyl]-N-methylamino]-2,6-dichlorobenzyloxy]-3-chloro-2-methylimidazo[1,2-a]pyridine

NMR ($CDCl_3$, δ) : 2.09 (3H, s), 2.42 (3H, s), 3.25 (3H, s), 3.64 (1H, dd, J=4, 18Hz), 3.90 (1H, dd, J=4, 18Hz), 4.05 (2H, d, J=5Hz), 5.43-5.54 (2H, m), 6.39 (1H, d, J=16Hz), 6.62 (1H, t-like), 6.70 (1H, d, J=7.7Hz), 6.80 (1H, t-like), 6.86 (1H, t, J=7.7Hz), 7.32-7.55 (7H, m), 7.72 (2H, d, J=6Hz), 8.85 (1H, s)

Example 84

A solution of 8-[3-[N-(4-aminocinnamoylglycyl)-N-methylamino]-2,6-dichlorobenzyloxy]-3-bromo-2-methylimidazo[1,2-a]pyridine (150 mg) and succinic anhydride (26 mg) in dioxane (3 ml) was refluxed for 2 hours. After cooling, the solution was removed in vacuo to give 3-bromo-8-[3-[N-[4-(3-carboxypropionamido)cinnamoylglycyl]-N-methylamino]-2,6-dichlorobenzyloxy]-2-methylimidazo[1,2-a]pyridine (175 mg) as amorphous.

NMR ($CDCl_3$-$CD_3OD$, δ) : 2.41 (3H, s), 2.59-2.70 (4H), 3.27 (3H, s), 3.64 (1H, d, J=18Hz), 3.98 (1H, d, J=18Hz), 5.51 (2H, s), 6.41 (1H, d, J=15Hz), 6.76 (1H, d, J=7Hz), 6.90 (1H, t, J=7Hz), 7.38-7.62 (7H), 7.79 (1H, d, J=7Hz)

Example 85

A solution of 8-[3-(N-glycyl-N-methylamino)-2,6-dichlorobenzyloxy]-3-bromo-2-methylimidazo[1,2-a]pyridine (300 mg) and dimethylcyanodithioiminocarbonate (93 mg) in dimethylformamide (3 ml) was heated at 70°C for 1 hour. After cooling the reacting mixture, to the mixture was added 70% solution of ethylamine in water (0.57 ml) and the mixture was heated at 60°C for 2 hours. To this mixture was added water (3 ml) in an ice-water bath. The precipitates were collected by vacuum filtration and washed with ethyl acetate to give 3-bromo-8-[3-[N-[(2-cyano-3-ethylguanidino)acetyl]-N-methylamino]-2,6-dichlorobenzyloxy]-2-methylimidazo[1,2-a]pyridine (278 mg) as colorless crystals.

mp : >250°C

NMR ($CDCl_3$-$CD_3OD$, δ) : 1.20 (3H, t, J=7Hz), 2.39 (3H, s), 3.16-3.31 (5H), 3.61 (1H, d, J=17Hz), 3.73 (1H, d, J=17Hz), 5.47 (1H, d, J=10Hz), 5.57 (1H, d, J=10Hz), 6.79 (1H, d, J=7Hz), 6.91 (1H, t, J=7Hz), 7.42 (1H, d, J=9Hz), 7.56 (1H, d, J=9Hz), 7.80 (1H, d, J=7Hz)

Example 86

A suspension of 3-bromo-8-[3-[N-[4-(3-carboxypropionamido)cinnamoylglycyl]-N-methylamino]-2,6-dichlorobenzyloxy]-2-methylimidazo[1,2-a]pyridine (110 mg) and anhydrous sodium acetate (16 mg) in acetic anhydride (1.1 ml) was refluxed for 5 hours. After cooling, the solvent was removed in vacuo. The residue was dissolved in dichloromethane (5 ml) and washed with water, saturated sodium bicarbonate twice and brine. After dried over magnesium sulfate, the solvent was removed in vacuo. The residue was purified by preparative thin-layer chromatography (ethyl acetate - methanol) to give 3-bromo-8-[2,6-dichloro-3-[N-[4-(succinimido)cinnamoylglycyl]-N-methylamino]benzyloxy]-2-methylimidazo[1,2-a]pyridine (12 mg) as amorphous.

NMR ($CDCl_3$, δ) : 2.43 (3H, s), 2.91 (4H, s), 3.29 (3H, s), 3.68 (1H, dd, J=18, 4Hz), 3.91 (1H, dd, J=18, 5Hz), 5.48 (1H, d, J=10Hz), 5.53 (1H, d, J=10Hz), 6.50 (1H, d, J=16Hz), 6.68 (1H, br t, J=4Hz), 6.72 (1H, d, J=7Hz), 6.88 (1H, t, J=7Hz), 7.22-7.39 (3H), 7.48-7.67 (4H), 7.78 (1H, d, J=7Hz)

Example 87

To a solution of 3-bromo-8-[2,6-dichloro-3-[N-[4-(2-chloroethoxycarbonylamino)cinnamoylglycyl]-N-methylamino]benzyloxy]-2-methylimidazo[1,2-a]pyridine (128 mg) in methanol was added dropwise sodium methanolate (28% in methanol, 37 mg) under nitrogen in ice-water bath and the mixture was stirred for 1 hour at same temperature and then at ambient temperature for 2 hours. The reaction mixture was poured into water and extracted with dichloromethane. The organic layer was washed with water and brine, dried over magnesium sulfate and evaporated in vacuo. The residue was purified by preparative thin layer chromatography(ethyl acetate) to give 3-bromo-8-[2,6-dichloro-3-[N-[4-(2-oxo-3-oxazolidinyl)cinnamoylglycyl]-N-methylamino]benzyloxy]-2-methylimidazo[1,2-a]pyridine (77 mg) as an amorphous powder.

NMR ($CDCl_3$, δ) : 2.44 (3H, s), 3.28 (3H, s), 3.66 (1H, dd, J=4, 18Hz), 3.91 (1H, dd, J=4, 18Hz), 4.10 (2H, dd, J=6, 8Hz), 4.53 (2H, dd, J=6, 8Hz), 5.45-5.57 (2H, m), 6.43 (1H, d, J=16Hz), 6.60 (1H, t-like), 6.72 (1H, d, J=7.5Hz), 6.85 (1H, t, J=7.5Hz), 7.34 (1H, d, J=8Hz), 7.45-7.62 (6H, m), 7.76 (6H, d)

Example 88

(1) 3-Bromo-8-[3-[N-[N'-[3-(4-bromobutyramido)phenyl]ureidoacetyl]-N-methylamino]-2,6-dichlorobenzyloxy]-2-methylimidazo[1,2-a]pyridine was obtained according to a similar manner to that of Example 82.

(2) 3-Bromo-8-[2,6-dichloro-3-[N-methyl-N-[N'-[3-(2-oxo-1-pyrrolidinyl)phenyl]ureidoacetyl]amino]benzyloxy]-2-methylimidazo[1,2-a]pyridine was obtained according to a similar manner to that of Example 87.

NMR ($CDCl_3$, δ) : 2.01-2.27 (2H), 2.41 (3H, s), 2.59 (2H, t, J=8Hz), 3.23 (3H, s), 3.63 (1H, dd, J=17, 5Hz), 3.74-3.92 (3H), 4.58 (1H, br t, J=6Hz), 5.46 (2H, s), 5.99 (1H, br t, J=5Hz), 6.72 (1H, d, J=7Hz), 6.88 (1H, t, J=8Hz), 7.02-7.50 (5H), 7.60 (1H, d, J=9Hz), 7.77 (1H, d, J=7Hz)

Example 89

A mixture of 3-bromo-8-[2,6-dichloro-3-[N-[4-(3-carboxypropionamido)cinnamoylglycyl]-N-methylamino]benzyloxy]-2-methylimidazo[1,2-a]pyridine (60 mg), ethanol (12 mg), N-ethyl-N'-(3-dimethylaminopropyl)carbodiimide hydrochloride (21 mg), 1-hydroxybenzotriazole (17 mg) and N,N-dimethylformamide (0.6 ml) was stirred for 3 hours at ambient temperature. Ethyl acetate was added thereto, and the mixture was washed with water four times, dried over magnesium sulfate and concentrated in vacuo. The residue was purified by preparative thin layer chromatography (methylenechloride:methanol = 10:1, V/V) to give 3-bromo-8-[2,6-dichloro-3-[N-[4-(3-ethoxycarbonylpropionamido)cinnamoylglycyl]-N-methylamino]benzyloxy]-2-methylimidazo[1,2-a]pyridine (7 mg) as an amorphous.

NMR ($CDCl_3$, δ) : 1.28 (3H, t, J=7Hz), 2.44 (3H, s), 2.60-2.82 (4H), 3.28 (3H, s), 3.66 (1H, dd, J=18, 4Hz), 3.91 (1H, dd, J=18, 5Hz), 4.18 (2H, q, J=7Hz), 5.49 (2H, s), 6.39 (1H, d, J=15Hz), 6.61-6.79 (2H), 6.87 (1H, t, J=7Hz), 7.30-7.60 (7H), 7.78 (1H, d, J=7Hz), 8.12 (1H, br s)

Example 90

To a solution of 8-[3-[N-[N'-(3-acetylphenyl)ureidoacetyl]-N-methylamino]-2,6-dichlorobenzyloxy]-3-bromo-2-methylimidazo[1,2-a]pyridine (200 mg) in methanol (2 ml) was added sodium borohydride (24 mg) under ice-bath cooling, and the mixture was stirred for 1 hour at ambient temperature. To the reaction mixture was added 1N hydrochloric acid, and the mixture was stirred for 30 minutes. Saturated aqueous solution of sodium bicarbonate was added thereto, and the mixture was extracted with methylene chloride three times. The combined organic layer was washed with water and brine, dried over magnesium sulfate, and concentrated in vacuo. The residue was purified by silica gel column chromatography (methylene chloride:methanol=30:1, V/V) togive 3-bromo-8-[2,6-dichloro-3-[N-[N'-[3-(1-hydroxyethyl)phenyl]ureidoacetyl]-N-methylamino]benzyloxy]-2-methylimidazo[1,2-a]pyridine (160 mg) as an amorphous.

NMR ($CDCl_3$, δ) : 1.41 (3H, d, J=6Hz), 2.41 (3H, s), 3.22 (3H, s), 3.61 (1H, br dt, J=17, 3Hz), 3.88 (1H, dd, J=17, 5Hz), 4.79 (1H, m), 5.49 (2H, s), 6.09 (1H, br t, J=5Hz), 6.72 (1H, d, J=7Hz), 6.87 (1H, t, J=7Hz),

6.96 (1H, m), 7.10-7.49 (4H), 7.59 (1H, br d, J=7Hz), 7.78 (1H, d, J=7Hz)

Example 91

To a solution of 3-bromo-8-[2,6-dichloro-3-[N-methyl-N-[(thiomorpholinoacetyl)glycyl]amino]benzyloxy]-2-methylimidazo[1,2-a]pyridine (200 mg) in methylene chloride (2 ml) was added m-chloroperbenzoic acid (80% purity, 76 mg) under ice-bath cooling, and the mixture was stirred for 1 hour at the same temperature. The reaction mixture was washed with water twice and brine, dried over magnesium sulfate, and concentrated in vacuo. The residue was purified by preparative thin layer chromatography (methylene chloride:methanol = 5:1, V/V) to give 3-bromo-8-[2,6-dichloro-3-[N-methyl-N-[[(1-oxothiomorpholino)acetyl]glycyl]amino]benzyloxy]-2-methylimidazo[1,2-a]pyridine (96 mg) as an amorphous.

NMR ($CDCl_3$, δ) : 2.44 (3H, s), 2.70-3.02 (6H), 3.11 (2H, s), 3.15-3.36 (5H), 3.57 (1H, dd, J=17, 5Hz), 3.85 (1H, dd, J=17, 5Hz), 5.47 (1H, dd, J=10Hz), 5.52 (1H, d, J=10Hz), 6.71 (1H, d, J=7Hz), 6.86 (1H, t, J=7Hz), 7.31 (1H, d, J=9Hz), 7.50 (1H, d, J=9Hz), 7.79 (1H, d, J=7Hz), 7.84 (1H, br t, J=5Hz)

Example 92

To a solution of 3-bromo-8-[2,6-dichloro-3-[N-methyl-N-[(thiomorpholinoacetyl)glycyl]amino]benzyloxy]-2-methylimidazo[1,2-a]pyridine (200 mg) in methylene chloride (2 ml) was added m-chloroperbenzoic acid (80% purity, 190 mg) under ice-bath cooling, and the mixture was stirred for 2 hours at the same temperature. The reaction mixture was washed with water twice and brine, dried over magnesium sulfate, and concentrated in vacuo. The residue was purified by preparative thin layer chromatography (methylene chloride:methanol = 3:1, V/V) to give 3-bromo-8-[2,6-dichloro-3-[N-[[(1,1-dioxothiomorpholino)acetyl]glycyl]-N-methylamino]benzyloxy]-2-methylimidazo[1,2-a]pyridine (99 mg) as an amorphous.

NMR ($CDCl_3$, δ) : 2.43 (3H, s), 2.81-3.01 (2H), 3.21 (3H, s), 3.48-4.20 (10H), 5.50 (2H, s), 6.71 (1H, d, J=7Hz), 6.86 (1H, t, J=7Hz), 7.40 (1H, d, J=9Hz), 7.50 (1H, d, J=9Hz), 7.77 (1H, d, J=7Hz)

Example 93

The following compounds were obtained according to a similar manner to that of Example 60.

(1) 3-Bromo-8-[2,6-dichloro-3-[N-methyl-N-[(thiomorpholinoacetyl)glycyl]amino]benzyloxy]-2-methylimidazo[1,2-a]pyridine dihydrochloride

NMR ($CDCl_3$-$CD_3OD$, δ) : 2.61 (3H, s), 2.70-4.30 (15H), 5.65 (1H, d, J=10Hz), 5.75 (1H, d, J=10Hz), 7.52-7.70 (4H), 8.22 (1H, dd, J=5, 3Hz)

(2) 3-Bromo-8-[2,6-dichloro-3-[N-methyl-N-[[(1-oxothiomorpholino)acetyl]glycyl]amino]benzyloxy]-2-methylimidazo[1,2-a]pyridine dihydrochloride

NMR ($CDCl_3$-$CD_3OD$, δ) : 2.67 (3H, s), 3.00-3.19 (2H), 3.22 (3H, s), 3.57-4.24 (10H), 5.60 (1H, d, J=10Hz), 5.70 (1H, d, J=10Hz), 7.39-7.66 (4H), 8.10 (1H, d, J=6Hz)

(3) 3-Bromo-8-[2,6-dichloro-3-[N-[[(1,1-dioxothiomorpholino)acetyl]glycyl]-N-methylamino]benzyloxy]-2-methylimidazo[1,2-a]pyridine dihydrochloride

NMR ($CDCl_3$-$CD_3OD$, δ) : 2.63 (3H, s), 3.00-3.19 (2H), 3.24 (3H, s), 3.35-3.57 (2H), 3.72 (1H, d, J=16Hz), 3.93 (1H, d, J=16Hz), 4.20-4.41 (2H), 4.62-4.98 (4H), 5.67 (2H, s), 7.38-7.67 (4H), 8.09 (1H, d, J=6Hz)

(4) 3-Bromo-8-[2,6-dichloro-3-[N-methyl-N-[[(2-pyrimidinylthio)acetyl]glycyl]amino]benzyloxy]-2-methylimidazo[1,2-a]pyridine dihydrochloride

NMR ($CDCl_3$-$CD_3OD$, δ) : 2.57 (3H, s), 3.26 (3H, s), 3.67 (2H, s), 3.86 (2H, s), 5.64 (1H, d, J=10Hz), 5.73 (1H, d, J=10Hz), 7.19 (1H, t, J=5Hz), 7.48-7.69 (4H), 8.21 (1H, dd, J=5, 1Hz), 8.66 (2H, d, J=5Hz)

(5) 3-Bromo-8-[2,6-dichloro-3-[N-methyl-N-[(phenoxyacetyl)glycyl]amino]benzyloxy]-2-methylimidazo[1,2-a]pyridine hydrochloride

NMR ($CDCl_3$-$CD_3OD$, δ) : 2.58 (3H, s), 3.30 (3H, s), 3.78 (2H, s), 4.51 (2H, s), 5.67 (1H, d, J=10Hz), 5.77 (1H, d, J=10Hz), 6.90-7.10 (3H), 7.25-7.42 (2H), 7.51-7.70 (4H), 8.21 (1H, dd, J=5, 2Hz)

(6) 3-Bromo-8-[2,6-dichloro-3-[N-[(heptafluorobutanoyl)glycyl]-N-methylamino]benzyloxy]-2-methylimidazo[1,2-a]pyridine hydrochloride

NMR ($CDCl_3$-$CD_3OD$, δ) : 2.62 (3H, s), 3.28 (3H, s), 3.77 (1H, d, J=17Hz), 3.89 (1H, d, J=17Hz), 5.62 (1H, d, J=10Hz), 5.71 (1H, d, J=10Hz), 7.39-7.63 (4H), 8.09 (1H, dd, J=6, 1Hz)

(7) 3-Bromo-8-[2,6-dichloro-3-[N-(n-heptanoylglycyl)-N-methylamino]benzyloxy]-2-methylimidazo[1,2-a]pyridine hydrochloride

NMR ($CDCl_3$-$CD_3OD$, δ) : 0.89 (3H, t, J=7Hz), 1.21-1.40 (6H), 1.51-1.70 (2H), 2.26 (2H, t, J=7Hz), 2.58 (3H, s), 3.29 (3H, s), 3.68 (2H, s), 5.67 (1H, d, J=10Hz), 5.77 (1H, d, J=10Hz), 7.52-7.70 (4H), 8.24 (1H, dd, J=5, 2Hz)

(8) 3-Bromo-8-[2,6-dichloro-3-[N-methyl-N-(cinnamoylglycyl)amino]benzyloxy]-2-methylimidazo[1,2-a]pyridine hydrochloride

NMR ($CDCl_3$-$CD_3OD$, δ) : 2.60 (3H, s), 3.30 (3H, s), 3.81 (2H, s), 5.67 (1H, d, J=10Hz), 5.78 (1H, d, J=10Hz), 6.60 (1H, d, J=15Hz), 7.32-7.69 (10H), 8.20 (1H, dd, J=5, 2Hz)

(9) 3-Bromo-8-[2,6-dichloro-3-[N-methyl-N-[(trans-3-pentenoyl)glycyl]amino]benzyloxy]-2-methylimidazo[1,2-a]pyridine hydrochloride

NMR ($CDCl_3$-$CD_3OD$, δ) : 1.73 (3H, d, J=6Hz), 2.58 (3H, s), 2.99 (2H, d, J=6Hz), 3.29 (3H, s), 3.68 (2H, s), 5.40-5.80 (4H), 7.52-7.70 (4H), 8.25 (1H, dd, J=5, 1Hz)

(10) 3-Bromo-8-[3-[N-[(3-butenoyl)glycyl]-N-methylamino]-2,6-dichlorobenzyloxy]-2-methylimidazo[1,2-a]pyridine hydrochloride

NMR ($CDCl_3$-$CD_3OD$, δ) : 2.59 (3H, s), 3.06 (2H, d, J=7Hz), 3.28 (3H, s), 3.68 (2H, s), 5.18-5.31 (2H), 5.61-6.01 (3H), 7.50-7.69 (4H), 8.21 (1H, dd, J=5, 3Hz)

(11) 3-Bromo-8-[2,6-dichloro-3-[N-methyl-N-[(4-phenylbutanoyl)glycyl]amino]benzyloxy]-2-methylimidazo[1,2-a]pyridine hydrochloride

NMR ($CDCl_3$-$CD_3OD$, δ) : 1.83-2.02 (2H), 2.29 (2H, t, J=8Hz), 2.57 (3H, s), 2.66 (2H, t, J=8Hz), 3.28 (3H, s), 3.68 (2H, s), 5.65 (1H, d, J=10Hz), 5.77 (1H, d, J=10Hz), 7.10-7.32 (5H), 7.51-7.69 (4H), 8.22 (1H, dd, J=5, 2Hz)

(12) 3-Bromo-8-[2,6-dichloro-3-[N-[(N,N-dimethyl-β-alanyl)glycyl]-N-methylamino]benzyloxy]-2-methylimidazo[1,2-a]pyridine dihydrochloride

NMR ($CDCl_3$-$CD_3OD$, δ) : 2.60 (3H, s), 2.79-2.95 (8H), 3.25 (2H, s), 3.36-3.49 (2H), 3.60 (1H, d, J=17Hz), 3.82 (1H, d, J=17Hz), 5.67 (1H, d, J=10Hz), 5.77 (1H, d, J=10Hz), 7.53-7.70 (4H), 8.24 (1H, d, J=6Hz)

(13) 3-Bromo-8-[2,6-dichloro-3-[N-[N'-(5-isoquinolyl)ureidoacetyl]-N-methylamino]benzyloxy]-2-methylimidazo[1,2-a]pyridine dihydrochloride

NMR ($CDCl_3$-$CD_3OD$, δ) : 2.58 (3H, s), 3.29 (3H, s), 3.75 (1H, d, J=17Hz), 3.90 (1H, d, J=17Hz), 5.66 (1H, d, J=10Hz), 5.78 (1H, d, J=10Hz), 7.52-7.66 (4H), 7.91 (1H, t, J=8Hz), 8.10 (1H, d, J=8Hz), 8.20 (1H, br s), 8.50 (1H, d, J=6Hz), 8.60 (1H, d, J=8Hz), 8.88 (1H, d, J=6Hz), 9.60 (1H, s)

(14) 3-Bromo-8-[2,6-dichloro-3-[N-methyl-N-[N'-(3-pyrazolyl)ureidoacetyl]amino]benzyloxy]-2-methylimidazo[1,2-a]pyridine dihydrochloride

NMR ($CDCl_3$-$CD_3OD$, δ) : 2.59 (3H, s), 3.30 (3H, s), 3.79 (1H, d, J=17Hz), 3.93 (1H, d, J=17Hz), 5.67 (1H, d, J=10Hz), 5.76 (1H, d, J=10Hz), 6.40 (1H, d, J=3Hz), 7.50-7.70 (4H), 8.12 (1H, d, J=3Hz), 8.21 (1H, dd, J=5, 3Hz)

(15) 3-Bromo-8-[2,6-dichloro-3-[N-methyl-N-[N'-(4-pyrimidinyl)ureidoacetyl]amino]benzyloxy]-2-methylimidazo[1,2-a]pyridine dihydrochloride

NMR ($CDCl_3$-$CD_3OD$, δ) : 2.60 (3H, s), 3.29 (3H, s), 3.71 (1H, d, J=18Hz), 3.88 (1H, d, J=18Hz), 5.71 (2H, br s), 7.52-7.71 (4H), 8.07-8.79 (2H), 8.58 (1H, br s), 8.99 (1H, br s)

(16) 3-Bromo-8-[2,6-dichloro-3-[N-methyl-N-[N'-(6-quinolyl)ureidoacetyl]amino]benzyloxy]-2-methylimidazo[1,2-a]pyridine dihydrochloride

NMR ($CDCl_3$-$CD_3OD$, δ) : 2.59 (3H, s), 3.28 (3H, s), 3.70 (1H, d, J=16Hz), 3.88 (1H, d, J=16Hz), 5.64 (1H, d, J=10Hz), 5.76 (1H, d, J=10Hz), 7.47-7.66 (4H), 7.90 (1H, dd, J=9, 5Hz), 8.04-8.46 (4H), 8.81 (1H, d, J=9Hz), 8.90 (1H, d, J=5Hz)

(17) 3-Bromo-8-[2,6-dichloro-3-[N-[(1-indolylcarbonyl)glycyl]-N-methylamino]benzyloxy]-2-methylimidazo[1,2-a]pyridine hydrochloride

mp : 224°C (dec.)
NMR ($CDCl_3$-$CD_3OD$, δ) : 2.58 (3H, s), 3.31 (3H, s), 3.83 (1H, d, J=17Hz), 3.95 (1H, d, J=17Hz), 5.67 (1H, d, J=10Hz), 5.74 (1H, d, J=10Hz), 6.65 (1H, d, J=4Hz), 7.18-7.38(2H), 7.53-7.70 (6H), 8.12 (1H, d, J=8Hz), 8.21 (1H, m)

(18) 3-Bromo-8-[2,6-dichloro-3-[N-methyl-N-[(morpholinocarbonyl)glycyl]amino]benzyloxy]-2- methylimidazo[1,2-a]pyridine hydrochloride

NMR ($CDCl_3$-$CD_3OD$, δ) : 2.58 (3H, s), 3.27 (3H, s), 3.31-3.48 (4H), 3.61-3.79 (6H), 5.63 (1H, d, J=10Hz), 5.78 (1H, d, J=10Hz), 7.52-7.69 (4H), 8.23 (1H, dd, J=5, 1Hz)

(19)
3-Bromo-8-[2,6-dichloro-3-[N-methyl-N-[N'-[3-(morpholinocarbonyl)phenyl]ureidoacetyl]amino]benzyloxy]-2-methylimidazo[1,2-a]pyridine hydrochloride

NMR ($CDCl_3$-$CD_3OD$, δ) : 2.55 (3H, s), 3.29 (3H, s), 3.41-3.86 (10H), 5.69 (1H, d, J=10Hz), 5.78 (1H, d, J=10Hz), 7.00 (1H, d, J=7Hz), 7.27-7.71 (7H), 8.26 (1H, d, J=5Hz)

(20) 3-Bromo-8-[3-[N-(N'-n-butylureidoacetyl)-N-methylamino]-2,6-dichlorobenzyloxy]-2-methylimidazo[1,2-a]pyridine hydrochloride

NMR ($CDCl_3$-$CD_3OD$, δ) : 0.89 (3H, t, J=6Hz), 1.20-1.41 (2H), 1.48-1.66 (2H), 2.62 (3H, s), 3.11-3.29 (5H), 3.87 (1H, d, J=17Hz), 4.00 (1H, d, J=17Hz), 5.56 (1H, d, J=10Hz), 5.71 (1H, d, J=10Hz), 7.36-7.61 (4H), 8.06 (1H, d, J=6Hz)

(21) 3-Bromo-8-[2,6-dichloro-3-[N-methyl-N-[N'-(3-quinolyl)ureidoacetyl]amino]benzyloxy]-2-methylimidazo[1,2-a]pyridine dihydrochloride

NMR ($CDCl_3$, δ) : 2.69 (3H, s), 3.23 (3H, s), 3.94 (2H, s), 5.59 (1H, d, J=10Hz), 5.69 (1H, d, J=10Hz), 7.36-7.60 (4H), 7.69-7.79 (2H), 8.00 (1H, d, J=9Hz), 8.11 (1H, d, J=6Hz), 8.41 (1H, d, J=9Hz), 9.07 (1H, br s), 9.28 (1H, br s)

(22) 3-Bromo-8-[2,6-dichloro-3-[N-[N'-[3-(1-hydroxyethyl)phenyl]ureidoacetyl]-N-methylamino]benzyloxy]-2-methylimidazo[1,2-a]pyridine hydrochloride

NMR ($CDCl_3$-$CD_3OD$, δ) : 1.42 (3H, d, J=6Hz), 2.53 (3H, s), 3.28 (3H, s), 3.73 (2H, s), 4.79 (1H, q, J=6Hz), 5.62 (1H, d, J=10Hz), 5.77 (1H, d, J=10Hz), 6.99 (1H, d, J=6Hz), 7.13-7.31 (3H), 7.51-7.67 (4H), 8.20 (1H, dd, J=5, 3Hz)

(23) 3-Bromo-8-[2,6-dichloro-3-[N-methyl-N-[4-(methylcarbamoyl)cinnamoylglycyl]amino]benzyloxy]-2-methylimidazo[1,2-a]pyridine hydrochloride

NMR ($CDCl_3$-$CD_3OD$, δ) : 2.52 (3H, s), 3.29 (3H, s), 3.84 (2H, s), 5.64 (1H, d, J=10Hz), 5.70 (1H, d, J=10Hz), 6.65 (1H, d, J=16Hz), 7.28-7.33 (1H, m), 7.45-7.61 (6H, m), 7.77 (2H, d, J=8Hz), 8.07-8.14 (1H, m)

(24) 3-Bromo-8-[2,6-dichloro-3-[N-[4-(dimethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.02 (3H, br s), 3.12 (3H, br s), 3.30 (3H, s), 3.81 (2H, s), 5.66 (1H, d, J=10Hz), 5.77 (1H, d, J=10Hz), 6.68 (1H, d, J=15Hz), 7.37-7.70 (9H), 8.21 (1H, dd, J=5, 2Hz)

(25) 3-Bromo-8-[2,6-dichloro-3-[N-[4-(2-methoxyethyl-carbamoyl)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.29 (3H, s), 3.42 (3H, s), 3.61 (4H, s), 3.81 (2H, s), 5.67 (1H, d, J=10Hz), 5.77 (1H, d, J=10Hz), 6.69 (1H, d, J=15Hz), 7.50-7.70 (7H), 7.82 (2H, d, J=8Hz), 8.21 (1H, dd, J=5, 3Hz)

(26) 8-[3-[N-[4-[N,N-Bis(2-methoxyethyl)carbamoyl]cinnamoylglycyl]-N-methylamino]-2,6- dichlorobenzyloxy]-3-bromo-2-methylimidazo[1,2-a]pyridine hydrochloride

NMR ($CDCl_3$-$CD_3OD$, δ) : 2.60 (3H, s), 3.21-3.87 (16H), 5.68 (1H, d, J=10Hz), 5.77 (1H, d, J=10Hz), 6.65 (1H, d, J=15Hz), 7.34-7.70 (9H), 8.21 (1H, m)

(27) 3-Bromo-8-[2,6-dichloro-3-[N-methyl-N-[4-(4-pyridylcarbamoyl)cinnamoylglycyl]amino]benzyloxy]-2-methylimidazo[1,2-a]pyridine dihydrochloride

NMR ($CDCl_3$-$CD_3OD$, δ) : 2.60 (3H, s), 3.30 (3H, s), 3.84 (2H, br s), 5.67 (1H, d, J=10Hz), 5.77 (1H, d, J=10Hz), 6.72 (1H, d, J=15Hz), 7.44-7.77 (7H), 8.11 (2H, d, J=8Hz), 8.21 (1H, t, J=4Hz), 8.49-8.61 (4H)

(28) 3-Bromo-8-[2,6-dichloro-3-[N-methyl-N-[4-(2-oxo-1-pyrrolidinyl)cinnamoylglycyl]amino]benzyloxy]-2-methylimidazo[1,2-a]pyridine hydrochloride

NMR ($CDCl_3$-$CD_3OD$, δ) : 2.18 (2H, quint, J=7.5Hz), 2.54-2.68 (5H, m), 3.29 (3H, s), 3.81 (2H, s), 3.89 (2H, t, J=7.5Hz), 5.65 (1H, d, J=10Hz), 5.74 (1H, d, J=10Hz), 6.50 (1H, d, J=16Hz), 7.44-7.68 (9H, m), 8.11 (1H, t, J=4Hz)

(29) 3-Bromo-8-[2,6-dichloro-3-[N-[4-(methoxyacetamido)cinnamoylglycyl]-N-methylamino]benzyloxy]-2-methylimidazo[1,2-a]pyridine hydrochloride

NMR ($CDCl_3$-$CD_3OD$, δ) : 2.66 (3H, s), 3.29 (3H, s), 3.51 (3H, s), 3.82 (2H, s), 4.03 (2H, s), 5.60-5.77 (2H, m), 6.50 (1H, d, J=16Hz), 7.40-7.65 (9H, m), 8.04-8.15 (1H, m)

(30) 3-Bromo-8-[2,6-dichloro-3-[N-methyl-N-[4-(propionamido)cinnamoylglycyl]amino]benzyloxy]-2- methylimidazo[1,2-a]pyridine hydrochloride

NMR ($CD_3OD$, δ) : 1.19 (3H, t, J=7.5Hz), 2.40 (2H, q, J=7.5Hz), 2.50 (3H, s), 3.24 (3H, s), 3.79 (1H, d, J=16Hz), 3.86 (1H, d, J=16Hz), 5.69-5.80 (2H, m), 6.60 (1H, d, J=15Hz), 7.36-7.80 (9H, m)

(31) 8-[3-[N-[(4-(Acetamido)cinnamoylglycyl]-N-methylamino]-2,6-dichlorobenzyloxy]-3-bromo-2-methylimidazo[1,2-a]pyridine hydrochloride

NMR ($CDCl_3$-$CD_3OD$, δ) : 2.18 (3H, s), 2.58 (3H, s), 3.28 (3H, s), 3.76 (1H, d, J=16Hz), 3.90 (1H, d, J=16Hz), 5.65 (2H, s), 6.43 (1H, d, J=16Hz), 7.25-7.39 (2H, m), 7.39-7.64 (7H, m), 8.03-8.16 (1H, m)

(32) 3-Bromo-8-[2,6-dichloro-3-[N-[4-(N-methylacetamido)cinnamoylglycyl]-N-methylamino]benzyloxy]-2-methylimidazo[1,2-a]pyridine hydrochloride

NMR ($CDCl_3$-$CD_3OD$, δ) : 1.91 (3H, br s), 2.61 (3H, s), 3.28 (3H, br s), 3.31 (3H, s), 3.81 (2H, br s), 5.66 (1H, br d, J=10Hz), 5.78 (1H, br d, J=10Hz), 6.63 (1H, d, J=15Hz), 7.22 (2H, d, J=9Hz), 7.46-7.70 (7H), 8.20 (1H, br s)

(33) 3-Bromo-8-[2,6-dichloro-3-[N-methyl-N-[4-[N-(3-pyridylmethyl)acetamido]cinnamoylglycyl]amino]benzyloxy]-2-methylimidazo[1,2-a]pyridine dihydrochloride

NMR ($CDCl_3$-$CD_3OD$, δ) : 1.99 (3H, s), 2.60 (3H, s), 3.76 (1H, d, J=18Hz), 3.88 (1H, d, J=18Hz), 5.10 (2H, s), 5.68 (1H, d, J=10Hz), 5.78 (1H, d, J=10Hz), 6.70 (1H, d, J=15Hz), 7.20 (2H, d, J=8Hz), 7.47-7.70 (7H), 8.04 (1H, dd, J=8, 6Hz), 8.22 (1H, dd, J=6, 1Hz), 8.51 (1H, br d, J=8Hz), 8.72-8.84 (2H)

(34) 3-Bromo-8-[2,6-dichloro-3-[N-methyl-N-[4-(isonicotinoylamino)cinnamoylglycyl]amino]benzyloxy]-2-methylimidazo[1,2-a]pyridine dihydrochloride

NMR ($CDCl_3$-$CD_3OD$, δ) : 2.60 (3H, s), 3.30 (3H, s), 3.82 (2H, s), 5.66 (1H, d, J=10Hz), 5.75 (1H, d, J=10Hz), 6.59 (1H, d, J=15Hz), 7.44-7.69 (7H), 7.40 (2H, d, J=9Hz), 8.20 (1H, t, J=5Hz), 8.60 (2H, d, J=6Hz), 9.00 (2H, d, J=6Hz)

(35) 3-Bromo-8-[2,6-dichloro-3-[N-[4-(ethoxycarbonylacetamido)cinnamoylglycyl]-N-methylamino]benzyloxy]-2-methylimidazo[1,2-a]pyridine hydrochloride

NMR ($CDCl_3$-$CD_3OD$, δ) : 1.34 (3H, t, J=7.5Hz), 2.55 (3H, s), 3.28 (3H, s), 3.50 (2H, s), 3.77 (1H, d, J=18Hz), 3.89 (1H, d, J=18Hz), 4.26 (2H, q, J=7.5Hz), 5.60 (2H, s), 6.45 (1H, d, J=16Hz), 7.20-7.35 (2H, m), 7.35-7.64 (7H, m), 7.98 (1H, d, J=6Hz)

(36) 8-[3-[N-[4-(Benzamido)cinnamoylglycyl]-N-methylamino]-2,6-dichlorobenzyloxy]-3-bromo-2-methylimidazo[1,2-a]pyridine hydrochloride

NMR ($CDCl_3$-$CD_3OD$, δ, 3:1 V/V) : 2.60 (3H, s), 3.29 (3H, s), 3.83 (2H, s), 5.66 (1H, d, J=9Hz), 5.74 (1H, d, J=9Hz), 6.56 (1H, d, J=15Hz), 7.36-7.70 (10H, m), 7.78 (2H, d, J=9Hz), 7.93 (2H, dd, J=9, 0.5Hz), 8.21 (1H, m)

(37) 3-Bromo-8-[2,6-dichloro-3-[N-methyl-N-[4-(4-pyridylacetamido)cinnamoylglycyl]amino]benzyloxy]-2-methylimidazo[1,2-a]pyridine dihydrochloride

NMR ($CDCl_3$-$CD_3OD$, δ, 3:1 V/V) : 2.59 (3H, s), 3.29 (3H, s), 3.78 (1H, d, J=16Hz), 3.84 (1H, d, J=16Hz), 4.19 (2H, s), 5.64 (1H, d, J=10Hz), 5.72 (1H, d, J=10Hz), 6.51 (1H, d, J=15Hz), 7.38-7.77 (9H, m), 8.10-8.29 (3H, m), 8.72 (2H, d, J=7Hz)

(38) 3-Bromo-8-[2,6-dichloro-3-[N-[4-(methanesulfonamido)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.00 (3H, s), 3.25 (3H, s), 3.82 (2H, s), 5.63 (1H, d, J=10Hz), 5.73 (1H,

d, J=10Hz), 6.51 (1H, d, J=16Hz), 7.22 (2H, d, J=8Hz), 7.38-7.65 (7H, m), 8.07-8.19 (1H, m)

(39) 3-Bromo-8-[2,6-dichloro-3-[N-methyl-N-[4-(3-methylureido)cinnamoylglycyl]amino]benzyloxy]-2-methylimidazo[1,2-a]pyridine hydrochloride

NMR ($CDCl_3$-$CD_3OD$, δ) : 2.53 (3H, s), 2.80 (3H, s), 3.27 (3H, s), 3.73 (1H, d, J=17Hz), 3.95 (1H, d, J=17Hz), 5.63 (2H, s), 6.34 (1H, d, J=16Hz), 7.19 (2H, d, J=8Hz), 7.24-7.43 (3H, m), 7.43-7.64 (4H, m), 8.05-8.14 (1H, m)

(40) 3-Bromo-8-[2,6-dichloro-3-[N-methyl-N-[4-[3-(3-pyridyl)ureido]cinnamoylglycyl]amino]benzyloxy]-2-methylimidazo[1,2-a]pyridine dihydrochloride

NMR ($CDCl_3$-$CD_3OD$, δ) : 2.60 (3H, s), 3.30 (3H, s), 3.76 (1H, d, J=17Hz), 3.91 (1H, d, J=17Hz), 5.66 (1H, d, J=10Hz), 5.75 (1H, d, J=10Hz), 6.50 (1H, d, J=15Hz), 7.36-7.70 (9H), 7.89 (1H, dd, J=8, 5Hz), 8.20 (1H, t, J=5Hz), 8.31 (1H, d, J=6Hz), 8.63 (1H, br d, J=8Hz), 9.34 (1H, d, J=1Hz)

(41) 3-Bromo-8-[2,6-dichloro-3-[N-methyl-N-[4-(morpholinocarbonylamino)cinnamoylglycyl]amino]benzyloxy]-2-methylimidazo[1,2-a]pyridine hydrochloride

NMR ($CDCl_3$-$CD_3OD$, δ) : 2.60 (3H, s), 3.30 (3H, s), 3.50-3.60 (4H), 3.70-3.85 (6H), 5.66 (1H, d, J=10Hz), 5.76 (1H, d, J=10Hz), 6.49 (1H, d, J=15Hz), 7.39-7.70 (9H), 8.21 (1H, dd, J=5, 3Hz)

(42)
3-Bromo-8-[2,6-dichloro-3-[N-methyl-N-[N'-[3-(4-pyridylcarbamoyl)phenyl]ureidoacetyl]amino]benzyloxy]-2-methylimidazo[1,2-a]pyridine dihydrochloride

NMR ($CDCl_3$-$CD_3OD$, δ) : 2.56 (3H, s), 3.27 (3H, s), 3.83 (2H, s), 5.60 (1H, d, J=10Hz), 5.70 (1H, d, J=10Hz), 7.22-7.38 (1H, m), 7.38-7.67 (6H, m), 7.80-7.93 (1H, m), 8.05-8.16 (1H, m), 8.45 (2H, d, J=7.5Hz), 8.53 (2H, d, J=7.5Hz)

(43)
3-Bromo-8-[2,6-dichloro-3-[N-methyl-N-[N'-[3-(1-pyrrolidinylcarbonyl)phenyl]ureidoacetyl]amino]benzyloxy]-2-methylimidazo[1,2-a]pyridine hydrochloride

NMR (DMSO-$d_6$, δ) : 1.71-1.91 (4H, m), 2.39 (3H, s), 3.13 (3H, s), 5.51-5.65 (2H, m), 6.42-6.51 (1H, m), 7.00 (1H, d, J=7Hz), 7.19-7.44 (3H, m), 7.51-7.75 (2H, m), 7.79-7.88 (2H, m), 8.24 (1H, d, J=6Hz), 9.07-9.16 (1H, m)

(44)
3-Bromo-8-[2,6-dichloro-3-[N-methyl-N-[N'-[3-[N-methyl-N-(3-pyridylmethyl)carbamoyl]phenyl]ureidoacetyl]amino]benzyloxy]-2-methylimidazo[1,2-a]pyridine dihydrochloride

NMR ($CDCl_3$-$CD_3OD$, δ) : 2.54 (3H, s), 3.10 (3H, s), 3.25 (3H, s), 3.65-3.98 (2H, overlapped with $H_2O$), 4.89 (2H, br s), 5.61 (1H, d, J=10Hz), 5.72 (1H, d, J=10Hz), 7.02 (1H, d, J=7Hz), 7.29 (1H, t, J=7Hz), 7.41-7.62 (6H), 8.09 (1H, dd, J=9, 6Hz), 8.18 (1H, t, J=5Hz), 8.60 (1H, br s), 8.80 (1H, d, J=5Hz), 8.91 (1H, br s)

(45)
3-Bromo-8-[2,6-dichloro-3-[N-methyl-N-[N'-[3-(4-methyl-1-piperazinylcarbonyl)phenyl]ureidoacetyl]amino]benzyloxy]-2-methylimidazo[1,2-a]pyridine dihydrochloride

NMR ($CDCl_3$-$CD_3OD$, δ) : 2.54 (3H, s), 2.90 (3H, s), 3.00-3.80 (13H), 5.62 (1H, d, J=10Hz), 5.74 (1H, d, J=10Hz), 7.01 (1H, d, J=7Hz), 7.26-7.68 (7H ), 8.20 (1H, m)

(46)
3-Bromo-8-[2,6-dichloro-3-[N-methyl-N-[N'-[3-(3-pyridylmethylcarbamoyl)phenyl]ureidoacetyl]amino]benzyloxy]-2-methylimidazo[1,2-a]pyridine dihydrochloride

NMR ($CDCl_3$-$CD_3OD$, δ) : 2.51 (3H, s), 3.26 (3H, s), 3.76 (2H, s), 4.76 (2H, s), 5.62 (1H, d, J=10Hz), 5.72 (1H, d, J=10Hz), 7.29 (1H, t, J=8Hz), 7.49-7.74 (7H), 7.99 (1H, dd, J=7, 5Hz), 8.18 (1H, t, J=4Hz), 8.61-8.72 (2H), 8.90 (1H, br s)

(47) 3-Bromo-8-[2,6-dichloro-3-[N-[N'-[3-(dimethylamino)phenyl]ureidoacetyl]-N-methylamino]benzyloxy]-2-methylimidazo[1,2-a]pyridine dihydrochloride

NMR ($CDCl_3$-$CD_3OD$, δ) : 2.59 (3H, s), 3.21 (6H, s), 3.29 (3H, s), 3.66 (1H, d, J=17Hz), 3.89 (1H, d, J=17Hz), 5.64 (1H, d, J=10Hz), 5.76 (1H, d, J=10Hz), 7.21-7.68 (7H), 7.89 (1H, br s), 8.21 (1H, t, J=4Hz)

(48) 3-Bromo-8-[2,6-dichloro-3-[N-[4-(ethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.27 (3H, t, J=7Hz), 2.60 (3H, s), 3.30 (3H, s), 3.47 (2H, q, J=7Hz), 3.83 (2H, s), 5.68 (1H, d, J=10Hz), 5.77 (1H, d, J=10Hz), 6.70 (1H, d, J=15Hz), 7.49-7.70 (7H), 7.82 (2H, d, J=9Hz), 8.22 (1H, dd, J=5, 3Hz)

(49)
3-Bromo-8-[2,6-dichloro-3-[N-[4-(N-ethyl-N-methylcarbamoyl)cinnamoylglycyl]-N-methylamino]benzyloxy]-2-met hylimidazo[1,2-a]pyridine hydrochloride

NMR ($CDCl_3$-$CD_3OD$, δ) : 1.11-1.33 (3H), 2.60 (3H, s), 2.94-3.12 (3H), 3.23-3.42 (4H), 3.59 (1H, m), 3.81 (2H, s), 5.68 (1H, d, J=10Hz), 5.77 (1H, d, J=10Hz), 6.68 (1H, d, J=15Hz), 7.37-7.69 (9H), 8.21 (1H, dd, J=5, 3Hz)

(50)
3-Bromo-8-[2,6-dichloro-3-[N-methyl-N-[4-(1-pyrrolidinylcarbonyl)cinnamoylglycyl]amino]benzyloxy]-2-methylimi dazo[1,2-a]pyridine hydrochloride

NMR ($CDCl_3$-$CD_3OD$, δ) : 1.78-2.07 (4H, m), 2.63 (3H, s), 3.28 (3H, s), 3.34-3.51 (2H, m), 3.51-3.70 (2H, m), 3.76 (1H, d, J=18Hz), 3.90 (1H, d, J=18Hz), 5.63 (1H, d, J=10Hz), 5.72 (1H, d, J=10Hz), 6.63 (1H, d, J=16Hz), 7.38-7.67 (9H, m), 8.03-8.17 (1H, m)

(51) 3-Bromo-8-[2,6-dichloro-3-[N-methyl-N-[4-(morpholinocarbonyl)cinnamoylglycyl]amino]benzyloxy]-2-methylimidazo[1,2-a]pyridine hydrochloride

NMR ($CDCl_3$-$CD_3OD$, δ) : 2.61 (3H, s), 3.30 (3H, s), 3.35-3.95 (10H, m), 5.64 (1H, d, J=10Hz), 5.74 (1H, d, J=10Hz), 6.65 (1H, d, J=16Hz), 7.40 (2H, d, J=8Hz), 7.46-7.65 (7H, m), 8.09-8.18 (1H, m)

(52)
3-Bromo-8-[2,6-dichloro-3-[N-[4-[N-(2-methoxyethyl)N-methylcarbamoyl]cinnamoylglycyl]-N-methylamino]benzyl oxy]-2-methylimidazo[1,2-a]pyridine hydrochloride

NMR ($CDCl_3$-$CD_3OD$, δ) : 2.62 (3H, s), 3.00-3.15 (3H, m), 3.60-3.93 (4H, m), 5.63 (1H, d, J=10Hz), 5.73 (1H, d, J=10Hz), 6.61 (1H, d, J=16Hz), 7.41 (2H, d, J=8Hz), 7.47-7.65 (7H, m), 8.08-8.20 (1H, m)

(53) 3-Bromo-8-[2,6-dichloro-3-[N-[4-(isopropylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.28 (6H, d, J=6Hz), 2.63 (3H, s), 3.29 (3H, s), 3.77 (1H, d, J=16Hz), 3.90 (1H, d, J=16Hz), 4.25 (1H, m), 5.63 (1H, d, J=10Hz), 5.72 (1H, d, J=10Hz), 6.65 (1H, d, J=16Hz), 7.41-7.63 (7H, m), 7.74 (2H, d, J=8Hz), 8.04-8.17 (1H, m)

(54) 3-Bromo-8-[2,6-dichloro-3-[N-[4-(n-propylcarbamoyl)cinnamoylglycyl]-N-methylamino]benzyloxy]-2-methylimidazo[1,2-a]pyridine hydrochloride

NMR ($CDCl_3$-$CD_3OD$, δ) : 0.99 (3H, t, J=7.5Hz), 1.65 (2H, m), 2.61 (3H, s), 3.30 (3H, s), 3.34-3.47 (2H, m), 3.84 (2H, s-like), 5.63 (1H, d, J=10Hz), 5.72 (1H, d, J=10Hz), 6.66 (1H, d, J=16Hz), 7.40-7.65 (7H, m), 7.77 (2H, d, J=8Hz), 8.05-8.16 (1H, m)

(55)
3-Bromo-8-[2,6-dichloro-3-[N-[4-(3-methoxypropylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.90 (2H, quint, J=6Hz), 2.64 (3H, s), 3.28 (3H, s), 3.40 (3H, s), 3.55 (4H, q, J=6Hz), 3.78 (1H, d, J=17.5Hz), 3.89 (1H, d, J=17.5Hz), 5.64 (1H, d, J=10Hz), 5.73 (1H, d, J=10Hz), 6.74 (1H, d, J=16Hz), 7.44-7.64 (7H, m), 7.75 (2H, d, J=8Hz), 8.05-8.16 (1H, m)

(56)
3-Bromo-8-[2,6-dichloro-3-[N-[4-(2-ethoxyethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.22 (3H, t, J=7Hz), 3.63 (3H,s), 3.50-3.71 (6H, m), 3.88 (1H, d, J=18Hz), 3.90 (1H, d, J=18Hz), 5.65 (1H, d, J=10Hz), 5.72 (1H, d, J=10Hz), 6.65 (1H, d, J=16Hz), 7.46-7.65 (7H, m), 7.79 (2H, d, J=8Hz), 8.05-8.14 (1H, m)

(57)
3-Bromo-8-[2,6-dichloro-3-[N-[4-(2-hydroxyethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.26 (3H, s), 3.57 (2H, t, J=5Hz), 3.78 (2H, t, J=5Hz), 3.85 (2H, s), 5.59-5.74 (2H, m), 6.64 (2H, d, J=16Hz), 7.40-7.64 (7H, m), 7.82 (2H, d, J=8Hz), 8.03-8.15 (1H, m)

(58) 3-Bromo-8-[2,6-dichloro-3-[N-[4-(diethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.03-1.33 (6H, m), 2.65 (3H, s), 3.13-3.35 (5H, m), 3.45-3.63 (2H, m), 3.77 (1H, d, J=18Hz), 3.90 (1H, d, J=18Hz), 5.63 (1H, d, J=10Hz), 5.71 (1H, d, J=10Hz), 6.60 (1H, d, J=16Hz), 7.33 (2H, d, J=8Hz), 7.41-7.63 (7H, m), 8.02-8.13 (1H, m)

(59) 3-Bromo-8-[2,6-dichloro-3-[N-methyl-N-[4-(2-oxopiperidino)cinnamoylglycyl]amino]benzyloxy]-2- methylimidazo[1,2-a]pyridine hydrochloride

NMR ($CDCl_3$-$CD_3OD$, δ) : 1.90-2.03 (4H, m), 2.50-2.66 (5H, m), 3.60-3.72 (2H, m), 3.80 (2H, s), 5.65 (1H, d, J=10Hz), 5.75 (1H, d, J=10Hz), 6.55 (1H, d, J=16Hz), 7.27 (2H, d, J=8Hz), 7.45-7.67 (7H, m), 8.09-8.21 (1H, m)

(60)
3-Bromo-8-[2,6-dichloro-3-[N-methyl-N-[4-(N-methyl-2-methoxyacetamido)cinnamoylglycyl]amino]benzyloxy]-2-methylimidazo[1,2-a]pyridine hydrochloride

NMR ($CDCl_3$-$CD_3OD$, δ, 3:1 V/V) : 2.60 (3H, s), 3.30 (6H, s), 3.35 (3H, s), 3.76-3.96 (4H, m), 5.68 (1H, d, J=9Hz), 5.76 (1H, d, J=9Hz), 6.69 (1H, d, J=15Hz), 7.26 (2H, br d, J=8Hz), 7.48-7.73 (7H, m), 8.24 (1H, br d, J=8Hz)

(61) 3-Bromo-8-[2,6-dichloro-3-[N-methyl-N-[4-(1-pyrrolyl)cinnamoylglycyl]amino]benzyloxy]-2-methylimidazo[1,2-a]pyridine hydrochloride

NMR ($CDCl_3$-$CD_3OD$, δ) : 2.61 (3H, s), 3.30 (3H, s), 3.81 (2H, s), 5.68 (1H, d, J=10Hz), 5.78 (1H, d, J=10Hz), 6.39 (2H, s), 6.60 (1H, d, J=15Hz), 7.38-7.70 (11H), 8.20 (1H, dd, J=5, 3Hz)

(62) 3-Bromo-8-[2,6-dichloro-3-[N-[4-(3-methoxypropionamido)cinnamoylglycyl]-N-methylamino]benzyloxy]-2-methylimidazo[1,2-a]pyridine hydrochloride

NMR ($CDCl_3$-$CD_3OD$, δ) : 2.55-2.70 (5H, m), 3.30 (3H, s), 3.44 (3H, s), 3.76 (2H, t, J=6Hz), 3.83 (2H, s), 5.67 (2H, s), 6.46 (1H, d, J=16Hz), 7.33-7.63 (9H, m), 8.02-8.14 (1H, m)

(63) 8-[3-[N-[4-(Acetamidoacetamido)cinnamoylglycyl]-N-methylamino]-2,6-dichlorobenzyloxy]-3-bromo-2- methylimidazo[1,2-a]pyridine hydrochloride

NMR ($CDCl_3$-$CD_3OD$, δ) : 2.07 (3H, s), 2.56 (3H, s), 3.27 (3H, s), 3.75 (1H, d, J=17Hz), 3.88 (1H, d, J=17Hz), 4.00 (2H, s), 5.59-5.75 (2H, m), 6.48 (1H, d, J=16Hz), 7.35-7.45 (2H, m), 7.45-7.67 (7H, m), 8.08-8.19 (1H, m)

(64)
3-Bromo-8-[2,6-dichloro-3-[N-methyl-N-[4-[(dimethylamino)acetamido]cinnamoylglycyl]amino]benzyloxy]-2-methylimidazo[1,2-a]pyridine hydrochloride

NMR ($CDCl_3$-$CD_3OD$, δ) : 2.58 (3H, s), 3.04 (6H, s), 3.27 (3H, s), 3.83 (2H, br s), 4.21 (2H, br s), 5.64 (2H, br s), 6.46 (1H, d, J=16Hz), 7.26-7.67 (9H, m), 8.06-8.16 (1H, m)

(65) 3-Bromo-8-[2,6-dichloro-3-[N-[4-(succinimido)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 2.95 (4H, s), 3.30 (3H, s), 3.81 (2H, s), 5.68 (1H, br d, J=10Hz), 5.78 (1H, dr d, J=10Hz), 6.64 (1H, d, J=16Hz), 7.35 (2H, d, J=9Hz), 7.50-7.60 (7H), 8.21 (1H, m)

(66)
3-Bromo-8-[2,6-dichloro-3-[N-[4-[(E)-3-(ethoxycarbonyl)acrylamido]cinnamoylglycyl]-N-methylamino]benzyloxy]-2-methylimidazo[1,2-a]pyridine hydrochloride

NMR ($CDCl_3$-$CD_3OD$, δ, 3:1, V/V) : 1.36 (3H, t, J=7.5Hz), 2.49 (3H, s), 3.28 (3H, s), 3.37 (2H, q, J=7.5Hz), 3.81 (2H, s), 5.66 (1H, d, J=9Hz), 5.75 (1H, d, J=9Hz), 6.56 (1H, d, J=15Hz), 6.91 (1H, d, J=15Hz), 7.17 (1H, d, J=15Hz), 7.36-7.80 (9H, m), 8.20 (1H, br d, J=4Hz)

(67) 3-Bromo-8-[2,6-dichloro-3-[N-[4-(2-oxo-3-oxazolidinyl)cinnamoylglycyl]-N-methylamino]benzyloxy]-2-methylimidazo[1,2-a]pyridine hydrochloride

NMR ($CDCl_3$-$CD_3OD$ , δ) : 2.60 (3H, s), 3.28 (3H, s), 3.81 (2H, s), 4.11 (2H, dd, J=6, 8Hz), 4.52 (2H, dd, J=6, 8Hz), 5.64 (1H, d, J=10Hz), 5.74 (1H, d, J=10Hz), 6.54 (1H, d, J=16Hz), 7.37-7.69 (9H, m), 8.07-8.20 (1H, m)

(68)
8-[3-[N-[N'-[3-[N,N-Bis(2-methoxyethyl)carbamoyl]phenyl]ureidoacetyl]-N-methylamino]-2,6-dichlorobenzyloxy]-3-bromo-2-methylimidazo[1,2-a]pyridine hydrochloride

NMR ($CDCl_3$-$CD_3OD$, δ) : 2.56 (3H, s), 3.22 (3H, s), 3.52-3.73 (4H, m), 3.79 (1H, d, J=18Hz), 4.01 (1H, d, J=18Hz), 5.58 (1H, d, J=10Hz), 5.65 (1H, d, J=10Hz), 6.88 (1H, d, J=7.5Hz), 7.20 (1H, t, J=8Hz), 7.33-7.56 (6H, m), 8.06 (1H, d, J=6Hz)

(69)
3-Bromo-8-[2,6-dichloro-3-[N-[N'-[3-(2-methoxyethylcarbamoyl)phenyl]ureidoacetyl]-N-methylamino]benzyloxy]-2-methylimidazo[1,2-a]pyridine hydrochloride

NMR ($CDCl_3$-$CD_3OD$, δ) : 2.58 (3H, s), 3.24 (3H, s), 3.38 (3H, s), 3.55 (4H, s-like), 3.88 (2H, s), 5.61 (2H, s), 7.19 (1H, t, J=8Hz), 7.25-7.40 (1H, m), 7.40-7.54 (5H, m), 7.60-7.66 (1H, m), 8.07 (1H, d, J=6Hz)

(70)
3-Bromo-8-[2,6-dichloro-3-[N-[N'-[3-[N-(2-methoxyethyl)-N-methylcarbamoyl]phenyl]ureidoacetyl]-N-methylamino]benzyloxy]-2-methylimidazo[1,2-a]pyridine hydrochloride

NMR ($CDCl_3$-$CD_3OD$, δ) : 2.53 (3H, s), 2.94-3.07 (3H, m), 3.55-3.69 (2H, br peak), 3.75 (1H, d, J=16Hz), 3.87 (1H, d, J=16Hz), 5.58 (1H, d, J=10Hz), 5.69 (1H, d, J=10Hz), 6.92 (1H, d, J=7.5Hz), 7.22 (1H, t, J=7.5Hz), 7.28-7.59 (6H, m), 8.05-8.15 (1H, m)

(71)
3-Bromo-8-[2,6-dichloro-3-[N-[N'-[3-[N,N-bis(2-ethoxyethyl)carbamoyl]phenyl]ureidoacetyl]-N-methylamino]benzyloxy]-2-methylimidazo[1,2-a]pyridine hydrochloride

NMR ($CDCl_3$-$CD_3OD$, δ) : 1.05-1.28 (6H, m), 2.54 (3H, s), 3.25 (3H, s), 3.30-3.80 (13H, m), 3.86 (1H, d, J=17.5Hz), 5.58 (1H, d, J=10Hz), 5.67 (1H, d, J=10Hz), 6.94 (1H, d, J=7.5Hz), 7.15-7.35 (2H, m), 7.35-7.60 (5H, m),8.07 (1H, d, J=6Hz)

(72)
3-Bromo-8-[2,6-dichloro-3-[N-[N'-[3-[N,N-bis(2-hydroxyethyl)carbamoyl]phenyl]ureidoacetyl]-N-methylamino]benzyloxy]-2-methylimidazo[1,2-a]pyridine hydrochloride

NMR ($CDCl_3$-$CD_3OD$, δ) : 2.55 (3H, s), 3.23 (3H, s), 3.53-3.75 (4H, br peak), 3.75-3.94 (4H, br peak), 5.57 (1H, d, J=10Hz), 5.67 (1H, d, J=10Hz), 6.99 (1H, d, J=7.5Hz), 7.21 (1H, t, J=7.5Hz), 7.25-7.60 (6H, m), 8.08 (1H, d, J=6Hz)

(73)
3-Bromo-8-[2,6-dichloro-3-[N-[N'-[3-(methoxyacetamido)phenyl]ureidoacetyl]-N-methylamino]benzyloxy]-2-methylimidazo[1,2-a]pyridine hydrochloride

NMR ($CDCl_3$-$CD_3OD$, δ) : 2.54 (3H, s), 3.28 (3H, s), 3.51 (3H, s), 3.74 (2H, s), 3.92-4.09 (2H, overlapped with $H_2O$), 5.63 (1H, d, J=10Hz), 5.74 (1H, d, J=10Hz), 7.13-7.20 (3H), 7.51-7.66 (5H), 8.19 (1H, t, J=3Hz)

(74)
3-Bromo-8-[2,6-dichloro-3-[N-[N'-[3-[(ethoxycarbonyl)acetamido]phenyl]ureidoacetyl]-N-methylamino]benzyloxy]-2-methylimidazo[1,2-a]pyridine hydrochloride

NMR ($CDCl_3$-$CD_3OD$, δ) : 1.31 (3H, t, J=7Hz), 2.52 (3H, s), 3.28 (3H, s), 3.47 (2H, s), 3.75 (2H, br s), 4.23 (2H, g, J=7Hz), 5.63 (1H, br d, J=10Hz), 5.73 (1H, br d, J=10Hz), 7.11-7.20 (3H), 7.49-7.66 (5H), 8.18 (1H, br t, J=4Hz)

(75) 3-Chloro-8-[2,6-dichloro-3-[N-[4-(dimethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.02 (3H, br s), 3.12 (3H, br s), 3.30 (3H, s), 3.81 (2H, s), 5.68 (1H, d, J=10Hz), 5.78 (1H, d, J=10Hz), 6.69 (1H, d, J=16Hz), 7.41-7.70 (9H), 8.20 (1H, t, J=4Hz)

(76) 3-Chloro-8-[2,6-dichloro-3-[N-methyl-N-[4-(methylcarbamoyl)cinnamoylglycyl]amino]benzyloxy]-2-methylimidazo[1,2-a]pyridine hydrochloride

NMR ($CDCl_3$-$CD_3OD$, δ) : 2.61 (3H, s), 3.98 (3H, s), 3.29 (3H, s), 3.84 (2H, s), 5.62 (1H, d, J=10Hz), 5.70 (1H, d, J=10Hz), 6.64 (1H, d, J=16Hz), 7.42-7.66 (7H, m), 7.77 (2H, d, J=8Hz), 8.02-8.14

(1H, m)

(77) 3-Chloro-8-[2,6-dichloro-3-[N-[4-[(2-methoxyethyl)carbamoyl]cinnamoylglycyl]-N-methylamino]benzyloxy]-2-methylimidazo[1,2-a]pyridine hydrochloride

NMR ($CDCl_3$-$CD_3OD$, δ) : 2.61 (3H, s), 3.29 (3H, s), 3.41 (3H, 5), 3.47-3.73 (4H, overlapped with $H_2O$), 3.82 (2H, br s), 5.65 (1H, d, J=10Hz), 5.74 (1H, d, J=10Hz), 6.68 (1H, d, J=15Hz), 7.48-7.66 (7H), 7.80 (2H, br d, J=9Hz), 8.14 (1H, t, J=4Hz)

(78) 3-Chloro-8-[2,6-dichloro-3-[N-[4-(N-ethyl-N-methylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.07-1.33 (3H, m), 2.61 (3H, s), 3.27 (3H, s), 3.44-3.66 (2H, m), 3.76 (1H, d, J=18Hz), 3.88 (1H, d, J=18Hz), 5.63 (1H, d, J=10Hz), 5.73 (1H, d, J=10Hz), 6.61 (1H, d, J=16Hz), 7.23-7.44 (2H, m), 7.44-7.63 (7H, m), 8.01-8.15 (1H, m)

(79) 3-Chloro-8-[2,6-dichloro-3-[N-[4-(ethylcarbamoyl)cinnamoylglycyl]-N-methylamino]benzyloxy]-2-methylimidazo[1,2-a]pyridine hydrochloride

NMR ($CDCl_3$-$CD_3OD$, δ) : 1.25 (3H, t, J=7.5Hz), 2.61 (3H, s), 3.26 (3H, s), 3.45 (2H, q, J=7.5Hz) 3.78 (1H, d, J=17.5Hz), 3.90 (1H, d, J=17.5Hz), 5.63 (1H, d, J=10Hz), 5.70 (1H, d, J=10Hz), 6.64 (1H, d, J=16Hz), 7.41-7.64 (7H, m), 7.76 (2H, d, J=8Hz), 8.00-8.13 (1H, m)

(80) 3-Chloro-8-[2,6-dichloro-3-[N-methyl-N-[4-(2-oxo-1-pyrrolidinyl)cinnamoylglycyl]amino]benzyloxy]-2-methylimidazo[1,2-a]pyridine hydrochloride

NMR ($CDCl_3$, δ) : 2.20 (2H, quint, J=7Hz), 2.55-2.70 (5H, m), 3.28 (3H, s), 3.80 (3H, s), 3.90 (2H, t, J=7.5Hz), 5.65 (1H, d, J=10Hz), 5.74 (1H, d, J=10Hz), 6.51 (1H, d, J=16Hz), 7.40-7.58 (9H, m), 8.04-8.15 (1H, m)

(81) 8-[3-[N-[4-(Acetamido)cinnamoylglycyl]-N-methylamino]-2,6-dichlorobenzyloxy]-3-chloro-2- methylimidazo[1,2-a]pyridine hydrochloride

NMR ($CDCl_3$-$CD_3OD$, δ) : 2.19 (3H, s), 2.60 (3H, s), 3.28 (3H, s), 3.78 (1H, d, J=18Hz), 3.89 (1H, d, J=18Hz), 5.67 (2H, s), 6.46 (1H, d, J=16Hz), 7.24-7.64 (9H, m), 8.02-8.13 (1H, m)

(82) 3-Chloro-8-[2,6-dichloro-3-[N-methyl-N-[4-(3-methylureido)cinnamoylglycyl]amino]benzyloxy]-2-methylimidazo[1,2-a]pyridine hydrochloride

NMR ($CDCl_3$-$CD_3OD$, δ) : 2.58 (3H, s), 2.81 (3H, s), 3.29 (3H, s), 3.74 (1H, d, J=17Hz), 3.83-4.00 (1H, overlapped with $H_2O$), 5.63 (1H, d, J=10Hz), 5.71 (1H, d, J=10Hz), 6.40 (1H, d, J=16Hz), 7.28-7.46 (5H), 7.51-7.68 (4H), 8.18 (1H, dd, J=5.1Hz)

(83) 3-Chloro-8-[2,6-dichloro-3-[N-[4-(methoxyacetamido)cinnamoylglycyl]-N-methylamino]benzyloxy]-2-methylimidazo[1,2-a]pyridine hydrochloride

NMR ($CDCl_3$-$CD_3OD$, δ) : 2.60 (3H, s), 3.29 (3H, s), 3.52 (3H, s), 3.64-3.98 (2H, overlapped with $H_2O$), 4.03 (2H, s), 5.66 (1H, d, J=10Hz), 5.77 (1H, d, J=10Hz), 6.52 (1H, d, J=16Hz), 7.42-7.68 (9H), 8.19 (1H, t, J=4Hz)

(84) 3-Chloro-8-[2,6-dichloro-3-[N-methyl-N-[4-(propionamido)cinnamoylglycyl]amino]benzyloxy]-2-methylimidazo[1,2-a]pyridine hydrochloride

NMR ($CDCl_3$-$CD_3OD$, δ) : 1.23 (3H, t, J=7.5Hz), 2.42 (2H, q, J=7.5Hz), 2.59 (3H, s), 3.28 (3H, s), 3.76 (1H, d, J=17.5Hz), 3.88 (1H, d, J=17.5Hz), 5.66 (2H, s), 6.43 (1H, d, J=16Hz), 7.32-7.63 (9H, m), 8.01-8.12 (1H, m)

(85) 3-Chloro-8-[2,6-dichloro-3-[N-[4-(3-methoxypropionamido)cinnamoylglycyl)-N- methylamino]benzyloxy]-2-methylimidazo[1,2-a]pyridine hydrochloride

NMR ($CDCl_3$-$CD_3OD$, δ) : 2.55-2.70 (5H), 3.29 (3H, s), 3.92 (3H, s), 3.70-4.00 (4H, overlapped with $H_2O$), 5.61-5.69 (2H), 6.50 (1H, d, J=16Hz), 7.39-7.68 (9H), 8.18 (1H, br s)

(86) 8-[3-[N-[4-(Acetamidoacetamido)cinnamoylglycyl]-N-methylamino]-2,6-dichlorobenzyloxy]-3-chloro-2-methylimidazo[1,2-a]pyridine hydrochloride

NMR ($CDCl_3$-$CD_3OD$, δ) : 2.09 (3H, s), 2.59 (3H, s), 3.82 (2H, d, J=6Hz), 4.00 (2H, s), 5.68 (2H, s-like), 6.47 (2H, d, J=16Hz), 7.35-7.69 (9H, m), 8.06-8.15 (1H, m)

(87) 8-[3-[N-[N'-[3-[N,N-Bis(2-methoxyethyl)carbamoyl]phenyl]ureidoacetyl]-N-methylamino]-2,6-dichlorobenzyloxy]-3-chloro-2-methylimidazo[1,2-a]pyridine hydrochloride

NMR ($CDCl_3$-$CD_3OD$, δ) : 2.54 (3H, s), 3.28 (3H, s), 3.31-3.79 (16Hz), 5.63 (1H, d, J=10Hz), 5.75 (1H, d, J=10Hz), 6.99 (1H, d, J=7Hz), 7.20-7.48 (3H), 7.51-7.67 (4H), 8.18 (1H, t, J=4Hz)

(88) 3-Chloro-8-[2,6-dichloro-3-[N-methyl-N-[N'-[3-(4-pyridylcarbamoyl)phenyl]ureidoacetyl]amino]benzyloxy]-2-meth ylimidazo[1,2-a]pyridine dihydrochloride

NMR ($CDCl_3$-$CD_3OD$, δ, 3:1 V/V) : 2.55 (3H, s), 3.27 (3H, s), 3.70 (1H, d, J=15Hz), 3.79 (1H, d, J=15Hz), 5.65 (1H, d, J=9Hz), 5.75 (1H, d, J=9Hz), 7.39 (1H, d, J=8Hz), 7.51-7.75 (6H, m), 7.98 (1H, m), 8.15-8.26 (1H, m), 8.50 (2H, d, J=8Hz), 8.56 (1H, d, J=8Hz)

(89) 3-Bromo-8-[2,6-dichloro-3-[N-methyl-N-[(E)-3-(6-methylcarbamoyl-3-pyridyl)acryloylglycyl]amino]benzyloxy]-2-m ethylimidazo[1,2-a]pyridine dihydrochloride

NMR ($CDI_3$-$CD_3OD$, δ) : 2.62 (3H, s), 3.05 (3H, s), 3.29 (3H, s), 3.70-3.85 (1H, overlapped with $H_2O$), 3.91 (1H, d, J=18Hz), 5.64 (1H, d, J=10Hz), 5.73 (1H, d, J=10Hz), 6.90 (1H, d, J=16Hz), 7.50-7.67 (5H), 8.11-8.27 (2H), 8.32 (1H, br d, J=8Hz), 8.82 (1H, br s)

(90) 8-[3-[N-[(E)-3-(6-Acetamido-3-pyridyl)acryloylglycyl]-N-methylamino]-2,6-dichlorobenzyloxy]-3-bromo-2-methyli midazo[1,2-a]pyridine dihydrochloride

NMR ($CDCl_3$-$CD_3OD$, δ) : 2.41 (3H, s), 2.60 (3H, s), 3.28 (3H, s), 3.75 (1H, d, J=18Hz), 3.91 (1H, d, J=18Hz), 5.63 (1H, d, J=10Hz), 5.75 (1H, d, J=10Hz), 6.92 (1H, d, J=16Hz), 7.40-7.65 (5H), 8.00 (1H, d, J=9Hz), 8.20 (1H, t, J=5Hz), 8.46-8.61 (2H)

(91) 3-Bromo-8-[2,6-dichloro-3-[N-[4-(2-hydroxyethoxy)cinnamoylglycyl]-N-methylamino]benzyloxy]-2-methylimidazo[1,2-a]pyridine hydrochloride

NMR ($CDCl_3$-$CD_3OD$) : 2.63 (3H, s), 3.29 (3H, s), 3.80 (2H, s), 3.94 (2H, t, J=5Hz), 4.10 (2H, t, J=5Hz), 5.60-5.77 (2H, m), 6.43 (1H, d, J=16Hz), 6.90 (2H, d, J=8Hz), 7.40-7.64 (7H, m), 8.07-8.17 (1H, m)

(92) 3-Bromo-8-[2,6-dichloro-3-[N-(3,4-dimethoxycinnamoylglycyl)-N-methylamino]benzyloxy]-2-methylimidazo[1,2-a]pyridine hydrochloride

NMR ($CDCl_3$-$CD_3OD$, δ) : 2.64 (3H, s), 3.30 (3H, s), 3.80 (2H, s), 3.91 (3H, s), 3.94 (3H, s), 5.65 (1H, d, J=10Hz), 5.75 (1H, d, J=10Hz), 6.44 (1H, d, J=16Hz), 6.87 (1H, d, J=8Hz), 7.03-7.15

(2H, m), 7.39-7.65 (5H, m), 8.05-8.19 (1H, m)

(93) 3-Bromo-8-[2,6-dichloro-3-[N-methyl-N-[3,4-(methylenedioxy)cinnamoylglycyl]amino]benzyloxy]-2-methylimidazo[1,2-a]pyridine hydrochloride

NMR ($CDCl_3$, δ) : 2.60 (3H, s), 3.80 (3H, s), 5.65 (1H, d, J=10Hz), 5.74 (1H, d, J=10Hz), 6.00 (2H, s), 6.40 (1H, d, J=16Hz), 6.80 (1H, d, J=8Hz), 6.94-7.04 (2H, m), 7.37-7.66 (5H, m), 8.09-8.19 (1H, m)

(94) 3-Bromo-8-[2,6-dichloro-3-[N-[(E)-3-(6-dimethylcarbamoyl-3-pyridyl)acryloylglycyl]-N-methylamino]benzyloxy]-2-methylimidazo[1,2-a]pyridine dihydrochloride

NMR ($CDCl_3$-$CD_3OD$, δ) : 2.53 (3H, s), 3.02 (3H, br s), 3.10 (3H, br s), 3.20 (3H, s), 3.70 (1H, d, J=17Hz), 3.86 (1H, d, J=17Hz), 5.56 (1H, d, J=10Hz), 5.67 (1H, d, J=10Hz), 6.85 (1H, d, J=16Hz) 7.38-7.59 (5H), 7.71 (1H, br d, J=8Hz), 8.09 (1H, t, J=4Hz), 8.21 (1H, br d, J=8Hz), 8.80 (1H, br s)

(95) 3-Bromo-8-[2,6-dichloro-3-[N-[(E)-3-(6-ethylcarbamoyl-3-pyridyl)acryloylglycyl]-N-methylamino]benzyloxy]-2-methylimidazo[1,2-a]pyridine dihydrochloride

NMR ($CDCl_3$-$CD_3OD$, δ) : 1.22 (3H, t, J=7Hz), 2.57 (3H, s), 3.46 (1H, q, J=7Hz), 3.73 (1H, d, J=17HZ), 3.89 (1H, d, J=17Hz), 5.58 (1H, d, J=10Hz), 5.68 (1H, d, J=10Hz), 6.81 (1H, d, J=16Hz), 7.38-7.59 (5H), 8.00-8.19 (2H), 8.26 (1H, d, J=8Hz), 8.75 (1H, br s)

(96) 3-Bromo-8-[2,6-dichloro-3-[N-[(E)-3-[6-(3-methoxypropylcarbamoyl)-3-pyridyl]acryloylglycyl]-N-methylamino]benzyloxy]-2-methylimidazo[1,2-a]pyridine dihydrochloride

NMR ($CDCl_3$-$CD_3OD$, δ) : 1.85 (2H, quint, J=6Hz), 2.58 (3H, s), 3.23 (3H, s), 3.41-3.60 (4H, m), 3.73 (1H, d, J=17.5Hz), 3.88 (1H, d, J=17.5Hz), 5.57 (1H, d, J=10Hz), 5.67 (1H, d, J=10Hz), 6.78 (1H, d, J=16Hz), 7.37-7.60 (5H, m), 7.98-8.11 (2H, m), 8.17 (1H, d, J=8Hz), 8.66-8.74 (1H, m)

Example 94

3-Chloro-8-(2,6-dichlorophenyl)methylamino-2-methylimidazo[1,2-a]pyridine was obtained according to a similar manner to that of Example 2.

mp : 142.9°C (dec.)

Example 95

Sulfuric acid salt of 3-bromo-8-[2,6-dichloro-3-[N-methyl-N-(butyrylglycyl)amino]benzyloxy]-2-methylimidazo[1,2-a]pyridine was obtained by mixing 3-bromo-8-[2,6-dichloro-3-[N-methyl-N-(butyrylglycyl)amino]benzyloxy]-2-methylimidazo[1,2-a]pyridine with sulfuric acid.

mp : 154-156°C

Example 96

Maleic acid salt of 3-chloro-8-[2,6-dichloro-3-(N-methyl-N-acetylamino)benzyloxy]-2-methylimidazo[1,2-a]pyridine was obtained by mixing 3-chloro-8-[2,6-dichloro-3-(N-methyl-N-acetylamino)benzyloxy]-2-methylimidazo[1,2-a]pyridine with maleic acid.

mp : 193-195°C

Example 97

Methanesulfonic acid salt of 3-chloro-8-[2,6-dichloro-3-(N-methyl-N-acetylamino)benzyloxy]-2- methylimidazo[1,2-a]pyridine was obtained by mixing 3-chloro-8-[2,6-dichloro-3-(N-methyl-N-acetylamino)benzyloxy]-2-methylimidazo[1,2-a]pyridine with methanesulfonic acid.

mp : 165°C (dec.)

Example 98

Oxalic acid salt of 3-chloro-8-[2,6-dichloro-3-(N-methyl-N-acetylamino)benzyloxy]-2-methylimidazo[1,2-a]pyridine was obtained by mixing 3-chloro-8-[2,6-dichloro-3-(N-methyl-N-acetylamino)benzyloxy]-2-methylimidazo[1,2-a]pyridine with oxalic acid.

mp : 180-181°C

**Claims**

**1.** A compound of the formula:

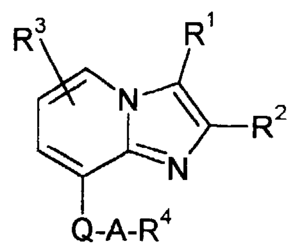

wherein

$R^1$ is halogen,
$R^2$ is $(C_1-C_6)$alkyl,
$R^3$ is hydrogen,
$R^4$ is phenyl substituted with substituent(s) selected from the group consisting of halogen; $(C_1-C_6)$alkyl; halo$(C_1-C_6)$alkyl; carboxy; $(C_1-C_6)$ alkoxycarbonyl; benzoyl; 4-methyl-1-piperazinylcarbonyl; phenyl; phenyl substituted with halogen or cyano; phenyl$(C_1-C_6)$alkyl substituted with hydroxy; $(C_1-C_6)$ alkoxy; nitro; amino; amino substituted with substituent(s) selected from the group consisting of $(C_1-C_6)$alkyl,

acyl which is: alkanoyl selected from formyl, acetyl, propionyl, butyryl, isobutyryl, valeryl, isovaleryl, pivaloyl, hexanoyl, heptanoyl or 3,3-dimethylbutyryl, halo$(C_1-C_6)$alkanoyl, hydroxy$(C_1-C_6)$alkanoyl, $(C_1-C_6)$alkylsulfonyloxy$(C_1-C_6)$alkanoyl, $(C_1-C_6)$alkoxy$(C_1-C_6)$alkanoyl, $(C_1-C_6)$alkylthio$(C_1-C_6)$alkanoyl, $(C_1-C_6)$alkanoyloxy$(C_1-C_6)$alkanoyl, aryloxy$(C_1-C_6)$alkanoyl, aroyl$(C_1-C_6)$alkanoyl, carboxy$(C_1-C_6)$alkanoyl, $(C_1-C_6)$alkoxycarbonyl$(C_1-C_6)$alkanoyl, carbamoyl$(C_1-C_6)$alkanoyl, $(C_1-C_6)$alkylcarbamoyl$(C_1-C_6)$alkanoyl, ar$(C_1-C_6)$alkanoyl, optionally substituted heterocyclic$(C_1-C_6)$alkanoyl, heterocyclicthio$(C_1-C_6)$alkanoyl, $(C_3-C_6)$alkenoyl, $(C_3-C_6)$alkynoyl, cyclo$(C_3-C_6)$alkylcarbonyl, cycle$(C_3-C_6)$alkenylcarbonyl, carboxy, $(C_1-C_6)$alkoxycarbonyl, aryloxycarbonyl, aroyl, $(C_1-C_6)$alkoxyaroyl, haloaroyl, $(C_1-C_6)$alkoxycarbonylaroyl, ar $(C_3-C_6)$ alkenoyl, $(C_1-C_6)$ alkoxy-ar $(C_3-C_6)$ alkenoyl, $(C_1-C_6)$alkylenedioxy-ar$(C_3-C_6)$alkenoyl, nitro-ar-$(C_3-C_6)$alkenoyl, halo-ar$(C_3-C_6)$alkenoyl, hydroxy-ar$(C_3-C_6)$-alkenoyl, hydroxy$(C_1-C_6)$alkoxy-ar$(C_3-C_6)$alkenoyl, amino$(C_1-C_6)$-alkoxy-ar$(C_3-C_6)$alkenoyl, $(C_1-C_6)$alkylamino$(C_1-C_6)$alkoxy-ar$(C_3-C_6)$alkenoyl, heterocyclic$(C_1-C_6)$alkoxy-ar$(C_3-C_6)$alkenoyl, optionally substituted heterocyclic-ar$(C_3-C_6)$alkenoyl, amino-ar$(C_3-C_6)$alkenoyl, $(C_1-C_6)$alkylamino-ar$(C_3-C_6)$alkenoyl, $(C_1-C_6)$alkanoylamino-ar$(C_3-C_6)$alkenoyl, hydroxy$(C_1-C_6)$alkanoylaminoar$(C_3-C_6)$alkenoyl, $(C_1-C_6)$alkoxy $(C_1-C_6)$alkanoylamino-ar$(C_3-C_6)$alkenoyl, halo$(C_1-C_6)$alkanoylamino-ar$(C_3-C_6)$alkenoyl, amino$(C_1-C_6)$alkanoylamino-ar$(C_3-C_6)$alkenoyl, $(C_1-C_6)$alkylamino$(C_1-C_6)$alkanoylamino-ar$(C_3-C_6)$-alkenoyl, $(C_1-C_6)$alkanoylamino$(C_1-C_6)$alkanoylamino-ar$(C_3-C_6)$-alkenoyl, carboxy$(C_1-C_6)$alkanoylamino-ar$(C_3-C_6)$alkenoyl, $(C_1-C_6)$

alkoxycarbonyl($C_1$-$C_6$)alkanoylamino-ar($C_3$-$C_6$)alkenoyl, ($C_1$-$C_6$)alkoxycarbonyl ($C_3$-$C_6$)alkenoylamino-ar($C_3$-$C_6$)alkenoyl, halo ($C_1$-$C_6$)alkoxycarbonylamino-ar($C_3$-$C_6$)alkenoyl, heterocyclic($C_1$-$C_6$)alkanoylaminoar($C_3$-$C_6$)alkenoyl, aroylamino-ar($C_3$-$C_6$)alkenoyl, heterocyclic-carbonylamino-ar($C_3$-$C_6$)alkenoyl, ($C_1$-$C_6$)alkylsulfonylamino-ar($C_3$-$C_6$)alkenoyl, N-($C_1$-$C_6$ alkanoyl)-N-($C_1$-$C_6$ alkyl)amino-ar($C_3$-$C_6$)alkenoyl, N-[($C_1$-$C_6$)alkoxy($C_1$-$C_6$)alkanoyl]-N-($C_1$-$C_6$)alkyl)amino-ar($C_3$-$C_6$)-alkenoyl, N-($C_1$-$C_6$)alkanoyl)-N-[heterocyclic($C_1$-$C_6$)alkyl]amino-ar($C_3$-$C_6$)alkenoyl, ureido-ar($C_3$-$C_6$)alkenoyl, ($C_1$-$C_6$)alkyl-ureido-ar($C_3$-$C_6$)alkenoyl, heterocyclicureido-ar($C_3$-$C_6$)alkenoyl, ($C_1$-$C_6$)alkanoyl-ar($C_3$-$C_6$)alkenoyl, carboxy-ar($C_3$-$C_6$)alkenoyl, ($C_1$-$C_6$)alkoxycarbonyl-ar($C_3$-$C_6$)alkenoyl, carbamoyl-ar($C_3$-$C_6$)-alkenoyl, ($C_1$-$C_6$)alkylcarbamoyl-ar($C_3$-$C_6$)alkenoyl, hydroxy($C_1$-$C_6$)alkylcarbamoyl-ar($C_3$-$C_6$)alkenoyl, N-[hydroxy($C_1$-$C_6$)alkyl]-N-($C_1$-$C_6$ alkyl)-carbamoyl-ar($C_3$-$C_6$)alkenoyl, ($C_1$-$C_6$)alkoxy($C_1$-$C_6$)alkylcarbamoyl-ar($C_3$-$C_6$)alkenoyl, N-[($C_1$-$C_6$)alkoxy ($C_1$-$C_6$)alkyl]-N-($C_1$-$C_6$ alkyl)carbamoyl-ar($C_3$-$C_6$)-alkenoyl, heterocycliccarbamoyl-ar($C_3$-$C_6$)alkenoyl, heterocycliccarbonyl-ar($C_3$-$C_6$)alkenoyl, ar($C_3$-$C_6$)alkynoyl, heterocyclic($C_3$-$C_6$)alkenoyl, aminoheterocyclic($C_3$-$C_6$)alkenoyl, ($C_1$-$C_6$)alkylamino-heterocyclic($C_3$-$C_6$)alkenoyl, ($C_1$-$C_6$)alkanoyl-amino-heterocyclic($C_3$-$C_6$)alkenoyl, ($C_1$-$C_6$) alkylureido-heterocyclic ($C_3$-$C_6$)alkenoyl, carboxyheterocyclic($C_3$-$C_6$)-alkenoyl, ($C_1$-$C_6$)alkoxycarbonyl-heterocyclic($C_3$-$C_6$)alkenoyl, ($C_1$-$C_6$)alkylcarbamoyl-heterocyclic($C_3$-$C_6$)alkenoyl, ($C_1$-$C_6$)-alkoxy($C_1$-$C_6$)alkylcarbamoyl-heterocyclic($C_3$-$C_6$)alkenoyl, hydroxy($C_1$-$C_6$)alkylcarbamoyl-heterocyclic($C_3$-$C_6$)alkenoyl, heterocycliccarbonyl and heterocycliccarbonyl which is substituted with a substitutent selected from 4-methyl-1-piperazinylcarbonyl, 4-ethyl-1-piperazinylcarbonyl, dimethylaminopiperidinocarbonyl, and 4-methylcarbamoyl-1-piperazinylcarbonyl, aryloxycarbonyl which may be substituted with nitro, ar($C_1$-$C_6$)alkoxycarbonyl which may be substituted with nitro, carbamoyl, thiocarbamoyl, ($C_1$-$C_6$)alkylcarbamoyl, carboxy($C_1$-$C_6$)alkylcarbamoyl, ($C_1$-$C_6$)alkoxycarbonyl ($C_1$-$C_6$)-alkylcarbamoyl, ($C_2$-$C_6$)alkenylcarbamoyl, cyclo($C_3$-$C_6$)alkylcarbamoyl, halo($C_1$-$C_6$)alkanoylcarbamoyl, arylcarbamoyl, arylthiocarbainoyl, ($C_1$-$C_6$)alkoxy-arylcarbamoyl, halo-arylcarbamoyl, halo($C_1$-$C_6$)alkylarylcarbamoyl, nitro-arylcarbamoyl, cyano-arylcarbamoyl, hydroxy($C_1$-$C_6$)alkyl-arylcarbamoyl, amino-arylcarbamoyl, ($C_1$-$C_6$)alkylamino-arylcarbamoyl, ($C_1$-$C_6$)alkanoylamino-arylcarbamoyl, N-($C_1$-$C_6$ alkanoyl)-N-($C_1$-$C_6$ alkyl)amino-arylcarbamoyl, ($C_1$-$C_6$)alkoxy($C_1$-$C_6$)alkanoylamino-arylcarbamoyl, ($C_1$-$C_6$)alkoxycarbonyl($C_1$-$C_6$)alkanoylamino-arylcarbamoyl, carboxyamino-arylcarbamoyl, ($C_1$-$C_6$)alkoxycarbonylamino-arylcarbamoyl, ureido-arylcarbamoyl, ($C_1$-$C_6$)alkylureido-arylcarbamoyl, hydroxyimino($C_1$-$C_6$)alkyl-arylcarbamoyl, ($C_1$-$C_6$)alkoxyimino ($C_1$-$C_6$)alkyl-arylcarbamoyl, ($C_1$-$C_6$)alkylhydrazono($C_1$-$C_6$)alkylarylcarbamoyl, optionally substituted heterocyclic-arylcarbamoyl, carboxy-arylcarbamoyl, ($C_1$-$C_6$)alkoxycarbonyl-arylcarbamoyl, heterocycliccarbonyl-arylcarbamoyl, heterocycliccarbonyl-arylcarbamoyl substituted with ($C_1$-$C_6$)-alkyl, heterocycliccarbonyl-arylcarbamoyl substituted with aryl, heterocycliccarbonyl-arylcarbamoyl substituted with a heterocyclic group, heterocycliccarbonyl-arylcarbamoyl substituted with ($C_1$-$C_6$)alkanoyl, heterocycliccarbonyl-arylcarbamoyl substituted with ($C_1$-$C_6$)alkoxycarbonyl, heterocycliccarbonyl-arylcarbamoyl substituted with ($C_1$-$C_6$)alkylamino, heterocycliccarbonyl-arylcarbamoyl substituted with ($C_1$-$C_6$)alkylcarbamoyl, carbamoyl-arylcarbamoyl, ($C_1$-$C_6$)-alkylcarbamoyl-arylcarbamoyl, hydroxy($C_1$-$C_6$)alkylcarbamoyl-arylcarbamoyl, N-[hydroxy($C_1$-$C_6$)-alkyl]-N-($C_1$-$C_6$ alkyl)carbamoyl-arylcarbamoyl, ($C_1$-$C_6$)alkoxy-($C_1$-$C_6$)alkylcarbamoyl-arylcarbamoyl, N-[($C_1$-$C_6$)alkoxy($C_1$-$C_6$)alkyl]-N-($C_1$-$C_6$ alkyl)carbamoyl-arylcarbamoyl, ($C_1$-$C_6$)alkylamino($C_1$-$C_6$)alkylcarbamoyl-arylcarbamoyl, N-[($C_1$-$C_6$)alkylamino($C_1$-$C_6$)alkyl]-N-($C_1$-$C_6$ alkyl)carbamoyl-arylcarbamoyl, heterocycliccarbamoyl-arylcarbamoyl, N-(heterocyclic)-N-($C_1$-$C_6$ alkyl)carbamoyl-arylcarbamoyl, heterocyclic($C_1$-$C_6$)alkylcarbamoyl-arylcarbamoyl, N-[heterocyclic($C_1$-$C_6$)alkyl]-N-($C_1$-$C_6$ alkyl)carbamoyl-arylcarbamoyl, N-[heterocyclic($C_1$-$C_6$) alkyl]-N-($C_1$-$C_6$)alkoxy($C_1$-$C_6$)alkyl]-carbamoyl-arylcarbamoyl, arylcarbamoyl-arylcarbamoyl, ($C_1$-$C_6$)alkylamino-arylcarbamoyl-arylcarbamoyl, ($C_1$-$C_6$)alkanoyl-arylcarbamoyl, ar($C_1$-$C_6$)alkylcarbamoyl, heterocycliccarbamoyl, heterocyclic($C_1$-$C_6$)alkylcarbamoyl, arylaminocarbamoyl, aroylcarbamoyl, ($C_1$-$C_6$)alkylsulfonyl, arylsulfonyl, ar($C_1$-$C_6$)alkylsulfonyl, ar($C_2$-$C_6$)alkenylsulfonyl, phthaloyl, amino acid redidue, wherein the amino acid is selected from glycine, sarcosine, alanine, β-alanine, valine, norvaline, leucine, isoleucine, norleucine, serine, threonine, cysteine, methionine, phenylalanine, phenylglycine, tryptophan, tyrosine, proline, hydroxyproline, glutamic acid, aspartic acid, glutamine, asparagine, lysine, arginine, histidine and ornithine, which may be substituted with substituent(s) selected from ($C_1$-$C_6$)alkyl, aryl, ar($C_1$-$C_6$)alkyl, a heterocyclic group, heterocyclic($C_1$-$C_6$)alkyl, cycloalkyl selected from cyclopropyl, cyclobutyl, cyclopentyl, cyclohexyl, cycloheptyl, cyclooctyl and adamantyl; amidino, substituted amidino selected from methylamidino and N-ethyl-N'-cyanoamidino; alkanoyl selected from formyl, acetyl, propionyl, butyryl, isobutyryl, valeryl, isovaleryl, pivaloyl, hexanoyl and heptanoyl; halo($C_1$-$C_6$)alkanoyl, hydroxy($C_1$-$C_6$)alkanoyl, ($C_1$-$C_6$)alkylsulfonyloxy-($C_1$-$C_6$)alkanoyl, ($C_1$-$C_6$)alkoxy($C_1$-$C_6$)alkanoyl, aryloxy($C_1$-$C_6$)alkanoyl, ($C_1$-$C_6$)alkylthio($C_1$-$C_6$)alkanoyl, ($C_1$-$C_6$)alkylcarbamoyl-($C_1$-$C_6$)alkanoyl, ($C_1$-$C_6$)alkanoyloxy($C_1$-$C_6$)alkanoyl, carboxy($C_1$-$C_6$)alkanoyl, ($C_1$-$C_6$)alkoxycarbonyl($C_1$-$C_6$)alkanoyl, ar-($C_1$-$C_6$)alkanoyl, optionally substituted heterocyclic($C_1$-$C_6$)alkanoyl, ($C_3$-$C_6$)alkenoyl, ar($C_3$-$C_6$)alkenoyl, ($C_1$-$C_6$)alkoxy-ar($C_3$-$C_6$)alkenoyl, ($C_1$-$C_6$)alkylenedioxy-ar($C_3$-$C_6$)alkenoyl, nitro-ar($C_3$-$C_6$)alkenoyl, halo-ar($C_3$-$C_6$)alkenoyl, hydroxy-ar($C_3$-$C_6$)alkenoyl, hydroxy($C_1$-$C_6$)alkoxy-ar($C_3$-$C_6$)alkenoyl, amino($C_1$-$C_6$)alkoxy-ar($C_3$-$C_6$)alkenoyl, ($C_1$-$C_6$)alkyiamino($C_1$-$C_6$)alkoxy-ar($C_3$-$C_6$)alkenoyl, heterocyclic($C_1$-$C_6$)alkoxy-ar($C_3$-$C_6$)alkenoyl, optionally sub-

stituted heterocyclic-ar($C_3$-$C_6$)alkenoyl, amino-ar($C_3$-$C_6$)alkenoyl, ($C_1$-$C_6$)alkylamino-ar($C_3$-$C_6$)alkenoyl, ($C_1$-$C_6$)-alkanoylamino-ar($C_3$-$C_6$)alkenoyl, hydroxy($C_1$-$C_6$)alkanoylamino-ar($C_3$-$C_6$)alkenoyl, ($C_1$-$C_6$)alkoxy ($C_1$-$C_6$)alkanoylamino-ar($C_3$-$C_6$)-alkenoyl, halo($C_1$-$C_6$)alkanoylamino-ar($C_3$-$C_6$)alkenoyl, amino($C_1$-$C_6$)alkanoylamino-ar($C_3$-$C_6$)alkenoyl, ($C_1$-$C_6$)alkylamino($C_1$-$C_6$)-alkanoylamino-ar($C_3$-$C_6$)alkenoyl, ($C_1$-$C_6$)alkanoylamino($C_1$-$C_6$)alkanoylamino-ar($C_3$-$C_6$)alkenoyl, carboxy($C_1$-$C_6$)alkanoylamino-ar($C_3$-$C_6$)alkenoyl, ($C_1$-$C_6$)alkoxycarbonyl($C_1$-$C_6$)alkanoylamino-ar($C_3$-$C_6$)alkenoyl, ($C_1$-$C_6$)alkoxycarbonyl ($C_3$-$C_6$)alkenoylamino-ar($C_3$-$C_6$)alkenoyl, halo($C_1$-$C_6$)alkoxycarbonylamino-ar($C_3$-$C_6$)alkenoyl, heterocyclic($C_1$-$C_6$)-alkanoylamino-ar($C_3$-$C_6$)alkenoyl, aroylamino-ar($C_3$-$C_6$)alkenoyl, heterocyclic-carbonylamino-ar($C_3$-$C_6$)alkenoyl, ($C_1$-$C_6$)alkylsulfonylamino-ar($C_3$-$C_6$)alkenoyl, N-($C_1$-$C_6$ alkanoyl)-N-($C_1$-$C_6$ alkyl)amino-ar($C_3$-$C_6$)alkenoyl, N-[($C_1$-$C_6$)alkoxy($C_1$-$C_6$)alkanoyl]-N-($C_1$-$C_6$)alkyl)amino-ar($C_3$-$C_6$)alkenoyl, N-($C_1$-$C_6$ alkanoyl)-N-[heterocyclic($C_1$-$C_6$)alkyl]amino-ar($C_3$-$C_6$)alkenoyl, ureido-ar($C_3$-$C_6$)alke-noyl, ($C_1$-$C_6$)alkyl-ureido-ar($C_3$-$C_6$)alkenoyl, heterocyclicureido-ar($C_3$-$C_6$)alkenoyl, ($C_1$-$C_6$)-alkanoyl-ar($C_3$-$C_6$)alkenoyl, carboxy-ar($C_3$-$C_6$)alkenoyl, ($C_1$-$C_6$)alkoxycarbonyl-ar($C_3$-$C_6$)-alkenoyl, carbamoyl-ar($C_3$-$C_6$)alkenoyl, ($C_1$-$C_6$)alkylcarbamoyl-ar($C_3$-$C_6$)-alkenoyl, hydroxy($C_1$-$C_6$)alkylcarbamoyl-ar($C_3$-$C_6$)-alkenoyl, N-[hydroxy($C_1$-$C_6$)alkyl]-N-($C_1$-$C_6$ alkyl)carbamoyl-ar($C_3$-$C_6$)alke-noyl, ($C_1$-$C_6$)alkoxy($C_1$-$C_6$)alkylcarbamoyl-ar($C_3$-$C_6$)alkenoyl, N-[($C_1$-$C_6$)alkoxy($C_1$-$C_6$)alkyl]-N-($C_1$-$C_6$ alkyl)carbamoyl-ar($C_3$-$C_6$)alkenoyl, heterocycliccarbamoyl-ar($C_3$-$C_6$)alkenoyl, heterocycliccarbonyl-ar($C_3$-$C_6$)alkenoyl, ar($C_3$-$C_6$)alkynoyl, heterocyclic($C_3$-$C_6$)alkenoyl, amino-heterocyclic($C_3$-$C_6$)alkenoyl, ($C_1$-$C_6$)alkylamino-heterocyclic($C_3$-$C_6$)alkenoyl, ($C_1$-$C_6$)alkanoylamino-heterocyclic($C_3$-$C_6$)alkenoyl, ($C_1$-$C_6$)alkylureido-heterocyclic($C_3$-$C_6$)alkenoyl, carboxy-heterocyclic($C_3$-$C_6$)alkenoyl, ($C_1$-$C_6$)alkoxycarbonylheterocyclic($C_3$-$C_6$)alkenoyl, ($C_1$-$C_6$)alkylcarbamoyl-heterocyclic($C_3$-$C_6$)alkenoyl, ($C_1$-$C_6$)alkoxy($C_1$-$C_6$)alkyl-carbamoyl-heterocyclic ($C_3$-$C_6$) alkenoyl, hydroxy($C_1$-$C_6$)alkyl-carbamoyl-heterocyclic($C_3$-$C_6$)alkenoyl, heterocyclicthio-($C_1$-$C_6$)alkanoyl, optionally substituted heterocycliccarbonyl, cyclo($C_3$-$C_6$)alkylcarbonyl, ($C_1$-$C_6$)alkoxycarbonyl, aryloxycarbonyl, aroyl($C_1$-$C_6$)alkanoyl, aroyl, nitro-aryloxycarbonyl, carbamoyl, ($C_1$-$C_6$)alkylcarbamoyl, ($C_1$-$C_6$)alkoxycarbonyl ($C_1$-$C_6$)alkylcarbarnoyl, ($C_2$-$C_6$)alkenylcarbamoyl, cyclo($C_3$-$C_6$)alkyl-carbamoyl, arylcarbamoyl, ($C_1$-$C_6$)alkoxy-arylcarbamoyl, halo($C_1$-$C_6$)alkyl-arylcarbamoyl, halo-arylcarbamoyl, hydroxy($C_1$-$C_6$)alkyl-arylcarbamoyl, nitro-arylcarbamoyl, cyano-arylcarbamoyl, amino-arylcarbamoyl, ($C_1$-$C_6$)alkylamino-arylcarbamoyl, ($C_1$-$C_6$)alkanoylamino-arylcarbamoyl, N-($C_1$-$C_6$ alkanoyl)-N-($C_1$-$C_6$ alkyl)amino-arylcarbamoyl, ($C_1$-$C_6$)alkoxy($C_1$-$C_6$)alkanoylamino-arylcarbamoyl, ($C_1$-$C_6$)alkoxycarbonyl($C_1$-$C_6$)alkanoylamino-aryl-carbamoyl, carboxyamino-arylcarbamoyl, ($C_1$-$C_6$)alkoxycarbonylamino-arylcarbamoyl, ureido-arylcarbamoyl, ($C_1$-$C_6$)alkylureido-arylcarbamoyl, hydroxyimino-($C_1$-$C_6$)alkyl-arylcarbamoyl, ($C_1$-$C_6$)alkoxyimino ($C_1$-$C_6$)alkylarylcarbamoyl, ($C_1$-$C_6$)alkylhydrazono($C_1$-$C_6$)alkyl-arylcarbamoyl, optionally substituted heterocyclic-arylcarbamoyl, ($C_1$-$C_6$)alkanoyl-arylcarbamoyl, heterocycliccarbonyl-arylcarbamoyl, carboxy-arylcarbamoyl, ($C_1$-$C_6$)alkoxycarbonyl-arylcarbamoyl, ($C_1$-$C_6$)alkylcarbamoyl-arylcarbamoyl, heterocycliccarbonyl-arylcarbamoyl having ($C_1$-$C_6$)alkyl, heterocycliccarbonyl-arylcarbamoyl having aryl, heterocycliccarbonyl-arylcarbamoyl having a heterocyclic group, heterocycliccarbonyl-arylcarbamoyl having ($C_1$-$C_6$)alkanoyl, heterocycliccarbonyl-arylcarbamoyl having ($C_1$-$C_6$)alkoxy-carbonyl, heterocyclic-carbonyl-arylcarbamoyl having ($C_1$-$C_6$)alkylamino, heterocyclic-carbonyl-arylcarbamoyl having ($C_1$-$C_6$)alkylcarbamoyl, hydroxy($C_1$-$C_6$)alkylcarbamoyl-arylcarbamoyl, N-[hydroxy($C_1$-$C_6$)alkyl]-N-($C_1$-$C_6$ alkyl)carbamoyl-arylcarbamoyl, ($C_1$-$C_6$)alkoxy($C_1$-$C_6$) alkylcarbamoyl-arylcarbamoyl, N-[($C_1$-$C_6$)alkoxy($C_1$-$C_6$)alkyl]-N-($C_1$-$C_6$ alkyl)carbamoyl-arylcarbamoyl, ($C_1$-$C_6$)alkylamino ($C_1$-$C_6$)alkylcarbamoyl-arylcarbamoyl, N-[($C_1$-$C_6$)alkylamino($C_1$-$C_6$)alkyl]-N-($C_1$-$C_6$ alkyl)carbamoyl-arylcarbamoyl, heterocyclic-carbamoyl-arylcarbamoyl, N-(heterocyclic)-N-($C_1$-$C_6$ alkyl)carbamoylarylcarbamoyl, heterocyclic($C_1$-$C_6$)alkylcarbamoyl-arylcarbamoyl, N-[heterocyclic($C_1$-$C_6$)alkyl]-N-($C_1$-$C_6$ alkyl)carbamoyl-arylcarbamoyl, N-[heterocyclic($C_1$-$C_6$)alkyl]-N-[($C_1$-$C_6$)alkoxy($C_1$-$C_6$)alkyl]-carbamoyl-arylcarbamoyl, arylcarbamoyl-arylcarbamoyl, ($C_1$-$C_6$) alkylaminoarylcarbamoyl-arylcarbamoyl, arylthiocarbamoyl, ar($C_1$-$C_6$)alkylcarbamoyl, aroylcarbamoyl, heterocycliccarbamoyl, heterocyclic($C_1$-$C_6$)alkylcarbamoyl, aryl-amino-carbamoyl, ar($C_2$-$C_6$)alkenylsulfonyl, ($C_1$-$C_6$)alkylsulfonyl, phthaloyl, unsubstituted amino acid residue selected from glycine, sarcosine, alanine, β-alanine, valine, norvaline, leucine, isoleucine, norleucine, serine, threonine, cysteine, methionine, phenylalanine, phenylglycine, tryptophan, tyrosine, proline, hydroxyproline, glutamic acid, aspartic acid, glutamine, asparagine, lysine, arginine, histidine or ornithine, amino acid residue substituted with ($C_1$-$C_6$)alkyl, wherein the amino acid is as defined for the unsubstituted amino acid residue defined before, amino acid residue substituted with a heterocyclic group, wherein the amino acid is as defined for the unsubstituted amino acid residue defined before, amino acid residue substituted with heterocyclic($C_1$-$C_6$)alkyl, wherein the amino acid is as defined for the unsubstituted amino acid residue defined before, amino acid residue substituted with cycloalkyl selected from cyclopropyl, cyclobutyl, cyclopentyl, cyclohexyl, cycloheptyl, cyclooctyl and adamantyl, wherein the amino acid is as defined for the unsubstituted amino acid residue defined before, amino acid residue substituted with aryl, wherein the amino acid is as defined for the unsubstituted amino acid residue defined before, amino acid residue substituted with ($C_1$-$C_6$)alkanoyl, wherein the amino acid is as defined for the unsubstituted amino acid residue defined before, amino acid residue substituted with ($C_1$-$C_6$)alkoxycarbonyl, wherein the amino acid is as defined for the unsubstituted amino

acid residue defined before, amino acid residue substituted with phthaloyl, wherein the amino acid is as defined for the unsubstituted amino acid residue defined before, and amino acid residue substituted with ar($C_1$-$C_6$)alkyl, wherein the amino acid is as defined for the unsubstituted amino acid residue defined before, wherein in the above and below definitions "ar" and "aryl" is selected from phenyl, naphthyl, phenyl which is substituted with ($C_1$-$C_6$)alkyl, the heterocyclic group or moiety is selected from:

- unsaturated 3 to 8-membered heteromonocyclic group, containing 1 to 4 nitrogen atom(s),
- saturated 3 to 8-membered heteromonocyclic group containing 1 to 4 nitrogen atom(s),

  - unsaturated condensed 7 to 12-membered heterocyclic group containing 1 to 5 nitrogen atom(s),
  - unsaturated 3 to 8-membered, heteromonocyclic group containing an oxygen atom,
  - unsaturated condensed 7 to 12-membered heterocyclic group containing 1 to 2 oxygen atom(s),
  - unsaturated 3 to 8-membered, heteromonocyclic group containing a sulfur atom,
  - unsaturated condensed 7 to 12-membered heterocyclic group containing 1 to 2 sulfur atom(s),
  - unsaturated 3 to 8-membered, heteromonocyclic group containing 1 to 2 oxygen atom(s) and 1 to 3 nitrogen atom(s),
  - saturated 3 to 8-membered, heteromonocyclic group containing 1 to 2 oxygen atom(s) and 1 to 3 nitrogen atom(s),
  - unsaturated condensed 7 to 12-membered heterocyclic group containing 1 to 2 oxygen atom(s) and 1 to 3 nitrogen atom(s),
  - unsaturated 3 to 8-membered, heteromonocyclic group containing 1 to 2 sulfur atom(s) and 1 to 3 nitrogen atom(s),
  - saturated 3 to 8-membered, heteromonocyclic group containing 1 to 2 sulfur atom(s) and 1 to 3 nitrogen atom(s),
  - unsaturated condensed 7 to 12-membered heterocyclic group containing 1 to 2 sulfur atom(s) and 1 to 3 nitrogen atom(s),

and substituents of aryl as defined above and the heterocyclic group as defined above are selected from halogen; halo($C_1$-$C_6$)alkyl; ($C_1$-$C_6$)alkyl; the above-mentioned aryl, optionally substituted with substituent(s) selected from halogen and cyano, ar($C_1$-$C_6$)alkyl substituted with hydroxy wherein the aryl moiety is as defined above, ($C_1$-$C_6$)alkoxy; oxo; nitro; amino, optionally substituted with ($C_1$-$C_6$)alkyl, the above-mentioned acyl, ar($C_1$-$C_6$)alkyl wherein the aryl moiety is as defined above, heterocyclic($C_1$-$C_6$)alkyl wherein the heterocyclic moiety is as defined above, carboxy($C_1$-$C_6$)alkyl, ($C_1$-$C_6$)alkylaminomethylene, or N-methylpyrrolidinylidene; the above-mentioned heterocyclic group optionally substituted with oxo,

phenyl ($C_1$-$C_6$)alkyl, pyridyl($C_1$-$C_6$)alkyl, carboxy($C_1$-$C_6$)alkyl, ($C_1$-$C_6$) alkylaminomethylene and N-methylpyrrolidinylidene; pyrrolidinyl and pyrrolidinyl substituted with oxo; or unsaturated condensed 7 to 12-membered heterocyclic group containing 1 to 2 oxygen atom(s) or unsaturated condensed 7 to 12-membered heterocyclic group containing 1 to 2 sulfur atom(s) and 1 to 3 nitrogen atoms(s), each of which is substituted with substituent(s) selected from the group consisting of halogen, ($C_1$-$C_6$)alkyl and oxo,

Q is O or NH, and
A is ($C_1$-$C_6$) alkylene,

and pharmaceutically acceptable salts thereof.

2. A compound of claim 1, wherein

$R^4$ is phenyl substituted with substituents selected from the group consisting of halogen, nitro, amino and a group of the formula:

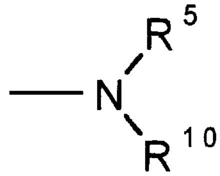

in which $R^5$ is hydrogen or $(C_1-C_6)$alkyl, and $R^{10}$ is acyl as defined in claim 1, and A is methylene.

**3.** A compound of claim 2, wherein

$R^4$ is phenyl substituted with one or two halogen(s) and a group of the formula:

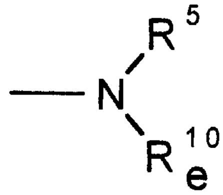

in which
$R^5$ is hydrogen or $(C_1-C_6)$alkyl, and
$R_e^{10}$ is an amino acid residue as defined in claim 1 or an amino acid residue as defined in claim 1 substituted with a substituent selected from the group consisting of $(C_1-C_6)$alkyl, alkanoyl, selected from formyl, acetyl, propionyl, butyryl, isobutyryl, valeryl, isovaleryl, pivaloyl, hexanoyl and heptanoyl, halo$(C_1-C_6)$alkanoyl, ar$(C_1-C_6)$alkanoyl, heterocyctic$(C_1-C_6)$alkanoyl, $(C_3-C_6)$alkenoyl, ar$(C_3-C_6)$alkenoyl, $(C_1-C_6)$alkoxy-ar$(C_3-C_6)$alkenoyl, $(C_1-C_6)$alkylendioxy-ar$(C_3-C_6)$alkenoyl, nitro-ar$(C_3-C_6)$alkenoyl, halo-ar$(C_3-C_6)$alkenoyl, hydroxy-ar$(C_3-C_6)$alkenoyl, hydroxy$(C_1-C_6)$alkoxy-ar$(C_3-C_6)$alkenoyl, amino$(C_1-C_6)$alkoxy-ar$(C_3-C_6)$alkenoyl, $(C_1-C_6)$alkylamino$(C_1-C_6)$alkoxy-ar$(C_3-C_6)$alkenoyl, heterocyclic$(C_1-C_6)$alkoxy-ar$(C_3-C_6)$-alkenoyl, heterocyclic-ar$(C_3-C_6)$alkenoyl optionally having oxo, amino-ar$(C_3-C_6)$alkenoyl, $(C_1-C_6)$alkylamino-ar$(C_3-C_6)$alkenoyl, $(C_1-C_6)$alkanoylamino-ar$(C_3-C_6)$alkenoyl, N-$(C_1-C_6$ alkanoyl)-N-$(C_1-C_6$ alkyl)amino-ar$(C_3-C_6)$alkenoyl, hydroxy$(C_1-C_6)$alkanoylamino-ar$(C_3-C_6)$alkenoyl,$(C_1-C_6)$alkoxy-$(C_1-C_6)$alkanoylamino-ar$(C_3-C_6)$alkenoyl, halogen$(C_1-C_6)$alkanoylamino-ar$(C_3-C_6)$alkenoyl, amino$(C_1-C_6)$alkanoylamino-ar$(C_3-C_6)$alkenoyl, $(C_1-C_6)$alkylamino$(C_1-C_6)$alkanoylamino-ar$(C_3-C_6)$alkenoyl, $(C_1-C_6)$alkanoylamino$(C_1-C_6)$alkanoylamino-ar$(C_3-C_6)$alkenoyl, carboxy$(C_1-C_6)$alkanoylamino-ar$(C_3-C_6)$alkenoyl, $(C_1-C_6)$alkoxycarbonyl$(C_1-C_6)$alkanoylamino-ar$(C_3-C_6)$alkenoyl, $(C_1-C_6)$alkoxycarbonyl$(C_3-C_6)$alkenoylamino-ar$(C_3-C_6)$alkenoyl, halo$(C_1-C_6)$alkoxycarbonylamino-ar$(C_3-C_6)$alkenoyl, heterocyclic$(C_1-C_6)$alkanoylamino-ar$(C_3-C_6)$alkenoyl, aroyl-amino-ar$(C_3-C_6)$alkenoyl, heterocycliccarbonylamino-ar$(C_3-C_6)$alkenoyl, $(C_1-C_6)$alkylsulfonylamino-ar$(C_3-C_6)$alkenoyl, N-[$(C_1-C_6)$ alkoxy$(C_1-C_6)$alkanoyl]-N-$(C_1-C_6$ alkyl)amino-ar$(C_3-C_6)$alkenoyl, N-$(C_1-C_6$ alkanoyl)-N-[heterocyclic$(C_1-C_6)$alkyl]amino-ar$(C_3-C_6)$-alkenoyl, ureido-ar$(C_3-C_6)$alkenoyl, $(C_1-C_6)$alkylureido-ar$(C_3-C_6)$alkenoyl, heterocyclicureido-ar$(C_3-C_6)$alkenoyl, $(C_1-C_6)$alkanoyl-ar$(C_3-C_6)$alkenoyl, carboxy-ar$(C_3-C_6)$alkenoyl, $(C_1-C_6)$alkoxycarbonyl-ar$(C_3-C_6)$alkenoyl, carbamoyl-ar$(C_3-C_6)$alkenoyl, $(C_1-C_6)$alkylcarbamoyl-ar$(C_3-C_6)$alkenoyl, hydroxy$(C_1-C_6)$alkylcarbamoyl-ar$(C_3-C_6)$alkenoyl, N-[hydroxy$(C_1-C_6)$ alkyl]-N-$(C_1-C_6)$alkyl)carbamoyl-ar$(C_3-C_6)$alkenoyl, $(C_1-C_6)$alkoxy$(C_1-C_6)$alkylcarbamoyl-ar$(C_3-C_6)$alkenoyl, N-[$(C_1-C_6)$alkoxy$(C_1-C_6)$alkyl]-N-$(C_1-C_6$ alkyl)carbamoyl-ar$(C_3-C_6)$alkenoyl, heterocycliccarbamoyl-ar$(C_3-C_6)$alkenoyl, heterocycliccarbonyl-ar$(C_3-C_6)$-alkenoyl, ar$(C_3-C_6)$alkynoyl, heterocyclic$(C_3-C_6)$alkenoyl, heterocyclicthio$(C_1-C_6)$alkanoyl, aminoheterocyclic$(C_3-C_6)$alkenoyl, $(C_1-C_6)$alkylamino-heterocyclic$(C_3-C_6)$alkenoyl, $(C_1-C_6)$alkanoylamino-heterocyclic$(C_3-C_6)$alkenoyl, $(C_1-C_6)$alkyl-ureido-heterocyclic$(C_3-C_6)$alkenoyl, carboxy-heterocyclic$(C_3-C_6)$alkenoyl, $(C_1-C_6)$alkoxycarbonyl-heterocyclic$(C_3-C_6)$alkenoyl, $(C_1-C_6)$alkylcarbamoyl-heterocyclic$(C_3-C_6)$alkenoyl, $(C_1-C_6)$alkoxy$(C_1-C_6)$-alkylcarbamoyl-heterocyclic $(C_3-C_6)$alkenoyl, hydroxy$(C_1-C_6)$alkylcarbamoyl-heterocyclic $(C_3-C_6)$alkenoyl, heterocycliccarbonyl, cyclo$(C_3-C_6)$alkylcarbonyl, $(C_1-C_6)$alkoxycarbonyl, aryloxycarbonyl, aroyl$(C_1-C_6)$alkanoyl, aroyl, nitro-aryloxycarbonyl, carbamoyl, $(C_1-C_6)$alkylcarbamoyl, $(C_1-C_6)$alkoxycarbonyl$(C_1-C_6)$alkylcarbamoyl, $(C_2-C_6)$alkenylcarbamoyl, cyclo$(C_3-C_6)$alkylcarbamoyl, arylcarbamoyl, $(C_1-C_6)$alkoxyarylcarbamoyl, halo$(C_1-C_6)$alkylarylcarbamoyl, halo-arylcarbamoyl, $(C_1-C_6)$alkanoyl-arylcarbamoyl, hydroxy$(C_1-C_6)$alkylarylcarbamoyl, heterocycliccarbonyl-arylcarbamoyl, carboxy-arylcarbamoyl, $(C_1-C_6)$alkoxycarbonyl-arylcarbamoyl, $(C_1-C_6)$alkylcarbamoyl-arylcarbamoyl, nitro-arylcarbamoyl, cyano-arylcarbamoyl, amino-arylcarbamoyl, $(C_1-C_6)$alkylamino-arylcarbamoyl, $(C_1-C_6)$alkanoylamino-arylcarbamoyl, N-$(C_1-C_6$alkanoyl)-N-$(C_1-C_6$ alkyl)amino-arylcarbamoyl, $(C_1-C_6)$alkoxy$(C_1-C_6)$alkanoylamino-arylcarbamoyl, $(C_1-C_6)$alkoxycarbonyl $(C_1-C_6)$alkanoylamino-arylcarbamoyl, carboxyamino-arylcarbamoyl, $(C_1-C_6)$alkoxycarbonylami-

no-arylcarbamoyl, ureido-arylcarbamoyl, ($C_1$-$C_6$)alkylureido-arylcarbamoyl, hydroxyimino($C_1$-$C_6$)alkyl-arylcarbamoyl, ($C_1$-$C_6$)alkoxyimino($C_1$-$C_6$)alkyl-arylcarbamoyl, ($C_1$-$C_6$)alkylhydrazono($C_1$-$C_6$)alkyl-arylcarbamoyl, heterocyclic-arylcarbamoyl optionally having oxo, heterocycliccarbonyl-arylcarbamoyl having ($C_1$-$C_6$)alkyl, heterocycliccarbonyl-arylcarbamoyl having aryl, heterocycliccarbonyl-arylcarbamoyl having a heterocyclic group, heterocycliccarbonyl-arylcarbamoyl having ($C_1$-$C_6$)alkanoyl, heterocycliccarbonyl-arylcarbamoyl having ($C_1$-$C_6$)alkoxycarbonyl, heterocycliccarbonyl-arylcarbamoyl having ($C_1$-$C_6$)alkylamino, heterocycliccarbonyl-arylcarbamoyl having ($C_1$-$C_6$)alkylcarbamoyl, hydroxy($C_1$-$C_6$)alkylcarbamoyl-arylcarbamoyl, N-[hydroxy($C_1$-$C_6$)alkyl]-N-($C_1$-$C_6$ alkyl)carbamoyl-arylcarbamoyl, ($C_1$-$C_6$)alkoxy($C_1$-$C_6$)alkylcarbamoyl-arylcarbamoyl, N-[($C_1$-$C_6$)alkoxy($C_1$-$C_6$)alkyl]-N-($C_1$-$C_6$ alkyl)carbamoyl-arylcarbamoyl, ($C_1$-$C_6$)alkylamino ($C_1$-$C_6$)alkylcarbamoyl-arylcarbamoyl, N-[($C_1$-$C_6$)alkylamino($C_1$-$C_6$)alkyl]-N-($C_1$-$C_6$ alkyl)carbamoyl-arylcarbamoyl, heterocycliccarbamoyl-arylcarbamoyl, N-(heterocyclic)-N-($C_1$-$C_6$alkyl)carbamoyl-arylcarbamoyl, heterocyclic-($C_1$-$C_6$)alkylcarbamoyl-arylcarbamoyl, N-[heterocyclic($C_1$-$C_6$)alkyl]-N-($C_1$-$C_6$ alkyl)carbamoyl-arylcarbamoyl, N-[heterocyclic($C_1$-$C_6$)alkyl]-N-[($C_1$-$C_6$)alkoxy($C_1$-$C_6$)alkyl]carbamoyl-arylcarbamoyl, arylcarbamoyl-arylcarbamoyl, ($C_1$-$C_6$)alkylaminoarylcarbamoylarylcarbamoyl, arylthiocarbamoyl, ar($C_1$-$C_6$)alkylcarbamoyl, aroylcarbamoyl, heterocycliccarbamoyl, heterocyclic($C_1$-$C_6$)alkylcarbamoyl, arylamino-carbamoyl, ar($C_2$-$C_6$)alkenylsulfonyl, ($C_1$-$C_6$)alkylsulfonyl, phthaloyl, amino acid residue as defined in claim 1, amino acid residue as defined in claim 1 substituted with ($C_1$-$C_6$)alkyl, amino acid residue as defined in claim 1 substituted with a heterocyclic group, amino acid residue as defined in claim 1 substituted with heterocyclic($C_1$-$C_6$)alkyl, amino acid residue as defined in claim 1 substituted with cycloalkyl selected from cyclopropyl, cyclobutyl, cyclopentyl, cyclohexyl, cycloheptyl, cyclooctyl and adamantyl, amino acid residue as defined in claim 1 substituted with aryl, amino acid residue as defined in claim 1 substituted with alkanoyl selected from formyl, acetyl, propionyl, butyryl, isobutyryl, valeryl, isovaleryl, pivaloyl, hexanoyl, heptanoyl, amino acid residue as defined in claim 1 substituted with ($C_1$-$C_6$)alkoxycarbonyl, amino acid residue as defined in claim 1 substituted with ar($C_1$-$C_6$)alkyl and amino acid residue as defined in claim 1 substituted with phthaloyl; in the above definitions aryl, heterocyclic group or moiety are each as defined in claim 1.

**4.** A compound according to claim 1, wherein

$R^4$ is unsaturated condensed 7- to 12-membered heterocyclic group containing 1 to 2 oxygen atom(s) or unsaturated condensed 7- to 12-membered heterocyclic group containing 1 to 2 sulfur atom(s) and 1 bis 3 nitrogen atom(s), each of which is substituted with substituent(s) selected from the group consisting of halogen, ($C_1$-$C_6$)alkyl and oxo.

**5.** Halogenating a compound of the formula:

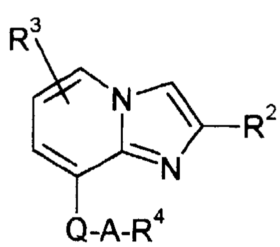

wherein $R^2$, $R^3$, $R^4$, Q and A are each as defined in claim 1, or its salt to give a compound of the formula:

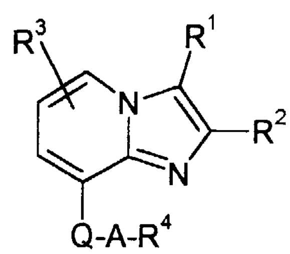

wherein $R^1$, $R^2$, $R^3$, $R^4$, Q and A are each as defined in claim 1, or its salt.

**6.** Reacting a compound of the formula:

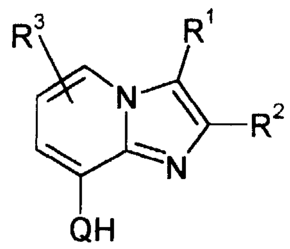

wherein $R^1$, $R^2$, $R^3$ and Q are each as defined in claim 1, or its salt with a compound of the formula:

$$X\text{-}A\text{-}R^4$$

wherein X is a leaving group,
and $R^4$ and A are each as defined in claim 1, or its salt to give a compound of formula:

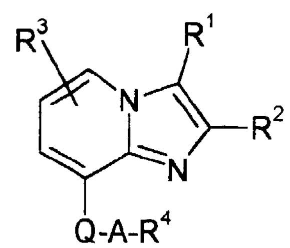

wherein $R^1$, $R^2$, $R^3$, $R^4$, Q und A are each as defined above, or its salt.

**7.** Acylating a compound of formula:

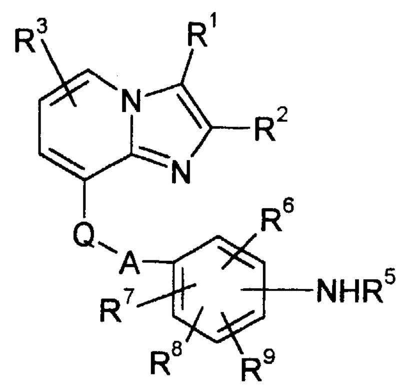

wherein

$R^5$ is hydrogen or $(C_1\text{-}C_6)$alkyl,
$R^6$, $R^7$, $R^8$ and $R^9$ are each hydrogen or halogen, and
$R^1$, $R^2$, $R^3$, Q and A are each as defined in claim 1,

or its salt to give a compound of the formula:

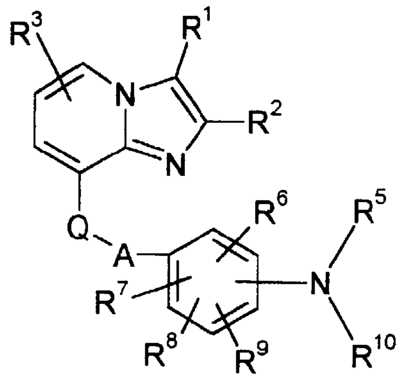

wherein $R^{10}$ is acyl as defined in claim 1, and

$R^1$, $R^2$, $R^3$, $R^5$, $R^6$, $R^7$, $R^8$, $R^9$, Q and A are each as defined above,

or its salt.

**8.** Acylating a compound of the formula:

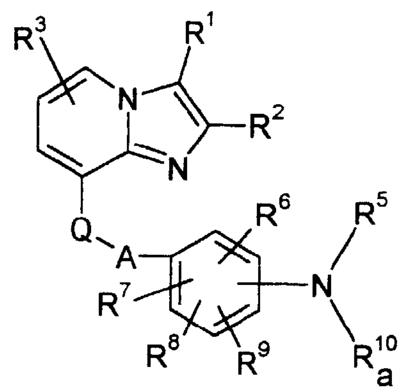

wherein $R_a^{10}$ is acyl as defined in claim 1 having amino, $R^1$, $R^2$, $R^3$, Q and A are each as defined in claim 1, and $R^5$, $R^6$, $R^7$, $R^8$ and $R^9$ are each as defined in claim 7,

or its salt to give a compound of the formula:

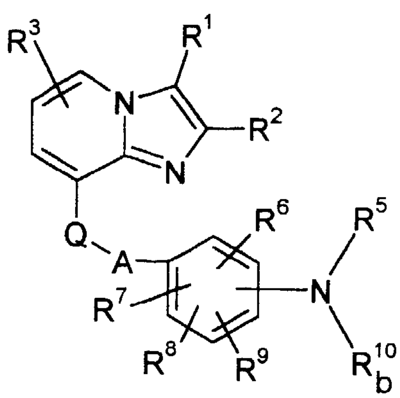

wherein $R_b^{10}$ is acyl having acylamino wherein acyl is each as defined in claim 1, and $R^1$, $R^2$, $R^3$, $R^5$, $R^6$, $R^7$, $R^8$, $R^9$, Q and A are each as defined above,

or its salt.

**9.** Alkylating a compound of the formula:

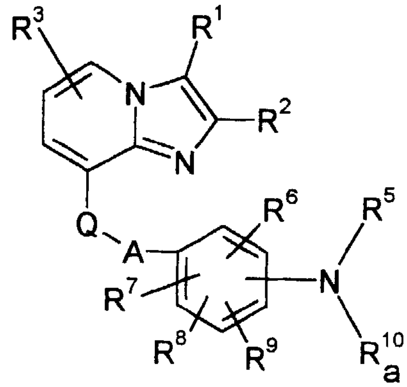

wherein $R^1$, $R^2$, $R^3$, Q and A are each as defined in claim 1, $R^5$, $R^6$, $R^7$, $R^8$ and $R^9$ are each as defined in claim 7, and $R_a^{10}$ is as defined in claim 8,

or its salt to give a compound of the formula:

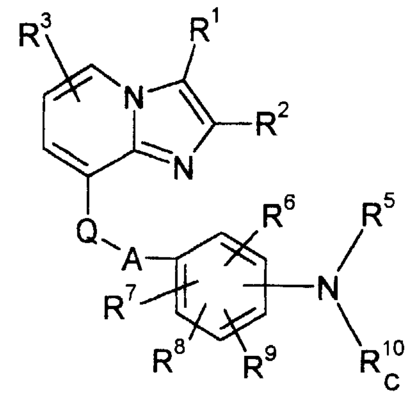

wherein $R_c^{10}$ is acyl having $(C_1\text{-}C_6)$alkylamino or acyl having ar$(C_1\text{-}C_6)$alkylamino wherein acyl is each as defined in claim 1, and $R^1$, $R^2$, $R^3$, $R^5$, $R^6$, $R^7$, $R^8$, $R^9$, Q and A are each as defined above, or its salt.

**10.** Reacting a compound of the formula:

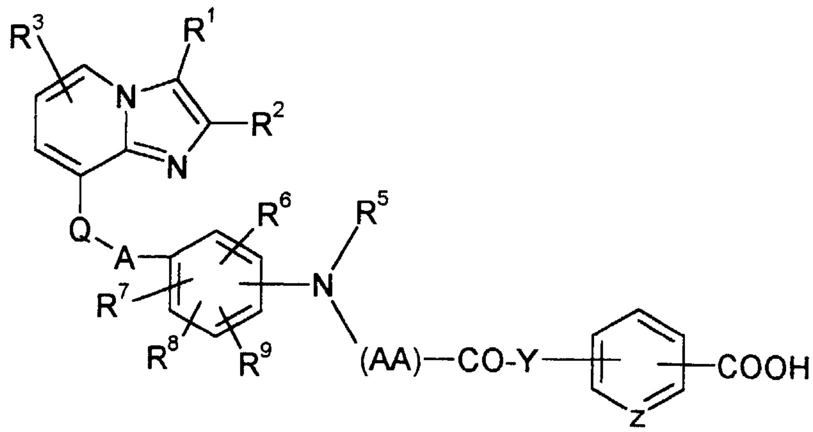

wherein (AA) is amino acid residue as defined in claim 1,

Y is NH or $(C_2\text{-}C_6)$alkenylene,
Z is CH or N,
$R^1$, $R^2$, $R^3$, Q and A are each as defined in claim 1 and $R^5$,
$R^6$, $R^7$, $R^8$ and $R^9$ are each as defined in claim 7,

or its reactive derivative at the carboxy group or a salt thereof with a compound of the formula:

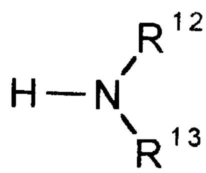

wherein

$R^{12}$ is hydrogen, $(C_1\text{-}C_6)$alkyl, $(C_1\text{-}C_6)$alkoxy$(C_1\text{-}C_6)$-alkyl, $(C_1\text{-}C_6)$alkylamino$(C_1\text{-}C_6)$alkyl, heterocyclic$(C_1\text{-}C_6)$alkyl, a heterocyclic group, protected or unprotected hydroxy$(C_1\text{-}C_6)$alkyl or aryl optionally substituted with $(C_1\text{-}C_6)$alkylamino, and
$R^{13}$ is hydrogen, $(C_1\text{-}C_6)$alkyl, $(C_1\text{-}C_6)$alkoxy$(C_1\text{-}C_6)$alkyl or protected or unprotected hydroxy$(C_1\text{-}C_6)$alkyl, or $R^{12}$ and $R^{13}$ are taken together with the attached nitrogen atom to form a heterocyclic group optionally having suitable substituent(s), wherein aryl, the heterocyclic group or moiety are each as defined in claim 1,

or its reactive derivative at the amino group or a salt thereof to give a compound of the formula:

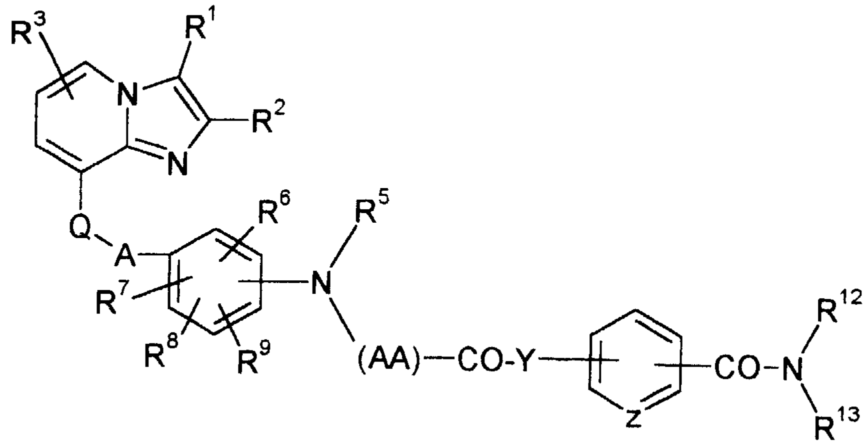

wherein $R^1$, $R^2$, $R^3$, $R^4$, $R^5$, $R^6$, $R^7$, $R^8$, $R^9$, $R^{12}$, $R^{13}$, A, (AA), Q, Y, and Z are each as defined above, or its salt.

**11.** A pharmaceutical composition comprising a compound of claim 1, as an active ingredient, in association with a pharmaceutically acceptable, substantially nontoxic carrier or excipient.

**12.** A compound of claim 1 for use as a medicament.

**13.** A compound of claim 1 for use in the prevention and/or treatment of bradykinin or its analogous mediated diseases.

**14.** Use of compound of claim 1 for the manufacture of a medicament for the prevention and/or treatment of bradykinin or its analogous mediated diseases.

**15.** A compound of claim 3, wherein

$R^4$ is phenyl, substituted with two halogens and a group of the formula:

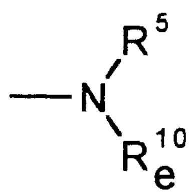

in which
$R^5$ is $(C_1\text{-}C_6)$alkyl, and
$R^{10}_e$ is glycyl, substituted with $(C_1\text{-}C_6)$alkylcarbamoyl-ar $(C_3\text{-}C_6)$alkenoyl.

**16.** A compound of claim 15, which is 3-bromo-8-[2,6-dichloro-3-[N-[4-(dimethylcarbamoyl)cinnamoylglycylj-N-methylamino]benzyloxy]-2-methylimidazo[1,2-a]pyridin and its acid addition salt.

**17.** A compound of claim 15, which is 3-bromo-8-[2,6-dichloro-3-[N-methyl-N-[4-(methylcarbamoyl)cinnamoylglycyl]amino]benzyloxy]-2-methylimidazo[1,2-a]pyridin and its acid addition salt.

## Patentansprüche

**1.** Verbindung der Formel:

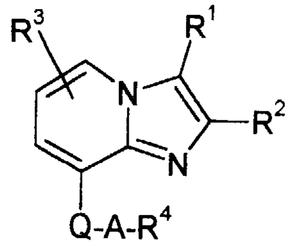

worin

$R^1$ Halogen ist,
$R^2$ ($C_1$-$C_6$)Alkyl ist,
$R^3$ Wasserstoff ist,
$R^4$ Phenyl, substituiert mit Substituent(en) ausgewählt aus der Gruppe, die besteht aus Halogen; ($C_1$-$C_6$)Alkyl; Halogen($C_1$-$C_6$)alkyl; Carboxy; ($C_1$-$C_6$)Alkoxycarbonyl; Benzoyl; 4-Methyl-1-piperazinylcarbonyl; Phenyl; Phenyl substituiert mit Halogen oder Cyano; Phenyl($C_1$-$C_6$)alkyl substituiert mit Hydroxy; ($C_1$-$C_6$)Alkoxy; Nitro; Amino; Amino substituiert mit Substituent(en), ausgewählt aus der Gruppe, die besteht aus ($C_1$-$C_6$)Alkyl,

Acyl, das die Bedeutung aufweist: Alkanoyl ausgewählt aus Formyl, Acetyl, Propionyl, Butyryl, Isobutyryl, Valeryl, Isovaleryl, Pivaloyl, Hexanoyl, Heptanoyl oder 3,3-Dimethylbutyryl, Halogen($C_1$-$C_6$)alkanoyl, Hydroxy($C_1$-$C_6$)alkanoyl, ($C_1$-$C_6$)Alkylsulfonyloxy($C_1$-$C_6$)alkanoyl, ($C_1$-$C_6$)Alkoxy($C_1$-$C_6$)alkanoyl, ($C_1$-$C_6$)Alkylthio($C_1$-$C_6$)alkanoyl, ($C_1$-$C_6$)Alkanoyloxy($C_1$-$C_6$)alkanoyl, Aryloxy($C_1$-$C_6$)alkanoyl, Aroyl($C_1$-$C_6$)alkanoyl, Carboxy($C_1$-$C_6$)-alkanoyl, ($C_1$-$C_6$)Alkoxycarbonyl ($C_1$-$C_6$)alkanoyl, Carbamoyl($C_1$-$C_6$)alkanoyl, ($C_1$-$C_6$)Alkylcarbamoyl($C_1$-$C_6$)alkanoyl, Ar($C_1$-$C_6$)alkanoyl, wahlweise substituiertes Heterocyclyl($C_1$-$C_6$)alkanoyl, Heterocyclylthio($C_1$-$C_6$)alkanoyl, ($C_3$-$C_6$)Alkenoyl, ($C_3$-$C_6$)Alkinoyl, Cyclo($C_3$-$C_6$)alkylcarbonyl, Cyclo($C_3$-$C_6$)alkenylcarbonyl, Carboxy, ($C_1$-$C_6$)Alkoxycarbonyl, Aryloxycarbonyl, Aroyl, ($C_1$-$C_6$)Alkoxyaroyl, Halogenaroyl, ($C_1$-$C_6$)Alkoxycarbonylaroyl, Ar($C_3$-$C_6$)alkenoyl, ($C_1$-$C_6$)Alkoxy-ar($C_3$-$C_6$)alkenoyl, ($C_1$-$C_6$)Alkylendioxy-ar($C_3$-$C_6$)alkenoyl, Nitro-ar($C_3$-$C_6$)alkenoyl, Halogen-ar($C_3$-$C_6$)alkenoyl, Hydroxy-ar($C_3$-$C_6$)alkenoyl, Hydroxy($C_1$-$C_6$)alkoxy-ar($C_3$-$C_6$)alkenoyl, Amino($C_1$-$C_6$)alkoxy-ar($C_3$-$C_6$)alkenoyl, ($C_1$-$C_6$)Alkylamino($C_1$-$C_6$)alkoxy-ar($C_3$-$C_6$)alkenoyl, Heterocyclyl($C_1$-$C_6$)alkoxy-ar($C_3$-$C_6$)alkenoyl, wahlweise substituiertes Heterocyclyl-ar($C_3$-$C_6$)alkenoyl, Amino-ar($C_3$-$C_6$)alkenoyl, ($C_1$-$C_6$)Alkylamino-ar($C_3$-$C_6$)alkenoyl, ($C_1$-$C_6$)Alkanoylamino-ar($C_3$-$C_6$)alkenoyl, Hydroxy($C_1$-$C_6$)alkanoylamino-ar($C_3$-$C_6$)alkenoyl, ($C_1$-$C_6$)Alkoxy($C_1$-$C_6$)alkanoylamino-ar($C_3$-$C_6$)alkenoyl, Halogen($C_1$-$C_6$)alkanoylamino-ar($C_3$-$C_6$)alkenoyl, Amino($C_1$-$C_6$)alkanoylamino-ar($C_3$-$C_6$)alkenoyl, ($C_1$-$C_6$)Alkylamino($C_1$-$C_6$)alkanoylamino-ar($C_3$-$C_6$)alkenoyl, ($C_1$-$C_6$)Alkanoylamino($C_1$-$C_6$)alkanoylamino-ar($C_3$-$C_6$)alkenoyl, Carboxy($C_1$-$C_6$)alkanoylamino-ar($C_3$-$C_6$)alkenoyl, ($C_1$-$C_6$)Alkoxycarbonyl($C_1$-$C_6$)alkanoylamino-ar($C_3$-$C_6$)alkenoyl, ($C_1$-$C_6$)Alkoxycarbonyl($C_3$-$C_6$)alkenoylamino-ar($C_3$-$C_6$)alkenoyl, Halogen($C_1$-$C_6$)alkoxycarbonylamino-ar($C_3$-$C_6$)alkenoyl, Heterocyclyl($C_1$-$C_6$)alkanoylamino-ar($C_3$-$C_6$)alkenoyl, Aroylamino-ar($C_3$-$C_6$)alkenoyl, Heterocyclylcarbonylamino-ar($C_3$-$C_6$)alkenoyl, ($C_1$-$C_6$)Alkylsulfonylamino-ar($C_3$-$C_6$)alkenoyl, N-($C_1$-$C_6$ Alkanoyl)-N-($C_1$-$C_6$ alkyl)amino-ar($C_3$-$C_6$)alkenoyl, N-[($C_1$-$C_6$)Alkoxy($C_1$-$C_6$)alkanoyl]-N-($C_1$-$C_6$ alkyl)amino-ar($C_3$-$C_6$)alkenoyl, N-($C_1$-$C_6$ Alkanoyl)-N-[Heterocyclyl($C_1$-$C_6$)alkyl]amino-ar($C_3$-$C_6$)alkenoyl, Ureido-ar($C_3$-$C_6$)alkenoyl, ($C_1$-$C_6$)Alkylureido-ar($C_3$-$C_6$)alkenoyl, Heterocyclylureido-ar($C_3$-$C_6$)alkenoyl, ($C_1$-$C_6$)Alkanoyl-ar($C_3$-$C_6$)alkenoyl, Carboxy-ar($C_3$-$C_6$)alkenoyl, ($C_1$-$C_6$)Alkoxycarbonyl-ar($C_3$-$C_6$)alkenoyl, Carbamoyl-ar($C_3$-$C_6$)alkenoyl, ($C_1$-$C_6$)Alkylcarbamoyl-ar($C_3$-$C_6$)alkenoyl, Hydroxy($C_1$-$C_6$)alkylcarbamoyl-ar($C_3$-$C_6$)alkenoyl, N-[Hydroxy($C_1$-$C_6$)alkyl]-N-($C_1$-$C_6$ alkyl) carbamoyl-ar ($C_3$-$C_6$)alkenoyl, ($C_1$-$C_6$)Alkoxy($C_1$-$C_6$)alkylcarbamoyl-ar($C_3$-$C_6$)alkenoyl, N-[($C_1$-$C_6$)Alkoxy($C_1$-$C_6$)alkyl]-N-($C_1$-$C_6$ alkyl)carbamoyl-ar($C_3$-$C_6$)alkenoyl, Heterocyclylcarbamoyl-ar($C_3$-$C_6$)alkenoyl, Heterocyclylcarbonyl-ar($C_3$-$C_6$)alkenoyl, Ar($C_3$-$C_6$)alkinoyl, Heterocyclyl($C_3$-$C_6$)alkenoyl, Amino-heterocyclyl ($C_3$-$C_6$)alkenoyl, ($C_1$-$C_6$)Alkylamino-heterocyclyl($C_3$-$C_6$)alkenoyl, ($C_1$-$C_6$)Alkanoylamino-heterocyclyl($C_3$-$C_6$)alkenoyl, ($C_1$-$C_6$)Alkylureido-heterocyclyl($C_3$-$C_6$)alkenoyl, Carboxy-heterocyclyl ($C_3$-$C_6$)alkenoyl, ($C_1$-$C_6$)-Alkoxycarbonyl-heterocyclyl($C_3$-$C_6$) alkenoyl, ($C_1$-$C_6$)Alkylcarbamoyl-heterocyclyl($C_3$-$C_6$)alkenoyl, ($C_1$-$C_6$)Alkoxy($C_1$-$C_6$)alkylcarbamoyl-heterocyclyl($C_3$-$C_6$) alkenoyl, Hydroxy($C_1$-$C_6$)alkylcarbamoyl-heterocyclyl($C_3$-$C_6$)alkenoyl, Heterocyclylcarbonyl und mit einem Substituenten substituiertes Heterocyclylcarbonyl, ausgewählt aus 4-Methyl-1-piperazinylcarbonyl, 4-Ethyl-1-piperazinylcarbonyl, Dimethylaminopiperidinocarbonyl und 4-Methylcarbamoyl-1-piperazinylcarbonyl, Aryloxycarbonyl, das mit Nitro substiuiert sein kann,

Ar($C_1$-$C_6$)alkoxycarbonyl, das mit Nitro substituiert sein kann, Carbamoyl, Thiocarbamoyl, ($C_1$-$C_6$)Alkylcarbamoyl, Carboxy($C_1$-$C_6$)alkylcarbamoyl, ($C_1$-$C_6$)Alkoxycarbonyl($C_1$-$C_6$)alkylcarbamoyl, ($C_2$-$C_6$)Alkenylcarbamoyl, Cyclo($C_3$-$C_6$)alkylcarbamoyl, Halogen($C_1$-$C_6$)alkanoylcarbamoyl, Arylcarbamoyl, Arylthiocarbamoyl, ($C_1$-$C_6$)Alkoxy-arylcarbamoyl, Halogen-arylcarbamoyl, Halogen($C_1$-$C_6$)alkyl-arylcarbamoyl, Nitro-arylcarbamoyl, Cyano-arylcarbamoyl, Hydroxy ($C_1$-$C_6$)alkyl-arylcarbamoyl, Amino-arylcarbamoyl, ($C_1$-$C_6$)Alkylamino-arylcarbamoyl, ($C_1$-$C_6$)Alkanoylaminoarylcarbamoyl, N-($C_1$-$C_6$ Alkanoyl)-N-($C_1$-$C_6$alkyl)aminoarylcarbamoyl, ($C_1$-$C_6$)Alkoxy($C_1$-$C_6$)alkanoylamino-arylcarbamoyl, ($C_1$-$C_6$)Alkoxycarbonyl ($C_1$-$C_6$)alkanoylaminoarylcarbamoyl, Carboxyamino-arylcarbamoyl, ($C_1$-$C_6$)Alkoxycarbonylamino-arylcarbamoyl, Ureido-arylcarbamoyl, ($C_1$-$C_6$)Alkylureido-arylcarbamoyl, Hydroxyimino($C_1$-$C_6$)alkyl-arylcarbamoyl, ($C_1$-$C_6$)Alkoxyimino($C_1$-$C_6$)alkyl-arylcarbamoyl, ($C_1$-$C_6$)Alkylhydrazono($C_1$-$C_6$)alkylarylcarbamoyl, wahlweise substituiertes Heterocyclyl-arylcarbamoyl, Carboxy-arylcarbamoyl, ($C_1$-$C_6$)Alkoxycarbonyl-arylcarbamoyl, Heterocyclylcarbonyl-arylcarbamoyl, Heterocyclylcarbonyl-arylcarbamoyl substi-tuiert mit ($C_1$-$C_6$)Alkyl, Heterocyclylcarbonyl-arylcarbamoyl substituiert mit Aryl, Heterocyclylcarbonyl-arylcarbamoyl substituiert mit einer heterocyclischen Gruppe, Heterocyclylcarbonyl-arylcarbamoyl substituiert mit ($C_1$-$C_6$)Alkanoyl, Heterocyclylcarbonyl-arylcarbamoyl substituiert mit ($C_1$-$C_6$)Alkoxycarbonyl, Heterocyclylcarbonyl-arylcarbamoyl substituiert mit ($C_1$-$C_6$)Alkylamino, Heterocyclylcarbonyl-arylcarbamoyl, substituiert mit ($C_1$-$C_6$)Alkylcarbamoyl, Carbamoyl-arylcarbamoyl, ($C_1$-$C_6$)Alkylcarbamoyl-arylcarbamoyl, Hydroxy($C_1$-$C_6$)alkylcarbamoyl-arylcarbamoyl, N-[Hydroxy($C_1$-$C_6$)alkyl]-N-($C_1$-$C_6$ alkyl) carbamoyl-arylcarbamoyl, ($C_1$-$C_6$)Alkoxy($C_1$-$C_6$)alkylcarbamoyl-arylcarbamoyl, N-[($C_1$-$C_6$)Alkoxy($C_1$-$C_6$)alkyl]-N-($C_1$-$C_6$ alkyl)carbamoyl-arylcarbamoyl, ($C_1$-$C_6$)Alkylamino($C_1$-$C_6$)alkylcarbamoyl-arylcarbamoyl, N-[($C_1$-$C_6$)Alkylamino($C_1$-$C_6$)alkyl]-N-($C_1$-$C_6$alkyl)carbamoyl-arylcarbamoyl, Heterocyclylcarbamoyl-arylcarbamoyl, N-(Heterocyclyl)-N-($C_1$-$C_6$ alkyl)carbamoylarylcarbamoyl, Heterocyclyl($C_1$-$C_6$)alkylcarbamoylarylcarbamoyl, N-[Heterocyclyl($C_1$-$C_6$)alkyl]-N-($C_1$-$C_6$ alkyl)carbamoyl-arylcarbamoyl, N-[Heterocyclyl($C_1$-$C_6$)alkyl]-N-($C_1$-$C_6$ alkoxy)($C_1$-$C_6$)alkyl]carbamoylarylcarbamoyl, Arylcarbamoyl-arylcarbamoyl, ($C_1$-$C_6$)Alkylamino-arylcarbamoyl-arylcarbamoyl, ($C_1$-$C_6$)Alkanoyl-arylcarbamoyl, Ar($C_1$-$C_6$)alkylcarbamoyl, Heterocyclylcarbamoyl, Heterocyclyl ($C_1$-$C_6$)alkylcarbamoyl, Arylaminocarbamoyl, Aroylcarbamoyl, ($C_1$-$C_6$)Alkylsulfonyl, Arylsulfonyl, Ar($C_1$-$C_6$)alkylsulfonyl, Ar($C_2$-$C_6$)alkenylsulfonyl, Phthaloyl, ein Aminosäurerest, worin die Aminosäure ausgewählt wird aus Glycin, Sarcosin, Alanin, β-Alanin, Valin, Norvalin, Leucin, Isoleucin, Norleucin, Serin, Threonin, Cystein, Methionin, Phenylalanin, Phenylglycin, Tryptophan, Tyrosin, Prolin, Hydroxyprolin, Glutaminsäure, Asparaginsäure, Glutamin, Asparagin, Lysin, Arginin, Histidin und Ornithin, die substituiert sein können mit Substituent(en) ausgewählt aus ($C_1$-$C_6$)Alkyl, Aryl, Ar($C_1$-$C_6$)alkyl, einer heterocyclischen Gruppe, Heterocyclyl($C_1$-$C_6$)alkyl, Cycloalkyl, ausgewählt aus Cyclopropyl, Cyclobutyl, Cyclopentyl, Cyclohexyl, Cycloheptyl, Cyclooctyl und Adamantyl, Amidino substituiert mit Amidino ausgewählt aus Methylamidino und N-Ethyl-N'-cyanoamidino, Alkanoyl ausgewählt aus Formyl, Acetyl, Propionyl, Butyryl, Isobutyryl, Valeryl, Isovaleryl, Pivaloyl, Hexanoyl und Heptanoyl, Halogen($C_1$-$C_6$)alkanoyl, Hydroxy($C_1$-$C_6$)alkanoyl, ($C_1$-$C_6$)Alkylsulfonyl($C_1$-$C_6$)alkanoyl, ($C_1$-$C_6$)Alkoxy($C_1$-$C_6$)alkanoyl, Aryloxy($C_1$-$C_6$)alkanoyl, ($C_1$-$C_6$)Alkylthio($C_1$-$C_6$)alkanoyl, ($C_1$-$C_6$)Alkylcarbamoyl($C_1$-$C_6$)alkanoyl, ($C_1$-$C_6$)Alkanoyloxy($C_1$-$C_6$)alkanoyl, Carboxy($C_1$-$C_6$)alkanoyl, ($C_1$-$C_6$)Alkoxycarbonyl($C_1$-$C_6$)alkanoyl, Ar($C_1$-$C_6$)alkanoyl, wahlweise substituiertes Heterocyclyl($C_1$-$C_6$) alkanoyl, ($C_3$-$C_6$)Alkenoyl, Ar($C_3$-$C_6$)alkenoyl, ($C_1$-$C_6$)Alkoxy-ar($C_3$-$C_6$)alkenoyl, ($C_1$-$C_6$)Alkylendioxy-ar($C_3$-$C_6$)alkenoyl, Nitro-ar($C_3$-$C_6$)alkenoyl, Halogen-ar($C_3$-$C_6$)alkenoyl, Hydroxy-ar($C_3$-$C_6$)alkenoyl, Hydroxy($C_1$-$C_6$)alkoxy-ar($C_3$-$C_6$)-alkenoyl, Amino($C_1$-$C_6$)alkoxy-ar($C_3$-$C_6$)alkenoyl, ($C_1$-$C_6$)Alkylamino($C_1$-$C_6$)alkoxy-ar($C_3$-$C_6$)alkenoyl, Heterocyclyl($C_1$-$C_6$)alkoxy-ar($C_3$-$C_6$)alkenoyl, wahlweise substituiertes Heterocyclyl-ar($C_3$-$C_6$)alkenoyl, Amino-ar($C_3$-$C_6$)alkenoyl, ($C_1$-$C_6$)Alkylamino-ar($C_3$-$C_6$)alkenoyl, ($C_1$-$C_6$)Alkanoylamino-ar($C_3$-$C_6$)alkenoyl, Hydroxy($C_1$-$C_6$)alkanoylamino-ar($C_3$-$C_6$)alkenoyl, ($C_1$-$C_6$)Alkoxy($C_1$-$C_6$)alkanoylamino-ar($C_3$-$C_6$)alkenoyl, Halogen($C_1$-$C_6$)alkanoylamino-ar($C_3$-$C_6$)alkenoyl, Amino($C_1$-$C_6$) alkanoylamino-ar($C_3$-$C_6$)alkenoyl, ($C_1$-$C_6$)Alkylamino($C_1$-$C_6$)alkanoylamino-ar($C_3$-$C_6$)alkenoyl, ($C_1$-$C_6$)Alkanoylamino($C_1$-$C_6$)alkanoylamino-ar($C_3$-$C_6$)alkenoyl, Carboxy($C_1$-$C_6$)alkanoylamino-ar($C_3$-$C_6$)alkenoyl, ($C_1$-$C_6$)Alkoxycarbonyl($C_1$-$C_6$)alkanoylamino-ar($C_3$-$C_6$)alkenoyl, ($C_1$-$C_6$)Alkoxycarbonyl($C_3$-$C_6$)alkenoylamino-ar($C_3$-$C_6$)alkenoyl, Halogen($C_1$-$C_6$)alkoxycarbonylamino-ar($C_3$-$C_6$)alkenoyl, Heterocyclyl($C_1$-$C_6$)alkanoylamino-ar($C_3$-$C_6$)alkenoyl, Aroylamino-ar($C_3$-$C_6$)alkenoyl, Heterocyclylcarbonyl-ar($C_3$-$C_6$)alkenoyl, ($C_1$-$C_6$)Alkylsulfonylamino-ar($C_3$-$C_6$)alkenoyl, N-($C_1$-$C_6$ Alkanoyl)-N-($C_1$-$C_6$ alkyl)amino-ar($C_3$-$C_6$)alkenoyl, N-[($C_1$-$C_6$)Alkoxy($C_1$-$C_6$)alkanoyl]-N($C_1$-$C_6$ alkyl)amino-ar($C_3$-$C_6$)alkenoyl, N-($C_1$-$C_6$ Alkanoyl)-N-[heterocyclyl($C_1$-$C_6$)alkyl]amino-ar($C_3$-$C_6$)alkenoyl, Ureido-ar($C_3$-$C_6$)alkenoyl, ($C_1$-$C_6$)Alkylureido-ar($C_3$-$C_6$)alkenoyl, Heterocyclylureido-ar($C_3$-$C_6$)alkenoyl, ($C_1$-$C_6$)Alkanoyl-ar($C_3$-$C_6$)alkenoyl, Carboxy-ar($C_3$-$C_6$)-alkenoyl, ($C_1$-$C_6$)Alkoxycarbonyl-ar($C_3$-$C_6$)alkenoyl, Carbamoyl-ar($C_3$-$C_6$)alkenoyl, ($C_1$-$C_6$)Alkylcarbamoyl-ar($C_3$-$C_6$)alkenoyl, Hydroxy($C_1$-$C_6$)alkylcarbamoyl-ar($C_3$-$C_6$)alkenoyl, N-[Hydroxy($C_1$-$C_6$)alkyl]-N-($C_1$-$C_6$ alkyl)carbamoyl-ar($C_3$-$C_6$)alkenoyl, ($C_1$-$C_6$)Alkoxy($C_1$-$C_6$)alkylcarbamoyl-ar($C_3$-$C_6$)alkenoyl, N-[($C_1$-$C_6$)Alkoxy($C_1$-$C_6$)alkyl]-N-($C_1$-$C_6$ alkyl)carbamoyl-ar($C_3$-$C_6$)alkenoyl, Heterocyclylcarbamoyl-

ar($C_3$-$C_6$)alkenoyl, Heterocyclylcarbonyl-ar($C_3$-$C_6$)alkenoyl, Ar($C_3$-$C_6$)alkinoyl, Heterocyclyl($C_3$-$C_6$)alkenoyl, Amino-heterocyclyl($C_3$-$C_6$)alkenoyl, ($C_1$-$C_6$)Alkylamino-heterocyclyl($C_3$-$C_6$)alkenoyl, ($C_1$-$C_6$)Alkanoylamino-heterocyclyl ($C_3$-$C_6$)alkenoyl, ($C_1$-$C_6$)Alkylureido-heterocyclyl($C_3$-$C_6$)alkenoyl, Carboxyheterocyclyl($C_3$-$C_6$)alkenoyl, ($C_1$-$C_6$)Alkoxycarbonylheterocyclyl($C_3$-$C_6$)alkenoyl, ($C_1$-$C_6$)Alkylcarbamoylheterocyclyl($C_3$-$C_6$)alkenoyl, ($C_1$-$C_6$)Alkoxy($C_1$-$C_6$)alkylcarbamoyl-heterocyclyl($C_3$-$C_6$)alkenoyl, Hydroxy($C_1$-$C_6$)alkylcarbamoyl-heterocyclyl($C_3$-$C_6$)alkenoyl, Heterocyclylthio($C_1$-$C_6$)alkanoyl, wahlweise substituiertes Heterocyclylcarbonyl, Cyclo($C_3$-$C_6$)alkylcarbonyl, ($C_1$-$C_6$)Alkoxycarbonyl, Aryloxycarbonyl, Aroyl($C_1$-$C_6$)alkanoyl, Aroyl, Nitro-aryloxycarbonyl, Carbamoyl, ($C_1$-$C_6$)Alkylcarbamoyl, ($C_1$-$C_6$)Alkoxycarbonyl ($C_1$-$C_6$)alkylcarbamoyl, ($C_2$-$C_6$)Alkenylcarbamoyl, Cyclo($C_3$-$C_6$)alkylcarbamoyl, Arylcarbamoyl, ($C_1$-$C_6$)Alkoxy-arylcarbamoyl, Halogen($C_1$-$C_6$)alkyl-arylcarbamoyl, Halogen-arylcarbamoyl, Hydroxy($C_1$-$C_6$)alkyl-arylcarbamoyl, Nitro-aryl-carbamoyl, Cyano-arylcarbamoyl, Amino-arylcarbamoyl, ($C_1$-$C_6$)Alkylamino-arylcarbamoyl, ($C_1$-$C_6$)Alkanoylaminoarylcarbamoyl, N-($C_1$-$C_6$ Alkanoyl)-N-($C_1$-$C_6$ alkyl)aminoarylcarbamoyl, ($C_1$-$C_6$)Alkoxy($C_1$-$C_6$)alkanoylamino-arylcarbamamoyl, ($C_1$-$C_6$)Alkoxycarbonyl($C_1$-$C_6$)alkanoylaminoarylcarbamoyl, Carboxyamino-arylcarbamoyl, ($C_1$-$C_6$)Alkoxycarbonylamino-arylcarbamoyl, Ureido-arylcarbamoyl, ($C_1$-$C_6$)Alkylureido-arylcarbamoyl, Hydroxyimino($C_1$-$C_6$)alkyl-arylcarbamoyl, ($C_1$-$C_6$)Alkoxyimino($C_1$-$C_6$)alkylcarbamoyl, ($C_1$-$C_6$)Alkylhydrazono($C_1$-$C_6$)alkylarylcarbamoyl, wahlweise substituiertes Heterocyclyl-arylcarbamoyl, ($C_1$-$C_6$)Alkanoyl-arylcarbamoyl, Heterocyclylcarbonyl-arylcarbamoyl, Carboxy-arylcarbamoyl, ($C_1$-$C_6$)Alkoxycarbonyl-arylcarbamoyl, ($C_1$-$C_6$)Alkylcarbamoyl-arylcarbamoyl, Heterocyclylcarbonyl-arylcarbamoyl mit ($C_1$-$C_6$)Alkyl, Heterocyclylcarbonyl-arylcarbamoyl mit Aryl, Heterocyclylcarbonyl-arylcarbamoyl mit einer heterocyclischen Gruppe, Heterocyclylcarbonyl-arylcarbamoyl mit ($C_1$-$C_6$)Alkanoyl, Heterocyclylcarbonyl-arylcarbamoyl mit ($C_1$-$C_6$)Alkoxycarbonyl, Heterocyclylcarbonyl-arylcarbamoyl mit ($C_1$-$C_6$)Alkylamino, Heterocyclylcarbonyl-arylcarbamoyl mit ($C_1$-$C_6$)Alkylcarbamoyl, Hydroxy($C_1$-$C_6$)alkylcarbamoyl-arylcarbamoyl, N-[Hydroxy-($C_1$-$C_6$)alkyl]-N-($C_1$-$C_6$ alkyl)carbamoyl-arylcarbamoyl, ($C_1$-$C_6$)Alkoxy($C_1$-$C_6$)alkylcarbamoyl-arylcarbamoyl, N-[($C_1$-$C_6$)Alkoxy($C_1$-$C_6$)alkyl]-N-($C_1$-$C_6$ alkyl)carbamoylarylcarbamoyl, ($C_1$-$C_6$)Alkylamino($C_1$-$C_6$)alkylcarbamoylarylcarbamoyl, N-[($C_1$-$C_6$)Alkylamino($C_1$-$C_6$)alkyl]-N-($C_1$-$C_6$ alkyl)carbamoyl-arylcarbamoyl, Heterocyclylcarbamoyl-arylcarbamoyl, N-(Heterocyclyl)-N-($C_1$-$C_6$ alkyl)carbamoyl-arylcarbamoyl, Heterocyclyl($C_1$-$C_6$)alkylcarbamoyl-arylcarbamoyl, N-[Heterocyclyl($C_1$-$C_6$)alkyl]-N-($C_1$-$C_6$ alkyl)carbamoyl-arylcarbamoyl, N-[Heterocyclyl($C_1$-$C_6$)alkyl]-N-[($C_1$-$C_6$)alkoxy($C_1$-$C_6$)alkyl]carbamoyl-arylcarbamoyl, Arylcarbamoyl-arylcarbamoyl, ($C_1$-$C_6$)Alkylaminoarylcarbamoyl-arylcarbamoyl, Arylthiocarbamoyl, Ar($C_1$-$C_6$)alkylcarbamoyl, Aroylcarbamoyl, Heterocyclylcarbamoyl, Heterocyclyl($C_1$-$C_6$)alkylcarbamoyl, Arylaminocarbamoyl, Ar($C_2$-$C_6$)alkenylsulfonyl, ($C_1$-$C_6$)Alkylsulfonyl, Phthaloyl, unsubstituierter Aminosäurerest, ausgewählt aus Glycin, Sarcosin, Alanin, β-Alanin, Valin, Norvalin, Leucin, Isoleucin, Norleucin, Serin, Threonin, Cystein, Methionin, Phenylalanin, Phenylglycin, Tryptophan, Tyrosin, Prolin, Hydroxyprolin, Glutaminsäure, Asparaginsäure, Glutamin, Asparagin, Lysin, Arginin, Histidin oder Ornithin, Aminosäurerest substituiert mit ($C_1$-$C_6$)Alkyl, worin der Aminosäurerest wie der zuvor definierte unsubstituierte Aminosäurerest ist, Aminosäurerest substituiert mit einer heterocyclischen Gruppe, worin der Aminosäurerest wie der zuvor definierte unsubstituierte Aminosäurerest ist, Aminosäurerest substituiert mit Heterocyclyl($C_1$-$C_6$)alkyl, worin der Aminosäurerest wie der zuvor definierte unsubstituierte Aminosäurerest ist, Aminosäurerest substituiert mit Cycloalkyl, ausgewählt aus Cyclopropyl, Cyclobutyl, Cyclopentyl, Cyclohexyl, Cycloheptyl, Cyclooctyl und Adamantyl, worin der Aminosäurerest wie der zuvor definierte unsubstituierte Aminosäurerest ist, Aminosäurerest substituiert mit Aryl, worin der Aminosäurerest wie der zuvor definierte unsubstituierte Aminosäurerest ist, Aminosäurerest substituiert mit ($C_1$-$C_6$)Alkanoyl, worin der Aminosäurerest wie der zuvor definierte unsubstituierte Aminosäurerest ist, Aminosäurerest substituiert mit ($C_1$-$C_6$)Alkoxycarbonyl, worin der Aminosäurerest wie der zuvor definierte unsubstituierte Aminosäurerest ist, Aminosäurerest substituiert mit Phthaloyl, worin der Aminosäurerest wie der zuvor definierte unsubstituierte Aminosäurerest ist, und Aminosäurerest substituiert mit Ar($C_1$-$C_6$)alkyl, worin der Aminosäurerest wie der zuvor definierte unsubstituierte Aminosäurerest ist, worin in den obigen und unten folgenden Definitionen "Ar" und "Aryl" ausgewählt werden aus: Phenyl, Naphthyl, Phenyl, das mit ($C_1$-$C_6$)Alkyl substituiert ist,

die heterocyclische Gruppe oder Anteil ausgewählt wird aus:

- ungesättigten 3- bis 8-gliedrigen, heteromonocyclischen Gruppen, die 1 bis 4 Stickstoffatom(e) enthalten,
- gesättigten 3- bis 8-gliedrigen, heteromonocyclischen Gruppe, die 1 bis 4 Stickstoffatom(e) enthalten,
- ungesättigten kondensierten 7- bis 12-gliedrigen heterocyclischen Gruppen, die 1 bis 5 Stickstoffatom(e) enthalten,
- ungesättigten 3- bis 8-gliedrigen, heteromonocyclischen Gruppen, die ein Sauerstoffatom enthalten,
- ungesättigten, kondensierten 7- bis 12-gliedrigen heterocyclischen Gruppen, die 1 bis 2 Sauerstoffatom(e) enthalten,

- ungesättigten 3- bis 8-gliedrigen, heteromonocyclischen Gruppen, die ein Schwefelatom enthalten,
- ungesättigten, kondensierten 7- bis 12-gliedrigen heterocyclischen Gruppen, die 1 bis 2 Schwefelatom(e) enthalten,
- ungesättigten 3- bis 8-gliedrigen, heteromonocyclischen Gruppen, die 1 bis 2 Sauerstoffatom(e) und 1 bis 3 Stickstoffatom(e) enthalten,
- gesättigten 3- bis 8-gliedrigen, heteromonocyclischen Gruppen, die 1 bis 2 Sauerstoffatom(e) und 1 bis 3 Stickstoffatom(e) enthalten,
- ungesättigten, kondensierten 7- bis 12-gliedrigen heterocyclischen Gruppen, die 1 bis 2 Sauerstoffatom(e) und 1 bis 3 Stickstoffatom(e) enthalten,
- ungesättigten 3- bis 8-gliedrigen, heteromonocyclischen Gruppen, die 1 bis 2 Schwefelatom(e) und 1 bis 3 Stickstoffatom(e) enthalten,
- gesättigten 3- bis 8-gliedrigen, heteromonocyclischen Gruppen, die 1 bis 2 Schwefelatom(e) und 1 bis 3 Stickstoffatom(e) enthalten,
- ungesättigten, kondensierten 7- bis 12-gliedrigen heterocyclischen Gruppe, die 1 bis 2 Schwefelatom(e) und 1 bis 3 Stickstoffatom(e) enthalten, und die Substituenten von Aryl, wie es oben definiert ist, und der heterocyclischen Gruppe, wie sie oben definiert ist, werden ausgewählt aus: Halogen; Halogen($C_1$-$C_6$)alkyl; ($C_1$-$C_6$)Alkyl; das oben erwähnte Aryl, wahlweise substituiert mit Substituent(en), ausgewählt aus Halogen und Cyano; Ar($C_1$-$C_6$)alkyl substituiert mit Hydroxy, worin der Arylanteil wie oben definiert ist; ($C_1$-$C_6$)Alkoxy; Oxo; Nitro; Amino wahlweise substituiert mit ($C_1$-$C_6$)Alkyl, das oben erwähnte Acyl, Ar($C_1$-$C_6$)alkyl, worin der Arylanteil wie oben definiert ist, Heterocyclyl($C_1$-$C_6$)alkyl, worin der heterocyclische Anteil wie oben definiert ist, Carboxy($C_1$-$C_6$)alkyl, ($C_1$-$C_6$)Alkylaminomethylen oder N-Methylpyrrolidinyliden; die oben erwähnte heterocyclische Gruppe wahlweise substituiert mit Oxo;

  Phenyl($C_1$-$C_6$)alkyl, Pyridyl($C_1$-$C_6$)alkyl, Carboxy($C_1$-$C_6$)alkyl, ($C_1$-$C_6$)Alkylaminomethylen und N-Methylpyrrolidinyliden; Pyrrolidinyl und Pyrrolidinyl substituiert mit Oxo; oder eine ungesättigte kondensierte 7- bis 12-gliedrige heterocyclische Gruppe, die 1 bis 2 Sauerstoffatom(e) enthält, oder eine ungesättigte kondensierte 7- bis 12-gliedrige heterocyclische Gruppe ist, die 1 bis 2 Schwefelatom(e) und 1 bis 3 Stickstoffatom(e) enthält, die jeweils mit Substituent(en) substituiert sind, die aus der Gruppe ausgewählt sind, die aus Halogen, ($C_1$-$C_6$)Alkyl und Oxo besteht,

Q O oder NH ist, und
A ($C_1$-$C_6$)Alkylen ist,

und pharmazeutisch verträgliche Salze davon.

**2.** Verbindung nach Anspruch 1, worin

$R^4$ Phenyl ist, das mit Substituenten substituiert ist, die aus der Gruppe ausgewählt sind, die besteht aus Halogen, Nitro, Amino und einer Gruppe der Formel:

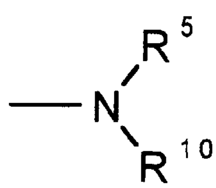

worin

$R^5$ Wasserstoff oder ($C_1$-$C_6$)Alkyl ist und
$R^{10}$ Acyl, wie in Anspruch 1 definiert ist, und

A Methylen ist.

**3.** Verbindung nach Anspruch 2, worin

$R^4$ Phenyl ist, das mit 1 oder 2 Halogen(en) und einer Gruppe der Formel substituiert ist:

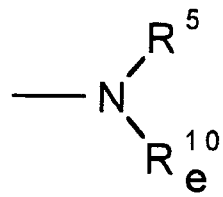

worin

$R^5$ Wasserstoff oder $(C_1-C_6)$Alkyl ist und

$R^{10}_e$ ein Aminosäurerest, wie in Anspruch 1 definiert, oder ein Aminosäurerest, wie in Anspruch 1 definiert, ist, substituiert mit einem Substituenten ausgewählt aus der Gruppe, die besteht aus: $(C_1-C_6)$Alkyl, Alkanoyl, ausgewählt aus Formyl, Acetyl, Propionyl, Butyryl, Isobutyryl, Valeryl, Isovaleryl, Pivaloyl, Hexanoyl und Heptanoyl, Halogen$(C_1-C_6)$alkanoyl, Ar$(C_1-C_6)$alkanoyl, Heterocyclyl$(C_1-C_6)$alkanoyl, $(C_3-C_6)$Alkenoyl, Ar$(C_3-C_6)$alkenoyl, $(C_1-C_6)$Alkoxy-ar$(C_3-C_6)$alkenoyl, $(C_1-C_6)$Alkylendioxy-ar$(C_3-C_6)$alkenoyl, Nitro-ar$(C_3-C_6)$alkenoyl, Halogen-ar$(C_3-C_6)$alkenoyl, Hydroxy-ar$(C_3-C_6)$alkenoyl, Hydroxy$(C_1-C_6)$alkoxy-ar$(C_3-C_6)$alkenoyl, Amino$(C_1-C_6)$alkoxy-ar$(C_3-C_6)$-alkenoyl, $(C_1-C_6)$Alkylamino$(C_1-C_6)$alkoxy-ar$(C_3-C_6)$-alkenoyl, Heterocyclyl$(C_1-C_6)$alkoxy-ar$(C_3-C_6)$-alkenoyl, Heterocyclyl-ar$(C_3-C_6)$alkenoyl wahlweise mit Oxo, Amino-ar$(C_3-C_6)$alkenoyl, $(C_1-C_6)$Alkylamino-ar$(C_3-C_6)$alkenoyl, $(C_1-C_6)$Alkanoylamino-ar$(C_3-C_6)$alkenoyl, N-$(C_1-C_6$ Alkanoyl)N-$(C_1-C_6$ alkyl)amino-ar$(C_3-C_6)$alkenoyl, Hydroxy$(C_1-C_6)$alkanoylamino-ar$(C_3-C_6)$alkenoyl, $(C_1-C_6)$Alkoxy$(C_1-C_6)$alkanoylamino-ar$(C_3-C_6)$alkenoyl, Halogen$(C_1-C_6)$-alkanoylamino-ar$(C_3-C_6)$alkenoyl, Amino$(C_1-C_6)$alkanoylamino-ar$(C_3-C_6)$alkenoyl, $(C_1-C_6)$Alkylamino$(C_1-C_6)$alkanoylamino-ar$(C_3-C_6)$alkenoyl, $(C_1-C_6)$Alkanoylamino $(C_1-C_6)$alkanoylamino-ar$(C_3-C_6)$alkenoyl, Carboxy$(C_1-C_6)$alkanoylamino-ar$(C_3-C_6)$alkenoyl, $(C_1-C_6)$Alkoxycarbonyl$(C_1-C_6)$alkanoylamino-ar$(C_3-C_6)$alkenoyl, $(C_1-C_6)$Alkoxycarbonyl$(C_3-C_6)$alkenoylamino-ar$(C_3-C_6)$alkenoyl, Halogen$(C_1-C_6)$alkoxycarbonylamino-ar$(C_3-C_6)$alkenoyl, Heterocyclyl$(C_1-C_6)$alkanoylamino-ar$(C_3-C_6)$alkenoyl, Aroylamino-ar$(C_3-C_6)$alkenoyl, Heterocyclylcarbonylamino-ar$(C_3-C_6)$alkenoyl, $(C_1-C_6)$Alkylsulfonylamino-ar$(C_3-C_6)$alkenoyl, N-[$(C_1-C_6)$Alkoxy$(C_1-C_6)$alkanoyl]-N-$(C_1-C_6$ alkyl)amino-ar$(C_3-C_6)$alkenoyl, N-$(C_1-C_6)$alkanoyl)-N-[heterocyclyl$(C_1-C_6)$alkyl]amino-ar$(C_3-C_6)$alkenoyl, Ureido-ar$(C_3-C_6)$alkenoyl, $(C_1-C_6)$Alkylureido-ar$(C_3-C_6)$alkenoyl, Heterocyclylureido-ar$(C_3-C_6)$alkenoyl, $(C_1-C_6)$Alkanoyl-ar$(C_3-C_6)$alkenoyl, Carboxy-ar$(C_3-C_6)$alkenoyl, $(C_1-C_6)$Alkoxycarbonyl-ar$(C_3-C_6)$ alkenoyl, Carbamoyl-ar$(C_3-C_6)$alkenoyl, $(C_1-C_6)$Alkylcarbamoyl-ar$(C_3-C_6)$alkenoyl, Hydroxy$(C_1-C_6)$alkylcarbamoyl-ar$(C_3-C_6)$alkenoyl, N-[Hydroxy$(C_1-C_6)$alkyl]-N-$(C_1-C_6)$alkyl)carbamoyl-ar$(C_3-C_6)$alkenoyl, $(C_1-C_6)$Alkoxy$(C_1-C_6)$alkylcarbamoyl-ar$(C_3-C_6)$alkenoyl, N-[$(C_1-C_6)$ alkoxy$(C_1-C_6)$alkyl]-N-$(C_1-C_6)$alkyl)carbamoyl-ar$(C_3-C_6)$alkenoyl, Heterocyclylcarbamoyl-ar$(C_3-C_6)$alkenoyl, Heterocyclylcarbonyl-ar$(C_3-C_6)$alkenoyl, Ar$(C_3-C_6)$alkinoyl, Heterocyclyl$(C_3-C_6)$alkenoyl, Heterocyclylthio$(C_1-C_6)$alkanoyl, Aminoheterocyclyl$(C_3-C_6)$alkenoyl, $(C_1-C_6)$alkylamino-heterocyclyl$(C_3-C_6)$alkenoyl, $(C_1-C_6)$Alkanoylamino-heterocyclyl $(C_3-C_6)$alkenoyl, $(C_1-C_6)$Alkylureido-heterocyclyl$(C_3-C_6)$alkenoyl, Carboxyheterocyclyl$(C_3-C_6)$alkenoyl, $(C_1-C_6)$Alkoxycarbonylheterocyclyl$(C_3-C_6)$alkenoyl, $(C_1-C_6)$Alkylcarbamoylheterocyclyl$(C_3-C_6)$alkenoyl, $(C_1-C_6)$Alkoxy $(C_1-C_6)$alkylcarbamoyl-heterocyclyl$(C_3-C_6)$alkenoyl, Hydroxy$(C_1-C_6)$alkylcarbamoyl-heterocyclyl$(C_3-C_6)$alkenoyl, Heterocyclylcarbonyl, Cyclo$(C_3-C_6)$alkylcarbonyl, $(C_1-C_6)$Alkoxycarbonyl, Aryloxycarbonyl, Aroyl$(C_1-C_6)$alkanoyl, Aroyl, Nitro-Aryloxycarbonyl, Carbamoyl, $(C_1-C_6)$Alkylcarbamoyl, $(C_1-C_6)$Alkoxycarbonyl$(C_1-C_6)$alkylcarbamoyl, $(C_2-C_6)$Alkenylcarbamoyl, Cyclo$(C_3-C_6)$alkylcarbamoyl, Arylcarbamoyl, $(C_1-C_6)$Alkoxyarylcarbamoyl, Halogen$(C_1-C_6)$alkylarylcarbamoyl, Halogen-arylcarbamoyl, $(C_1-C_6)$Alkanoyl-arylcarbamoyl, Hydroxy$(C_1-C_6)$alkylarylcarbamoyl, Heterocyclylcarbonyl-arylcarbamoyl, Carboxy-arylcarbamoyl, $(C_1-C_6)$Alkoxycarbonylarylcarbamoyl, $(C_1-C_6)$Alkylcarbamoyl-arylcarbamoyl, Nitro-arylcarbamoyl, Cyano-arylcarbamoyl, Amino-arylcarbamoyl, $(C_1-C_6)$Alkylamino-arylcarbamoyl, $(C_1-C_6)$Alkanoylamino-arylcarbamoyl, N-$(C_1-C_6$ Alkanoyl)-N-$(C_1-C_6$ alkyl)amino-arylcarbamoyl, $(C_1-C_6)$Alkoxy$(C_1-C_6)$alkanoylamino-arylcarbamoyl, $(C_1-C_6)$Alkoxycarbonyl $(C_1-C_6)$alkanoylamino-arylcarbamoyl, Carboxyamino-arylcarbamoyl, $(C_1-C_6)$Alkoxycarbonylamino-arylcarbamoyl, Ureido-arylcarbamoyl, $(C_1-C_6)$Alkylureido-arylcarbamoyl, Hydroxyimino$(C_1-C_6)$alkyl-arylcarbamoyl, $(C_1-C_6)$ Alkoxyimino$(C_1-C_6)$alkyl-arylcarbamoyl, $(C_1-C_6)$Alkylhydrazono$(C_1-C_6)$alkyl-arylcarbamoyl, Heterocyclyl-arylcarbamoyl wahlweise mit Oxo, Heterocyclylcarbonyl-arylcarbamoyl mit $(C_1-C_6)$Alkyl, Heterocyclylcarbonyl-arylcarbamoyl mit Aryl, Heterocyclylcarbonyl-arylcarbamoyl mit einer heterocyclischen Gruppe, Heterocyclylcarbonyl-arylcarbamoyl mit ($C_1$-$C_6$)Alkanoyl, Heterocyclylcarbonyl-arylcarbamoyl mit ($C_1$-$C_6$)alkoxycarbonyl, Heterocyclylcarbonyl-arylcarbamoyl mit ($C_1$-$C_6$)Alkylamino, Heterocyclylcarbonyl-arylcarbamoyl mit ($C_1$-$C_6$)Alkylcarbamoyl, Hydroxy($C_1$-$C_6$)alkylcarbamoyl-arylcarbamoyl, N-[Hydroxy($C_1$-$C_6$)alkyl]-N-($C_1$-$C_6$ alkyl) carbamoylarylcarbamoyl, ($C_1$-$C_6$)Alkoxy($C_1$-$C_6$)alkylcarbamoylarylcarbamoyl, N-[($C_1$-$C_6$)Alkoxy($C_1$-$C_6$)alkylj-N-($C_1$-$C_6$ alkyl)carbamoyl-arylcarbamoyl, ($C_1$-$C_6$)Alkylamino($C_1$-$C_6$)alkylcarbamoyl-arylcarbamoyl, N-[($C_1$-$C_6$)Alkylamino($C_1$-$C_6$)alkyl]-N-($C_1$-$C_6$ alkyl)carbamoyl-arylcarbamoyl, Heterocyclylcarbamoyl-arylcarbamoyl, N-(Heterocyclyl)-N-($C_1$-$C_6$ alkyl)carbamoyl-arylcarbamoyl, Heterocyclyl-($C_1$-$C_6$)alkylcarbamoyl-arylcarbamoyl, N-[Heterocyclyl($C_1$-$C_6$)alkyl]-N-($C_1$-$C_6$ alkyl)carbamoyl-arylcarbamoyl, N-[Heterocyclyl($C_1$-$C_6$)alkyl]-N-[($C_1$-$C_6$)alkoxy($C_1$-$C_6$)alkyl]carbamoyl-arylcarbamoyl, Arylcarbamoyl-arylcarbamoyl, ($C_1$-$C_6$)Alkylaminoarylcarbamoyl-arylcarbamoyl, Arylthiocarbamoyl, Ar($C_1$-$C_6$)alkylcarbamoyl, Aroylcarbamoyl, Heterocyclylcarbamoyl, Heterocyclyl($C_1$-$C_6$)alkylcarbamoyl, Arylaminocarbamoyl, Ar($C_2$-$C_6$)alkenylsulfonyl, ($C_1$-$C_6$)Alkylsulfonyl, Phthaloyl, Aminosäurerest wie in Anspruch 1 definiert, Aminosäurerest wie in Anspruch 1 definiert substituiert mit ($C_1$-$C_6$)Alkyl, Aminosäurerest wie in Anspruch 1 definiert substituiert mit einer heterocyclischen Gruppe, Aminosäurerest wie in Anspruch 1 definiert mit Heterocyclyl($C_1$-$C_6$)alkyl, Aminosäurerest wie in Anspruch 1 definiert substituiert mit Cycloalkyl ausgewählt aus Cyclopropyl, Cyclobutyl, Cyclopentyl, Cyclohexyl, Cycloheptyl, Cyclooctyl und Adamantyl, Aminosäurerest wie in Anspruch 1 definiert substituiert mit Aryl, Aminosäurerest wie in Anspruch 1 definiert, substituiert mit Alkanoyl, ausgewählt aus Formyl, Acetyl, Propionyl, Butyryl, Isobutyryl, Valeryl, Isovaleryl, Pivaloyl, Hexanoyl und Heptanoyl, Aminosäurerest wie in Anspruch 1 definiert substi-tuiert mit ($C_1$-$C_6$)Alkoxycarbonyl, Aminosäurerest wie in Anspruch 1 definiert substituiert mit Ar($C_1$-$C_6$)alkyl und Aminosäurerest wie in Anspruch 1 definiert substituiert mit Phthaloyl; in den obigen Definitionen sind Aryl, die heterocyclische Gruppe oder der heterocyclische Anteil jeweils wie in Anspruch 1 definiert.

**4.** Verbindung nach Anspruch 1, worin

$R^4$ eine ungesättigte kondensierte 7- bis 12-gliedrige heterocyclische Gruppe, die 1 bis 2 Sauerstoffatom(e) enthält oder eine ungesättigte kondensierte 7- bis 12-gliedrige heterocyclische Gruppe ist, die 1 bis 2 Schwefelatom(e) und 1 bis 3 Stickstoffatom(e) enthält, die jeweils substituiert sind, mit Substituent(en) ausgewählt aus der Gruppe, die aus Halogen, ($C_1$-$C_6$)Alkyl und Oxo besteht.

**5.** Halogenierung einer Verbindung der Formel:

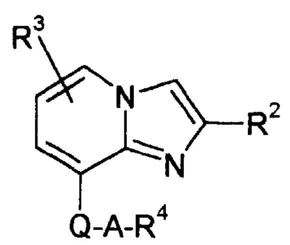

worin $R^2$, $R^3$, $R^4$, Q und A wie in Anspruch 1 definiert sind, oder deren Salz, um eine Verbindung der Formel:

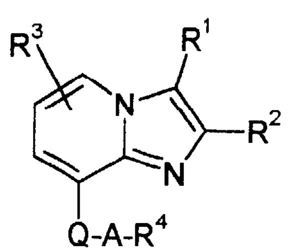

worin $R^1$, $R^2$, $R^3$, $R^4$, Q und A wie in Anspruch 1 definiert sind, oder deren Salz zu ergeben.

**6.** Reaktion einer Verbindung der Formel:

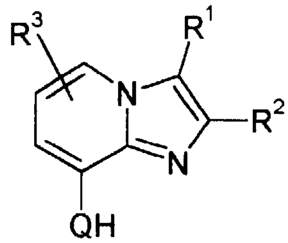

worin $R^1$, $R^2$, $R^3$ und Q jeweils wie in Anspruch 1 definiert sind, oder deren Salz, mit einer Verbindung der Formel:

$$X\text{-}A\text{-}R^4$$

worin X eine Abgangsgruppe ist, und $R^4$ und A jeweils wie in Anspruch 1 definiert sind, oder deren Salz, um eine Verbindung der Formel:

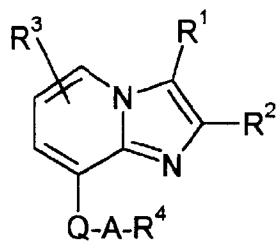

worin $R^1$, $R^2$, $R^3$, $R^4$, Q und A wie oben definiert sind, oder deren Salz zu ergeben.

**7.** Acylierung einer Verbindung der Formel:

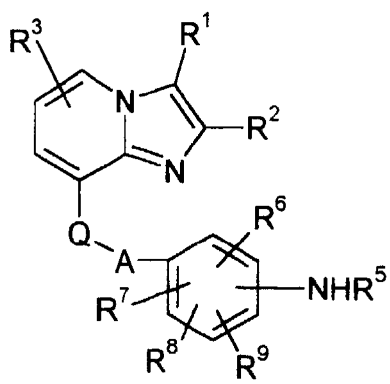

worin

$R^5$, Wasserstoff oder $(C_1\text{-}C_6)$Alkyl ist,
$R^6$, $R^7$, $R^8$ und $R^9$ jeweils Wasserstoff oder Halogen sind und
$R^1$, $R^2$, $R^3$, Q und A wie in Anspruch 1 definiert sind,

oder deren Salz, um eine Verbindung der Formel:

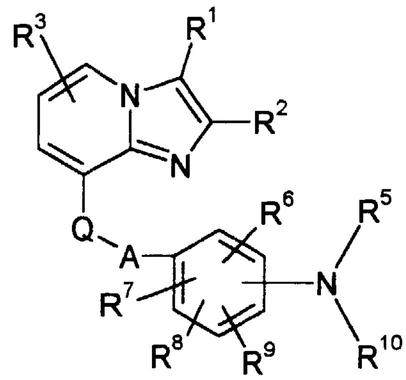

worin

$R^{10}$ Acyl wie in Anspruch 1 definiert ist, und
$R^1$, $R^2$, $R^3$, $R^5$, $R^6$, $R^7$, $R^8$, $R^9$, Q und A jeweils wie oben definiert sind,

oder deren Salz zu ergeben.

**8.** Acylierung einer Verbindung der Formel:

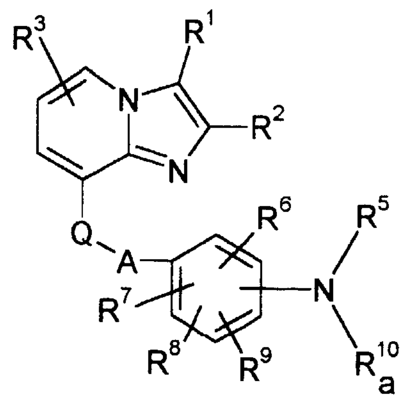

worin

| | |
|---|---|
| $R^{10}_a$ | Acyl wie in Anspruch 1 definiert mit Amino ist, |
| $R^1$, $R^2$, $R^3$, Q und A | jeweils wie in Anspruch 1 definiert sind und |
| $R^5$, $R^6$, $R^7$, $R^8$ und $R^9$ | jeweils wie in Anspruch 7 definiert sind, |

oder deren Salz, um eine Verbindung der Formel:

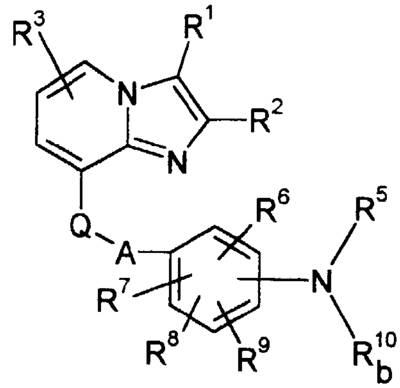

worin

| | |
|---|---|
| $R^{10}_b$ | Acyl mit Acylamino ist, worin Acyl jeweils wie in Anspruch 1 definiert ist, und |

$R^1$, $R^2$, $R^3$, $R^5$, $R^6$, $R^7$, $R^8$, $R^9$, Q und Ajeweils wie oben definiert sind,

oder deren Salz zu ergeben.

**9.** Alkylierung einer Verbindung der Formel:

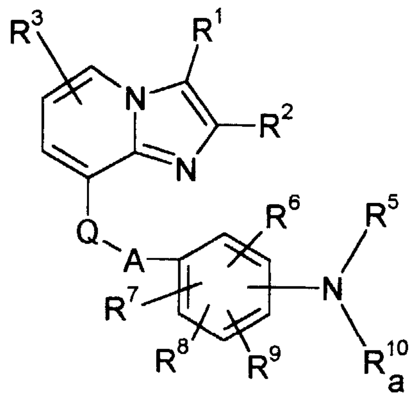

worin

| | |
|---|---|
| $R^1$, $R^2$, $R^3$, Q und A | wie in Anspruch 1 definiert sind, |
| $R^5$, $R^6$, $R^7$, $R^8$ und $R^9$ | jeweils wie in Anspruch 7 definiert sind, und |
| $R^{10}_a$ | wie in Anspruch 8 definiert ist, |

oder deren Salz, um eine Verbindung der Formel:

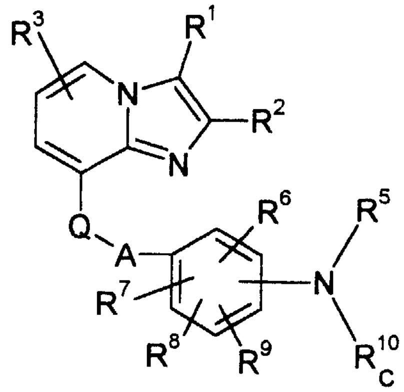

worin

| | |
|---|---|
| $R^{10}_c$ | Acyl mit $(C_1-C_6)$Alkylamino oder Acyl mit $Ar(C_1-C_6)$alkylamino ist, worin Acyl jeweils wie in Anspruch 1 definiert ist, und |
| $R^1$, $R^2$, $R^3$, $R^5$, $R^6$, $R^7$, $R^8$, $R^9$, Q und A | jeweils wie oben definiert sind |

oder deren Salz zu ergeben.

**10.** Reagieren einer Verbindung der Formel:

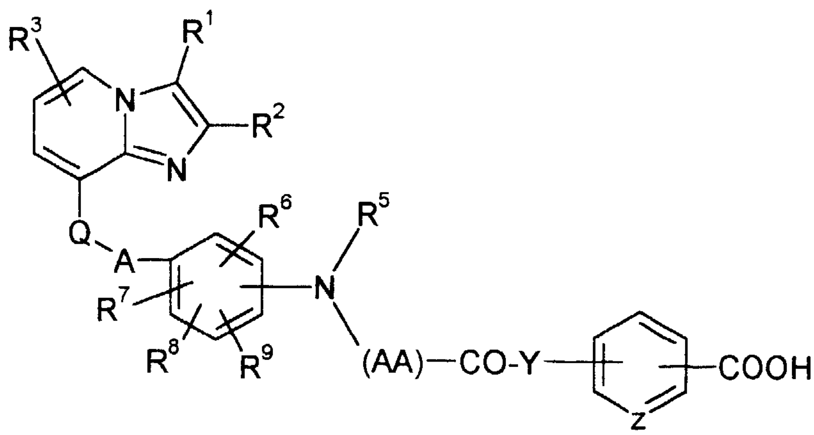

worin

| | |
|---|---|
| (AA) | ein Aminosäurerest wie in Anspruch definiert ist, |
| Y | NH oder $(C_2-C_6)$Alkenylen ist, |
| Z | CH oder N ist, |
| $R^1$, $R^2$, $R^3$, Q und A | jeweils wie in Anspruch 1 definiert sind und |
| $R^5$, $R^6$, $R^7$, $R^8$ und $R^9$ | jeweils wie in Anspruch 7 definiert sind, |

oder eines reaktiven Derivates an der Carboxygruppe oder eines Salzes davon mit einer Verbindung der Formel:

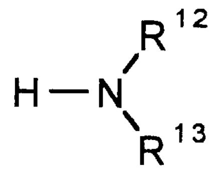

worin

| | |
|---|---|
| $R^{12}$ | Wasserstoff, $(C_1\text{-}C_6)$Alkyl, $(C_1\text{-}C_6)$Alkoxy$(C_1\text{-}C_6)$alkyl, $(C_1\text{-}C_6)$Alkylamino$(C_1\text{-}C_6)$alkyl, Heterocyclyl$(C_1\text{-}C_6)$alkyl, eine heterocyclische Gruppe, geschütztes oder ungeschütztes Hydroxy$(C_1\text{-}C_6)$alkyl oder Aryl wahlweise substituiert mit $(C_1\text{-}C_6)$Alkylamino ist und |
| $R^{13}$ | Wasserstoff, $(C_1\text{-}C_6)$Alkyl, $(C_1\text{-}C_6)$Alkoxy$(C_1\text{-}C_6)$alkyl oder geschütztes oder ungeschütztes Hydroxy$(C_1\text{-}C_6)$alkyl ist oder |
| $R^{12}$ und $R^{13}$ | zusammengenommen werden mit dem verbundenen Stickstoffatom, um eine heterocyclische Gruppe zu bilden, die wahlweise geeignete Substituent(en) besitzt, worin Aryl, die heterocyclische Gruppe oder der heterocyclische Anteil wie in Anspruch 1 definiert sind, |

oder deren reaktives Derivat an der Aminogruppe oder einem Salz davon, um eine Verbindung der Formel:

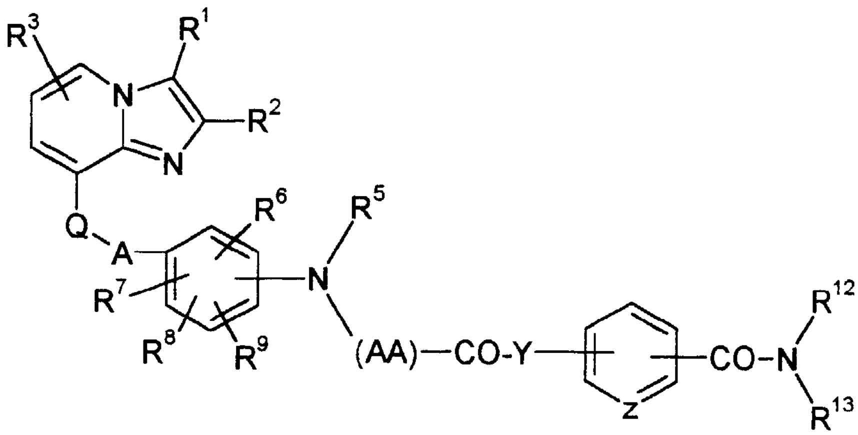

zu ergeben, worin $R^1$, $R^2$, $R^3$, $R^5$, $R^6$, $R^7$, $R^8$, $R^9$, $R^{12}$, $R^{13}$, A, (AA), Q, Y und Z jeweils wie oben definiert sind, oder deren Salz zu ergeben.

**11.** Pharmazeutische Zusammensetzung, die eine Verbindung nach Anspruch 1 als aktiven Bestandteil in Assoziierung mit pharmazeutisch verträglichen, im wesentlichen nichtgiftigen Trägern oder Exzipienten umfaßt.

**12.** Verbindung nach Anspruch 1 zur Verwendung als Medikament.

**13.** Verbindung nach Anspruch 1 für die Verwendung in der Prävention und/oder Behandlung von Erkrankungen, die durch Bradykinin oder dessen Analoge vermittelt werden.

**14.** Verwendung der Verbindung nach Anspruch 1 für die Herstellung eines Medikaments für die Prävention und/oder Behandlung von Erkrankungen, die durch Bradykinin oder dessen Analoge vermittelt werden.

**15.** Verbindung nach Anspruch 3, worin

$R^4$ Phenyl, substituiert mit 2 Halogenen und einer Gruppe der Formel:

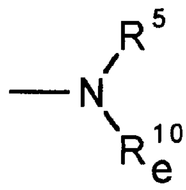

ist, worin

$R^5$ $(C_1-C_6)$Alkyl ist, und

$R^{10}_e$ Glycyl, substituiert mit $(C_1-C_6)$Alkylcarbamoyl-ar$(C_3-C_6)$alkenoyl ist.

**16.** Verbindung nach Anspruch 15, die 3-Brom-8-[2,6-dichlor-3-[N-[4-(dimethylcarbamoyl)cinnamoylglycyl]-N-methylamino]benzyloxy]-2-methylimidazo[1,2-a]pyridin und deren Säureadditionssalz ist.

**17.** Verbindung nach Anspruch 15, die 3-Brom-8-[2,6-dichlor-3-[N-methyl-N-[4-(methylcarbamoyl)cinnamoylqlycyl]amino]benzyloxy]-2-methylimidazo[1,2-a]pyridin und deren Säureadditionssalz ist.

## Revendications

**1.** Composé de formule :

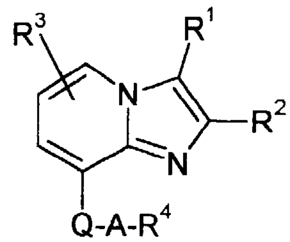

dans laquelle

$R^1$ est un halogène,

$R^2$ est un alkyle en $C_1$-$C_6$,

$R^3$ est un hydrogène,

$R^4$ est un phényle substitué avec un ou des substituants choisis dans le groupe constitué par un halogène; un alkyle en $C_1$-$C_6$; un haloalkyle en $C_1$-$C_6$; un carboxy; un alcoxy(en $C_1$-$C_6$)carbonyle; un benzoyle; un 4-méthyl-1-pipérazinylcarbonyle; un phényle; un phényle substitué avec un halogène ou un cyano; un phénylalkyle en $C_1$-$C_6$ substitué avec un hydroxy; un alcoxy en $C_1$-$C_6$; un nitro; un amino; un amino substitué avec un ou des substituants choisis dans le groupe constitué par un alkyle en $C_1$-$C_6$, un acyle qui est un alcanoyle en $C_1$-$C_6$, un haloalcanoyle en $C_1$-$C_6$, un hydroxyalcanoyle en $C_1$-$C_6$, un alkyl(en $C_1$-$C_6$)sulfonyloxyalcanoyle en $C_1$-$C_6$, un alcoxy(en $C_1$-$C_6$)alcanoyle en $C_1$-$C_6$, un alkyl(en $C_1$-$C_6$)thioalcanoyle en $C_1$-$C_6$, un alcanoyl(en $C_1$-$C_6$)oxyalcanoyle en $C_1$-$C_6$, un aryloxyalcanoyle en $C_1$-$C_6$, un aroylalcanoyle en $C_1$-$C_6$, un carboxyalcanoyle en $C_1$-$C_6$, un alcoxy(en $C_1$-$C_6$)carbonylalcanoyle en $C_1$-$C_6$, un carbamoylalcanoyle en $C_1$-$C_6$, un alkyl(en $C_1$-$C_6$)carbamoylalcanoyle en $C_1$-$C_6$, un aralcanoyle en $C_1$-$C_6$, un alcanoyle en $C_1$-$C_6$ hétérocyclique éventuellement substitué, un thioalcanoyle en $C_1$-$C_6$ hétérocyclique, un alcénoyle en $C_3$-$C_6$, un alcynoyle en $C_3$-$C_6$, un cycloalkyl-(en $C_3$-$C_6$)carbonyle, un cycloalcényl(en $C_3$-$C_6$)carbonyle, un carboxy, un alcoxy(en $C_1$-$C_6$)carbonyle, un aryloxycarbonyle, un aroyle, un alcoxy(en $C_1$-$C_6$)aroyle, un haloaroyle, un alcoxy(en $C_1$-$C_6$)carbonylaroyle, un aralcénoyle en $C_3$-$C_6$, un alcoxy(en $C_1$-$C_6$)-aralcénoyle en $C_3$-$C_6$, un alkylène(en $C_1$-$C_6$)dioxy-aralcénoyle en $C_3$-$C_6$, un nitro-aralcénoyle en $C_3$-$C_6$, un halo-aralcénoyle en $C_3$-$C_6$, un hydroxy-aralcénoyle en $C_3$-$C_6$, un hydroxyalcoxy(en $C_1$-$C_6$)-aralcénoyle en $C_3$-$C_6$, un aminoalcoxy(en $C_1$-$C_6$)-aralcénoyle en $C_3$-$C_6$, un alkyl(en $C_1$-$C_6$)aminoalcoxy(en $C_1$-$C_6$)-aralcénoyle en $C_3$-$C_6$, un (alcoxy(en $C_1$-$C_6$) hétérocyclique)-aralcénoyle en $C_3$-$C_6$, un (hétérocyclique éventuellement substitué)aralcénoyle en

$C_3$-$C_6$, un amino-aralcénoyle en $C_3$-$C_6$, un alkyl(en $C_1$-$C_6$)amino-aralcénoyle en $C_3$-$C_6$, un alcanoyl(en $C_1$-$C_6$)amino-aralcénoyle en $C_3$-$C_6$, un hydroxyalcanoyl(en $C_1$-$C_6$)amino-aralcénoyle en $C_3$-$C_6$, un alcoxy(en $C_1$-$C_6$)alcanoyl (en $C_1$-$C_6$)amino-aralcénoyle en $C_3$-$C_6$, un haloalcanoyl(en $C_1$-$C_6$)amino-aralcénoyle en $C_3$-$C_6$, un aminoalcanoyl(en $C_1$-$C_6$)amino-aralcénoyle en $C_3$-$C_6$, un alkyl(en $C_1$-$C_6$)amino-alcanoyl(en $C_1$-$C_6$)aminoaralcénoyle en $C_3$-$C_6$, un alcanoyl(en $C_1$-$C_6$)aminoalcanoyl(en $C_1$-$C_6$)amino-aralcénoyle en $C_3$-$C_6$, un carboxyalcanoyl(en $C_1$-$C_6$)amino-aralcénoyle en $C_3$-$C_6$, un alcoxy(en $C_1$-$C_6$)carbonylalcanoyl(en $C_1$-$C_6$)aminoaralcénoyle en $C_3$-$C_6$, un alcoxy(en $C_1$-$C_6$)carbonylalcénoyl(en $C_3$-$C_6$)amino-aralcénoyle en $C_3$-$C_6$, un haloalcoxy(en $C_1$-$C_6$)carbonylamino-aralcénoyle en $C_3$-$C_6$, un (alcanoyl(en $C_1$-$C_6$)amino hétérocyclique)-aralcénoyle en $C_3$-$C_6$, un aroylamino-aralcénoyle en $C_3$-$C_6$, un (carbonylamino hétérocyclique) -aralcénoyle en $C_3$-$C_6$, un alkyl(en $C_1$-$C_6$)sulfonylamino-aralcénoyle en $C_3$-$C_6$,
un N-(alcanoyl(en $C_1$-$C_6$))-N-(alkyl(en $C_1$-$C_6$))aminoaralcénoyle en $C_3$-$C_6$, un N-[alcoxy(en $C_1$-$C_6$)alcanoyl(en $C_1$-$C_6$)]-N-(alkyl(en $C_1$-$C_6$))amine-aralcénoyle en $C_3$-$C_6$, un N-(alcanoyl(en ($C_1$-$C_6$))-N-[alkyl (en $C_1$-$C_6$) hétérocyclique]amino-aralcénoyle en $C_3$-$C_6$, un uréido-aralcénoyle en $C_3$-$C_6$, un alkyl(en $C_1$-$C_6$)uréido-aralcénoyle en $C_3$-$C_6$, un (uréido hétérocyclique)aralcénoyle en $C_3$-$C_6$, un alcanoyl(en $C_1$-$C_6$)-aralcénoyle en $C_3$-$C_6$, un carboxy-aralcénoyle en $C_3$-$C_6$, un alcoxy(en $C_1$-$C_6$)carbonyl-aralcénoyle en $C_3$-$C_6$, un carbamoyl-aralcénoyle en $C_3$-$C_6$, un alkyl(en $C_1$-$C_6$)carbamoyl-aralcénoyle en $C_3$-$C_6$, un hydroxyalkyl(en $C_1$-$C_6$)carbamoyl-aralcénoyle en $C_3$-$C_6$, un N-[hydroxyalkyl(en $C_1$-$C_6$)]-N-(alkyl(en $C_1$-$C_6$))carbamoyl-aralcénoyle en $C_3$-$C_6$, un alcoxy(en $C_1$-$C_6$)alkyl(en $C_1$-$C_6$)carbamoyl-aralcénoyle en $C_3$-$C_6$, un N-[alcoxy(en $C_1$-$C_6$)alkyl(en $C_1$-$C_6$)]-N-(alkyl(en $C_1$-$C_6$))carbamoyl-aralcénoyle en $C_3$-$C_6$, un (carbamoyle hétérocyclique)-aralcénoyle en $C_3$-$C_6$, un (carbonyle hétérocyclique)-aralcénoyle en $C_3$-$C_6$, un aralcynoyle en $C_3$-$C_6$, un alcénoyle en $C_3$-$C_6$ hétérocyclique, un amino-alcénoyle en $C_3$-$C_6$ hétérocyclique, un alkyl (en $C_1$-$C_6$)amino-alcénoyle en $C_3$-$C_6$ hétérocyclique, un alcanoyl(en $C_1$-$C_6$)amino-alcénoyle en $C_2$-$C_6$ hétérocyclique, un alkyl(en $C_1$-$C_6$)uréido-alcénoyle en $C_3$-$C_6$ hétérocyclique, un carboxy-alcénoyle en $C_3$-$C_6$ hétérocyclique, un alcoxy(en $C_1$-$C_6$)carbonyl-alcénoyle en $C_3$-$C_6$ hétérocyclique, un alkyl(en $C_1$-$C_6$)carbamoylalcénoyle en $C_3$-$C_6$ hétérocyclique, un alcoxy(en $C_1$-$C_6$)alkyl(en $C_1$-$C_6$)carbamoyl-alcénoyle en $C_3$-$C_6$ hétérocyclique, un hydroxyalkyl(en $C_1$-$C_6$)carbamoylalcénoyle en $C_3$-$C_6$ hétérocyclique, un carbonyle hétérocyclique et un carbonyle hétérocyclique qui est substitué avec un substituant choisi parmi un 4-méthyl-1-pipérazinylcarbonyle, un 4-éthyl-1-pipérazinylcarbonyle, un diméthylaminopipéridinocarbonyle et un 4-méthylcarbamoyl-1-pipérazinylcarbonyle, un aryloxycarbonyle qui peut être substitué avec un nitro, un aralcoxy(en $C_1$-$C_6$)carbonyle qui peut être substitué avec un nitro, un carbamoyle, un thiocarbamoyle, un alkyl(en $C_1$-$C_6$)carbamoyle, un carboxyalkyl(en $C_1$-$C_6$)carbamoyle, un alcoxy(en $C_1$-$C_6$)carbonylalkyl (en $C_1$-$C_6$)carbamoyle, un alcényl (en $C_2$-$C_6$)carbamoyle, un cycloalkyl(en $C_3$-$C_6$)-carbamoyle, un haloalcanoyl(en $C_1$-$C_6$)carbamoyle, un arylcarbamoyle, un arylthiocarbamoyle, un alcoxy(en $C_1$-$C_6$)-arylcarbamoyle, un haloarylcarbamoyle, un haloalkyl(en $C_1$-$C_6$)-arylcarbamoyle, un nitro-arylcarbamoyle, un cyano-arylcarbamoyle, un hydroxyalkyl(en $C_1$-$C_6$)-arylcarbamoyle, un amine-arylcarbamoyle, un alkyl(en $C_1$-$C_6$)amine-arylcarbamoyle, un alcanoyl(en $C_1$-$C_6$)amino-arylcarbamoyle, un N- (alcanoyl (en $C_1$-$C_6$))-N-(alkyl (en $C_1$-$C_6$))amine-arylcarbamoyle, un alcoxy(en $C_1$-$C_6$)alcanoyl (en $C_1$-$C_6$)amino-arylcarbamoyle, un alcoxy(en $C_1$-$C_6$)carbonylalcanoyl(en $C_1$-$C_6$)amino-arylcarbamoyle, un carboxyamino-arylcarbamoyle, un alcoxy(en $C_1$-$C_6$)carbonylamino-arylcarbamoyle, un uréido-arylcarbamoyle, un alkyl(en $C_1$-$C_6$)uréido-arylcarbamoyle, un hydroxyiminoalkyl(en $C_1$-$C_6$)arylcarbamoyle, un alcoxy(en $C_1$-$C_6$)iminoalkyl(en $C_1$-$C_6$)arylcarbamoyle, un alkyl(en $C_1$-$C_6$)hydrazono-alkyl (en $C_1$-$C_6$)-arylcarbamoyle, un (hétérocyclique éventuellement substitué)-arylcarbamoyle, un carboxy-arylcarbamoyle, un alcoxy(en $C_1$-$C_6$)carbonyl-arylcarbamoyle, un (carbonyl hétérocyclique)arylcarbamoyle, un (carbonyl hétérocyclique)arylcarbamoyle substitué avec un alkyle en $C_1$-$C_6$, un (carbonyl hétérocyclique)-arylcarbamoyle substitué avec un aryle, un (carbonyl hétérocyclique)arylcarbamoyle substitué avec un groupe hétérocyclique, un (carbonyl hétérocyclique)arylcarbamoyle substitué avec un alcanoyle en $C_1$-$C_6$, un (carbonyl hétérocyclique)-arylcarbamoyle substitué avec alcoxy(en $C_1$-$C_6$)carbonyle, un (carbonyl hétérocyclique)-arylcarbamoyle substitué avec un alkyl (en $C_1$-$C_6$)amino, un (carbonyl hétérocyclique)arylcarbamoyle substitué avec un alkyl(en $C_1$-$C_6$)carbamoyle, un carbamoyl-arylcarbamoyle, un alkyl(en $C_1$-$C_6$)carbamoyl-arylcarbamoyle, un hydroxyalkyl(en $C_1$-$C_6$)carbamoyl-arylcarbamoyle, un N-[hydroxyalkyl(en $C_1$-$C_6$)]-N-(alkyl(en$C_1$-$C_6$))carbamoyl-arylcarbamoyle, un alcoxy(en $C_1$-$C_6$)-alkyl (en $C_1$-$C_6$)carbamoyl-arylcarbamoyle, un N-[alcoxy(en $C_1$-$C_6$)alkyl(en $C_1$-$C_6$)]-N-(alkyl(en $C_1$-$C_6$))carbamoyl-arylcarbamoyle, un alkyl(en $C_1$-$C_6$)aminoalkyl(en $C_1$-$C_6$)carbamoyl-arylcarbamoyle, un N-[alkyl (en $C_1$-$C_6$)aminoalkyl (en $C_1$-$C_6$)]-N-(alkyl(en $C_1$-$C_6$))carbamoyl-arylcarbamoyle, un (carbamoyl hétérocyclique)-arylcarbamoyle, un N-(hétérocyclique)-N-(alkyl(en $C_1$-$C_6$))carbamoyl-arylcarbamoyle, un (alkyl(en $C_1$-$C_6$)carbamoyl hétérocyclique)-arylcarbamoyle, un N-[alkyl (en $C_1$-$C_6$) hétérocyclique]-N-(alkyl (en $C_1$-$C_6$))carbamoylarylcarbamoyle, un N-[alkyl(en $C_1$-$C_6$) hétérocyclique]-N-(alcoxy(en $C_1$-$C_6$)alkyl (en $C_1$-$C_6$))carbamoyl-arylcarbamoyle, un arylcarbamoyl-arylcarbamoyle, un alkyl(en $C_1$-$C_6$)amine-arylcarbamoyl-arylcarbamoyle, un alcanoyl(en $C_1$-$C_6$)-arylcarbamoyle, un aralkyl(en $C_1$-$C_6$)carbamoyle, un carbamoyle hétérocyclique, un alkyl(en

$C_1$-$C_6$)carbamoyle hétérocyclique, un arylaminocarbamoyle, un aroylcarbamoyle, un alkyl(en $C_1$-$C_6$)sulfonyle, un arylsulfonyle, un aralkyl(en $C_1$-$C_6$)sulfonyle, un aralcényl(en $C_2$-$C_6$)sulfonyle, un phtaloyle, un résidu d'acide aminé où l'acide aminé est choisi parmi la glycine, la sarcosine, l'alanine, la bêta-alanine, la valine, la norvaline, la leucine, l'isoleucine, la norleucine, la sérine, la thréonine, la cystéine, la méthionine, la phénylalanine, la phénylglycine, le tryptophane, la tyrosine, la proline, l'hydroxyproline, l'acide glutamique, l'acide aspartique, la glutamine, l'asparagine, la lysine, l'arginine, l'histidine et l'ornithine, qui peut être substitué avec un ou des substituants choisis parmi un alkyle en $C_1$-$C_6$, un aryle, un aralkyle en $C_1$-$C_6$, un groupe hétérocyclique, un alkyle en $C_1$-$C_6$ hétérocyclique, un cycloalkyle choisi parmi un cyclopropyle, un cyclobutyle, un cyclopentyle, un cyclohexyle, un cycloheptyle, un cyclooctyle et un adamantyle; un amidino, un amidino substitué choisi parmi un méthylamidino et un N-éthyl-N'-cyanoamidino; un alcanoyle choisi parmi un formyle, un acétyle, un propionyle, un butyryle, un isobutyryle, un valéryle, un isovaléryle, un pivaloyle, un hexanoyle et un heptanoyle; un haloalcanoyle en $C_1$-$C_6$, un hydroxyalcanoyle en $C_1$-$C_6$, un alkyl(en $C_1$-$C_6$)sulfonyloxy-alcanoyle en $C_1$-$C_6$, un alcoxy(en $C_1$-$C_6$)alcanoyle en $C_1$-$C_6$, un aryloxyalcanoyle en $C_1$-$C_6$, un alkyl(en $C_1$-$C_6$)thioalcanoyle en $C_1$-$C_6$, un alkyl(en $C_1$-$C_6$)carbamoylalcanoyle en $C_1$-$C_6$, un alcanoyl(en $C_1$-$C_6$)oxyalcanoyle en $C_1$-$C_6$, un carboxyalcanoyle en $C_1$-$C_6$, un alcoxy(en $C_1$-$C_6$)carbonylalcanoyle en $C_1$-$C_6$, un aralcanoyle en $C_1$-$C_6$, un alcanoyle en $C_1$-$C_6$ hétérocyclique éventuellement substitué, un alcénoyle en $C_3$-$C_6$, un aralcénoyle en $C_3$-$C_6$, un alcoxy(en $C_1$-$C_6$)-aralcénoyle en $C_3$-$C_6$, un alkylène(en $C_1$-$C_6$)dioxy-aralcénoyle en $C_3$-$C_6$, un nitro-aralcénoyle en $C_3$-$C_6$, un halo-aralcénoyle en $C_3$-$C_6$, un hydroxy-aralcénoyle en $C_3$-$C_6$, un hydroxyalcoxy(en $C_1$-$C_6$)-aralcénoyle en $C_3$-$C_6$, un aminoalcoxy(en $C_1$-$C_6$)-aralcénoyle en $C_3$-$C_6$, un alkyl (en $C_1$-$C_6$)aminoalcoxy(en $C_1$-$C_6$)-aralcénoyle en $C_3$-$C_6$, un (alcoxy(en $C_1$-$C_6$)hétérocyclique)-aralcénoyle en $C_3$-$C_6$, un (hétérocyclique éventuellement substitué)aralcénoyle en $C_3$-$C_6$, un amino-aralcénoyle en $C_3$-$C_6$, un alkyl(en $C_1$-$C_6$)amino-aralcénoyle en $C_3$-$C_6$, un alcanoyl(en $C_1$-$C_6$)amino-aralcénoyle en $C_3$-$C_6$, un hydroxyalcanoyl (en $C_1$-$C_6$)amino-aralcénoyle en $C_3$-$C_6$, un alcoxy(en $C_1$-$C_6$)alcanoyl(en $C_1$-$C_6$)amino-aralcénoyle en $C_3$-$C_6$, un haloalcanoyl(en $C_1$-$C_6$)amino-aralcénoyle en $C_3$-$C_6$, un aminoalcanoyl(en $C_1$-$C_6$)amino-aralcénoyle en $C_3$-$C_6$, un alkyl(en $C_1$-$C_6$)aminoalcanoyl (en $C_1$-$C_6$)aminoaralcénoyle en $C_3$-$C_6$, un alcanoyl(en $C_1$-$C_6$)aminoalcanoyl (en $C_1$-$C_6$)amino-aralcénoyle en $C_3$-$C_6$, un carboxyalcanoyl (en $C_1$-$C_6$)amino-aralcénoyle en $C_3$-$C_6$, un alcoxy(en $C_1$-$C_6$)carbonylalcanoyl(en $C_1$-$C_6$)aminoaralcénoyle en $C_3$-$C_6$, un alcoxy(en $C_1$-$C_6$)carbonylalcénoyl(en $C_3$-$C_6$)amino-aralcénoyle en $C_3$-$C_6$, un haloalcoxy(en $C_1$-$C_6$)carbonylamino-aralcénoyle en $C_3$-$C_6$, un (alcanoyl(en $C_1$-$C_6$)amino hétérocyclique)-aralcénoyle en $C_3$-$C_6$, un aroylamino-aralcénoyle en $C_3$-$C_6$, un (carbonylamino hétérocyclique)-aralcénoyle en $C_3$-$C_6$, un alkyl(en $C_1$-$C_6$)sulfonylamino-aralcénoyle en $C_3$-$C_6$, un N-(alcanoyl(en $C_1$-$C_6$))-N-(alkyl(en $C_1$-$C_6$))amino-aralcénoyle en $C_3$-$C_6$, un N-[alcoxy(en $C_1$-$C_6$)alcanoyl(en $C_1$-$C_6$)]-N-(alkyl(en $C_1$-$C_6$))amino-aralcénoyle en $C_3$-$C_6$, un N-(alcanoyl(en $C_1$-$C_6$))-N-[alkyl(en $C_1$-$C_6$) hétérocyclique]amino-aralcénoyle en $C_3$-$C_6$, un uréido-aralcénoyle en $C_3$-$C_6$, un alkyl(en $C_1$-$C_6$)uréido-aralcénoyle en $C_3$-$C_6$, un (uréido hétérocyclique)aralcénoyle en $C_3$-$C_6$, un alcanoyl(en $C_1$-$C_6$)-aralcénoyle en $C_3$-$C_6$, un carboxy-aralcénoyle en $C_3$-$C_6$, un alcoxy(en $C_1$-$C_6$)carbonyl-aralcénoyle en $C_3$-$C_6$, un carbamoyl-aralcénoyle en $C_3$-$C_6$, un alkyl(en $C_1$-$C_6$)carbamoyl-aralcénoyle en $C_3$-$C_6$, un hydroxyalkyl(en $C_1$-$C_6$)carbamoyl-aralcénoyle en $C_3$-$C_6$, un N-[hydroxyalkyl (en $C_1$-$C_6$)]-N-(alkyl (en $C_1$-$C_6$))carbamoyl-aralcénoyle en $C_3$-$C_6$, un alcoxy(en $C_1$-$C_6$)alkyl (en $C_1$-$C_6$)carbamoyl-aralcénoyle en $C_3$-$C_6$, un N-[alkoxy(en $C_1$-$C_6$)alkyl(en $C_1$-$C_6$)]-N-(alkyl (en $C_1$-$C_6$))carbamoyl-aralcénoyle en $C_3$-$C_6$, un (carbamoyl hétérocyclique)-aralcénoyle en $C_3$-$C_6$, un (carbonyl hétérocyclique)-aralcénoyle en $C_3$-$C_6$, un aralcynoyle en $C_3$-$C_6$, un alcénoyle en $C_3$-$C_6$ hétérocyclique, un amino-alcénoyle en $C_3$-$C_6$ hétérocyclique, un alkyl(en $C_1$-$C_6$)amino-alcénoyle en $C_3$-$C_6$ hétérocyclique, un alcanoyl(en $C_1$-$C_6$)amino-alcénoyle en $C_3$-$C_6$ hétérocyclique, un alkyl(en $C_1$-$C_6$)uréido-alcénoyle en $C_3$-$C_6$ hétérocyclique, un carboxy-alcénoyle en $C_3$-$C_6$ hétérocyclique, un alcoxy(en $C_1$-$C_6$)carbonyl-alcénoyle en $C_3$-$C_6$ hétérocyclique, un alkyl(en $C_1$-$C_6$)carbamoyl-alcénoyle en $C_3$-$C_6$ hétérocyclique, un alcoxy(en $C_1$-$C_6$)alkyl(en $C_1$-$C_6$)carbamoyl-alcénoyle en $C_3$-$C_6$ hétérocyclique, un hydroxyalkyl(en $C_1$-$C_6$)carbamoyl-alcénoyle en $C_3$-$C_6$ hétérocyclique, un thioalcanoyle en $C_1$-$C_6$ hétérocyclique, un carbonyle hétérocyclique éventuellement substitué, un cycloalkyl(en $C_3$-$C_6$)carbonyle, un alcoxy(en $C_1$-$C_6$)carbonyle, un aryloxycarbonyle, un aroylalcanoyle en $C_1$-$C_6$, un aroyle, un nitro-aryloxycarbonyle, un carbamoyle, un alkyl(en $C_1$-$C_6$)carbamoyle, un alcoxy(en $C_1$-$C_6$)carbonylalkyl(en $C_1$-$C_6$)carbamoyle, un alcényl(en $C_2$-$C_6$)carbamoyle, un cycloalkyl(en $C_3$-$C_6$)carbamoyle, un arylcarbamoyle, un alcoxy(en $C_1$-$C_6$)-arylcarbamoyle, un haloalkyl(en $C_1$-$C_6$)arylcarbamoyle, un haloarylcarbamoyle, un hydroxyalkyl(en $C_1$-$C_6$)arylcarbamoyle, un nitro-arylcarbamoyle, un cyanoarylcarbamoyle, un amino-arylcarbamoyle, un alkyl (en $C_1$-$C_6$) amino-arylcarbamoyle, un alcanoyl(en $C_1$-$C_6$)amino-arylcarbamoyle, un N-(alcanoyl(en $C_1$-$C_6$))N-(alkyl(en $C_1$-$C_6$))amino-arylcarbamoyle, un alcoxy(en $C_1$-$C_6$)alcanoyl (en $C_1$-$C_6$)amino-arylcarbamoyle, un alcoxy(en $C_1$-$C_6$)carbonylalcanoyl (en $C_1$-$C_6$)aminoarylcarbamoyle, un carboxyamino-arylcarbamoyle, un alcoxy(en $C_1$-$C_6$)carbonylamino-arylcarbamoyle, un uréido-arylcarbamoyle, un alkyl(en $C_1$-$C_6$)uréido-arylcarbamoyle, un hydroxyiminoalkyl(en $C_1$-$C_6$)arylcarbamoyle, un alcoxy(en $C_1$-$C_6$)iminoalkyl (en $C_1$-$C_6$)-arylcarbamoyle, un alkyl(en $C_1$-$C_6$)hydrazonoalkyl(en $C_1$-$C_6$)-arylcarbamoyle, un (hétéro-

cyclique éventuellement substitué)arylcarbamoyle, un alcanoyl(en $C_1$-$C_6$)-arylcarbamoyle, un (carbonyl hétérocyclique)-arylcarbamoyle, un carboxy-arylcarbamoyle, un alcoxy(en $C_1$-$C_6$)carbonylarylcarbamoyle, un alkyl(en $C_1$-$C_6$)carbamoylarylcarbamoyle, un (carbonyl hétérocyclique)arylcarbamoyle ayant un alkyle en $C_1$-$C_6$, un (carbonyl hétérocyclique)-arylcarbamoyle ayant un aryle, un (carbonyl hétérocyclique)-arylcarbamoyle ayant un groupe hétérocyclique, un (carbonyl hétérocyclique)-arylcarbamoyle ayant un alcanoyle en $C_1$-$C_6$, un (carbonyl hétérocyclique)-arylcarbamoyle ayant un alcoxy(en $C_1$-$C_6$)carbonyle, un (carbonyl hétérocyclique)-arylcarbamoyle ayant un alkyl(en $C_1$-$C_6$)amino, un (carbonyl hétérocyclique)arylcarbamoyle ayant un alkyl(en $C_1$-$C_6$)carbamoyle, un hydroxyalkyl (en $C_1$-$C_6$)carbamoyl-arylcarbamoyle, un N-[hydroxyalkyl(en $C_1$-$C_6$)]-N-(alkyl (en $C_1$-$C_6$))carbamoyl-arylcarbamoyle, un alcoxy(en $C_1$-$C_6$)alkyl(en $C_1$-$C_6$)carbamoyl-arylcarbamoyle, un N-[alcoxy(en $C_1$-$C_6$)alkyl (en $C_1$-$C_6$)]-N-(alkyl(en $C_1$-$C_6$))carbamoylarylcarbamoyle, un alkyl(en $C_1$-$C_6$)aminoalkyl (en $C_1$-$C_6$)carbamoyl-arylcarbamoyle, un N-[alkyl(en $C_1$-$C_6$)aminoalkyl(en $C_1$-$C_6$)]-N-(alkyl(en $C_1$-$C_6$))carbamoylarylcarbamoyle, un (carbamoyl hétérocyclique)arylcarbamoyle, un N-(hétérocyclique)-N-(alkyl(en $C_1$-$C_6$))carbamoyl-arylcarbamoyle, un (alkyl (en $C_1$-$C_6$)carbamoyl hétérocyclique)-arylcarbamoyle, un N-[alkyl(en $C_1$-$C_6$)hétérocyclique]-N-(alkyl(en $C_1$-$C_6$))carbamoyl-arylcarbamoyle, un N-[alkyl(en $C_1$-$C_6$) hétérocyclique]-N-(alcoxy(en $C_1$-$C_6$)alkyl(en $C_1$-$C_6$)]carbamoyl-arylcarbamoyle, un arylcarbamoylarylcarbamoyle, un alkyl(en $C_1$-$C_6$)aminoarylcarbamoylarylcarbamoyle, un arylthiocarbamoyle, un aralkyl(en $C_1$-$C_6$)carbamoyle, un aroylcarbamoyle, un carbamoyle hétérocyclique, un alkyl(en $C_1$-$C_6$)carbamoyle hétérocyclique, un arylaminocarbamoyle, un aralcényl(en $C_2$-$C_6$)sulfonyle, un alkyl(en $C_1$-$C_6$)sulfonyle, un phtaloyle, un résidu d'acide aminé non substitué où l'acide aminé est tel que défini ci-dessus, un résidu d'acide aminé substitué avec un alkyle en $C_1$-$C_6$ où l'acide aminé est tel que défini ci-dessus, un résidu d'acide aminé substitué avec un groupe hétérocyclique où l'acide aminé est tel que défini ci-dessus, un résidu d'acide aminé substitué avec un alkyle en $C_1$-$C_6$ hétérocyclique où l'acide aminé est tel que défini ci-dessus, un résidu d'acide aminé où l'acide aminé est tel que défini ci-dessus substitué avec un cycloalkyle choisi parmi un cyclopropyle, un cyclobutyle, un cyclopentyle, un cyclohexyle, un cycloheptyle, un cyclooctyle et un adamantyle, un résidu d'acide aminé substitué avec un aryle où l'acide aminé est tel que défini ci-dessus, un résidu d'acide aminé substitué avec un alcanoyle en $C_1$-$C_6$ où l'acide aminé est tel que défini ci-dessus, un résidu d'acide aminé substitué avec un alcoxy(en $C_1$-$C_6$)carbonyle où l'acide aminé est tel que défini ci-dessus, un résidu d'acide aminé substitué avec un phtaloyle où l'acide aminé est tel que défini ci-dessus et un résidu d'acide aminé substitué avec un aralkyle en $C_1$-$C_6$ où l'acide aminé est tel que défini ci-dessus, où dans les définitions ci-dessus et ci-dessous,

le terme "aryle" est choisi parmi un phényle, un naphtyle, un phényle qui est substitué avec un alkyle en $C_1$-$C_6$,

le groupe ou fragment hétérocyclique est choisi parmi

- un groupe hétéromonocyclique de 3 à 8 membres, insaturé, contenant 1 à 4 atomes d'azote,
- un groupe hétéromonocyclique de 3 à 8 membres, saturé, contenant 1 à 4 atomes d'azote,
- un groupe hétérocyclique condensé de 7 à 12 membres, insaturé, contenant 1 à 5 atomes d'azote,
- un groupe hétéromonocyclique de 3 à 8 membres, insaturé, contenant un atome d'oxygène,
- un groupe hétérocyclique condensé de 7 à 12 membres, insaturé, contenant 1 à 2 atomes d'oxygène,
- un groupe hétéromonocyclique de 3 à 8 membres, insaturé, contenant un atome de soufre,
- un groupe hétérocyclique condensé de 7 à 12 membres, insaturé, contenant 1 à 2 atomes de soufre,
- un groupe hétéromonocyclique de 3 à 8 membres, insaturé, contenant 1 à 2 atomes d'oxygène et 1 à 3 atomes d'azote,
- un groupe hétéromonocyclique de 3 à 8 membres, saturé, contenant 1 à 2 atomes d'oxygène et 1 à 3 atomes d'azote,
- un groupe hétérocyclique condensé de 7 à 12 membres, insaturé, contenant 1 à 2 atomes d'oxygène et 1 à 3 atomes d'azote,
- un groupe hétéromonocyclique de 3 à 8 membres, insaturé, contenant 1 à 2 atomes de soufre et 1 à 3 atomes d'azote,
- un groupe hétéromonocyclique de 3 à 8 membres, saturé, contenant 1 à 2 atomes de soufre et 1 à 3 atomes d'azote,
- un groupe hétérocyclique condensé de 7 à 12 membres, insaturé, contenant 1 à 2 atomes de soufre et 1 à 3 atomes d'azote,

et les substituants de l'aryle comme définis ci-dessus et le groupe hétérocyclique comme défini ci-dessus sont choisis parmi un halogène; un haloalkyle en $C_1$-$C_6$; un alkyle en $C_1$-$C_6$; l'aryle mentionné ci-dessus éventuellement substitué avec un ou des substituants choisis parmi un halogène et un cyano, un aralkyle en $C_1$-$C_6$ substitué avec un hydroxy où la partie hétérocyclique est telle que définie ci-dessus; un alcoxy en $C_1$-$C_6$; un oxo; un nitro; un amino éventuellement substitué avec un alkyle en $C_1$-$C_6$, l'acyle

mentionné ci-dessus, un aralkyle en $C_1$-$C_6$ où la partie aryle est telle que définie ci-dessus, un alkyle en $C_1$-$C_6$ hétérocyclique où la partie hétérocyclique est telle que définie ci-dessus, un carboxyalkyle en $C_1$-$C_6$, un alkyl(en $C_1$-$C_6$)aminométhylène, ou un N-méthylpyrrolidinylidène; le groupe hétérocyclique mentionné ci-dessus éventuellement substitué avec un oxo.

un phénylalkyle en $C_1$-$C_6$, un pyridylalkyle en $C_1$-$C_6$, un carboxyalkyle en $C_1$-$C_6$, un alkyl(en $C_1$-$C_6$)amino-méthylène et un N-méthylpyrrolidinylidène; un pyrrolidinyle et un pyrrolidinyle substitué avec un oxo; ou un groupe hétérocyclique condensé de 7 à 12 membres, insaturé, contenant 1 à 2 atomes d'oxygène ou un groupe hétérocyclique condensé de 7 à 12 membres, insaturé, contenant 1 à 2 atomes de soufre et 1 à 3 atomes d'azote, chacun d'entre eux est substitué avec un ou des substituants choisis dans le groupe constitué par un halogène, un alkyle en $C_1$-$C_6$ et un oxo,

Q est O ou NH, et
A est un alkylène en $C_1$-$C_6$,

et les sels pharmaceutiquement acceptables de celui-ci.

**2.** Composé selon la revendication 1, dans lequel

$R^4$ est un phényle substitué avec des substituants choisis dans le groupe constitué de un halogène, un nitro, un amino et un groupe de formule:

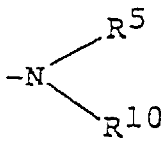
,

dans laquelle

$R^5$ est de un hydrogène ou un alkyle en $C_1$-$C_6$, et
$R^{10}$ est un acyle tel que défini dans la revendication 1, et

A est un méthylène.

**3.** Composé selon la revendication 2, dans lequel

$R^4$ est un phényle substitué avec un ou deux halogènes et un groupe de formule:

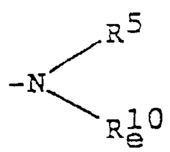
,

dans laquelle

$R^5$ est un hydrogène ou un alkyle en $C_1$-$C_6$, et
$R_e^{10}$ est un résidu d'acide aminé tel que défini dans la revendication 1 ou un résidu d'acide aminé tel que défini dans la revendication 1 substitué avec un substituant choisi dans le groupe constitué par un alkyle en $C_1$-$C_6$, un alcanoyle choisi parmi un formyle, un acétyle, un propionyle, un butyryle, un valéryle, un isovaléryle, un pivaloyle, un hexanoyle et un heptanoyle, un haloalcanoyle en $C_1$-$C_6$, un aralcanoyle en $C_1$-$C_6$, un alcanoyle en $C_1$-$C_6$ hétérocyclique, un alcénoyle en ($C_3$-$C_6$), un aralcénoyle en $C_3$-$C_6$, un alcoxy(en $C_1$-$C_6$)-aralcénoyle en $C_3$-$C_6$, un alkylène(en $C_1$-$C_6$)dioxy-aralcénoyle en $C_3$-$C_6$, un nitro-aralcénoyle en $C_3$-$C_6$, un halo-aralcénoyle en $C_3$-$C_6$, un hydroxy-aralcénoyle en $C_3$-$C_6$,

un hydroxyalcoxy(en $C_1$-$C_6$)-aralcénoyle en $C_3$-$C_6$, un aminoalcoxy(en $C_1$-$C_6$)aralcénoyle en $C_3$-$C_6$, un alkyl(en $C_1$-$C_6$)aminoalcoxy(en $C_1$-$C_6$)-aralcénoyle en $C_3$-$C_6$, un alcoxy(en $C_1$-$C_6$) hétérocyclique-aralcénoyle en $C_3$-$C_6$, un (hétérocyclique)-aralcénoyle en $C_3$-$C_6$ ayant éventuellement un oxo, un amino-aralcénoyle en $C_3$-$C_6$, un alkyl(en $C_1$-$C_6$)amino-aralcénoyle en $C_3$-$C_6$, un alcanoyl(en $C_1$-$C_6$)amino-aralcénoyle en $C_3$-$C_6$, un N-(alcanoyl(en $C_1$-$C_6$))-N-(alkyl (en $C_1$-$C_6$))amino-aralcénoyle en $C_3$-$C_6$, un hydroxyalcanoyl(en $C_1$-$C_6$)amino-aralcénoyle en $C_3$-$C_6$, un alcoxy(en $C_1$-$C_6$)alcanoyl(en $C_1$-$C_6$)amino-aralcénoyle en $C_3$-$C_6$, un haloalcanoyl(en $C_1$-$C_6$)amino-aralcénoyle en $C_3$-$C_6$, un aminoalcanoyl(en $C_1$-$C_6$)amino-aralcénoyle en $C_3$-$C_6$, un alkyl(en $C_1$-$C_6$)aminoalcanoyl (en $C_1$-$C_6$)amino-aralcénoyle en $C_3$-$C_6$, un alcanoyl (en $C_1$-$C_6$)aminoalcanoyl (en $C_1$-$C_6$)aminoaralcénoyle en $C_3$-$C_6$, un carboxyalcanoyl(en $C_1$-$C_6$)aminoaralcénoyle en $C_3$-$C_6$, un alcoxy(en $C_1$-$C_6$)carbonylalcanoyl(en $C_1$-$C_6$)amino-aralcénoyle en $C_3$-$C_6$, un alcoxy(en $C_1$-$C_6$)carbonylalcénoyl (en $C_3$-$C_6$)amino-aralcénoyle en $C_3$-$C_6$, un haloalcoxy(en $C_1$-$C_6$)carbonylamino-aralcénoyle en $C_3$-$C_6$, un (alcanoyl(en $C_1$-$C_6$)hétérocyclique)amino-aralcénoyle en $C_3$-$C_6$, un aroylamino-aralcénoyle en $C_3$-$C_6$, un (carbonylamino hétérocyclique)-aralcénoyle en $C_3$-$C_6$, un alkyl(en $C_1$-$C_6$)sulfonylamino-aralcénoyle en $C_3$-$C_6$, un N-[alcoxy(en $C_1$-$C_6$)alcanoyl(en $C_1$-$C_6$)]-N-(alkyl(en $C_1$-$C_6$))amino-aralcénoyle en $C_3$-$C_6$, un N-(alcanoyl (en $C_1$-$C_6$))-N-[alkyl en $C_1$-$C_6$ hétérocyclique]amino-aralcénoyle en $C_3$-$C_6$, un uréido-aralcénoyle en $C_3$-$C_6$, un alkyl(en $C_1$-$C_6$)uréido-aralcénoyle en $C_3$-$C_6$, un (uréido hétérocyclique)-aralcénoyle en $C_3$-$C_6$, un alcanoyl(en $C_1$-$C_6$)-aralcénoyle en $C_3$-$C_6$, un carboxy-aralcénoyle en $C_3$-$C_6$, un alcoxy(en $C_1$-$C_6$)carbonyl-aralcénoyle en $C_3$-$C_6$, un carbamoyl-aralcénoyle en $C_3$-$C_6$, un alkyl(en $C_1$-$C_6$)carbamoyl-aralcénoyle en $C_3$-$C_6$, un hydroxyalkyl(en $C_1$-$C_6$)carbamoyl-aralcénoyle en $C_3$-$C_6$, un N-[hydroxyalkyl(en $C_1$-$C_6$)]-N-(alkyl (en $C_1$-$C_6$))carbamoyl-aralcénoyle en $C_3$-$C_6$, un alcoxy(en $C_1$-$C_6$)alkyl(en $C_1$-$C_6$)carbamoyl-aralcénoyle en $C_3$-$C_6$, un N-[alcoxy(en $C_1$-$C_6$)alkyl(en $C_1$-$C_6$)]-N-(alkyl(en $C_1$-$C_6$))carbamoyl-aralcénoyle en $C_3$-$C_6$, un carbamoyl hétérocyclique-aralcénoyle en $C_3$-$C_6$, un carbonyl hétérocyclique-aralcénoyle en $C_3$-$C_6$, un aralcynoyle en $C_3$-$C_6$, un alcénoyle en $C_3$-$C_6$ hétérocyclique, un thioalcanoyle en $C_1$-$C_6$ hétérocyclique, un amino-alcénoyle en $C_3$-$C_6$ hétérocyclique, un alkyl(en $C_1$-$C_6$)amino-alcénoyle en $C_3$-$C_6$ hétérocyclique, un alcanoyl(en $C_1$-$C_6$)amino-alcénoyle en $C_3$-$C_6$ hétérocyclique, un alkyl(en $C_1$-$C_6$)uréido-alcénoyle en $C_3$-$C_6$ hétérocyclique, un carboxy-alcénoyle en $C_3$-$C_6$ hétérocyclique, un alcoxy(en $C_1$-$C_6$)carbonyl-alcénoyle en $C_3$-$C_6$ hétérocyclique, un alkyl(en $C_1$-$C_6$)carbamoyl-alcénoyle en $C_3$-$C_6$ hétérocyclique, un alcoxy(en $C_1$-$C_6$)alkyl(en $C_1$-$C_6$)carbamoyl-alcénoyle en $C_3$-$C_6$ hétérocyclique, un hydroxyalkyl(en $C_1$-$C_6$)carbamoyle-alcénoyle en $C_3$-$C_6$ hétérocyclique, un carbonyle hétérocyclique, un cyloalkyl(en $C_3$-$C_6$)carbonyle, un alcoxy(en $C_1$-$C_6$)carbonyle, un aryloxycarbonyle, un aroylalcanoyle en $C_1$-$C_6$, un aroyle, un nitro-aryloxycarbonyle, un carbamoyle, un alkyl(en $C_1$-$C_6$)carbamoyle, un alcoxy(en $C_1$-$C_6$)carbonylalkyl (en $C_1$-$C_6$)carbamoyle, un alcényl(en $C_2$-$C_6$)carbamoyle, un cycloalkyl(en $C_3$-$C_6$)carbamoyle, un arylcarbamoyle, un alcoxy(en $C_1$-$C_6$)-arylcarbamoyle, un haloalkyl(en $C_1$-$C_6$)-arylcarbamoyle, un haloarylcarbamoyle, un alcanoyl(en $C_1$-$C_6$)-arylcarbamoyle, un hydroxyalkyl(en $C_1$-$C_6$)-arylcarbamoyle, un (carbonyl hétérocyclique)-arylcarbamoyle, un carboxyarylcarbamoyle, un alcoxy(en $C_1$-$C_6$)carbonylarylcarbamoyle, un alkyl(en $C_1$-$C_6$)carbamoylearylcarbamoyle, un nitro-arylcarbamoyle, un cyano-arylcarbamoyle, un amino-arylcarbamoyle, un alkyl(en $C_1$-$C_6$)amino-arylcarbamoyle, un alcanoyl (en $C_1$-$C_6$)amino-arylcarbamoyle, un N-(alcanoyl(en $C_1$-$C_6$))-N-(alkyl(en $C_1$-$C_6$))amino-arylcarbamoyle, un alcoxy(en $C_1$-$C_6$)alcanoyl (en $C_1$-$C_6$)amino-arylcarbamoyle, un alcoxy(en $C_1$-$C_6$)carbonylalcanoyl(en $C_1$-$C_6$)amino-arylcarbamoyle, un carboxyamino-arylcarbamoyle, un alcoxy (en $C_1$-$C_6$)carbonylamino-arylcarbamoyle, un uréido-arylcarbamoyle, un alkyl(en $C_1$-$C_6$)uréido-arylcarbamoyle, un hydroxyiminoalkyl(en $C_1$-$C_6$)arylcarbamoyle, un alcoxy(en $C_1$-$C_6$)iminoalkyl(en $C_1$-$C_6$)arylcarbamoyle, un alkyl(en $C_1$-$C_6$)hydrazonoalkyl(en $C_1$-$C_6$)-arylcarbamoyle, un (hétérocyclique)arylcarbamoyle ayant éventuellement un oxo, un (carbonyl hétérocyclique)-arylcarbamoyle ayant un alkyle en $C_1$-$C_6$, un (carbonyl hétérocylique)arylcarbamoyle ayant un aryle, un (carbonyl hétérocylique)-arylcarbamoyleayant un groupe hétérocyclique, un (carbonyl hétérocyclique)arylcarbamoyle ayant un alcanoyle en $C_1$-$C_6$, un (carbonyl hétérocyclique)-arylcarbamoyle ayant un alcoxy(en $C_1$-$C_6$)carbonyle, un (carbonyl hétérocyclique)-arylcarbamoyle ayant un alkyl(en $C_1$-$C_6$)amino, un (carbonyl hétérocyclique)arylcarbamoyle ayant un alkyl(en $C_1$-$C_6$)carbamoyle, un hydroxyalkyl(en $C_1$-$C_6$)carbamoyl-arylcarbamoyle, un N-[hydroxyalkyl(en $C_1$-$C_6$)]-N-(alkyl(en $C_1$-$C_6$))carbamoylarylcarbamoyle, un alcoxy(en $C_1$-$C_6$)alkyl(en $C_1$-$C_6$)carbamoyl-arylcarbamoyle, un N-[alcoxy(en $C_1$-$C_6$)alkyl(en $C_1$-$C_6$)]-N-(alkyl(en $C_1$-$C_6$))carbamoylarylcarbamoyle, un alkyl(en $C_1$-$C_6$)aminoalkyl(en $C_1$-$C_6$)carbamoyl-arylcarbamoyle, un N-[alkyl (en $C_1$-$C_6$)aminoalkyl (en $C_1$-$C_6$)]]-N-(alkyl (en $C_1$-$C_6$))carbamoyl-arylcarbamoyle, un (carbamoyl hétérocyclique)-arylcarbamoyle, un N-(hétérocyclique)-N-(alkyl(en $C_1$-$C_6$))carbamoyl-arylcarbamoyle, un (alkyl(en $C_1$-$C_6$)carbamoyl hétérocyclique)arylcarbamoyle, un N-[alkyl(en $C_1$-$C_6$)hétérocycli-

que]-N-alkyl(en $C_1$-$C_6$)carbamoyl-arylcarbamoyle, un N-[alkyl(en $C_1$-$C_6$)hétérocyclique]-N-[alcoxy(en $C_1$-$C_6$)alkyl(en $C_1$-$C_6$)]carbamoyl-arylcarbamoyle, un arylcarbamoyl-arylcarbamoyle, un alkyl(en $C_1$-$C_6$)aminoarylcarbamoyl-arylcarbamoyle, un arylthiocarbamoyle, un aralkyl(en $C_1$-$C_6$)carbamoyle, un aroylcarbamoyle, un carbamoyle hétérocyclique, un alkyl(en $C_1$-$C_6$)carbamoyle hétérocyclique, un arylaminocarbamoyle, un aralcényl(en $C_2$-$C_6$)sulfonyle, un alkyl(en $C_1$-$C_6$)sulfonyle, un phtaloyle, un résidu d'acide aminé tel que défini dans la revendication 1, un résidu d'acide aminé tel que défini dans la revendication 1 substitué avec un alkyle en $C_1$-$C_6$, un résidu d'acide aminé tel que défini dans la revendication 1 substitué avec un groupe hétérocyclique, un résidu d'acide aminé tel que défini dans la revendication 1 substitué avec un alkyle en $C_1$-$C_6$ hétérocyclique, un résidu d'acide aminé tel que défini dans la revendication 1 substitué avec un cycloalkyle choisi parmi un cyclopropyle, un cyclobutyle, un cyclopentyle, un cyclohexyle, un cycloheptyle, un cyclooctyle et un adamantyle, un résidu d'acide aminé tel que défini dans la revendication 1 substitué avec un aryle, un résidu d'acide aminé tel que défini dans la revendication 1 substitué avec un alcanoyle choisi parmi un formyle, un acétyle, un propionyle, un butyryle, un isobutyryle, un valéryle, un isovaléryle, un pivaloyle, un hexanoyle, un heptanoyle, un résidu d'acide aminé tel que défini dans la revendication 1 substitué avec un alcoxy(en $C_1$-$C_6$)carbonyle, un résidu d'acide aminé tel que défini dans la revendication 1 substitué avec un aralkyle en $C_1$-$C_6$ et un résidu d'acide aminé tel que défini dans la revendication 1 substitué avec un phtaloyle; dans les définitions ci-dessus, un aryle, un groupe ou un fragment hétérocyclique sont chacun tels que définis dans la revendication 1.

**4.** Composé selon la revendication 1, dans lequel

$R^4$ est un groupe hétérocyclique condensé de 7 à 12 membres insaturé contenant 1 à 2 atomes d'oxygène ou un groupe hétérocyclique condensé de 7 à 12 membres insaturé contenant 1 à 2 atomes de soufre et 1 à 3 atomes d'azote, où chacun d'eux est substitué avec un ou des substituants choisis dans le groupe constitué par un halogène, un alkyle en $C_1$-$C_6$ et un oxo.

**5.** Halogénation d'un composé de formule :

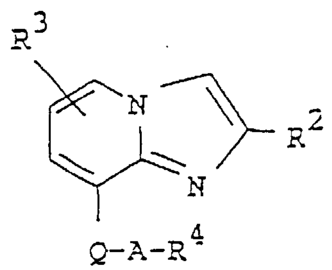

dans laquelle $R^2$, $R^3$, $R^4$, Q et A sont chacun tels que définis dans la revendication 1, ou de son sel pour donner un composé de formule :

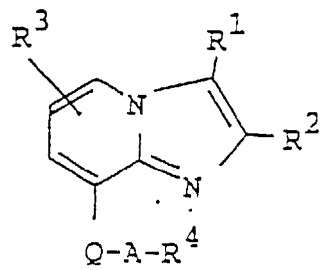

dans laquelle $R^1$, $R^2$, $R^3$, $R^4$, Q et A sont chacun tels que définis dans la revendication 1 ou son sel.

**6.** Réaction d'un composé de formule :

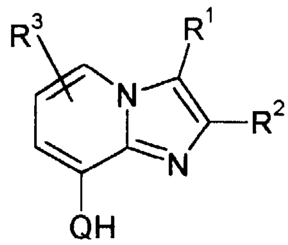

dans laquelle $R^1$, $R^2$, $R^3$ et Q sont chacun tels que définis dans la revendication 1 ou de son sel avec un composé de formule :

$$X\text{-}A\text{-}R^4$$

dans laquelle X est un groupe partant, et

$R^4$ et A sont tels que définis dans la revendication 1, ou son sel pour donner un composé de formule :

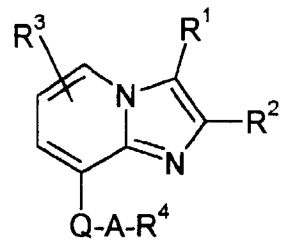

dans laquelle $R^1$, $R^2$, $R^3$, $R^4$, Q et A sont chacun tels que définis ci-dessus ou son sel.

**7.** Acylation d'un composé de formule

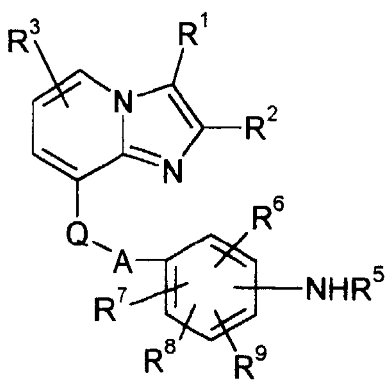

dans laquelle $R^5$ est un hydrogène ou un alkyle en $C_1$-$C_6$, $R^6$, $R^7$, $R^8$ et $R^9$ sont chacun un hydrogène ou un halogène, et $R^1$, $R^2$, $R^3$, Q et A sont chacun tels que définis dans la revendication 1 ou de son sel pour donner un composé de formule :

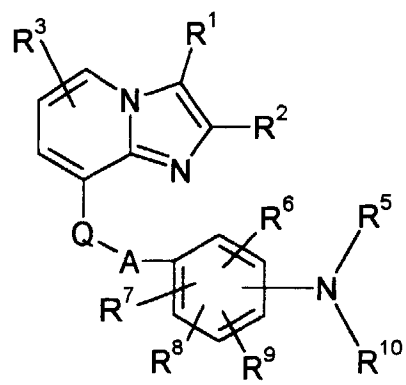

dans laquelle $R^{10}$ est un acyle tel que défini dans la revendication 1, et

$R^1$, $R^2$, $R^3$, $R^5$, $R^6$, $R^7$, $R^8$, $R^9$, Q et A sont chacun tels que définis ci-dessus,

ou son sel.

**8.** Acylation d'un composé de formule:

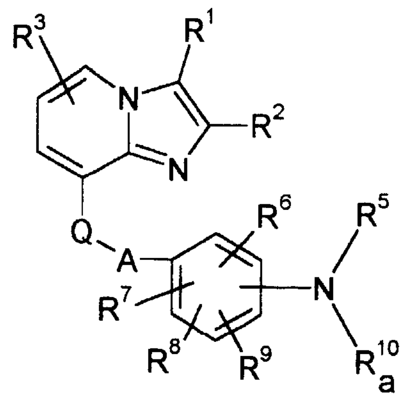

dans laquelle $R_a^{10}$ est un acyle tel que défini dans la revendication 1 comprenant un amino, $R^1$, $R^2$, $R^3$, Q et A sont chacun tels que définis dans la revendication 1, et $R^5$, $R^6$, $R^7$, $R^8$ et $R^9$ sont chacun tels que définis dans la revendication 7,

ou de son sel pour donner un composé de formule :

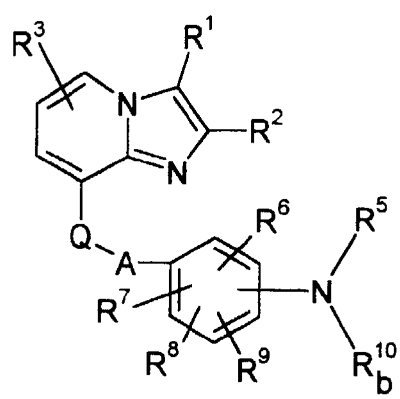

dans laquelle $R_b^{10}$ est un acyle comprenant un acylamino où chaque acyle est tel que défini dans la revendication 1, et $R^1$, $R^2$, $R^3$, $R^5$, $R^6$, $R^7$, $R^8$, $R^9$, Q et A sont chacun tels que définis ci-dessus,
ou son sel.

**9.** Alkylation d'un composé de formule:

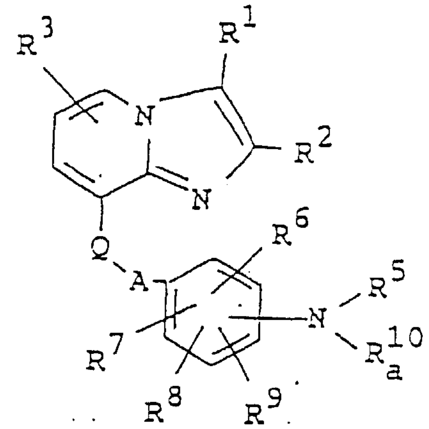

dans laquelle $R^1$, $R^2$, $R^3$, Q et A sont chacun tels que définis dans la revendication 1, $R^5$, $R^6$, $R^7$, $R^8$ et $R^9$ sont chacun tels que définis dans la revendication 7, et

$R_a^{10}$ est tel que défini dans la revendication 8 ou de son sel pour donner un composé de formule :

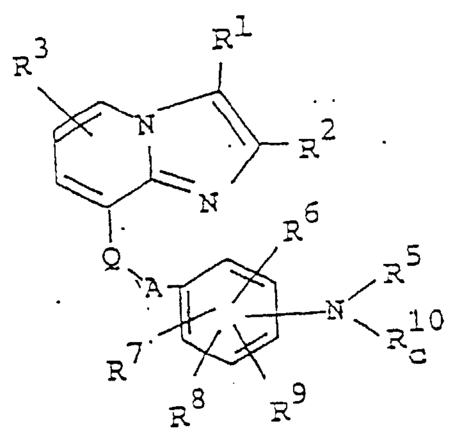

dans laquelle $R_c^{10}$ est un acyle comprenant un alkyl(en $C_1$-$C_6$)amino ou un acyle comprenant un aralkyl(en $C_1$-$C_6$)amino où chaque acyle est tel que défini dans la revendication 1, et $R^1$, $R^2$, $R^3$, $R^5$, $R^6$, $R^7$, $R^8$, $R^9$, Q et A sont chacun tels que définis ci-dessus,
ou son sel.

**10.** Réaction d'un composé de formule:

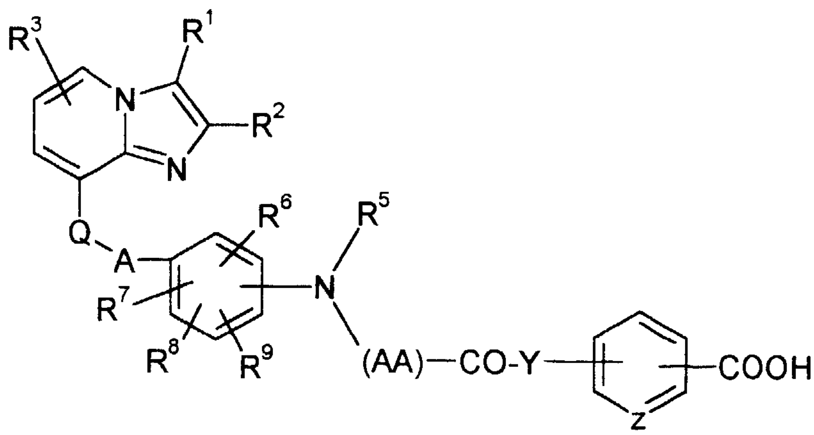

dans lequel (AA) est un résidu d'acide aminé tel que défini dans la revendication 1,

Y est NH ou un alcénylène en $C_2$-$C_6$,
Z est CH ou N,
$R^1$, $R^2$, $R^3$, Q et A sont chacun tels que définis dans la revendication 1 et $R^5$, $R^6$, $R^7$, $R^8$ et $R^9$ sont chacun tels que définis dans la revendication 7,

ou de son dérivé réactif au groupe carboxy ou d'un sel de celui-ci avec un composé de formule:

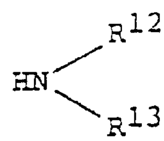

dans laquelle

$R^{12}$ est un hydrogène, un alkyle en $C_1$-$C_6$, un alcoxy(en $C_1$-$C_6$)alkyle en $C_1$-$C_6$, un alkyl(en $C_1$-$C_6$)aminoalkyle en $C_1$-$C_6$, un alkyle en $C_1$-$C_6$ hétérocyclique, un groupe hétérocyclique, un hydroxyalkyle en $C_1$-$C_6$ protégé ou non protégé ou un aryle éventuellement substitué avec un alkyl(en $C_1$-$C_6$)amino, et
$R^{13}$ est un hydrogène, un alkyle en $C_1$-$C_6$, un alcoxy(en $C_1$-$C_6$)alkyle en $C_1$-$C_6$ ou un hydroxyalkyle en $C_1$-$C_6$ protégé ou non protégé, ou
$R^{12}$ et $R^{13}$ sont pris conjointement avec l'atome d'azote lié pour former un groupe hétérocyclique comprenant éventuellement un ou des substituants appropriés, où l'aryle, le groupe ou fragment hétérocyclique sont chacun tels que définis dans la revendication 1,

ou de son dérivé réactif au groupe amino ou d'un sel. de celui-ci pour donner un composé de formule :

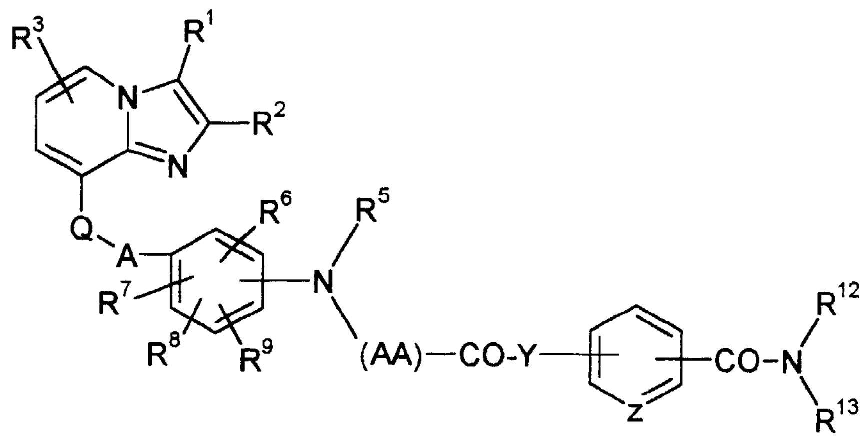

dans laquelle $R^1$, $R^2$, $R^3$, $R^5$, $R^6$, $R^7$, $R^8$, $R^9$, $R^{12}$, $R^{13}$, A, (AA), Q, Y et Z sont chacun tel que définis ci-dessus ou son sel.

**11.** Composition pharmaceutique comprenant un composé de la revendication 1, en tant qu'ingrédient actif, en association avec un support ou un excipient essentiellement non-toxique pharmaceutiquement acceptable.

**12.** Composé de la revendication pour utilisation comme médicament.

**13.** Composé de la revendication pour utilisation dans la prévention et/ou le traitement des maladies à médiation par la bradykinine ou ses analogues.

**14.** Utilisation d'un composé de la revendication pour la fabrication d'un médicament pour la prévention et/ou le traitement des maladies à médiation par la bradykinine ou ses analogues.

**15.** Composé de la revendication 3, dans lequel

$R^4$ est un phényle substitué avec deux halogènes et un groupe de formule :

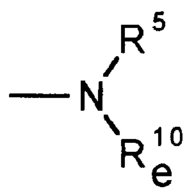

dans laquelle
$R^5$ est un alkyle en $C_1$-$C_6$, et
$R_e^{10}$ est un glycyle substitué avec un alkyl(en $C_1$-$C_6$)carbamoyl-aralcénoyle en $C_3$-$C_6$.

**16.** Composé de la revendication 15, qui est le 3-bromo-8-[2,6-dichloro-3-[N-[4-(diméthylcarbamoyl)cinnamoylglycyl]-N-méthylamino]benzyloxy]-2-méthylimidazo[1,2-a]pyridine et son sel d'addition avec un acide.

**17.** Composé de la revendication 15, qui est le 3-bromo-8-[2,6-dichloro-3-[N-méthyl-N-[4-(méthylcarbamoyl)cinnamoylglycyl]amino]benzyloxy]-2-méthylimidazo[1,2-a]pyridine et son sel d'addition avec un acide.